# EUROPEAN PATENT APPLICATION

(11) **EP 0 818 465 A1**
(43) Date of publication of application: **14.01.1998**
(21) Application number: 96730001.3
(22) Date of filing: 12.07.1996
(51) Int. Cl.: C07K 14/195, C12N 15/74, C12N 15/31

(54) **Genomic sequence of Rhizobium sp. NGR234 symbiotic plasmid**

(71) Applicant: Institute of Molecular Biotechnology (IMB) Department of Genome Analysis, 07745 Jena (DE); Laboratoire de Biologie Moléculaire des Plantes Supérieures University of Geneva, 1292 Chambésy/Geneva (CH)
(72) Inventor: Broughton, William John, 1292 Chambésy, Genf (CH); Rosenthal, André, 07751 Zöllnitz b. Jena (DE); Freiberg, Christoph Bernward, 37083 Göttingen (DE); Perret, Xavier Philippe, 1205 Genf (CH)
(74) Representative: Mercer, Christopher Paul

(57) **Abstract**

The sequencing and analysis of the complete nucleotide sequence of symbiotic plasmid pNGR234*a* isolated from *Rhizobium sp*. NGR234. The complete sequence of pNGR234*a* is presented. The analysis includes the identification of a number of novel ORFs and the proteins expressible therefrom which have been ascribed putative functions.

SEQUENCE LISTING

(1) GENERAL INFORMATION:

(i) APPLICANTS:

(A) NAME: Institute of Molecular Biotechnology (IMB)
Department of Genome Analysis
(B) STREET: Beutenbergstrasse 11
(C) CITY: Jena
(E) COUNTRY: DE
(F) POSTAL CODE : 07745

(A) NAME: Laboratoire de Biologie Moléculaire
des Plantes Supérieures University of Geneva
(B) STREET: 1 Chemin de l'Impératrice
(C) CITY: Chambésy/Geneva
(E) COUNTRY: CH
(F) POSTAL CODE : 1292

(ii) TITLE OF INVENTION: Genomic sequence of NGR234 symbiotic plasmid,
its gene map, and its use in diagnostics and gene transfer in
agriculture

(iii) NUMBER OF SEQUENCES: 1

(iv) CORRESPONDENCE ADDRESS:
(A) ADDRESSEE: Carpmaels & Ransford
(B) STREET: 43, Bloomsbury Square
(C) CITY: London
(E) COUNTRY: GB
(F) POSTAL CODE: WC1A 2RA

(v) COMPUTER READABLE FORM:
(A) MEDIUM TYPE: Floppy disk 720K
(B) COMPUTER: PC
(C) OPERATING SYSTEM: MS-DOS
(D) SOFTWARE: ASCII Text editor

(vi) CURRENT APPLICATION DATA:
(A) APPLICATION NUMBER: EP96730001.3
(B) FILING DATE: 12th July 1996

(2) INFORMATION FOR SEQ ID NO:1:

(i) SEQUENCE CHARACTERISTICS:
(A) LENGTH: 536165 base pairs
(B) TYPE: DNA
(C) STRANDEDNESS: double
(D) TOPOLOGY: linear

(ii) MOLECULE TYPE: DNA (genomic)

(iii) HYPOTHETICAL: No

(xi) SEQUENCE DESCRIPTION:SEQ ID NO:1:

GATCTCGATT GGCAGAACCG GCACCGCGCG CCAGCTAACT ACGCCATAGA GCGCGAGCAG 60
GAAGCGCGTG AGTACGTTGC TGTTGGCGGC GCCGCCGCGC GCGCGGATCG CCTCGCGCGC 120
CTTCACCATA TGAGCCGCGT CGACGGAATC ACCGATCATC TTGAGCGCGA AGTAAGACTT 180
CACGCTTGCG CTCATATCGA AGGGCCCGTC ATGCACCAGC GGCCAGCCGC CGTGCGCGCC 240
TTGAGTGCGG CGCAGATAAT TTCCGATTTT GGCTTCGAGC ACGACGTCAA TGGGCTCGGC 300
CAGGTAATGA CGTAGCAGGA TGTATTCGGA AGGGATGGTG GAATCCGCTT CAAGCTCGAA 360
CGCCCAATGG CCGTCGGCAT GACGATAAGC GAGCAGCGCC TCAGTGGCTG AGGCGATGCT 420
CATTTCCAGC GCGGCGGGAT CGATGGCAGT GCGATTTCCG GAATGCTTAT TCACTGACTT 480
CCTGCGTGTT CGGCAGCGTG CAAAACCGTC TGGATTTATC AATCAAGTGT TTGTCTCTGA 540
AAGAGCCAGA TCGGCGGCAC GGTTACCCGA CCGCACCGAT CCCTCGATGG TTGCCGGCAA 600
ACCGGTTGCG GTCCAGTCGC CAGCAAGGAA CAGGTTTTTG CAACCGGTCA CGGGCCCTGG 660

ACGTAGCGCG TTCTGCTCCG GGGTGGCCTG GAATGTGGCC CGGCGCTCAC ACACGATCTG 720
CCACGGCGGC AGCTCGCCTG AGATCCCACC GGCCTCGCAC ACGTCGCGCC AGATCACTCG 780
CACGACCTCC TCACGCGGAA TGTCAAGCAG GCGGTCGCCA TTGCTAATGG TGACTGAAAG 840
CCGCTGCGGA TAGGCGAACA GCCACTCCAC GAGCCCGCCG ACCACGCCCA GGATCGGATC 900
CGCACCGACT GGCGGATCGA AGCGAAAATG GGCATTAACG ACGGCGCGGA ATTCGGTCGG 960
GGTTTTCAGG CCGGGCAGGA GCGTCGCGGC CGCCCGTGGC GGCACGGCGA GGATCACGGC 1020
GTCGTCGGGG CCGACCGCTA TCTTGTCGTC ACCGAAATCA AGTTCGCTGA TGATCTCGGC 1080
CGATTTGGCA AGCTTGCGCA GTTTATGGCT GAGGCGGACG GTGGCGCCCC GCCTCTCCAG 1140
GAGCTTGACC GCCGGCTCGA CCAGAACGGC GCTCAGTCCG TCGCGCGCGA CTAGCGGACG 1200
GCAAGCCTCG CCGCCCGCGA GCAGCGTTTC CCGTACGATG GCACCGGCAA GCCCTGCCGA 1260
GCCCTCCGGC GGATCGCAAT TCAGGGCGGC GAGCAGCAGC GGCCGCACCA GACGCCGGTA 1320
CAGCGTACCA TTGCAGGGTA TAGTGTTTCC GACCAGTTCA TCCGCGCCGG CCCATAGGAT 1380
TGGCGCGAGC TTCAGGTAGT CCCACAGCCT GGTGTCGGGG ACGCGACGCG CCTTGTGGAA 1440
CACCCAGGTC GGAAGCCTGC CGCCGCCGAG ATCGACCTGC CAGCGCTGTA CGGTGGAGAT 1500
GTCGACGAAG GGAAACTTGG CACTCGTCGG ACCGACAAGA CCGGATTCCG TTCCGATCGC 1560
ACGGGCGTAG TTGCGCACAT ATTGGTTGCC CGATAGAACC AGATGGTTGC CGTTGTCGAT 1620
GGTGAGATTG GTTGCGGAAT CGAAAAAGGA GCGGCATCGA CCGCCGGCCT GTTGCGTCGC 1680
TTCATAGACA TGCACCGGCA AACCTGCATT GCTCAACTGC ACGGCCGCGG AAAGGCCGGA 1740
GATCCCGGCG CCGATAATGT GAACATTTTT TGGCATCACA ACATCAAGGC GTAACGGAAG 1800
AGAATGGCGC GCTTTGTGAG ATTGGTTACA CGCACCGGTT CCCGGGGAGC CGCAAAACCC 1860
CTTATCAGCA GCAGGTCCAA GATCGCGCGG TAACATTTGG ACATGATGGT GGGGGCGCGT 1920
ACTATTCTGC GCGGGTTGCA ATTCATGATC TCGTCGGATT CGGCAAAATG CATCATCGCG 1980
CGTTGGGCCA GCGGTGCGCA CGCCTTGGGC AGCGCCTTGT CGGCGATGAC TTTATGTGGG 2040
TCGCTGCTGG TGATACCGGC GTGATCGAGG CTTTCGCGTG GTATATAGAG CCGACCCAGC 2100
CCAGCATCCT CGTCTATGTC GCGCAGGATG TTGGTGAGTT GCAGCGCACG ACCGAGGTGA 2160
TGCGCAAGTG CAATGCCATC CTCCTCGCTC AAGCCGAACA CACGCACCGA CATCCTTCCC 2220
ACGGCGCTGG CGACACGGTC GCAATAGAGA TCCAGGGTTG CCATTTTGGG CGCGCGGATG 2280
TCTTGTAGTA CGTCCATTTC CATGCCGTCG ACAACGGCTA GGAAATCCTC TCGCTTCAGC 2340
CCGAAGGTCG TTACCGAGGC GAGATAGTCC TTTAGCCGCG GCGGCGGTAC ACATTGGTAG 2400
AGCGCGTCAA TGTGATCGCG CCATTGCTGA AGTGCAGCAA GGCGGTGCTC GCGCGGTTCG 2460
TCGGAGTCGG CGATGTCGTC GACCTGGCGG CAGAAGCTGT AGATCTGGAA TATCGCCTCG 2520
CGCTGCACCG GCGGGAGGGT ACGCATTCCC AGATAGAACG AGCTGCCAAG GGCCGTAGAG 2580
CGGTGGTTCG CGTGGGCCGC CGCCTCCGCC CTCATACGTC GACAGTCGTC TTGAATCGCG 2640
TACCGCGGCC GAACGCACGG CGCGCGGCTT CGTTTGCCAT TGCGCCGACG CCATGGAGCA 2700
GCAGCTCAAG CGGCGACAGA TGCACGCGCT CCCTTAGAGG ATCGCGCATC TTCAGCATCG 2760
ACACGATCTT ATCGGCAAAA GACAGTATGA CGGAGACCTC GAACCGGAGG CGAAAGTCCT 2820
TCACCTGCGA CACCAGCGCT CCGCCCCCGT TGAGCAGCAC CTCGGCCTTC GCTGCGAGAG 2880
AGCGCAGGCA TTGTAGCATC TGCAACGAGG ATTTTGCGGC GCCCAGCTCT TCGATGGAGG 2940
CGCCGCTGGC TGAAAGCGCA TCGCGCGGAA TATAGACGCG ATTGAGATTA AGGAAATCCT 3000
TGCCGCAATC CTGCAGGTGG TTACAGACCT GCAGTCCCGC GCACAACACA TCCGATGCCG 3060
GCCACGTCGC GGCACTTTCG CCGTGGACGT CGAGCACAAA TCGGCCGACC GGCATCGCCG 3120
AGTATCGGCA ATAATCGATC ACCTCGTCCC AGTTCTCGTA ACGCTGCTTA GTCACGTCCA 3180
TTCGGAAGGC GGTGAGCAGA TCGAGTGCAT GACGCGGCGG CATGCCGCGT CGGTCAAGCG 3240
CAGTGCGTAG GTGTACAGCT TCTGCTCGCG TTTCGCCCTT GCCGAGCAGC TCGGCTTCCA 3300
GGAGGTCGAG GTACAGAAGC TTCTCGTCTG GCGCGAGCGT CGCGTGATCG GCAATGTCAT 3360
CGGCCATGCG GACGAATCGG TAGAAGGCAA GAATCAGCGC TCGGTGCCGC GGATGGATAA 3420
TCCACGACGC AACGGGGAAA TTTTTCTCGC GGTGCATTGC GTCCGACCTG GGGCCGCTGC 3480
CGCTCGTCAT TTAGCACCAT CGTCGGTTTT GCAGGCGTTG GATGCTGCTG AAATGCAGTC 3540
GGGCCCCCGA ATAAATCGTA CAATCCACGA AGGTACGGCG GCGGCCAAAG ACGTTATCGA 3600
CCGGCGCGCG TTTTTATCCC CGAGAATTAT CCGATGCGAA TCTGTTTTTC GAGTATACTG 3660
ATTCGTTCTG ACGAATTCTT TGCTGAAAGC GTCGGATGAG ACTATATCCC AGTAGCTAAA 3720
GGCGATTCGA CTACCGCGCA TTATGAATAC ACCACATCCC TGCCATGCAA ACATATCCCA 3780
CCACTCAATT ATTACGCGGT CATCTTCTTC GTAGATGAAC TCCGGAGCGC AAATCCTCTC 3840
CGCAATGGCG CGATTTGGTC GAAAAATCTT TTGGATTGCC GCGCGTCCTA TCACGGGGCG 3900
CTGCATCGGC CAGAGGCTCA TTGCATTTTC GTGGTACAGC TCAGCCAAAG CGATCGCATC 3960
ACCCTTATTG AATCGTCGAA TCCACTCTTC GACCGTCCCG CGCGCGCTCA TTGAAATGCT 4020
CCTTTCTCAA GACCTTTGCT ACCATCGGTC CGTCAGGATC CGGCATTTTC CAGCTGTCGA 4080
TTCGATAGCC AGAGGGATAT CGCCCGACTC GATCCCCAAA CATAATGAGG CACAAAGCGG 4140
CGCACAGCGA TTGCTGTGCG AGCATAAGCG CCGCGTTTCG TTCGTTCAGT CATTGCAGGT 4200
GATGTCCACG GTGATCACTC GATGCCGCTT CTAAAAGCGA TCGGTGCACC TTCAACGAAA 4260
GTCGCGGAGC CCCACGCAGC GTGGAGAAAC TCGCGTATGG GTCACTGGAC GGGTTGTGGC 4320
CGTGTTTTTT GTAGGAAACC GCGACTTTGG CAAAGCTCGA ACTCACCCTC TCAGTTGCGC 4380
GCGCGACGCC CTTTTCAGGC ACGGCACCGG CCCACGCTAG TGTTGCTGAA ATACAACCGT 4440
GATCCGAGTG GAAAAGCGGT CCACCGGAAG AGCTAAATCA GATGTATTTT TCACCAGAGT 4500
TTCACATAGT CTCGCTTTAT TGAAATCCAT TTTTTCAATG ACATAAATCT ATACGGTAGA 4560
TTCACAGCCG AGTATGCTTC AGCCACAGGC GGTTCTGCAG TCAGAACGTC GCGGTTCTCA 4620
ATTTGAGGAG TTATCAGTGA TGTTTAAGAC AGAACGAGGC TGCGATCGTG AAGGAATGGA 4680
TGAGCGTTTT TTCGAGTGCG GTGTCGCGCG AGACCGGTGC GCCACTGGCA GACAGTGTAG 4740
GTCATCGCTG CTCCGGTCAA TGTCACGGCG AAGACCGGCA CTTGTCCTGG GGCCGGCGGC 4800

GCCTATCCAC TGGGAACAAC GGCGGTCGAG CGGGCCTGTT TCTACCTGCC AATGCGAGCC 4860
TGATCACCCG TACCGGCGCT GTAGTCGGCT CCGAGTGCAG CGTTCAGGCA GGCGGTATAT 4920
TTTCACGGCC CCCCGTCCGC CGTCGATTAC GATGCTGCAT CTTCAGGCGG CACAAATCTT 4980
GCGCCGACGA ACCGCGAACT CGTCGACGCC GTTGATGAGC GGGTAGGGGT GATCGCGGAA 5040
GAGACCGGGC GGCCAGACGT TTGCGGAACG CACAAGCGAC TCGCCCGTGA CCCGCAGGCC 5100
GATGGTGTGG GAGGGAGCGG CGCCGTGGGG CGTCTTTGCA AACCGGTGTC AGTGCCAGAG 5160
TCGCGACCCG CCTTAATTAT CAGCCACATT GCTGACAAAC GATTCATAGG ATTTGGCCAG 5220
TCCCAGCTCG AGCGAGGTCT TCGGGCGCCA ACCCATCGAC ACGAGTCTTT CGCTAGATAA 5280
AAGCTTTCGT GGCGTTCCGT CCGGCTTGGA TGTGTCGAAG ACTATATCGC CTTTAAAACC 5340
AACAACACGG CAGACGATGT GGGCTAGTTC AATGATACTT ATTTCCCCCC CGGAGCCTAT 5400
GTTAATGTGT TCCGTTTCGG AATAATGCTT AAGTAGGAAG ACCAGGGCGT CGGAGCAGTC 5460
TTCACTGTAC AAAAAGTCTC GAGTAGGTGT GCCGCTTCCC CATATAGACA AGCACCCAAG 5520
GTCTTTAATC TTTGCCTCAT GTGCTTTGCG TATTAAGGCA GGGACGACGT GGCTGGAGTT 5580
AAGATCGAAC TTATCGCGTG GGCCATAGAG ATTTGTCGGC ATGGCTGATA TGAAGTTTGC 5640
GCCGTATTGC TTACGATACG CTTGACACAA CTTAATGCCG GCGATTTTGG CGATCGCATA 5700
CCACTCGTTG GTCGGCTCAA GTGGTCCGGT TAATAGAGCC TCTTCCCTTA TGGGCTGCGC 5760
CGCATACTTC GGATATATGC AACTCGATCC AAGGAAAAGA AGCTTTTCAA CGCCACTGCG 5820
GAAGGAGCCC TCAATGACAT TAGCCTCCAT GATAAGGTTT TGATAGATGA AGTCAGCGGG 5880
CATAGTATCA TTTGCCAGGA TCCCACCCAC CTTCGCTGCC GCCATTATGA CCGCGTGCGG 5940
CTTTTCCTTT AATAGAAATT TCTCAACTTC CTCTTGCCGC GTCAGATCAA GCTTTTGCCT 6000
ATCTGCAACG ATGACTTCGC AATCCTCGGA GGCAAGCGAT CGAATTATGG CGCTGCCGAC 6060
CATACCTTTG TGTCCTGCGA CCCAAATACG CTTTCCGTCT AGCAAATACA TCGGCATCTC 6120
GCCTCTTACT GTCGGGTGCC CCTCAAATAA GAGAGATCTT CCCTTACCAT TTCGCAAGCA 6180
AGATCTCTCA CACTTGTCTC GTGCCTCCAG CCCAAAACCT GCCGGGCCTT GGTGGCATCG 6240
CCTAAAAGTA AATCTACTTC CGTGGGCCTG AAATAGCGCG GATCCACCGC AACGACGCAT 6300
CTGCCTGTCG CGGCGTCTAT GCCCCTTTCT TCAATGCCTT CTCCCACCCA TTCAATCGTC 6360
ATTCCGGTTT CCTCGAAGGC CCATTCGACA AACGTGCGAA CCGATGTTGT CACCCCGGTA 6420
GCCAGGACAT AGTCGCCTGG TCTATCCTGT TGACACATCA TCCACATGCC CCGCACATAC 6480
TCACGGGCAT GTCCCCAGTC ACGTTGAGCA TCTAGATTTC CAAGATAAAG GACCTCTTGT 6540
TTACCTAGAC TGATTGCCGC TGCAGCCCGG GTGATCTTGC GAGTCACGAA CGTCTCCCCA 6600
CGAAGCGGAC TTTCGTGGTT GAAAAGAATA CCGTTGGAGG CATGCATGCC GTAAGCCTCT 6660
CTATAATTTA CGACAATCCA GTACGCGTAC AGCTTTGCGG CCGCGTAGGG TGAACGCGGG 6720
TAGAATGGCG TCTTTTCGTT TGGGGGCTCC TCTTGAGCCA GCCCATATAG CTCTGAAGTC 6780
GATGCCTGAT AAAAGCGAGT CCGATTGGTC AGCCCAAGAA TCCGAATAGC TTCCAGCATG 6840
CGCAATGTAC CAATTGCATC GGCGTTTGCA GTGTACTCGG GGGTTTCGAA GCTTACCTGA 6900
ACGTGGCTTT GCGCTGCAAG GTTGTAGATC TCATGCGGCT GGGTTTGCTG AACAATGCGC 6960
AGCAAATTGG TCGAATCCGT CATATCCCCA TAGTGGAGAA AAAATCTCGC TTCAGGATCG 7020
TGACGTTCTT GATAAATGTG CTCTATCCGT TGAGTGTTGA ATGATGACGA ACGACGCTTT 7080
ATGCCGTGAA CAATATAGCC TTCGTCCAAC AGCAGTTCAG CAAGATAAGC CCCATCCTGA 7140
CCCGTTACGC CAGATATTAA AGCTACTTTC CGGTCTGTCA CTTTGTCGAA TCTCCAGTGA 7200
TATTCCTGCG GGAGAGGGCG TGCGCTAGGG CAGCTGGTGT GAAGGAAATC GTCGTGCTTC 7260
GGCGACAGAG CGGGCCTATG GAGGCATTGC GGTTTTCAAA GCAGTATTGC CGGAGACGTG 7320
CGGGCCGGGG CAACTGGGCA TGAGGCGCTC GTGTCCCTTG GAGCGCGCCC AAGTTTCTCC 7380
ATGACTGCTC TTCACCACGC CTAGAATTGA GGTGACGATT TTCATTCAAG AGGCGGTATT 7440
TGTTGAAGAT CTTTCGATCA CGACCAGGCG ACTAGGATCG TTCAGATCAA ACTCAATAAC 7500
CCTTGGGACA GAAAGGCGTG CGTAACGTGT AAAGGCGCTT GTCGGAGGAA ACCGAATGAC 7560
GGTGTCGCAA AGCCCAAGAA GATACATCTC GACGAGGGCG GAAATTCCGC CCTCAACCCC 7620
GAGATCTGCG CTGTGTAATG GCCAGACTGG ATCCGCTCGG AAACTTTTCG GGATCGTGAG 7680
AAGATCGGGG AACCTACTCG ACACCTGGTC CAATACCCGC GCGCTATCGG TGCACAAAAT 7740
CACCCGCACA GGCTTCGGGT GGGGCAACGC TTTGGCAGCA TTAATGGCAG TGCACACTTG 7800
ATGCACGGCA AGGTCCGGAT CGGCCCAGTA GGGTGCATGG TCCATAACGT CTTCGCCGTT 7860
ACCATGCCGA ACATGAACCC CGATCGCACT GTACCCATAG AAGTGCTCCT GATAGATAGC 7920
GTCTATGCGA GCCTGAATTT CAGCCCGAGG CTTGACACTG CAGAAAATCT GCCGCTCTGC 7980
CTCCTCGTCG CAGCGCCACA TCAAACACGC ATCACACACT ACCGTGTTGG CCTCGACATC 8040
ATCTTGGGCC TGGAAAAGTT CATCGAGTTC GTCTCGCTCT CGAAAAACCT GAGCATCTGG 8100
GCGGTAAACA CATTCAATCG CAGGTTTATT CCACCAATTT GGGAAGAATG GTCCTGGGAA 8160
GGAAAACTCG TTGACCCGGT TATCGCAAAT AAAAGGGACA CCTGCGATAT CCTTGATTGG 8220
TTCAAAAAAC ACCGGAAAGG CGTTTGTGAA GGGCTGATCA AGGTAGCAGG ACCCGCGCCA 8280
GTCAACGGCT AACGTTCGCG CCGTCCTTTG GGCGTAGCGC CAAGCGGCCG CCAGCGACCA 8340
CAGACAGTCA CCGAAACCAG TACGTCTCCG AGAGAGGACA TATCGATTGT ACAATGCTGC 8400
CCGTCCTTAG TGCCGCCGCT CTGACTTACT GTCTTCTACG GATTTCCAAT CAATTCAGAG 8460
CTTCCTAATG GAGTTCGGCA ACATCAGTAA AATTTATATT TTGAATGGCA ACCATCCACA 8520
TATTGGATGT AAAGGGGACG TGTCTTTCAG GGCTTATCTT CACGTTAGGT GGCGAACCAT 8580
CGACGCTAGC GGTAACAGAT CTGCATATCA GCAAAGCCTA ACTTACACCA TTGAACTGTT 8640
AAGGACCGCC AGTCCCTGAG CCAGCAAATG TTCGGCAGCA TCTGGGTCGT CTGCCTCGAC 8700
GTAGCAACGC AGTTCAGGCG CATTTCCTGA AGGGCGGATG TGCAGGATAC GGCCGCCTTC 8760
AAATGTCAGG CGCAGGCCAT CCACGTCGTC CGTGCCCGCC ACGCGACCGA TCCGGCTGAA 8820
TAAATGGGAA ACATTAGCTT TTGACGCTTT CAAGAATGCC ACTAGGGCGT CGCTCCTATC 8880
GAACGGATAG TTCTCGATCC GGCCGGACAA AGCGACTGGC AAACGATGCA TCGCGACGAT 8940

CCCCGAGAGG GGAGTTTTGG CCTCTACTGC CATGTGGAGC GCTGCAATGA TGGGAAGGAC 9000
GCAGTCCCGG GTCGGCAAGG CGGGAAGCGA CGCGCCTCCG AACGAGAAGT TCGAACCTAG 9060
CATGACGCCA CCGTTGGCCT CGAAGCCCAT CACCCGCTGC TTGCCACGGG CAACCGCCTC 9120
AGTCATGGCT GCAATGACGT AAGGTGAGCC AACGCGGGTA CGCACCACCT CGACGCCACT 9180
CGCCGCCTCG ATCCCGGAGT TGCTGGTTAT TGGCGTAGCA ATAAGCTTGG CCTCCAGCAG 9240
CCTTGCGCAA ATAAGACCGA GCAGATCACC GCGCAATGGA GTTCCCGTTT CATCCGTTAG 9300
CAGGGGCCGA TCGGCATCTG CGTCGGACGA TACAATGGCG TCAAAGGCGA ACTCCTTCGC 9360
CCAGGCAGCG AGCATCTTAC ATGTCGCCGC CGAAATAGCT TCCGTGTCAA CAGGGATGAA 9420
GACCTCAGAT CGGCCTACCG GTACGACATT TGCGCCATGA CCTTCCAAGA TGGTGGTCAA 9480
AATATCGCGG GCAACGCTGC TGTGCTGATA TAAGCCGATC TTTAGCCCCT TAAGGCCCGA 9540
TTTTGGAAGC AGCGTTTCGT AACGTTGGAT ATAGAAGTCA GTTGCTTCGC TGGAGTGGTC 9600
GGCGCCGCGG CCACACTCAA CCCTGGTTGC ATCTGCATCG GCTGAAAGCT GCTCCGCCAA 9660
CGCCGTAATC GCCTGCTCGT CGGCCTTGTT GATCTCACCG TCAGGCAGAT AGAACTTAAT 9720
GCCGTTTCGG TCGGCCGGAA TATGCGAGCC TGTGATCATG AGTGATGCTG CACCGAGTTT 9780
TCGGCCGTAC AATGCAAGCG CTGGTGTCGG CAGCCCTCCG CAGTCGACCG GCACCATTCC 9840
CGCCCGGGCG AGCGCCGCCA TGCAGATAGC GGCGATTTCC GAACTTGAAT CGCGGAAGTC 9900
GCGACCGACC AATACGGTCG CCCCTGGCGC GACCCGTCCT CTGTCGAGGA GCATCCTCGA 9960
AAAGGCTGTA GCATAGAGCG CCGACACACT ACCCACGAGT TCTGTTGCCA ACCCGCGCAG 10020
ACCACTTGTG CCGAATTTGG GACCCATTAA CTAGCCTTTT TTTGCCGGGC AGCGTTCAGC 10080
AGGTAGTCGA CGCCACCGGG GCAAGCCGAA TGAATTCGCA TCCACTAATG AGATTGCTTC 10140
GCTCTCGCCA CACTGTTATG AAGCTTCCAT CAAACGCAAT CAAAAATGAC ACCATGCTTC 10200
CAAAGATTAT CCCAGCTATC ATGGCAGGGG GTAGGGGCAC AAGGCTCTGG CCTCTGTCAC 10260
GCGCAACTGC CGCAAAACAG TTTCTAAAGC TAATCGGCGA GGAAACGCTC TTTCAAGACA 10320
CGCTTAAGCG CGTTTCCGAT GCCAAAGTTT ATGGAGCACC ACTCGTCATC ACAAATGAGG 10380
AATTTCGCTT CCTGGTTGCC GAACAGGCGC GCGAGCTCGG GGTCACGCTT TCCAGCATAG 10440
TACTTGAGCC GGTGCCGCGC AACACGGCAG CCGCAGTAGC CGTCGCCGCC CGGATCGTGG 10500
CTGATCGGTT TGGTGAGGAC GCGCTATTGC TTGTGCTGCC GTCGGACCAC GCGATTACGG 10560
TAGACGATAC TTACAAGAAA TGCGTGCGCT CCGCCTGCAT CGCCGCAGCG GAGGGCAAGC 10620
TCGTGACGTT CGGTATTCAA CCCACTTGGC CAGCAACAGG ATACGGATAC ATCGAACGTG 10680
GCACTTACCT TGGCAAGGAC GTCCATGCGG TTCAATGTTT TGTCGAGAAA CCAAGTCTCG 10740
AAAAGGCGGC GGCTCTTTTG GAGACCGGCA ATTACTATTG GAACTCTGGG ATGTTCCTTT 10800
TCCAGGCGGC CAGCATTATC GCCGAACTTG AGGAACATGC ACCCGACGTG TTGTCGGCGG 10860
TGCACGCCGC GGTCAGGGGC TCAACGGTCG ACGCCGATTT TATACGACTT GCCCCGGAGA 10920
GTTTCAGCCA AGCGCCATCC ATCTCGATCG ACTATGCACT GATGGAGAAA ACCGCAAACG 10980
CCGCCGTGGT CTGCTCGGAT TTTGCTTGGT CGGATCTCGG TAGTTGGGAT GCTGTATGGA 11040
AGAATGAGGA GCAGAATGCT GATGGTAACG TGCTCAAGGG CAATGTTACG GCCTGCAACA 11100
CGAAGAATTC GCTTGTGTTG TCGCATACCG CGCATCTCGC TGTACAGGGG ATGGATGGAG 11160
TTGCAGTCAT CGCTAGCGAG GACGCAGTCT TCGTCGGGCG ACTGGAGGAG GCCCATGAGA 11220
TCGGAAACTT GGTGAAGCGC CTCGCCGCGG ACGAAAATAC GGCACGTCTT ACGGAATTGC 11280
ACCCAACTTT GATACGGCCA TGGGGCGGCT ACACCACTAT GCTTAACGGC GATCGCTTCC 11340
AAGTAAGGCG GTTGTTCGTA CGCCCTGGGA AGATGCTTTC TCTCCACAAG CATTTTCATC 11400
GATCAGAGCA CTGGATCTGC GTGAAGGGCA CCGCAGAAGT GACAATCGAG GATCGAGTAA 11460
CGATCCTGCA CGAGAACCAG TCAATCTACA TCCCCGAGGG GGCGATACAT CGCTTGGGCA 11520
ACCCTGGCAA GATCATGCTG GAGTTGGTCG AGATTCAAAC TGGTGCCTAC CTGGGCGAGG 11580
ACGATATCAT CCGCGTCGCA GACGAATCGA GAAATGAAAT GCCAGATTCG AGGCGTACGG 11640
GCCCATAGGG ATAGCGTGCC CTTTGCGCAG TCCGGCACCT TCGCACGCAT TACGGTATCT 11700
GGAGCGAGGA CGTGGTGGCA CCATCGGCAA GCGTTGCACC GGCGGCCGCG GACGGGTCTG 11760
ACGACATGCG ATGGATACCG CCCTCTGCGT CGACACGATC GTAGATGCGT GTTCTCCTCC 11820
GTGGGCCCGG CACGATCGTT CTCCTGCGCA ACGCATACCT CGCGACGAGG TAGGCCGAAA 11880
AATCCGGCTT TCTTCGCGCG CAGCATTTCG AGACGACCTT TCTCGACGGG CAGATCGCCC 11940
GGCGTCTGCG ACCCGTCCGG CTTCTTTTTC TTTGAGCGGT TTCTCGCAGC AGCACCGCTT 12000
GCACGCGGCT CCATTCCGGC TTTTTTTACC GACAGATCAG CTATGCGCTT GAAGCGCTGC 12060
TTGCCGTCCC GTCTGGCGGA AGGCCCGCGA GGAACGGCGA AAAGGCGGCG CCGAAGCCCC 12120
TGCATTGTTA GTGGCAAGGC TGTTGCGGTG GGCGTTGACG GTTTCGCTCC GGCTCGATCC 12180
CTACGGTCGA AAAGACGAAA TGGCAGGAGC GGTCGGCCGT TTTGCCACAA AGCCTGTTCT 12240
TCAGGAGGCG CATGCAAGAG GGCGGGCGAC GGCATGAAGC CGTGAATGCA CTTGACTTCA 12300
GATTAATTAA GCGTTTTCTA ACGATTTGCA TAATTGATCG TTCGGATGAC AACCATCCGC 12360
ACTGTGGATT CGCCAGAACA TGCGTTTTAA GGGCCTTGAT CTCAATCTCC TCGTTGCGCT 12420
CGACGCACTG ATGACCGAAC GCAAACTCAC GGCCGCTGCA CGCAGCATCA ACCTGAGCCA 12480
GCCGGCGATG AGCGCAGCCA TCACCCGGCT TCGGACCTAT TTCCGCGACG AGCTATTTAC 12540
CATGAATGGT CGCGAACTTG TACCAACTCC GCGAGCAGAA GCGCTCGCAC CCGCAGTCCG 12600
CGAAGCCCTG CTGCACATCC ATCTCTCCAT CATTTCATGG GATCCGTTCA ACCCAGCGCA 12660
GTCAGATCGC AGTTTCAGGA TCATTCTTTC CGACTTCATG ACGCTAATGT TTCTTGAAAG 12720
GGTTGTGGTG AGAGTGGCGC GGGAAGCGCC CGCCGTCAGT TTCGAGTTGC TGCCGTTTTC 12780
CGATGAGCCA GATGAGCTTC TCCGGCGTGG TGATGTCGAT TTCCTGATCC TGCCAGAAAT 12840
GTTCATGTCG CACACGCATC CCAGAGCGAA GCTGTTCGAT GAGAGATTCG TGTGCGTGAG 12900
CTGCCCAACG AACCAGAAGC TACCGCCGCA GCTCTCCATC GACAACTATG TATCAATGGG 12960
GCATGTTGCG GCCCAATTCG GGAAGCAGCG GCCTTCCGTG GAGGAATGGC TATTGCGCGA 13020
GCACGGATTG CGAAGACGGG TCGAAGTCGC CGTGCCGGGT TTTACCATGA TCCCGTCTTT 13080

TTTGTCGGGC ACTGACCGCA TAGCGACCCT CCCGTTACGA CTGGCGATGC ACTTCGCAAA 13140
AGCCATTCCC CTGCGGATCA CCGAACTTCC GCAACCCATT TTTCCCGCGT TCACCGAGGC 13200
TGTCCAGTGG CCCGCGCCTC ACAGCAGTGA TCCGGCCAGT CTCTGGATGC GCGAGATATT 13260
TCTACAGGAG GCGTCTCGCG TTGAATTTCA ATCCGAAACT TCGGCGCATG CTCTATCTAA 13320
AGCCGACGGC GCAATTCGCC ACCCTTGAGC CCGCAACTGC ACGGTCGGCT CAGGGTCGAG 13380
CAGTTTGGCT GCAGGTTGCG GCCTACCAGA GGGGCTCGCA CGGCCCGAGC GTACCGCATG 13440
CCACACTCCG ACCATTTTCG GCCTATGCGC AGCATTAGCT CTTCAGCTTC AGCAGCCGGG 13500
AGACCATCTC ATTCAGCGTG ATATCCTTCG ATACCTCGAT CAAACCGCGC ATGGGCATTA 13560
AGGCGAGGTT CCTACTGTCC GCCATGCTAC AAGTCACTCG CAAGTTGCCC TTCCGGACGC 13620
TCCCAATCCA GCCAATTTGC GGTCGGAACG CCAATCCAAC CGCCTCGCAG CAGCCTCCGC 13680
CGACAGCCCG TTCCGTCTTC GATACTCGGC TCTGAAGATC GTGACTCAGT GCTCTTCCAT 13740
TGCCTCCTGA TTCTAAGAAA AGAACCGAGA CTGCGGACAC GGAGATGCCT CAATTGAGTT 13800
GCTCACTTTG GAGTTGAGCT GTATCAAACG GGTGCCCTCG ACAAAGGCCA TATTCGATAT 13860
AGCGGATCCC AGTAAATCAT TGGTGAAACG ATCCTTCACC AGGAGTAAGT GTGTTGAGTG 13920
TTGACCCTGA GAAGAAAAGA GCCAAAGCGA ACGCTGTGGA TATTTACGTC GGCCGTCGAA 13980
TTCGCCAGAG GCGACGATGG CAGAATATGT CCCAAGCGGC CCTCGGAGAA GCTATCGGGG 14040
TGACGTTTCA GCAGGTGCAA AAATATGAAA AGGGCTCGAA CCGGGTAGGT GCGGGCCGAC 14100
TTCAGCAGAT ATCTGATGCA CTGGAAGTGC ACCCTTCCTA TTTTTTTGAG GACATGCCTG 14160
ACGACACTCA GTCGATAGGG CAGGGCGCCC CTAATCAGGC CTATATCCCG CCCGAAGTGA 14220
TTGAGTTCGC TGCAAGCGAC GAAGGGGTCG AGCTCATCCG GGCGTTCTCC CGTGTCGGCA 14280
ATCTCAACGT GCGCTGTCGA ATCGTGAAGC TGCTCAAGTC TCTCGGCGAA CACGACTGGT 14340
AAGCATGAAT TCGCCCATTT CTGAGTCGAA CGCGGCTACG CTCTCGGAAT GAAATCAACC 14400
GTAAGTGACA TCGCCATGGG ACGCTTGCAT TGACTCTGTA GCTCATTGAG TTCAAATCAT 14460
ACAAGTGAGC TAGGGGAGGA AATCACAGTG GCTGACGAGA GTAACACAGG ACCAGTTGCC 14520
GCGGCTGAGG CGGTTGCCGA AACGCAAGCA CCAGCTGGCA AAAGGAAGAG TTCGTCGCGG 14580
CGTCAAAGAA CGGCTGCCGG ACAAGTTGCG GAATCAAAGA CGACTGCTAA GCCTAAGAGA 14640
TATAGCGAGA CAGAAAGAGC TGACAAGCTC AATCTGATCG AGGCCGAAGT GGCTCAAGGC 14700
AATAGCACCC TAAAGGACGC GATCAAGAGC GCCGGCATAT CGGAGCAAAC CTACTACGTG 14760
TGGAAGAAAT CTGCTAAGCC TGCCGATCGA AAACCAGAGG AGTCTGTGGT GGCCGGCGAT 14820
GACCTTGCCG ATCTCATTCA ACTCGATAAG GAAAACTTGA GGCTTCGCAA CCTTTTGTCG 14880
GACAAACTGC GTGCGGAAAA CGCCGAACTG CGCAAGAGAC TTGGGCTGGA TTAGTTCAAG 14940
AGTAGCCTCT CAAGGCTGTG GTAAGGCGCC CTGCCGGCTT CCGAAAGGGA CGGCGTGCCT 15000
AGCCTTACGG CCACTTGCAT CGAGTCCGAA ATGGCGGTAT TTGGCCGAAT GGTAAAGGAA 15060
TGTCGTGGCG CGTTTTGGCG CTGATGAGTC GTGGATTGCT TGCGAGATAC ATATGAAAAC 15120
ACCACCACGG AGGTTTATTG TCGAATTCAA GCAAGCACGG CGGCGATCAA AGGAACGAAC 15180
GAATTCAGTC TGGGGAAACA CGGACCTTAA GGCTTTGACC CGCGAAGTGG AAGAGATGGC 15240
TCCGCATTTG TTCAACTTAA CGGAAGAGCC AGTATCCCCT AACGTAGCCT ACGGTCCGCC 15300
GGCCAGCCCG AATGCAGAGT TTCTCATTGC AGACAAAGAG AATATCGGGC TTGGGCCAGA 15360
AGCCGTGACA CCGGCCGAAG GTTCGGAAAA CGCGTTCTCG GGGCTGTTGC AGGAGACGCC 15420
ACCGGTCTCC CACACCCGAA CAAACTCCAA ACAGCGCATT TCGCGAAAGC TACCCGAGCG 15480
GTTGTCATTG GACGTGCGTG AGAATGTAGA ACGCCCGATT TCTCGCGGTG AATTGGCCAC 15540
TCTCGAAGCA GAAAACAAGC GCCTCAAAAG GCTGCTCGCT GAACGGATCC TTACCCAAAA 15600
CTTGCAACTC AAGAAGATGC TTGATCGGTT TAACCTCAAT TGACGATGTC GCATCCGCGC 15660
GAAAGTCTTA GGAATCTCTT GGGGGCCGGC GTTTTAGCAA TCGCTGATAG CTCTTCAATC 15720
CCACCTCAAT GACACAGTTC AGGACCTTCC TCCATGTATC TAGCCATAGC ACGCCAACCG 15780
AATATATTCA AACAAACCTG AGCCTACGGC AGAATTAATG GTTTTTTAAT AGACTTGGTA 15840
GCTGCGGATT AAAATTATGT TTTGACATAT CCTCGATCGG TGTTAATCCA CTAAATGAGA 15900
AGTGTCGTCG CAGATAAATA CTTTGACGAA ATTCCAGCTT GGGTTGTGAT TCTTCCCTGT 15960
TGCTTTGAGC CGTCAGCCAG CTCAGCGCCT TGGTGGCGGC TTCCCATCAC GACTTGAGCC 16020
TTGATGTTGC GGCGGAGCCT GGCTCCTGTG CAATGACCGT CCAGTTTCAA GAGGCAAGTG 16080
AGTTCAGCGG CATTTTCTCG CACGTTTCCT CCTGAAACGT CGTTTTGGGA TATGTTGCTA 16140
AGATCCGATA GGAGAAGAAG ATGACAGAAA CCACGCTCGG TGCGAGCAAC GAACTTTTGG 16200
CAGAACTGAC GGCAGAAATC GTAGCCGCCT ATGTCAGCAC CCACGTGGTT CCGGGTGCCG 16260
AGCTTCCGAC GCTTATCGCT GACGTTCATT CAGCGCTCAA CAATGCCACT GCTCCCGCGC 16320
CGGTGATTGC TCCCATTGAG AAGCCGAAGC CAGCGGTCTC GATCCGCAAG TCTGTGCAGG 16380
ACGATCAGAT CACCTGCCTC GAATGCGGCG GCGCCTTCAA GTCGCTGAAG CGCCACTTGA 16440
TGACCCACCA CAATCTGTCG CCGGAAGATT ATCGCGAAAA GTGGGACCTA CCCGCGGACT 16500
ACCCGATGGT CGCGCCCGCT TATGCGGAAG CCCGCTCGCG TCTGGCCAAG GAGATAGGCC 16560
TCGGCGAGCG CCGCAAGCGT CGTGGTAAGT AGAACGTTAT CGCCGGCGGC GATCGCTGCC 16620
AGCTACGCGC CACGCCTTTA ATTTGCGCGA AATGCTATGC AGAATGCGGT GTTTCGGCCG 16680
CACCTTCCAG ATGCACGGCG TGATTGACGA ATGTTGACCC CGAACGAAAG CTTGGACATA 16740
TGTAGATTTC ACAGCTTACG CTTAAATCCA GCGATGACGA GACGGTGCGC CGCTTCAAGG 16800
TTTTCATTAA TCTTGTCGTT GGCGAAGCCG CCAAGTTCAA CCGTCTCGAT CCGGAGGTCC 16860
ATCTTCGAGA TATCCTCACG CGCATCGCCG GCCATCCGAT CAACAGGCTT GCCGAATGGT 16920
TACCGCGGAG CATGCGGCAT CAGGGTCGAC CATGGCCGGG CCTGCCAACA CGACACTCAT 16980
GCGCAGCGCG CCTGCGGCCT TCCTCGGTGG CGGCGTCATC GGCCCGCCTC CGAAGAGCCG 17040
GTCAGCTTGA CGACATCGAC GAACTTGCGT CGCGCGTGAG CAAGGCAGAA CGCCAGCCGC 17100
ATGGGGGCGA CATTGCTCTC TCCCATCACG CGCGCCGACG CGGGCGCTTG CACAATGACG 17160
CTTTCACCGG CCCGGCATGC ATAGCTTGGC CGGATCGTCT GCTTTACCCG GACAACCGCC 17220

GGCACGATGT CGAGCGCCTC ACTGACGTCC GTGCCGACGC AATGAAGCTC GAACGAGCAG 17280
CACGGGCAAA TCTTGCTCTC CGGCTCGATG AGCTCTTCAT ACCGTGGGAG GTGCCTGGGT 17340
AAACGGCCGA TGTTGCGCGA CGGTGACCGT CGCTCCTGTT TTGGCCCTTC AGCCACGGGT 17400
GCAACATCGT CATTGGCCGC CGCGGGAATG TCGGTGAGAT CGCCAAGGTC AAGCGTCGCC 17460
TGCGTCGGGT CGATCGCCGT CATCTTCTCC GATTTCGGCC CAAAGAGCTG CTGCTCCAGG 17520
AAGGCAACGC GCGCCTTGAG GTCGGCGTTC TCTGCATCAA GCGAGAGAAT GATCCGGCTC 17580
AATTGCGCGG CATCCTGAGG TAAAGAGTCG GGTCAAAGCG GCATGGCAGA CCCTAGCACA 17640
TGATCCTGAA TCTTCAAGCA AATCCAATAG GATCAGCCCG CTTTCGTCGG CCGCTTTACG 17700
ACGTTTCGCT TGCCCGAGTC CAGTCGATAC CGGTCAGCAA AAAATTTGTT CGGCCTTGAA 17760
ATGTACGTTC GTCTAACGTA CAGTTGGCTT AAACGACATG AAGGAGCCGG TCATGCGAGC 17820
AATCAAGGAC GTCACGATTG ACATCGATGA GCCGAACACC GTTCGAATGA ACTTCCGTAC 17880
GAAGGAGCGC GTGAAGAGGA CGATCCAGCG TGCCGCGGCA CTTTCCGGCC TCGACGATTC 17940
TGCGTTCACG ATCAACGCTG CCTACCAGTC TGCCATTGCC ACGATCGCGG CGCATGAGGC 18000
GACCTTGTTG CAGACAACGG ACTACCAGGC GTTTTTCGAC GCTTTGGATA ATCCGCCCAA 18060
ACCGACCGAT CGGCTGAGAG ACGCGTTCAA GCGTTATAGC GAGACCGTAG TCTCTAAGTA 18120
ATGGCAAATA CCGGCGCCGC AAAACGCATA ATCGAGCCGC TCGATCCCAA CCGGCATGAT 18180
CGGGCGGCTT TTTTTTGTGG AATCATCCAG GTCGACAACT TCTTCAAGAA AACGGCGAAC 18240
AAGCTATCGA AAGCAGACAA CCTTCGTGTC TATGTGATGA CCGAAGACGA TGGCACAACG 18300
GTTATAGGGT TCTACGCCAT CAACAGCCAC TCGATCAGCT ATGCTGATCT GCCGGAACGC 18360
TTTTCCCGCA CCCGGCCGGG ACACGGCTCG ATACCGGCCG CCTATATCTC CATGATCGGT 18420
CGCGATCAGC GATATAGGGG TGGCGGTTAC GGCGGCGATC TGCTTACCGA TTGCCTGCAA 18480
AGGATCGCCG GAATTGCTGA CCAGATTGGG ATCGCCGTCG TTCTTCTCGA TGTCCTTGTT 18540
TGCGGTGACG AAGAAAAGAC GAGCCGGCGT GTCGCGCTTT ACAGCGAATA TGGCTTTCAA 18600
CCTTTGCCGT CAATGCCGCT TCGGATGTTC CTGCCGATCG CGACGATCAG GAGCCTAATG 18660
GGCTAACGCG CATTCGGTGC GCGATGCACG CATTTAATTA AGCGCAAAAA GCAACAGAAA 18720
CAAAACCGCT CTGCTGTTCC ATAAGATAGA TTATGCGATC ATTTTTTGTA GCGGCATTTT 18780
AGAGATGCGA CATATGAGCC AGTTAGAGAT GCGACGGTCG TCGCCTCTTG GGATGCTGGT 18840
CAAACGTCAA CGTTTGGAAG GAAGACTGGC GACGGGAAGC GTGGTTGAGA CCATGAGCGT 18900
TCGGAGTCGT CATGTCTTGT TTGATCACCA TGTCGCAGAA GGAATTGCAT CGTCTTGAAC 18960
TGATCCAACG GATTCGCGGC CGCAGCCTGA CCGTCGTCGA GGCGGCCGCG TTGCTTCGTC 19020
TCAGCCGCAG TCAGGTGCAC CGGTTGTTGC AGGCCTATGA CTTGGCCGGC GCCGACGGTC 19080
TGGTCTCGAA GAAGCGCGGC CGTCCGAGCA ACCGGCGTCA CAGCGAGGAT TTCCGCAACC 19140
TGGTGCTCGA CCTGGTGCGT GAGCATTACC TGGATTTTGG ACCAACCCTG GCGGCCGAGA 19200
AGCTGATCGA ACGCCACCGG ATTGCCGTCA GCAAGGAGAC GCTGCGCCAA TGGATGATGG 19260
AAGCCGGCAT CTGGGTGTCG CGACGCGAGC GCAAGAAGCG GGTCTTCCAG CCGCGCGGCC 19320
GGCGCGATTG TTTCGGCGAA CTCGTTCAGA TCGATGGCTC GCTTCATTGG TGGTTCGAGA 19380
ACCGCGGTCC CAAATGCGCC CTGCTCGTCT ATATCGACGA TGCCACCGGC AAGCTCTTGC 19440
ATCTGCGGTT CGCCGGATCG GAGAACACCT TCGACTATCT GCACGCGACG AAGGCCTACT 19500
TGCAGCAATG GGGAAAGCCG ATCGCCTTCT ACAGCGACAA GCATGGCATC TTCCGCACCA 19560
CCCATGCTTC CAAGAAGGAC AGAACCAGTG GCCTGACGCA GTTCGGCCGG GCTCTTTATG 19620
AGCTCAACAT CGACATCATC TGCGCCAATA CCCCGCAGGC CAAGGAGCGG ACATCACGAT 19680
GCACCTTGTT GGAGTGGTTG AGCGACAATT AATCTGGGTT GACGGCCACC ATATCGACTG 19740
CCGGCGCCTT TTTCGAGTCT GACGTTGCCC GGCGGTTCGT ACAAGTATCG ATGGTTGCTG 19800
GCGTATCGAT GACGCTTGAG AGTGCCTTTG CGCCCGAGTT GGTGAACGGT CGATTCGCAT 19860
ACGCCGAGCT GCTGGGCTAC TTCCTCGCCG GTGAGCATGC CGCCCTCGCG CAGCCGTTCG 19920
TACCGGCTCT TCAAACCATA GGTGCGGCGC ACGAGCATGA CCTTCTTCGG CGTGAAGGGT 19980
TCGCCGCGCC AGTTGCGATG CCCGAGTTCG TTGAGACGAG CGGCGATCTG CCGGTCGTTG 20040
GTGGCCTCCA GCAGCTCGTT GATCAACGCC ACGACCTGTG CCGGCGTCTT GCGTATCACC 20100
GACATAGGCC GAGGTCTGGC CACCGACAGG CTCTGCGTGC GCCCGCCGCG CCAACGGATG 20160
TTGATGTTGA CCTCATCGTC GACGAGCAGC GTCACGTCTT CGATGAGAAG GCCGAGCATG 20220
CGCTTGCGTT CGACGGCACC GGTGCGCTCG TCGTTCCAGA TGCGCGGGAA ATCGGCGGTC 20280
AGCGCCCTGA TGCGCTGCTG CGCTGGCTCG TCCAGCAGCG AGTGGTCGGC CTCGTTCTGG 20340
CGCTCGTGCT CGCGCTGGAG TGCGTCGAGG TCGCGCAGCC GTGCGTTCCA GTCGGCTTCC 20400
AGCGCGTCGG CGACCAGGCG GTTGTCCGGA TCGACCTTCA GGTAGCGCCG GCGGGCCAGT 20460
TCAGCCTCAT AGCGGGCGCG TTGCAGCTGC GTGCCGCGCA GCGCCGCGGC CTGCTCCACG 20520
CGCTGGGTGA TCTCCTGCTG CACGGCAAGC GCGACATCGA TCGCTGCCGG CGCCATTACC 20580
TCCAGCAGTA GCGCGCTCAC CGCCTCGTCG ACCGGAGCAC CGCGCACCCA TTGGCAGTGT 20640
TTGCCGGCGT GTCGCACGAC GGCTTCGTTG CAGACGTAAT ATGGGCGCAG CCGCCCCTCG 20700
AACGGCTCGT AATGGACCCG CATGCGTGCA CCGCAGCGGC CGCACAGCAG CCGTCCCTGC 20760
AACAGGCCGC TGCCCTGGCG GGGCATGCGT CCACGCAGGC CGGGTGAAAA ACCGGTGGCG 20820
TTCTGTTCCA GGGCTGCCTG ATTGCGTTCG TATTCGGCCC AGGAAATGTA GCCGTCGTGG 20880
GCGTCGGGAA TCAGTACCTG CCAGTCGGAT CTCGCCACGC GCAGTTGCAC GGGCTTCAGC 20940
TTGGCATTGT AGGCGGTGCG CGTGCGTCCA TAGGCGAAGG CGCCGGCATA GCGCGGGTTG 21000
TGCAAGATCT GGAGCACGCG GGAATGGTCG ATCTCGTTCC ACAGGACGTC GCCCTTGCCG 21060
ATGCCGCGAC GGATACGCCG CGGAAAGAGG ATCTTCTCGC CCCGCAGGCG GCGCACGACG 21120
GCGCAGGCCG AGCCGGTCTG GCGGAAAGTA TCGAACAGGA GCCGCACCGT GTCCTGGATC 21180
TGTCGGTCGG GATCGAGCAC GACCCGCGCG TCAGGGGTGT AGACCAACCC GATCGGCAGC 21240
GGCATCTCGA GTTCGCCGCG GCGCGCCTTG TTGAGAATGC CGCCTTGCAG CCGCGACTTC 21300
AGGATGTGCA GTTCGGCCTC GCTCATCGTG CCCTTCAGGC CGAGCAGCAT GCGGTCGTTG 21360

AAATAGGCCG GATCATAGAC GCCGTCCTCG TCCATGATGA GCGTGCGCGA CAAGGCGGCC 21420
AGCTCAAGGA GACGGTGCCA GTCGGCATTG TTGCGTGCCA GGCGCGACAC TTCCAGCCCC 21480
AGCACGATCC CTGCATGTCC CATCGCCACT TCGCTCACCA GGTGCTGGAA GCCGTCGCGG 21540
TCCTGCGACT GCGCGCCGGA GAGGCCGAGG TCGCTATCGA TGACGTGGAC ACGTTCGATC 21600
GGCCAGCCCA GCGCCACGGC GCGATCGCGC AGGGCGTATT GCCGCTTGGT GCTCTCGGTG 21660
TTCTCGAACA CCTGACGAAG CGAGGACTGA CGCACGTAGA GGAATGCATC ACGCCGCAGA 21720
TGGTCGGCGT CAACCTTTAT GGCGATGTCA GTAGACATGG TTCCCTCGGG TCTCTGCTGC 21780
CAGCACCATG TTGGCGAGCA TGTGCACGAG CTGGCGATCG TGCACTGCGA CCGAAGCCGT 21840
AGGCCGTGAT TCCAGCCGCC GATGCGTCGG CGGGGGAGGC TGCGCGATCA GCACCGCCAG 21900
CCCTTGCCAA AGGCCGTGGA AAACGATGGC GCCCATGTCC TTGGCGCAGG CCTGACCGCA 21960
AAGGACGGCG GTGCGCAGCG TCTCGTAGAA ATCCTTGCAG CTGCGCGGTA GCACCGGGGT 22020
GCGTGCATGG GGCTCACCGT TTTTTTTTAC GGGCGATCGC GCGTTCGATG CTGCGGGGAT 22080
GGACACTGAT GCCGAGCGTC GAATGCAGCA GTTCTGCCAG GGAGCGCGCC TGCAGCGGGG 22140
CGCCCGCATG CTGGTTCTGC TCGATGAGCT GCATGACCTC GTCGGTGAGT TTGTGGGCCG 22200
ACTTTGGCCC TCGGGTGCGC GGCAGCAGGC CGGCAATGCC GTCGCGTTCG AACGCGGCCT 22260
CGGCCTGATA GTAGGTTGGC CGCGACAGAC CGAAAAGCGC GGCGGCATCG GCCTTGTTGA 22320
CGCCATCCTC CTGGACGTGA CGCAGCATCT CGTACTTCAC CTGCACGAGA TCGAGAGGAT 22380
CGAAGAAGGT GGACTCACGA AACCAGGGTG CACGGACGGC TTCGGGCCGC GCATTGAGTG 22440
CTCCGAGCTC GCGCAGTCGC TCACGTTTGG TCTCGTTGGG CATGGCATAA CCTCTTCCTA 22500
GTGGTGTAAA TCAAATTACG CCTCGATAGA GAACGTGTCA ACCTAACGAC GCCGGGCGAT 22560
ATGGCTGTTT ATGCTGCATA TAACTACAAT GTAGAGGCAA TTATCGCTGT GAGCGCCCGT 22620
ATGATTCGGC ATAATTAGAT TTACATAATC GGCATAAATT GCCTGACACG CATTCAGCCG 22680
CCATGGCTGC GCTCGATTTG CTCGATCAAT GCGGCTAGGG TTGCCAGATC ATCTGGACGC 22740
ACGAACGACC TACCGGCCGC GATCTGAATG AAAACATCGG GTGCAGCGAC GTCCGTCCAT 22800
GACGCAAGCA GCGAGCGAAG CCGCCCATCC GCGTCGTAGA ACATGACGCG ATCCTCGCCC 22860
CAGTTCTGCT TGCGTGTCGA CAACACGAAG CGCTGGCCGC GCCACGGATG GAACGGGTGC 22920
GTGACCTCGA ACTCTATACG CACTTGAGTG TCAGTACGAA CTGCAGTCCT GGACTTGAAA 22980
GAAGGGGCGC GTCGAGCGCG CCAACCAGAC GCTGCAGGAT CGGCTTGTCA AGGAACTGCG 23040
GCTGCGCGGC ATCGACACGA TCGCGGCGGC CAATGCCTAT GCGCCGGAAT TCATGGCCGA 23100
CTTCAATCGT CGGTTTGGCA AGGCGCCGCG CAATCCGAAG GACATGCATC GGCCGTTTGC 23160
CGCGCATGAG AACCTCGATG GCGCCATGTG CCGCAAGGAG ATCCGCAAGC TGTCGCAGTC 23220
GCTGACGCTG CGCTATGACA AGGTGATGTT CATCCTCGAT CCGACCGACC TCGCCACGGC 23280
GCTCGCCGGC AAGAAGGTCA TTGTCTGCGA CTATCCCGAC GGTCGCCTCG AGGTCACCCA 23340
TGAGGGGACG TCCCTGCCCT ACAGGACCTT CGACACGCTG CGCTCGGTGC ACCGCTGCGA 23400
GGTGGTTGAG AACAAGCGCC TTGATGACAT GCTGGCGCTG GTCGCCGAGA TGCAGGCCGG 23460
ACGAGAGCAA CAGCGCAGCA AGGGCGGCCC GCGCCGCACC GGCCAGACGG ACCATATGTT 23520
CGGCATTCGC GACGGCAGCC AGAGCAATGG CTATCAAAAG CGCGGCACCA AGCCTGGCCG 23580
GAAGACGGAT TTTACCAAGG ATCCGGTGGT CATCGCCAAG CGGCAGCAAG CCCTTGCGCA 23640
GCTGAAAGCG GCGGAGTGAT CGGGGTGTCA AAGCTGAAGT TTATCTTGCG GCTGGAGCCA 23700
TCCGCCGCCG ACCGGCCTGC GCAACCCTGA CCAGCTCCAC CCGGCCGGCG GCTCGCGTCT 23760
GCGTACTTTG TAAATATATC AACTTGCAAA CGCAGTACTA AGGAATAGGA TGATGTCGCG 23820
TTTCTAAGTT GATTACTTTG TCTCATCTTT AAATAGCTGT GACATTTTTT GTAGCCGCAA 23880
AGTTAAAGTG TCCTGTTCCT GCAAAGTAAG AATGTCACTC TCCCCGCGTT TTGATGGCGT 23940
GGGAGATTGC GGATGGGATT GATTGCGATG AGCGAGCGCG ACCTGCAGCG GATTGAGGTT 24000
TTGTCGAAGG TGATCGACGG CCGCATGACG ATGGTGTCGG CGGCGCATGT CCTGGGCTTA 24060
AGCACGCGCC AGGTGCGTCG GCTGCTGGAT CGGATCAGCG CTGGCGGTGC GGCGTCGATC 24120
CGCCACAAGG CGATCGGTCG CCCATCGAAC AATCGGATTT GCGACGGCGT GCGCGATTAT 24180
GCCATGGCGA TCGTGCGCGA GCGCTATGCG GATTTCGGTC CGACGCTGGC GGCTGAGAAG 24240
CTAGCGGAGC TTGATGGCCT GACGGTGTCG CGCGAGACCT TGCGCCAATG GATGGCGGAC 24300
GCCGGTCTGT GGCTGTCGCG CAAGCAGCGG CGGACGTTTC ATCAGCCGCG GTTGCGGCGC 24360
GAGGCCTATG GCGAACTGGT GCAGATCGAC GGTTCCGAGC ATCGCTGGTT CGAGGATCGC 24420
GGTGACCCCT GTTCATTGCT GGTGTTCATC GATGACGCGA CCGGCAAGCT GATGCAGTTG 24480
CGCTTCGTAC GATCGGAAAG CGCGTTTACG TATTTCGAGG CGCTGGAGCT CTATCTGAAG 24540
GCGCATGGTG CTCCGGTCGC CTTCTATTCC GACAAGCATT CGGTGTTCCG GGTGGCGAAG 24600
AAGGACGCCA AGGGCGGCCA GGGCATGACC CAGTTCGGGC GTGCACTTTG CGAGTTAAAC 24660
ATCGAGATTC TTTGCGCAAA CTCGAGCCAA GCCAAGGGCC GAGTCGAGCG GATGAACCGG 24720
ACGCTACAGG ACCGGTTGAT CAAGGATCTG CGCCTGGAGG GCATCTGCGG CATGGACGAC 24780
GGCAACGCTT TCCTGCCTCG GTTCATGGAG CGCTATAACC GGCAATTCGC CATTACCCCT 24840
GCCCGGTCTG ATGATCTGCA TCGGCCGCTG AACCTTGCCC CGGATCGGCT GCGCGACGTC 24900
CTGTGCAAAC GTGAGCAGCG TTATGTCGGT GCGCAGCTGA CGTTTTCGTT CGAGCGCCAG 24960
CGGATCATGC TCGAGGAGAA CGAAGTGACG CGCGGACTGG TCGGTCGCTA TGTCGAGACC 25020
CATGCCTATG CCGACGGCCG GCTCGATGTG CGCTGGAAGG GGCATTCCCT GCCCTACCGG 25080
GTGTTCGACA AGGACCAGCG GGTGACGCAT GCGGCGATCA TCGAGAACAA GCGGCTTTCC 25140
GATGTGCTGG CCTACATCAA GGAGCGCCAG GACGAGCGGC CGGCGCCGAA GGTCAGGACC 25200
AACAGCGAGA AGAACGGCTA CACGCCGCGC GGTCGCAAGC CGGGCAAGCG GACGGATTTC 25260
ATGAACGATC CCGTGGTCAT CGCCCGGCGG CGGCAGGCGC TCTCCAATCT TGATGCCGCG 25320
GAATGATCCA TTGATCTGGC TGTCAAGGCT AAAGCCGATA GGTGCCCCTC ACCCGCCCCG 25380
ATCTCACCTT GCAACCCGGA CCAGCCGGTC AGATCGGGGC TGGTCGTCGC GCCGGGCCGC 25440
AGCTCAAAGT GATATTTCTA CTTTGCGCCA CAGCGGACAG TTCAACCTGG CCGCCACATT 25500

TTTTGAGTGA TTTGAGTGTA GCACAGAAGG CGAACATCCC CCACTACGCC TGAGCCGGAG 25560
CCTTTTCGTG TGCGGCTCCC CCCGATCCAC ACCCATCATT CACCGTTTCC GGTCGCGGCT 25620
CTCAAGTGCC GGTCGTAAAG GCATCCACGG CCGGCTTTCG TAATCGCCCA CTCGAAATCA 25680
GTCAGGTGGA AGCGACGGCG GGTCACGCAA AGCCGGCAAG CCAAGGCCTA ATTGAATCAT 25740
TATCCGCCCG CCGACAAAAC TCCCGTAGTC TGCGACCTAA TCTGAAATGG TCGTCTCCGA 25800
GCTCGTCGGG GGGTTCGCAA AGCTTCAACG ATACAATACA ATACTACCAT CGATTGTGAG 25860
GCGGTTATCT TGGCGAGAGA GTTGTTATTC TTGGCGTGCG CAATTGTGAT TGCCGACAGC 25920
TGGCCGGCGA AGGCGATCGA CATAGAAAGC CTGTTCGGAC ACACCATCCG CATTGAGGGC 25980
GAGTACCCTG AACGCACCTT GAAAGTCGAT GATCGCGAAT TGCACCGCAA CGCCCTTCTC 26040
CTCTTCGACG GCCTGTTCAT TGTGGACGGC GTTCCAGCAC TGATCGGCAG CAGCTCGAAT 26100
GGGGGCAACG CCTGCGACGG CACCCCTTTC GTCGTTTCGT TTCCACCTGG TGCCAGGCCC 26160
CGTTTCGATG GACCGATCGA AGCCTGTGCT TACATCGGGC ACGAGGTCTC CGACGAGAGA 26220
ATCCTATTCT CCACCAATAA CATTCCGGGA CAGGGCCGAG AGCAATGGGC TTGGACGCCT 26280
GCCGACGGAA TGAAGGAATT AGGCGTCGCA GCATTTGTCC CCGACGACAA ATCGGGTTGG 26340
CAGGCGCTCA GGGAACGCTC GTTTGAGCAT CCTTCCGACG CCCTTAAGAA CGCGGATATC 26400
GCGGCAACAA TCAAGTCGCT GCTCGGAGCT GATTTCGAAG CCTTTCAGGC AATCATCACC 26460
GGAACGGGCA GCGGCGAATT TAAGAGCGAC GACTATATTG GTAGAACGTG CACGCCCCAC 26520
ATGTGCAGAG AACAGGAAGC GTTGCTCTTC CTGTCTGCCA AGGATCGTCG CGCCTATGCC 26580
GCTTGGAAGC CGCACCAAAA GAAAATCATT GTTCACCCAC CTGTGAAGCA GTGGCCCGAG 26640
AAAGCAAAAC AGGAATTGAG AGCATGGGCA GAAACGTGGA AGTAACAGCG CTAGCGGGGC 26700
TGATCTCCAT ACAATAGTGC CCCAGAAACC GGTGACTTAC TTAAATAGTC CCTAAAGCCC 26760
ACCAGATGCG CCAGGTTGTT TCTGGGGCGA ATAGTCAATA ATGCAGTAGT CATCGCCGAT 26820
TTGCAAAAAA ATGACGACAT TCTGATTGGC GCTATCCCAC CACTTATCAA TTGATGTCAC 26880
TGGCGTTCTT GAACTCTTTG GCGGGCCATA GGTGTTTGAA AGTTTGCCCG TCAGCGAGGG 26940
GCAATCTTGT CCGGACAACA ATTTCAATCT CACGAGCGCA AGACCCGAGT TTCGGCCAAA 27000
AACGAATGCG GCCTTGAATT TGAATTGGCC GGCAGAGTAC TTCCCGTCAA GCAAGTGCTG 27060
CAGCCGCCTG TCCCCTTTTA ACCGTTCCGG TTTTTTTGGC TTGGTGATTG AGGCGTCATC 27120
AGGCGCATTT TGACGACTTC CTTTGGCGTC ATGCCCCATC TGGTATACTG CCAATCCGCC 27180
CATGCATGTG TGCCAGCCGT AGCCATCAAC ATGAGCATAC CTAAACCGCC TGCATGGACC 27240
ACCTTCATTT CTTGCCCCCC AGAGCGCAAA CAGACACTTT TCATTGGCAA GGAGTAAGCT 27300
TGGCCGACTC CGCATTCTTC GGACCATAGC GAGCAGGACC ACGCCTACTG AGCTTAAGTA 27360
TGCAGGGAGA GTCGTTCATA GGCGATTTTC TCATTCGCAG GCCACTCCGT CGCCATCCCT 27420
GTCGAGCTTG CGACGGTAGC CGGGCTCGCC AGCGTAAATA GGCGCTGCAC CAGCAGCGCG 27480
TGCCGCGGCG CAGTTTTGAA AGTAGACCTC GCCACCTTCG GGCGTGGAAT TACCGTCATC 27540
GTTCGTCTGA GCCATTGCCG GCAGATTGCC AACAAGGATC GCGAAGAGGG CGACGGCCGT 27600
GGTAAGCAAA CACTCTTTCA TTCGGTTTCT CCTGGTACCG AGCGGCGCGC AAAGGTCGGG 27660
GTGACAAGTC TTTGCGTTGT CCGGTACAGG ATTATACAAA CTGCAACTGC GCGTATATTC 27720
GGCCAGATGG CTTAGCCCGC TAGGGTAGGC CTTACGGGCG TCAACCGAGG TGACGAGCTG 27780
GGGCAGTTGG TCACTGAGCC TCAGACCGAG CAGCCCTCAC TGCGAGGCGT TGATCGCGCG 27840
ACGGGTATAT TTTTCGTCAC TGCGTGAGTC CGGGCCGCCG GAACTCCGCT CGCGCGGTGT 27900
GCCGGCCTTG GCCAAAGCCG AGACTACACA ATTGGAATCG AACCGCGGAC GCCATGCTGC 27960
GCGTTTCTGG ATTTACCCCG CACTCAATGC TTAAAGCAGT TTTCCCGGTG CCAGCGGAGG 28020
AAGTGCGGAT GTGGCCTTTC GAATAGTCGC TGCGGAACGA TCGCGCGTCC CGTTCGGTTG 28080
ATGACTGGAT GCTCTCCGGG TCGTTCCCCT GCCGCGAAAC AAGGATGTTT AGCTCGTCGG 28140
AAAGGCTGGA TCAAACCCCG ATCGAACATC CAGTGGGCGG TCCCGGAAAG CGCAATGCCA 28200
TTGCTCAGGA TGTCGGGACC GTTGGCTTAG ACCAAGCGAA TATGCGCAGC ATCGACTTCA 28260
GCACGACCGC CGCCGTTAAT CAGCTTTAGT CCGGTGATCG CGCATCGCTT GTCGTATGCT 28320
TTGAGGACAA GGCGACGGAA AAGACGATTG CGAACGATTC GAGACGTGAG CTGAGCGACA 28380
CGATCACGCT CCTGCTCAAA AACGAAGGAT GACTGCAGTT GCTCGCCCAT TCCGGGTGCC 28440
AAATCGACGA TTTCTGGCTC GCCGACCTGC CCGTGCGTTT ACGATCGAAG CGCGAAATGG 28500
TGGAGAGGGG GCATGCACTA TCGCTCGCAA AGGAGGTCGG TGCTGTTCAC GGCACCCGGA 28560
CTTATCGTCG GTGCGCTCGC GATAGGCGCT GCGGGCGGCA TCAGCGTAAG TCCGGGCGAC 28620
ATTCTTGCGC TCGTGGAAAA GCCTCACCTG CTGGTCGCCG TTCTGTTCGT CGGCGCCTTC 28680
ACTGGCATCA TGGTTGAGCA GGCTCTGTCA AGGATGCGAA GGCAGGATGG CGCGCGAGGA 28740
ACCGCTCGCG CTGGGAGAAA CAGCGCTCGG CGACGAATGC CGTCGTAGGG CCATGGGCTC 28800
CCACAAGCGT TCCGGAATCA CCGAAACGAC CCGACGCTAC CGAACAGCTC CGGATTGTTA 28860
TGAACTCCAC CTTCACGAGT CAGCCGCTCC TCAACAGGAG CGAACCGCGG GTATTCAAGG 28920
AATTCTATCG CCTGGTGATT GGCTGCAATC CGGCTTGGCA GGTGATGGCC CAAGTCTCGT 28980
TAGGCGAAAT CCTCCGCAGC AAAGACGCGG ACGCGTACAG CTGCATAAAC GCCAAGCGGG 29040
TCGACCTCCT GCTGGTGGAC GGCAACTGCC GCCCTCGCCA TGTCGTCGAA TATCAGCGCG 29100
GAGCGCACCA TCAGGGTGCC GCAGCCGCGC GCGACGCGGT TAAGAAGGCG GCGCTCAGGT 29160
GCGCAGGGAT CGGCTATTAC GAAGTCGTTG CCGGCCAGAC CACTCCGTCT GACCTCAGAC 29220
GATTGGTCGA GAAGCTCGTG GAGAAGCCGG AACTTCCAAT ATGCGCTCCG GACTGACGGG 29280
GGGCTCGGCC GCGGCTGTTG GCGCTCAGAA GCGCTCTGCC CCTCGACACA ACTCAGACGC 29340
CGCAGCGGGC GAATAGTCCG CACGCCCCTG ACCACATTGC ATACTGGTTG GATGCTCTCT 29400
TCAAGGTTGG GGCTTTTCAA TATGTTCAGA CTACTTGCGG CGATACTCGT TTTCACGGGA 29460
CTAGCTTCGG CGGTGAATGC CGATCTTCTG CAGACTGGGG GTAGCCGATG GGTTGTGCTC 29520
GCAAGTACGC GAGATCTCGA CAACGCCATT GGCATCGCGA ACCTTTACCA GCATCGCTTT 29580
GATGACGTTC GCGTGGCGGA GGCCTCGAAC GGTTGGCTCG CAGTGATCGC AGGACCAGTA 29640

TCAATCGCCC GAGGAGCGAA GGCGGCACGC GAAGAATTGT GGAGTGCTGG CGGGTTTCCC 29700
CCTGACTTGT TCCTTAGCAA TGGGCAAAGC TTGAGACGCA CCGTGTGGGA GCCGCCGAAA 29760
GGTCGGCCCA TTCCCACCTG GTCGTATAAA GGTGGCCAGC CACTAGTCTT TTCAGCTGGA 29820
GGGCTCGAGA TTGAAGTGTC TCATCTTGCC GAGGGCAATA CTCGGTATCC GTCTATCACG 29880
CTTCGCAGGG CCGGACGTCT GCTCATAAAG GAAGTTCTGA AGGGGTCCGA GTCTTTCGGG 29940
GACAACATGA ATGCTGAAGT GCGGCTCGTT TGGCTCGACC GCGCGGTCGC CGAACCACAG 30000
ATCATCTTTT CATCTCATTG GAATGGAGCA CATTGCTGCA CAGTTTCTAA AATACTAACA 30060
AAGGTCGGCA ACGGCTGGAC AAGTATCGAA GGCGCTACGC TCGATGGCGG TGGGTATCGA 30120
CTTCAGGACA TCGACGGCGA TGGCAGCGTG GAATTGCTCA GCGTCGACAA TTCCTTCCTT 30180
TACACGTTTG CTCCTTACGT CTTCTCATCA GCTCCGTTGG TAATATCGAA ACTCGATGGA 30240
GATCGTTTGA TTGACATGCG CTGGAATTCT GCCTTCCGAC GCTACTATCG TCGCGAGCTC 30300
TTTGGATGGG AGTACCGGGC AAAACTGGAA CCAGAGATCT GGCGAAAGAA CGGTTTTCTG 30360
AGCGCTTGGC TGGCATTGAA ATCAGTGTTG GGCGAATCAG ACCAAGCCTG GACCGTGGTC 30420
TTGGAAAATT ACGATCGTTC AAGCGAATGG CCCTTGACCG TATGTGATGC TCCTCTGAAG 30480
GAAGGTGTGT GCCCAGAGGA AGCCACTCGC GAGGTGAGTT TCCCGGAGGC GCTGCGCGAT 30540
CACCTTGCGC GTAATGGATA CTTGGGTCCG CAAGTGGCGA AAATCGAGGA AACTTCAAAG 30600
CCCACTGAGC AGCCGTCACC CGCCGATAGC ACATCAACGC CTGCGGCACC TGAGAAGGGC 30660
GCCGCCTCCA GTGCAGGAAC AGGCTTCTTC ATCTCAAAAC AAGGCCACCT TGTCACCAAT 30720
CACCATGTGA TCAAGGGTTG CAGCGCCATC GAAGTGCGCC GCCCCGGCCA GTTGAGGCTT 30780
CCGGCAAATA TTGTGGCAGT GGACCCCACC AATGATCTAG CTCTACTACG TGTGGAAAGC 30840
GATACGGGCG CATACGCATC TGTCCGGGTA GAAACACGGC TGGGCGAGTC AGTCGCCGTA 30900
TTTGGCTATC CGCTTTCTCA TGTCTTGGCC AGCGGAGGCA ACTTCACCCT CGGCAATGTT 30960
ACGGCGCTGG CTGGCCTTGG CAACGATACG CGTTTCATCC AGATATCTGC TCCCGTCCAA 31020
CCGGGTAATA GTGGCGGCCC GCTAATTGAT AGTTATGGCA ATGTCATCGG CGTTGTGACG 31080
TCGAAGCTCG ACGCACTGGC TGCACTTGCG GTAACAGGGG ACATCCCCCA GAATGTAAAT 31140
TTCGCCCTAC GCGGAGCAAG CCTGTATGCT TTCTTGCTCT CCTACGGGAT CTCGCCCGTG 31200
GCTGGGTCGA ATACACAAAA ACTGGACGCG CCGGAACTCG CTGAACGTGC TTCGTCATTC 31260
AGTGTGGCAG TCACCTGTGA ATGACTTGGC ACCGGTCCGC TGCGAACGTG TTACGTGCAG 31320
CTCTCTCGCA CATGGCCACT ACCACTGCAA CGTGCAAGTC GGAGCATACC TCGAGCTCGA 31380
GCTCTTGATG GGAGTAGAAC CTGAGACTTC GTCAATTCGA CGTCTGGCGC AGTTGAATGG 31440
ATCCGAACCG GCGGCACCAA GCTGTAAAAA CTTAAGTCAT TCCGTGCTCG AGATTTTTTC 31500
CAAAAATTGG CTTACGCGAG ATCTTACATC GTTACCTCCC ACCACAGTGA TCGTCGCAAC 31560
GCCCTCACGC TGGCGAAGTT GACGAACCTG ACGAGATAGC CCATATTTTC GTTGGAGGTG 31620
CCCTATGGCC ATGACCGAGA AAAAAGGTGG TTCCCGCAAG CGCAACGAGC GCCGTAAGAA 31680
CCTTATCACC GGATATGCCG GCAAATGGGA ACCGCTCTTT GAGAAAAAAG CGGCGGCCCG 31740
CGCGACCGCA GCGCGCGTGC TTTCTGGCGT ATCCGAAGGT GTCAAGCGCG CCAAGAACAC 31800
GGGCGGCACC GTTCGTGTGA CCTATGTCAT TCGATCGGCG GACGAAGAGC CGGAAATCGA 31860
GATCGAAGAG ATCGCCCCTC AGAAGGACGC GCTGGACACC GCCCTCGCGG CCGCAAAGCA 31920
GCGCGGCGCC GAGCGTGTCG CCGAGATCCT CAAGGGACCC GAGATGTTGT CTGCGGACGA 31980
GTTCGCCGAA GAGATCGGCG CCACGCGCGA GACGGTGAAC AGAAAGCGTA AGCGTCACGA 32040
AATTCTCGGG CTCGAAGGAG CAAAGCGAGG CGTGCGCTTT CCGAAATGGC AAATCGGCGA 32100
TGATGGTCAA CTGCTTGGCG GTCTCGCGCA ACTGTTCGAA GCTTTGAACG GGCACCCTTG 32160
GGCGGTTTAC CGGTTTCTTC TTCAGGAAAG CGATAGCCTC GGCGGTCGCA CAGGTCTTGA 32220
GATGCTGCGG AAGGGCATGA TCGAGCATGC AGTTGGAGCA GCGCGCTCCA TCGGCAGAGG 32280
TGATTTCTCC TGAGCATCTC GCTGAATTTG CTGCCGCCGG CCGACTTCGG CTCGCGTGAG 32340
CTTGTGACCC AAAAATTGAC TTCGAACGCC CCGTGGTACC GGCTCTACCA AAGCCGGTAT 32400
CCCAACCCTC TTGGCTACGG CTTCGGATCA AGCCGCTTCA GTGATCCCGA AGTGACCCTG 32460
TCTGCGGGTT TCGGCAAAGT CCGGACAGGA TTTCCGGTAA TCTCCGGACA CAGTTTTCAG 32520
TAATTCCCGG ACAGCGATTC CGCTAAATCC CGGACAGTTT CCGGCGGCGG TTTGACGGGT 32580
TGGTTCGCGG CTGCGCCGTC TTGTTGGTTA GGCCTCTCAC GACAACGTTG AGAGGGGCCG 32640
ATGCCGAGAC GGAAGCAAGC AAGACGAACG ACAGTGAGGG ATATCCGGAC TATTCTGCGC 32700
CTGACCCATG AAGAGGGTCT TTCGGTGCGC GAGATTGCCG AGCGGCTGAA GATCGGCAAG 32760
AGCTCGGTAT CGACCTATTT GCTGCGATCT CGGGAAGCCG GGCTTTCGTG GCCGTTGCCA 32820
ATCGGCGCGG ACGAGGATGC AAAGCTGGAG CGGCGGCTGT TCGGCCGAGC CGGTCGACCG 32880
CCGCGCGATC TCAGCGAGCC GGACTGGGCG CTGGTGGTTC GGGAGCTGAA GCGCAAAGGC 32940
GTGACGCTGA CGCTTCTATG GCAGGAATAC CGCGCCAGCC ATCCTGATGG TTACGGCTTC 33000
ACCTGGTTCT GCGAGCAGGT TGCCGCCTTT CGGCAGCGCA CCAGTGTAGC GTTCCGCAAT 33060
CGGCACGCGG CGGGCGCCGT GATGCAGACC GACTATGCCG GGCCGACGGT GCCGGTGATC 33120
GATCCGGCAA CCGGTGTCAT CCATCCGGCC CAAATCTTTG TCGCGGTGCT GGGCGCCTCC 33180
AACCTGACCT TCGCCCATGC CAGCTTTAGC CAGCAGTTGC CGGATTGGAT CGACGGTCAG 33240
GTGCGTGCTC TGACCTTCTA TGGTGGGGTC ACCAAGGCAA TCGTGTGCGA CAACCTCAAA 33300
TCGGGGGTGG CCAAGGCCCT TTGGTTCGAG CCGACATTGA CTGCGACGTT CGCCGCCATG 33360
GCGGAGCATT ACGACACCAC GATCCTGCCG ACCCGCAGCA GGAAACCGCG CGACAAAGGC 33420
CGGGTCGAAG GCGCGGTATT GATCGTGGAA CGCTGGATTC TGGCCCGGCT CAGGAACCGT 33480
ACCTTCTTCT CGCTTGCCGC CCTCAACACG GCGATTGCCG AATTGCTCGA GGACCTGAAC 33540
AACCGGACGA TGCGCCATGT CGGCAAAAGC CGCCGCGAGC TGTTCGAGGA GATCGAGCGG 33600
CCAGCCTTGA AGCCCTTGCC GGCGATACCG TTCGAATATG CGGAATGGAA GTCGGCGAAG 33660
GTCCATCCGG ACTATCATGT CGAGGTCGAC AAGACCTTCT ACTCGGTGCC GCATCGGCTG 33720
ATCGGATGCA CCCTCCAGGT GCGGCTCACC CACCGGGTGG TCGAGATCTT CCACGACCAC 33780

CAGCGTGTCG CCAGCCATGT TCGCCGCTCC CAGCGTTCCG GCCACGTCAC CGTCAACGAC 33840
CATATGCCCA AGGCGCATCA GCGCTATGCC AACACCACGC CGGCCAATCT GATCGGCCGT 33900
GCGACCCAGA TCGGCCCCAA TGCCGCCATC CTGGTCGAAC GCATGATGCG CGACAGGCCG 33960
CATCCGGAAC AGGGATACCG CTCGGCCATG GGCATTCTGT CGCTGGCGCC GCGCTATGGA 34020
TCCCAGCGCC TCGAGGCGGC CTGTGAGCGG GCGCTCACCA TTAATGCCAT CACCTATTCC 34080
TCCGTCGCCT CCATCCTCAA ATCCGGCCTC GACCGGGAAA GACCGCAGGC TGAACACGCG 34140
GCCCCCACGC CTGCGCATAC CAATATCCGT GGCCGATCCT ACTACCAGTG AAGGAAGCAA 34200
GAATGCTGAC GAACCCCACC CTCGACCAGA TGCAGGCCCT CGGGCTGACG GGCATGGCCG 34260
CCGCCTGGCG CGAATTGACC GAGCAGTCCG GCACCAATGA GCTCAGCCGC GATGAGTGGC 34320
TCGGACTGAT GCTCGACCGC GAAGTCACCC TGCGCGCCGA CAAGCGCATC CGTAACCGGC 34380
TCGCCTCCGC CAAGCTACGC TTTGCCCAGG CCTGTATCGA AGATATCGAC TTCGCCGCCG 34440
CCCGCGGTCT CGACCGGCGC AACACCATGG CGCTCGCCCA GGGGCAATGG CTCACCGCCC 34500
ACGAGGGCCT GATCATCACC GGCCACACCG GCACCGGCAA GTCATGGCTG GCCTGTGCCT 34560
TCGGCAGGCA AGCGGCCAGG CTCGGTCACT CCGTGCTCTA TGTGCGCGTG CCCCGAATGT 34620
TCGAGGAGCT CGCGCTTGCC CGCCTCGACG GCTCCTTCCC CCGCCTCATC GACAGGCTCA 34680
CCCGCGTCCA GCTCCTCATC CTCGATGACT TTGGAACCCA TACGCTCTCC GATCAGCAGC 34740
GCTTTCACCT CTTCGAAATC GTCGAGGAGC GTTATCAGCG AAAGTCCACC CTGATCACAG 34800
CTCAGGTTCC CGTGGCAAGC TGGCACGACC TTATTGCCGA CAGCACGGTC GCCGACGCCA 34860
TACTCGACCG TATCGTCCAC AATGCTCACC GCATCACCCT CCGGGGCGAG AGCATGCGAA 34920
AGCAAAAAAG CGCACCCCTC TTGACTGGGG CAGAAAACGG CGAAATCAAT CAGCCCTGAT 34980
GCTAACCAGG CTGCCGAGGA CCAACCGCAT CAAACTGTCC GGACTTTAAT GAAATTGCTG 35040
TCCGGGATTT AGCGGAATCA ATGTCCAGTT TTTGCGAAGC GCGCAATTGC CGGCAGCATC 35100
GCCGTTCCCC AAGATCTAGG CACGGCCGAT TTGTGGGGGC ATGGAACACG CGTTGCCGGC 35160
ATTGCCGCCT TCGGCGATCT GCGGGCACAA TTGGCGACTG GAGAACTACG TCGAGGTTCC 35220
AGGATCTGTG CGGCCAAGGT CACAAACGAT ACAGGCGGCT TTGACGACCG GCGCCTCGTG 35280
CCTTCACAAA TGCGCGAAGC AATCACTCGG CTTAACGAAG AATTCGGTTG CAGGATTTTT 35340
GTCATCTCGC TCGGCGACAA GAAGCGCGTT TTCGACGGTG GCAAGGTCGG GACTTGGGCC 35400
GCGACGCTCG ACGAACTCGC GCGAGAGCGC AATGTTGTCA TTATCGTGTC CGCTGGAAAC 35460
CGCGGGCCAC GCGCCGGTAG CCGCGTGGAG CAAGGCGTCA CCGAGTATCC GGACTACCTC 35520
CTCGAGGCAA ATAATCGCCT GCTTGAGCCT GCCGGCGCCA TGAATGTAAT CACGGTCGGT 35580
TCGATTGCGC AGGGAGACGG TCTCGACGCC GATATGGCGG GCGATGTGCG GGTCCGCCCG 35640
ATAACCCGCG CGAACGAACC TTCTCCGTTT TCGCGCGTGG GGCCAGGTCT CGGTGGTGGC 35700
ACCAAACCCG ATCTTGTCGA CGTTGGTGGC ACGTTGATCT TCGACCCTGT CGTCGCCCGC 35760
CTTCGAGGCG GCGAGGATCG GCCGAGCGCT GGGGTTCTCA CCCTCAACCA CAACTATCTC 35820
AACCGTCTTT TCACGGCTGG GTCCGGCACA TCCTACTCGG CGCCACGCGT CGGGTTCAGC 35880
GCCGGTCAAA TTCTCGCTCG CTTTCCTGGC GCTTCAGCGA ACTTGGTTCG TGCACTTTTG 35940
ATCAATTCTG CAGAGGTCCC GCAGCAGGCA AGCGAGCGCC TTCAGATACT GGGAAGCGAG 36000
GCGGTGCGTT CGGTTTGCGG CCACGGTCTC ATCGACCTTG AGCGCGCCGG TTTTTCTGAT 36060
GATGCCCGTG TGACATTGTA TACGGAAGAC GAGCTTCCGC TCGATCATTT CGCTGTCTAT 36120
CGCATCCCAA TCCCTGAGGT CTTTCAGGAA GGAAACACCG AGCGCACAAT TCGCGTAACG 36180
CTCGCCTATG ACCCTCCCGT CCGCCATACC CGGAACGACT ATGCTGGCGT CGGGATGAGC 36240
TTTCGGCTGG TGCGCGGGTG CGAGCCTAAT TTCATCTTCG AGCACTATCG CAAGAGAGCG 36300
GAGGTTGAAG GCCCCTTCCC CGAAATGGAG AACCGCTTTA ACTGCAAACT CGAGCCTGGA 36360
CCGAAGGTAA GAGAGAAGAG CTCAGTGCAA CGCGCCAGCA TCACGTTCAA GCGCGGAATC 36420
GAGCAATACG GCGACAGCTA CTACCTCGTC GTGCGCTGCG AGAGTGGTTG GGCGACCCAT 36480
GTGGATCGGC AACCGTTTGC GGTTGTCGTA GAGCTGCTTC AGAAGGCTGA AGTCCGGTTA 36540
TACGAACGCC TGCGTCAGCG CGTTCGGGCA TAACAGAACA TCTGAACCAG TAGACTCCTA 36600
TGCGGCCCTC TCACCAGTCA TGCCCAACGG AGTATGGAAC GTCAAATGTC TCAATCTATA 36660
CAAATTAACC GCTCTGCCGC TGACGCATGG TCGCAGCTGG TCAAAGGCGG AGCCCTCGAC 36720
ATTGATGGCC GCGACAAAGA GTTCGCCGGG GCGATCTGCA AGATGCTGGA CCCGACGGGA 36780
ACTGATCTCG GGGACGCGCT CGCGAAAGCG ACGACAGATC AGCTAATCCA GGTGTTCTTT 36840
CGTGCCCTCC AGCCTTTCTC AGAAATGTAC CGTGAAATCC TACGGTTTTT CACAACAGCC 36900
GGCGCAAAAT ACGGGCGGGA CCAATGGCGG ATTGGGATTA GCGAAAAGCA CTTCGAGCTG 36960
TCTGACTTTG AAGATTTCGT GCGGATGTGG GACGGCGTTC CCGGTGAAAT CGATGTTCCG 37020
GCATTGGGAC ATGAAGACTT TGGCATTGTT TGGCTTGCCT GGGAAGCGTA TCCGCAACTA 37080
GGAAACGTCG CCAATAAGGC GGCGCGAGAA CCTGCCGAGC TCGGAATTGC CGACGTCGAT 37140
CGGTGGCTCG TCGACTACCG ATCGGGCCAA ATCAACCCCC TCCCACTATC GGTGCTATCA 37200
GAACAACAGG ACGCAGGTTT CCGAGAATTG GCCGCACTGG TCGCCACAGT TCATGAGGGA 37260
TTGGTGGCGT TTAGTCAGAA CCGAGCTGTG TTGCGGTCGC AATTCGATTG GCGAACGTTG 37320
AAGGGCAGTG ATGCTTTTTC AGTCAACCAA CTTGCCTTCC TCGATACGGA CAACTGGCTG 37380
GGGACAGCTG TCGCAGGCCT GGCGCTGGCA AAGGGGATCG CCCCTGAAAG CAGAAGCAGA 37440
ATCGCCCGCG TTCTCCAAAA TGCGTTCGAG GGTCTGCCAA CTCGCAAGCT GAAAGCGAGA 37500
GTCTCCTTGA ACGATCTCGA CCGGTTTTTG TCATTGCCGG TTTGGCAGAA GCGCCACGAG 37560
ATCTATGCGG TCTGGGTATT CACAGAATTA GCTGCGGCCG CGAATGAGCA CGACCTGGAG 37620
ATATATCACG ACCAAGGTAG GATCGCCTTC GAGTTCCGGG AGTCTCGACT TGCCACTGTC 37680
ACGTCGGCAC GGCCACCAAT CGATATGATA ACGGAGCGTC GCTCTCCAAT TGCAAATCCA 37740
GTCGGCGCTG GTCGAACCGC CAATGTCCAG CCAGATTTCG GACTTTGGGC ACGCGATCCG 37800
GGGGCCGAGC GCTGCGCTCT GGTGGTCGAG GTGAAGCACT ATAAACGCAC CGCGAAGAAG 37860
TCGTTTTCGG AAGTCATGAC CGACTATGCG GCTGCTCATC CGAATGCGCC GATCGTTCTG 37920

GTCAACTACG GTCCGATCGG CGACATTCTG GACTGCATCC CGCTCAGCGC GAAGGCTCGT 37980
TGCGCCACCG TCGAGCACCT GACTGCCCTC AACCGGACAG CCAGGGAGGA ATTTCGAGAC 38040
TTGGTTCGAA AGGTGATTGG ATGGCCAGTG CGGGCGGCGC CCGAGATCGG AGGGACAACC 38100
CGGGCGAAAA GTGCTGTCGC GGTCGATGTC TCGCGTTCCA TGGCTCCAGT CCTGAGCGAT 38160
CCTCGATTCA GCTCCGCGCT GGCCGCGATT GCGGTGGCGC CGCACGCCTC TGAAATCTTT 38220
CCCATCGATC GACGTGTCCA CACCGCAATT TCGGTCGAGC AGGCGATCGA GCGGCTGCGT 38280
AGCATCCAAG GCGACATAAA CGCGTTGGAA GCTCCGGTTA CTGAGCTGCT TGGGACTTAT 38340
GACGAAGTGA TCGTTTTGAC GGATCGGGAT GGGCTTAACG ATTTGAGCGC ATTAAGATCG 38400
GCAAGTGAGG AATTTGCCGC AGGTAGCTTT TTCCGGGTGA CTGTCAGCAG GCCTGAGTCG 38460
ACGTGATCGG CCGAGGCTCG CCGCCAACCG ATTAGACTAC CCATCACAGC ATTGCCCCCG 38520
GCAGAAATGC CGGGGGCAAT GTTTGAATTC TTCCCGTCGG GAGCCGAAAG ATGGCTCAAT 38580
GACTGACTGC TAAAAGCCGC AGCTTATGCA TCCTGCGATC AACGGCCAGA GCCGGGTTAG 38640
CCGCAGCGAC AGCATCCAAA ATGGCGGCGA TATCCGATGT GCGGCCCTTG CACGGCGGCC 38700
CGCATGCGGC CCACACCAGC CTGAGGGCGG CGACCGGGAA GCGCGTGGCG AGCTCGGCAT 38760
TGTTACGTTC GAGATCGTGA AGGCTGAGCC AGTCATCCTC GAATGTCGAG ACATATGGTA 38820
GAAGGGCTTC GACCGCAGCT TCAAACGCAG TGCCGGCCGC CGTTGCTAAC GAAAATACTT 38880
CCTTGCTCAC GTCATTTGAA AGATAGCTGC GGTCGCGCGG CCAGATCGCC TGGAATGTTG 38940
GGCCATATAG GTCAGTCCAG GCGGCCGCTC GCTCGTCGTC CGGATTTTCC TCAAGCCAGT 39000
TTGTCATAAT TTTCAAGGCA CCGACGCGTA TGTCCGCGGC AGCCTCCCGA AGCGTCTGCC 39060
GCGCCTGCTG CGGCGAGATA CCCCTGACCA CATTCGGTTC GTCAAAAGAC GCAACCACCG 39120
CTCGGACGAG ATGCGAGGCT GCATTGGCGG CCGAGACATC TTTTTGCCGG CTCTCCCGAA 39180
CTCCCTGTAG AATGAGATCG GCAAAGCTGA TTTCAGTATC CGCAACGAAG CTGTTGTATT 39240
CGACCAAGAC CCCGCGTAGT CGCCTCCCCT CATCATTGTC CGCAGCGATG AAGGGTCTCA 39300
AGTGGTCGAG AGCAATTTCG TTCGAAACGC TGAAAACGAA TGACAGGTAC CGGACGCACG 39360
CTGCACGTGC CATTTCGCCG GAATAGGTCA TGGACACGAT CATCCGCTGC AATCGTTGAA 39420
AGTCCGCGGC GTTCACGTCG TCTCCATTTT CCCTTTGAGA GCTGATAGCT TGAAGCAGTG 39480
CTTCCGCAAG TGATCCACCA GTCGAGTTGA TGACCCGGTC ATAGGTCTCG ACATCATCCC 39540
CCCAATCAAC GTCGGGCTCG CCCTCAGCAG CTTGCCACAG CCGATCCCAC CAGTTCGCGG 39600
GAAGAGCGAG TTGAAGCTGC CGTGAGTGTT CTGCTATGCG AGCCAGGGGA CCGACAAGAG 39660
CGATGACGAT CTCGTCATCT GCCTCTCCGA GAAGCTCGAC CGCTTCTTGA ATGACACGCA 39720
GCCACTCTGG GCTTGTCATA TTCGCACGAC TGGGAATGAT CTCTAGCCAC GTCGCCCAAC 39780
GTGCAACTAC GCCGATTTCA AGGGGATGAG AACGCAGCGC AGCATAGGCT CCGACCGGAT 39840
CCTCTCGACA ATATTCCACC CAGCCCTCTC GATCTTCGAT GTCGCTGCTT TCCTCGAGCC 39900
GATCGGCGAT CGTGAGGCGT TCGCTTGGTT GAGCCGACAC AATTTGCGCG GTGCGCCCGC 39960
GGACGGCCCT GACTCCGGAG CTCCAAGTGC GGAAAAGATC CCGCTCATCG ATTTCACGCG 40020
ACAGGTATGT GCGCCGCTCA CGAATTTCGT GTAGCAGGGT TTCGGCCTCC TGCGGGAGAA 40080
CTACGTGCTC ACTGATCGCG GTCAATCGAA CCCAAATATC GCGGTCACGT GCACGGTCCT 40140
GCCAGTCGAT ACCGTCAGCG TTCTGTTCCC GGTCGGCATA TCTTTGTGGA CCCTCGACAA 40200
GGAGGCGCCG AACTAGACGT TCGACCGCCT CTCCGCTTGC CTGAGCAATG CGAGCGCGAA 40260
CAATGGCGAC GAACTCCAGC GCGAAGGACC AGAAGGCCGC CTCGTTGCTG GTTGCCAGCG 40320
CCTCAATAGC CTCGTCGGCG GTGAAAAGAG CTTGATGTGT AAGTGCGTGC ATCCAAAGCC 40380
GGGTGGTCAG GTTGAACGGC TCCGCCCGCC ATCCGCCGAC CACCCTTTTC GTCCCCTCCA 40440
AATTGGCTTG AGCGGCCATG GGCAACGCGG ATGTGATAAA TACGGTGAGC GGAACAAACC 40500
CGCGGCGATG CTGATTTTGC TGATGATTAA CAACGGACGG TACACCGAAA TCTGTAACAT 40560
CGCGTACCTG CCCGATCAGT TCAGCATCGC GCGCAGTCCG GAGCTGGCAA GAAAGTCGCT 40620
GCGAACCCCG CTCGAGCAGC CGCAGGATGT TTTCCGCGTT GCCGGCGGGG GACGCCAAAA 40680
CATCCCGCAA GAACTCCTGA TGCTCGCTCT CCATCGAAAA ACGAGCAATA TCCGAGAGCT 40740
GGTTTGGGTG CTCAGAAGGA TCCTCATCGC GAAACGGCGC CTCGATTGTC AGGCGCGGTC 40800
CGATGGCATT GACCAAGTGT CCGAGGTCGG ACTCCACGAC CGGCCCTCGG TTCAGCCGGT 40860
GACGCAGCTG ATAGTCTCGC AAACTGTCGT GTGATGCCGC CGCGGCCGCT TCGGCGAGCA 40920
AGCGCCAGGC TTTCTCCCAT ATCGCATCCT GCGGACGGTT CTGATCGAGT TCGCGAAACA 40980
CGGCATCGGA GAACTCCTGT GATCCCCTCC CCAGATGTTT AATGGCGGTT AGGTATCTGA 41040
CCTTGTCATC CCATCCTCTT GCGAACCATC GGGCAAGCAT CCAGGCTCGC GCGCGTTCGC 41100
CGAAAGCTCG GATCACCACG GCAAGTGTGT CGAACCATGC AGCATCTTCG CATCGCGTAA 41160
TGAAGTGCTC GAAAAGGTCG GCCCTCGCGC CCAGCGCCCA TTTGAGGGTG CGAATATCGT 41220
GGTCTGTAGT TTCGGATACC GGGCCCGAGA CGATGGCGGC GATCATCTCG CGACGACGAA 41280
CATCTGGATT CTCCACGAGG TTGGCCAGGC CTTCAAGACT TGACCATAGT GGCCCGTATG 41340
GATTGCCGCC TTCTGGATCG AATAGGAGCG GCTCCACCGC CAGAGCCTGC CACGGGGCGG 41400
CCGCGGCTTG GGGGTCGCCA TTTGATGAGC TGAGCGCATA TACGCGCCTG AGATCGGTAA 41460
AACGCTCCCT GTCACCTTCC AGAACGTTCA GGATGTAGCG CACCGGAGCA TCGTTCGCAG 41520
TATAGCCCAC CAAAACCAAG GTGCGACAAC GCATGAGGTC GAAAAGGAAG CGAGCGGCCC 41580
AGCCGGCTCG CAGATATGCC TCCCCATACT GAGCGCTGGT CAGTACCAGT TCTGTTTGGG 41640
AAAGATTGAG CTCATCGTCG GTCAGCCGGC CGTGGGTGTG GACAACTCCC GTGAACCTCG 41700
CGCTCCCTGG AGCGGGTATA TCCTGTCCTG CCGCGCTCTC CTGCGTCGCG AAAGCTGCCG 41760
AATGCGAAAC CATGAGTGAG CGCTCGAACA GCGTGTCAAA GTTCGTGGTG ACGATAATCG 41820
GCCTTCCAAC CTGATCACGT GATAGCCGCA GGATGACGTC GTGATGCGCG GTATCAGGAT 41880
TTGCGGGAAC CTGCAGTTCT GCGGCCGCAG CGTCTATAAC ATCCTCTGAT CGTACAAGGC 41940
GCCGTGACAG GGCACCCAAA ACTTCTTCAT AGCGAGACGC CTGGAAAGCA TGGTCTTCGG 42000
CAGGCGTGCG CTCTTCCCCC AATCGATTAT AGACGTTGAG CACGAGGTCG CGAAAATTTG 42060

GCAGTTGCGG CGCGCTGACC CCTGCACCGC AGAGAAATAC GACATTCCCT TCGAGCAGTT 42120
CGTCGACGAG CGCTTCCGGG AAATCAGGAC CGAGGTGATG AAGTCTCATG GCACTCTGAC 42180
AAGCTCTTCC AAGTTGAATA CTGCGCTGCG TCGCATGCGC CGACGCTAAA CCAATGCATG 42240
AAACATGTCT TGGAGAATTG GAAGCGCGAT TGCGGTTCCC TGACAAGCGG CCGCGAGAGC 42300
GGCGGCTTTT GCGGCTCGAT TGTTCAGCGC ATTGGCGGCG TTCTGCGTCT TGAAGACATC 42360
AATATCGCCC TTTGGGCCGG AGGCTAGGAT AGCCGCGGGT CTCCATCCGT CTTCCGGGAT 42420
CTCGTGATCG AATGGTACGG CAATTACCGT TGACCGCCAC CATAATCCAG CAGCGGTGAA 42480
CGCTGCCATT GCTGAGAGCG CGTTTAATAA GGTGACTATC ATTTTGCAGT CTCGCAGCTT 42540
CGGGATCCAC TGGGCCTATA TGTAGAACTT AAACGGCTGT TTCTTCTCGC CTTTGATCGC 42600
CGATCCCATC GTCAGGACAT CGCCGACCAT TCGAGCGAAC CGCACGGTGA CCGGAAGGCC 42660
ATCGTTGAAG TTGCAAGAAT TGTAGTTGAT CTTGGTCAGG CCCATGATGT CGGACAGCAC 42720
GTCACGAATC TCGGGCTTGG CATCCTTGCT CCGCGTCACG GTGACATGTA GCGGATTTGG 42780
GGTTTCCGGG CCGATATAGG TATCAAGCTG CGGAACGTAT CCGGTCGTCC AAATGTAGGC 42840
TGTCTGCGCA TCGAGGATCA AGGCTGTGCC GCGCAACACA GGATAGTCTC CATCGCGGAA 42900
GAGCTTCATT TCGCCACCGG TTGGGCGAAT CCGCACGCCA ACGACATTTG TCCCCTTCGG 42960
CGCCGCATCG GCAAACGCGG CCCATTCCTC ATCGTTGAAG TAGGTCTGAC CATGGATGAA 43020
GAGCTCCTTA GGCGCCTCGC CGTGGGTATC GACATAGCTA TCAAGAACGA GCTTCAGGAG 43080
GTCTTTGGCT GCGGGAGCCT TCAAATGATA GTCGTAGTCC CCGGTCTTCC AGGGGCCGTT 43140
TGCACCACGG AAAACGACAC CGTCGCCCTC GTTCAGAAAC ATTTGCGCCG CGCAGCACGC 43200
GTGGCCATCG GGATCATTGG GCAGGCTCTT GTAGACCATG CCGAGGTAGC AAACGCCAGG 43260
CCGGATCGTC GATAACCGCC ACGGTGGGCG CGGCTGTGTC TTATAGTACA GACCAGTGCC 43320
GAGATTCCAG GCAACCGTGG CACGATCCTG CGTCTTGCGG GTCGGGTAAC CTGCCGTATT 43380
GAGAAACGCG CCTGGCGCAA GCGTGGTTTC TCGAATGAGC TGCGTCGGCG CGATGTTCAA 43440
GAAATCGGCT TTGATGCGCC GATGAAAATC CGGAACGTCG TCAAAAATGG TCTCACCACT 43500
TTGATCGATA ATACCTGCCA AGAGCGGCAG GCTTTCGCGC TTCTTCTGCT TCTTGGCGAA 43560
ATCCCCTTTC TCCGTTGGCA GACCAGTGCG CTTAGAATTT GGTCGGCACC TTTCATAGAC 43620
GATCTCAGGA AGCACCAGCA CCCAGATGTC TACCGCGCGC TCCTCGTTGT GGAGATGTTT 43680
GTTTACACGC TCGATATAGA GCTTCGCGAC CTTGTCCACT GCTTCGTGCT GGTTTGTCAC 43740
CCGCGTCGCG CGATCGATAT CCGCGAGCGG GATCGATAAT GCGCTGAGCT CGTCTGGGTC 43800
GTAGGAAATT CCAAAGGTCT CTTCCAATCC CGGGAAGTTC GCGAGGTGCA GCCGGTTCGC 43860
CTTTTCTCCC TTCCCGGGCG GGGGCACCTT GACAATGGAT TGTAGCTCGC GGCTCCACGT 43920
CCGGAAGTGA CCGATGCCTT CACTTGTGCC GATGACACCG ACGCGGATTT CTTTCGCCTT 43980
TTTCCCCTTG AGATGGGGTC CGTAAAGGTG GAGGCCGTCC TTTGGGTGCG CGCTCTTCTG 44040
ACCGAAGGCA AACTCAAGTT CCGGCTCATG GATATGATCG ACCTCAAGCG ATCGTTCCAG 44100
AAATTTCATT CGCCGCCCTC CTCATCACTG TCAGGCCGCC CGAGAGTGCT GTCATCCAGC 44160
TCCTCGTCCT CGTCATCTAG GTCGTTTGGA AGAACGGTAC TGACCGGCGA TGAAAACAGC 44220
ATCGGCGATG CCTCGATAAC GAAGGTTTCC GTCTCGGAAA GAGGGAGGCG AACAAAAGCC 44280
GATTCTCCGG AAAGCAATTC TAGAAACGCC AGAAGCCGCC CGTACCACTG CTTGTTGCGC 44340
CAGCCCTTAC AGACCGATCG CCTCAAGCGG TGTAGCTTCT TGGGATCGTC GACAACAAGG 44400
CCGGCTGGGG TGTCGTTATC TTGCGCAAAC AGGACCCTCG ACTTCAGCTT GAAGTGCCAG 44460
AACGGCCAGA AAGCTGGCAT GGCAGTCACA CCGAATTGCC AGATATGGCC CTTAGCGGAA 44520
TTGCGCAGCA TCGATGACCG CTTCTTCTCG GCCTGGTTCC CCCACGGAAT TCGCTTGCCC 44580
GTCGGCGCCT GTTCTGGTGA CGCGTGGAAG CCGATGCCAT TCGAATATCC GTGTTCGATG 44640
AACCCGTTGC GTTTGCAGTA GTTGAACCAG GCCTTCTTGA GCATCGCTAC AACGAGGTTG 44700
GAGGCAATCT GGCGGTCAAT TCGCAGTCGC TGAAAGCCGC CCTCCGTGAA TTCCACGAGC 44760
GGAATTTCAT GTTTCAGCTT GAAGCGGCCG ACGGCTTCGA ATGCCATTTC GATTTCCGGC 44820
ACACCACCGA ATGCCAGAAA ACCTGATCCC CCGACCAATG CGCAAGGATG TCCATAGCTG 44880
TCGATCGCTC CCTGGAGGCG CCCTTTGTTG ATCGATCCAG AGCATTCGAA GTATCGGATG 44940
AAATCTGGCG CCTCGGCGAC CCGAAGCCAG TTTGAGGTCA GTCGCTCAGG AACTTCGATG 45000
ATCGGCACGG CGCCGCGCCG GCGATAGATC TCCCAGTTGG GGTTTATCAT CGTGTCGATG 45060
CTGCGCTGGA CTTTTTGCCG TTGCAGGGTA TCGATGAGCT TGAGCAGGCC TTCTCCCCAA 45120
TTTCGAACGA AATCGACCGC GACAGTGTCA CCGAGACCTT TGATCTTCCG AAACGTCTCC 45180
ATGCGCAGCG GAATGACAAA CCGCTGATCA CCGAGTTCCT TTCCCGTTTC TAGAGCAATG 45240
TCGAGGTCGT CCTGGATGCT GCTATCCTCA AGACTGGAAT CACGGCAGAG AAGCAGGACC 45300
TTGACCGCGC GATGGCGCAG CGCACTGGTT ATCTCCCTTC GCCAACGTTC GCCAGGCATG 45360
AGGGTGAGCA CGTCGGCAAA GACCTGGTAG CCCTCGGCCT CAAGCTTTGG AGCAAGCCAT 45420
AGCGTGAACT CGTCGTCGAT GGGTGAAGCC TTGATTATGA AGATCGTATC GCGCTTTACG 45480
GGCTCGATCG GAGCATCGGC TGCCCCGGGT GGCGGCATCA GGCGAGCGAG CGTTTTGGGA 45540
ACCTCCGCCG GCGCCATTGC CTTGCGCACC GCGTTGGTGA CGACCTCTTC GAGCACTGGG 45600
GTGCTGTGTG CCCAGAGCCC TTCATGAGCC TTCACAATCT CAGGATAGGA TCTGAGCAAG 45660
CCGTTGAGGT CGTCCGCGCC AAATATGTCG CCGGGTCCGA CTAGGCTTGG TCCAATGATT 45720
TCCGTCAGCG TTGCCTTGTT TGCCGGGGTA AGCGGTACGG ACGTCGTCAG AATGTATCGT 45780
TGCGGGGCCA GTTCGTCGAT TGAAACACGT TCTTCTCGCA TCTTCGACTT GAGTTTCGAG 45840
AAGCCAGATC GGAGATAGTG CTTGGCCTGG AGGATGATCG AACCATCGGC TTTGGCGTGG 45900
CGTCCATCCA TACCGTCATC TGCGCCAACG GTGAACGCTT CGAATCGAAC TCCGGTTTCG 45960
GCGCCCACAA GATCGCGACT CAGGTTCTCG AAGTCATCCG GAGAAAGTGT TGAGAAATCA 46020
TACTTCATTT GTCGGATGAT GGGTCGCGAC AATACTAGGC GTCAAGGCAT CAATAGATGT 46080
CTTCTCAGTT GTAGTGCGGT TACCAACAAC TCTGATCGCG TGAAAGCGCT GACTGCAGTC 46140
CCGTTTCGCC AGAGTCGCCC TCCGCTGAGC CCCTCGTGAA ACCGAATACC CCAGCACTCG 46200

AGAAATCCGG AAGCCCGCTC GGTTCTCAAC TACGAGGACG GATCGGTAGG TAAGGGCCCC 46260
ACCTGTTCCA AGGTAGCAGA GACTGCCGCG CTGTAGCTGG GTTTGGCGGT GACAAAATCT 46320
CGAAGCGAAT ACCGCTCCGT AGAACGGCTA CTGCGCACCA AGCCATCCTC TTCGACCCAA 46380
TCAAAGGCCG AGATAAACGC ATGCAGGCCG TTCGCCCGCT CGTACCGAGC ATCATCTCCT 46440
CGCCTCTATC CGCACGCTAC CCCGTCCCGC GTGCCGCCAA CCCTATCACG CGAAAAGCAC 46500
CTCGGTGAGG TTCTCCGGCG GCGACCAGAG GCCAGCCTTG GCCTTAACGT AGGTTTGGCA 46560
AAACGTGACG ATCTTCTCCT GATCGCCAAA TTTCAGCTTA TGCTTGGCAA TAGTCGTGAG 46620
CTGTTCGAAC GCATTCGACG TGAAACCGTA TGTCGACAGG AAGAGGCCAC CATCTTTCTT 46680
TTCTTCGACG ATGATCTTCA AGAGCTTTTC CACGGCCGCC GATCCCACCT TCGTGGATGA 46740
ACGCCAATGT TTGATCTCTA CGTAATACTC AGCGAGCTTA CCCCTCACCG TGCACGTCAG 46800
AATGACATCC TTTCCACCAT CCTTCGACCC CGGCGTAAGC GTCACGGCAA AACCCAACCC 46860
TTCGAACACC TCTGCGACGA CCTGCTCGAC CATGCGCCAT TCGAGATGAT CCAGGGCATC 46920
CGGACTTTTC GCGATCAATC GCGCTAGCCC GCTGCTTAGA TCCCGCAGCA TGATCCGAAC 46980
CTCAGCCTCC ACATCGACCT TCTCTTCGCG CAGGCGACTG ATCCAGGCAC GCATTCCGCC 47040
AATGTCGAGC AATTCGATCC TAAGCGGCAA GCTTTTCTCC ACTGCGGCAT GCGCCTCCCG 47100
CGGAAATTCG CCGTTGCTCA CCAGCACGAC GCGAGTGAGA TCCTGCAACA ATGCAGCGCC 47160
GATCAACTTC TGCAGATCTG CCATCCCCAC GCGGCTAACA GTCTTCCTGT AAAACTTGGC 47220
CTGAACTCCG ATGGTCTCGG CGGGTGCAGA ACTGGTCTCA TCCGCCGTAC GCGAGGCGCG 47280
GAAATCGATT CCCTGATCGT TCAATCCGCC AGTATGCTCA ACCTCGTAGC CTTCGATCCC 47340
GAAAAGCGGC TCCAACACGC GGCCCAGGAC CAATTCCGAC TCTTGGGGAT CGAGGCTCTC 47400
GTTCGCCCGC AATTTATTGA GCTCGAGATC GAGTGCATCG CACTCCGCTG CACTTAGTTT 47460
TCCCAACAAC CGCCCCAACC GCGTGCTCCT TCCAATATTC CCATACCTAA TTACCAAACG 47520
GATTTGGTGA TACCCGTAGA GCCTGACTTG AGGGTTGAGA AAGCCGAATT TGAAAGTGTT 47580
GGTGTCAATT TTAACAACAT GTCGCTTCCG CCCAGCAAAG GTGGGACATG CACGCTTGGC 47640
ACCAGGATCT CGTAGACCCA CTACCGCCGA AAAAAGGGGC GTGGGCTTAT ATCACCCCCC 47700
ACGCCCGAAA CCTCCCCCTC CCCGTCATCT CTCTCAACCC CAGCTCCGTC ACGATCCGCC 47760
GCGCCGCCTG TGGGGTGACC TCGAGCGTCT TGGACACCAT CCCGGCCGAA ACGAGCGGTT 47820
TTGCCATCAC AAGTTCGACC AGCTCTGGCA GTTTTGACGA GGTCCGCCGT CCGTCCAGCT 47880
TTCGGTCCAT CATCGTTTTC GCCAGCGTCA GCCGATCATG TTCTTTCATG CCGATCTCAG 47940
CAGCCGCCAG CAAACCGTGG GCGATGGCAA GCAGCCGGGT CTCCCGGCCG CGATGGCGGC 48000
GCCGATCGAC GGGTATGGTT TTCAGACCGA GATTGATGGC GGCAAGATGG GCGCCGGTGG 48060
TGATGCCGGC TTCCCGCAGG ATTGAGGCGC AAAGCAGCCG GCCGAGCCAG GGCGCGTGCT 48120
GCAGGACAGA CAGTTCGTTC CAGGCATCAA GGGCGACGAT CGCCTGCAGC ACCGCCGGCA 48180
AGTCTTGAGC CTGGCGCAGC ACGCTGCGCC ATTCCACCAG CCGGGCGTCC TCATCCCAAT 48240
CGAGGTCGTA GACCAGTGGA TCCTTCTCGG CAGCCCAGCT GTTCGCGCCG GCACGGCCGG 48300
GTCGCCTTGC GTCTTCGATC GCGGCCTCCG AACGTGCCAG CACCGCATCG ATGGCGGCAT 48360
AATCGACGCC GGGCAGATCT TTGGCGTCGT TACCATCGTC CCCCTCCCCT TCCGGATCGA 48420
TAGCGACCGC GGGCCGAATG ACGTCGGCCG GCTCCACCGC CTCGACGCCG ACCGGGTTGA 48480
TGTCCGACGT CTGTCGCAAA GTTCGGATAC CCTCTGTCGA TAACGGCCAA TCTGGCGACT 48540
GCGCAGCGAT GCGCCGGCGG GTCCGCAAGA CGTCGCGGGC GATTGTCAGT TCGTGGGTTG 48600
GGGTGCGGAT GTCGCGGGTA GCGTCGTGGA GGACGAGGTC TTCGAGATGG ACGAGTTCGC 48660
CGTCGATCCA GAGGGAGGCG CAGGCGTCGA GGAAATGCAT GCGCTCAATC CACCCGCTTC 48720
CGACCGGTGA GCGGGCGATG CGCTCGTCGA GACGCGTGAA ACCGATGCCG GCCTCGAAAG 48780
CCGGCCGCAT CAGGGCTGTC GTGCTGATTT TCGCCAGATC ATAAGCCATT GAAATTATGG 48840
TAAACAATTT TCTAAAGGAA CTCTAGTGCA ATATTACTGT TCCTCACATC GTACGCTTTC 48900
GGGTTGCTTC TAACCATCGA TAGGTTTCCA GTATCGATAG TTATGGATTT GGCGTTGCAA 48960
ATCACCTACG TCGGTCGTAC CAGATCGGGG ATTCGCCTGG AAACCCGGGC TTTTCAGCCC 49020
CTTCCTCACC CTCTTTCAGC GAACTCATTC CCCTGATCGA GTGAACTGGA CCTCTGCCGA 49080
GCGGCCGACC GCGGAGCCGT CCTCCGGCTA TTTCATCATC CCGGCCTGTC AGAAATGCAA 49140
CGGTGGTACC CGATCCCATG CGTAACAGGG GATTTCATCG CATTTCGGCC ACAGAAGGCC 49200
GCTGGCGCGA TTTCCCGAAC TTCGACGGTC CGAGACCTGT TGCAGACCAG ATTCCGCGCT 49260
GTACGGCCTT CCATTTTGAA CATAAAACAC GCTTGCAAAG GAGCCTTTTC TCACACATGT 49320
TTCATTTGTA CAAACCGCCC CGAAACGACC GAAAAATTCG ATGGCAACGG CTGCATACCT 49380
CAATTCTCCT TCCCTGCAGC AAAGGCTGAT CGGATACGCC CGTGTTTCGA CCGAGGATCA 49440
GCTCAACGAC GCTCAGGTCG ACGAATTGCG GGCGGCTGGT TGCCACCGCA TCCACCAGGA 49500
GCACGGATCC GGCGCATCAC GCGCGCGGCC GGTGCTTGCG AAGCTTCTTA AAGACCTTGC 49560
CATGGGCGAT GTCCTCGTCG TCGTTCGCCT CGACCGTCTG GCCCGATCGG TCAGCCACCT 49620
GCTCGACGTC ATCGAAGACC TCGAGAAGCG CGGCGTCCAT TTCCGCTCGC TGCGTGATCC 49680
GATCGATACC TCGACGCCGC ACGGAATGTT TTCCCTGCAG GTGCTCGGCG CCGTCGCCCA 49740
GCTCGAGCGC GCGCTGATCG CGGAGCGGAC CAAGTCCGGT ATGCAGGCCG CCAAGGCGCG 49800
CGGCCGGCTT GCCGGCAATC CCGGGCTTCG AGAACGCCGG CCAGAAGCCA TCCGTGCGGT 49860
CTCAGCGGCG CGCGAGCGGG CCTACCTCGA TGAACTGATT GTGTCAGCGC AGACCTGGCT 49920
GCCGACAGTC CGGCGACTGC GCCCGCGACA CAGTTGGGAC AATGTCGTGC GGATCCTCAA 49980
TCGCAGGGGG CACGACTGGA CCGTCGAACG GTTGCGGCGG GCGGTCCACC GGCTAGTGCG 50040
CGAAAAGCTC GCGGAACCGG AACTGCTTGC CCGATCGCTG CGCCGGCCGC CCGAGGATCA 50100
TCTGATGCGG CTGGTTGCCG GGATCGCCAT CGCCGATCCC AATCTGTCGC TGCGCGACAT 50160
TGCCGCCCAG TTGGACCAGA TGCAGGAGCG ACCGCCACGT GGCGGCCGCA AATGGCAACC 50220
GTCTTCCGTC CGAGCACTAC TGGACGAGGC GAGTCGCATC GGGCTGGTTC GCGCTTGAGA 50280
GGCTTGGTTC ATGAATTCAA GAGTGTTCAC GCGGCGTCGA AAGACGGGTG CGGCACTGTT 50340

TCCGGTCGCC GCATGCTTCC AAATGGAAAA AGACCGAGCA ACCCCCGGGC TATTCCAGAA 50400
AGCTCGCGTT GTGAGGAAGG GGCCGCCTAG GCCTGCAAAT TGTCAGCCGA CATCTTGCCG 50460
GACTTACGAT CCTTTACCAG CTCGTAACTG ATCTTCTGCC CATCCTTCAG CTCCCGCAGG 50520
CCAGCCCGCT CCACCGCTGA AATGTGGACG AAGACATCGG CGCTCCCGTC GTCCGGCTGA 50580
ATAAAACCAA AGCCCTTCGT CGCGTTGAAC CATTTTACCG TTCCGGTCGC CATCATGCCT 50640
TCCTTCGATC ATTTGCCGTA CCCGCCACAG GGCGGGCGAG GCTCGCTTCG TATACGCGCG 50700
CACAGATAGA CGCTTCTCCG CTCCGGGTAA AGCAGCCCGA ACGTTGACGC AACTGCCCAG 50760
CCCAAGCAAG CGATCTTCGT CGCCATAGAC GCTACTTTTT GCCTCTTTGT TTCAGCCGCG 50820
ATGCTGACGG GAGCATCCTC TGCTTGCCAC ACGTCGAACT GATTTGTCGT ACAGATAAGA 50880
TCACTGAGTT TTCGGAACCC AAGCGTGCAC GGGTCGAAGT CCGGCGCCAG GTGAGACCTC 50940
CATTTGCCGC AGAGCCTCAT CCGAGGCGCG CTTTCCGCAG AGCCCCGCAA GGTTGGCTCT 51000
GGCGGCTGAA CTCGTCGTCG CGAGTCGTGG GCTAGGTGCC TGAGTCGCCC TCGATGCCGA 51060
CGCGCGACAC GGCGATGCTC GCGCTGACTG CCTTTCTCTA GGCAACGGTG ATCCCCGCGC 51120
TCACCGGCGC GGTTACGCCG GGCCCGGCCG ACCTCGATGC GCTCGCCCTG AACGTGTTCG 51180
ATTGAATGAG GAACCCGTTC GAGGATCATG CTCCCGCGCC ACCTGATCTG CCCCACGTAC 51240
AGATCAGTCT GCCCGTTCGA GTACCGCTAG ACGCTCGCGG TAAGATGGTT TCTACTGCCG 51300
AAGCAGGAAG TTGGCGCGCT CCATCAGCCA GTACAGCGCT TCCTCCGAGA CTTCAAAATG 51360
GCAAGGGAAC ATTTCGACAG GCAACACAGC AGAGCCCAAC GGTTGCTTGA GCTTAGCCGT 51420
GTGGCAATCT CCCGCGACTG GGACAAACAC GCGGCGTTCC TGCCCCCGTG CCGTTGAGGG 51480
CGCTTATCCC ACTCTGCTAC TAACCCTGAC GAACCACGGC CCTCCCCTCG CATAGCCTGA 51540
CGTTCCTGCG CGACTTGGCC GAACACCGGG ACAGCGGCTC GTCGAAGCTT GGCCGCGCGA 51600
ACTTTCTCGC ACCGCGCGCG ATCAGCTGCG ACCGGCGTCG ATATGACCGT GTCCCACTTC 51660
AACCGGTCGG AGGCCTTGTT CGAGTATTGC GGCTCAGCGA GCTTGCCGTC GCTGACGAGG 51720
ACGAGAATTC CGAAGTCCGA GGACCGCCCA ACGTTGTGTG GGGATCGACT ACAGCGTCGC 51780
AGGCATAGAA GCCGAACATA ATGGTCTTCA GGGTCCGCCG CGCTTCTTGA AAGCAGTGGA 51840
GCTGCCTTCG AGCGCATCTA ATCGTGTGAG CGCGGGAACG TACGGGAAAG CCCGTCGCCG 51900
AGGCTGCTCG GGGCACGCTG GCAAGGGGCA CGACCCTGTT CGTCGTCGAG TTTCGTTGCT 51960
TCGCCTGCCT TCGTGGCAGC GTCTTAGCCT GCGTTCCAGT CGCTGTTGTG GGTAGGGATT 52020
TTCCTCCTTG CACATCCCGA TCTCCGCCGT GGTGAGGCCT TCATGGAAGG CCGACAGTAC 52080
CGTTGCCATC GCTGCCCCCG AACTTCGTTA CGGCCCTCGT CAACCCGCCG GACTGTACCA 52140
AATTGGCGAA GCGTAAGCGC GCTGCGTCAA TCGAACTCAG GACCCCTTCC CGCCTCTGAT 52200
TTTAATTGAG CAGGAACATA CGGTAGAACA TCGAATAGCG AACGAAGGTC CCAGGACCAC 52260
CGCGGCCGGT CAATCAGAGC GCCACACGTT GATGTCTCAC CGACCAGGAA CAGGTCCGTG 52320
CCAACCGACG AAGCCTCCCC TGCCCCACCC TGCGTTTCAA TCCGCCTCGA CCGCACCAAA 52380
ACGACGACGA GCGATGATGA CGAACATGTG CTCTTGATCT GCCGGCCGGC GATCGCCTTG 52440
AAACGCTGGC TCATGGAAGG CCGGAACCAA CAGCGCGGTG GTGTTCCGCA TCTACCACAT 52500
GAAGCAAATA TCGACCGGCG AGCGCTACCA GCCGCATGAA ATACGCTTAG CCAAGCGATA 52560
ACTAATGCTC TGTTGGAAGT GTGACAGTCA TTCTGGCAGA ATGACGAAAC GCGGTCACCT 52620
ACGGACTGGC TAAACTTTAC GACTCGCGTC CTACAAACAG GCGATCAACG CCTCGAAAGT 52680
TCGACCGAAC CCCTCCCTTC AACAGCAAAC GAAGACAGTT TAGAACTAAC GCTCTGATTA 52740
ATTTGCGCAA ATTTCCGTTT TTGTCAGCTG ACAACTTTTT ACAGTCTGTT TTAAGGAAGC 52800
AATAGTCTCC CCTGCCTTTG CAACCAACTA AAAATCAAGA TCTGGTGTAG CATTTGGCCT 52860
TTCACAGCAT CGTCCTGGCG CTCAAGAAAC ATCTCTGGTC TGATGCTCGA ATGCCTCCGC 52920
CAACTTCTCG GACACCATGC TGAGGGAGCT GCGGACTGGT CGGGCTTGTC CCTGCCTTTG 52980
GTGTCGTCCA TATTGCCATC GAATGTCCCT GCCTGCACTG TCCTCGTACC GACGTGGACG 53040
ATTGGAGTTG CTCCCCCTAC TGAGACACCA TAAACGAAAG CGTCATAACT TCTCCTAATC 53100
GGTTGACTCA ACGTCCCAAT CTGCCGATTT TGTCAGCCGA CAACAAGCGG TCGCCCAAGA 53160
GCTGAGGGGA TTGGTTCCCG CCGATTAAGC GGGTTCTTCG GTCAGGAGGC CAATCGATCA 53220
CTTCCGCCGC GCGCCCTCAG CAGTGCCATC AACACTGGCC CCGGCGAAAA CTCGCCCCGC 53280
TTTGCCCGAG ACGTTAGGTC GCGGAGATAT CCGCCGGGCG ATTTGATGTG CCCTGACCGC 53340
TCCAGAATGC AGGCCATGAC AGCGGATGCG TTCTCGGGCC CCATAACCTC ACAGGCATCC 53400
TGGTAGGCCG ACGGACTGAC GCAGAGCATC GAGCGGACGA CCACTGCGGC CTGCATCAGG 53460
TCACGCGGGC TCGCAACTAC GCCTCCGGGG CCATAATCTG AGATGCTTGG GCAAGCCCGC 53520
AACACCATCC CCAACGGAAA CACCTTCATC GGCTCGCGTT GCGGTTCGAT GGCTGGGCTC 53580
GGCCTTTCGC CCTGCTCTTC TCGAGAGCTA GGTTCAAATT CATTAGTGGA TTCGGGATGT 53640
GAATTCTGTT TGTGCCGCTC AATTTGATCA GGATTGCCGC TCAAATTTTC GATTTTTAAC 53700
CTGATTTCCA GAAGGTTGGA GATTTCCTCG CGCAACATCT CCATCTCTTC GAGGATGGGG 53760
GCGACGGTCG CGGCCGTTGC CACTCGAGGA ATGGTCGCTA CTAGGCTGCG GTAGTGCTGG 53820
TGAATTCCGT CCCAATTACC GTCCACTCCC TCCTCAATGG CGACTTCAAT CAGCTTACCG 53880
ATATCGCGCC GGCAGATCGT CAGGCGCTCG CGAAGTCTAC GCAATTGCAG TCGCTCAATT 53940
TTGACATAGG CAGCTATTTG CTCGATCTCG CGTGCGCGTG ACAGCAACGG AGCCAACGAA 54000
AACCCATAGG CCTCATCGAT CGAACCGTCT CCGTCCTTGC GGGCGTAGCG TTTGCCATTT 54060
GGGCTATCCC GGCGTGCCAG AAGGCCAGCG TCGACGAGCG CTGCGATGTG GCGACGCAAC 54120
GTCGCTTCAG CCATGCCATG CGCACGAAGC GACAACTGCA TGTTAGAGGG GAACACGATG 54180
AGGCCGTTTT CCTCAGACAA GGTCGTCTCG GGATAAAAGC TCAAAAGTGC GTTCATAACA 54240
GAGAGCGCAC GTTCGCTGAT GCCGAGCTTC GGCTTAGCTT CGCAGAGCGC TCGAAACAGC 54300
TTCCACTTGT CAGCCGATGT TTCAGGTTCG AGTTCACGCG ACATATATTG GCTTGCCAAC 54360
ATGCCAAGCG TCATCGACCG CCGCCCGAAG GGCGTCGTCA CACTTCCATC TTGCATCTTC 54420
TTTCACCTAT CCTCAGGCAA AAGACGTCCG CTCACCCGTT CCGGTGCCAA AGACTCTTGA 54480

CTGCGATTCG TGGAAATGCG ATTCTCAGGC TGCTAAACGA TGAGAGAGGC TTCCACGATG 54540
GTTACCGTCT GGGGGCCTTT TTCTTTTGCG TCAGTCTTCC TTCCGATTAC CCTGCTCCAG 54600
GAACTCGGCA TAGAGCCGGT CCAGGTTGTC TGCAATATAC CGCCCAAAGG GGCCGGCATT 54660
CCTTGATTTC ATAGACAAGG CAAACGCCTT GGCGGAGTTC GAATACTTAG CGGAGACGGT 54720
CTTGTCCGCC GGTTGCCAGT TCTCCAGGAT CTTTGGAGTC GTCTTCCGGA CAGGCTTCGC 54780
CGCGCTGTTC AGACCAGAAA ACAGGGAATT GAAGCGATCG TCAGTGCTCA GTTCATTGAA 54840
GGAAGGATCA GAGACGATTG CCTTCACCTT CGCTTCGTTG GCGGTTTTAC CCACAAGCAA 54900
TGACAACTCG ACCCAGCGTT CTCGGCCGAC CGCCGGGCAG GGCCCGATTC TTGCGATTGT 54960
CTCTTCAGGA ATACGATCTA TTACGGCGAT CATTTTCGAG ATAGCGGCCG CATTGGCAGC 55020
AAGCGCCGAT CCTATGATTG TCCGGTCATA GCCAAGCTTC TCGAGGTGCG AGGCGAAGTT 55080
CGCCCGTTCA ATGAAGGAGA GGTTGGCGCG CGCCGAGTTT TCCTGTCCCT GGGCGATAAC 55140
GTGCGTGCGG TCATCCAGCG CCTTGACGAC CGCCTTAACC TTGCGGCCGA GCTCGCGGGC 55200
AACGCGGGCT CGGCGGTGTC CAAAGACGAT CTGGTAACGA TCGGCTGCCG ACGGGTGGGG 55260
GCGCACAAGG ATGGGCGTAT CCTGCCCGCG CTCGGCAATC GCCTGCTTCA ATTCCTCGAA 55320
CTGCTCGGAA CTATCGCCCA TGCGATCAGA GACGAACGAA CCATCGAGTG TCTCAGGGTC 55380
GAGTTCGACG ACCGTTTCTC CTTCGAGAAA TTTCTCGGCC TGGCGCGACA ACTCGTCGAG 55440
CGAGCGCACC ATGCTCTTCG AAGCCCCGCG CATCGGGTAG GCGGGCGCAA CGTCTGCGTG 55500
CGGGATTTCC GCTGTGTCTA CCAGTCCCGC CAGTAGGTTC TTTCTCGCCA TGTGCCTGCT 55560
CCGTTTGCCT GAGATCTACC GGGCCGTCCC GGGGAAGTTT ACACTTCTCA ACTGACAACC 55620
TTCCTCCCCC AAGAGGCATG GACGAGTTCG ACGATCTCGG CGTTGACTGC GTTCAAGGAG 55680
TCCATCGCGC GCTCATAGGT GGACCGCGTC ATCTGACTCT TGTCGACTTC GTAAAGGGTC 55740
TGCTTGGTGA TTCCGGCGTC TGAAACCGCT GTCGATTTCA GCATCTGGTT CTTCAGCACA 55800
AACTGGTGGA ACAAGGTCTG CATGAAGCCC ACCATCTGCG CCTGCGGCCC ATCCGTTGGC 55860
TCGTACCGGG TGATCAGATA GCGATACCAT TCGAGATTGA CCGCAGCACC CACATCCCGG 55920
ATCGGCTTCA GGATCCCGCC CAGCATCAAA AGGAACTGGC CCATCGACAT GACGTCGAGC 55980
ATCTGTGGAT GGATCGTGAT CAGGACGCTC GTAGCCGCAG TCAGCGCGGT GATCGTGAGA 56040
TAGCCGAGCT GGGGAGGGCA GTCGATGACG ACAACGTCAT AGCGGTCGTT GACTTCCGAC 56100
AGCGCGCGCG AAATCCGCGT GAAGAAGGTC TTGCCATCGT TCGAGCTCTT GTTCGACATC 56160
GCAAGCGGGG TGTCGTACTC GTACTCCTGA AGTTCGAGGT TCGCAGGCAC GATATCGAGG 56220
CCCGGGAAAT TGGTAGGCTG GATGATCTCA CTGATCGATC GGCGTTGATC GTCATATCTG 56280
AGCGCCTCAT AGAGCGACGG CGACATGTCG AGTTCGGGCT GAAAACCGTG AAGCGACGAA 56340
AGTGACGCCT GCGGGTCGAG GTCCACCGCC AAGACACGGT GCCCGGTCAA CGCCATGTAC 56400
TGCGCGAGAT GGGCGGCCGT CGTCGTCTTT CCCGAACCGC CTTTGAAATT GACAACGGCA 56460
ATGACCTGGA GCTTTTCGCC CGACCGACGG TGCGGCACGT AGTTCCTGGC TTCCGATCGT 56520
CCATGCTGAT CGAGATACTG ACGCAGTTCC GCCATCTGCT CAGCACTATA AGAACGGCGA 56580
CCCGACGACG ATGTCTGGGG GAGGGGGCCT TTTCCCTCAA GATGAAGTTT CTTCAGCGTA 56640
CTTTGAGAGA CGCCGAGGAA CTGCGCCACT TCAGAGAGCG AGAACTGGCG AAGCAGTTTC 56700
TTGGCATTCG GTGGGAACTG CTGCATGCTT AAAAGATGCA ATTTCTTTGA AATCAGATCG 56760
CCCTGTTCGA GGATGAGATC CTCGAAATGC ATCGGCTGTG CGGGAGCGAT GGGCGGCGAA 56820
TTAGCGTTCA TTGCAGGGGA ACTCTCGAAT AACGGTTTTT CGGCCCGAGA ACCGACATAA 56880
TCGTTATTAT GTGCGATTCT CGCCGACTGA CAAGGAATAT AGAGTTAACA AAGGGTTTAT 56940
ACCGCGTCCC GAGCAGCCCC TGCTCGCCGG CAAAACATGC GTGTTTCTCC TTCATTCCTA 57000
GGCACTTAGA TGGACGCTTG CGCTCCGCCA GGCGCCAGCA CTTTTCCAGG GGAGAAATAC 57060
GGCCGTTCGC CTGACTACCA AACGTCACCG CGAAATAGCG GTCGTATTGC TCCCCGCGAC 57120
TCACCCGACT CAGCCTTGGT GAATATATTC GAGTTCTGAG TTGCTGCGGT TGCCCGGAAA 57180
GAGAGCCGGG GCACATAACT TTGCTGCCAA CTTCTTCATT GGGCCCAGCA ATTGGTTCTG 57240
CTCGACTTGC TTTCACCATC TGTCCCTCTC TGCGGCGTAC TGTCCGTTAC AGGCCATGCC 57300
CCGCAAAAAT AGAAGGTGAT TCTTCCTGAC CGTCCAGAAG ATTGGGATTG ACAATGGGTT 57360
GAAAAGACCC TGCTTGGCTC AAGTGAGCCG AAGGATTACT TTCGCAAATT TGTATTTGTC 57420
CGCTGAGGGT TAGACGCGGA TCACGGCCGT CTATGGACAC GAATTGTTCT CCTGTGAATC 57480
CTACAAGACG CAAGGGGGCC TTCGGGTCTG CAAAGGCAGA CGTGACGCGA CGCAACGCCC 57540
AACGCGTTCC AGGGAACCTT GTAGGATCCT ACAACTGCCA CCCGATTCCG ATTTGTGCTG 57600
ATTTCCCCGC GGTCCAACAA CGACGGAGAA ATTCATGCAG ATTCTCGCGA TCTCAAAGCC 57660
GCGAAATATC GAAGAAGCCC AACTTCTCCG CAGCCATCAC GAACTGCGCG CCCGCGTCTT 57720
TTCCGATCGT TTGGGCTGGG AAGTCAATGT CGTCGGAGGC TGTGAGTCAG ATACTTTCGA 57780
CGATCTTCAA CCGACCTACA TCCTAGCCGT ATCGAGCAAC GATCGAGTGG TTGGATGTGC 57840
TCGCCTACTG CCTGCGCTCG GCCCCACGAT GGTGGCCAAC GTTTTCCCCT CGCTCCTTTC 57900
TGCCGGTCAC CTCAACGCTC ATTCCTCTAT GGTCGAGAGC TCGCGCTTTT GCGTGGACAC 57960
CTTCCTTGCT GAGAGCAGGG GAGACGGCTC CATTCATGAG GCGACGCTCA CAATGTTCGC 58020
GGGGATCATC GAATGGTCGG TGGCTAACCG CTACACCGAA ATCGTCACGG TGACGGATCT 58080
TCGGTTCGAA CGGATCCTCG CCCGCGTCGG TTGGCCGCTT CAGCGCATTG GCGAGCCAAG 58140
GCCGATCGGA GCGACCGTGG CCGTGGCGGG CACTCTGCCG GCAAAGGCAG ACACATTCAT 58200
GAGGCTCCGC CCCGCCAACT ACCGCTCTCA AATCATCAGC ACTTTTGGCC AATCAGCGTA 58260
AGGAGAAATC AGTGGAACAG CTTCGCTCTC ATCCTCGCCT CGTCCGCAAG CTTCAGGAGG 58320
CGCTTGGCGA CCAGCTCTGC GTTGCTTTGG ACGATAGCAA CGTAGTAGAG ATAATGCTGA 58380
ATCCCGATGG TAAGCTGTTC ATAGAACGGC TCGGGCACGG CGTCGCGCCC GCTGGCGAGA 58440
TGTCGTCGGC TGCGGCGGAA ATGGTGATTG GGACGGTTGC TCATGCCCTC CAATCCGAAG 58500
TGGACACCGA ACAACCCATC ATTTCCGGTG AGCTTCCGAT AGGCGGACAT CGTTTCGAGG 58560
GTCTATTGCC GCCAGTGGTC GCGAAGCCCT CCTTCACAAT CCGTCGTCGG GCATCTCGCC 58620

TGATCCCACT CGACGACTAT GTCCGCTCCG GTGTGATGAC CGAGGCCCAG GCTGCGACGA 58680
TCCGCAGCGC CATTGACTCA CGCCTCAACA TCATCATTTC CGGTGGAACT GCCTCCGGCA 58740
AAACGACGCT CGCAAATGCA GTAATCCATG AGATCGTGAG GAGCGCGCCG GAAGACCGGC 58800
TCGTCATCCT CGAAGACACC GCCGAAATCC AGTGTGCGGC CGACAACGCC GTTCTTCTTC 58860
GTACCAGCGA TACGGTTGAC ATGGCGCGGC TGCTCAAGAG CACGATGCGC CTGCGTCCCG 58920
ACCGAATAGT CGTCGGCGAG GTGCGCGATG GTGCTGCCCT CACCTTGCTC AAGGCATGGA 58980
ACACCGGACA TCCAGGCGGC GTGGCAACAA TCCACTCGAA CACGGCGACT TCCGCGCTCC 59040
GGCGCCTGGA GCAGCTGACG GCCGAAGCAA GCCAACAGCC GATGCACGAG GTGATCGGGG 59100
AGGTTGTCGA CCTCATCGTT TCCATCGAGC GTACGCCGCG AGGCCGGCGT GTGCGCGACA 59160
TCATCCAGGT CGAGCGCTTC GCCAACGGCC GATACGAGAT CGAATCTGAC CAACTCACCG 59220
AAGAACGGGA GGAGCGGCAT GTCGCGTAAG AATGAATTTA TCTCCATCGC GCTTCTTGCT 59280
TCGCCACTCA TTTTGGCGTC GGTAGCACCG GCACTGGCCA GTTCAGGCGG CAGTCTGCCC 59340
TGGGAAGGAC CGCTCGAGCA GATCCAGGAG TCCATCACGG GGCCAGTCGC GGGCTACATA 59400
GCACTTGCGG CAGTCGCGAT CGCCGGCGGC ATGCTGATTT TCGGCGGTGA GCTGAACGAT 59460
TTCGCGCGGC GGCTGATGTA TGTCGTGCTT GTTGCCGGCA TCCTCCTCGG CGCCACGACC 59520
ATTGTCGGCC TGTTCGGCGC GACCGGCGCT TCGATCGGCC TGACCAATGA TCGCGCCACT 59580
TCGACGCGCC CGAGTGCAGA AGGGGAGGGG GCACATGGCT GAGGCGCTAT CCGAACGTTA 59640
CCGCAATCGC ATTCACCGTG CGCTCTCCCG GCCGAACCTC CTGATGGGCG CGGACCGGGA 59700
ACTGGTTTTG ATCACCGGGC TTGCCGCGGT CATCCTGATC TTTGTGGTGC TGACTGTCTA 59760
CTCGGCGCTC TTCGGGGTTG TCGTGTGGAT CGTGATCGTC GGCTTGCTCC GCATGATGGC 59820
GAAGTCCGAT CCGCTGATGC GGCAGGTCTA TGTCCGGCAC ATTTCCTACA AACCCTACTA 59880
CAAGGCGACC ACCTCTCCGT GGCGGCGGTA TTGAGGAGGC TGATATGGTC GCGCTCAAAC 59940
GGTTTCGGGC AACCGGCCCA TCCTTCGCGG ATCTCGTCCC CTATGCCGGC CTGGTCGACA 60000
ACGGCGTGCT TCTGTTGAAA GACGGAAGCC TGATGGCCGG CTGGTATTTT GCCGGGCCGG 60060
ACTCCGACAG CGCCACAGAC TTCGAACGCA ACGAACTATC GCGCCAGATC AACGCAATCC 60120
TGTCGCGGCT CGGAACCGGC TGGATGATTC AAGTCGAAGC CGCCCGTATC CCGACGTACG 60180
ATTATCCCTC GGAAGACCGC TGCCATTTCC CCGATGCTGT GACGCGCGCA ATTGACGCTG 60240
AGCGACGGGC GCATTTCGCG CGCGAGCGAG GACATTTTGA AAGCAAGCAT GCGCTGATCC 60300
TGACCTATCG GCCGTCGGAG GCCAAAAAGA CAGCCGCTCA GCAAATACAT CTACTCGGAC 60360
GGGGAGAGCC GTAGGAAGAC CTATGCGGAC ACTGTGCTCT TCATCTTCAA GAACGCCATC 60420
CGCGAGATCG AGCAGTATTT CGCCAACACC CTTTCGATCC GGCGCATGGA AACGCGCGAG 60480
GCCGCCGAGA GGGGAGGGGA GCGTGTCGCC CGCTATGATG AACTGCTGCA ATTTATCCGA 60540
TTCTGCATCA CCGGCGAAAA CCATCCGATC CGATTGCCAG CTGCTCCCAT GTATCTTGAC 60600
TGGATCGCGA CCGCCGAACT CGAGCATGGT CTGACGCCGA AGGTCGAGAA CCGGTTCCTC 60660
GGTGTCGTGG CGATCGACGG GCTCCCCGCC GAGAGCTGGC CGGGCATCCT CAACAGCCTC 60720
GACCTTATGC CTTTGACGTA TCGCTGGTCG TCGCGCTTCA TCTTCCTTGA TGCGGAAGAG 60780
GCGCGTCAGA AGCTCGAACG CACGCGCAAG AAGTGGCAGC AGAAGGTCCG GCCGTTCTTC 60840
GACCAGCTAT TTCAGACGCA AAGCCGGTCG GTCGACCGGG ACGCCATGAC AATGGTGGCC 60900
GAAACCGAGG ATGCCATCGC GCAAGCGTCT TCGCAGCTTG TCGCCTATGG CTATTACACA 60960
CCCGTGATCA TTCTCTTCGA CAGTGATCGC GAAGCGCTGC AGGAAAAAGC CGAGGCTATC 61020
CGCCGGCTGA TCCAGGCCGA AGGATTTGGG GCGAGGATCG AAACACTCAA CGCGACCGAG 61080
GCCTATCTCG GGAGCTTGCC CGGCAACTGG TATTGCAATA TCCGCGAACC GCTGATCAAC 61140
ACCAGCAATC TCGCCGATCT CATTCCCCTG AACTCCGTGT GGTCGGGAAG CCCGGTCGCA 61200
CCTTGCCCTT TCTATCCTCC CAATTCTCCG TCGCTGATGC AGGTTGCAAG CGGTTCGACA 61260
CCGTTCCGGC TGAACCTGCA TGTCGACGAC GTCGGCCACA CACTGATCTT TGGTCCGACG 61320
GGATCCGGCA AATCCACGCT GCTGGCGCTG ATCGCGGCAC AGTTCCGACG CTATGATCGT 61380
TCCCAGATCT TCGCCTTTGA CAAAGGAAGC GCGCTCCTAC CGCTAACGCT GGCTGCCGGG 61440
GGCGATCATT ACGAGATCGG CGGCGACAAT GCGGAAGGGC GTAAGGCACT GGCCTTTTGT 61500
CCGCTGTCGG ATCTCGAAAG TGACGCCGAT CGGGCTTGGG CCGCGGAGTG GATCGAGATG 61560
CTGGTCGCTC TGCAGGGTGT CACTATCACG CCCGATCATC GCAATGCCAT GTCCCGCCAG 61620
GTCACCCTAA TGGCCAGCGC TCCCGGACGA TCGCTCTCCG ATTTCGTGAG CGGCGTGCAG 61680
ATGCGCGAGA TCAAGGACGC CCTGCATCAC TATACAGTCG ACGGGCCCAT GGGTCAGCTT 61740
CTCGACGCCG AGCACGACGG ACTATCGCTC GGCGCCTTTC AGACCTTCGA GATCGAGCAA 61800
CTCATGAACA TGGGCGAGCG CAATCTCGTC CCCGTGCTCA CCTATCTGTT CCGTCGCATT 61860
GAGAAACTGC TCGACGGCTC GCCGAGTGTG ATCGTGCTGG ACGAGGCTTG GTTGATGCTC 61920
GGCCACCCAG TATTCAGAGC CAAGATCCGC GAATGGCTGA AGGTGCTACG GAAAGCCAAT 61980
TGCGCCGTCG TCCTCGCGAC ACAATCAATC TCCGACGCGG AGCGTTCCGG CATCATCGAC 62040
GTGCTGAAGG AATCCTGCCC GACGAAGATC TGCCTTCCGA ATGGAGCCGC TCGCGAACCC 62100
GGGACGCGCG AGTTCTACGA ACGGATCGGC TTCAACGAGC GGCAGATCGA GATCATTTCG 62160
AACGCCACTC CGAAGCGCGA ATACTATGTC GTCACCCCTG AAGGCCGGCG GCTTTTTGAT 62220
ATGGCGCTCG GTCCGGTGGC GCTTAGTTTC GTTGGCGCAT CCGGCAAGGA AGATTTGAAT 62280
CGCATCCGAA CACTTCATTC CGAATACGAC CGCGACTGGC CGGTCCACTG GCTTCAGATG 62340
AGAGGATTTC ACGATGCCGC GTCGCTGTTC AATGTCGAAT AATTTGCTCG CCGGTCTGGC 62400
GGTCGTCGCC GTAACGGTCG GCACAGGGGA GCCGGCCGAT GCCGGCACCG CCACTGGCGT 62460
CGCAACCGAG TGGACGCAGG TTCTCAACAA TGGGGAGCTG GTTGCCCTGG TGGGGAAGTC 62520
CAATGAGCAA ATTCAGAACC AGCTCACCCA GATCAGCCAG TTCGCACAAC AGATCGAGAC 62580
GCAGCTGAAC ATCTATCAGA ACCTGCTCCA GAACACGGCG ACGCTTCCGT CTCATATGTG 62640
GGGGCAGGTC GAGCGTGATC TCAATCGGCT CCGCAGCATC GTCGATCAAG GTCAGAGCAT 62700
CGCCTTTTCC ATGGGCAACG CCGACCACGT ACTGCAGCAG CGATTCCAGA GCTATGCAAC 62760

CCTCAAGACC AACCTGCCAA GGAATGAGAC ATTCTCCTCG ACTTATCAAG CCTGGTCGGA 62820
CACCAACCGC GATACGATTG CCAGCACGCT GAATGCGGCG AGCCTCACGG CCGATCAGTT 62880
CGACAGCGAG GAAACGACAA TGTCCTCGCT GCGCTCGATG TCAGAGACCG CTGACGGGCA 62940
GATGAAGGCT CTGCAGGTGG GCCACGAAAT CGCGGCCCAA CAGGTCGGGC AGATGCAGAA 63000
GCTCCGCGGC CTTGTCTCGC AGCAGATGAC CATGATGGGA ACCTGGCTCC AGACGGAGCA 63060
GACCGACAAG GACCTGGCGC AAGCACGGCG GGAAAAATTC TTCAACGCCG ACGTCAAGAG 63120
CATTCCGGAA GGTCAGAAAA TGGAGCCACG CTGGTGAGCC GCGCCGTTAT CATTGCCTTA 63180
GTGATCCTCG TCGCTGCGGT TTCGACCACA GCGACAGCGC TGATCGTCAA CTCCAGAGCC 63240
TCCAACCCAT CCGCACCTGA GGAGCAACGC ACCGCCAAAG AAAAATTCTT CGGCGCGGGG 63300
AAGGCGCTTC CGCCGATCAA GGATGGTCAG GAGATGGGTC CAAGATGGTG AAGATCAGTC 63360
TTGCACGTTC GTTGCTGATT GCAGGCTTGG CTTATGTCGC CTTCGAGGCA CGCGCTTTCG 63420
CGCAGGAAGG TCTAGTCCTC ACGGAGTTGG AGAACCACGT CTCGGCAGCC GCGAAAGGAT 63480
GGGAAACCAC CATCCTTGAC GCGGCGAAAT CGTTGTTTTG GATTTTGGCC ACCATCGAGA 63540
TCGGCATCGC GGCCGTCTGG CTGGCGCTCC AGGCGGCTTC GCTCGACAGC TGGTTTGCTG 63600
AGCTTGTCCG GCGGATCATG TTCGTCGGAT TTTTTGCTTT CGTTCTGACG CAGGGCCCGA 63660
CCTTCGCGCG CGCCGTGATC GATAGCCTGT TCCAGATCGG CGCTGGCGGC GGATCGGCAT 63720
CCCCCGCTGA AATCTTCGAC GCTGGCATTC GGGTCGCCTC ACAAATGTCG CAGCAAGCCC 63780
AGTTCGGCGT CTTCGAGGAC AATGCGCTCG CAATCGCTGC GGTGCTGGCG ATGGGTGTCG 63840
TGGTCATCTG CTTTTCGCTG GTTGCAGCAA TTTTCGTGTC GGTCATGGTC GAGATGTATG 63900
TCGGCCTGCT CGCTGGCATG ATCATGCTTG GGCTTGGCGG TTCCTCTTTC ACGAAAGACT 63960
TCGCTGTCCG ATATCTTGTC TATGCCTTCG GCGTCGGCAT GAAACTGATG GCGCTCGTGA 64020
TGATAGCTAA GATTGGATCG CAGGTCCTCT TGGGCCTGGC GAATGCTCCA ACCGCGTCTT 64080
CCGACCAATT TGTCACGACC CTGGCGATCG CCGGCATCTC CGTCGTGGTT TTCATCATCG 64140
CCATGTACGT GCCGAGCATC ATCCAAGGTG TAGTGCAAGG CGCCTCGGTT TCCGGGGGGA 64200
TGGAAACGAT CCGTCACGGC GGCCAGGCGG CATCCTTCGC CGCCGGCGCT GGGTTCCTCG 64260
CGGGCGGCGC CGCGAGAACG GGTTTCACGG CCGGTCAGTC GGCACGAGCG GCGGGTTCAT 64320
CCCTCGCCGG CGCGGCGCTT CGGGGTTTCG GCGCGGGGAT CGGATCCGCC AGCAGTGCTG 64380
CTGGTTCTGC CGCCAAGGAA AAGGCGATCG GTTCACCCGG CGCCTATGCC GGATCAATCC 64440
TCGGCCTCGC CAACGCCAAG CTCGATGAGA GCCGGGGCGG TCATAGCGGG CACAAGCCAC 64500
CTCCCGAACG CAAAGACTAA CAGCAATCAG AAAGTGATAG ACGATGGCAG CGAACCGCGC 64560
CCCCGAAAAC CCCTATCTTG CCGCGCGGCA GGAATGGACG GAACGATACG GATCCTATGT 64620
GCGAGCCGCC GCGGCATGGC GCACAGTCGG CATTCTGGGC CTGGCCATGG CCGTGATCGG 64680
TTTCGGATAT GCTATGTATC TCAGCACCGA GGTCAAGCTC GTGCCTTACA TCGTCCAGGT 64740
CGACAAGCTT GGCACGTCGG TCACGACCGG TTTCCCCGAA CAGATTGAAT ATGCGGATGT 64800
GCGGGTAGTG CGCGCCACGC TCGGCAATTT CGTGACCAGC TTCCGTTCGA TCACGCCGGA 64860
CGCCGCGGTG CAGAAACAAT ATATCGACCG CACCTACGTG CTCCTTCGGA CCTCCGATCC 64920
GTCGACGGAG AAGATCAACG CCTGGTTCCG AGGCAATTCG CCATTCGAGA AGGCAAAAAC 64980
CGCAACGGTC GCGATCGAAG TCAACAACAT CGTGGCGCTC TCAAACCAGA CCTATCAGAT 65040
CGACTGGACC GAATACGAAC GGGACCGCAA GGGCAAGGAG ATCGGCACGC GCCGGTTCCG 65100
GGGCATCGCG ACGGTGACAC TCACCGCGCC GCAGGACGAG GCAACCATCC GCCTCAATCC 65160
AATCGGCCTT TATGTCAGGG ATTTTGACTG GACAGCGCAG CTTTAAGGGC AGGGGAATTC 65220
TCAATGCACA GAACCGGATT GATCGCAGCC GCCGGCTGCC TGGCAACACT CGTCTTTGCG 65280
AGCGCCGCTG AAGCGCAGAG CATGACCACC AATGAGGTGA AGGGCACCAG TATTTCGAGG 65340
AAATGGCGCG GCACGGCCGG GTTGGTGACG ACTGGTCCCG ACGGCAAGGT CATCTTCCTG 65400
TTCGGCGAGA CGCAGCCGTC GGTCGTCTGC TCGCCCTTGC AGGTCTGCGA TATCGAGTTG 65460
CAGGGCGGCG AAATCGTGCG TGACGTTCTC GTTGGCGATA GCGTGCGCTG GAAGGTCGAA 65520
CCGGCGACCT CGGGTGCCAC CGGCGGGCAG GCGATCCACC TGATCGTCAA GCCGTCGGAG 65580
CCAGGCCTTG TCACCTCGAT GGTGGTCACG ACGTCGCGCC GCACCTATCA CATCCAGCTG 65640
AAATCCCATC CGAGCCAGTA TATGGCGCGC GTCGGTTTCG AGTATCCGGA GGACGTTTCG 65700
ACGAAGCTTG CTGACATCAA TTCAAGGCTC GAGACCGGCG GCATTCCAGG AACGGCACCC 65760
GACAAGCTGA ACTTCTCCTA CTCGGTCAGC GGGAGCGCGC CGTGGAAGCC GAAACGGGTC 65820
TATTCGGACG GCGCAAAAAC CTACGTCCAA TTCTCGAAAT CGATCTCCGG ACAGGACGCG 65880
CCGGTGCTTT TCGTCGTCAG CGGCGGCCAA AACCGCATCG TCAACTACCG CATGAACAAT 65940
GACATGATGA TCGTGGACTA CGCGGTCGAC AAGGCGATCC TCGTCTCCGG CGTCGGCTGG 66000
CGGCAGCAGA AGATCACTAT TCGGCGGGGA GGCTGAACGA TGCGGAAGCT TCTGATCTCC 66060
TTTGTTACGG TGGCTATTCT TTCCGGCTGC CAAACGGCCG AGGACGGGCT GACGACCAGT 66120
TCCACGCCAA CCACCGTCAC CGGGCCCGCG GCGAGCGCGA TTGCCGGCGA TATGGCAAGC 66180
CGGCTCGCCG AGCAGATCGG CCCGTCTGCG ACGATGACCA TCAAGATCGA AAAAGACTCG 66240
TCGGATTTCG CGAGCGCGCT CGAGGCGGCG CTGAAAGGCT GGGGCTATAC GGTCGTGACT 66300
GATGGCAAGG TCGGCAGGGT CGGCAAGGAC GTGAAACTGG TCGAGCTCGG CTATTCGATC 66360
GACGGTGTTG ACGGTCAGGT GCTCGCGCGA CTTTCGACGC CCTCTATCGC GCTTGGCCGG 66420
GCCTACACGG CAACGGCAGC GGGTGCTGTG CCGGCAAGTC CGCTTTCCAT CATGCAGCGC 66480
AACTGACGGA GACAGATATG GTCCAGTCTC TGCGGCTTGG TGCCGCCAAC GAGGCCGAAG 66540
ATCAGAACGG CATGCGCCGT CTCAATCGCC TGCCGATCAT CGTCGCCATC GTCATCGTTG 66600
CGCTGTTCGT CGGCTTCGTC GTCATCGGAC TGGCATGGCG CGGTCTTCCG TTCAACCGCA 66660
ACAATGATAT CCACAGCGCA TCGAATACCC CGGCAACGAA TTTCGGCGAC CAGCTCAAGC 66720
GCGGCGTCAC CGACGGTATC ATCGGCGAGC CGGTGAAACG GGAAGCGTTT CAGCCGACGC 66780
CCACCATGAA ACAGAAGGTC GACAAGGAAC CGACTGTCGT CGACCGGCCT ACGGAACCGG 66840
AAGAGCGACG CCCGCGGCTC GAAACCGAGG AAGAATGGAA GGCGCGCCTG ATGCGCGAGC 66900

AGGACGAACA AATTATCCGC GAGGCCCAGC GGCAGCGGAT GGCCCGGCTG CAGGCACGAG 66960
CAACGGCTTT GGACTCGCCG TTGAAGGTGG ATATTTCCGA AGGCGAGAAA GCACCCAAGA 67020
ACTCCACGGA CACGGGTCAA AATCCCACGG CGACAGAAAA CAATGCCTCG GACCTCTACC 67080
CCGCCGCCAT GAAATCGGGG ATCATGGGCG AGAACCTCGA TCCGAACGCC CAGGCGTCGA 67140
AGGAGGATTT CTTCAACCAG GACATCAAGG ATGATGGCTA CCTCCCAAAT CGGGTCGTGC 67200
CGCAGATGTC GGTCTATGAG TTGAAGCGCG GCTCGGTCAT TCCCGCCACC TTGATCACCG 67260
GCCTCAATTC CGACCTCCCG GGTCGCATCA CAGCGCAGGT CAGCCAGAAT GTCTATGACA 67320
GCGCCACCGG CTACCGGTTG CTTATCCCGC AGGGCGCCAA ACTGTTCGGG CGCTATGACT 67380
CAAAGGTCTC GTTTGGCCAG GAGCGCGTTC TCGTCGTCTG GACCGATCTC ATTTTCCCAA 67440
ATGGATCGAC CCTGCAGATT GGCGGTATGG CTGGCACGGA CGCCGAGGGC TATGGTGGCT 67500
TCAAGGACAG GGTCGACCGG CATCTCTGGC GCACTTGGAG TTCGGCAGCG CTGATCGCGC 67560
TGATTGGGAC CGGAATCGAC ATGTCGATGC CCGAGAGCTC GACGCTGGCG ACACAGGATA 67620
CTGCCTCAGA CGCCGTACGC CGAAACTTCG CTGAATCGTT CGGCCGGGTG GCTGAGCAGA 67680
CCATATCGAA GAACCTGAAC GTCCAGCCAA CAATCCGCAT CCGACCCGGC TATAAGTTCA 67740
ATGTCCTCGT TGACCAGGAC ATCATCTTTC CGTCCGCCTA TCGTGACAAT TAGCGGCGCA 67800
GGGCCCACAG ATCTGATCTC CGTATCTGCC GCTGGTTGGC GCGCGGTGTG GCGCTACAAT 67860
CACTGCAGCG CAAAACCTTT CCTCACTTGT ACTATTTCTA GGCCGACGTG GCTTGAAAGC 67920
TGCATCTCCA CAGGTAGTGC TAATGTACAC GGTCCGAGGG AATACTCCGC ATGCTCTATT 67980
AACCTGCGGT TGCCGCAAAA AGTAATTGTG TGCGTAAATC GCAGTCATGA CCCGATTGCC 68040
CAAACGGCCG CTCGTTTCGC AAAGGACGAG CTCGGCGGTC AAGAAGATAG GAGAGATGTC 68100
CGTGAACGGA AACCTTCGCT CGCTCATCGA TATGCTGGAA GCCGCGCAAG ATGGGCACAT 68160
GATCAAGATT GCTCTTAGAA GCTTCGCGCA TTCCTGCGGG TATGACAGGT TCGCCTACCT 68220
GCAAAAAGAC GGCACCCAGG TTAGGACGTT CCATTCCTAT CCCGGACCAT GGGAAAGCAT 68280
CTACCTCGGT AGCGACTATT TCAACATCGA TCCCGTGCTG GCGGAAGCAA AGCGCCGGCG 68340
GGACGTTTTT TTCTGGACCG CCGACGCTTG GCCTGCCCGA GGATCGTCTC CTCTTCGGCG 68400
GTTTCGTGAC GAGGCAATCA GCCACGGCAT TCGCTGCGGC GTCACCATTC CCGTGGAGGG 68460
AAGCTACGGA TCCGCGATGA TGCTGACCTT CGCATCGCCG GAAAGGAAAG TGGACATTTC 68520
GGGCGTGCTC GATCCCAAAA AGGCAGTTCA ATTGCTGATG ATGGTGCACT ACCAATTGAA 68580
GATCATTGCG GCGAAAACGG TGCTTAATCC CAAGCAAATG CTGTCGCCGC GCGAGATGCT 68640
TTGCCTGGTG TGGGCTTCGA AGGGAAAAAC TGCGTCCGTA ACCGCCAACC TCACTGGAAT 68700
CAATGCCAGA ACCGTGCAGC ACTATCTCGA TAAAGCACGT GCAAAGCTTG ACGCGGAATC 68760
AGTGCCGCAA CTTGTTGCAA TTGCCAAGGA TCGCGGCCTG GTCTGAGTTC TAGTTGTCAT 68820
CGTCGAAAGG CACGTCGGGC ACAAAGCCAA GCGCGTCCAG AATGTCCGAT AGCTCCTCCT 68880
GTTGGGCCTC CGACCTCTTC TGGCGTGCAA CGTACTCATC CTGGAGCGTC TGGAGGACCG 68940
GACCGGAAAT CGACGGATCG TCGCTTGCGC GCAACCACTC CTCGTAAACG GCCTGATCGG 69000
CCCAGAGGAG CCGGCGGTGT TCGCGAATCG CGGAAACTGC CAGTGCCTCA AGCTCTGACT 69060
TGGTCATGAC ACTGTAGCGC GCTTCTTTCT CCTTGTTTTC GTCGTTCACC TCGGATGAGC 69120
CCATATCGTT CATCCGCTTC TCCCTGATTT GATTTCCTCA CTGCCCAGCA CCCAAGGTCG 69180
GCATTGTATC GGTCTAGCCG CTACAGCCAA ATTCTCTGGG CTGAGGCAAG GCAGTTGAAT 69240
CTAAACTCGA CATTTAAACA ACTGCTTCGA GCCGACGGGC GTTCCGAGCC GCCGCGTTTA 69300
CATACTTTAG TTGATAAACT TATGGTGTGT AAACGACGAT CTTTCAGCGC ATGGATATGC 69360
GCAAGCTGGT CGGCTCTAAT TTCGCCCGCC TGCGTCGGGA GAAGGGCCTG ACACAGGAAG 69420
AGGTCGAAGC TCGTTCCGGT TTCAGCCAAC AATATCTGAG CAGCCTTGAG CGCGGGCGAC 69480
GCAACCCTAC CGTGATTACT CTTTATGAGC TGGCTCAAGC CTTGGGTGTA AGTCACGTCG 69540
AGTTGGTTAC CCCGACTGAT GAGACTCCGT TTGGCACGCG ATGAGGCGAA TAATCCTACT 69600
GCAGGGTAAA ATCGAGCGTA TGCGCCGCTT ACATCCCATT GATTTCGCGT GTTTGTGAAC 69660
ATTGTGATTC TGCCAGAAGG TTTTCGGCAA GAGCTGGAGA TTTGGGATGG CGGATGGTGA 69720
TGGTTGCAAG GCGTCCCATG CGAAGAGCCG TTTTGATGCG GCTGTTCTCA CGCAGGGTCA 69780
GTTCCTCCGA TAGAAGGATG TCGATTGCTT CCAGGGCTGA GAGTTCGCCA TGCTCGAGGC 69840
GGCGGACCAC GTGGTCGAGC GCCTCGAGAG CACGCGGCAT CTTGAGGCCG ACGAGATCGT 69900
GACGGATGCG TTCGATCATC GACGGCATAG CATCAAGGGC GGCGCTCATC GGCGGGTCTC 69960
CCGAGCCATA GCCGTTGCAA CAGCGTCATG ACCATGCCGT CACGCGAGAT TCGCCGCTCC 70020
AGCTTCAGGA CCGATCGGAA CAGGGCCAGG GGAAGTGGCC ATAGATGAGA TCGCTCTTCG 70080
GCGAAAGCCT CATTGACGAC GCGCAAGGTC GTGGCATGCA CCCTGGGATT GGCGACGGTA 70140
TCCAGCCAAT GGCGCAGCTG CGCGTTCAGG TCATCAAGGT TGCGGAACGA GCGAGCCAGG 70200
AAGAAGTCTT CGCGTATGTA GCGGAATGGC CTCTCGACCT TGGCTTTCTT TCAAGTCCAG 70260
GACTGCAGTT CGTACTGACA CTCAAGCGCG TATAGAATTC GAGGTCACGC ACCCGTTCCA 70320
TCGGTGGCGC GGCCAGCGCT TCGTGTTGTC GACACGCAAG CAGAGCTGGG GCGAGGACCG 70380
CGTCAACGAT ACCTGCAGGG TCTGCGCAGG TTGATCTGCT GCGCGCACTG ATGGGTGGCT 70440
ATAGGAGCTG ATGGGTAACT GTGAGTCCTA AGACTGCAAG TTGATATATA AGTCCATATA 70500
AGTCAGCGTT TTGATACGGT TTTGTATATC GACATTGCAT TGCTCTCTAA CTCCCTATAT 70560
ATTATCCTGT ATGGTTCGCA TCCTGCGATA CAGGATAATG ATATGGCATA TAAAACGGAG 70620
CATATAACGC AGCAGCTGCG CGCTGCGCGC GAAGCGCAGA AAATGAGCCA GCGCGAGCTG 70680
AGCGCACGAT CGGGCCTGAC GCAAAGCCAT ATTTCTCAGA TAGAACGGGG CACCATGGAG 70740
CCCGGACTTG GCAGCCTAGT GGATGTGGCG CGCGCGCTCG ACCTCGAGAT AGTCCTTGCC 70800
CCGAAAAAAC TGATGCCAGC AATCCGCAAT ATCCTCGATA GTGCATCGGC ATCGAGTGAT 70860
GTCCTGACAT CCGACCAGCG CAAACTGGTG GGCGCCCTTG AGCGGTGGTT TGCGCAGCAC 70920
AGGGGCGGGT TCGGTAGTGC TTCCGAGGCA GATACATTCA AAGATAGCCT CGCGCTCTTA 70980
CGGCATCTTC CTCTGTCGGC CGAGGAGATG GACACATTCA ACGAGGCGAC TGCACGCCTC 71040

GACCGGTCGC AGGCTGATCC TCCATCCCGT CAGGAACTGA GCAGGATCGC ACATGCCGTA 71100
AGACACCTAC GCAACGCCGC GGTGCACCGC GACCGTGACG ACGCGGTCCC TCGCTCGGCC 71160
TATGCGCTGG ATGAGGAAGA CGATAATGCC TAAGGTCACG GTACTCGATG TCAGGCTGTA 71220
TGGGGAACCG GTTTCGACCC TGACGAATCT GCAGGACGGG CGCACCATCT TCGCGTTCAA 71280
CGAGGCTTAT ATCGAAGACG AAAAGCGGCC GACGCTTAGC CTGTCCCTGA AAGACCCGTT 71340
CGGGGCGCTC ATTACGAAGT TCAGGCCGTA CAACATGGTG GTCCCGCCCT TCTTCTCGAA 71400
CCTACTGCCA GAAGGACCGC TGCGCAAATA CCTCGCTGAT CGTGCGGGTG TGAAGCCGTC 71460
GCGTGAATTC CTCCTGCTCT GGATGCTCGG CCGCGACCTG CCTGGGGCTG TGACGGTGCA 71520
TCCCTCCGAA GGGGATGACG CGCCTCCGGA TGACGAGCAG GCTATCGTCG AAGCGCGGCC 71580
GAACGCGCTA CGCTTTTCGC TGGCAGGTGT GCAGCTGAAG TTCTCGGCCT TTAAGAACGA 71640
CAAAAAGGGC GGCGGCTTGA CCATTCCGGC CGAGGGCACG GGTGGCTCGT GGATCGTGAA 71700
GCTGCCGTCT CAGCAATATA GCGGCGTGCC CGAGAATGAG TTCGCGATGA TGACCATCGC 71760
CCGCATGATG GGTATGGACG TGCCTGAGCT CCAGCTGGTT GATCTCGACG CCGTAAGCGG 71820
CCTGCCGCAG GGGGTGGGTG AACTGCACGG GCAGGCGCTG GCGATCAAGC GTTTTGACCG 71880
CACGCCGGAG GGAGCGGTGC ATATCGAGGA CTTTGCGCAA GTCTTCGGCG TCTTTCCCGA 71940
CCACAAATAC GATAAGGGCA ATTACCGGAT GATCGGCCGC GTGCTGGGGA TCGAAACCAG 72000
CACGGCCGAC GTGGCGGAAT TCATCCGGCG TCTAGTCTTC AGCACGCTAA TCGGCAATGG 72060
CGACATGCAC CTGAAGAACT GGTCGCTCAT CTATCCTGAT CGCCGCACCC CTGCGCTCTC 72120
GCCAGCGTAT GACTTGTTGT CGACTATCCC TTATATCGAG GGGGAAGATA CGGCGGCGCT 72180
CAATTTCTCC CGCACAAAAA AGATGGCGGC GCTGTCCAAG GACGAGCTGG CGCATCTGGC 72240
TGCGAAAGCG GAGCTTTCCG AAAAGCTGGT GATCGACACG GCGCGCGAGA CGGTTGAACG 72300
TTTTAGGGCG GTATGGGAGG CGGAAAAGAA AAATCTGCCG ATGGCCGCCA AGGTCGCCGA 72360
CACGATCGAT ACGCACGCAC CGACGGTCGA GCTGTATCGT GAGTTCGCGA AGTAGTGCCT 72420
TGCGGCTCGG CATTCAAATT ATCGAGCCTT GCTGTATATT CAAGGCGTTA GCCCATCCAA 72480
AGCATGGCCG TTGGTTCGAA TCCCATCAAG ACCCACAAGG CGGAAATGGC ACATCGGCCG 72540
CATAAGTCCA AAAACTCCGG CCGACGAAGT CCAGGTCGAG TAGCCAGCCT CTTTCCTGCG 72600
ACATGACGCT GACATCTTGA AGGCCCACCA GATGAAAGTC GCTCCGAAAA CCGGGGCGTC 72660
CGGTTACAGT TCTTTGGACC CGACGACGGA GGTCATGATG CGGGGATCCT TTAACGACCT 72720
GGTCTATGCG CAAAATGGCC GCACTCTCGT GAAGGAAATC TGTTCCGTGA GCAAGCGATC 72780
GTCTTCGTGG AGCGAAAGCG ACTCGACGCG GAGGATCTCG TCGCCCGCAT CACTTGGCAG 72840
GCGTTAAGCA AATGCCTTGT TGGAGAGATG TATGTGAATG CGGCGCGCGA GACGTTTTTC 72900
CGCACGGCGA AAAACAAGGG CCTCCTTGCC GAACCGGAGA TGTCATTGAG CTCAGTTGGC 72960
CGGTTGGCTA AGTGACTGAG CTTCCGCTTC CAGGAACGCG GACGGCACGT GCCTTGGAGC 73020
GGGCTCATCC ACGCCTGGTC GATCAAGATC GTTTACGACA GCACTGGAGC GGCGGCTTCG 73080
TTGCCTTTGA CGGTGTGCAA GCCGACCAAG GTCAATGTGG CAAGTGGATG AATCAGCCAT 73140
TTGGAGCCGA AGGTAATAGC CGCACCGTTG CCTTGTGTTC GAGATGATTG AGGACAGACA 73200
TCAGGGCAAG GGCCATGCCC ACAACAACGA CGCCGATCGC CGCGCCGAGC GGCCAATCGA 73260
ATGAGACGCC CATCAAGTTG ATGACCACGC CGGCGATCAT GACGCCCTCG GCACCGCCGA 73320
CCAATGTAGC AGCAATAAAA TCACCGAAGC CGAGAGAGAA GGTAATGGTC GCACCAGTGA 73380
TGATACCCGG AATGGAGATC GGCAGAACGA TTGAAAAATT TCCCAATCGG TCGCACCCGC 73440
CTCCTTGCCC GCCTCGATCA GTTCAGGAAC CGGTTCATGT AGATGACCGA GAGCAGCGAG 73500
CTTGCGACCC CACCTAGCGG CGTGCCACGC GTGCTGCTTT TGCCGCCGCT CATGCGCCGG 73560
TTCCCGTCAC CGTCCCGCTC TTCGACCGGC GCCTTACCGA CCGATCCCGA ATGGTCGGGA 73620
TGCCGAGCGG GCGCTCACCG CCCCCCGGCT TCGGGATCAT CACCCGTCGC ACCAGCTGTG 73680
GCCGGTAGCT CTTCGAGACG AGTTCCTCCC GCAGGCCCGC CAGCCACCCT TCCACGCCCT 73740
CCGCCTCGAT TTGCGCGAAG GTCACCCCGT CCACGCCCGG CGAACCCGCA TTGGCGCGGG 73800
CCAACCCATA GGCATGGCGC AGCATGTCCT CACGGCAGAT CTGGTCGTAG AGCACGTAGA 73860
AGCGGAAAGC GGGCTCCGCC TTCGCCTTGC GATACAGCTT CCTCTGCAGG TTCCTGATCT 73920
TTCGGGTGTT TCGAGGGTCA TCGCCAATCA CCCCCTCCTC ACCAACATCG AAAGCACATC 73980
AGAAGTCAGG GCCCTTCCCT CCACCGGCAT TACCCGGCTT CAGCGGTATT ATGGCCCTGT 74040
CCGACTCCCG TTCGCACCGC CGCCCTTGGC TGCGTTGGGT CTCCCCCGTT ACCCGCATCA 74100
CCCTTCCAAC GTGCCGTGCC CAATACCCGG GTGGATCGAA CGGGTGCCCA TGTCGATTGC 74160
TTCCCCGTTC ACGCGGCCTT CCCCGAATCT CAGGCACGTC GGCATCCACA TCATCACTTT 74220
CGAGGCCTGC TCAGGCTTTA CTCGCATTAC GGCCCATCGG ATCGCTCAAC CGCCCAAGGT 74280
GGCCTTTGTC ACGAGGCTTC GACCCGGCTA GTTACCCAAC CGAACCGGTC GCTAGCTACC 74340
CCTATCGACA ACTATCTGGG TGGAACCTTC CTCCACTGGT GATACGCGCC ATCGGGGCGC 74400
ACTGAGAGAT TCGGGTTAGG CCTCACGTTC CCCCGATGCG GGAAAGATCG ATCCGCATTG 74460
CTGCCGATCC AACGCTGTCG TTTCCCTCAG GCCATTCAGA GGCCGAGTCT TGAGTATGAC 74520
TATCGCGGTC ATCCGACCGC AACACTTCTT CCGTCGCAAC GCTGCCACCC GGATCAGGAG 74580
CCTGAAACAC GCTCACGCCT TCGAACTCAC CCGTCTTCCA TGCGTAGACA GCATCCTCGA 74640
ATATTTGAGG GATGACGTCG TCGCTCTCGG GATTGCCATA CGACTTGGCA ACGATCCTAA 74700
GAACCTTGGC AAACATCGAG GTTCCCTTGC GAAGGTTTTC GCAGGCGTCG ACGAGGTCCG 74760
GCTGAAGGTC TGCTGCGTTC TTCACGCCGA CGCCCACTGG AAACTGTGTC AGGCCGACGC 74820
GAACCACCGC CTGACCCACA TATTGCCTCA CGATTGCCAT CGCCTCGCCG GCGGAAGTCG 74880
CTTTCGGCAC GAGGATCAAT CGTCCACCCG ATTCAACTGA AACGGCGAGC GGGTCCGTCG 74940
AGCCGGCGGC TGCGACGAAC TGCTCGACGA TCGCGGGCTT GAGAGAAGGA TCGGCGCATT 75000
CTTTGATTAG GGCAGCGTCG AGCATGACGT CTCCTCGGGC TTCAGGTGTT GAAGGAAAGA 75060
ACAAGCGGTT TCGCGAACAG CTTTGCCCAA GCGATCGCGT TGACTCGCTG AATCGCGACG 75120
ATGGATGTCC CTTTGGTCCA CCATCCGTTT CCGACTGCGA CGATCAGGTC CGGCTCGTAG 75180

AGCATCGATT GCAAAGTGGG CCAGACGACG AGCTGCTCGT AACAAATCAG GGGAGCGACC 75240
CGGCTATTGC CTACGGTCAC GACAGGGTTG GCAAAGAAGT GCGCCCGGGC GCCGTCGCTC 75300
TCACCGAGCC ACGATCCCCA TGGCTGCCAC ATGGAGCCCG GGACCGGCAT TCGCTCGCGA 75360
TAGAGGACCC TGCCGCCATC GGCCGAGAGG GTGACCAACA CATTGTCGTA TCCGGTCGCG 75420
TCGATCGCCG CCGCGCCGGA GATGACGGAG ATCTCCGTGC CCTGCAGAGC CTTGACCCAG 75480
AGCCGTTCGA AGGTCGGTGT CCAGAAGCCC AAGGCGCTTT CGGGCAGAAC TACATTCCGG 75540
ACGCCACCAG ATGCGCGGTC CTTCACCGTG GCAATCAGAT CACGGTGACG CTGAATGCTG 75600
GTGTTCCGGC CGAGCGTTGA CCCCAGTTTG AGATCGACGC CCTGCCAGCT CGCAGGTAAT 75660
TTCGACTCGG TCCAGTTTGC GGCGGACCAA AGCCAAAAGC CTGTGAAGGC GATTGCGACA 75720
GCCGGCCACA TGCGGGTTAC GAGGCCCATG AGGCCGGCTG TCGTCGCGAC CAGTCCCCAC 75780
CATCCCCATC CCGGAAAGAG AATTCCCGCG GCCGTGACAG GATGCGCCCA GCCGGTGATG 75840
CCCATTGGCG GCACAGCCAT AAGGACAGCC GCCAGGAGAT AGCGAAACGG TCGACAATCC 75900
GAACGCTTCG TCCAGAGCAC CGTATGCACG ACAACAAAGC TCCCCGAGGC GCACAGCCAC 75960
AGCAGCAAGC CCGGCCACAG ATCGGAGGTA TAGAACGCCG CGACGCCTTG CGGCAGCCCG 76020
CGTGACGCCG CCAGGAAGTA TCCAGCGGAA ACGAGTGCCG CTACCCTCCG CGTGCGCGCG 76080
CGCGACCAAA GAATTGGGAA TGCCAGTGCG ACCGGAAGAA GGAGGGCATA ACCGCTCCAC 76140
CCGACCGTTC CGACTACAAT TGAAGCGATG GTCAGCAGTC TCGGCTGGAG GTGATCACGG 76200
CGCATAGGTC AGGACTTCTT GCGCCAGGCC GAGAATGCCG GCGGCCGGGA TCGGACCGAA 76260
ATAACGGGAG TCATAGGAGC CCGAGAACGG CGAGTGCAGG AACACGTAAT CGGTCGGCAC 76320
GATTCCGCCC GAGAACGGCT TCATCGGCCG TCCCTGTCCG TCGCGCAGAG CAAGAGCGGA 76380
CGAGGGGACA GGATGCCCGT CGACGTTGAC GCTGACCCCC ACCTCGACCC GTTGCCCTGC 76440
AACTGCGATG ACCGACTTGA TGAGCGGCGC GATGCCGCCC GGGCATAGGC CGGCGCGCAG 76500
ATAACCACGC ATTCGCGCCT CCCGCATGGC CGAGGTTCGA GGCGGACAAA TGAAGACGAG 76560
ATCGCCGACG GCGGATGGGC GATCGATCTT GACGATACGC CAGAGACCGA GAGGTTCACT 76620
CGGCGTGAGA TTGATCCGGT AGCCGCCGAA CCAGGCGGCC GAGATCGTGC CGACCGCCGC 76680
CATTGTCATC GTGGCTAACC CGAGGAGGAG CGGCTTACCG CGTCGCCTCC TCGAGGTCAA 76740
TTCAACGAGA GGAATGTTCA TTTCAGCGAC AGCCCCTGGC TCTTCGTCTG CCGCAGCGTC 76800
TCGGCCTGCT TTAGGGCTTC GATCGTTCGC TCATGGGCAT TGAGTTGCTG CACCGTACGC 76860
ATCGTGTTCC AGGCCGATTG GACCTCCGCC TTCGGGCCTG GGTTCATTCC AGCTGTGATC 76920
GTCTTGAACG TTTCTCCGTT CGTGTCCTTG GCGGCCAGTG GCAGGAAGGC GCGCTCCCCG 76980
AAGCGCTCGG TGACCGCCCT GGCAAAGCCT TCCAATTCGG CCTTGACTTG TCTGTCGGCG 77040
AGAGCAAACT CCAACGCCGC GGGTAGGTCA TTGCGGTCGA TAGCATCACG CACGCGCTCC 77100
AGCGTCTGCC TGGCGCCCGG AGACAGCGCC GGAATCTCGA TCGCCACCTT GCGGCGGACT 77160
GCCAGTTCAT CGGCCTGATA GCACTGTTCG GCCCGGCTGC GCTGGCGCAT GTAATCGCTG 77220
AGGCTCTGGG CCAGAGCCGG TACATTTCGT TCGGCCCGCT CGCGGTCCTG CTTGTCGGCA 77280
CGGCTTGCGA GCAAGCCGGT TTTTCCTTTC AGCGAACCGT AGGATTCCGG CTGATTGGCC 77340
ATGTTCGATA GCGCGGACTT CGCGACGGTC TCGTCCTTCA GCATCGCGTC GACATTGACC 77400
GCCTTGAAGG CGACTTCCGG CTGGGTATAG ACGAGATGGA AGCGGGTCGA GACATCCTCC 77460
CATTGCTTCT TCAAACCGGG ATCGACTTCG AGTCTGTCAG CGACTGCCTG CTCCATCGAT 77520
TTCGGGAAAG TCGCGATACC GGCAACCATG GGCCTGGTCT CCTTGAGGTT TTGCGAGTTG 77580
GTTGTTTTGG CGCCCACGGC GAGACCGAAC CTCGCGCCGA TGGCGACGAG CCGCTGAGTC 77640
AGATCGGCCA GTTTCTGTTT CTGCCGGACG GTCCACTCGA GCCGGTCGCG GGCAATCGTG 77700
CGGGCGACAT TGACGAGGTG CATGCCGCGG GCTTCGGCGA AGCGCAGCGC CTGCCGGTAG 77760
AAGGTTGCCT TCTCGTAATC GAGCGTGGTT TCCTTCGAAT TCTTCTGCGA CAGGATCTGG 77820
ATGAGGCCAC CGGACTTCGC AAAGGATCGG GCGCCGTAAT AGACCCCGAG ATCCTCGCGA 77880
TGGCGGGTCA TCGCGACATA GGTCAGATGC CGATCGAGGG AGAGCGAAGC CAGCACCTTC 77940
ACCCGGTCGA CGGTGGCCCC CTGGCTCTTA TGGATGGTCG TGGCATAACC GTGATCAAGA 78000
TTGTTGTAGA AGCGGCTCTC GACCATGACC TGACGCCGAT GCTCGACTTC GCCGATCTCC 78060
GCGACGATGC GGTTCTGAGC GGCCTCGACG ACCTTGCCGC GCATGCCGTT CTTGACGCCG 78120
AGCGCGCCCT CGTTTTTCAG GAAGACGATC TGGTCGCCAG GTGCGAAGCG GCGATTTCCA 78180
TCCTCCGCCT TGAATGAGAA ACCAGCATCG ACAATGCCAC GCTCGACGAG CTTGGCGCGC 78240
GCCATCTGGT TGAGCATGCG CACATCGCGC CGAAGGTGCG CGAGGATCAA TGTCGTCTTC 78300
GTCGGATCGT AATCCCGGTC CCAGTCCGCG ATCAGATTCT GAACAGCTTC AGCCTTGAGC 78360
TCGGAGCGCA TCATCCTGCC ATTTGCACGA TAGGTATCGA CTACCTTGCC GACATTGCCA 78420
CGTGCGAGGT CGAGCGACGC ATCGCACATC CACTGTTCGC GCTGGCGATA GATGGTCTCC 78480
AGTTCGGCAT AACCGATCCG ATCGGCAATG GCGCGGAAAG CTGCACCCGC TTCGATCGGC 78540
TGCAGCTGTT CCGGATCGCC GACCAGGACG AGCTTGGCGC CGGCCTTCGT CGCCGTTTCG 78600
ACGAACAGCG CCATCTGCCG CGATGACACC ATGCCGGCTT CGTCCAGCAC GAAGATCGTC 78660
TTGTCGTCGA GCTGCTTTCG GCCTTCGTTC CAGCGCAGCT CCCAGGACGA CAACGTGCGA 78720
GACGCGATTC CCGCTTCCTT TTCCAAACCT TCGGCGGCCT TGCCCGCAAG CGCACCACCG 78780
ACGACACGAT AGCCGGCCGC CTCCCATGCT TCGCGCGCAG CCTTCATCAT CGTCGTCTTG 78840
CCGGCGCCGG CGCGGCCAAT GACGGCGGCG ATCCGCTCGG CTCCGGCCAC GTGCTCGATC 78900
GCCGTCTTCT GTTCGTCCGA CAGACGGGAA TGGCGACTGA ACGCCGCCTC TAGCACTTCC 78960
TTCCGAACGC CATGCGAGGA TCGCCTCGAG AGCCAGATCG CTCGGGATCC CATCTCCGCT 79020
TCGAGCCGGA TCAGCTCGCG CGTCGTGTAT TTTGCTGGCG CCCGGATGCC GGTCGCAAAC 79080
GCAATCCGCT CGCGTTCGAG ACGGAGCGTT TTGGGGCTCT GGAGAATGCG GGCCATCAGG 79140
CTATGGAAGA CCCCGGGGTC GTCGATATAG CGATGCAGGA TCTTCGCCAC ATCACGCTCG 79200
TCGAAGACGC TCTTCTCCCG GGTGATCAGG TCGAGGACGA TTTCCGGCGG GCGCTGGATG 79260
CGTCGAGCAT TCTCGGCGCG TTTCTCCTCC TGCAGCTCCA GCCGCTCAAG CGAAACGGCC 79320

TGGTCATCGG TCGTCGCTTT CCGCTCGATC GCCTTGGTGC CTACCCCGAG ATGAATGGTC 79380
GGGGTAAGCT CGATGCCCTG CTTCTCGAAC GAACGGCCAT CGATGCGAAT GTCGAGACCG 79440
GCAAGTGCGA GATGCCGGTT CTGGCAGGCA AACCAGCCGT CGCGAAACGC ATTGAAGTCG 79500
TCCAGGCTTC CGGCCCACAG TTCATAGACG ATCTTGCCGG CATCGTTGCG GAGCGAATTG 79560
CCGTCCGGCC CAACAACCGC AAGTTTCTTG GCCCCGAACC CATCAGCAGT CAGCGGCCTC 79620
AACGTCGTCA TCAGATGGAC ATGCGGGTTG CCCGGTGCAT CGTGATAGAC CCAGTCCGCC 79680
ACCATCCCCT TGGCGGTGAC ATGCCGCTCG ACGAAGTCAC GCACCAGCAC GATGTTCTGC 79740
TCGGTTGTCA ACTCGATCGG CAACGCGATC GTGACATCCT TGGCGAGTTG CGCGTCGGAG 79800
CGCTTTTCGA AATCCTCGAC CTTGTTCCAG AAGGCTTCCG AGGCGCTGGC GACCGAGCGA 79860
TCGGCGATCA TCGACCGCAA CCACTCGGGT GATTCGGCTG GAATGACGAA CTCTTCGTGA 79920
AGAAGACCCT GCTTGGCGCG ATAGTCGATC GTCCGGGCTT CCCGCTCATA GTCCATCCTG 79980
GCGCAGTGCC GATAGGCCGC CGACAGCACG GCGCTGCGGC CGGAGCCGCG GGCGACGACG 80040
CTGACGGAGA AATGAGGAAC GGCCACGGCA GAGTCTTCCC GATTGCACAC GAAATCAATG 80100
GGTTCGTCGG GAGCGGTGGC CCCGCCAGGG CCGCAAAACA GGATGTCGCG ACAGCGACGT 80160
ATAATTGCGC CCTTGGAAGC CGCTCCTTCG GAACGGCCGG GATCATTCAC CAAAGCATCG 80220
CCCTTGGCGA TTGTAGGATT CACAGTAGTG CGTTCCGCAC TCCGCTCCGA AAAACTGGCT 80280
TCGAAAGACA AGCTTTCTCA GCCACGGAGA CGGACGGAAT GAAGAAACCT GCCTCGAAAA 80340
TTCGCGACGA AATCGCCAAG CTTCAGGAAC AGCTCAGACA AGCGGAAACC CGCGAAGCCG 80400
AGCGAATTGG CCGGATTGCG CTGAAGGCAG GACTTGGCGG AATCGAGGTG GAGGAAGCCG 80460
AGCTTCAGGC AGCCTTCGAA AGCCTGGCAA AACGCTTTCG CGGAGGCCAA GGGGGAGCGA 80520
ACGGGGCTTC GAGCGGAGGG AAAAAGCCAG ATGGGAATGC CACGGCCGGC GCATCGGCCG 80580
CGGCGGTCGG GTCTGGCGCG GCTCAGGGCG GCAATGGCGA GGCTTGAGCG GATGGCGAGG 80640
TCAGCGACAT CCGACGCCCG CAAAAAGGAC ACGCGGGAGA AGATCGAACT CGGCGGCCTG 80700
ATCGTGAAGG CGGGCTTGCG ATACGAGAAG CGAGTGCTCC TGCTCGGGGC GCTGGTCGAC 80760
CTCAGCCGCC GTCTCAATTC CGACGAAAGC GAGCGCGCCC GGTTGATCGC GATCGGTGCG 80820
GAGGCATTCG GCGATGACGG TGAATAGGAT CGCACTCGTC GTCGCCCCTG TCGTGCTGAT 80880
GGTTTTGACC GTCATCGGCA TGAGCGGGAT CGAGCAATGC CTGTCGACCT TCGGCAAGAC 80940
TGACGCTGCA AGGCTGGCTC TCGGCCGGAT CGGCATCGCC TTCCCCTATG TCACCGCCGC 81000
AGCGATCGCG GTGATCTTCC TGTTCGCGAG CGCGGGGTCG GCGATCATCA AGTTGGCCGG 81060
CTGGGGCGCG TTGGCGGGAA GCGCGGCAAC GATGTTGATC GCGACGATGC GCGAGGCGTC 81120
ACGCCTTGCC GCATTCGCAG CGCAGGTGCC GGCGGGCAAG TCCATTGCCT CGTATCTTGA 81180
TCATGCAACG CTAATCGGCG CGACGGCGGC GTTGATGTCG GGATGTTTCT CGCTGCGCGT 81240
CGCGCTGATC GGCAACGCCG CCTTCGCACG CGCCGAGCCG AGACGCATCC GGGGAAAAAG 81300
GGCGCTGCAC GGCGAAGCCG ACTGGATGAA CATGCAGGAC GCGGCAAGGC TCTTCTCCGA 81360
TGCCGGCGGC GTCGTCATCG GCGAGCGTTA TCGTGTTGAC AAGGACAGCA CGGCGGCGCG 81420
GTCTTTTCGG GCCGATGATC CCGAAACCTG GGGCGCCGGC GGCAAGTCGC CGCTGCTTTG 81480
CTTCGACGGT TCGTTCGGAT CCTCGCACGG CATCGTCTTC GCGGGTTCGG GCGGTTTCAA 81540
GACGACGTCG GTGACAATCC CGACGGCACT CAAATGGGGC GGAGCGCTTG TCGTTCTCGA 81600
TCCTTCCAAC GAAGTGGCCC CGATGGTCTA TGAGCATCGC AAAGCAGCCG CGCGCTTCAT 81660
CCGCATTCTC GATCCGAAGG AACCGGAAAC AGGATTCAAC GCGCTCGACT GGATTGGCCG 81720
GTTCGGCGGA ACAAAGGAGG AAGACATCGC CTCGGTCGCG TCATGGATCA TGAGCGACAG 81780
CGGCGGCATG CGCGGCGTGC GCGACGACTT CTTCCGGGCA TCGGCGCTGC AGCTGCTGAC 81840
CGCGCTGATT GCCGACGTCT GCCTGTCCGG CCACACGGAC AAAGAGAACC AGACCTTGCG 81900
GCAGGTTCGG GCCAATCTGG CGGAACCGGA GCCGAAACTG CGCGAGCGGC TGCAGTCGAT 81960
CTACGACAAT TCAAATTCGG ATTTCGTGAA AGAAAATGTG GCCGCGTACG TCAATATGAC 82020
GCCGGAAACC TTCTCCGGCG TCTACGCCAA TGCGGTCAAG GAAACCCACT GGCTGTCCTA 82080
TCCGAACTAC GCCGCACTCG TATCCGGTTC GACCTTCTCG ACCGACGCGC TTGCGGCCGG 82140
TGAAACCGAT GTGTTCATCA ACATCGACCT GAAGACATTG GAGACCCATG CCGGTCTTGC 82200
GCGTGTGATC ATCGGTTCGT TCCTTAACGC CATTTACAAT CGCGACGGCG AGGTAAAGGG 82260
GAGGGCGCTC TTCTTTCTCG ATGAGGTCGC GCGGCTCGGC TACATGCGGA TTCTGGAAAC 82320
CGCGCGTGAT GCCGGTCGCA AGTATGGCAT CATGCTCACC ATGATCTACC AGTCAATCGG 82380
CCAGATGCGC GAGACCTATG GCGGACGAGA CGCGGCGAGC AAATGGTTCG AGAGCGCGAG 82440
CTGGATTTCG TTTGCCGCGA TCAACGATCC GGAGACGGCG GACTATATCT CGAGGCGTTG 82500
CGGCATGACC ACGGTCGAGA TCGACCAGGT CAGCCGCAGT TTTCAAACGA AGGGATCCTC 82560
AAGGACGCGC TCGAAGCAAC TGGCCGCTCG ACCGCTGATC CAGCCACACG AAGTTCTGCG 82620
CATGCGCGCC GATGAACAGA TCGTGTTTAC GGCCGGCAAT GCGCCGCTCC GATGCGGCCG 82680
AGCGATCTGG TTCCGGCGCG AAGACATGAA GGCTTGCGGC GGCGAACCGG TTTCACAAGG 82740
TCGGAAAGAT GTCTGAAGCC CGCCTGATCC AGCCGGCGCG CAGTGCAACG AGCAAGGCGG 82800
ATCCAGGAGC ATGAAGAGTA TCGGAGAAAT CAGCGTGAGC GCCGCCCAAC AGGCGGGATT 82860
TCGGGCATTC TCGTCGTGCG AGGAGCGGGA AACAGGGCAG CCGCAGGAGC CTCAACATAG 82920
TGGCCCGAAG GGGAGGCGGG AACGACTGCT CCCCGCTTCT GCACACAGTT TTGTCTCCTG 82980
ACTTTAGCCA ACACGTCAAA AGCGGACTTG CTGTCGGCTT CGCTTTCGAT CTGCGGTGAT 83040
GGCCGCCACA TCAACTGCGT CGTCGATGGC GGTTCTGGTT CGAGGGCGAA AAAATCCGCA 83100
TTGCCGATTT CGACACACCT GAGTCGCGCC CTCCGCGATG CGAGGCCGAA CGGATAAGGG 83160
GGAGGAGGCG AACGCACGCC TTCTCACCCT GCTCAACGCG GGGACATTCT CGCTTTCAAC 83220
CGGGTTACGA GATGAGGACA AGTATGGGCG CAAGCTCCGC ACGGTGACAC GAGCGGGGCG 83280
CTCGCTGGGG CAGCACGCTC GTTCATGAGG GCCTTGCCAG GCGCTGGGAC GGTGTGCGAC 83340
ACGGCTGGTG TGACTGAGAG GTCGCGAGAG AATCGTCACC CGACGGGGCG GGGACCGCGG 83400
GCGGGCTCCG TGAGCGCAGC GAGTAGAGCC GTGCGCCATA GGCGTCTCGC CCAACATGCT 83460

CTTTCTCATA ACATCAGCGA TATGAATCGA TCCGTGCCCA CCGATTCACA AGTGTCGTTG 83520
GACGCGATAG TCGGGATCAG CGATTCTGAT CTCATGATCT CGCAACCCTA CCGGCTCTAT 83580
GTCGAGCGCC TGGATCCCTC GAGGAATATG GCCCGCTACT ATGCCATGTC GATCGAACCG 83640
AACCTCTTCG GCGACATCTG CCTGCTTCGC AAGTGGGGTC GCATTGGAAC CAAGGGACAG 83700
ATGATGGTCC ATCATTTCGG CCAGGAAGAG GATGCGGTCC GATTGTTTCT CGATCTGCTC 83760
CGACAGAAAC GAAAACGCGG TTATCGTCCG CGACCTTCCT TGCCGAAGGA AAAATGGCCC 83820
GCCGAAGCGG AGCACGAAAG CGCTACATCC GCGCCGGTTC CAGATCCGGA AGGACACTTG 83880
GATTTAGATG AAGAACGGAA TCTTGATGGC GAGCTTTAGA CCGAGCTTTC CCTTCCGGAA 83940
GGAATGGCTG CTCACCTTTG CCGAGGACGC GGAGCCGTCG GCCGGCGCGA AATGTTGCGG 84000
GGTTCCGGCA AGAAGCCGAT CCAACGCCTG GCGAAGGCGC CAGCCGCGAC AGCCTCATCG 84060
AAGACATCGG AATGGCGCGC CACCACCGCT TATGGGTTCC TCCCGGCCGG AGGGGACGTC 84120
CGGCCTCACT CGCCCCGGTA CGAATCTCAG GGTGTGCTAT CTGACCCGGC TGATCTCGGT 84180
GAGCGATTTG CGGCGCGCGA TGAACTCGCG GAGGAGGGGA AGGAAGAACG CTTTGCTGAA 84240
CCGCCCGATC GGCGGCTCGG GCTCGATGTA AAAGGAGCGG CCAACGATAT AGGTGTTGAA 84300
TTCATCGAAG ATGATCCAGA GGCGCAGGTC TGCATCGAGG CCGGCCCGCC GCTTCTCGAT 84360
GGCCGGAATT TCAGCCGCGC AGCGAGCCTG CTCGGGCTGC TTGGTGGTGA TCGGGAAAAA 84420
CAGAACCAGG TCGTCATCAG GTCGGGCAAG CCTCACGCCG ACGGCGACTG GCCGATACTT 84480
CCGCCGCTCT ATCTCTCCTT TTCGGGCTTG CCGCGCGCAT AAATAGGGAT AACGGATGAC 84540
CGTCGCGGTC TGGATGCCAT TATAGCTGCT CATCGATTGG TCTCGTCGGT TTCCCCATAG 84600
GCGTCCACCG CCGCCTCGAA TTCGTTGAGC AGCTCGCCCG GCAGGGTTTC GAGTGTGCCG 84660
ACTGAACGGG CATCGTATTG CCGCATCAGC CGTTCGTAGT CGTCGATGTT AAGGAGGACG 84720
AGGCGCGGCT TGTTGCGTTG GGTGATCGTG ACCGGATGGC GCAACGCCTC GGCGATGATA 84780
TCGCCGGACT TCCGGGATAG ATCGCTTGTT GAGTAGGAAC CACCGGGGCG AGGCATGTTG 84840
ATCTCCAGAT GTCTTTGCCG GCGGGCACCA TAGCATCAGA ACCGACGCAA ATCCGGCATT 84900
TACAGTATTG TTGTAAATGC TGTGTATTTC CGCGGTGAAA GGCCCCGCCT GGCGGCGAGG 84960
CCTTAATCGA GAGTATCAGT CCCGGTTGGG GCGGGACCAG ATGAGCTGGT AGCCATCTTC 85020
GCCTTCGACT TCCGTCAGCG TCGCGTAGAT CGGGGCCGGG AAGCTCGGGT CGTCGAGCTT 85080
GACCGAGAGG TAGTCACGGT CCTGCTCGGA GCGCTTCTGC CAGGCGGCGC CTAGTTCGAC 85140
GGCACCTGCG TAAATGCGGA AGTGCGGGCC CTTGTCCGAG GGATTTTCGA TGCGGCCGAT 85200
GCGAGCTTTG ACGTTAAGGG CGAGGGTGCG GATTGAGCCG GTGAAGCCGT TTTCGGTAGA 85260
GGTGAAGGTG CCAATGGTAG CCATTGTCAT GTTCCTTTCG TTGTTTCGGG CCGCGCCCTT 85320
CGCGGCCTCG ATGGCTGTCG GAAAGACCGG GGGCGATCGG TCCGCACCTG AAAGGCCGAA 85380
ACATTGTGGA GGGCGGGCAG GAAGAACTTT GTTGTTCCGT GAGGAATGAC GGCGGAGCCG 85440
GCAGGGGAAG AAAGTTCTGA AGGGCCGTTG CGGGAAGCCA ATCGAGGCGA AGCCGTTCTC 85500
CGGTCAGGCA TGCCTCATCG AGCGCCAGAA GTGAGCGCCG CAACAATGAA GAGAAGGAAC 85560
ATGGGACTGG CTACGCTCGC GCCTTTCGAT CGAATGTGTC CAAACGGCTC ATAGGTGAAA 85620
CCGCTCCGGA TGCCTGGTCG TTTGCCGCGA GCACCTCCGT ATGGAGCTTC GCAGACGCGT 85680
GATCCGGCCG CGGCTCCAGT TCGGGCGCGA TACCGAGATC GGCGCGTAGG AAGCAACTAC 85740
CAAGCTCCGC GACATATCCT GACAGTCAAG CCGTCAGCAG CGACGGGGCG ACGGATTTCT 85800
CCCAGTAAAC CCCTGTCAAC AGCGTTGATC AGCCGGCACA GCCCGACAGC CTGGCGACGT 85860
CCTTGTCGAA AGCCAGCGCC ACGAGTTTCA GCCCTTCGAC GGTGGTGAGG TACGGAAAAA 85920
TCGTGTCCGC CAGTTCGTCG ACGGTGAGGC CCTGCCGGAC CGCCAGCGTC GCGGTCTGGA 85980
TGCTGTCGGC ACCCTCCGGC GCGAGGATGT GCGCGCCGAG TAGGCAACGG GTTCCAGCGT 86040
CGGCGACGAG CTTGATGAGC CCTCAGGTAT CGCGAGCGGC GAGCGCGGGG GGCACCTGGT 86100
CGAGACCGAT CGTCAAGACA GGGACCGCAT TTCGCTCGGC GCGCGCCGCG GCCTCGGTCA 86160
GCCCGACGCT CGCCATCTGC GGATCGGTGA ACACGATCGC CGGCATGGCG CTGTTGTCGT 86220
AGCACAGGCC GTCGCCGTTG AGGGCGTTCC TCGCCGCGAG CCTGGCGCCG TAGGCGGCCA 86280
TGTAGACGAA CTGGTCGCGG CCGGTGACGT CGCCGGCGGC ATAGATGCCG ACCCGGGTGG 86340
TGCGCATTCG GTCGTCGACG ACGATGCCGC CGTTCAGCGA GACGGCGATC CCATGGTCGG 86400
CGAGGCCAAG GCCGTCGATG TTGGGGGTGC GGCCGGTCGC GATCAGCACC TGATCGGCGT 86460
CGATCGCAAC GTCTTGGCCG TCGCGCGTGA CGGTCAGTAG AACGCCGCCC TCGATCCTGC 86520
GGATGGCGCG GTAGGCGATG CCGGAGACGA CGGTGATACA CTCGTCCTCG AAACCCCCGT 86580
CAGCGCCGCG CCGATCTCGG GCTCAGCCTC GGGGAGCAGG CGCGACCGGC AGACGAGCGT 86640
CACCTTGACG CCGGCGCGGG CGAACATCTG GGCGAGCTCT GCGCCGATAT AGCCAGCGCC 86700
CATGACGAGC AGCGAACGCG GCAGTTCCTC GAGGTCGAGC GTGGTCGTGC TGGTCAGATA 86760
CGGCACGGTC TCGATGCCGG GAATGGCGGG GGCCGCCGGA CGTGCGCCGG TGGCGACGAT 86820
GATCCTGCCG GCCGGAATGC GCGTGCCATT CACCTCGACA CCGCCGTCGA TGAGGCGCGC 86880
TGGACCGACG CGATAGGCGA TGCCGTTATA GACGGGGAGC AGGTCGACAT ACTTGGCCCG 86940
GCGCAGATCG GAAACCAGCA TCTCCTTCTG ATGGACGGTT CCACACCAGT CGGTCAGTTC 87000
GGCCTCGGCG GTGATGCCGG CAAAGCGGGC GGCGGCGCGT GCATTGTGCA GCGTCTCGGC 87060
GGCGCGGATC AGGGTCTTCG ACGGCACGCG GCCGACATTG ACGCAAGTGC CGCCGATGGT 87120
GCCGCTGCCG ATGAGCGCGA CTTGCGCGCC CTGATCGGCG GCCGTGATCG CGGCCGAGAA 87180
GCCGGCCGAG CCGGCGCCAA CCACGACGAG GTCATAGGCA CCTCCATTGC GGCCGTTGGC 87240
CGGGAAGGGC CGGCTTGCGA CCGTCGCGCT CTTCGATGCC GCATCGGACC GGCAAGGCTC 87300
GGCCCGAAAA GCGCGCGGCC GGCCTCGAGC CCTTCCTCGA TCGACAGCCG GAAGCCCGGC 87360
TCGTCGGCGG AACGTCTGTC GCGCCAGGCG GATAGATGCT CGTCAGAGCA GAAAAACGCC 87420
GTCGTCGCGC AGAGCGCTGC GCAGCCGCGT TCATAGCGGA CAGTCTGCCA CATGACGACC 87480
GTCGACGGCG CGACCTCGGC CAGCGCCCGT CCTCGCTGCC GCGTGGTGAT CCGGATCGGC 87540
GCGCCGCAAT GGCGGCATTG CGAGGCAATA GCGATGTCAC GATTGGTCAT GGCGCCGATG 87600

CCGAGCGCGT CGACGGTGCA CATGGCGTTG ACTACGTGTC CGTACAGCGT CACCCGATGG 87660
CCCGTCTCCC GGTCGGTGAA GGGATAAGCG CCGACGATCT GCTCGCCGTC GAGAACGACC 87720
AGAGCGCGCC GGCGAAGTTC TGCGAGCAGA GGCCGGATGG CCGCCCCGCT GAGTTCGGCG 87780
CGCTCCGCAA GCGCGCTCCG GCTTGGGGCT CGTCCGTCCT CGGCATACTT AACCAGCGCG 87840
ATGCGCACGC GGTCCGCCGC TGTATCATAG CCGCTCCAGC GCTTGAGCAC ATGGTCGGAG 87900
CCGACCATGG CCGGCAGCGC GTTCCGGACT ACCGGCGACG TGACGACCGA CCAGTTGGGA 87960
AACGTCACGC CCGGCCGCAC GGCAAAGCTC GGCGGTGTCG CGGGCACAGA AATGACAGCC 88020
TTGTCCTGGG GGGAGGATGC GCAGCAATTG TTTATGGCTT CATGCTCGGA CGACAGCGCC 88080
TGGTCGCAGC CGGTGGCATT CTCGGCGCGC TCGCCGCCTC GTCCTGTTGC ATCATTCCCC 88140
TTGTCCTGTT CAGCCTTGGC ATCGGCGGCG AATCGCCGAC CTGAGGCGCA TGGAGCGCGT 88200
TCTCAAATCC GTCGTTGCCC AATGCGGCGA CGGCACGCTC CCGGAGTGTC CGTTGATCGA 88260
AACGTTGCTC CAGGAGCGAA CTGTCAACTG ATCGCTGGGG GCGCCTATGC AGCGCCCCGG 88320
ACCTCGTTCC TCTGAGCGTC GCTCTGTTGC GCGTGCATCC AGTCGGCGAT CTGCTGCGCC 88380
TTGCTCGCCG CAGTGAAGAT GGCGCGCTGG TCGGAGCGCA GCGCCTCGAG CCAGGAGGCG 88440
ATGTACCGGG CGTGGTCGGG TCTCGCCTCG ACGCTGAGGT TGAGATCGGC GCAGATCATC 88500
GCGCTCAACA ACTCGACCGT GCATTCCTCC ATAGCGTATG CGGCCGAGCC GAAGCGTCCG 88560
GAAAGATCGC GGTCGAGGCG ATGCTTGGCG CCGGAAGCGT GGCCACATTC ATGGAGCAAT 88620
ACGGCGTAGT AGGCGACCGC GTCGCGGAAG CAGGCGAACT CCGGCATCTG CACATGATCG 88680
GTAGACGGAC GGTAATAGGC CTGCGAACCG CCGTGGCGGA TGTCGATGCC GAGGGAAGCG 88740
CAGAACCGTT CGGCCCGCTC GATCCGCTCG GCCTCGGGCA GCACCGGCAT CTCGGGCGGC 88800
GTGTAGCCGT CGACCTGGGC GCAGTTGAAT ACGGTGTAGC CGCGGGCGAA CAGACGCCGG 88860
GCGGGCTCGT GACGATCCTC GTCGCCGTGT TGGGCGTCCG CCGCGTCACG ATCGGTCGTC 88920
TTCCAGAACA CGACCAAGTG ACCGCGCTCG CCCTTGCGAA CCTGAGCGTC GAGCGCCTGC 88980
CATTGGCGGT AGGTGCCCCA GATGCCGGCG GCATAGCCTT CAGCGTGCGC CGCAGCCCAG 89040
AGCGAGAGGA TGTTGACGCC GCGATAAGCC TTGCTTGAGG CGACGTTGAC GGGCGTGGTG 89100
ATGGCCGATC CGTCGTGATG CCAGGGCATC CGGTAGTCGC TGGCGCCCGC CTCGATGGCG 89160
GCGATGATCT GGCTGGTGAC CCGCTCGTAG ATATCGGTCG GGGTCTGGGG GGTGCGATCT 89220
GTGGTCATTG CTGCCTCCTT AAGCTCGCCC TCTCCGTCGA GGGCGAGGCG CCAGGGGCAG 89280
ACGCGGGAGG CCGGCCCGTC ACACCCTGTT CTCGGTGCTG GTGCGGAACA GGGTTGATGG 89340
GCTGGCAGCG TCTGCCACAC CCGAGCCCTG CCTCGACGGG CGAGCTAAAG GAGGCGCAGA 89400
TCATGATCGG CGCGCGCGGA ACAGAGCGGT TAGATGATGC GCGGCTCTCT CAGCCGGCGT 89460
CGTCTCGCCG CGAGCACTTC AAGGCGACAA CCCTGGCGTC AACAGATCGG GCCAGCTCCT 89520
TCCGCAGCCC CTTCGATGAC GGCATCCCGC TCGGCGATCG AAGCCTGCCA CGGCCCGCTC 89580
AGCATCAGGA GCCAGACTCA GTCCGAACCT CGTCTATGTT GATCAGGACC ACCATCATTC 89640
GCTCGAGGTC CTGCTGGAGG ATACTGCGCT GAAGATGGAC GAGTCGATTC TCGTTCAACA 89700
GGCTCCTGGC TTCTTGCGCT GAACGGGCCC AGGCGGGCCG ATGTCTTGCG GTGATGCAGG 89760
CATCAGGACC AGGGTCGGCT GGCAAAACAC TGTCAGCGGG CCTTTGGCAT CAGTTGCCTG 89820
CTTCAGGCAG GCGGCAGTGC CGGGATCGAA GAAGCAGCCG GCCAGCACCC GACATGCAAT 89880
GTTCGCTCGA GGCTCGCGAG CATTGTGCGG AGCCGGGCAC GGTCCGCAAT CATCGCCGGA 89940
AGCGGGCCGA GTTCTGCCGA TATGCCGTCA AGCGTCTTCG CTTATGCGCG TTGCCGCGGG 90000
TACGGGCTGC GTCGCGCCGA CCTGGCGCAG GATCATGCCA TCCTCCGTGA TGGAAAGACA 90060
ATCACCTCCA CTCGAGGTGA GATCTTATTT GGATATAGAG ATATTGGCCT TTCTGAGCGC 90120
TTCCTCTATC GATTTTGCTC CAAGCGCCTC AAGCGCACGA GCAAGGCTTT GTTCGACTTC 90180
AGGGATACGT TTTCCCTGAA TCAGCGCGAT CTCGGCGACA GATCTACCTT CGGACACCCA 90240
TCGAAGGCAA GATTTTTCGA TGGGTGAGAG CATGGTTTCC CAAATCCAAC CGCCAGATTA 90300
TGCACTACGG ACCATCGGTC TCGGAAACCA AGAGCATCGC CGGCGAAAGG ATGGCTTACA 90360
GGCGGCCATA TACTCCTACC GACTTCTTGA TGAGGGATGG CGAGCGCAGT GAAGCCCAAT 90420
GCCTCCCCTA TCCGGCGAGA CCACCCTCCG CCACTTAGTC CACGTGGAGT TCAGTTCGAG 90480
TTCGGACCTC AGCAGCAGGA AAGCGGCTTC CGGGAGGAAG CCGACCTTTC CATGAGAGGA 90540
TCCCCTCTGC CTGCGCAGGG TAGTTTAAAG CCATACGCAG CGCCGGCTGC CACAGCGCGC 90600
GCGTCCGCCG CCTTGAGCAC TGTTGATGGT CAGCGCGCCG GCTCTCGCCG AAAATGTCGC 90660
AGCAATCGCG ATGGCTGCGG AATGCAAGGA CGAGTGACAG TTTCATGAGC TTCTCCCAAG 90720
TTTGAAGATG CACACCTCTT GCGTGGGCCC CTTTGCCACT TAATTACATT GCGCAAATTA 90780
ATCTTCGTTG CAACAGATTG TTCGAACCGT TTGTCCTAAG CCGGACAAGA ATGTTGGTGG 90840
CCGGCCAGGA TTTCGTTAGT TCCGGACCGC GGCGCCGACA AATGGTGCGG CGTAAGGGGC 90900
GCGCCAATTC GGCACGGAAC TTGCTGCGAG GATTTCGTTT AAAGCAGACG GCAGCGGCAG 90960
ATCTTCGGTG GATTAGCCGG CCGTGGGTGA TTAGCGCTTA GTATGGCGTC ATGAACAGGC 91020
GTGTCACGTT GTTTTCATCG GGGATTGATC ATGCGAGTAG CGCTGATTTC TACGTCAATA 91080
TTTGCCTTAT GTTGTTCGTC AAATGTCAGG GCAGAGGCGG TGCTTCCAGT GAACCCGTCA 91140
CCATATCTGG TCGGCGCCAT CACGAGAAAC ATGTTTAGAG TGCCAAAACC TGACCAGGAC 91200
TTGGTTCTAC TATCTGCGCA CCGCGGATCT TGGGAGGTAT ATCCCGAGAA CTCAGCCTAC 91260
GCCTTGCAGG ACGCCTGGAA TTCACAGATT GAGGCCGTGG AGGTCGATGC CCGCTTTACC 91320
GCCGATAAGG AAGTCATTCT ATCTCACGAT TACAGGATGG AGCGCGAGTC CACTGGAACC 91380
GGCTTCTTGT ACGATCAGAA CTACTCGCAA GTACAGCAAG CCGATCTACG CGACCGTCAT 91440
GGGCGCGTGT TCAAGGACTC GCGAGGACGC AACGCCAAGT TCATGACGTT TTCCGCGGCG 91500
CTTGATCTGC TCGCGCAGTA CGTCACAGAT GATGGGCATG GTTATGTGAT GATCATCGAT 91560
GTGAAAGGGG CGGTTGACGA TCAGGATCCC ACTGATCCCA TCGAGCTCAC GCAGCGTTGC 91620
CTTGATATTC TCGCGGCCAA AAAGAACCCA AAGCTCAGCA AGGCTGTGGT GTTTAAGCTG 91680
AAGGCTAAGG ATGCGGTGGA TATTGGTACG TTTCTGAATC GGACCACCTA CGATCCCAAC 91740

GTCATGGGCG GGTTGATCGT AGTTGAAAAT CCGGACGATC AGAACGTTAA GGATTCCAAC 91800
TATGATCCTC ACGAAGATAC AATTTACGAT CAGTGGAATG TGGCGCCGTT CCCCGTCCAG 91860
TTTGAAATGA ACCAATTCTA CAAGGGTGAC GGTCTTCAGG CGTATCTCGA CTATGTCGAT 91920
CAAAAGCAGG GCTTCGCCAC CTATCATGAA AGTAATTATT ATCCGGAGGG CGTCGCCAAC 91980
AGCGCGGGAA AATGTTGTTT TGAGCATAAC ACCGATCCCA GATCCGTCGC TCACGGAGGT 92040
ATAGTGCCGG ACTATCGCGG CGAACCGGAA ATGGCGATCG TCAATCGTAC GAATCTGATC 92100
ACCACTGACT GGCCCGACGT GGTTGCCGAC ATGCTGCGCC AGTTGGGGCG CCGCAACACC 92160
AGCAAACTGA GCCGCTAAGG TAAGACCTAC CGCTTAGAGT GGAATCTTCC AATGATAACT 92220
GGAAAGACAA TCTCTCTACC ACTCTCTGTA ATAGCGTGTC TTGGTGCTGG CGCAAGCCAA 92280
GCTGCCGCAT GCGCCAATCA GACTGACTGC CTTAAAAGCG CCTCGGCAAA AGTGTCTCAG 92340
ACACTCACCC CCAAGGTAGT GCCTGTTTTT GGCGATTATC CGAAGCTGTA TATACACGAC 92400
TCTGCTGCGA ATGAGGATTA TACGGTGCCT GATACTACAA AGCAGGAATT TGACTTTTAT 92460
ACGAATAAGA CTTCATTTAA CCTCCCGCGT CGCTCTTCAA TCGAGACAAG AGAGATCGCC 92520
TCGATCAGCC CGGCCATGGG GTTCACTGTC CTTTTGGTCG AAAAGACCCG CGATAATACG 92580
GTTTCCAATG GCGGTAGCAC ACTCTCGCTA TTAAGTTCCA ATTCCAACGA CGCTTACTTG 92640
TCATTCGCTT TAGGCGCCAA GCCCAGCTCA AGTGGGCAAA AGTGGTCTTT TGCCAAAAGC 92700
TTGCTTCAAG GCAACAAGGC GCAAACCGCA TATTGGCAGA AGCCGTGGGA TATGAATCTT 92760
CTGGGACCAA CAGGCGACTT CTCCGGAACC GAGTGGATCT ATTACACCTT CACTCCAGAC 92820
GGCAAGGTTC GCATCGACCG TTTCGCGCCG TACACCTATT TTCTGGCGTT TACCGCTTAC 92880
CAGTGGGATG AGGCCGTGCT TGACGGTGGC TTCCCGCATT TTCCCTTCGA CACCGGTACG 92940
CCGACCAACC GGCCGCAGTC CGTCACTTTC GGATCCGTCG GGCCATGGCT TATTGGCGCT 93000
GCTGGTGCGC CCGGACAAAC CCCGCCGACG AGCGAGCCAA TAGAGGCATT GCCCGGCTTT 93060
GAGGGAGCGA CAATATTCTC AGCGCCCTTG TCGATGAAGG AAGTTATAGC TTACCAGAAT 93120
TCGCTGAGGG GAAGTTATTT TCTCGACGTG GACATGCTTC CCTGCAACAG CGGCCAGTAC 93180
CTTTCCGGTC GAATTAGTAC TCCCTGCGGT ACAGGTAGTC CCGGCAATCC GCCACCGAAC 93240
ATTAACTCTG TCGCTCAGCA TAGGATCAGC ACCAATACCA ATCGTTAGGT CGTGGCAAGC 93300
AAAACTTTGG CCTTGCCTCA ACGACGATTG AAAAGATTTT CAAGGCAAGG AGGTCGATCC 93360
GATGACCAGC AAACTCGGGC CAGGCCGCGA CTCCGGCTTT GCCCGCCGGA TGCTTCGACC 93420
AATGCTAATC AGAAGAGCTC GCTGACGAGC GCCGCGGAGG TTTGCTGACG CACCGCGACA 93480
CGGGCCTCAG GGCATTGGCT GCGGATGGTC TCCTCAACCT GCCAGAGCTT CGCCATCCGC 93540
TCGACCGTCG CTGTTGCGAC CTTCGAGCCT TCCGCGACAG TGCGCAGCGT TTCGGCGGTG 93600
GCGCGAAAGA GCAGGCCGTA GACGAGCGCC TTGTTCTGGT AGGCGATCGC GGCGATGGCC 93660
TCCGGCAGTG TGAAGACGAC ATGAAAGTAC TGTGTGTCGA GCAACTCGGC GCGGCGATCC 93720
TCCAGCCATT GCGCACGGGC GAGCGATTGG CAGCGGGGAC AATGCCTGTC ACGGCAGCGG 93780
TTGAAGGCGA TGCGCCGGTA GCCCGCATTG ATCGCACGCT TCGACGTGCC CGCCGAGCGC 93840
GGCGGTGGGG CACAACTCGA TCGCCGTCAT GACGCGACGC TGAGCGGTCG ACAGCGACGT 93900
GTGCTGGGCA CGATAGGACT CGCCATAGCG ACGGAATATA TCCGCCACTT CCGGCCTCGA 93960
ACGGGGCATG GGCGCGAGTT CAGAAGTACT GAGGCTTGGA GGGCGGCGGG GTGGGCGCTG 94020
GACGTGGCAA AAGCTCGAAG GGGCTTTCCG TAGCGCAGAC CTTGTTGGTG GCGATGCGCA 94080
GGTAGTGGGC GGTGGTTGCG AGGCTGCGGT GACCGAGCAG CGGCTGAATG GTGCGCACGT 94140
CGGCGCCGGC CTCCAGGAGA TGGACGACGA AGGCATGCCG CAAGCTGTGC GGTGTCACCG 94200
GTTTCGACAA GCGGGAGAGA TCGTGTGGCT TCACGCAGGC TTGCCCAACT GCATACCGAG 94260
TAATTGGGTG ACCGGCGCGA TCTCCAGGGA AGAGCCACGC CTCTGGCCGC CACATCCTCC 94320
AATAGTCGCG AAGGATCTCG AGGAGCTTGG GCGACAGCAT CACATAGCGG TCCTTTTGGC 94380
CTTTACCATG CTCGACACGC ACAACCATTC TCTGGCTGTC GATATCCGTA GGCTTCAGCT 94440
GCACCGCTTC CGAAATGCGC AGGCCGGCGG CATAGCACGT CGTCAGGATG GCGTGGTGTT 94500
TGACATCGGC CACGCAGCCA AGAAAGTGCT GTACTTCATC GGGACTGAGG ATGATCGGCA 94560
GCTTTTGCGG TTTCTTCGGA AGCGGCAATA CCTCTTCAGG CGCCCAGTCC CTCTCGAGCG 94620
TGACGTTGAA GAAAAAGCGC AACGCCGAAA GGGCGATGTG GATCGAGCCG GGCGCCAACT 94680
TCTTCTCGTT GGCCAGATAG ACCTGATAGG TCCGAATATC CTCGCGGCCG AGCAAGTCCG 94740
GCGCCTTGCC GAAGTGTCGG GGTCATGATG AGCTCCTCTG TCTGATGGGA TAGGCTGCCA 94800
AACAGCCGTC TCATCCTCTC GCAGTTGGGG CTCCTACTGA ATACATGACC TCCTTGCCGC 94860
TCCGCGGTAG CGGAGCGGGT TAGTCCAACC CTATATATGT GTCGGATGAG ATTATGTGGC 94920
GGCGTCGCTC GCAGTTGCTG CGTTTCCCGG CCGGGTGCCC GCTCGGCCGC CACATAATAG 94980
TTCCGTCTCA GATGGCCTCC AGCAATCGCA GCAAGGCATC CTTCGGCTGG AACCGAGCCG 95040
GCTGGCTGCC GGCAACGCCC GCCTTGTCCA AGCCGCGCCG CATCATCTCG ACGTCGGCTG 95100
CCGCGTAGCG ATGCGTCGTG GCCACCTGCG CGTGGCCTAG CCAGGCCTGG ATGGTCAGCA 95160
GGTCCACACC GGATTGCAGA AGCGTCATGG CCAGCGTGTG ACGCCTATGT TTCTGTCGAC 95220
ATAGGCGCCA CTATCTACCC TGCCGGACTA TGTCGGGTAG CTTTGTTCGC AACGGTAAAT 95280
CTCTGAGGGA TAAGGATATT TCCTACAGAG CCGCTCAGGG CACCCGACAT AGTCAACCCG 95340
GTTCACAGGC TGTTCAAGAA CCCCAACAAA TTGTCATCGG GTCGAAAGCG GGATGACTTG 95400
CCGGAATATG GGGAGGTCTT TGCCAAGGCT GCCTCCTTCA TCGCCAATGT CGCCTCGAGA 95460
TAAATCTGTG TGGTCTCGAC GGATTCATGG CCGAGGCACA AGGCGATTAC GGCCCGGTCT 95520
ACGCCCGCCT GCAGGAGGTC CATGGCCATT GTGTGCCTCG GCCTCTGGCA CTGGGAAACA 95580
GGATACTCTG GTCTCCGCGC TGCGGCTCGT TCAGCGAGTT TCTCAACACT GCGATTGTAG 95640
ACTTGGCGAA CGGAGTGCAG CGTTCCCCGC GCAGGTGGGC CCCCGTGCCG AAGAACAGGC 95700
GGTTGGTGTG AGGCGAAATC GAGCGACGCG CGAGTTGCGG GTAACGTTCC GCAGCGGCCG 95760
AGACCGCAAG TTCAAGGCGC TGGGTGACGT CCGAACGTGC CATGCGCCCT CCGCCCCGGT 95820
TCGGAAACAG GAAGTTCTCC TCGCCCGCGC AGTCCAACCG GCGTTTCCAG TTCCGGATCA 95880

GGTTGGCGGT CGTACGCCAG AGCGGCACGC TGCGGTCTTT GCGCCCCTTG CCATGCAGGT 95940
GCGCGACCGC CGAGCCATCG ACGACGACCT CGCCGACGCG CAGACCGATG GCTTCAGACA 96000
CGCGGGCGCC GGTGTTATAC ATCATGCTGA ACAGCGCGCG TCCCGCTCAC CCGCCCAGGT 96060
CCGCGCATAC GGCGCGTCAA AGATCGCCTG CATCATGATG ATCTCTTGAG ACGTCGCGGC 96120
GCTCCGACGA TTATCCGTGA CACGTATCCA AGGTCGTTCA CAATCCAGAG AACTGCCGTC 96180
GCGGCGCCAA AAATCGCCGC TCCAATCGCA CTTGCAACGA CAATTCTGGT CTTGCCGTCC 96240
CAAAGTTCTC CGAATTGTAT CAAGTCCGTT AAGCGAAAGA CGGCGCCCTG AACTAAGTAA 96300
AGCAGCAAAG TACTTTGACC AAGCTCCACC GCAACAAGGC GAGCTAATCG AGTCGAGCAT 96360
AGAACCCTCC AACAGTGAAA CAAAGATTGC GCCGCCACTG CTGCAGCGGC TGCAGACCCG 96420
GTGAACATCA GCAAGACCTG TTTAGCCGAT TGTGCATCAT GAACTAAAAC AAGATTGTTG 96480
TAAGCGTAAG TGTCCCTCCC CCAATCAAGA AAGCACAGGC AAGCCACTGC CCAGCATGAG 96540
AACATCAAAA GAGATTTGTG ACGTAGCATG GTGTTCGCCT GCTCCTCGAT CGTCTGGGCA 96600
AACAAGAACC CCAAACAGAA GAACGGATAG GTATATCTCA GCAATGGTAC TATCGATAAT 96660
GTGATAGGCA TAAGTGCGAT CACAATTGCA GACGCGCACA GGATCCATTT CGATAAAAGG 96720
TTGAAAGCCG CAAAGAGCTT TGTGAGAAGG AAAGAAGCAA GTACTGCCCA CAAGAACCAA 96780
TATGTGCCAA CCAATTCACG TGAAAAGTCC AGAAGTCCGG CAGTCACGCC GCTGAAGTGA 96840
AAAAACGCGG CTAGCTTGGC TGTTTCAATA AGTGCGCACC AAAACAGCAT TGGAATCAGT 96900
AATTGAACCG CACGATCACC GACGGCGCGA CAAAACGATG TTCGCAGAAT TGTTCCAGAA 96960
GCAAGATATC CACTTATGGC CATAAAGAGG GGCATGTGAA ACATATAGAT CGCCTTAAAA 97020
TACGGCGACA ACCAAAAGTT ATCTGTGTTT CGATAGATCA CCAATTGTAA TAGATGACCC 97080
AATACTACCA AAATTATGAG AATGCCTTTG ACAAAATCAA AGGTTATATC GCGATTGTTC 97140
GCCCCTGCGG AGCACGAACC GCGCTCCACA GGGGGGACTG CGGCGTACCC CATGACCAGC 97200
TCCTAAATTT TCATCCAGCG ACGGTTATGC ATTCTCGAAT AGACGATCGG GAGACTTGGG 97260
ACAGGAGAAA ATCAGTTGCT GCTCAACGTC CGTCTGCCAC CGGCGATGAC ATCTGACTTG 97320
GCTCCCTGCA GATCCACCAG ATAATCAATC TTGTTTAGAT TAGGCCTTCG GCTCAACTTA 97380
GATTGCTTCT TGGATGGTCT ACTAACTGCC CTCAATGAAG TGACCTTACA GGCCGGCTCT 97440
TCCAATCCGC TTTCTTATCC GCTCTTTCAA AATTGGTGCA ATCAATTGTT TGGATGAACC 97500
GCATTTACAT GATGGATTGA TATCGGACTT TCTAACCGCC AACGAGTCCG CCGTGAAGAC 97560
GCGCTTGGCC GCAACTGTTC CGGCACAAAA GATCGTTCAA AATGTAACCC GGTATGAGCT 97620
CCCACGTCTC CGGTGATCAG CGGGCGCGTG CGTAGCGGAT CGGCATCCAG TATTGGCGCA 97680
TGGCGTCGAC GGCCGCTGGA ATAAAGGCGT AGGCGTTACG GAGAAAGTCC CCGGTCTCGG 97740
AGCCCCTTCG TGGCGGTTCG GGCAGCGGAC GGCCCCGATC GTCGCGACAG TGCAGCAGGA 97800
TGGGCAGAAG CAGGCTGTGA TTGACGTTAC CAGCGTCCAG CAACGGCGCC CACGCCGGCA 97860
ACCTGAGCCG CGTCGCGGCG TAGAATCCCT GACACCAGGG GCACGGATCA ACATCACCAT 97920
GGGTTTGCGC CGGTGCATCG GCTCGAACTG GTCGGGCGCG GTCGTAAGAC GTTGCTGATG 97980
TCGTTATGGC GCAGCGCGAC GGCGAAGATC GCTGCGAACT CCGGGGTATC GCCGTGGTTG 98040
AAAGCATCGG CATCGATGGC GAGCAGCGGG CAGATCCAGT CGAGCGGACT CATCGACACC 98100
GGTCCGGCCA CGATCGCGGC AACGTAGCCG TCGAGCATGG CGAGACTGGT GGCGATAGGC 98160
TGCCGATCAA CGCGAGCCTG CAGCCATCGC TCGAGTTCCT CAAGCGGCAT CGCGGCGGCC 98220
GCCATCGACG ACGATGCTGA TGAATGGCGA TGTCGGCTTA TGCGGCGGTC TGTGCGATGC 98280
GCTCGCGGGC CGCCTTCCAG TTCCAGGCGA GCAGCTCGTG CAGCTGATGG TCGTCCTGGT 98340
CATCTCCGAC GAAATCTATG AACATGGCGA GAGGATTGGC GAAGATCCCC GAACTCCAAT 98400
TCACCCCTCC GGACGGTGCC TTCTACGCCT TCATCGGCGT TGGCAATCTC ATTGGTCGCA 98460
AGACCCCGGA CGGAACCGTT CTCGCTGATG ATACAGCTAT CGCAGCCTAT CTCCTCAACG 98520
AATTCGGTCT GTCCAGCGTT CCGGGTGCTG CCTTCGGCTC GCCTTCGTTC ATCCGCCTGT 98580
CGATTGCCGC TTCGGACGCG GAACTCGAAA GCGCGTGTGA ACGTCTCGCC AAGCTGGTTG 98640
CGTCGCTTAG CAAATAGCAC TCGAGGACTA AGGATTGCAT CCGATGAACA GATACGAAGT 98700
TGCCCTTGTC ACAGGGGCCT CGAGCGGTAT TGGGAAGGCA ATCGCGCTTG AGCTTGCCTC 98760
GGCCGGGCTC AGGGTCCTTG CTCTTGGCAG GGACCGTGCT GCGCTTGACG AGCTTCACTC 98820
GACCGCGGGC ATCGTGCCCG TCGTGTTCGA TCTCTCTGAC GTTTCCGAAG TCTATGGGAA 98880
AATCGCGGGG GAAAAGATCG ACGTGCTGGT GAACAACGCC GGATTACTCA CCGCCTCGGC 98940
AAGCCTCGTC GACCTGTCTG AAGACGATAT TGACGCAATG ATCGACATCA ACATTCGGTC 99000
GGTCTTCAAG CTGACGCGGC ATGTCCTGAA GCAGATGATC GAACGTCGCC GCGGCCATAT 99060
CTTTTTCACC GGTTCAAGCG GCGGCCTTGC CCCTCATCCC AACTCTTCCG TTTACGGCGC 99120
GACGAAGGCG GCCGTCAGCC TGTTTTCATC TGCACTGCGC TGCGATCTGA TCGGTCTACC 99180
GATCAGGGTG ACGGAGCTTT TTCCCGGCCG CGTTGAGACG AACCTCTATC GGACAGCGCT 99240
CGGCAAAGAG GGCGCAAAGA AGAAGCTCTA CGACGACAAT GAGGCGATCC AGCCCGAACA 99300
CATGGCGCGA CTGCTGCGTA CGGCGCTGGA GTTGCCGGAC TTCGTCGACG TAACCCGCCT 99360
GGAAGTGATG CCGACGGGCC AAGTAGTCGG CGGCGCCCAG ATGTCCAAAC TTTCTCGTTA 99420
ACTGCGAACC TTGGAAGGAA AAGATCATGC AGGGTATGAA CATCGCCGTC GTCGGTGGTG 99480
CGAGCGGCTT CGGCCTCGAA ACGGCAAAGC TCATGGTCCG TGACGGCGTG TCGAAGATCG 99540
TCGTCATCGA CATCAATCAG GAGCGTCTTG ATGCAGCGAA GGGAGAGCTG GCCGGAAAAG 99600
GCACCGAGAT TTTCACGGTC GTCCGCGACA TCGCACGGGC AGAAGGCGCG CACGCTTCCT 99660
TCGACGCTGC GGTCAAGACC GCAGGGCGCA TCCGCTCGTC AATTTGCGCA GCGATCTATC 99720
CGCGCGCACC GCTGCTCGAA ATCACCGACG ATATGTGGGA CGCTGAAAAT GCTATCAACA 99780
TCAAGGGCAC CTATCACATG ATGGTGGCGG CGGTGAAGCA CATGCAGGGC CACAAGGCCG 99840
AGGGCGAAGT TTCCGGACGC ATCGTCAACA TTACCTCTGT CGACGCTTTC AAGGCGCACC 99900
CGCAGAACGC ACACTACGCC GCGACCAAGG CCGCGGTCGT CAGCTTGACC CGGTCCTTTG 99960
CGCATGCGTT CGCCAAGGAT CAGGTTCTGA TCAACTCCGT CGCGCCGGCG GGCATGGCGA 100020

CCGAAAAAGC CAAATCTCTT GGCTTCCTCG GCGAGCTTGC CGCGGCCAAT CCGTTGGGCC 100080
GGGCTGCCGA ACCAGCAGAA ATTGCAGAAT TCGTAGTGAT GGTCGCGGGC CCGAAGAATA 100140
CATACATGAC CGGTGAACAA GTGATCGTCT TGGGTGGTTA CATCTACGCC TGATGGCGTT 100200
CGGGTCGCGC CTGGCCTTCT GGCGCGACCC CATAGGAATC TAGATAGCAA GAAAGTGAAG 100260
GCGGAGACGA TGGGAAACGG CTTACAGGAG CGAAGCGGGG GCCTGATCCT TGTCCACGCC 100320
CTCAGGATCC ACGGTGTGGA ACGCGTCTTC GGCGTGCCCG GCGAGGGCTA CCTGGCGGCG 100380
CTTGACGCCT TCCACGATGC CGAGCGTGCG ATCGAGTTCG TCATTTGCCG GCAGGAGGGC 100440
GGGGCGGCCT ATGGCAAACT CACCGGCAAG CCAGGCATCT GCTTCGTCAC CCGTGGGCCC 100500
GGCGCCACCA ATGCTTCCGT TGGTGTGCAT ACCGCCTTCC AGGATTCAAC GCCGATGCTC 100560
CTCTTCATCA GCCAGGTGGC GCGTGAACAG ACGGAGCGCG AGGCCTTTCA GGAGATCGAC 100620
TATCGCCGGA TGTTCGGCCA GATGGCAAAA TGGGTAGTGG AGATCGAGGA TGCTGCTCGC 100680
ACCCCGGAAC TCGTCAGCCA GGCGTTCCAT CGCGCCCGCC AATGGCCGGC CGGGACCCGT 100740
CGTCATCGCG CTGCCGGAGG ACATGTTGAC GAATCGCGTG ACGGTCGCCG ATACACCCGC 100800
TTACAAGCGC GTCGAGACCT ATGCCGGTCA GCCGCAACTC GATGAGCTGA CCGAACTGCT 100860
AGCGAAAGCC AAGCATCCGT TGGTCGTCGC CGGTGGTGGC GGCTGGACGC AGCAGGCTGT 100920
CGCCGACCTG CGTTCCTTCT CGGAAGGTTT CTCACTGCCG GTTGCTGCCT CGTTCCGCTG 100980
TCAGGACTAT TTCAACAATA TACATGCTAA TTATGCCGGT GACCTCGGCA TTGCGGCCGG 101040
GCCGAAACTG ATTCGACACA TGAAGGACTG CGACCTGCTG ATCGCAATTG GCGCCCGTCT 101100
CGGCGAAATG ACGACCGGCG CCTATTCGCT CATTGATATC CCGGTGCGGA AGCAGACCCT 101160
GGTCCATATT CACCCGGGCG CAGAAGAGTT GGGCCGCGTT TATCACGCGA CCCTGCCGAT 101220
CAATGCCAGC GCCGTGGGCT TCCTGTCGCA GGCGACCGGG CTCGAACCGG CTGTTGCGCC 101280
AGTCTGGGGT GATTGGACGA AGGCTGCCGA TGCCGACTAT CTGAAGAAAC CGCAGGTGCC 101340
CGGCCCCGTC CAAATGGGCG AGGTGATGGC ATGGCTGCGT GCTCATCTGC CTGACGATGC 101400
CATTCTGACC ATGGGAGCCG GGAATTACAC TGCCTGGGCG CATCGCTTCT ACCAGTACCG 101460
CACGTTCCGC ACCCAGCTCG GCCCTACCAA CGGCTCCATG GGCTACGGCG TGCCGGCCGC 101520
GATTGCGGCC AAAATTACCG CGCCGGCGAG GACGGTCGTG GCCTTTGCCG GCGACGGCTG 101580
CTTCCTAATG AACGGACAGG AGCTGGCGAC GGCCATGCAA TACGACGTTC GCGTCATCTT 101640
CCTCGTCATC AATAATGGCA TGTACGGCAC AATCCGCATG CACCAGGAGC GGAGCTATCG 101700
GGGCCGCGTT TGCGGAACGC GGCCCGCAAT CCCGACTTCG CCGATCTTGC CCGCTCCTAC 101760
GGGTTGCACA GCGAGACTGT GAAGGCGACC GAAGATTTCG TCGACGCCTT TCGCCGCTCG 101820
GAAGCCTCGG GCAAGCCGGC GCTAATCGAG ATCCGCATCG ATCCGGAAGC GCTGACGCCC 101880
AACCCTGACG CAGATCCGTG AATAGGCAAT CGCGTCGGGG AGATATCAGC AAGGGCGGCG 101940
GCGTTACTCC TCCACTTCGT CTAGCATTGC GCGAAAGCGA TCCAGGATGC GCTACCAGCG 102000
CCCGCTCCGA ACGCTGGTAG CGTTCGAACA GCTCAGTGGA GAAATGCCCG GAGCGATCCT 102060
GCGGCAACCG CAGTTCCAGT TTGCCGACCC GCGTCATCAG CGTCCGACCG TAGTAGCCGG 102120
AGCGGTAGCC GAGCCGCTCT GGCGTGCGCT CGCCTTTCCC CGCCCCCAGG GCCTCGTCCA 102180
TCTCCGCTTC GAGCACCTCC TGCATGACCG CGCGGACTAC CTCGCGCAGC CCATCCGGGT 102240
TCGAAAGCAG AATGTCTTTG ACCGGAGTGC TGGCGGTCTT ACCTTCAATC TTGGTCATGG 102300
TAGCGTTCCT CGTGAGTGAA TCAGGTGACT TCGACATCAC CAGCCTGCCA TGACCGCCCC 102360
TCGCAGAGAA TTTGCAGAAC CTTCGGCACA CTACCGCGGT GTTCGTACCC GTGACTACAG 102420
TGTTCCGCGC AATGAACCTA TCGTTGCTCC GAACGAAACA GTGCGAATCG CCGTGCTGAA 102480
GGATCGGTTA GCTGACCCTT CATTTCCCCC CGAGCCTTTT TGATTATTCT TTCAGGAGAG 102540
CGTACCGAAG CACGCGAAGG TCTTCGTCTT CGAGTGAGAT CTCCAGGACC CGATTGACGA 102600
GATTGTCGTC ATTGTCGAGT TCGATCAACG CCTGCTCAAA CAGCTCGATC TGCATGTCGA 102660
GGTCCATGCC GGGTGGCCGT TAAGGCAGAT CAGCCCGGCA TGGATGGCGA CGTCGGCATA 102720
TTGCCCCTTG GTGCCCGGCT TGTCTTTTGG TCCCCGGAAA TCGACCGAAT TCTTCGTGAC 102780
AAACGTCCAG TCGCCCTCAA GGATGATTGG TTTAAGCTCC CAGTCCTTCA GGCCGCCCAG 102840
TTTCATCCAG ACGACATGGC TTGTTTCGCC GTGGCCTTTT TCGATAGCCA TTTTTGCCAG 102900
TTCAGGACTG AGACACTCAT CGATGAGAAA CTTCATCCCG TCTTCCTGCG ACGGGCGACG 102960
CGACGATCTG AGACGATGAC CGAGCCTTCG GGAAGGTCGC CGAAGACGCG GCGCCGGCCG 103020
CGCAAGGGGT TCGCTTCCGC GTAGATTGAG GCAAGCCTTA TCTTTTCCGC GTCCAGGCTA 103080
GGCCAGGCCT CCAGCATGCG CTCCACCGAA TGACCGGCAG CTACGGATGC GGCAACATCA 103140
TAGACCGGAA CGCGGGTGCC CCGGACGACA GGCGTACCGC CAAGGATGTC GGGCGAGGAT 103200
GTGACGATCT CTCGTGCTGC GGCAAGGTCG TCCAGTCGTT CGCTCGCACC TCTCATAAAG 103260
GGCATAAGAT CGATCGTCAG AAACTCGTGG TGCACGGTCC AGTCCTCGCG CAACAGGGCG 103320
GACCAAGGGA GCGTACGCGC TCTTGCCAGT CGACCCTCTG CTATCCGGAT GGCAAACAGC 103380
CGCTCTTCCG AGGTGAGACG GCGGGCGCTC TCGAAATAGA AGGCGATGAG GGAGCAGGCC 103440
CCGGCCAGGA CATGGCGACC GTTGTCAAGC GACACGAAGG CTTCTGGCAG AATATGCTCA 103500
TCGATGACGC GATTGACGTC GCGTAGGCTC ACGCGCGAGA CGACGGCAGC CTCGCTCGCC 103560
TTCAGCATTT CCGCAACTGC GGGCATGTGG GCCTCCATAT AATGCTCCGA TCCGTCGGAA 103620
TATATAATGT GCAGGTGGTC ATATCAAGGT CTTGAAGACG AATGGGTAGG CATCGCCTGA 103680
TGGTGGGGCG CGTGCGACAC TCGATCGACC GGCGGGAAGA GGGGTCGTTT TCACCGCCCC 103740
TGATCCCCGC GCCGCAATCC AAATGGCAAT GGTGCTTGCT GTTAAATATT TTGACGGGGT 103800
TTCTTCAGGC AGCCTTCATT GTTTCCGATG CGCTGCCGCT GTTCTCACCA AGATCGAACC 103860
GGTCGACGAG TTGCATCAGG TAGTCCGCCT CGCCCGACAG CGTCCAGCTC GCGGCTTGCG 103920
TCTCCTCGGC CATGGCCGCG TTGCTTTGCG TCAGCTGATC AAGCCGCGTG ATTGCAACGT 103980
TCACCTCGGC GATCGACGAA GCCTGCTCCC GGCTCGACAG CGCGATTGCT TCGATCTGGG 104040
TCGAGATCTC GAGGATGTGC TTGGAGATAT CCAGCAACGC AGCACCGGTT TCGGAGACGA 104100
GGCCGGCGCC CTTGGCGACT TCGGCCGTGG AGTTTCCGAT TAACGCGGCA ATCTCCCGAG 104160

CAGCCCCGGC GGACCGCTGC GCGAGTTCCC GCACCTCCTG CGCGACCACG GCAAAACCTT 104220
TGCCGGCCTC GCCGGCGCGC GCCGCCTCGA TGCCCGCGTT GAGCGCCAGC AGATTGATCT 104280
GGAATGCGAT GTCGTCGATG ACCTCGATGA TGTGGCCGAT ATGGCTGGAG GCGGCCTCAA 104340
TCCGCCCCAT GGCCGCAGTC GCATTGCCCA CGACCACGGT GGAGCGATCG GCGTCTTGCT 104400
TGGCCTTGGA GACCGTATTG TCGGCATTGG CCGCCTGGTC GCCTGCCCTG CGGATGGTGG 104460
CGACGATCTC ATCAACGCTT GCGGCTGTTT CTTCGAGCGA CGCGGCCTGC TGCTCGGTGC 104520
GACCGGAGAG CGCCTTGGCG CCCTCGGCCA TCTGACGGCC GCTTCCCTGG ATGACGGACG 104580
ACGTCGTGCG GATGTCGAGG AGGGTTTGCC TGAGGCCCGC GACGGCGGCG TTGAAATCGG 104640
TGCGCAGTTT GTCAAGCGCC GGGGCGAAGG GTGTCTCGAT CGTTGAGGTC AGCCGGCCCT 104700
TTGCCATGGC GTCGAGCCCA TCTGCCAGTG CGTCGACTGC GAAGGCGATT TCCTGGTCGC 104760
GGCGCAACTT TTCCGCCTCG TTTCGCTCAC GCTCGGCATG GGCGGCGACG CGCTCCTGCT 104820
CGGACCGCTG TTCGAGCGCG ATCCTCGCCT TCGCATTGGT CTTGAAGACG TCGAGGGCAC 104880
GCGCCATCTC GCCGATCTCG TCGGCGCGGT TCCGGTTAGC GACAGCCGTG TCGAGCAGGC 104940
CACCGGCCAG ATTGCGCATC GTCTGGGTGA TCTCCCCGAT CGGCCCCTTC AGCGTGAGCA 105000
CCAGCGCCGC GCCGGAAACG AGAGCGATGA TCACGCCCAG AACCATGGCG CCGATGGACA 105060
GCCTATTGGC ATCCTGGCTC TGGCGCCCGG CGGCGCTCCG TTGCAGGTCG GCGAACAGCG 105120
ATATCTGCTC GCCGATATGG TCGATCTCGT CGGCAGCGGC CTGGAATTGC TGTGTGCGCT 105180
TGCCGGTGGC GCCGACAAGT TCGACGGCGG ACTGTTCAAT CCGGTCGAAG GAGGTTGCGA 105240
GCTTGGTCGA CTGCTCCCCG GCGAAGCCGA GATCGCCCGC CGTGGAGCTC AGCGCAAAGA 105300
GCGCGTTGCG GCATTGCGCG AGGTGCATTG CCAATGTCGA CTGCTTTTCT GCGGAACGGT 105360
CGAGCATGAA TTCGGCAAGT GCGATGCGCG TCAGGTAGAT GGCATCGGTG AGATGCCGCG 105420
TGTCCTGCAT TACGAGCCCG GCCTTGGCAA CCGAGGCATC GAGCTCCTCG AAAGTCGTCG 105480
ACACATGCTG CATCTTCGCG GTCGCCGCCG CCTCCAACGG CTCCTTAAGC GCTTTGAGCT 105540
CAGCGAAGAC ATCCAGGATT TTCTCTATGT TTTCCGGCGG GCGCGCCTTG TCATCCACCA 105600
GCGCACTCAA GGCCCTGACG TTGGCCGAAA AGGCCTTGCC CAGCGGCTTG GATTTCTTCG 105660
GCAACGCGAT CGGTAGCAGC TTCTGGTGAA TCTTCAGGTC GCCCATGGCT GCGGCGATCG 105720
CCCGATAGCC CTCGCCATCG TCCTTGCGCG CGGCAAATGC ATCTCTGAGC GCAACGACAA 105780
AGCTTGTCGC AGAGAGGATC CTTTGCGCGT CACGCAGTTT CTCCTTGGCG TCGTCTTCCT 105840
GCGTGGAGAT CGACTTGTCG AACGCGGTGG CGGCGGTCAA GAGATCCGCC TGCGATGTCG 105900
AGATGACCCC CAAGGCCTCC TTGATGCCGG CTTCGAGGCG GGCTTCATCG GCATGCAGAG 105960
CCCAAAGGTC GTCCATTCGG CCGTTGATCG CAGAAAGCGC CGCGGATGCG CTTTCGAGCT 106020
CCGCCCAGCC TTCGGCCTTC GGCGTCAGGC GTGCGCCGGC AGCGTCGAGC GCGGCGCGCT 106080
GCTCGTCGAG CTTGCTGAAG ACGAGCGCCC GGTTTTCTTC GGTCGTCTGG TCGAGAAAGC 106140
CGATCATTGC CGCATAGACA TCCTTGAAGC CGCGCAGCGA TGCCGCGACG TCGTTGGATA 106200
TCGCCATGCG GCTTTGCAGA AGCCCGGATG CGAGCATGCC AGTCACCCCG ACCGCGCTGA 106260
TGCTCAGGAT GAAGGGCAAC ACCAGCAGCA TCACCTTCGT TCGGATTTTG AAACGCGAGA 106320
GGAGGCGATC GACCCAGCCC ATGAGATGTC CCGAAATTTT GTTGAGGTGG CAGCCGCGTG 106380
CAGCGCAGCA TCACGGCATC GATAGGCGGC GAGGGATAAC AAATCGATAA TGAAGCGGCG 106440
ATCACATGCA AATGTCACAA AACATTCACA CAAGCTAATT TGTATTACTT TTCAATCGAC 106500
TGGGGGATAC TGCAAACCGC GAGCAATCCT TTCCGCTATT GTTGGAACAC CAGTCGGTTC 106560
GTCACGTCGC TGTTGTTTCC GTGCTGTAGT TTTTCGCCAC AGTTGAAGAG GGGCTACCAA 106620
TGGAAATCTC CAAATTTGCG GACGAACAAC TGGCCGATCA GATCGCCGCA GGCCGCGAGG 106680
CTGTGCCCGA AGTTGATCCC ATACGGTTGA TTGAGCAGGT CGTCCATCGG GCGCGCGCTG 106740
GGGCCGGGGT GGATTTCGAC GTCTTTGAGT CCCTGATCGG CGAAATCGAT CTCGTCGAAA 106800
TCAACTATCT CGAGCGTGGT TTGATGGCAT CAAGGGCGGT GTGCAGGATC AATGTTCCGG 106860
CACCCGTTGG CGACAGTAGC GACTGGGGGA CAGGGTTCTT GATCGGGCCG CGGCTCTTGT 106920
TGACCAACAA TCACGTCATC GACTCCGCTG AAGACGCTCT CAAGGCTACG GTCGAATTCG 106980
GCTACGAACT CGACGCCGAA GGGCGGCTGA AACGCACGAC ACGCTTTCGG CTTTCCCCGC 107040
AAGATGGTTT TGTAACGAGC CCGAGGGATG CGCTCGATTA CACGGTCGTT GCGATCGAGG 107100
AAAACAGCGA GGATGGCGCG ACGCCAATCT CCGACTTCGG TTTCTTGCGT CTCGACGCGC 107160
GCACCGGAAA GACGGAGGTC GGCCAGTATG CGACCATCAT TCAACATCCC GATGCCAAGA 107220
CGAAGCGCAT CTCGCTCAGA GAAAACAAGT TCGTCAAATA TGGGGACGAG GCGGACCCGG 107280
AGCGCGACAA TTTCCTCTGG TACTCTTCCG ATACGGCAAA TGGTTCGTCT GGCGCAGCCG 107340
TCTTCACGGA TGCCTGGCAG GTCGTTTGCC TGCATCACGC AGGCGTTCCA GACCGAAGGA 107400
TGATCGATGG CAAGGAACAC TGGGTGCTGA CCGACAAGAC AACGGCGCCC GCTCGCATCG 107460
CCATCAATCT GCCCGTCGAC AAAGTTCACT GGCTCGCGAA TGAAGGCGTT CGCATTTCCA 107520
AGCTCATCGC CGACGTTCAA GAGAGAATTG CGGCGCCAGG CTTTGCCCGT TCCACCCTGG 107580
TCGATGACCT TGTTGCCGAT GCGACCGGGG TGAAAGCCTT TGCCGGCACG ACGCCAGGCG 107640
AGAGCATCGT CGGCCCGCCG CTCGTCGCCG CACCGGCCCT CAGGCTTGTT GCCGCCGAGG 107700
CACGACGCCG GCCGACGCGG CATACGCACC CGGCCAATTA TTTCGACGGA CGCGAAGGAT 107760
ATCGATCGGA TTTCCTGCCC GTCGAAATCC CGCTGCCCAC ACTCGGGGTC AATGCATTGC 107820
GGCATGGTTC GCCGGCCAAG GTCACCGGTG CAGCCGACGA CGTCCTGCGC TACCAGCATT 107880
TCTCCGTGGT CCTGAATGCC AATCCCAAGC GCAAGATGGC CTTCTACACC GCCGTGAACA 107940
TCGACGGTGC CCGCTGGACG AACCTGGAAC GGGGAAATGA CGTGTGGTTT TACGACCCGC 108000
GCATCCCCGA GGAACTGCAA AACGGCGACG AACTCTATGG CGACGAGCCT GTGCCATCGA 108060
AGAACTATTT CGATCGCGGT CACCTCGTGC GCCGCCTCGA TCCGGTTTGG GGCGAGATCC 108120
GCGTTGCCAA GCAGGCCAAT GACGACACGT TCCAGTGGAC CAATTGCTCG CCGCAATATT 108180
GGGGCTTCAA TCAGGGCGCC GATCTCTGGC AGGGTCTGGA AAATTTCCTG CTCTACAACA 108240
CCGACGACGA GAATGTTCAG GCTTCGGTCT TCTCCGGGCC GCTGTTTCGC GCGGACGATG 108300

AAGAGCACAG GGGAATTCTG ATCCCGCAGT TTTTCTGGAA GGTCATTGCG GTAACCGACA 108360
AGAACGACAG GCTCTATACC AGCGGCTACA TGGTCAGTCA GCAGGACTAT GCCCTCGACA 108420
TTCCGTTCGA ACGACTCCCG GTCGGGCCGA ACAGCACAAA ACCTGGTCAA AATTTCCAGG 108480
TCCCGGTGGC AAAGATTATC GAAGACACCG GTATTGTCTT TGCAGACGAG ATCTTGAGCG 108540
CCGACGCCTA TTCCGGACCG TCAACCGGGC GAGCATTGCG CACCGTGGCG GACATACAGC 108600
ATCCCCGGCG CTGATCATGC AAAGCGGCTC TTTGCCGCGG GCTGCATCCA AGGTTTGCGG 108660
GCGCATCGTG AAGCCAAACG TCGCCAGTTG CACCGCCGGC CCGTTGATCG CCGTTGAGGA 108720
TCTGTCGCCC TGGACATCAC GCGGCCTTTG CAAATCTGGC ACGTTCGGGA GGGACATTCG 108780
GTTTCTCGTC TGATTGCCCC CGTTCACGTC TACGGTTGCG TGTCATAGAA CAGTCATAAA 108840
CGGAAGCAAG CATGCTGCGA TTGCCAGCCT TGCCGCGGGG CTATGAGGTG AAGGGGGACG 108900
ATCGCTTGCC ATCAAGCCGC TTGCCCGACC GGAGGCCGTC GCCGTCTTGC CGCGCACCAT 108960
CGAGCAAGCT ATGAGCTATT CCGTCGACGT CGACGTCGAC GTCGACGTCG ATCCGGACGG 109020
GCGGTACACA TTCAATATTC TGGATGAGAC GCACAGGTAT CCGGCATGAC GCTCTGCTGG 109080
AAAGATCTAT ACAAACCTGC TCACGGCAAC TGGGTGTGCA CTGAACGGTG GCGTTGCGAA 109140
CGGGCGAACC GTCATTTGAA CGAATAATCG TCGAGCCAGC GGATCCCCTT AGACCCTTAA 109200
ACTATTTGTT CGTTGACTCT TCGAGCGCCG CTTATTCGGA AGCCGAGTTT TTTGAACCTT 109260
AAGGCCCGCG GAGCGGCCGA GGTTTGCAGC GGAGGACTCG CGCAAAGATC AAAATCCGCA 109320
ATCCAATACG GTCAACGATC CATTCTTGAC GGGTTTGCCG CTCTTGAGGC GGATACATCA 109380
CCCGCGAAAG CGGCAGCCTG ATCATCGGCT GCCGTCTCAT TAAGGAGCTC GGCAAGAAGC 109440
ACCGGACGCT TGTCGCAAAC GCTCGGTCGG GCACGGATCG AACTGCGCAC GCACATCCGC 109500
AGCGCCGCCT TCCAGGGCTA TGTCGTCGTC TTTCGCTACG TTGATGAGGG CATTTCGAGG 109560
ACAATTTCCT CGAGGGGCAC AGGGATGCCG GCACGGTGTT TTCCCAAGAG CGGCCTGTCT 109620
TTGGAAGATA TCCGCGACGC TCTCATCGAT GCGGCCGACT GGGATTTCGT GGTCAGTCAG 109680
GCCCGTTGAA CTCAACGCAT TTTCCGCTGA CAAGTCCGGT ACTCAAGGCG GCGGGACAGA 109740
CCGGCAAGGG GGTCTGTCGG CTCGCCGCAT GGAGTCAATC GCATAAATAT ATTCCCCCAA 109800
ATAATCCATA AAAACTATAT TTTCTGTATT ATACTCCTGT GGCTTCCTAA TGTCTATCCC 109860
GTGAATGCAA GCGAGACCAT CCGATGACCA AAACGATACG CTTCAACGCT TTTGAAATGA 109920
ACTGCGTCGG CCATCAGTCG CCGGGGCTCT GGCGCCATCC GCGCGACAGG TCGTGGCAGT 109980
ACAAGGACCT GGAGTATCGG ACGGATCTGG CAAAGGTGCT CGAGACGGGC TTCTTCGATT 110040
CCATCTTTAT CGCGGACGTG ACCGCCTATT ACGACGTCTA CAAGGGCAAT CTCGACAATG 110100
CCCTGCTGCA GGGCGTGCAG ATACCGGTCA ACGATCCGCT TCAGCTCGTC AACCCGATTG 110160
CGCTTGCGAC GAACCATCTC GGCATCGGCG TCACGGCGTC GACCAGCTTC GAGCATCCCT 110220
ACACCTTCGC GCGCCGGCTT TCGACGGCCG ATCACCACAC CAAGGGGCGT ATCGGCTGGA 110280
ATATCGTCAC GTCCTATCTT GAAAGCGGTG CGAAGAACGT CGGGCAGGGC GGATTGCGTT 110340
CCCATGACAA CCGCTACGAT GTGGCATCGG ATATCTCGAA GTGCTCTACA AGCTCTTCGA 110400
AGGAAGTTGG GAAGAGGGGG CGGTCGTTCG CGACAAGGAG CGCGGGATCT TCGCCGACCC 110460
GACCAAGATT CACGAGGTCG GTCACAAGGG AAAATATTTC GTGTAGGAAA GGGTGTCAGG 110520
TCACTGATGT GGTAATTGGT TCCGCCGGGG TATTCCCTGT CGCGACTGGG CGTGATCCGG 110580
CACGGCGCAT CTCGATGACA AGTCTGAGGG TCAAATAGAC GGACACTGCG GTAAGGATGA 110640
CAGCGAGAAC CGGCTCAACC AGGGGATCAT AGGTAGGGCC CCAATTTGCA AGCCGATATG 110700
TGTCGTCCCA GGGACCGGCA TTGACCGGTC TTGACCAGAT ATCTGCGCAG TTCACGAGCG 110760
CGTTGGATAC GTGTTGGCAG ACCTGCATTC CGACAACGAT GCGCCACAAG TGGATTGCAG 110820
TGAGAAGGGG CAAGCAGATT AAGACAACGA ACGCGATGCC TTGGACCCAT TTTCCCGGAC 110880
GGCTTCTGGG CTCGATATCA AGGAGCCTAA ACGGAAAGGC CTCCGATGCA GAACCGGATG 110940
ACCTCTTGGC GTGATATACC GCGAGAAAAG GAACCAGCCA GAAGAAGAGC GCGAAGAAGC 111000
GCATTCCATG AACAGCATAG GCTTCGCGTG TCAATTCGCC GTCAAACTTA AGCTCAATCG 111060
GCAGTTTGAT AATCAACGCG AAATCAGATT TTTGCGTCGA TCCATGGATG TTGATCATCA 111120
CGACTAGCGT GAGGATCGCC AGCGTTATCC ATAGCTTTCT GAGCGTTTCC TGCGTCATTC 111180
CGGCCCCTGA CACGTTAAGC CTTGCCGCAG ATGCGGTTCA GTTGTTCCTG GGCCCAACCG 111240
CGCAGCACCG GTTGCAGGAG CCCAAGCCCT GACAGGTATT CGCCTACGTC ACCTGAAACG 111300
TCGAGGTCCA AAGAGGTAAT GCGACACGCT CCAACATCGA CAGTGACGTC TGGTGTGACA 111360
GCACCCTTCA CTGAAAACCG AATAAATGCT GCGTCGCTTG TCGCGCAGGT GACCTCCACC 111420
TTTGATCTAA CTCGAAACCC TTCAGGTGCT GAGCAAATAT CGATCTGAGT TGCCTCAAAT 111480
CGCCGTGTAC GGTTTGGACC CTCAGGCAGA GCCTCGCAAA CCTTGATCTG CTCGATAGCC 111540
GCCTTGATTT TTGACCAGTC AGGTGTGTTG AGAAGTGACC AACATTCTTC CGCTTGAACC 111600
GTTGAGGTTC CTATCACCAG GATGAGCATT CCGAATAAGG CGGGCTTCAC CATTGGCACG 111660
ATCCCCAAGC CGCACATAGA AGCTGCGTTA ACACAACATG ACGTCGGATA ATTTTCAACT 111720
AGCTGTTGAA AGATTATCTC ATAGCGGGTG CACTTTGTTT GAGGTTTCGA ACCCGGCCTT 111780
ATGTGGGTCC GATGCTTGGA TACTAACGTC CGAAGCCAAA GACGATTTCC TCGTCGTTTT 111840
CACGCGCTGC CACAGGAGAA GACTTACGAG CCGCTGGATC AGCTCCTAAG ATCGTCAGTG 111900
ACGGGTAACC CTTCCAATCC AGTTCGAGTT GGGCGTCAAA ACCCCTCTTC TGGGCCTTCG 111960
CGACCAGATT CTTAGGGGCG CTTGGGTCGT ATCCCATGGT CACGTCAGCG AGCGGATGGC 112020
GTAGATATTG AACGCTTTGC AACGCGAGCG TCATTGCCGC AACAATAACA GTACGGTCCG 112080
CAAACAAGCT TCCGTCGCGA TCGTTTGCCA AACGGAACGG ACCATTCTTT ATCGGCGTGT 112140
GCCCTGGAAC GGTGAAATAT GCATAGCGAG AAACGCCACG GAGAAATTCG CCCGTCGTCT 112200
GGTCGTCGTT TCCGGCGAAC GTGGGATCGC TGCCGACCAG TATGATATCG ATGCCGCCGT 112260
CGCCCTCATC GGACCTGGAA ACATATGCGC CTTCGATGAA CAAGCCCGAT TGGTTATCGA 112320
CCTGCAGGCC TGGCTGTTCA CCGAACATGA TGAAAGCCTG TTTCGCTGGA AGCTTGATGT 112380
CCTGAGCCTC CGTTTTCCCG CAAGTCCAGT TCCATCAGAT CGGCACCAAT CCTTGAAACG 112440

TCGAAAATGC AATCATCTGA CGCTAGGACA TGAGCTGCAA TAGGCTCTAG GTGTGCAATG 112500
GTCAGCAGGC GTTCGTCGCG CTCTTCCGGC GACATACCGC TGAACTCTTT CGAAACGGCC 112560
CAGTGGCGCA TGATCGTTTC CTGGCGGAAT GTGTTCCGAA GCTCCGATGT TGCCAGAGAG 112620
CCCGATATCG TCATGCCCAA TGCATCGAGC CTGCGGCGGA AGACCGCCTC GAACATGGTC 112680
GCGCGGCACG GATGAGGATA TCGCATTGTT TTTGATCCCG GATGCATTCA CACCCAGCCT 112740
GATCAAAATC TCTGCAAGCA AGAAGCTCGG TTCGGTGTGG GCAGTTTATA ACAGGAATGA 112800
AGCCCAGGCA TTTGAAATGA TGTCCCCAGC AATCTTGCCA CCTTCGTCAA TTTGCAGATC 112860
GAGACCTGTC ACTGTGAAGC CAGTATCCTC GGCCCATTCG ACGATGGCGT TTTTGCAACC 112920
TCGAAATCGG GGACAGAGTT TCGTTTTGAA AGTTCGGGGG AAATCACCTA CATAGCCCCA 112980
AAAACCTCAG CCCTACCGCT AGGCTGAAGC CTGTCTCCTA CGAACGGCGA TGATGAACCC 113040
GAAGAATTTG ATCGCGGTAG GTGATGTGCA CGGAATGGCA CAAACCTTAG AACTCCTTCT 113100
CAATGAAGCT GACCGTCACG AAAGTGGGAT GCACCGAAGA GCCGGAGAAT GGGGACCAAA 113160
GTTCATTGTT GGCTCAAGGT CTCCGCGCCC CTTTGTCCAG CCACCGAGCA AGGCGCATTG 113220
CCCAGCGTTC GATTACCCGC CGCGCAAGGC CTCCTGCCAG GTTCGGATGA TGGGCTGCGT 113280
GGCAGCCGTC GCAAAGAGTG ACAAGATTTG AGGGTTCATC GGTCCCGCCC ATAGACCGAG 113340
GCAATAGATG ATGCACATCG GCCTTCGGCG GACGTCACCT TAGTCGAGCA CGACACGCAT 113400
CTAAAGTCAT CACGCAGGAC AACGCGGCGC GTTTGCTTCC AGTTATCTGC CGGGGGCTGT 113460
TTGGCGGCTT TCTTCATTGG GCGAAGAATG GGGGAAAGTC GCTGAGAAAG CGTAACCAAT 113520
AGCACCAGTC ACAGCTCATT CGAACTGACC GCGAGACAGC GAAATTAAAA TCCCCGGAGG 113580
CCAGCATACT TTCGGCATCG TGATCCCCGG CATCGATTGC CAGAAACTTA TTAATGATTG 113640
ACGCCTTTGA AATGATTACA ACAGCGCTTT CAACAGCTGC ACTTTCCGAC GTCATCGCGG 113700
TATCGCCGGT GTAGTAGTAC AGCTGAAATA CACGCTTCTT GAAGTCGTAA TATGTTGATA 113760
TTTGAGCAGA ATACAGCATC ATTCTGCGGC TGATTTCCAG ATCACTATGG CTTGTTTGCT 113820
GCCCACATGA TCGCTGATGT GGTCGACCAC GCCAAGGTCA TGGGAAATGA ACAGATAAGA 113880
GATGCCCATC CGCGCCTGCA ATTCCCACAG AAGATCCAAA ATCTGTGCCT GGATCGGCGG 113940
CGATCTGTGA CCAGTCGATC GACCGCGACG CGAAGTGTCC GCCAGAAACG GCGCCTGTTT 114000
CAGCACGCGG AAGTGCTGCA TCTTCGCGGC GTATTCGGGG GTACAACTGG CCAGCTTCGG 114060
CAGAGGCGTC TCCTCCAATC GCAGGAATCG TTGGTTGCGA CAACCAACCT GCACCGGAAC 114120
CGCGTGCTTC GGTTATGGGT ATGTCAATCC GGTTCAAGAA CCGCACCCCA GCAAAATCAA 114180
AGACTTAAAT GCGCAGGTTC AAATCGCAAA AGCTTACCCC GCAACCAACA TGGCTGAGAA 114240
AACCGTTTGT TGAATGGGTC CGGAACTGCC GCCACAGTTC CGGACCACGG CAACATCGCT 114300
TCAGTGCGAT GTGCCCGAAC CGCCCACAGG TGGCGATTCA GCTCAGATTA GAAGTCGCGC 114360
TGCAGGCGGA CGAAGCCGCT GACGAAATCC TCCGTGTCTG CGCCGATGTC GCGACTTGTG 114420
TACTGCACGG AAACACGTGA CGCCAACCCA GAGGTGATCT TGTAGTCAAC CGCCAGACCA 114480
CCGCGCCATT GATCCGCATC GCTGACGAAG CCGTAATCAC CCCAATAATG AAGCCCCGGG 114540
GTGATCGCGA GCTTTTCGGT GGCGTTGTAG CGATACGAGG TGGCGACGGT CCACTCGGAA 114600
GCGGCGAAAT AGACGTTCGG GTCGGATGCC CAGACGCCGG CAATCTGGAA GACGCCCGGG 114660
CCGAGTTCGG CCGAAACCAG CGCGCGGATG GCTCCTTCCT CGACCTCGGT ATCGTAACCG 114720
CCGAGAAGGT CGATCGAGAC GCCGCCGACC GTGGCCGAAA CGATGCCCTG CACGCCGATG 114780
CCGTTTGCCT TCGTGGTCGC CCCTTCGAGC TCATTGATGG CAACGCCTGC CTGGAACGCG 114840
CCGCCCTCAT AGAGGTAGGC AATCGAGTTG AACTCGGTGA TGTTCGCCAG CGAGTCAGTT 114900
TCGCCGTTCA GGCCTTTATC CCACCAGCTG TAGAAGAAAC CCGCCTTGAA ACCGCCGAGC 114960
TGGATATAGG CCTCGTCGAG GTTGATCAGG CTGTCACCGA CGTCGCTGTC GTTGTCAGCG 115020
TTGAACTCGC TGGCGAAGAA GCCAGTGAGC GTTCCAAGTT CGGTGTCGCT CTTTGCGCTG 115080
AAGGCGATGT ATGCGCGCGA GAAGGCGTCC CAGTCGGACG TGCCCTGGGC CCCCCAGCCT 115140
TGGCGTTTGT TGACCTCATC CGGGCCAAAC GAGACCTGGA AACGAGCGTA ACCATTGATC 115200
TTCAGGCACG TTTCTGTGCC GGGGATATAG AAATAGCCGA TGCCGAAAGC GTCGCAGACG 115260
CGAACGTAGT CCATCGGTTC CGGCTCCGCA GCGACGATCG CGTCAGCCGC TTGTGCACCA 115320
GAGACTGCTG CAAGGGCAAC GCTGGAGCCG AGAAGCACAG TGCTAAAATT CATTCTCATG 115380
CGGTCGATCT CTCTTTTGGG TTGGGCAAGG GCCGGGGCAG GTCGGTCCTG AACTTCAACG 115440
GCACCCTCGC TCGCGCTGCG GCGAAACGGG CCGTCAATAT TGACGGGCAG CGCGCTACCT 115500
TTACCAACGA ATATATTCAT ATTCTAAAGG TATATGAATT CCGTAAGGCG ACGAAATGAA 115560
AGTGGTAGAC ATTCGTTGTT AACGCAACAC GTAAATATTG TCGCATTTTG TACAGCGCAA 115620
AAGACTGGGC AATGTTACTA ATCATACAAA TAGTTTGTAT GATGGCGCCG AATTGGCGCG 115680
TAATAAAACT TGAAACAACT TGACATATAA GAAACTGATG ATAGATATAT ATCGCCCGAT 115740
GAGAAGAGGG CTCTTCCCGA CTAGCGCCGC CAGAGCCTCC AGCCTGGCGG CGCATTTCAT 115800
CTCGACGCTA TGGGCGTTTT CCCAGGCGCT CTATACCGGT GGCGACACGG CTTTCATGGC 115860
CGTCGGACTT TGCCTCGCAG GATAATGGGG AAGGATCGCC CGCCTACAGC GCCGTGCGTC 115920
CTTCAGGACG CACAAAGGCG CTGTAAGTCT TCGAATCTAC CCATTGTGCT TTCCGAAAAT 115980
CGGTTCCGAT TTTCGGGCCG ATGCGCTAGG GCAAACCGTC GAGTGCGGTC CAGGAATGTC 116040
GGGACGCTGT CCGTTTTTAG TGGCAGAGCG CTCCCCGGGG CCAAGCGCTG CTTGGGTCTG 116100
TCGCCCTTGG ACTAGGCCTG CTCGACGGAG CCCTCGTTAC GGGTGAAACG AAGGGCGGAC 116160
GAGGGGCTGA GACCGTAGGC GCTCTTATAG AGAACCGAGA ACCGGCCCGG ATTGGAAAAC 116220
TGAAGATTCA AGGCCAGCTC GGTAATGCTG CCCGCCCGGC CGGCCTTGAT GGCGCCATGG 116280
GCGGCGGCGA GCCTATAGTT GCAGAGGACG CTCATGGGCG AGCCTCCTCG ATAGGTGCGG 116340
AACATCCTCT GGAGCGCCCG CGGCGTGCAC CGGGCAGCGG CGGCGAGGTC CTCGATCGTA 116400
ACAGGGTTGG TCAGGTTCTC GCGCATGAAC GCCTCAGCTT TAAGGAGCTG TCTTGGTGCC 116460
GCACCGCTGG TTCCCTGACA GAATGCCTCA GCGAGATTGT GCGGCATCGT CGAGAACAAC 116520
TTTACGAGGG CCAGCTGCTT GTACGCCTTT GCCAACAAAG GCATGGCTTT CGATGCTGCC 116580

GAGTTGAGAT CCCTTTCCGC CTGCCGGAGC GTCTGATACA GACCTTGAAC GTCATCTTGA 116640
GAAAAGCAGG TAGCCGGCAA TACGAACTCG TGCACATAGG GTCTACCGGT CAGGTCCTCG 116700
AAGCTTCGCC TGAGAGCCGA CTCGGGAATA TGGAAGGCCA ACCACGAACT TTCAGATTGG 116760
ATCGTCATTC GGTTGCCGGC ACGTTCGCGG GCGCTCGCGA CATGATTAGG CGAGATGCTT 116820
AGTGTTTTGC GTGTCCGCCG GTCGGTGATT TCGATATGTC CGGACAGCAC GAAATAGAAG 116880
CTGATGAGCC GCGTATCGCG CCTGGCAAGC GCTTGCAGAC AGACCGAATG CTCCGCGCTT 116940
GCCAGCCAGG CTGACAGAAC TCGTTGCCGG TGGTAGGCGA GCGAGCAAGC CTCCGGGGTC 117000
TCTGAGGAGA CCTTCCACCT CCAGTTCGGC TCGACAGATC CAAGATTGGC GAGGATCGTG 117060
TCGCGCGTAT GGGCGACGAA ACTGTCTGTC CGCAGTGGCT CGTCTGTGCT GGGAAGGGTT 117120
GCGCAAAAAA GCACGGTGTT CCAGCCGAGG CTCGGAAACT TGTCTAGGAG AGGCAGCGAA 117180
CCGCATTGAC GAACCAATGG TTCCGCCGCC GCGATCAGGT TCCAGTCGAG CATCGAAAAC 117240
AGCGCCAATG GGTTGAGAGG CGGGTAGAGC AGAACGGCTA TCAAAGCAGC AATCGTCCCA 117300
GCGACGAGCA GGCACAGGAC CAGCTTGCCG AACGGCAAGG GCATCACTTG GTGGCTCGTT 117360
TTATCAGCAG AGCCATGACG CGCGTCAATC TGCACTGCTC TCCATCCTGT GCGATATTCG 117420
ATCTGAACCC GGATCGAAGA TGTGTTTTTC GGATAGGGAT CGCGCGTCGG CTTTGGCCGT 117480
TACTCTGAGA TGCTGCGGAG CGGCAATGAT GGCGACTTTC CCATCTGCCT CGAAGAGAAT 117540
GGACGAAGGC GCACCGGCTG CGCGACACAA GACATGTCCG GCCGCACAAT CCCAAAGATG 117600
GGTGCCGTGC TCGACATAGC CGGCAAGCCG ACCCGCCGCG ACCATCGCAA GTGCTGCGGC 117660
ACAGCTTCCA AGGCAGACGA TATGATAACC GCGCGCCTCA AACGCGGCCT CGACTTCGAG 117720
GCGATGGGCG AGGGGCCATG TTCGGTTGCG GCCTATGCCA AAGGCGATCG GCTGCGCCCC 117780
GACAAGCGGC GAAACCGAGC CTGTCCCATG CAATGGGCCA TCCACAGAAG CCCACAACAG 117840
AGTATCGAGT GCCGGTAGCG CAACAGCGCC GAGGACCGGC TCCTTGTTGC GGACAAAAGC 117900
GATAGAGACC GCCCAGAGCG GTATGCCCGA CAGGAAATTC GCGGTCCCAT CGATCGGATC 117960
GATCAACCAA TGGTCAACTT CGGCATCGCC AAGCTGTTCC TCGCCGACGA TCGCGTCGGC 118020
GGGGAACTGG GCGTGGATCA GCCTGCGGGC AAGACTTTCG GCGTCACGAT CGGCTGCGCT 118080
GACGTAGTCG GCCTCACCCT TGGTCTCGAT GACGATGTGG GAAAGATTGC CAAACCGGGC 118140
CAGCGCGAAG GCTCCAACCT TGCTGGTGAT GTTCCGAAGC AAATCGAGCC GAGTGTCGAG 118200
GTCGCTTGTC AATTTGCCGC CCGATCCCGC GCCGACGCAA CCATCTCTTC AAGCCGCAGA 118260
GCGATAGCGT GTGCCCGCTG ATAGTAGGAC GACTCCGACA GCACGTAGCG GTCCATCGGC 118320
GTCTCGAAGT TCAAGGGCGT CCTGTAATTT GTTGCCGCAA TGGCTTTTGT GATCGCATCC 118380
CAGTCAAGCC TGCCGTCGAA TGGCAGCAGA TGGTCGTCCT TTGCGCCGAA GTTGTCATGA 118440
ATATGCGTGG CGATCAGCCG ATCGCCATGG CGCTCCAGCA CCGACAGCTT GCCGGGCTCG 118500
ATCAGTTCCC AATGGCCGCT GTCGTAGCAG ACGCCAACGA AGTCGGAACT GTAGCGGCTA 118560
AAGAGGCGGG CATAGAGCTT GAGGTAATTT TCCGCATTTG CCTTGACCAG GGTTTCGACC 118620
GCGATCTGCA CGTTGCGTTC GATGCAATGG GCCTCGACGT CGTCGAAGGA TCGGAACAGG 118680
GGCTCGAACA ACAGGTTCTC GTTTTCAACG CTGCGGAACG TGTCGTCGGT GATGTCGACG 118740
TGGAGCACGA CATTCGGAGC GCCGAGGGCC GCAGCGAGAT CGATACGGTT TTTTAGAAGC 118800
TCCACCCCGC TTTGACGCTG CCAATCGTGG TGAGAGAGGA AGTTCTTGCG TGTCTCCTGC 118860
GCGAATGGGA CAGGTCCGGC ATGGGCGAGC TCGCTCACCG GGTTGACGCC GTTGCTGGCA 118920
TGAACGGAGT GTGTTTGCAG GCCCGCCTCC GCAACGATGC GGCGGGTGAA CTCGATTTCG 118980
GCGTCGGCAA GCATATAGGA GCTGCCGGAA TCGGGGTTCC AGTTGATATG CGTAAATCCG 119040
GCTTCACGGA TCAGGTCGAG GGAGTGGCGG ATGCCCTCGT CGTACCAGCG TTCATGGGTG 119100
GGCCAATGCC AGACGGTGAT GCAAGTATCC ATTTCAGTGT CCATTCAGAG GTTGCTCGAT 119160
TCCGGGGTGC GGCCGAGGCT CAGGCGCTCG GCGATGAGGA TGACGGCCAG CGAGAAGGCC 119220
ATGATCAGGG TCACGTAGAC GAGACTGACG GCGGCCTGGT CGGGATCGGC CGAAACTGCG 119280
CCATCGACGA TGGCGATCGG CAGAGGCTTG TTGTTGACGT TGTAAAGGAA GGCCGAGAGC 119340
GGGTATTCCG CCAGCAGATT GTTGAACTTC ATGCCGGCGA CCAGGATTGC GGTCGGCGCC 119400
ACGAGCGGCA GGACAACACG GCGGAGCCGA TACGGCCCCG AGGCGCCCAT TGCCCGGGCC 119460
GCTTCGTCGA AGGCCGGATC GATCCCGATG AAGGCCGAGC GCATGAAGCG GACCATGAGC 119520
GGCAGGCTGA AGATGACGTA GCCGATCGGC AAAAGCCAAT AGCTGCCGAG CAGAACGAGA 119580
TTGCCGACGA GCGGATTGGG CGCATCGAAA GCAACGATCA GCCCTACGGC AAGCAGCATG 119640
GTGGGGATGA TCCAAGGCAG GACAAAGCAG ATATCCAGGC CTCGCGTCAG CCAGTTGCGC 119700
TTGCGCACCA TGATCGGCAC GGCAAACAAA GTGATGGCGA GGCCGACGGC AACCGCGATC 119760
GAGCTCATCA ACATCGAATT GAAGAAGGGG ACAAAGGCCG CGCCGCCGCC CAGCACCCGA 119820
GCGTAGTTTG CCAGCGTGAA GCTGGAAGGC AGGACGTCGA TGCCGATGCT CGAAGCCGGG 119880
GCGAAGGAGA ACAGCACAAT GAGCGCGACC GGCATCAGGT AGATCGCTGC CAGCAAATAG 119940
GCCAGCGCGG TCACCACGAT ACGGGCAAGC GGATTGCGTA TCGCCCGCAA CTGGATGCGT 120000
GTTGTCGCCT TAGCGCCGGC CGTATAGGCG CCCTTGGCCT CGAAATACTG CGACAGCAGG 120060
ATCAGCCCCA TGAGCACAAG CCCCATCAAC AGCGCCAGCA GCGCCGCCAT GTCTGGCCGG 120120
CGCAAGCTGT TCAGCGTCAG GACCATCTGG CTCAGCATGT GGAAGTCCCG GCCGCCGAGC 120180
ACCTGGGGCG CGGCAAAGGA ACCCATCGCC GTAATCAGCG TGAGCAGCGT AACCGCGAGC 120240
ACGGTTGGCA GGATGACCGG CAGGACGACG CACCGCAGAA TCGTCATTTC CGAGGCTCCC 120300
ATGCTACGGG CCGCCTCGAT CGTCGAATAG TCGACGCGGC GCATGGCGGC GCGCAGAAAC 120360
AGGAAGTGAA AGCTGGTCAT CAGAAAAGTG TGGGCGAAGA GAACCCCAAA CCAGCCGATG 120420
AACCAATCGC GCGGCAGCGA CGGGAAGAGC GCGGCCAAAG CAGCAGTGAT GGCACCGCTC 120480
GGACCATAGG TGAAGTTGTA GCCGGCAGCC GCAACGACGC CGCCGAAGAC GAGCGGCGTG 120540
GAGAAGGCGA TTTTCAGAAA GCCACGCCCC GGCACGCGAA AATACTCGAG CACCGCCACC 120600
TGGAACATAC CGACGATCGT GACGGTGAGG GTGGTCGCCG CCGTCATCCA AACCGTGTTC 120660
CAAAGCGCTG CGCGGACGCG GCGAGAGCGT GCGAGTTCCG ATACCGCATC AAACATGGCC 120720

AAACTGCCGA GCCTGAACAA CGCGGCCTGC AAGGCAGCAA GCATCGGAAC GAGAAGGAAG 120780
GTCAGCACGG CCCAGGCAAT CACGATCAAG CCGAGCCAGT AAAAAACACG GTCCAGGCGG 120840
GTGGATCGGC TCATCCGGTC ACCTCCTGGA ACACATGCGC GGCGCCGGGG CGAATTCCGA 120900
GCGAGACGGT ACTGCCGGGT TCAGGAAGCT GGTCGCCGAT CAGCATTGCC GAAAGCGCAT 120960
GACCCTCGAC CTCGAGATCG ACGCGGCTAT GGGCTCCGAG GAATTCGACG TGGTTCACTC 121020
GCGCCGGAAC GCCGGCGACC GCCCCAAGCA GGACGTCTTC ATGGCGCAGG AAGCTGCGGC 121080
CTTTGACATC GTATCCCAGC AGGCGCGCCG CGAGTGGCGG AGCAAGTTCG TTGGAGACGC 121140
CGATGAACTC GCATATGAAC GGAGTCGCCG GCTTGCGGTA GAGGGTACGA CCATCGCCGA 121200
TCTGCTCGAT CTTGCCGTGA TTGAGCACCA CGATGAGGTC GGACATGGTC AGCGCGTCCT 121260
CCTGGTCGTG CGTGACGAAG ATGGCGGTAA AGCCGAGCTC CGACTGAAGG CGCTTGATTT 121320
CCTTGCGCAT CGCGGCGCGC ACCTTCGCAT CAAGATTTGA CAACGGCTCG TCGAAGAGCA 121380
GCACGCGTGA TCCCGTGACG AGCGCGCGAG CAATGGCGAC GCGCTGTTGC TGGCCGCCCG 121440
ACATGTTCGC TGGCTTCTTG TCGAGCTGAT CGGAAATACC GGCCATCTCG GCAACCTCGC 121500
CGACGCGCCT TTCCACCTCC GCCTTGGACT TCTTCGCCAC GCGCAGCGCA AAGGCGATGT 121560
TCTCGAACGC CGACATCGTC GGAAACAGCG CGTAGTTCTG AAAGACGATG CCGACGTTGC 121620
GGCGCTCGGG CGGAAGGGCG GTAATGTCTT GCCCCTCTAG GAGCACCCTT CCGCCCAAAG 121680
CCGGGAGCAA CCCGGCAATG GTGCGCAACA CGCTGGATTT TCCGCTCCCC GACGGGCCGA 121740
GGAGCGACAC GAACGCCCCG TCGGCCACGT CGAGGTTCAG GTCACGGACA ACGGTCATGC 121800
CAGGGAAGCC GACGTCGAGT TTGTTGAGGG ACAGAACGCT GTCAGCCATG GCTCTAGACC 121860
TCCGATCCAT GCAGAGCCGC GCTCATTGAC GGATTTCAAG CTCGATCTTC TGCAGCCAGC 121920
TGTCGAGCTT TGGAGCGATT GCATCCCAAT CGATCGGCTG GGTGTTGACG AGCTTGGCAT 121980
TCGCCTGAAC GGTGGCCGGC GACTTGGCGA GCGCTTCCGG CAGGACAGGG ACCTGGCCGA 122040
ACTTGGCGGC GTATGCGGCC ATCACATCGG CCGATCCCCA CCAGTCGACG AAGGCCTTGG 122100
CCTCATCGAG TTGATCGGTC CCGGCCATGA TCGCCACACC TTCGGAAATC ACCGGAGTGC 122160
CGCCGTCGGT GTCGATCACC TGGACTTTCG CGTCGAGGTC ATCGGCCCGC TTCAAAGCGC 122220
CGCCGAGCCA ATTCAGATCG ATGGAGGCGC GACCGGACTT GAACGCCTCG AGCTGGGAAT 122280
CCGCATCGTT GGCGATAATG CCCTCAGCAT AAAACCTGTC CAGGAAATCC CATCCCTCAT 122340
CGGATACCTC ACCATCGGCG TCGAGGAAAC GGACGAGGAT GCCGGCAAGA TACATGCGGG 122400
TCGTCTGGGC CGCCGTGGAG CCGATGACAT ATTTGCCCTC ATAATCGTCC TTCGTCAGAT 122460
CCAGCCAGCT TTTCGGCGCG TCTGGGTCGG CCAGTCGGTC GGGGTAGTAC GCGAGCACGA 122520
TCGGCGTCTG CCAGAACTTG TGGACCAGGC CTTCCGCGTC CTTATATTGT GCCGGAAGGT 122580
CCTTGGCCCA GGACGGCGAA TAGGGCTGGA AGAGGCCTTC CTTCTTTAAG AGGGCCATCG 122640
AGGTGTCTAC CATCCCGAGA ACGACGTCGG CTTGCGGGTT GTTCTCCTCG GCGATCAGGC 122700
GATCGTAGAG TTCGCCGCCC CCTGCGTTCA GTAGCTTAAT CTCATGACCC GCTGCCTTGG 122760
CCTGTTCAGC GATCCATGCC CCGCGCTCAC CGCCTTGCGG GCTGTAGACG GTCAGTGTGT 122820
CGGCCAGCGC TGGAAAGGCC GCGGCCACGA CGATGGCGGC GGTCGCAAAT GCAAATGCCA 122880
GTTTCATGAT GTTCTCTCCC TTATCGAGCG ACGCGCTCCT CTTACGCCGG CTCCATGTCA 122940
GTTTAATTAC ATTGCGCAAT TTAATTTTCC TTGCAATAGC TTTCCTGAAA CTTCGACCGA 123000
TTTGCGGACT TTGCCATGCC TGTTTTGCCC GAGCTCTCCC TCAATGAACG ACGCCTCGTC 123060
GAGTTGATCT TCAAGAACAG GGGCGTGGCT CGCATCGAGT TGGCGCAGAT GTCCGGGATG 123120
ACCGGCGCCA CCGTGACGCG TCTGGTTGCG AGTTTGCTGG ATCTCGGCCT GATCACCGAG 123180
GAGGCGGATC GAAGCGGAGC GCAAGGCCAG CCACGCCGGT TGCTGCAGCT CCAGGCCCGC 123240
CGTTTCTTTG CCGCCGGCGT CACCTTTTCG GTGACCCGCA TGGAAGTCGT CATTATCGAT 123300
CTCAGCGGCT CGATCGTGGC AACGAGAAGC GTGGAGGTTC ATACGGCAAG TCCAATGGAA 123360
GTGGTGGAGG CGGCTCAGGC TGCCGTCGAT GACATGCTCG CCGCCTTATC CATCCAGAAG 123420
AACGACCTTG TCGGAATTGG CGTTTCCGTT CCGGGAAATT TTGGCACCGC CTCGAACCTG 123480
TTGAAAGCCC ATCCCTTCTT TCCTGCGTTC GAAGACGGCC AGGCGATCGA AGCGTTCCGT 123540
GAGGCGTTCG ACGTCCCATG CCATGTCGAA AACGACGGCA CGGCTGCCGC ACTTGGCGAA 123600
TACGTGTTCG CGGGTGACAA CGTGCTGGAT GATCCGCTGT TCTTCATCCA CATCGGCCAT 123660
GGCGTCGGCG GCGGCGCGGT GATCGACGGC AGACCTTATG GCGGCGCGCA CGGCAATGCT 123720
TGCCTGCCGG GTGTCCTCTA TCCCTACGAT CAGCCGCGGC CGTCCGGGCA GGACCTTCTC 123780
GCGACGTTGC ACGCGGCCGG TTTCCATCTT CGAGATTTCG AGGAGATGGA CGCGATGCCT 123840
CAGGCCGCTC GCTCCACCGT TATCGAATGG ATCACGCGCG CTGGCGCGCA GCTCCGGCAA 123900
GCCGTCCGTG TCGCAACAGC ATTTTTCGAT CCCGCGCGTA TTGTCGTAGG TGGGCGGTTA 123960
CCCGGCGATC TGAATAAAAG GCTTGTTGAA ACGATCCTGT CTGAGCCGAT AGAAGGACCA 124020
TCGCGAGGTC TGCCGACCGC TCCGGTTTCC GTATCTCGAC TTGGCATTCG CGCCGGCGCC 124080
GTTGGTGCCG GCTGTGTTCC GTTCTTCAGA GCATTTTTTA CGGGTGCCGT CGCCAATGGT 124140
GGAAGCGCCT ACCTGAACGG TAGACGACCT TTTCCTCGGA CTCCCGCCCA ATAGTTCCGT 124200
GCCGGAACAC GCAACGCTAC CACAGCCCCA AGCTGTGCAG CCGGAAGCTG TTGCACATGA 124260
GGCCGACTCG TCAGGAAATC CCGCGCAAGA CATGTCGAGA CCACTGCAGA CCCGCCCCTT 124320
GAGCAGGGAG CTTCACATGT CCGAGCTGAG GATATTCTCA TCGTCAGACC AATCGGCGCC 124380
GGCAGGTTAT TCCCTTACAG CGCCGCGCGT CTTATCGGAC GCGCAAAGGT CGCTGTAGCG 124440
CTTTGAATTG CTGCATGTTT TTCCTTGAGC CGAGGTTGAT TCAAAGAGAC ATGCAGTAGA 124500
GTGACCTTTG AGCAGCATTC CAACAGGCGA AGGCTGTCGC ATGTCTAACT GTGTGTAGCA 124560
TCTCTAAAAT GCTTCACTCC AAAAAATTGG GACCTCGATC GGGGTTCCAC CGGCGCATCA 124620
GGACCCTTCC GAAAATCGTC AGTTTATTGA CAGCTAAGCC TTGTAGAAGA TTCGGCGCTT 124680
CCGGGATTTT GCAGCCGCCC TCCATGATTG CCTGCTTCGG CAGTGGTCGG TGATCACCGT 124740
CGATTTTGAG AGCTCCTGCT TCTCAAAGCA CTCGGCGCAA TCCTTCATAT TGTTACCGAG 124800
GAGACAATAT CGATGAATGT ACAACGGCCC GGACTTGCCG TGGGCCCCCT TTTCGAGAAT 124860

CCCGAATCAG AAAGTTCAGA ACCAGGGTCT CCGGCCGCCG CCGCACGTTG GGTCGAAGCC 124920
TCTACGGAGG CGGAGGCGTC CGCCGCCTCC TCGTCGCAAG GCCAGATCGT GGCAGCCCCG 124980
ACCGCCGAAG AACGGCCATG GGAGGGTCGT CCGCAAGAGG CGGTAAGTCG AACGAGGGCC 125040
TGGCGGGAGG CGGGCGATGT CGATGAGCCG CTGGATCTGT CGTTCCTATC CCTGACCCCC 125100
TTGTCCATCC CCCTCGTATC CGGGCTCCGG CGCCTGAACG TCAACAACAA TCAGCTAGGG 125160
GATCTGCCAG ACACCCTCCC GGGAACGCTT CTGGAGCTGG AGGCCAGCGA GAACCGGCTG 125220
ACCCGCCTGC CCGACCTTCC TGCCGGGCTC CAGAGGCTGA ACGTCGAAAA CAACCGGCTG 125280
ACCAATCTCC CCGAGCCCCT CCCGGCCGCG CTCGAGTGGC TGGGTGCTGG CTACAACCAG 125340
CTAACCCGCC TGCCCGAGAT GATTCCCCCC GAGCTTATAT GGCTGGGCGC CAGAAACAAT 125400
CAGCTGACCA GCGTGCCCGA AAGCCTGCTG ACGCAGCTAG GCCAATGGTC TAGCATTGAT 125460
TTGGAAAATA ATCCGCTGCC GCATGGGGTA CAAACAAACT TGGTGACAGC CATGCATGCC 125520
GCGGGTTACG CAGGACCGCA GATCTTTTTG CCTATGGGAC CGGTGGAACT TGCACGACGG 125580
CCCCTTCACG AGGTTGTCGC GGATTGGCTC GAGGGGGATT TGGAAACGGT GGCCGCCTGG 125640
CGGGGCTTCG CCAACGAACA AGGTGCCCGG GATTATGCAC ATTTCCTCGA CCGGCTCCGT 125700
ACCACCGTGA ACTATGGCAA TGATGCGTTC CGGCAGGCGG TGGCCATAGG TCTGCGGCAG 125760
GCAGTGGCCA GGCCGCAATT GCGCGCACAG TATTTTGAGC AGGCTTCCGG AGCTAGCGAT 125820
AGCTGTGAGG ATCGCATTAC TTTGACCTGG AATGGAATGC AGACCGCACT TCTGATCGCT 125880
GATGTAGAGG ACGGGGTCTA TGACGGGTCG CTCCACCAGC TTCTCCAGCA CGGCCGTGTC 125940
ATGTTCCGTT TGGAGGCACT GGACGGGATC GCGCGCGAGA CGGTCAACTC GCTTCGACGT 126000
ACCGATCCGG ACGCCGACAT CGACGAGATC GAGGTCTATC TTGCCTACCA GACACAGCTG 126060
CGCGACACCC TGGAGCTGCG GCACGTCGCC CCAGACATGC GCTTCTTGAA CGTCTCTCAC 126120
GTTACCGAGG AGGACGTTGC CAGAGCTGCC AGTTCCGTGC GGGAACTTGA GGCGAGGGGA 126180
TTCGGCGAGT ATGTGGCAAC GCGCTGGCAA CCTTGGGAAA GAGTCATGAG GCGCATCGCC 126240
CCCGCAAGCC ATGCAGCGAT GCAGGAGCAG CTCATCGAAG CGATGGGAGA GGAGTTCAGA 126300
AGCCGCTTGG ACGAAAAGCT CGCCGAGCAC GGCCTGACGG GTGATGCCGA TGCCGAACGG 126360
GTATTCGGAG CCGAGATCCT CAACGACATC GCCCGCAGGA TCAAAGGCGA GACAATGGAG 126420
AAGGTGCTTC GGGGTCGCGG TCTTGAGCTG TGAAGCGGCC GTCCATGCGG TGGCTGTCAA 126480
TTGCTGTTCC TGCGGCGGTC GCCCGCGTCT ATTGCGCGAC CGAGATCAGG TTATGCCAGG 126540
CCGGTAGCAA CGGCGCCAGC GACGGTGAAT TGGTGCGCGT GGGAAACCAC CAAAGCCGTT 126600
CTGGCCGGGA CGGACATTGC AGGTGTTGCG GGCGTCACGG AATGCGTTTT CAAATGAAAA 126660
GCTCGAGTCA TTGTAGTTTT CTAACTCCCG CCATTAGACC GCCCGCCCAC TCGCCGGCCG 126720
TCCCTGCCCA GTCGGTACCA TTCAAGGATA CGGTTTACGA CCATCGAGTG GCGCAGATCG 126780
TGAACGCGTG GGCCGGCTTT GCCTTTCAAC CCGGCACGAC GTATGACGTC TGTAAGCAAC 126840
CAGGTGATCC TTCCTGGCGT GGAGCGGGAA CTGCCTTGTT CGTGCCAGAA CAGGGCGGAG 126900
TGAGGAGCCT GCTATGCGCC GACCCGACGC CGTGCAACGA TATAGGTCCG AAGCTAACCA 126960
CTACACTGTC GGGAAGCGGC AGGATCCGGG TCTTGAAGAA CTTTGTTTGC CGAACCGTGA 127020
TGGTGCCGTC GCCGAGGTTA ACGTCACCAA GATCAAGTCT GGCCAATTCG CCTCGCCGCA 127080
GACCCGCGCA ATAAGCCAGC AACAGCATGG TATACATGTT CTCCTGGCGA AGTGCAGCTC 127140
GCGGCGATGG ATAGGATCGC GCAATGTCGA GCATCCGTCT CAAGTCGGCA GGTGAGTAGA 127200
TATGGGGCTT TCGCCATTGC CTGGCCACCT CCTTCCGGGG TCTCGGATCC GACCGGCGCG 127260
GAGGGACCGA CGGGTCACGA TGCCGTAGGA TTTTCGCGAA GGCGCGCTCG AGTTTTTCGC 127320
GCTCGTACGC ATGGTTGCGC GTGCCCTTCG TTGCCGCCCA CTGACACACA CTCAGCGGCT 127380
CGGTTTCCAG TTACGGGTTC AGCTGCAGGA ACCGATCGAA CTGCAAGAAC AACACCGGTT 127440
GCGACGTATA CTTGTATCCC CTGCGACGCA TCATCGCGAC ATGCTCAGCC ATGATCCCGC 127500
CCAGCGCTGC CGAACGGTTC GGGCTGACGC AGTTGGGCAA GAGCCTGTTC TGGATCACAT 127560
GATATCAATG CGCGCCAGAC TGGCATGCAC TGTTTGATGT GGCATGCCTC ACGCAAATCT 127620
TCGACGGGAT TGTGATCAAT CGCAGCCGCT TCCAAGAAGT GGTCGAGGAA CCGATCGATA 127680
ATGCGGGTCC GATGTAGCAG TGTCGTCGCA GCCCAACGGT CAGACGACTG CCGCAACCAG 127740
GCCAGTAGCA CCTGTTGGTC GAGTGCCTCG TGACGTTCGA CAACGTCCTG GAAGCCATGC 127800
AGGACTTGAC GGTAACAAGT CCGGCTTTTG ATACTGCGCA GATCAAGGCT CGCGACATAG 127860
CGGCCAATGT ACGCGCGATC GGGATCGGGC CAACGGGCGG TCATGCCGAC ACCTCCGTTC 127920
CCGGCAAATC AAGAGCGATG GCCCTGAGAT CCTCGGTCGC AAGCTTGAGG TAGGGCGCCG 127980
TCGACGCAAT CGATCGATGC CCCAACAACT CACCGATGAT CTTTTGTGGC ACCGCGGCGC 128040
GCAACAGTTC GGTCGCGCGC GCGTGGCGGA AGATATGAGG CCCACGCTTG CCCCGGGGTT 128100
TGACGCCAGC GTCACGTAGC CGCCGTCGAA CCATGCTGTA TAGCTTGTCG AGCTTGCGAT 128160
AGGGTGCACG CGTGCGAACG AAGAGTTCTC TCGCATCTGT CGCGGGCCGT CCGGAACGCA 128220
GATAAACAAG GATTGCTTCG CCAACCGGCT CCGTCAAGGG AAGGAGCGTG TAGGCTTGCG 128280
TTTTGCTGTG ACGGACACGA ATGGTTTCGG TCCGCCAATC GATATCCTCA ATCCGCATGT 128340
TGCGGATTTC TCCTGATCGC AGTCCGTAGG TGGCGAGCAG TTGTAATATC GCATGGTCCC 128400
GCAAGCCGGC CGGCGTTTTG TCCCTGCTCG CACTTACCAG AACCGCTGCG ATTTGGTCCC 128460
GCTCCAAAAT GGAAGGCACG CCTTCATAGG CGTAAAGCCG CGGCGCCAGA ACATGCGGCG 128520
ACAGGTCCGT TCCAATGAGC CTTGTCCGAT GGAGATAACG CAGCAGCGAG TGAAGCCGCT 128580
ACGCTGCGCA TTTCAGCGAA CTGCGCGTCA GTTTCGAGGC AAGCATGTCC ACATAGCGGT 128640
CGATGTCGCC GACGCTCACT TCCATGAGGC CTTCGGCTCC GCATCGTTCC AGTTGCCAGC 128700
CCAGGAAGTG TCTGGCCTCC CACAAGAACG CGTCGATGCT CGCCCGAGCA AGACCACGCT 128760
CCTCGCGAAG CCAGGTTTCG TATTCGTTGC AGATCGCAAA CCGCAGTGCG TCGGCGGCGC 128820
AGGTTACTTT TGGAGCAGGT GGCCACCGAT CCTGCACAAG TCGCAGCAGT GCGTCACAGA 128880
TATCGCGCTA CCTCGGCTTC GGTCACATCT GCGACCAGAA TTCCTCGCTG CATTAAATAA 128940
TCGAGAAACT CCTGTGCATA GGCGCAGTAA TTTCCGATCA CCACCGGACT ATATCGGTGA 129000

CTGCTCAGTA TGGCTTTGAG TTCAGTGATT AGTTCTCGTT CAGATTTCGA CATCAATAGC 129060
TCCTCTGCTG GTCGAATGAC CGCAGAGGTT CGCTGGAAAA TAATACAGAG CAAAGACGGC 129120
GACAAACAGC AGAATTGTAT TCATTTTCTG CGCCGGTCCC GCGCTCCTTC GCATTATGGT 129180
CACCTGATGA TTAGTCAAAC GGGGGAAGAC GGATGCAGCC GACGCTGAGG CGATTAGTGA 129240
GGCAGTGACG CGCAAGACGA TGCGCTTCGT TCCGGTGAAA ATCCCCCGAA CAGCAGGCGG 129300
CGGCGATGGT ATTGAAGACA CGTGCCCTTC TGGTTCGTCA GCAAACTCAG ACAATCAACG 129360
CGCTACGTGC TCACCTGTCC GAACTGAGGA TCATTGCCGG CATCGGCACG GTGAAAATTG 129420
CAAGCCTGAT CTCGCCGATA AAGACGAGCC GCTTCGGATC GAAGCGGTCT TGCCAGGCCT 129480
TCCAGCGGGC TCTGCGCCGT GCAATCCCGC AACGATTCCG ATAGGAAATG ATCCGCTCTC 129540
GTTTTTCCAA GTCCGGCCCT CAACGGAACC ACCGGGGCCC TCAAGCCGAT CTGTTGGAAT 129600
GCATTCGTTC GCATATTCCA TGGTTTGAAT AGGCCCCATA CAAAAAATGG ATTTTCCCAG 129660
TCTTGCCACA TGGTGCATAG CGAAGCTCCC CACGCGTGCC TCGCCTACGC CACCTTTCGT 129720
TGGGGCATGA CACCACGACA ATAGAGAAAG GCTGATAGAT GAAGAACGTC ACATACCTCT 129780
TGGACCCGGA GTCTTTCGCA CTATTGGGAT TGCCGACGGT GTCGGCCTCG CGCCGATCTT 129840
TGGTTTGCTC GGTTTTGCCG CCTAGTGCAG ATGATCCGCT TTGACGATTC GCATGCGCTA 129900
TTCTTAGATG AAGAAGTACC GCGCGATGGG GAACAGCGTT CACGGTTTTT GCCTAGGCCG 129960
GTGGAACACG AAGGTAAGGG CACTTGTCTG CCGCGCCTGC CAGACAATCG GAACCGAAAA 130020
CACGGTCCAC CGCTATTGCA CGCCTCTTAC TGCCGCCCAT GTTGCAAATT TAACCCGAAT 130080
TTTCGGAGGT ATATCTCATG AATCGTAGGA CCGTGCTGAT GCTCGCATGC ACCGCAGCAG 130140
TCGCCACATG GCTCGTAGCC TCGCCGGACG TGCGGGCGGA CCCCGTTGCG GATATATGTT 130200
ACCTTTTTTG CCCAGCGGAG CAGGATGACA CCGTCGGCGC GTTTTGTTCA TCTTTTTGCT 130260
CCTCATCTTC GTCCCTTCCG CTTCCAACGG ACCCTCAGAG CGGAAGCCAG GTAGAGAGTC 130320
AAAATCCGAC TCGAACCACA GACACTGCGA GCCGAACGTC AGAGAAGCCG TGACAGGAGT 130380
AGTACAATTC AGGCCTTGGC GGATCGTCTG GGTCTCGAAA AAACGCTCTC CAGTTGCAGA 130440
GCCGCTGTCA AAGATCGGTA ACACGTTAGC GATTGTTTCG GCGAGCGATG TGGCTCGACG 130500
CGCATCGCGA TCTGGTTGGC TTAATGGCGC GAGACCTGTG TCGGCACAGC GGTCAAGGTA 130560
ACCGGGCGCC AAGGCCTGCC TGCGGAGGAG GTCCTGGGCT GCGCTGCTAA TGTCGCTTGG 130620
CACGTAAATG TGAGAATGCG GCTGTTTCAG CTGGGCTTAG CATTTAAAGC GGGTCTTCCT 130680
CACTTTGTGT GGTTCACTCG CCGTTAGACA TCCTGCCGCT ATGGGATGCA AACCAAATCG 130740
AAATGGTCGC CCGGCCCAGG GGCAAAAGTT TTGGGTGTCG CGCTCACCGA TGATGACAGT 130800
TGGGTTGTTT GCGCTGCCGG ACCAGCGTTC GGCATTTGCC CCCCCCTGAT TGCGGACGGC 130860
GGACCCGAAA TCGGCCGGCT GGACCCCAGA AGCCTGCAAG ATCTGCCGGT CCAGGGCAAG 130920
ACCGTAACGG TGAAGCTTCG GTTGAGCCGC TGGCGGTGCG CACATCAGAA CTGTCAGCGA 130980
CAAACGTTCA CCGACTGACT GCCGACGATT GCTTCCCCTT ATGCGCGCCG GACAAGGAGG 131040
GTCTCTGAGA TTGTCGGTCT GCTCGGTCAT AGTGCAGGCG CCGTCCCGGC GAGCGCTTGA 131100
TGCGAAGGCT CGGCATGCCG TCCGCGACGA CACGATCCTG CGGCAGCTGA AGCGGGATGC 131160
CGCGCCGCGC TCGCTCATTG CGACGCCGCA AGCGCGTCAG GTGGCTGATC GATTCCATCT 131220
TTATGCAGAT CTGCGGGTTG CAATCGAGGA GCAGATGAGC CTTTCCGGCC GTGCCAGGGG 131280
ACGAGCATTG CTGCCGGACA AAATCATCGG GAACGCGCAA GTCGATCTGA TTCAGGATGA 131340
TCCTCACGTT GACGCAACGC ATCGGCGCCG GGTGCGTCAC GGTCATCGAC AATCACGGCA 131400
GGTGGTGTTC GAGACTGTGC ACGCCTTGCG CAAGAAAGGT CTGTCCTGTT CGGCGATCGC 131460
ACGTCGCACC GGCTACGGCC GGCGCAGCAT CGCAAAATGG CTGACTTTCG AGACGCCACC 131520
CGACCGACGG AAGTCGGTGT TGAAGCCGAC ATCACCCATA AAAGATGGCA ATCGCTGCGG 131580
ACGGCATCTG TACACGATAT CAAACAGCGC GGTTACATCG GCAGTTTCTC GAATCTCGAG 131640
CGACTTCTCG CAGCCTGGCG CCGCGCCGAG AGGTCGAGCA AGGACAGTGC GTCGCCGGCT 131700
CCTATCATAC CCGATCAACC GGCTCGCGAT GTTGTCCCGA TGCGGGATCC GGAGACTGGC 131760
CACGTGATCT CGCCGGTGGT CGCAGCTGCC CTCTGCATGA GGCCGCGAAG CATGCTGACG 131820
ATCAATCAGG CGAGCAAGGT GGACGCCCTG AAGCAGCGAT CACATGAGTT TGTCCTGATG 131880
CGCAGCCTTG CCATGCGATT TCTGGGGCAA CGGCCAGGCA GAAGGCCAGA TCGACCGCCT 131940
GAAGACGATC AAACGTGCAA TCGATGGCAG AGCAGGCGCG GAGCTCCTGA GAGCACACAT 132000
GCTGCCGCTC GACAATACTC ATCACCACAC AGAGTGGCCC CACACCCTTG AGGAGATGGT 132060
CCTTTTACGC CGTGCATCAC GGTTTATCAG GCACCGGCTA TGTGCGCTAC GACGACCGAC 132120
TTAATTATCG GCGGCACGCT TCGGAGCTCG GCGAGGGAGA CATTCACTTT GGCAAGGGCC 132180
AAGACGGCAA TGCCATTCCG CTCAGCCGCC GGCCAGCATC CAGCGGATCG ATAGATAAGC 132240
ACCCCGAAAA CTACCTCGCC GCAGTATACC CGTCGCCTAT GGATGTCCTA AATAGGAGAG 132300
TTCGTCAGCT TTTCGAAAGC TCAGCCGAAT AGCAGTGGGA AGGCCAAGAC CGACCAACCT 132360
ACCTGAATTC AGAAATGGGC CTCCATCCAA ATTTCGCAAT GCATTACTTC AAGCAAAATC 132420
TCGTGCCGCG GAGGCAGTCG TCACCTGAGG CGTTAACCGA CCAAGTGGAG CTCTGCCCCA 132480
GCGAGCCACA TAACTCGCAT GCAGCTCTTC ACTGCCGAAA CTCCTCAGAC GCCAGTAAAC 132540
CCTGCTACCC GGCAAGGACG AGTATCTCCC CGTGCGGATA CAGGGCGGCC GAATTGCTTT 132600
ATACGAATGC AAACGACATC TGCGCCGGGC CGACTGCGGG GTGGCTCCTG AATCTCAATA 132660
ACAGCGCGCC GCCCCGAACG AACGCGCTAT CCGCGGCGAG GAGTCTGCAC CTGCGCACCA 132720
CAGCGAGAAG TTCATGCGCA TTTTGGTGAC TGGTGGCGCC GGCTTTATCG GATCAGCGCT 132780
GGTTCGGTAT CTCGTGAGCA TCAACGCGGA GGTCCTGAAC GTTGACAAGT TGACCTACGC 132840
TGGCAACCTC GCTTCGCTGA AGCCGGTCGA AGGTCTCCGC AACTATCGGT TCCTTCGCGC 132900
CGATATCTGC GACCGAGTGG CGATAAACGA AGCTTTCGAG ACGTTTCAGC CGGATTACGT 132960
CATTCATCTG GCGGCGGAAA GTCACGTAGA TCGCTCGATC ACCGGAGCGG ACGACTTCGT 133020
CCAGACTAAC GTGAACGGAA CTTTCACAAT GCTGGAGACA GCGCGGCAAT ACTGGAGCAA 133080
TCTGTCCCAG AATCGGAAGG CATTCTTTAA GATGCTGCAT GTGTCGACCG ACGAGGTTTA 133140

TGGCTCACTT GGAGACCGCG GTCAGTTCGA GGAGGTTTCA CCGTACGACC CATCTTCTCC 133200
CTACTCGGCT TCAAAGGCGG CGAGCGACCA TTTTGCAACC GCATGGCAGC GAACATATGG 133260
GCTTCCCGTG GTCATTTCGA ATTGCTCCAA CAACTATGGA CCGTTCCACT TCCCCGAGAA 133320
ACTGATCCCG CTGATGATTC TCAATGCATT GGATAGGAAG CCTTTGCCCG TCTATGGGAC 133380
GGGTTCCAAC ATTCGCGATT GGCTCTATGT CGACGACCAT GCCCGAGCCC TTTGGCTGAT 133440
CGTCAGGGAA GGCCGTCCTG GTGAGAAATA CAATGTCGGA GGTCGCAACG AGTTGCGCAA 133500
TATCGACGTC GTCAACCGCA TATGCTTGCT CCTCGATGAG CTTAGTCCCA ACGCTTCGCA 133560
CTATGGTGAC CTAATTACTT TCGTGAAAGA CAGGCCGGGT CACGACGCAC GCTACGCCAT 133620
TGACGCCACG AAGCTCGAAA CCGAGCTTGG CTGGAAGGCG CAGGAGAATT TCGATACCGG 133680
CATACGCAAA ACGGTGGAAT GGTATCTGGA AAATGGCTGG TGGTGGCAAC CGCTGCGGGA 133740
CAAGGTTTAT TCCGGTGAGC GCCTCGGTCT CCTGGAGAAA GCGTGACAGA TGCGGCTCGC 133800
GGTGACCGGC AAAAACGGAC AAATCGCCCT CGCTTTGAAG GCGCAGGCGC GACCTGACGT 133860
CGAGATACTT ACTTTGGGGC GGCCGAATTT CGATCTGGCT TGCCGCTCGA CTGTCGCAAG 133920
CTCCATCAGG GATGCCGCAC CGGACATAAT TGTCTCTTTG GCCGCCTATA CCGCCGTCGA 133980
TAAGGCCGAA AGCGAGCCAT ACGAAGCCTT CGCTGTGAAC CGCGATGGAG TGCAGGCACT 134040
GGCCGAGGCA GCCGCCGGGC TCGGTGTCCC AGTTATTCAT CTTTCGACAG ATTACGTGTT 134100
TGACGGTGCG AAGCCGGTGC CTTACTGCGA GGAAGACCGC ACAGGCCCGA TTTCAGTTTA 134160
TGGCAGGTCC AAACTTGAGG GTGAATTTGC GGTTGCCTCC GCCAACCCAA ATCATACGAT 134220
CCTGCGCACT TCATGGGTCT ATTCTCGATA CGGCCAAAAC TTCGTGAAGA AGATGCTCCG 134280
GCTCGCCGAT ACGAATGACG AATTGAATGT CGTCGCCGAT CAGCTCGGCT GTCCGACATC 134340
GGCTGACGAC ATCAGCGTCG CCGTCATGAC GATAGCCAGA CGGATGCTGT CGAGCTCATC 134400
GGCTGATTTG CGCGGGATAT TCCACCTGTC AGGATCGGGT GAGGCCAGCT GGGCGGCCTT 134460
CGCGAAATAC GTCTTTTCCG TTTACGACGA GATAACCGGG CGCCAAATCA AAGTCCATGA 134520
CATATCCGCT GCAGAATATC CCACACCGGC TCGCCGCCCA GCAAATTCGA GGCTCCACTG 134580
CGACAAGCTT GAGCGAACCT TTGGTATCCG TCTGCCAAAT TGGGAGGAGT CCACGCGCCG 134640
GCTGGTTTGG GCGCTGCTTT TAGAAGGGAA AGATGCGTGA AGGGAATTAT TCTAGCAGGT 134700
GGCAGCGGTA CGCGCCTTCA CCCGATGACG CTTGTCATGT CAAAGCAAAT CTTACCTGTC 134760
TACGACAAGC CGATGATCTT TTATCCGCTG ACCACGTTAA TGTTGGCCGG AATCCGCGAA 134820
ATCCTCATCA TCTCCACGCC GCACCACATG CCTCTCTTCC AGGCATTGCT GGGGGACGGT 134880
TCGCAATGGG GGATCTCCTT AAGATATGCT GTTCAGCCCA GCCCGAACGG GCTGGCGCAG 134940
GCCTATGTAA TCGGTGCCGA TTTCGTAGCT GGTTCACCCT CTTGCCTGAT CCTCGGCGAC 135000
AACATTTATT TCGGCCACGG CTTGCAGGGA CTTTTGCAGC AAGCGGCCGC CCTCCAGCAA 135060
GGAGCCACAA TTTTTGCCTA TCATGTCAAC GATCCGGAGC GCTATGGCGT CGTTGAGTTC 135120
GATGAGGGCA TGAACGCGCT CTCGATCGAG GAGAAGCCTG CGGCACCGAA ATCGACCTGG 135180
GCCGTCACGG GACTTTATTT CTACGATTCC GAAGTTGTTG ACATCGCCGC CAATCTCAAA 135240
CCTTCAGCGC GTGGCGAATA TGAAATCACC GACGTCAACA GAATTTATCT AGAGCGTGGG 135300
AAATTGAAGG TTGCTGTTTT GGGGCGCGGG TACGCTTGGC TCGATACTGG TACTCCCGAC 135360
AGTTTGCTCG AGGCTGCAGA GTTTGTGCGC ACACTCGAGA AGCGGCAAGC ATTCAAAGTT 135420
GCCTGCCCGG AGGAAGTCGC TCTGGCAATG GGCTTCATCT CAGTAGAAGA ATTTGCGCGA 135480
ATTGCGGAGA GGGCCGGAAA GGGAGATTAC GGCGCCTATC TTCGGCGGCT TGCGTGAAGC 135540
GCCGCGTGAT CAAGATGGCA ACAAGGGCTA GCGACATGCA TCTTCTGAAC GTTCCGCTCA 135600
GTCAGCCGCA CTTCTTTGAC CCCCACAGTG CGTGGCTGCG ACATGGCCCC TTTGGGATGT 135660
GGCTGGTTCA CGCGTTGCGG CCGCGGCGAA TTGTGGAACT GGGCACGCAT TACGGCTTTT 135720
CCTACTTTTG TTTTTGTCAG GCGGTGGCCG CGGGTGATCT GGCGACTGAG TGTTTTGCGG 135780
TCGATACATG GCAGGGCGAC GAGCATGCTG GGTTTTACGG CGAAGAGATC TATCATCGCG 135840
TGATGGCCCA CAATCAGCAA TACGTCAGGT TCAGCAGGCT TTTGCGCAAG ACCTTCACCG 135900
AGGCGCTGGA CGACATCGAG GACAGTTCCG TTGATCTGCT GCATGTTGAT GGTCGCCACT 135960
TTTACCACGA CGTAAAAGCG GATTTCGAAA ACTGGATTCC TAAGCTGTCG CCCCGCTCAG 136020
TTGTGCTGTT CCATGACACT GAAGTCCGCG AGCACGGGTT CGGTGTTTGG CAATACTGGG 136080
CGGAACTCGC AGCCATCCGT CCATCGTTGA ACTTCCCCTA TCAGCATGGG CTGGGAATGC 136140
TGTTCTGGGG CAAAGAAATT GCGGAGGGGC TCGCGCCATT GGCGCGGACG CTTACCGACA 136200
CAGAACGGCT CAATTGGCCG GTCGAGTACT TTGCCTTGGC AGGTGAAAGC TATGTGCGCG 136260
ACGCCACGCA ACGGGGAGTC ATAAAAAGGT TAGAATTGGC GGAAGCGACC ATCGAGGAGC 136320
GTAAAGCCGA GACCGTGTTG GTGCAACAGA AGCTGCAGGA AGCGCGCCGC CGACCGCTCA 136380
AACAATTAAA ACGCAAGCTG GTTTTCAACA TGTTGCGTGC AGCTGCGAAG GCTAGCCCGC 136440
CGCTGCCGTC GCGCACCGCT GAGCGATTTC GCCGTTCGGC GGCCAAGCGC GATCCGATGC 136500
GAGACGATCT GCAGACACTG AGCGGCCAAG GCTTCATGAC CTATGAGGCG GTGGTGCGAG 136560
GCTGGGGGAA GCAGCGTCAG GCGCTGGCCG GCCGGCTGAG CGAGCTTGTG CGGCGCTTGC 136620
AGAACGGACC GCTGATATCT GTAGTCGTGC CCGTTTATAA TCCCGATCCG GCCCTGCTGG 136680
TCGAAATGAT TGAGTCGGTT CGAGCGCAAA GCTATGCCAA TTGGGAATTG TGTCTGGCTG 136740
ATGATTGCTC GACCGATCCC GAAGTGGGCC GCGTTCTGCG CAACTACGCC GCACAAGATC 136800
CGCGAGTGCG CGTGGTCTTT CGTGAAGCGA ATGGTCATAT GTCGCAGGCG AGCAATAGCG 136860
CCATCGAGAT TGCGAGGGGC GCCTACATTG CGCTTCTCGA TCATGATGAT CTTTTGGACC 136920
CGGATGCGCT GGTGCTGGTG GTGCAGGTTA TCGATGCGCA TCCTGATGCC AAGATCATCT 136980
ACACCGACGA GGATAAGATT GTTGAGGGCG GTACTCGTTG CGATGCGCAT TTCAAGCCTG 137040
ATTGGAACCG CGATTTGCTT TACGGATCAA TTACATCTC GCATCTGGGG GTTTTTGATG 137100
CGGCGCTTGT GCGCGAGGTG GGGGCGTTCC GCGAGGGCTT TGAGGGCGCG CAGGATTACG 137160
ACATGCTTCT GCGCTGCATC GAGCGTGTCC AAGATCGCCA GATCCATCAC ATTGCCAAGG 137220
TGCTATATTC GTGGCGTGCA ACTCCTGGCA GCGCGGCGGC TTCGAATAGG GCGAAACCCT 137280

ACGCCAACGA AGCGGGACGC CGCGCGCTGG AAGAGCATCT GGCGCGTACC ACGGGCAAAT 137340
CGATTCCAGT TGTGCTGGGC CCGATTCCCT TCAGCTATCG CGCGCTTTGG CCGATGGAGG 137400
GGACGCCGCT GGTCTCGATT ATTATTCCGA CCCGCGACCA CCTAAACGTG CTGCGCGCGA 137460
CGGTGGAGAG CATTTTGGGT AGGACTATGT ATGGCAATTT CGAACTGATC GTTGTCGACA 137520
ACGGCTCGGT CGAAGCGGAT ACCCTGGAGT GGTTCGGTCA AATCGAGGGC AGCGATCGGC 137580
GTGTGCGCGT GCTGCGAGAC GCCCGCCCCT TCAACTATTC CGCGCTGAAC AATGCCGCCG 137640
TTGCGCAAAG TCGTGGTGAG ATCGTGGCGC TTGTGAATGA CGATGTCGAG GTGATCGCGC 137700
CCGACTGGCT ATCCGAGATG GTGGCATTGG CGCAGCGTCC GGGTGTGGGT TGCGTGGGTG 137760
CGAAGCTGTA CTATCCAGAT GGCCGTATTC AGCACGCTGG TGTGGTGATT GGTCTGGGAG 137820
GTGTGGCCGG GCATGGACAT TTGCTCTATC CGGGTGAGCA CGCCGGTTAT TTCTGTCGAC 137880
TTAAATTGCG GCAGAACTAC AGCGCTGTCA CCGCGGCCTG CCTTGTGATC AAGCGCGAGA 137940
TTTTCGACGC GGTTGGGGGG CTGAACGAGT CCGAGCTGAC CGTGGCCTTC AGCGACATTG 138000
ATTTGTGCCT GAAGGTGCGC GCGGCAGGTT ACAACAATGT CTGGACGCCA TGGGCTGAAC 138060
TCTATCACCA CGAGTCCGCC AGCAGGGGCC ACGAAGACAC CCCGGAAAAA CGGGCGCGCT 138120
TCCGGCGAGA GGTCGACTAT ATGAAAAGGC GATGGAAAAC GCATGACTTC GCCGATCCGG 138180
CTTACAATCC AAACCTAGCA CTTGAGAGAA ACGATTTCGT ATTGTCTTCA CCTCGTTGGT 138240
GTATCAGCAC GAAACTGACG TGATGGCCGA ATTGGCTGGC CCCTTACAGG CGTGTAGCGC 138300
TGCCGTGGCA ACTGTTGGGG CATCCAGGCA GCTCGCGCAT CTGAACCAGC TTCGCTTGGG 138360
GTCGCGTTCG ACTATCACCA CGCCAGAGGA GCTCATCGGG CACATGGGCA AGAGAGTCAT 138420
GCAGTCGCAA AGAATGATCC TGAACTGCGA ATCCGTGACC TAACTGAGCT GGGTTTGAAC 138480
GAAAGCGCAG ATTCCACCGA TTCCGCGTTG CGTGCCTGCG ACGGGATTGT CGACGCAACC 138540
GCCGACGCGC ACTGCTTTAA TTTTTGCGCC GCGGTCGCAG CCTCTCATAT GGGGAGAAGT 138600
GTTTGTCGGC AGCTTCTGCT TTCCCTCTGC AAGGAAGCCT GACAGCAATC TCTCCATTGC 138660
CGCCGTCAAG TCGTCCGGCT TCTCTTCGCG GAGCCTTTCC GCCTATGCGA CGAAAGCCGA 138720
GAGCAGGCTC TTCATTTCAT CCGGCGCGTT TCTCGAACCG CCTGAGCATC TCCAGCCCGC 138780
TTTGTGCATG GCCGAGAAAA TCGGCGTTTC GCTCCGTAAT CTCGGCCAGC ATCTCGTTTG 138840
TGATGCGCTC GCTTGGCGCG GCGAATTCAC CGTCGACCGC AGTCCACGTA AAGGACGCGT 138900
TCTTGGCTCG GGGCGACGTC TCGTGAAACT CGTCCTGGCT CTGCCCGAAC GCCTCCATGG 138960
CAGCCTCCCA GTCGCCACGG GCTTTCGCAT CTGTTTCGGC CTCGGAGCCC TTCAGCATAT 139020
ACGCATTCGA TTTGTTCTTG GCCGCGTGAC CCCCGAAGGC TGCCAGCACC TTCTTCTGTT 139080
CGAGCTGATC AGCACGGCCC AGTCCCCTTC CGGGCGTACC GGGGATCGCT TGCATTCGCC 139140
GCATTAGCGT GGCGATCGTT TGAGCAATCT GGGTTTTGCG TCGCGGTCGA CAGATACGGC 139200
CACAGGCGCG GAATATCGGC CGCCGGAGGG CCGGGTGGAT TTGCCAACGT TGGCAGGGGG 139260
CGATACGCAA CGCGGCCTGG GAGCGAGATA ATCCAGTACT CGCCGAAGCT CCCACTGATG 139320
AGTCTGCTCT GGATCGAACG CTACGCGCTC TTGGACGAAA GCCGACGGAG CGGATTGATC 139380
CTCCTGCCCT TCCCGAAGCT CCTGTAACCG ACTGCTAGCT TCGCGCGACG GCCTGGGCTG 139440
CGCAGTGGCG CCGCCGATAA GCCTCGGTTC CATCAGTACT GCGCTTTCAG GATTGATGGG 139500
GACCACAGTC TCGAGCTGAG GACTGAGCTT AGCTCGAGGA CCTACGATGG GCGCGCCCGC 139560
CGACCGAAAG GTCCGGAGAT GGTCTATTGC CCCAAGGAGT TTGCCGGGAT TACCCTTTCC 139620
GATGAACGCG GACACGTCAC CGCTCAATGA CTTACTGTCG ACCTGAGCAA CAAGGCTTGG 139680
TTTGTTTTTT GCGAAGAGCC AACGGCTAAA GCTACGAAGA GGACTCGCAT ATGTGCAGGC 139740
GGAGTCTCTT GCCATCCCGG CCTTGATGAA GGCCTCCTCA AGCCCCAAGA GTTCGTGAGC 139800
ATCCTCGGAA TAACAGGCCG CTCCCACGGA TCCCCGCGGG CCACAATCCC AGTTGGTTGA 139860
GCCGGTTGCA GCGCCGAGGC GGCTGGCATT GCCCCACCAC CTTCACCTGA ATTGGCGACC 139920
TCGCTCAGCT GCTGCTCAAT GGCAAGGGCT TGCGGATTGT TTAGGGTCCT CGGCCTCTTC 139980
GCTGCTCTCA ACTCAGCTGT GTCATGCTCA TCGTTGATGA GGATTTCCTC GCTTGGCAAG 140040
AGGCTGCTCG TGGCAACTGC ACCCATCGGC TCGTCCGGGG ACTGCTGGCT GTCAAACACA 140100
TTCGTTGAGG GACTACGGTG TGGCCCCTGT CAGGCATCGC GTTATCGGCT TGGCGCGCCT 140160
CGTGTAATTG CTGCTCAAAG TCCTCTTGCC CGGATCGGTC CGGTTGATGC TCTTGCGAGA 140220
CCTCTATCTG CGGCCCGGCA GTTACATTGA ACGATTCAAC ATTGTGCGGG TCCACGCTAG 140280
CCTCACGCCA TGAAATCAAT TGAGCTGGTC CTATTAGGAT AAGCTTTCGA GAAGCTGACG 140340
AGGACGTCGC TCGTGAAGCA AACGCACCCC GAGAATGCCC TGCGGTCCTC GATGGGTCGC 140400
GGGGCCTTTT TGCGTTTAAG GACACGTCGT CGCGGCTAAT ACAGCCTTTT GGCAGCGCCG 140460
GGGGCGAGAC GCGTTAGCGC TCTGTTGCGA AGGGCACGGC ATACCGGCGC CGCCGATCGT 140520
GTCGAGCGTG ATACTTGCAC TACGGACGTC GAGGCCGTGC CCTGCAAAGG GGGCGCGCAT 140580
GGCGCCAGCA TATAGCACGA CACGCGTGAC CGAAAGATGA CTGACGGCTG GAGATGTGGG 140640
GCAAGCGCCT GACGCACTAC TCGCCATGTC GGCCCTGGGT TTGCAACAAA GGCGTCGTCA 140700
AGTCGGACCC CACACAGCCA AGCTCGGAGC CGATCGTCGG CGATCCCGAA TTTCGGCAAG 140760
ATTGATAAAC TCCTCAAGGG CTGGGATCGT CTCATCCATG CGGCCACTGC TTCATCACGC 140820
CAATATTGCG CGAGAACGGA GAGCACGTCA TTGCCATGTG CAGCTAACTG GCTTTCGATC 140880
AAGTACCTTC GCTTCACTTC CCGATACCGT CCGCATGGCA CTTGAATCCC CCGCCATCAC 140940
GATATGATCT GTTTGCGGGT AAATCGATCA ATCGTTTGAA CGGGTCTCGG GATATTTGCG 141000
CGCGCCGCGA ACTGCGGGTG CGAGCGCCCC GCACGGGGAG AGACCGTAAG TTCGCGCCCA 141060
TTCATTCTAA ACCCGAGTGT TTTGGCGTAT CCATAGTGCT GATGAGTGGC ATGCAAACAT 141120
TCAATTTTTC CAGCGCCACC GATGTCTCGT AAAACTAGAG ATGTAAATGT GATTCAGGGT 141180
GTCACAGAAG AAATTCGGAC TTGAAAAGGG GAGGAAATGT TCGCTCTCAG GATAATCCAT 141240
ATCGCCCTAT CGTCGCCACA AGGAGACTTC CAAGCGGAGT CCTTTGCGCG CGCGCTCAGG 141300
TCCGGTGGAT ACTGTGCAGA AGAAGCAGTG GGGCGCCATG CGTTGTGATG AAAGATTTGC 141360
CGCGACTTTT TTCGTCTACA TATCATCCGC GTCGACTCTA CAGCCGGTAC AAAAATCGCG 141420

CCACGGCCCC GTTTTGTTTG AGGGAATGCC GGGGCCCCCT TGCAGTGGGC GCGCAGCGGT 141480
CGGCTGTCGC GGAGAAATGC ACAGGGCATG CTGCAACTGG AGTAAATGTG TTGCGCGGCG 141540
TTGTGTACCG ATCAGGCGAG CTTACCAGTC GTAATATCGC GCCGGATCTG AGTCTCCCCC 141600
AAGTGGCGGT CTGCTGCATC CGATTCGGCA ACCCCTTTCT GCGCGGCAGG GTTGGGAAAA 141660
GAAGACACGC GCCCGCTGAT TTTTCCTGAG CCCCTTTTAG CTTGAAGCTA CTTGAAGCAG 141720
TTAACCGCAC GAGAGGGCCT GCACAATCAA GAGTGAAGTA GTGACACCTG GAGATAAAAG 141780
ATCACAAGAA CTGCTGATCG AGGCAGCATG GCATGCATAT CTTGCAAAGA ACTTGCCAAA 141840
CACAGGGATG CGCGATGTTT TCATTGCGGG GTACGTGGCT TGCAATTCAA TTGCATCTCA 141900
CGAATGTGAA TGTAAACCCG ACTATGGTTC ATGTTGGGCC GTATCTAAGC TGGTTAGCAA 141960
GTTCCGCATC TGGCGACGCA ACGCAAAGTA AACATGCGCG CTACGCAATA TCAATCGAGT 142020
AATTGATGGA TACAATGGCC AATTCAATAT TAGGACAGGA AATCACTTTC CTCAAACTTG 142080
ATACCCGAGT GATCAAAAAT TTCGACAAGG TTCTTACGAA TAATATTGAG GTTGTGGAAA 142140
CGTCTCTGTG GTCTGTAGTA AGAAAGAACG CCTTAAAAAA GGGCGCAGCA AAGGCACTGC 142200
AAGCCTGCGC GTCGTCAGTG CTGCACAAAA GATCGGACTA TGAATTCTTG ACCACCGGCA 142260
ATGTGCTGCG AAAAAAAATT CGCCTCCCGC GTTTGTTCGG CAATAAGGTA ATTTCCGCGG 142320
CTTACTCGTG GATAAAACGC GTGCAGGCAG CGCTTTTGCT CGATTATTTG AAAGAACATT 142380
TATCCCAGTA TGACTGGGAC CAGCTGATAG TTGTCGTGTT TAACGGGTCG AATTATCCAG 142440
AAAGCGTGCT CGCCGAAGCA TCGAAGGGAT TTAAGCGCGT TTTTGTTGAG GATGGGTTTT 142500
TTCCTGGGAC GCTCCAAATC GACCCAGTAG GAATAAACGC TGCAAACTCC GTGCCTCGCT 142560
GTAGCGCTTT TTATAAAAGC GGCAGGGATT TTTCGGAGGG AGGGTTACCA ACAGCTGTGA 142620
CCAATAGGTC ATCAAAAAGA AAATTTACCC CTGTCGATCT GGCCCCGGGC TTTGTCTTTG 142680
TACCGTTTCA GGTCCCTTCC GATATGCAGG TAACGCTTCA TTCGCCTTGG GTGAAAGACA 142740
TGTACAATTT TTACGATATC GTTGTTAACG CAGCTGAGCA AAATCCTGAG GAGATGTTTG 142800
TCATCAAGGA GCATCCTAGA TTCAAGAGAT CGGTGATCGG CAGTCGTCCG CCACACCCGC 142860
GCGTGAAATT TGCAAACGGA AATATCACCT CCGAACTTAT CTCCAATGCG AGGACTGTTG 142920
TCACGATAAA TTCGACGGTA GGCATTGAGG CTCTGCTTTT GGGGAAGCAG GTTATTACGC 142980
TTGGCGACTC CTGCTACAAC ATTCAGGATC TTGTGTTGAG AGGAAATGAT ATGGGCCGGC 143040
TCAACGCCGC ACTTGCGCTT CGGGGATGGC TTCCCGACGA TGAATTGCGG AGGCAGTTTC 143100
TCGGCTTCCT GTGGAATTAC TACTTAGTTA AGGGTAGCTT TACAGAACCA CCAACCGCCC 143160
TGGCCTCTCG GATTCTCGAT TGTTTCGAAC TGGACTCAGA AGTGAGAGGT GCCATCGCAA 143220
ATTTGAACAA TTGAATCCAT CTCTGCAGAG TTCTCTGCGC CTCTTCAGGT TGTGAGTGTT 143280
TTACCTTATG GTGGTGGGCG GCCTCGGCTC CCGAGGCCTC CTCCACCATC AGACCACGTC 143340
GTCCATTTTT TATTGCACGC ATTTCGGGCT TGCTTGAGGA TTGAGGCAGT TCCAGCGTTC 143400
TGTTGATGGG ATTGCAAACG TTGCCGATGG TCTTCGAGAG TGCGTCGAAG GTCGTTTTGA 143460
AGTTGGCGAG AGCTACACCT TCACGTATTG AAACGAGACC GGAAGTTCGC AGAGTGATGG 143520
CTGACTGGAG TCCTTTTCCG ACAGAACGTA CGTGGTCATC TTTGGCCAAT TGATTCCGAT 143580
GGCGGGATCG TCCCATTTCA GTCCGCGATC ATGTTCAGCG CTATAGGGCG CTGTCACCTT 143640
GTAGCTGATT ACCGTGTCCG CCTTAAGCGT CATGAAACCA TGCGCGAAGC CCGCAGGAAC 143700
CCAAAGCTGC GCGCCATTTT CCTGAGATAG CTCGGCGGAA ACCCATTTCC CGTATGTTGG 143760
TGACCCGACC CTGACGTCCA CCGCGACGTC GAACAGCGCG CCGCGTGTAC AGCGGACAAG 143820
CTTTCCTTGC GCGAAAGGCT CGAGCTGAAA GTGAAGGCCC CTTACCGTCC CGATCTGAGC 143880
GGAGAGTGAT TCATTATCCT GAACAAGTCC GACATCGGCA ACATTTTTGC GGAACCATTT 143940
TTCCCTGAAT ACTTCGCTGA AATAGCCGCG GCAATCGCCA AACTTCCTAG GCCTAATTAA 144000
CTTGACTTCG GCTATCGACA GGCTTTGAAA ATACATTTCG TCCAACTCAG TTCCGCCCAC 144060
TAATTCTCGT GCTCTGGCAA GGAGTTGAGG ATAAAAACCG AACCTCAACT CATAGCGCGA 144120
TTGTGGGCTC CGATCCAGAG ACTCACTTAG GCGCTCGGCA ACAGCTGCAA CTCATAAAGG 144180
CGGCGGTATA CGCCATCTTG TGAAAGCAAT CTTCCCTGCG GTCCTTGTTC GGCGACCTGA 144240
CCGCCTTCCA TGACCACGAT GTTGTCAGCA GCTGTGACCG TCGAGAGGCG ATGGGCGATC 144300
ATGATAGTGG TCCGCTTCCT TGTCAGCCGG GCCAGAGCTT GGCGAATCTG GATTTCGGAT 144360
TCCGAATCGA GTGCGCTTGT CGCTTCATCA AAAATCAGGA TTTCTGCGTT CCGCAGCATC 144420
GCGCGGGCGA TGGTGATGCG CTGTTTCTGG CCACCTGAAA GTTTTATGCC GTTTTCGCCG 144480
ACCTCGGTGT CGTAGCCATG AGGCATTTTC ATAATGAAAT CGTGAGCGTT GGCCGTCTTG 144540
GCCGCCTCAA TGATCTCCTC GTCGGACGCA CCCTCGCGGC CCAAAGAGAT GTTGTATTTT 144600
ATCGTGCCAC TAAACAGAAA TGTGTCCTGG CCAACAAATC CTATCCGATC GCGCAAGGAA 144660
CGGAACGTGA CATCCTTCAG GTCGTGCCCG TCAACAGTTA CCGAGCCTAC GTCGGGGTCA 144720
TAAAGGCGCA TAATCAAGTT GATAATCGAC GACTTTCCTG CGCCGGAAGG CCCCACCAAG 144780
GCTGTTGTCT TTCCGGCTGG AAATGTGACG TTCAAATTCT GGAAAAGTCG TTCTCCGTTC 144840
TTGTAGGAGA AGTTGACATC CTTGAAGCGG ATTTCACCAG GGCCCTCAGG CAATGGTATT 144900
GCACTATTCT TCTCGGTCAG TTCAATGGGA TGATCTGCCA GTTGGTACAT CATGCGAACG 144960
CCTACGAGTG CCGATTCAAG ACTGATGCGC ATTCGCGCAA GGCGCTTTGC GGGCTCATAG 145020
GCCAGTAGCA ATGCAGTGAT GAAGGACATC AGTTCGCCGG GCGTGTTGCC CTGCTGCAGC 145080
ACCAACACTC CGCTTAGTGC GATCACACCG GCAATGGCGA AACCTGAGAG GGTCTCCATG 145140
ATGGGGCTGG ATGCAGCTTC CAGCCTTGCG ATTGAGTTAG CCCGCCGTTC GACATCTCCA 145200
ATGTACTTGT CCATCCGCCT TCTCATGAAA TCCTCAAGCG CGAAGGCCTT TACAATCCGA 145260
ATTCCCGTCG AGGTCTCCTG CACGCTCTGG ATTATCTGTC CGATTGAAGC AATCTCCAAT 145320
TCCATGATCT TCCGCACTTT TCTCGTTAGC ACACGAACCC CGAGGATAGC ACCAGGTCCA 145380
ACAGCGGCGG AAACAAGTGA AAGTAACGGT TGTTGGATCA CCATGACCGC GAGCAGCCCC 145440
ATTAACGAAA ACAGATCCCG TATGAAGGAG GTTACGACGA GCTCAATGAC CGAACGTACC 145500
GCCTGCGCAT TGTTGGTGAG GCGAACCAAC AGTTCTGAAG ATGGATAAAT CGAATAGAAA 145560

GACAGACCCT GCCTAAGTAC GTGCTCAAAA AGCCTGCGCT GCGTATGTGC GATTATATTG 145620
TTGCCAGCCT TGCTGAGGAA TATTGACTGC ACGTATGTCG CAAGTCCCTT GACTGCGAAA 145680
ATAATCGCGA CTGTCACCGC GACGCCGAAA ACCTTTTCAA TGTCTTTTGA GACGACTGTG 145740
CTATTGACAA CATCACGCAT TATCCAGGCG CTTGCGGAGG TCATCACCGC TACTACGACC 145800
ATCGAGCCGA TTGCAATTCC GTACCAAGGT GCCTGGTGTT TGAAATTTTC AGCTAAAAGC 145860
CGCATGAGAA GGCTTTGGTT TGACTTGGAA ATGAATTTCT GGAGCATTCA ATTATCACCT 145920
TTCCAGTCCG GTCTTGGAGA GGAGCGGACA GTCATTTTGT TAAGCAGCAT AGGCCTTCGG 145980
TGGCAGCCTA AATGACCCGT GTGGCGTTCA AATTGCAAGA CACATGAACC TGCTTGTGGA 146040
CACAATCATT TACTAAGGAC AATTCCACAG TGCTATGGAT CAAACCCTTA GTCGCCGCTT 146100
GTTTCTATGG CACAACCCGC AGACTGATAA CCAGTGTAGA AATGAAAGAC ACATGATTCA 146160
TACGTCAAAA ATTTACCGTG AAATCAACCG TATCGGCGTG CAAATCAAGG GGCTCCCCGA 146220
CTATTGGATG GGCCAGGCTA GAAGGATCAG TTATGATCGG GTTGGGTCGG CAGGAAACAC 146280
GATAACGGAA GGTAGAAAAC CAGTCTCGGC GGACATGGCT ATTTTGCTCA TTTACCAACC 146340
ACGTGGGCTT CTAGAGTCGC TTTTTTTACA ATTAGAGCAC CTGCTCTCGA AAGGACTGGG 146400
CGTCGTTATC GTCAGCAACC GCAAGGTTTT GGAGCACGAT CGCAATCGGT TGAGTGAATA 146460
TTGCCATCTG ATCATTGAAC GTAAGAATAT TGGCTATGAT TTTGGCGGCT ATCGAGATGG 146520
AATTCTCGCG CTTCATAAGC GTTCAATTCA TCCTAAGAGC CTCTTTGTGA TGAATGATAG 146580
TGTATGGTTT CCCATACGGA AAGACTGTGA TCTTATTGAT AGGTGTCGCG AATCTAGGTC 146640
AGATATAGTA GGCGTTTTTT ATAACAATAA AAGCAAGTTC CCCAAGAACC ATCATCTACA 146700
ATCATATTTT TATCGTTTCG GTGAAAAGGT TGTCTCTGAT AGTCGCTTTC TAGCGTATTG 146760
GCGTAAAATA CCAATGTACA ATGACAAACG CAATGTCATT AGGAATCTTG AAATAAAATT 146820
AACCAAAAAT TTTCAGTTGA TGGGCTTCGG CATCAGCTCT CTTTACGCGC CTGAGGATAT 146880
ATTAAAGGCG TTTAAAAATA TCGAAGTTCG AAATATCCGT CCCGTCCTGG ATTACTATAT 146940
CTCCGCGTTG GGATATGACC AGAATACGTT CTGGAGCGCG TATAAAAATG AGTCACCAAA 147000
AGGAGGTGAC ACCCATGCAT TACCGATAGA CGTTCCTAAT AGCCGAGTTT TCTTCCATTT 147060
TCTTGACGCT CACCCAGAGA TTCTCATTCG CAAGTTAAAC TCTCCAATCA TTAAAAAGAA 147120
TCGCGATAAA CGCTTTGTCG CACAACGAAA AGCGATAATT GAGGGAGGGT TTCTTAAAGA 147180
GATTGACGCC GTCATTCAAA AAGAATTGAT CAATTGGGAT AGGTAGTTGA TATTGTATCC 147240
AGGTCGTCGG AAGCGTGAAT AATCACACTA TCAAATCAGC CACGTAGATT AAGGCGCTGG 147300
TTATGCCCCT TAGAGCAGCT GGAACGGGCT CTTGATGCCT TACGTCCGCA TTAATGGGAG 147360
TCACGCAGCA GCGAAGTGAT GTGCACATTC CTGAGGTCGA CCATTTTAGG AGACGGCGCG 147420
TGCTGAAATG CGGCAGACCA CTGCTATTGA ATTTTCATGG AATGGCCGAA AGCAAGAGAA 147480
ATACCGTGAA TATCGCCAGA TGAACGGCGC CCTGTAGTAC GGTCGTTCTG CCCGTTCCCA 147540
GTGTGATCGT ACCGACGAAC AGCGTCAGGA TCAGCATAAT GAGGTTCTGT GGCGCGAGAC 147600
CCAAAGCAAG ATCTCGTCCA AGAGCGACCG ATATGGCAGC TACTACTGGA ATTGTCACCC 147660
CAATGCTCGC AAGCGCAGAT CCGAGTACGA GGTTTATGCT ATTTTGAAGA CGATTCATTA 147720
AAGCCGCTTT GACAGAGGTT ATCCCTTCTG GCAGCAGAAC GACTCCCGCG ATAGTTACGC 147780
CCACGACGGC TTGTGGCAGT CCCAGCGCCT CGACGGCGCT GTCGAGCGGA TAAGAAAGTA 147840
GCATTGCCAA TAGGATGACT GCAATGAGGG CCAGCACAAG CAAGGCTCCG GCCGCTAGCG 147900
GATTACGCGG GGTCTCATGG GTAGCCGGAG GCGAAGCGGC GTCTTCATCA TCGATGAAAT 147960
AGTCGCGATG GCGAACAGTC TGCACAAACA GAAACACACC ATAGAGGACC ACAGACACAA 148020
GTCCTATGAC GACAAGCTGT ATTGCAGCAA ACTGCTGTGG TTTTCCCGCG GTGACGAAAT 148080
TGGGCAGCAC GAGAGACAGT GTCGCGAGTG TGCCTAGTAC CGCCAGTGCC GCCGACGCGG 148140
CATTCAACTG GAACGACTGC TCGCGATGGC GTCGGCCGCC AAGCACGAGA CAGAGGCCGA 148200
TGACGCCATT GAGCACGATC ATCACGGCGG CAAAGACCGT ATCTCTCGCG ACTTCTTCGT 148260
TTCCCTCCGC GCCGGAAAGC ATCAAAGAAC CTATGATCGC CACTTCGATG ATTGTCACGC 148320
AGACGGCGAG GAGGATCGCT CCAGAAGGTT GGCCCACCCG AGCGGCAAGA ATTTCGGCGT 148380
GATGTACGGA AGCAAAGACC GCACCTGTTA GCAAGCCGGC CGACATCAAC AGAAGTACGA 148440
CGGATCGTTC CGGCAGCACG TGCGCCAATG TCATAGCCGC GATCACACAA CCGAAGAGCG 148500
GAATGATCCA AGACCACAGC GGGACGTGTG CGGACCTGAC CTGCAATCTT GATTTCATTT 148560
TCCATCCCCT GGTGATGAGT ATGCGTGGTT CCTTGCGCGG TAATCGGCGT AACAAAACTA 148620
AGGCTTCGGA AAGTCGGGCG AGATTCGCCA AGTCTAGTCA GACTCTAGAC GAGCCGTCGA 148680
GTTTGGCGTC AACCGCCCCT CATTGCAGAT GCAAGAAGTA TGCTCTGCCT GCCGTGCCTT 148740
TCATGTGATT AGGTGCGTCA AGGCTGTCAT GTACTTGAAG GTGCACGGAG CTAGGCTCCA 148800
CAGTCCAATC CAGGTCTCCT ATTTTGTGAG GCTCTGCTCG AGGCGCACGA CCTCGCGTCG 148860
CAACGCTTCG ATCGCTGCAT CGCGATTGCG AAGCGCTTCT AGCTGTGTGG CTCGGTCATC 148920
CTCGGAAGCC ATGAGCGCCC GTTTCAGCTG AGCCACCATT GATGCGCCCA GCTGCAGGTT 148980
TTCACCTAAT TTGAACTCGG GCAGCCGTTG CGCTTCTCCA AGTGTGGAGG AGCTGGAGCT 149040
AATGGTGTTG TCCTCTTCAG CTTGCTGAAA GATGCTGTTG TACTCACACG CACCGGCGGG 149100
AATGCCTTCT CCCTCGCCAC GACGTAAGTC CCACCAGGTT TGAAATACCC GACGGTATCG 149160
TTCCGTTACC GCTTTGCCCC TGTCCACGAT CCCAGCGTCT CTTGCGTGCT GAAGTGACTG 149220
CTCGTAACCG AGTTCCACCA ATAGGTCAGC GCCGACTAGA TCGGTTACTG TTTGCATCTC 149280
TTCGATGGTC AGTTCGGTTC GCCAAGTTTC GACTGAGCGG TTGTCGACTG CCTTTTTCTT 149340
CAGGAGCCGT CGATCCCCAA AGCTACTTAG CCGGAGATAT TCCGTTTGCT CAACTCCTGC 149400
AAATACAATG CCGGCTGGAT CGTAGCCGAG CCCAGCGATC ACGCGCTGGA TTTCCTCGTC 149460
TGGGCGCTCA ACCAAGCGTT CATAGCGTAC GACTTGCGTG TGCCGGCGCC CGCGTTGCAT 149520
CGCAAGCGCG GGTAGGCCTA GCACGAGATC GGCGAGAGCC AGTGCGACAG CAGCAGTAGG 149580
CGTGCCGGTT ACGAGCTCGG CGAGACAGGA TACACTGGTC GGCGGGCACC TTTCTGAAAC 149640
GAACGGAACG CCCCACGTCG ATTTCAGTGA TGCCGCAATC GCATATGGAT TACGCAACAA 149700

AAGGATGTGT GGTGCTTCTG GATACAGGGA ATGCAAATAG TCGAGCACCA TCCAGTACCG 149760
CGGAGTCTTG TCGATCAACG TGCGCTTGCC CGCTGCTGCT AAGTATTGAG AGTAAGCGGC 149820
ATCGGCAAAA ACTCGGCTGA CGCTAATGCG ATCTATCCGC CCCAAGAATT CGCACGCCGC 149880
AACCTGGATC AGTGATGCCC CTGCTGGGTG ACGGTGGTCC ACTCTACCAA ATGCCTCGAG 149940
TGCCAACATC AGCCAGGGCT CAGGCGGGGC TGTGATGTCC GGGTGCTGCT GGAGCAAATG 150000
CGCCAACAGC GTGGTCCCCG AGCGGGGGAT GCCGAGGAGA AAACAGATCA GAGGGAGAGG 150060
GCTGACATCG CGGCTCATGA GTTCCGATTC CTTATGCATG CTTGTGAGAA CACATACCCG 150120
CCGCGCGTGA GTTTCTGATC GAGCCACTGG GCGTGCGCCA CCGGTTGGTT ACAGTGGTGT 150180
ACACCGGAGA ATTCTTCCTC AGAACCACGG CACAGACCGA TAGCCATCCT GATGCGCCGG 150240
ACTTCTTCTA ACCTTCGTCT GCTATGCGCG GTTCTCGCAA GCCCGGTCAA ATCGTTTCTG 150300
CCAATGCAAC ACATCCATGC TTTGGATGAT GGCGCGAAGC ACGAAAACGC GTGCGAAGTT 150360
CGACATCTGG GATGGTGCAG ACTGCATTCT GCCCCAACGA GGAAACCAAT GTGCTGACAT 150420
CGGGAACGCA TTCGAGCATA GGTTTTTTGG CCCACAGTGG AATGTTTACG AAACGCACTC 150480
GTCAACCCCA TCAAAATGAT GGCCTCAGCC CTCATGTCCG TCGCAAATCT CCATTCCGCC 150540
GCGAACGGCG CATGGCGCGC GAGATGATGG ACGAAGGAGG GTTCATCGAA ATTCTTGTCG 150600
ATAAGATTTC GAGATGACGA TGGGGCACGC CGTCTTTAAG GCCCCTTTTT GCACCGCGCT 150660
TCATACCCGT CGAGCGGCGA GACGAGGTCA ATCTCACTGC CAGTAGTGCC GCTGGCCTTT 150720
CCCTTGGGGC CGATGCCAGG CAGTGATCAT TAACGACCGG TTTTTGGGTG GAGCGGCTGA 150780
CAAAGCAGCA TGGATTTTCC GTCACCCGCA GAACCACACG TGATGAATAT TAAGGGCGAG 150840
TTGGGTCGCG CTTCTAGCTC GCCCAGCGGT GAGGACGGAT GTGGCATCAT GAATATCTCC 150900
CGCTTCTTCT TCCCCATCCC GCTTCCACGA CACCCCCAAG CAACTGAAGT AGCATTTCTC 150960
CGGGCAGTCG TTCCCAAGCA GCAAGGGCGG CAAATGTGAT GCGCACCAGT GGGACAGCCC 151020
GCAACATGGA AGAGAACAAG CCGACTGGTG CTAAAGCTTC GTGTTTCTGA GCAAAACGTC 151080
ACCCTCCACT CCGCGTGGAA ACTGAGCCAC CAATTCGAAC GAAGGCAGTA GGTCAAGAAT 151140
TGCGCGCAGG GGCAGTTGAC CTTCATACAG CTCCTGGTCA CTATATTCAG TGTAGATGAA 151200
GCGCGTCTTG GTTAGAGTCT CTTTGCCGCC AGCGATGACG TCGGACTCGG CCCCCTGCAC 151260
ATCCATCCAG ATCAGGTCGA TACTCTCCAA GCCAGCCTCA CTGCACCAAT CGTCCAGTCT 151320
CCGAGTCTCA ACTGAGATGG GTCCGTCAAA CCGGACCCAC TGATATTCGG AAAGGTGATT 151380
CTTGGGCTGG CGTATTGAGC CTGACAGATC CCATTCCTTT GCGTCTCCAT CTCCATTACT 151440
GGGGTGGAAA TCGATTCTGC CGTTTCGATC ACTGATCGCG ACTTCAAATA GCCTCATCCT 151500
GTCCAAAGGC CCCATGTTTT CCCTACAGCG AGCAGCGGCT CGGGGGTCTG GCTCAAAGCA 151560
GAAAAGCTGG GCCTTCGGAC GAAGCTGGAG AAAGCGTCGC GCATCGCTTC CATCATTGCA 151620
ACCGATGTCC AGAATCACGG GGTCAGGCTT TTCAAGGAGC GAAACGATCT GTCGATGGAC 151680
CTTCAAGGAA GACGTTGGCT TTCTTATGCC CTCCGCAAAT TCGCGAAGGC GTTGCTGAAG 151740
AGAGATTAGC AGGCCTTCTT TAAGATACCG TCCTACTTCC ATGAAATTAC ACCTCAATAT 151800
TGATTGCAGT TGCGGGTGCT GACCGCTGGT GAGGCATTGA GTGCTCGTGC CGCGCTTCGG 151860
TGAATCCTCA AGGGTATGCT GTCAAATGCC GCGCCATCAC TCATGAGCGT CTCGGCCCCA 151920
CCAGCATGGG TGAAGGAGAG GGACGGAACT ACCGCGATTG TCATAGGCGG GCACCGCTTT 151980
ACAGATTGAT TGGACTTGAT AGTGGTTCCG CTGCAGAGTC GGCGCGCATT GGATGCAGTC 152040
GCACGCTACG CTGCTCCCAA AGCCCACGCA GTTCATTTAG GAGCGCTTCA CGCTGGTTCT 152100
GATTGGGCGC GATGAGGTCC AAGATATCGC CGAGCGGAGC TTCTCCATCA ATCCGCATTA 152160
GCAGTTCGAA CAACTCGGAT GAAATCCGCA CGGCTTCTCG GCTGGAGGGA CCCGTGCGGA 152220
TCTCGCATAC CGTCTCTTGG CGATCTAACG CCGTAAATGC ACGCACTTGA TGGAGGCTTG 152280
AATAAGGCGG CAACGAAAGC GCAAGTCTAC TCCAATCCTC CGGTGACGCA GTCCGCTTCT 152340
TGATGAGATA TGACCCAACC ACGAGTCCAT CCAAGTCGGT GGTCAAAAAT GTGGTCACCG 152400
CATCCGCAAC CGAATCCACT ATCGGCTCAA CATTATTGTT GAAGGACGTG TTGAGCAGAA 152460
TCGGGACACC TGTTCGTTTC CTGAAGGCGT TGATGACCTC CCAATACGCT TGATTGATGT 152520
TGCGCGATAC TGTTTGCAAG CGCGCCGTAC CATCGACATG CGTGACGGCA CCGAGCAGAT 152580
TGCGCTTCGA TTCGCGCACA GGCACTACGA AATTCATAAA GGGAAATTCC TGCCTGCTAT 152640
CTGGCAATTC AAAGAACTCG TTGGCGTCCT CCTCCAACAC GGATGGCGCA AAGGGGCGAT 152700
AGCCTTCGCG CTTCTTGACA ATCGCGTTGA TCCGATCCTT GTTTGTGGCT GGCCTAGGGT 152760
CGGCAAGAAT GCTACGGTTG CCAAGCGCAC GCGGTCCGAA TTCCGATCGT CCCTGCACCC 152820
AGCCGATCAC GGCGCCATCG GCGATCCACT CGGCTGCTCT GCTGGCCACA TCATCGCAGC 152880
GTTCGATTTC AATGTGGCCG CCCCATGCTA TTAATTCCTG CTCCACGGCG CGGTCGCTCC 152940
CGAGATCAGG ACCCCAATAG ACCTCCTGCA AACGCTCCCG AGGTGCGGAC TGCCCTAGTT 153000
CATTAGACAT CATCAATGCA GCGCCTAATG CGCAGCCAGC GTCGTGTGCC GCGGGTTGCA 153060
CGAAGATGTC TTGGAAGATT CCCGAGCGCA ACAGCTTGCC GTTCAATGTG CAGTTGTGAG 153120
CTACTCCTCC GGCCAAGCTC AAGCGCTTTA TGCCGGTGAT CTCGCTGTGA TGCCGTAGAA 153180
CGTGAAACAC AATCCGTTCG AGTGCTTCTT GCAACGAAGC ACTTAAATCT TTATGCTGCT 153240
GAGTGAACGG CATTCCTTTT CGGCGTACCT CAATGCTGCG AAGCAGCGTG GGACCAATCC 153300
GGTCCAGATA GATCCGGTAG CCACCGTTGT CTAATAGCTC GTAGAACTGT TCGAAGAGGT 153360
CGCGATGGGG AGCGGGGTCG CCGTACGGTG CAAGCCCCAT GACCTTGTAT TCATCAAACA 153420
TGCCGTAACC GAGATATTTT ATGGTCTCAA GGTACAAAAG CCCTAAAGAA TCGCTCTCCG 153480
GAAATGTCGC AAGCGGTTTG ACTTCCGTGC CGGATCCTAC CGCCAAAAGA CCCGAGGCGA 153540
AGTCTCCACC GCCATCAATT GTCAAGATGA GACTTTGTTC GAAACCAGAC ATAGAAAACG 153600
CGCTCCAGGC GTGCGACAGG TGATGGCTTA CGAATGAGAT TCGCGACGGA TCGACCTCAG 153660
TACCAAATTC CTGCGCGAGC AACCCACGCA ACAACAGCTT GGCATCCAAC GGTATTGACA 153720
TGTGCGACTG AGAAACGAGC AGGCGCTCGA GCATAGCATT GCAAAACGCC TCGGTTGCAT 153780
AATACGCGAT GCAGTCGATG TCGCTGAGTT GAACCCCGGC GTATTCAAGG CAGTATTGAA 153840

TCGACCGGCG CGGCAGCTTG TTGGAATGTT TGATTCTGTT AAGTCGCTCC TCTTCGACAG 153900
CAGCTATCAC TTGGCCGTCT CGGACGAGAA CCGCTGCACC GTCGTGCATA AATGTATTCG 153960
GCAGATCGAG CGAGTTTTCG TGAATCTTGC TTAGACCGCC ACTAAGTCCT AGACACAGCA 154020
TGTTGTCTCC AATCTCTCGT CACTTGTAAC AGGTAGGCCA TGTGCTCCGA CTGAATTTAG 154080
TCGTTTGCTA AGCTCAAGCA GTGCTCTTTC TGCCGTAGGT CAGGACAGCA AACGCCGCTG 154140
AAGCAGTCCC ACAGATAAGA AGAATGGCAT CACTGCAAAT ATGCAAAGCG CGCTGATATG 154200
CAGAGCGACG CCTCCGCCTG GGCGATCTAG CATCGCTGGA CGAATGAGCT CGATTGAATG 154260
TGCCAGCGGC AGCAGATGCG ATAGCTTCTG AAAAATGTCG GGCAGTTGGC TGACTGGGAA 154320
GACCGCTCCT GACAAAAACA GCATGGGTGT GAGGAAGAGC GTCTGGTAAA ATATGAAATA 154380
ATCGTAACCG GGCGCAAGCG CTGTGACGAT CATCGCCAGG CTTGCAAAGG CGAACCCCGT 154440
GAGTGCGATC ACTGGCAAGG CAAAGAGAAC GGATGGCCAC GATGCAAAGC CCATTGTGGC 154500
TGCAACGAGC ATCATTGCTG TCCCGGCCAA TAACGCCTTG CTGGCTGCCC ACGCCAGTTC 154560
ACCGAGGACG ATGTCCCCGA GCGTGAGTTG GGTGCACAGG GCTGATTCCC AAGTTCGTTT 154620
TGCCTGCATG CGAGCGAAGG TCGCGTGAAT AGTCTCGAAC GTCGCGGAGA TCATTGCGCT 154680
TGTTGCAACC ATGCCAGCCG CCAAGAACGC GACATATGGA ATGCCGTCGA CGCGACCCAC 154740
CATTGCACCC AGGCCGAAGC CGAGACCGAA CAGCGAAGTC ACGGGCTCGG CGAGGTTACC 154800
AAGAATTGAA GCGATCGCGG CTTTCTTCCA TGCCATGTAA TTACGGCGCC ACACCGCAAT 154860
CCAATTCCAA GGATTGGCGG GTAGGACCGC CGCGTAACGT TTCCACATCG CTCAGTCCTT 154920
CATTTCGCGC CCGGTCAACC GCAAGAACAC ATCTTCGAGA TTCGGTGGAC GCTGCAATAG 154980
GCGTAGACCG GTTCGCCCGC GCAATTGCAA GCGCACCTGT TCTGGATCGG AAGAATAGCA 155040
AAACAGCGTC TCACCGCTCA CTTCGACACG CTGAGCATAT GGTCCGACAA CTGAAGTCAG 155100
CTCCCGCGGA TTGCCGCCGT AGATCTCAAT CACGTCGCAC CCGATCTGCT CGTCGATTAG 155160
CGCGTGAGGG CGGCCTTCCA CGATCTTCCG TCCGTGCTCG ATAACACACA AGCGGTCACA 155220
TAATCGGTCC GCCTCTTCCA TGAAGTGGGT AGTCAGTAGA ATCGTCTTGC CAAGCGCCAA 155280
CAAGGACCGC AGCCGTTCCC ATATTAGGTG GCGAGCATGC GGATCGAGGC CGGTAGTCGG 155340
CTCATCCAGT ATCAGGAGCT GCGGGTCGTT GATCAGCGCA CAAGCCAGCG TCAGCCGCCT 155400
CTGCATACCG CCGGATAATT GCGCCACGGG TACATCCGCC TTGCTCTCGA GCCGAGCGAA 155460
ATCGAGCAGT GGTGGAATGG CTTCCTCGAG CTCGCGAGTA TGCAAGCCGA AGTAACGTCC 155520
GAACACCAGT AGGTTCTCGC GCGCCGTGAA CTCGCGATCA AGTGTGTCGA ATTGCGGAAC 155580
CACCCCTATG CGGCTGCGCG CCAAACGCGC GCGGGCTGGC ACCGGCTCTC CGAGCACTGT 155640
GATCGTGCCT TCATCAGGCG GTGCCAGGCC GAGAACCAGA CGCGATACCG TGCTTTTGCC 155700
CGCACCGTTC GGTCCCAGCA AGCCGAAACA TTCTCCTGAG GTGATGGTGA ATGACAGTTG 155760
GTCGACAACG ACTTTGTCGC CATAAGACTT ACTGACGTTG GTAAGCTCGA TTGCCACATC 155820
TGATTTGGAG CGAGATAGCG AATTATCCGC ATTGCTATGC CCGTGGCACT TCTGCTTGAG 155880
TGCATTGCTC TCAGGCCGCC GCGACGGTGA GCTTGAGACA TTTGCTCGAG TCAATAGCTG 155940
CAATTTCTCG TCCTATCTTA AATTCGCTTG CCTGTTTGGC ATAGCCGGCT ACCGTCACCG 156000
CAGTGACTGA CTGATGTCAG CAGCCTCGCA AGCTGCGCAA GACTGAGCCA GCGACGCGGG 156060
TGCCGCAGTT TCAGACGGCT CCGCAGCGCT TGTTTTAGCA ATGGGGTTCA GGAGAATCCT 156120
TTACGCCGTT GGGTGCGGCA GCCGGGGAAC CACGCGACAG CCAGTCGCTG TTGCTCAACG 156180
TGCACAACGC ATATGCTTTG AGGGGGAGCA GGAGAGCGAC GTTGAGGAGG GTGTGCAGCG 156240
AAAACCCAAG GAATCGCAGC TCTCGCGCTC GAAACGCCGC CACGCCGCAG CGGACCATTG 156300
TCATAGATGC AATCATCAGG ATCGTCGACC AAGGCACTGT GGCCGTCAGA GCGAGCTGTG 156360
CTAGCCCCGT CAGGACCGAG AGGGCTAGGA GCAGCGGACC AAGATTCTGT CCGATCACGT 156420
CCAGCGTAAG ATAGCGATCA AGGCCCGGCA GTAGGCGGAG CGCGAGCAAT GTGTCGCGAA 156480
ACGTGCTGCG TGCCCAGCGC AGTTGTTGGC GCAGATAGGC GCCCATCGAG TTTGGAACGA 156540
CCGTCGCCGC GATGGCTTCC GGAACGTACT CGGTTCGAAA GCCTGCATTC AGCATGAGGA 156600
TTGTGAGGTG GCGGTCTTCC CCGAAGTCGC TTGGCCTGCC TCGAAACAGT TGCGTCTCGT 156660
ATTTATCGAG CAGCAATAGG AGTGCGGACC GCCGGTACAT GGCACACGGG CCGCAGCAAC 156720
ACATAACGGC TCCAAAGCGA GCCTGTGCTG CGCGTTCCTC GTTGCAGGCG AGCCAGTACT 156780
CCATGTCGAT CAACCGCGTC AGCCATGTGT CGCTGCGGTT GCTGGCCGTC AACTGACCCA 156840
TCGCCGCGCC GACCGCGGGA CTGTACATCT TCAGGGCAAG TTTCGTGACT ACGTCCGGCG 156900
CAATGGTCGT GTCCGAGTCA ACGTTGAGCA CCAAATCTCC CGATGATTCC CGTATCGCGA 156960
CAATCTGCGC CTTGCGCTTT CCGACGTTCT TTGGCATCAG GATAAAGCGG AACCTCGGGT 157020
CGCACGCATA ATGATCGTGT ACAGGTATGA TGGCGTTGCG ATTGCCAGAA CCGTCGTCAA 157080
CCACGTAGAC CCGCAACTCT CCAGCGTAGT CCTGCTTTGC AATGGAAGCT AGGCACGCCG 157140
AGAGCGCGCG CGGATCCTCA TTGTAGCAGG GTACGATAAC ATCCACGCTC GGCGGTGCAC 157200
CGGAGCCGGT CACGGGCGTT GACGCAGCTG TGGTGTTTGT TGGCAAAGCG TATATAGCTT 157260
GCATGCCTTT GTAAGCCGTC GAGAGTGCTG CATACAAGGA GATGGCTACG GCGCCGGTCG 157320
TGCCAAGCAG ATCCATGAAA AACCGTCCGT TTAATGATTC TGAGGAAGCG AGCGGATTAT 157380
AAATCCGCGG TCGTGCAAGG CAGGGATGAG ACGAGACAAC GCCGCCACCG TCTGGTCGCG 157440
AAAACTGGCG TCAGTGTTCG CCAACTCGTC GGGAGGGCAC CCATCGTGCA GGAGAATGAT 157500
TGAACCGGGC CGGACCGAGG CGAGTACCGC ATCGACGATT GCGTCGATAC CGGGGCGGGA 157560
CCAGTCCCGC GGGTCCACAG ACCAGTGCAC AGCCGCCAGT CCAGCGTTTG CCGATGTTGT 157620
CAGCACGTCA TCGCTCCAAA TTCCGTAAGG CGCACGTATG TGCCGCACCG AAGCCTCGGG 157680
GCAGGCCATC CTGATGGCCC TGTTCGCCTC GAGTACTTGG CGTTCCACGT CCCGACGTCC 157740
GCAGGCAGAC AGGTCCGGAT GGGTCATTGT ATGGTTGGCG ACCTCGTGCC CTTGCGCAAC 157800
CATGCGTCGG ATGAGTTGCG GCCGGTCCTT AGCATAAGCC CCGATGACGA AGAAAGTCGC 157860
CGGGACACGG TGTTCGGCCA GCACATCGAG GACCTCAGGG GTAAAAACCG GGTTAGGACC 157920
GTCGTCAAAG GTCAGGTAGA CGCTCGGGGC CCCAGTGCCG CTTCTGGGCA CTGTCCGGAG 157980

ATAGTCAAGC TGTTTCATAG TTCCGGACCG TTTCGTTCGA TCAGTGTGCC GGATGGCCAT 158040
TCGCTCATTG GACGTCCAAT CGGAAAGACC ACAACGAGTA CGTCTTCAGT GCGCGTTGGC 158100
GGCAGGTTGA GATAGACATC TGAAAGGGTG GACCGCACGC GAACGCCAGC CACAATTGTC 158160
CCGAGACCGT TTCGGCAGAG TCTGCCCACA AGCTTGTACA GCGCGTGGCG AACGGCGCCA 158220
AATGCGAACG GAACGCCAAG CTGCTGCAGC ACCGGATACA TGACGCGGAT GGAGTGGTTG 158280
AGCCCGAGTC CTTCGAGGTC CGGGCGAACC CCCCACAAAC CCAGTTCCGC CACCAACAGA 158340
TCGGCATCGC CCACCCTGAT AAAGCGGCGC AACAGCCCCA TGTGAGCAGC CACTCCGTGA 158400
GCGTCGTAGC CGATTACACG GAGCTCCGGC CTTGCTCCGG CCCAACTGTG CCCACCTTCA 158460
AATGGTCTTG CGTTGAACGT CCCGGTCGGT CCATAGGTCT TCCGGAAGAA CTCAGCGAGT 158520
TCAGTGTGGT CGGAGAGCTC CAGCTCATTT TCCCAGCACA GTTTCCACCG CATCTGAGGA 158580
CGCATATCCA AAGAACTCCA CATCTTGCTG CTTCTTGAAA CGTACATCAA ACGTCCGAAA 158640
TGGCCGGGGT CGATGGACGC CCGACACTGT CGTCGAACCC GCATTGCGGA CCAAGTTGGT 158700
TGTTATTACC TGCACAGTGA GTGGGGGACA TTTCCCGACC TTCCCGTATC ACTCGCAAAT 158760
CTCTACCAGC GACACACTTG ATTTCTTCAA ACGTGAGCAT CTTCTAATAG TGATTGGATG 158820
GATCCGTAAA ATTGATTGTT TGGATTATAG CCATTCATCC TGTGGATACA CCCGGCCCAA 158880
GTCTCCCTGC TAGCAACGGT TGATGAACGA ACCCGACACC GCGGCGGGAG CATCAGCTTC 158940
GGCCCGCCAA TCATGGGCGT GCGGCCACGC TCTCGAAAAC AAGGAAGTTG GATCAAAGAA 159000
CAGGCTGAGA CTTGGCCGCC AACGGCAATG AAGCTAATAC CGCTACTGCT CCCGCGCCGT 159060
TCGGCAGATA CGTTTCGGCC ACAGCCACGG CTTGTTCGGC TCGGGCAGCT ATATCAGGCA 159120
GTGCCGTCTC GTCGATCAGG CGCTGATTTT TCCTGTAGGA TGGAACGTGG GCGTAACCAA 159180
ATACCGCAAG CCGGTCGGGG CGCATTGCGA CCGCTGCCGT CGCGCTCTGA ACGCAGGACT 159240
GCACGGTCTG GTGCGGGAGG CCGTACATGA GGTCGAAGTT GATGCGACTT ATCCCAATCC 159300
GGCGCAGGTT TTCAATAGCC GCGGTCGTCT GCGCCTTTCT CTGGACCCGG TTGGTTGCTT 159360
TTTGAACAAT GGGATCAAAG CTTTGCACGC CGAGGCTCAC GTGGCTTACC TCGGCCGCTC 159420
CTAAGGCTTC GGCCATCTCG GCCGTGAAGG TGCGCGGCTC GATCTCGACA GCGATGGTAG 159480
CCGTTTTCCT GAAGGCGAAA CTGCGACGCA GAAGATCCAT CAGAGCCAGA AAATCTTCTG 159540
GCTTGATGAT GGTTGGTGTT CCACCGCCGA AGTGCACGTC GCTCACGGGC AGCGCCTGCG 159600
GCGCTTGGGC TGCTACCAAA CTTATCTCCT CACTAGCGCC GTCAGATAGT CGAGGATCGA 159660
TGCGTCTCGA CGAGTTATGC TGTTGGGAAA TCCGCAATAC CAGCACATCG ACCGACAGAT 159720
CGGAATGTGG AGATACAACG ACACCGGATC TGACGCCGGC AAGTTCCTCA GCCACCCCTT 159780
ACTAGCTTCA GCGCCGACGG CTGCGGAGAA ATCCGGTATA GTCGATCGTG TACCATGGTA 159840
GGTGCGCCTC GCAGTACTTT TGCAAGAGTG GAGGTCAGCG GCGCGACACT TTTGATCAAA 159900
CGCGTTGCCG CGCCTGTCTT TTCGCCGTCA GCCATTATGG CCTCCCGCTC AAGCCACTCA 159960
GCAGGCCCCG CTGCCGTTCG ACGAGGATCT CAAAATCCAA CCAGTTGCGT CGAGAACATT 160020
GCCGGTCAGA TTAGCCGGTA CTTTTCAATT CCTTAGTCGA GACCGAAACA ACAAGGGACA 160080
TAAAAAATGA TCTCTGTCGC GGATTCGACA AACGGGACAG AGTTGTACGC AATCGATTTA 160140
CATTTTGCTT GCCAAAGGCC GACGGCAAAT TCCTGCAGAG GTTGAGCTAC CTGCGTGAGC 160200
TTGCTGCCGG GGACACGCCT TGGCGAAGGT GGAGAATGGT GCTGTTCAGA AGTAGCTCAG 160260
TTGAATATCC AACAAACGCC AGTAGCCTTG CTGTTTCGCG GAGGAGGAGG CTAGGCGGAG 160320
CCGTTATCCC GGCGCCCGCC GGGTTGCACC CCGCCGTTGC ATTTCTGCCA CTGAGGAGCA 160380
GACAGTCGGG TTGAGGTCCT TCCGTGGTCA AGTTGTGGAC CAGACAGCAG AACGCTGAGA 160440
CGGTGAGCAG ATGAATGCGG CGTCGGCAGC CCACAAGAGG AGACGCATCC GAGCGTTGAG 160500
AGGGGATGGA TGGTTGCCGG CCAAGCGGTA ACACGAAATA GCAAGCGACG GTGGGGAAGA 160560
ATTCCGACAT GGTCCATTTA TTGCAATTGA TAGCATTGTG AAAGCAGCCT TGGCCATTCT 160620
GCAACTTGAA CTAATCGTCG GATCCCCGCT TTCCGCGACT ACAAAATTGC CGCACCTTCA 160680
CGCGCTGGCA TGCTCGGATT CTCATAGAGG CTAAAACAGC AATGGACACG GAAGCAATCG 160740
GGCTCGCAAT AGCTTTTTTC GTCATTGCAC TGGCGTATGC AGCAGTCGGA CAAGCTGGCG 160800
CATCAGGCTA CATTGCCGCC ATGGCGCTTT CTGGCTTTTC GCCGTTGGCC ATTAAGCCGA 160860
CAGCTCTTGC TTTGAACCTG ATGGTCTCAG CGATCGGCAC GGCCCAATTC CTGAAGGTGG 160920
GGCAGGTGTC CTGGCGTAAC GTGTATCCTT TCGCAATTCT GGGCTTTCCG GCTTCGGCTC 160980
TCGGTGGCTC AGTGCACCTT CCCGAGAGAG TCTATCACCC GGTTCTCGGG CTGATCCTGG 161040
TAGTTTCCGC CATTCAAATG GCGCGATCCG CGCTTCGCAA GAGCGCCTTG GTCATAACCA 161100
TTCCGAAAAC GCCGCCACTA CACGCTGCAT TGATCACGGG AGCTGTTATC GGTTTCGTCT 161160
CGGGCACGAC CGGGAGCGGA GGCGGCGTAT TCCTCGCACC AGTGATCCTT TTCAAGAATT 161220
GGGGAACAGC ACATCAGACT GCTGCAACCA CGGCCGTTTA TAATTTAATG AACTCAACCG 161280
CTGCCTTGAT TGGAGCGTGC GCTTCCTGGA ATGCACTTCC AAACTTTCTC CCGTGGTGGT 161340
TGATTGCGGT AGCGGCAGGT GGATCGATAG GCGCTTTAAT CGGAAGCCGA TATCTTTCCG 161400
CTTCCTGGCT TCGCGTCATT CTTTCCGTGC TTCTGATGGT ATCAGGCCTC AAGCTGCTTT 161460
GGTGATGGAG TGCCCATTTG CTTGCACGGA AAACCTTTGC CCAGCATCAC GCAGGAGCAC 161520
GCGCGAAGGC GCACCATTCT CATCCGCGGC TAAGACCGAG AGGGCCAATT GTCGCTGAAT 161580
GTAATCTCCG ATGACCGAAC TCCTCACCAG CACGGTACCT GGAACGTGGC TGGCGCGCTT 161640
GCGCCGAAGG TCGAGACGTC CAATTCAGCA CATCATGACG CCGACAGCCA CTCTGTCGTG 161700
TACGATGCCC GATATGCCAA GCGTCCCGAA AACCACCTTG CCGCCATCGT CAACCTCGTC 161760
GCCACGAATC TGAATGGCGC GATCTCTAAA GAACATCGCA AATTCGAGAG CGGTCGCCGC 161820
TCATAAGATA AGGCTCGGCG TCACGGGTCA TCGGACAGCT CTCTGCCAAT ACGGCAGGCT 161880
GTCGGCGCCA TATCCAAAGT GCGGATAATC GTGATGCAAA ATATTTATTT TACTGGCCTT 161940
AGCAATGCTG CAAAAACGAC CACGCTTGAT TTGCATCGAT ATTAGAACAG TTCCAGAGTG 162000
CTGGCGTTGA AACCGTTTTG CGGCTCAAAG AAAGGATTAC CCGTGGAAAG CATCATCGGT 162060
TCGGCATGTA CAAGAATCCG GTTGTCCATA AGGGTGGACA GGTTTGGGCA GATGTAGCCG 162120

TCCTGTACGT TACACTTGAC CTAATCATTG CGTGAATGAG TTTTGCCGGC AGTTGGGGCA 162180
ACCATGGCCC CGTTTACGCG GCGCCCATCT AGAAGGCTGC GGGGAGCAGT CGCTCTTTCT 162240
GCCCGGCAGG GCTCGGCATG TGACTATTTC GGAGTTTCTA CATTGCCTTC AAATATGAGC 162300
TTAACGGCGG ATCTCATTGC AGATTTGCTC AGCGCGGCAA ACGAGGTCGA CAAACTCAAC 162360
CCCTATGAAA TAAGCGGCCT CCTAGACCGC TCGATTGACA CCATCCGCGA TGTGCGCGAA 162420
CAGACTGGTG TCGCAAGTTC ATATATGCAG GGCGTTGTGG TTTGTCTTCG AATATCGTCA 162480
GAGCGCGTTG GCCACCTCTC TGAGGAGGAT GTCAGGCGGT CCCTCGTCAC TGCCGCGGAA 162540
GTCCTGAGTG CATTGGTGTC AAACCGTGTC GATTGAGCTG TTCGCGCCGC CGGAGTCCGG 162600
ACCACTCCAG CTATCTTCGA TCCCGCAAAC CGATGCGGCG AATTCGGCTG CAATCTCGAC 162660
AGGTATTGCC GAAGATTGCT TCAGTCGACG CTGCTTATTG GAACCGGCGT ATCCGATTCG 162720
CGACGATGTC GAATACACGA CATAGGCGTG TCGAGAGGCA GCTTCTTGTT TGGAAACGAT 162780
TTCTCGTTGG CATGATACAT GCAGGCTAAC CCGCAAATGC CCGAGTTGAG GACTTGTGAG 162840
GAGGATGCAG GACTAACCCG CAAGAGTGCA GTTCGGGGTG CGTCAGCCTT CCCCGGGCAA 162900
CGAGGATCCG AACAGCAAGG GGGCGGCAAA TAGGAAAGCA ACGGCCGCGA GGGCGTTTAA 162960
CAGTGGGTGC TGTGCGAGGA TCAGTCCCTT GCAATGGCTT GCCGCCGATA ACGTTGTAGA 163020
AAAATAGACA AGGAGTGCTC AATGGTCTTC ACTGGAAAAT GTGATCTGCG GAGTCCGCTG 163080
CTCGGACGAC GCCTGCGCTT AGGATTCATC GGCGGCGGAA AGGGGGGGCT TGTTGGACGA 163140
TGGCATTTCG CTGGGGCCCG GCTGTCGAAC CACTGGGAAA TTGTTGCGGG AGCTCTTTCT 163200
TCGGACCCGG AAAACGCGCA CGCTTCGGCT GCAGAGTGGA TGATAGCGCC GGACCGGTCG 163260
TACAGCGACT GGAACGCTAT GGCAACAGCA GAAGCCGCAA GGGACGATGG TATAGAGGCA 163320
GTCTCGATCG TGACGCCAAA CTGGACACAC CATCGGATCG CGACGGCATT TCTGAAGGCC 163380
GGCATTGATG TAATACTAGA CAAGCCTATG ACCACAACAG TTTCCGACGC CCGAGAACTC 163440
GTGGAGTTAC AAAGAGCGAC GGATCGCCTG GTCATCATGA CTTACCCGTA TGCACATCAC 163500
GCGATGGTTC GCCAAGCTAA GCACATGATC CGAAATGGGG CCGTCGGCAC GGTCCGGCAG 163560
GCCCATGTGG AGTATGTTCA GGAGTGGGCG ACAGCGCCCT TCTCGTCCGA CGCGAAGGGG 163620
GCAATGTGGC GGCAAGATCC CGAGAAGGTG GGCCGCGCGT CGGCCACCGG AGATATCGGC 163680
ACGCACGCTT ATCATCTCCT TCACACACTG ACCGGACAGG ACATTGCCAG GCTCAGGGCT 163740
GAGTTTCACA CCTGTGGCGC TGTGAAGGCG ATGGAAGACA CCGCCTACGT CACTTTCCGT 163800
CTCGAGAATG GTGCACCTGG GATCCTTTGG GTAACGCAAG CTGCTCCTGG CCAATATTGC 163860
GGTCTTCGAA TTCGCGTGTG GGGCGACAGA GGCGGGCTTG AGTGGGATCA GGAAAAACCT 163920
GAAGCGCTGC GCTATGTTCC TTTAGGGGAG CAGGAGCAGA TTTTTGTGCG AGGCCACGGT 163980
AGTGGAATAG TACCCGAAGC AGAACGCCTG ATACATCTAC CGCGGGGACA TGGCGAAGCT 164040
TTAGTGGATG CATGGGCAAA TCTCTATGCC GAGGCTGGCG TGGCGGTAGC CGCACGCCGC 164100
GCAGGGTATG CTCTCCCGAA AGATAGCGTC GAGCTTTCCG GGGTTGAGGA CGGGCTCAAA 164160
GGTATGTTGT TCATCGATGC TTGTGCCGAC AGCCACGAAT CTGGCGGATC TTGGGTTTCG 164220
ATAAACGGTG AATCCATTCC CAACTTTTGC TGAGCTGTGG AGTGCGGCCC TTGGGGTGGA 164280
CAGACCGGAT GACGAATTGA CCGGCCTACC GGGTTTTCGG AAAGAAGGCC CATGGCAAAA 164340
CACCGAAGTC ACAGCGTCGA GTTCAAACGT CAGGTCGCGC AGGAATTCCT GGCTGAGCGG 164400
CCAAAGTTCG GGCGCCCGAT GTTTGGCTGA AGCGTCTTTG AGCACTGCTA GTCTTTCGCC 164460
CGATCACGAT GCTTTCGGCT CCGATTGTCC GGAAGGCGCG CCAGATAAGG GGTATCCGTC 164520
CGAGCGCGAC CGCCTTTGCA TATTGAGTAA AACTGTTCAG ACCCTGTCCT TATTCCCATG 164580
CATAAGGACA GTTGCCAACA TGGAGATTTT TGAAGGAGAC AGCGGGTCTC GGTGAGCCGG 164640
TCTTGAGGTG ATCAACACCG GACGGCGGCG TCGATTTACG GAAGATGAGA AGCTGCGGAT 164700
TGTCGCAGAA AGCTTTGCCG GGAGAGGCCG TGCGTCGGCC ACGGCCCGTC AGTACGGCAT 164760
CAGTCGCTCC CTGTTGAACC GTTGGCGCAA ATCGGTTCGC CAGGGTTTGC ACGGCCAGAA 164820
ACAAACCGAT GGTTTTGTGC CGGCGTTCGT CATGCCGGAA ACTTTTGTGC CGGTGAAGCA 164880
GGTCACTCCA CCTGCTGCGA TGGAGCAGCC GGTGGCGTCT CCTTCCGGCC GCATGGAGAT 164940
TGTTGCGGCG AACGGCCGTC GTGTGGTCGT GGACGGCAGC GTCGACGTTG AGGCGCTGCT 165000
GCGGATCATG CGGGGGCTGG AGACGTTGCG GTGATCATGC TTCCTTCCGG TCAAAATGTG 165060
CGGGTGTGGA TTGCAACGGG CCATACGGAC ATGCGGTGTG GGTTTCCATC GCTTGCGTTG 165120
CGGGTGCAGG AGGTGCTGAA ACTGAACCCT TTGGACGGCA ATCTTTTTGT GTTTCGCGGT 165180
CGCAGCGGAT CGCTGCTAAA AGTGATCTGG AGTGACGGCC AGGGGAGCTG CCTTTTTACA 165240
AAAAGATTGG ACCGTGGCCG GTTCGTCTGG CCTTCTGCCG AAGGCGGAGC GATAGCGATA 165300
TCACCCGCGC AGCTCAGTTA TCTTCTGTCC GGAATCGACT GGAGGCATCC TCAGGAAACC 165360
TGGCGGCCGA CGAAGGTCGG CTAGCATTAT TCTATTGAAA ATACAGGGGA AATCTGATCG 165420
AATGGCTTCA TGATCTCAAA GCCTGTCGAT CTTCCTGTGG ATGTTGTTGG CGCTTACCTG 165480
GCGCTGCGTG GCGAGCATGA AGCCTTGCAG GCTAAACACG CTATCGCAGT AGCGGAAGCC 165540
GCCAATGCGC AGGCGATGCT CTCTGACAAC GAGGCGCTGA TCGTTGCTCT GGAATTGAAG 165600
ATCGAGAAGC TCAGGCGCGA GTTGCGGGGC CAGCGCTCTG AGCGCACGGC GCGCCTGCTC 165660
GACCAGTTGG AACTGCAGCT CGAGGAACTC GTGGCGGCGG CGACGGAGGA TGAGGTCGCG 165720
GCACAAGCAG CAAGCGCCAG AACCTCGAGC GTACGTTCGT TCACGCGCAA ACGGCCGGTG 165780
CGCAAACCAT GGCCGGACGA TATCGAGCGC GAACGTGTTG TCATCGAGCC GCCGACGACT 165840
TGCACCTGCT GTGGTGGTTC GCGCCTGTCG AAGCTGGGCG AGGACGTCAC CGAGACGCTG 165900
GAAGAGATCC CACGCCGGTT CAAAGTGATC GAGACGGTGC GGGAAAAGTT CACCTGCCGC 165960
GACTGTGAGG CGATCAGTCA GACGCCCGCA CCCTTCCATG CCACGCCGCG CGGCTTTATC 166020
GGGCCGAACC TGCTGGCGAC GATCCTGTTC GACAAGTTCG GCATGCACAG CCCGCTCAAC 166080
CGGCAGAGTG CCCGGTTCAA ATGCGAGGGG ATCGATCTTT CGACCTCGAC GCTGGCCGAC 166140
CAGGTCGGGT ACGCAACAGC CGCTCTCATG CCTGTCTTCG ATCTGATCGA GGCGCATGTC 166200
TTCGCGGCCG AGCGTCTTCA CGGTGATGAC ACCACCATTC CCATTCAGGC CAGGGACAAA 166260

TGCACGACCG GACGCATATG GACTTACGTA TGCGATGACC GGCCATTCGG GGGAACGGCG 166320
CCGCCAGCCG CAATCTATTA TGCGTCGAGT GACCGGCGCG GCGAACATCC GCAGAAACAC 166380
CTGGCCGGAT ACGGCGGCAT TCTGCAGAGC GATTGCTACA ATGGCTTCGA GCCGATCGCT 166440
GTTGCCGCAA CGAAAGCGGT CCCGATCACA TTCGCCTTTT GTCACGCGCA TGCGGCGGCG 166500
AAATTCTTTG AGCTGGCCGA TATCCAGAAG AATGCGCGGG ATCGCAAACG GAGGGGCAAG 166560
CCGATCTCGC CGATCGCATT GGAAGCCGTC AAGCGCTACG ACGAATTGTT CGAGATCGAG 166620
CGCCAGATCA ACGGATTGAG CGCCGAAGAA CGACTGGCTG TGCGGCAGGA GAAGAGCAAG 166680
CCACTGTTCG ATGACATGCA CGAGTGGTTG ACGAAGGAAC GCGCCATGCT CAGCAGATCG 166740
TCCGAGGTCA TCGAGCCGAT CGATTACATG CTCAAGCGCT GGGAGGGCTT TGCTCTCTTC 166800
CTCAAAGACG GGAGAGTTTG TTTAACGAAC AACGCAGCCG AGCGGGCGCT GAGAAGTGTC 166860
GCATTGGGAA GACGGAACTG GACCTTCGCC GGTTCTCAGC GCGGGGCCGA TCGTGCCGCT 166920
GTCATGCTGA CCGTCATCAC CACCTGCCGC CTCAACGATA TCGACCCGAA GGCATGGCTT 166980
GCAGACGTGC TGGCTCGCAT CGCCGACCAT CCTGTCACGC GCCTGTACGA ACTGCTGCCC 167040
TGGGAGTGGA AACGTGCATC GGCGGCAACC GTCATGCTGG CGGCCTGACG ATGGTGGCTT 167100
CCGTCGTCCG CCTGAAGGTC ACCCTCGATC ATGTCGAGCC GATAGTGATG CGGCGCGTTG 167160
TCGTGCCGTT CACCATCAGG CTCAGTCGGC TGCACGAGGT GTTGCAGGCG GCGATGGGCT 167220
GGACCAACAG CCACCTTTAC GAATTCCGCA TGCGTGACGT CGGCTTTGGT TTGCCTGACG 167280
AGGAATGGGG GGATGGTCCG ATCGATGCCC GCAGGGTCTC GCTGCTGTCG GCAGTCCAGG 167340
ACACCGGTGC GAAATCGTTC AAATACCTTT ACGACTTTGG CGACGGCTGG GAACACAGCA 167400
TCAAGATCGA GCGCACCTTT CCTGCGGTCG GCACGGAAGG ACCGATGCTT CTCGAAGCAA 167460
CGGGGCACTG CCCGCCCGAA GACGTTGGTG GTCCATGGGG TTATCAGGAG TTCTGCGAGG 167520
CGCTTGCAGA CCCGGCGCAC GAGCGACATG CCGAAACCCT CGAATGGTGC GGCAGCAGCG 167580
ATTATGATTC CGCCGCCGCC AACTTCTCGC AGCTCAACAA AGCCGTCGAT GACCTCGCTG 167640
CAAAATGGGC CCGAAAAGCG CGCCGCAAAA CCTGAACGCC GGGCTGTGGC CGAAGGCCGA 167700
AAGTTGATTT TACCGTGTAA ACAACCGGCG GAACAGTTCT TCCGAGCGCC GTAGACCTTC 167760
GTCCGTCAGC ACCAGCGACT TCGACTTGTT CACCGGATCA CAGATCAGTC CCCTCTCATG 167820
CAACCGGTCC GTCGTCGCCC AGTCAAATCC CTTCCAGGCG CAACGCTCGT TGTGGAGCGT 167880
CAGCCATAAC AGTGCCAGCA CCGCGTCATC GATTTTGTCC TCGTCGATCT CCATTCTGCT 167940
ATCCTACCAC GCGCGAGACC CCCAAGTCCC GCGGCCCACC TCGGATGGAT ACGATAAGGG 168000
CACTCGTCTC CATAGACCTC TTCGCTGTTT CGCCGGGGAA ACGGGATCGG GCGAGCGGAT 168060
CCTGCTCCTG CGCCGGTGAT TGGCAACCTC GGCTCTGTCT AGAGCGGGAT GATTTTAGAT 168120
CCGTCATTGG AGTGCGCCCC TTGGGGTGGA CAATGGTCAG GCTGCTGATT TGCCAGTCTG 168180
CCGGATGTGT TGATCCTCGA ACTGTTGGTG AATGCCTGCG CACGGCAGCG CTCGATCAGC 168240
CATGCCCGAT GCTCACCGGC GATCGCCCGC GGCTTGTGCC CGCCCATCTG GCCGGCGGCG 168300
GCGCTGCCCG TCTCACGCCA GCGCCGTTGA TCGAAAGTCA TTGTCGATGC CGCCTCGGTT 168360
GCGGTGGCCC GCCGCCACCG ACGCGCCAAG ACCGATCGGC TCGATGGCGA GGTACTGGTC 168420
CGCACCCTGA TGGCCTGGAA GCGGGGAGAA CCGCGCGTTT GTTCGATGGT CAGGGTGCCG 168480
ACGCCGGAGG ACGAGGATCG TCGCCGGATT GGCCGGGAGC GCAAGGCGCG GGTCGCCGAA 168540
CGGGTTGTCC ATGTCAACCG CATCAAGGGC CTCCTCGTCA GCCAGGGCAT GCGGGACTAC 168600
GAGCCGTTGC GCCGTGATCG GCGTGATCGC CGGGACGAAC TTCGCACCGG CGATGGCCGT 168660
GCTCCCTGCA TGAAGGCCCA GATCGGCCGG GAGCTCGACC GTCTCGAATT GCTGCTCGAA 168720
CCGATCAAGG TGGTGGAGGC CGAGCGGGAC GCGCTGTTGG CCGTACCGGC GGCGAATACC 168780
CGGCGCCGGT GACGGCGATG TTGCTGGACC TCAAAGGGAT CGGTCCGGAG TTCGCGGCCG 168840
TTCTCCGTGC GGAAGGCCTG TTCCGGCATT CCGGCAACCG GAGACAGGTT GCCGCTTATG 168900
CGGGGCTGGC GCCGTCCCCA TGGCGCAGCG GATCGATCGA TCGCGAGGAA GGTGTCTCAA 168960
AGGCAGGCAA TCCGCGGCTG CGAACAACGA TGATCCAACT CGCTTGGTTG TGGTTGCGCC 169020
ATCAGCCGGC ATCGAATCTC CCCCGCTGGT TCCAGGAACG GGTCGCGCGC AATGACGGTC 169080
GCCTGCGGAA GTCGGCGATC GTCGCGCTTG CCCGCAAGCT GCTGGTCGCG CTGTGGAAAT 169140
ATGCGACCGC CGGCGTTGTC ATTGAGGGGG CGGTCATGAA ACCAGCGTGA CGACACATCC 169200
CAATGCCGAT TCCGCCTAAA TCATCCCGCT CTAAGAGTTC AAGCCGTCTC CGCGAGCTCG 169260
ATATACATCA TGCCTTCCGT CCATAAGCAT GGGCGGACGT TGCTCGATTC GAACTTTCAA 169320
GTCTGCCAAA GCGCCGGGAA GGATGAGACC CTTCGGAGTA GCATCATCTG CCGTGACAAA 169380
AACTGGGTCA AGCACCGGAC GACAGCATTT GAAATGAACG GCAGCGTCCT CGATTTTTAT 169440
CGAGGGGGAA AAATGAGGTT CGCTTCCATC ACCTCGGATA AGAACGATCT TCCCACCAAG 169500
AGGCTGAGGG TAGGAGGCAA AAACAGTGAA GGCTGTTATC CCATCCCGCA GTCCTTCGTC 169560
ATCTAAGAAT AGTGCATGCG AGTCATCAAA GCGGATCATC TGCACCAGTC GGCAACCGAG 169620
CAGACTAAAG ATTGGCGCAA GTCCGGTGCC GTCGGCAACC TCGACGGTGC GAAAGAGTGT 169680
CGTCTCCGGG TCTAAGAGGT ATGCGACATT CTTCATGTCC CAGCCTTTCT CATTGTCATA 169740
AATGGCGTGA CCCAACAAAA GGCATCCGTC ACAGGATCAA ACAGCGGCGT GAAGCGCACA 169800
TCGGGTGGTG TTTCGCTATG CAGCATTTGC CAAGATTGGG AAAATCGATT TTCTGTATGG 169860
GGCCTATCCA CACCATGGAA TGTGGACGG ATTGCGTTCA AGCACATCAG TGTATTGCGC 169920
TTTTGGTTTT AATTCACGAG TGACTGCGTG TGCCGGTCCA GTCGGACAAT GATTCCGAAA 169980
CGAAGCGGGC CACCCATTTC GATTTCATTC CGGTGATTCC GACGCGAAGC CGGACAGTAT 170040
TGGTGAGCCC TGGGTCGTTG CGATATGCGC AGTTGGTGTT GCGCAATGAT TCGTCTGCTG 170100
ACATCACTGA GCTCAACGCG GGAGGCATTG CGAAGCCAGG CTGCAACGCC ATAGCCGAGC 170160
TTCTTGCCAC GATGGCAGAT GACAAGCACC GATTGACGGT AGACCGGATC ATTGCAATCA 170220
TCGCACGCGA AGGCGCTTCT GCTCGCACAG TGGATGGGAG CGAGCGGTCC GTCAAGCGCG 170280
CGGATGACTT GGCCGATCAT GATCTCGGAG GCCGGCTTCG AGAGGCGAAT ACCCGCCATG 170340
TGGACCCTTC TTCGAACGCA GAGTACATTG CGAAGCTCCA GGAGGATCGT GTCGAGGAAC 170400

TTCTTCGGAA TTTTGTTGCG CATGGCGATT TCCGTCACGA ACGTCGTTCC TGGCAGGAGG 170460
CGGACTTTTC CCGTCTGGGT GCCGATCAGA CGCTCGATAT CACCGCCGAA GCGGGGCTCA 170520
CCCCGGCGGT CGCCATTTGA TTGCTGACCG TAGGCCGCCG CGTCAAGAAG GTCGACGCCG 170580
GAGGCGCGAG CCGGGGCGTT GAACGCCCTC TCGCGGTACA CCCGAAGTCT CTAATCGGGA 170640
ATCAACTTGC GGCATGGTAC GGTAGTCATC GAGATCCGCC TCCTTCAGCA AGTTCCAATA 170700
ATCGACCAGA CGCCAAGGGC TGTTTGTCAC GATTCGGCCT CGGTCGTTGC GGAAAAAGGT 170760
GGACATACCC TCATGGCTCC AGATCATCTG CTGGTGCATT TGTTGGATTT TCCGGTTGTA 170820
ATGTTGATAT ATATCTTGCC GGCATTCTAC TTCGACAAGT CTCTTCTCGC CCATCTGGCG 170880
CAGAACACTT AGTACATAAT CGATTTGGCT CTCGATCGTG AAGATGAAGC TGCCGCGATG 170940
GCCGAGCGCC GTATTGGGAC CATAGAGCAT GAAGAAATTT GGGAACCCCG CCACAACGGT 171000
GCCAAGATAA GCCTGACAGT CATCATCGTC CCAGACGTCG CGTACCGTCC GACCATTGCG 171060
TCCGATTACC TCAACCGGAA GTAGATATCG TGTCGTATCG TAGCCTGAGG CGACGATAAT 171120
AAGGTCTGCC TCATGCGTTT CATCATCAAT CGCATGGATC GACCTGCCCT CAACACGCGC 171180
AGCAGCTCCG TCTACCAGCG TAACATGCGG CTTTAGTAGG GTTCTGTACC AGCCGTTGTC 171240
CAGGAGCATC CGCTTGCCGA ATGGAGGGTA CGACGGGATG ACCTTGGCGA TAAGATCAGG 171300
GCGCCCCGCC AGCTGTCCCT CGATGTATTG TGTGTAAGCC TCGCGGTCGC GGTCGTTAAC 171360
CGCATTCACG GAACGGTCCG GATGCGGCCA GGCCGGATCC TTCTGCAGGG CCTCATGCAC 171420
TTCACTGTCG TAAATCCAAC TCAGGCGGAG CCTGTACAGC CATTCATAGT GTGGCACCTC 171480
GCGGAACAAG AACTGCATCG GTTCCGGGAC CGGTTTTTGA AATTTCGGGA AGGGCGCCAC 171540
CCATTGACGT GAGCGTTGGA AGATTGTCAA CGCACCCACC CGATCTGCGA TGGCGGGAAC 171600
AACCTGCATT GCCGAAGCGC CATTGCCAAT CACGGCCACA CGTTTACCGT CGAGCGCGAC 171660
TTCTGGATCC CATTTCGAGG TGTGCACCAC CGGGCCGTCG AAGTTGCGCA GGCCCGAAAG 171720
GTTGGGCCAT TTCGGCGTGG TGAAGCCCCC CACGGCCGAG AGGACCACAT TGGTCACGAG 171780
GGTTTCCTCC GTGCCGTTGG GCAGGCGTAA CCGGGAGTGC CAAGTTAGGC TCTCTTCGTC 171840
ATAGCGGGTG ACCAGGCACT CCGTGCCGTA GCGGATGGAA CTCTCGATCC CAAAGTCGCG 171900
GGCAACACGG TTGAAATAGT CGTCGATTTC CTTTTGCAAC GGGAAGAACG TGCTCCAGTT 171960
GCCACTGGCA AAAGTGTACG AGTAGAGATG CCCCGGCGTG TCCACTCCAC AGCCAGGATA 172020
GTGATGTGCG TGCCAGACCC CGCCGGTGCT GTCTTGCTTT TCGACCTGGA TGTAGGAAAT 172080
GCCCAATTGT CTTAACCGAA TTGCCGCTGC GACACCGGAC ATGCCCGCCC CGATGATGAG 172140
TACACGAAAT CCTTTCGGCA AGGACACGGG GTCCGGAATG GCGCCTGCAT ATCTCCGTAG 172200
CTTATGAAGC ATCATGTCAG CGTATTCTGG CGGGATAGGC TCGGCCTCCG ACACAGTCAG 172260
CATGCGGATC AACTCTTCTT CCGACAGGTC CTGCTTGGTG ACTGGTGCGC CGCTGTGCCA 172320
ACAGATGATG GCGTCCAGTG CTGCATTACG AATCTCCGTC TGTAATTCGG CCGCGAGGCC 172380
CCCTGAATCG TTGTCGCCCC AGACACTCTT TACGGGAAGA AACGGTGGGG TGAGCCAGTA 172440
TTCTTTTCCC GTGAACTGAT ACAGAAGGAG TAATAGGGTT GGAACATTGG CCGAAGGGAG 172500
TGCCTCGGCG ATGCGGTGGC GGAACAGGGC GAGGTCCGAA GAGTGAGCCA TGATTTAAGT 172560
TCCTCCTGTT GCATATATCC ACTCCCCCGC CAGACGCTAA GTTTACTGCC TCGGCTTCGG 172620
CGTGGTTGCG GCAATCGGCA TTAGGGAAGA TCCGACAAGC AAGCTGCCGA ATGTCTGATC 172680
CAAATCGCGC TTGATAAAGC GAGTCGAAGG TCGCCACGGC GGGGAGGTTC TCGGGCGCCG 172740
TCGTGCCGCC GGCCCCGGCT CGACTGCGAA ATATGCCCCT GTGCTGCCTC GGCTCGACCA 172800
GGCGCGCGCA GCCGGTTACG TCTGCATCTG AAACGATCCA CCCCTTGATG ATCGGAAGGC 172860
GGGCTCGGCA GAAACCATTG GCTAACATGT CGAAATCTCT GCGCGATAAG GTGGGTCCGT 172920
GACGTGTCCT GCAAATGCCT AGAAGCAATA TGCGTGCCAA AAGCAAGAAC TCTTCTTAAT 172980
GCAACGAAAT GACTCAAGCA GCGGTCTCGC GTGTCCAACA TTGTCGTACA TCGGACGAGC 173040
CTCAACCTGA CATTAATTGT AACACTGTAT GTCTGTCTTC GTGGCCGAGT GGCTCGTCCT 173100
ACACTGCACT TCCGAGGTTG CTCGGATGAG CCGACCCAAT CCTAATCATA GGAGGACGAG 173160
CCGTGGCAGA TTATCGGCTA CGCCGCATCA GCGTCTGGTT GCCGATGCTT GGACGTCAGA 173220
TGTAGCCCGC TCCCGTCCGC AGGAAATCTA CGCCGGGCTA GATCCTGTCC TGCTATTTGC 173280
GCGATGTCCG CACCGTGCAG GAGCGGCTCG CTGCGCTTGC CGACACTCCG CCGGCCATGC 173340
GCTCTGACGG GGTAGCTCAG CCGATCGACC TTTTCCTGGC GAGCTTGCGG ACGGCCTGGA 173400
AGGACGGAGC TACCCGGCCG ACGGATCGTC CGATTGTAAA AGCCAAAAGA GGACGGCGGC 173460
GTCCTGACCC TCTCGTCAAG GCGACCGCCG ACTTGCGAAG TTGGTTCGAA GCCGAACCAT 173520
GGCGAACCGG CAGCGAATTT CTGTCACGCC TGCAGGCCGA GTATCCCGGC GATTACCCCG 173580
ACAAGCTGCT TCGAACACTT CAGCGCCGGC TGAAGGTCTG GCGCAGCGAA CAGGCGGACG 173640
CGTTGCTGTT TGGCACCTTG AACAAGGAGC TGCCGGCCCA GCAGATCGCA AGGCCACACT 173700
GAGGCGACGC CGTGGCGCTC GCGAAGGAGG CCAGGCGCTG CGAAACCCCT ACCATCTTCG 173760
CGCCCGGCCT CCTTCGCCCC AACGCGGGAA CAAAATGAAT GAGGCAACGT GCGAATCTCG 173820
GGAAGCATCG TTACGTGCGG CAATACGGAT GTACCAGATT GTCGCTCCAC CGCGGCTGCA 173880
TGTCGGTTAC AACTGGGCCA GCGGCCAGGG CGCGGGGCGG GAAGCTGCTA CGCCTCAATT 173940
CCGACCGGGC GAGATCTTCG GCCGATCGGG CCTGCGCGCC CCGCGATCTG CGGCCGGTGC 174000
TCCACCAAGG CGCAGAGGTC ATCGTGGAGC ACCGGCTGGA ACTCGCTGCG CCTGTTGCAT 174060
CCCGAGGGCG CCGCCATCGA CCTCTTTTCC GCACTTGGCT CAGGCGGAGC AGGAAGGCGA 174120
AGTCAGCGTG CGCATCGACG AAGGCGAGGC GCCGCTGAGC CGCTGGTCCC AGAGTCTCTT 174180
GACATCACTC CTGGTCACTT TCTGTTTTGT TTAATTCACC TTCTGCAACA CGTGAATGGC 174240
TGAGCCGGCG TCGTCTGAGA GCACATCTAT TGGCACAAAA CCCGCTCGGT GTGCACATTT 174300
TTTGAAAAAC GACTCAGAGA AACAAAAAGA GTTCTTCAGA TGCAGTCGCT CGCCACTTAA 174360
TAGGATGATA TCCTTGCCGC CAATGCAAAA GTCCAGGTTC TTTTTGACCA CCGCTGTATG 174420
TATGACTTGC ATGAATTGGT CTCCGCCTAT AACATCCGGC TCATACTCAA ATGCCTCTGG 174480
ATCGAAATTG CCGTCGACCG GCAGCTCTGC CTTCATTCTA TAGAACACAT TTAGCTGAAA 174540

TTCAGGATGC GCTTCATAGT AGGACTTCGC GACATCCCTC CTCAAATCTG AGCCAATTGA 174600
AATCGCAACC CAGCACCTCT TCGCACTATT CGATATCTTC TTCAGCGTCT CGACGACCGC 174660
TTCCATGGGG TGCGTATCAT TTGTTGGGAC AGCAAGATTT CCAAATGTCC GGCCAAACAT 174720
GACAATAAAT GCCGCTTCAT CGTCGCAAAA ATAGCGTCGT CCAGAGAATA TATCGCCGCG 174780
TATGAACTCG ATTTTCAACC CTAAAGCGGA TGACAATTGT GCTATGTCCC CGAGGAATTC 174840
TCTTGACCGG TCGACGAGCG TGCAACGTGA GGTTTCGCCT CGCAGACCTA TAACAAGCGG 174900
AAGCGTTTTC TGTTGGAAGG CTCGCACTGT TCCAGGCCCA TACTCCAGAA CCGAAATTCC 174960
TTTACGACAT CGTTCGTCCT GGTGATGCCA TTGAACATAA TCTCTGACTC TCCCGTGAAG 175020
TTTTTGGGCT AACTTGAGTT CAGCATCCAC CATATTGGTT GTGCCAGTGA CGAGCGCACT 175080
ACTGATCCTC TGGGCTTCAG CCTTAGCCCA TAACAACGCG CCACTCATTG GATTGCCATT 175140
CCGGTCGACT CCAGTGTGGT GAGCGTATTG TTCAGCACCT AAGTCAGGCG CATACTTTTG 175200
CCGAAACAGC TGCAAACTTG TTTCAAGAAA GCTCAAGCTT ATCGCCTCTC GATTCTTTAA 175260
CGGCTCGTCG GATCCCCAGT TGACGCACTT ACCGCCGCAA ACGAAGATGG TGCGCGTAGT 175320
CCATGAGTCT GTTCATGGGG TGTTCACTGA ACCCATAGGA GAGGCTACAG GGTTGGAAGG 175380
AGTAATCCTT GACGTGGATC AAAGACAAAC CCGAATCTGG GAGGGGCTTG AACAAATGAC 175440
GCTGGAAGGG GCGCCCTGTC GATCACTACA CGATCTTTCG CTGGGCTAGA AATACGCGCC 175500
GGAGATCGAA AAGCGGCTGG GTTGGCACCG GCGTCGGCTG CAGTCGACAA GTTGGCGCGT 175560
GTGAAAGTCC GTGGTCAATG GACATACCTT TACCGCGCCG TCGATAAAAT TGGCAGCACG 175620
ATCGACTTTT TATCTGTCCC CGGCGAGCCG TCGATCTGAA TGAGTTCGCC GAAGCAATCG 175680
CGCCGGCCGC GCGGCTGGAA AACCCGCTTC TTGCGTTCGC GTCGCAACGC CCAGATGCCG 175740
GCTTCCATAA TCCACTGACG CAGCGTCTCC TTGCTGACGG CAAGCCGGTG GCGTTCGATC 175800
AGCTTCTCGG CCGCCAGCGT CGGTCCGAAA TCCAGGTAAT GCTCACGCAC CAGGTCGAGC 175860
ACCAGGTTGC GGAATTCCTC GCTGTGGGGC TATATTTCCG GAAGGCCGCC AGCCAAGTAA 175920
TGAAATGCAA CGCGCTTGTT CAGCCGAGCC GCGCAATCTT CATGGAGCGA GTTTATCCGA 175980
TACCATTTAG CGTTGATTGT AGACGCAAAG TTGCTAATCT CGCGTTCAAA TGGAAGGGGC 176040
TTTTATGAAA ACTATGGTTG CAATGCTTCT TGCAGCGGTT GGCGTTGCAG TCAGTGCCAG 176100
CAGCACTTTG GCGGTTAATT TCTGCCAGTC CAACAAAGAC CGGAATTGCT TTACCGGCAG 176160
TTTCGGACTT GTCGACATTC CCGGTGACGC AAGCCATAAG CTGCTCAAGG CAGATTTCGG 176220
ATATGTCGAT TTGAACGGAG TGGGTTGGCA GACCAACAAG GAGACGAAGA CTGACGGCGC 176280
TTCGATTCCG CCGCTATTGC AACCGTTTGT CGGTTCCCCA TGGGAAGACG GCTATATTCG 176340
TGCAGCCGTC ATCCATGACT GGTACTGCGA CAGACACGTT CGAACTTGGA AAGAGACGCA 176400
TCGCGTCTTC TATGACACCA TGCTGGCCAG CGGCTTGGAG AAACCGAAAG CGAAATTGCT 176460
GTTCTACGCC GTCTATGCCT TTGGTCCACG CTGGGGCTAT CTCGTTCCCG GAGAGAAGTG 176520
TGCGGCTGGG AAGAACTGCA TCCAGATGAC AGGAAAGGAC GCCGCGTTCG TGCAGTTGCC 176580
TGGGGAATTG GCCGATCAGT CGAGCGCAGG CGAATTGAAG GCGATCAAGG CGACCATCGA 176640
TCTCAAGGAG CGTTCTGGTG ATGCGTTAAC GCTTGATGAA CTTATGGCGA TCGCCGACGA 176700
AGCTCACCCC AAGCAAACAC TTCGTGATCA GCGGCCTGCC GGTGGTGATG AGATCACCAA 176760
GTAACGGTGC AAGTCGTCGA TGGAGGACGC GAGGAAGAGG GACCGTCTTT CAAGACCGAC 176820
AGCACTATAC GATCTTGAGA AGCAGATTCA TCGCTTCAAC CGGCGGCCAA GTTAGGCTTC 176880
GTCAACTAGC GGAAAGTCTC AGTTGGCTGC TGAAGTTCGC AGCGCACGAG TCTTTGCTAT 176940
TTCCGATCTA CGACAGGCTT TGCGAATAAT CGCGCAGGGC TCCGACCTTG CAGTAATGTG 177000
GCAAGGAAGG AGGGACGGCG TGAAACCAGG GTGTAGAGCG AAGGCTCGTC GGTCCGGGTC 177060
CACGGCGCAA GCCACACCTG GCAGTTCGCA CCCGCGGCGC CGGCGAGGGT ACTGAGAATT 177120
TCTCGGACCA GAGAGGTATC AAGTTTGGTG GTGCGGGCGA TGCCTGCTTC TGTGCGCAAG 177180
TCGAAAGCGG GATCGAGAAG AGCCGCGAGG ACGGCGCGCA CAAAGTCCGG CCGTAAGTCG 177240
GCCGCCGGGA GAGGCCAGCC TTCGATCTGG TCGCGCATCA CCAGCTCCTG CAGGAGTGTC 177300
AGGCGGATAT AGTCGACGAT CCTATTCCTG CCGACGAGCA GCAGGAAGTT CAGCCCGAGC 177360
TTCATGCGAG CGCTCGTCGT CTCGGTGCTG ACAAGACGCC GCGCGACGGC GACCAGTCGC 177420
GCCTCGATGC GTTTGACCAG CAATGCGAAG GCGTTTTCGT CTATTCGCGC CCAGCGAGGA 177480
TAGGGCACCT CCGGCAGGGC ATCGCCGACG AGGGGAATGA TCGGTACATA GTCATGCATG 177540
CTGCCGTCCG AAAGGGTCTT GCTGATCACC GCGTTTCGCC GTTCGGCTGC GCCCTCCGGC 177600
CAGTGTAGCG TCTGATGGTT CTTGTCGATG GTGAGATGGC GCATCAAGAA ATATTGGCAG 177660
TTACGGCGGC CGAGCTGGTA GTCGTGGTCG CGGAACGCCT CCAGGACGAA ACCGCCGAAG 177720
CCGCCGAGCA AACCGCTTGC GATCGAGAAG GTCTCCTCCC GCTCCTCGCC GCCGGGCGTA 177780
CTAGGCGGCA CGCGGTGTGG CGAGATCATG AACCGGCTGT GGGTGCCTTC GGCGGCGACG 177840
GCGAGCAACT GATCAGGCTT GAAGCGTACC TGCTGGCGGA GCGCCGTCAC CACGCGCCTG 177900
GCAATGCTGA GCACCCCGAG CGGCGGTTCG CCATCGCCGA GAAAAGGTGG CCCCTCGGGA 177960
AAGGGCGCGA TCATGATAAC GGCTCGATCG GCCTTTTCGG CGTTCCGTTC ATTGGGCCGG 178020
GGCGGGTCCT TCATCAGGGT GAAGCGGACA AATTCGAACG GGTCGTTGTT GATGAGGCCA 178080
CCATCGACAG AGACGAACGA AAAGGGATAG TCTTGCGACG CCGAGACATG CCACGCGTCC 178140
GGCCACGTGG GTATTGGTCT GAGCTCTGAT TGATCCTCGG AAATCGGAAA CTTCGCCTTT 178200
GCATATTGCG AGGTGGCCGT CGCTATCACG CGCGGTGAAA GGCCAATCGG GAAGGCGCCG 178260
GAGGCGAGGG CGGCATTGGC GAAGGCAAGC CATTCCGGCG GGCGCGTCGA CGCGCCCGCC 178320
GCGCCGAACA GTGACGTGGC CGCGATCGAC GTGCCCGAAT CTGTGTCGGC GAAGGGGCTG 178380
GGGGTCGGCT TCCACGTGCC GATGCCCTCG ACCACGTAGT GCACGCGGTC GGCGTGCGTC 178440
ATCATGTTGT ATTGGCCGCC GTTGAAGTGA ATCGCGTAAG GCACGCCGCG CAAGTTCGAC 178500
AGCATCATGT AGAGATGGAG CGAACTGGCG ACATAGGCGC GTGGGCTGAG GGTACCCGTC 178560
GCCTCCAACG CCTCAGCCCC AATCGCGGTC AACACGTTGG CGTTGAGAAC CGAGATGACC 178620
GGCCGCCCGC CCGCCAGGTC TTCGAGCGAC AGCAGGTCGG GTGAGCCGTC GGGGGAAACC 178680

AGCGTCGGGC TGGTCACCCA GGACCGGTAA AGACGTCCGA GAGTGAAGCG GATGTTCTGG 178740
CTGCCGGGGG CGGGACTTGG GAAGGTCGCG GGAGCCTCGC GGCCGCCGGC GGCGATCACG 178800
CCAATGGCGG CGGTGATCGC GCCAGCAGAG GCCCCGGAGA GCGCCTTCAG GCGAACGTCG 178860
TACTCCGGCA AGTCGGGAGC ACCTTCCGCC TTCGCCTTTT CCCATGCGTC GAGTGCCTGG 178920
ATCAGAAAGT CGAAGACGCC GGCCGAATAG GCTCCCGCCG AAATCGCACC CGACATCGCA 178980
ATGCCGATTT CGAACGTAGG TTTAGAGCCG TCGGTCATCA CTGTTCCCTC AGTTGAGCGG 179040
CGCCGCATTG ATCCCTGCGG GCGCCATGAC GTTCGTGCGC GGCTGCCGCC GGCTGCTTAC 179100
AATGTCTTCA GGTATGCAAT GAGCGCCGCC TTCTCATCCG GCTTCAACGC CGGGCCTATG 179160
ACGCCCGGGC CGGGGGTGTC GCTGAATTCG TGACCGGCAT TGCTGTTGCC CGGCTTTGCG 179220
GTGTCGAACT TGAAGCCACC CTTCAATTCG TCCGTTAGAT AGCCAAGCTT GTCCGGGTCG 179280
TATTCGCGAT TGCCGAGCCA GAACGTCTTC GGCCGTTCGC CGGCCGGGGA AAGCAGGGCA 179340
TCGATGGTCG GCACCGAGCC GTTGTGCAGG AAGGGCGGCG TCGCCCAGAT GCCGTCGAGC 179400
GGCCGTGCCT TGTAGGCAAG CGGCGCCTGG ATGCCGTTCT GGCGATTGCC ATTCATGATC 179460
TCCCGTTGCT CGGCCGGCAC CGGCGGGGTC TGGTTGTCGT ACCAGCGAGC GGCTGTCTTT 179520
GCGACGAGCG CGCCGAGCGC GCTGCCGAAC GAGTCGGTGT CGATGCCGAG CTCGGAGGGA 179580
AGCTTGACCT TGCGCTCCGC CATGCTTTGG GCCTGGGCCG GATCCGTGCC GATATTCTCG 179640
ACGTTGATGA TGGGCACGCG GAGATAGCGC TCGCCGGCCG AATTCTCGTT CGTCCAGTGC 179700
TTCTGCTCCC AAAAGCCCTC ACTGCCGACC GGTGGCAGGT GGCAGCCCTG GCACCGGTCG 179760
GCGTAAACTG CGGCGCCCTC GGTTGCGAGT TTGGTATCGA TCGAACCGAA CAGGTTGGAA 179820
GGCCAACGCG GGGGACGCAG GCCGGTGAAG CCGTTCTCTG CCGTCGGTTG CTTGCCGGCC 179880
AACTGCTGCT CGATCTCGAA AATCGTGTCG ACCTTCACCG TCGAGGCAAA GAGTGGCTGC 179940
TCCGACTTGG TGAGATTGAT GAAGGCGCGG ACCCCCAACG CTTCGCCCGC ATTGCGCACC 180000
ATCGGCTGCA TGATCGAGGC ATTGTATTGG ACCCAATCGA ACCAGGACGT GTCCCAGATA 180060
TGTGGGAAAG CGACTGGCGC GGATTGAGCG ACGTAGTTCT CCGGCCGCTC AAGGTCGAGC 180120
GAGAAGACCT GGTTGCCGAT CCGGTTGAGT GCATCCAACC GGCCGAACCC CTCTTCGATA 180180
CTTTTTTCGG CCACCTTCTT GTCGAGATCG ACTTCGATCC TGCCACCCGC AAGCACCTTG 180240
TCGAGCTGTT TCTTGAGCAC GGCCCTGGCT TTCTCATCCG CCTGCGGCCC GAGCACTGCC 180300
GTGGCGAAGC GGTCGAAACG GAAAGGCGCA TAGCGGGTGA AGAACAAGGC CAATCCTGAA 180360
GCCTTGCGGA ATTTTCCAAG ATCGGTGAGT GCCGGGCCGC CATCGACCAT CACGGCAGTA 180420
CCCTTGTAAG TGAAGCGACC GGTGTGGCAT GCCGCACAGG TGAGCCCGAC GGTGGTGAGC 180480
GGCTTGCCGG TCGCCGGATT GACCCACGGT TGGGCGGTTT TGGGATCGAC AAGATCGCCG 180540
CCATGGGCAA AGCCGACCGG AAGGCCGGAT GAATCCGCCG TAATGAAGCC GAAGCGGTCG 180600
AGATAATCGC TGGACAGGAA GGGCGGCCCG GCTGTCAGCG TGAAGGTGGG CTGCTCCAAC 180660
GCCGCCAGCC ATTCGTAGGG CATCGAGAAG GTCACCGTGC CCTGGTCGGC ATGGTGCATC 180720
CAACCGCGTT GTGATTGTGA CCAGTTCTGC TCGAGCCAGG TGACCTTGTC CACCTTCGGA 180780
TAGTCAGGAA GATCGACGCT AAACATCCGG ACCACATAAT AGAGGCCGGC TGCGACCAGC 180840
ACGGTGAACC CGAGCAGGAT AGGCCAAAAT CGCGACTTGC GTTTCATGCG TCTTTACCCC 180900
CAAAGCTTGA AAGCGCCCCT TCCAGTGCGG CGTGGTCCTC TAATTATTCT GGGGCGGCGG 180960
CACTGCCGCC CGCGCCGCTC GCGCGCCCGG ATAGACGATT GCGCGTGCGT GACCGAGCGA 181020
TGCGTAATCA TCTGGACTGG TCGCCAAAGG AATGCGAATG CATTCCGGGC TCCGGTTTGG 181080
ATTCATCTCC AGCCTGCTCA CGGCCGCCTG GTCGTCGATG ACCTCGTTAA GGCGAATTTC 181140
GAACATGTCC ACCCAGGGGA ACCAGTATTC GTTCCACGGC TGGGACGGGT CGAACAGCTC 181200
GTGATTGCCG ACCGGCGGCG GCGGCGTGTC ACGCAGCTGC GCCTGCAGGA TGTAGCGCAC 181260
GTCACTGTGC CTGACGCGGT GAATGAAATC CTTGCGCAGG AAGTCGGGAG CCCGATCGTC 181320
GTCAGCCTCA GCGTCCATGG ATGGATGGCC GTTAGCCTTG AATTCGGGGC GCGGCAGACC 181380
GCGATCGGGG CCGCGGTCGG CGTTGATCAG CCGGAAGCGG ACGTAGCGGC TGACGCCAGT 181440
TTTGTCGATG AATTCGAAAC AGACCCAGGT GTGATACCGC GAATCCGTGA ACGAGCCGCG 181500
GATCCGATAC GCCTCGCGTA GTTGATCATC GAGCATCAAT CCGGACTCGA CCAGCTTTAT 181560
TCGTTCTGCG TCCGGCGTGT GGACGAAAGT CGAGAAGTCG CGGATGCTGC GCACGAAGAG 181620
CTGCCGGCCG GCATTCATCA GAACGTCATA GAATCCGTCG CCATCGGTGC TGGCACCTGG 181680
CTCGAAAAAT TTTATCGAGG CGGCAACCCC ATCGCGTGCG CGGTCGTCGG CGCGACCGCC 181740
CGGCGAGGAA TGGCGCAGGA TGATCGGGAA GATACGGCCA GGCACGAACA TGTCGGTTTC 181800
AGGGAAACCG GCCGGCGTGA TGCAGCGCGC CTCGCCTTTT GCCACGACCC CGTAGGTGTG 181860
CGTCGCACGC CCCTGCTGAA GATTGGCGGC GTCGCCTGCA GCTTGGCAAG CACGGCCTTG 181920
AAGTCGTCAT TTATGCCGGC GGTCAGTTCC TCGGGTGAAA GGCGCATGTT GAAGCTCCAG 181980
TGTTCGTTGC CGGTCGGCTC GCGGCGAACG ACCTGGTTGG TCTCGTGCCG GAGGCCGGAG 182040
CTGCTGAGAT AGACCCGCCG GCGGACCTCG TTGATCTCGC CAAGCGGCCG GTGTTCAAGC 182100
AAGGCGTGCC ACGGCGTGAA GGAGAGGTTG TCCCCGAAGG CGAGCTGCGC CGCCGAGGTG 182160
AAGGTCTGCG GTGGTATGGT GATGCGGGCG ACCGCCTCGA CCGGTGTCGC CCATTCGACG 182220
GTCGGGTTGT CGATGGTGGT TGCACTTGTG TCGTCGTTGA GCTGGACGCG GAAGTCGAAG 182280
ACCGCCGGTC GCGCCGCCCG GGTGAGGTGA TCGGTCATCG CTTCGCGGAG ATAGCCGGGG 182340
CCGCGGGCCT CGGAGGGAAT GGACGAGACA AGGTTGTCCT TTGAGGGAAC CACGGAATAG 182400
CGACAGGCAT GCGCATTGCC CAATCCGTAA GCATAGGGCA CCTGGCTCCA GTAGCGTGCA 182460
GCAAGCGGGC TGTCCTGCAC CTGCTGGCGG AAGCCGAGAA GCACGGGCAG GTCTTCGGGC 182520
CGGTTTTCCT TCAACCATGC GATGAAGGCG AGCGGCGGTT CACCGCGAGG CGCCGACCGC 182580
GCGAATTCGG CCATGAACCG GACATAGTCG CTGGTGTTTC TGATGAAGAA CACCGGCTTG 182640
TCCGCCAGCA CGAAATCATG GGTGGCGGCG TCCTCCTCGC CTTCCAGTAT CTTTCGGCCA 182700
GCCACCCCGG TCAGCTTGAT CGCCATGCCG TGAATATCAG GCTTGGTGTC GTCGATCTGA 182760
GCGCCGTTGG AAAAGCGGAT ATAGGCCGTG TAGACCTTCG GCTCGGCGAA AAGCCCGACC 182820

CTAAGGCGCT CGGGAAGGTC CGCAAGCACC TCGAAGGTCG CAAAAACGCA GCCATGCTGT 182880
TTAAAGTGCT GGCCACGCAG GCCGGGGCCG GTGCCCCGCA TCAGCAGCCG CTGCAACTCA 182940
TCGATTTCAG CGATCCCGGC ATTGACGTCG GACGAGGCAG TTTCGGTCAT TGCGCCAGTG 183000
CTCCAAGCTA TCAGTGGGGT GAACGCGAGC CCGCGCTAGC GGGTCTTCAG ATATTCGATG 183060
AGGTCCTTGA GGGCATCGGC ATATTGCGGG CCACTCCAGT CGAGTCGACG GAGCTTGCCA 183120
TCGACCACGA GATTGCGGTC GTGGCCCGTG CCAGCATGGC CGTCCTTTCG CCTGTCGTAG 183180
ATGGTCAGCG TCGGCGACGT GTCGGCAACG AGGCCGGTAA CGGCCGGATC CAAGACATAT 183240
CCGACTTTCG CAGTGTCGTA TTGGATGGCG CCGCGCAGGA ATTGCTCCGG GCGGCTCGCC 183300
GGCACCAGGA GATGGTACAG CGTCGGAATG GAACCATTGT GCAGGTAGGG CGCGCGCGCC 183360
CACACCCCGT CAAGCACCTC GGCGACATAG CCCTGGACCG CCGGCGTCGT GCGGGCGGTG 183420
ATCACGTCCT CGCCCGCCAT CCGGCAGGGA CGGACTTCCT CCCCCGTCGG GCTGCGATAG 183480
AGGAAATCCT GATCGTGGCA GCTTGCCTGA AAGCCGGCGG CGAAAAGGTG AAACGCCGCG 183540
TCATTGAGGA CGGCCGCGCG GTTCATGTCC GTGCCTATAT CGCGGAACTG GTAGATTGTG 183600
TCGTTGTGCG GCTTGTGGCA GACGGCGCAA TTGTCTTTGA ATAGCGCTTC GCCGCGCACG 183660
GCGCTCGCCA TGTCGACATC GAACGGGTAG GCGGCCGGTG GCAGGCCGTC GAGGAAATTG 183720
GCGACAACGC CGGTATTGAC GAGATCGATC TTGGAAGGGT CGCCGACGAT CGGAAGCTGA 183780
GCGGCGATGT TGCGCCAGAA CGCCATCTTC ACGCTGCCGT CCCACTGGGC GAGCGAACGC 183840
TCCTGTTGGT TCCACACGCT CGGAATGTCG GTGATGGTCG CAAATGGTGG GATTTCTGGA 183900
TGCGAACTAT TCTTGAATGC CGTGGCATCG AAGTTCCGGT TGAGGCCCCC CGCCGCGTTC 183960
GGCTGACATT CGCCGGGGGG CATTGCCTTG GCCACCAACA ACTGGAAAAT GAGATCGCCG 184020
CTGCCATCCT GCTGTCCGGT GCTGAAGCCG GAAAAGCCAG GTCCGTTCCA GTTGCCGTAG 184080
CTGGTCTGTT TTTGAAGCTC GATCCCGGCT TCGCGCTGAA CGGTACCGCA AGCGAACGTC 184140
GCCAAAATGC CGGGAAGATT CTTTTTTAAA GCGGCTCGTT GAGCGACTTC GACATTCGGC 184200
GTGAAATTCA GGAATCCAGG TTTGCCGAAA TAAGCCGGAG GATAGAAATA CCCGTTCGGT 184260
TTGGCATCGA TGATGGCTGC GATGCGCTCG GCCGACGCTT GGATCTTTTC AGGGGTGGAG 184320
AAGTAGGCTG TCACCGTTCG CGAGTAGGCA TCCCGCCACT TGCGCACGTC CATCTTCGTG 184380
TTCGGCGCAC CCTCGACGAC CGCCGGTCCA TCCGGCGTCT GTACCTGTCC AATGTGGCCC 184440
GCAGGCAAGC GTCACGACAT CGAGCCCGGG CTTGGCGAAA TCAATCTCTC CTAACGGATT 184500
GCCGCCAGCA TCCAGGGGGC GGCCCGCGGT CGAAGCGATG CCGAGCCCGA GCGGCAGCGG 184560
CCGCGATTTG TCATCCGGAT CAGGAAAGAA GCCGAAACGC CCGAAATTCT CATCGTTTCG 184620
CCCCCAAATT TCGGGTGCGA GATCGGGTAA CACGCGCTGG AGCAAATACG GTGCGCCAGT 184680
AAAGCCATTG CCCGCATTCG CGAACCACGA CGAGCCGACC GGATTTTGCT CTCGGTAGGC 184740
GGTCCGTTCG GCATCACACC CAGCGCGAAT CTCTGTCCAA CTGATTGTCA TATTCGCACT 184800
CAGGTCATCT CGCGCCGAAC AGGCGGGGTT TGCTGCAGCC AGCCCGTGGC TCCCGAGGAT 184860
CCAGAGCGAC GCACCGTACG CCACCGCAAA AAAGTGCAAA GCAAAAGGGA GACGTTCCCG 184920
ACAGTGAGAC GGCATTCTAA CCTCGGTCTT TGCTTGCAAG GAGCAGATGA ATTGGGCAGA 184980
CTTTAAGAAA GGCTGTCAGG TAATGGCGCG CCCAGATGGA AATCCCCGCC GCCGTTTTAC 185040
ACGCATAAGT ATTAGCATGG GCTGGGAGGA TTGCAAGCCT CAAAGGAGAG GTCTTTTGCG 185100
TGTTCTTTAG CTTGACGCTG CTGCTTTCCT TTAGGTTACC AATGAATTAA GTACCGGACA 185160
ATTTCTCCAC GAGCAGGGCA ACAATATTCA AGGATAGATT TGTCCAGGTG TCGGCCAAAG 185220
GATGTTATTA TAATACTAAT TCGACTTAAA CGCGATCTCC GGGTCATCTG ATAGATCGGA 185280
ATATAATCAT GGCGCGCTGG CGCCGAGTTC ACCAGCAAGA CGCAGAGCGC CAGCCACGTC 185340
AAGCCGCTCC AATCCAGACG ATATCGTCCC GCGGCCCCCT CATATGATGA GTTGAAGGTC 185400
AAGAGGGTGG GGGCACCATT TTGCAAGCCT TTCTGTCGTC GACGGTATCC AGCTGCGCCC 185460
ATTCCCAGTA CTGGCAGGCC ATGCCTCACC TGTACCACCT CATATCCGGT CGCCGCTCAA 185520
GACGGCTGCA CCATAATCCC GCATTCGGGG GTGGGCTCAG GAGAACGGAA CCATTCTTTT 185580
AGGCGGCGAT AGAAGCCATG GGGGGCTTCG GTCCGTTCGC CTGGATCCGT CAGAGCTGTT 185640
CAGGCTCCGA GGGATTGGCT TGGTCAAAAG GTGGGTGGTC AGGCCTTGCC CATGACGGCT 185700
CCTTCGATCA CGACACCGCT GGTCGCATAC TTCCACAAGG CCACGAGCAG TTTGCGCGCC 185760
AGCGCGACGA TCGCAACCTT CTTGCCACGC CCGCCGCTGT GGCTGATCCG TTCATAGAAC 185820
CAGCGCGTCA GGGCCGAGCC CGGCTGGTGG CGCAGCCAGA GCCAGGCCAG TTGGATCATC 185880
ATCGTCCGCA GCCTCGGATT GCCTGCCTTC GAGACACCTT GCTCGCGATC GATCGAGCCG 185940
CTTCGCCACG GCGAAGGCGC GAGACCTGCA TAGGCTGCAA TCTGGCGCCG GTTGTCGAAC 186000
TGGCGGAACA ATCCCTCACT TGCCAGGATA TTTGCGAACT CTGCGCCAAT GCCCTTGATC 186060
GCCTTGAGCA TTGCCTGGGG CGCGGTACCA TCCACCTTGG CACACTGGGC ATCGCGCTCG 186120
GTCTCGACAG CTTTGATCTG CTCGTTCACC AATTCGAATC GATCGAGCTC ACGACTGAGC 186180
TGCGCCTTGA GATGGGCAGG CAATTGCCGG CCATCCCCCG TCCGTAGTTC ATCCAAGCGT 186240
TCACGCCGGT CGCGACGCGT AGGCTCATAA CCTCGGACGC CAACGCTGAA GAGCAGTCCT 186300
TTGATCCGGT TGACGTGTCT TTTGCGCTCC TCGATCAGCG CTTTGCGCTC TCGCCCGATC 186360
CTGCGACGAT CTTCATCGTC AACCGCCGGG ACTCGGACCA TCGAACAAAC CCGCGGTTCG 186420
CTCCGCATCC ACGCCATCAG CGTGCGAACC AACGTCTCTC CGTCAATGCG GTCGGTCTTC 186480
GCCCGGCGGT GCCGGCGCGA CACCGCATCG AGGCCGCATC CACGACATGG CTCGTGATCC 186540
AGTCTTCCGC CTCGAGCGCG CGGTGGATCC AGAAACCATC GAGCCCCGCT TCTTGGATGA 186600
CAATCAGGGG ATAGAGCCTG CCCTCGCGCG CCTCTGCCTT CTCGCGCAAG AGGTTCAAGC 186660
ATGCCAGCAA AGCAGGCAAG TCACCGCCGG GTACCGAATG ACGCGACATC TTCTCGCTGC 186720
CAGGCGATAG CGGCGTCACT AGCCAGATCG ACTTCGACAG TTCCAATGAA ACGAAAACTG 186780
CGTCAAGATC AACCCGGATA GCGGCAGGTG GCGAAGCTGG TTCTGCTGTC ATGGCAAGTC 186840
CCTTCAGAGT GGTTGGTAGC AAACCGACTC TGATCTCTCA CAGGCCGCTG TCCACCGCTC 186900
ATCATGGAAT CTTTCGAGTG AAACTCCGCT CCGGGAAAGC GCCAATCCTT GAGTTTGATC 186960

CCTTGCTGGC AGGCGGCGGT GAGCGAACGG TCCTTGGCGG CCTGAAAACC AAGAATGTCG 187020
ACGTCGTGGT CACTCGAGAA GGCATCGGAC CGGTTCTTGC CGTTTCGTGC AAGGTCCGGA 187080
ATCTGACGAA CCGCATGGAA GAGACCATCG GTATAGCGCG GCGATCAGGA TGAGACGGCG 187140
GCGACAATCT GGATGAGACT CTTATGTCGC GGTCGGCATG AAGGTATCAT GTTGATTGTC 187200
GCTGGCAAGG GTCTCATCGT GTTCTGATTG TCGCTCCGCG ACAATCAGGG AAGCACTTTT 187260
GGTTGTCGCG AAGGCGGCGG GTCTGCCGCG GTGGCGTTTG GCTTCCATGG CGGAACGTCG 187320
CCGATAGCTT TCGACGTTCA TTTCGAAGAT CGTTGCGTGA TGAACAAGTC GGTCCACCGC 187380
GGCAAGCGTC ATGGCCGGGT CCGGAAAGAC GCGGTTCCAT TCTCCGAAGG GCTGATTGGC 187440
GGTTATCATG ATGGAACGCC GCTCATATCT TGCGGAGATG AGTTCGAACA GCACGCTGGT 187500
TTCGGCCTGG TCCTTGGTGA CGTAGGCCAG ATCGTCGAGG ATGAGCAGAT CGAACTTGTC 187560
GAGCTTTGCG ATGGCGGATT CGAGCTGGAG TTCACGCCGT GCGACCTGAA GCTTCTGGAC 187620
GAGGTCGGTC GTGCGCGTGA ACAGCACCCG CCAACCATTC TCGATCAGCG CGAGGCCGAT 187680
GGCGGCGGCG AGATGGCTCT TTCCGCCGCC CGGCGGACCG AACAGGAGGA TATTGGCACC 187740
TTTGGCGAGC CAACTGTCGC CGGCGGCAAT GGCCATGACC TGGGCCTTGG AGACCATGGG 187800
CACGGCGTCG AAAGCGAAGC TCTCGAGCGT CTTTCCGGGC GGCAGATGCG CCTCGGCGAG 187860
GTGACGTTCG ATCCTGCGAT GCGCCCGTTC AGCCAGTTCA TGCTCGGCGA TGGCCGAAAG 187920
GAAACGAGCC GCAGGCCACC CTTCACGATC GGCCTGTTCG GCAAATTGCG GCCACAGCGT 187980
TTTGATCGTC GGCAGCCTCA GGTCATTGAG CATGATGCCG AGGCGGGCTT CGTCGATGGT 188040
GTTGTGGACG TTTTTCATGC GGCCTCTCCC GCATAAGCAG ACCCCATCAG GGCTTCATAG 188100
CTATTGAGCG ATGCGAGCTG CACATGCACG GTCGGCAACT GATCCGGGTC CGGACCGAAG 188160
ATGGCTCTCA AGGCCATCAG GTCGGGCAGT TTGCGGGCGT CGAGCGTTCT GGCAAGCTCC 188220
TCGGCCAGTT CACGCTCGCA ACCGCGATCA TGAGCCAGGG CCAGCAATTC GACGGTGATC 188280
TTGCAAGCCT GCCGGTCAGG CAGTTGCTCG ATGAGAGCGT CGAAAGCCCT GCGATATTCC 188340
GGCCGGGGGA AGAGCTTGTC GCGATAGACC AGGTTGAGAA GCGCCATGGG CTTTTTGCGC 188400
AGAGAGTGGA TGACGTGGTG ATAGTTGACG ACCTGGTCAT GCTTGCCGCT CGCGTGGGCG 188460
CGACCTCGTG GCAACGTCAG CAGATGCGTG CCGCCGATGA AGACGTCGAG ACGATCGTCA 188520
AACAAACGCA CACGCAGCCG ATGGCCGATC AAACGGGAGG GGACGGTGTA GAAGACTTTG 188580
CGCAAGGCGA AGCCGCCGGT GCGCGACACG GTGACGACTA CCTCCTCGAA GTCGGTGGTG 188640
CGGCGCTCGG GAAGCACCTG CAGATGCGGG CGCTCGGCAT CAATGCGCTT GCCATGCGCG 188700
GCATTGCGGC GGCTGACGAT CTCGTCGATG AAGGCGCGGT AGGAGCGCAG ATCGTCGAAG 188760
TCTCTGGTGC CGCGCATCAG GAGTGCATCG CGGACTGCGT TCTTGAGATG GCCGTGGGAG 188820
CTTTCGATTG AGCCGTTCTC GTGGGCGACG CCCTTGTTGT TGCGCGTCGG CGTCATCCGG 188880
TAGTGAGCGC ACAGCTCCTC ATAGCGGTTT GTCAGATCGA CCTTGGCATC GGCATCGAGG 188940
TTGCGGAAGG CAGCCGACAG GCTGTCGCTG CGGTGATAGA GCGGCGAACC GCCGACCGAC 189000
CACAGGGCGT TCTGCAGGCC CTCCGCCAAG GCGACGAAGC TTTCGCCGCC AAGGATGACA 189060
TGGGCGTGCT CAAAACCCGA CCAAACCAGC CGGAAGTGAT AGAGCAGATG GTCGAGCGGT 189120
TGGCCGGCGA TCGTCACGCT GAGGCTGCCC ATGTCGGTAA AATCCGACAG CCCTAGTCGG 189180
CCGGGCTCGT GCGTCTGGCG GAAGATCACC TCCTGTGCTT CACCGTGAAC CGCCCGCCAT 189240
GACCGGATGC GCCGCTCAAG TGTGCGGCGA ATGCCTTCGG GCAGTTCCGG ATGACGCCGC 189300
AGCATCTCGT CGTAAACGGC GACCGCACGA ATGCCGGGAG CGGCCTTGAG GAGCGGAACG 189360
ACCTCCGCAT CAAAGATATG CTCAAGCGGA TCGGGTCGAC GCCGACCGCG GGGCGGCTTG 189420
TTCTGCGACG GAAGGCGCTG CTCTTTCTCC ATGCGGAACG CCGTCGCCCG GCTGATCGAC 189480
GCCTTCGCGG CGGCGACCTC AACAGAATGC GTTTGTCGGT ACTTCATGAA TAATCTCATC 189540
TGATGATCGG TTACATGGCG ACCCGGCACA AAGGTGGTTC TCCATTCCAG AAAACCGCCA 189600
CCGTAGCGGG CCGACCGCGA TCATGAGACG CCTAAAAATT GCGCCGCGGC GGGGGTGTAA 189660
CTCCGGTCGG GCTACGCCCT CCCTTCGTCA CACCCCCACC GCCGAGTCTC ATCCTGATTG 189720
ACGCTGAGTC TCACCTTGTT TGTCGCCGCG CACCATCGGC GAGTGCACTA ACTTGCACAT 189780
CACCTATCCC GCACTCGTGC TGGGATATCT CGTTCTCCTT CGAGCAAACC GGATGATCGA 189840
AGCGGCACTC GATGACGCTG CGGAAGTCGC CGAAGATGCC GGCGCTGTGA GCGGGACAAA 189900
AACACTACGC TATGACCGAA GGCGGCGCAC CGGTGGCAGG CATCGTTCGC TTCCACAACG 189960
CTCTGTGTGA ACTCTTCGGC CGGCGAGGCA TCCGTGACAA CGTCAGCCGT TACGAGGCGA 190020
TTGGCTTCGG TCTCGTCGAG ATGCATGGCG AGGAGCGCAA CAGCCTCCTC GAAGGTCTCC 190080
CGCAATCGGA CAGTCCATTT CGGCTGAGTG GGTTTTTTGA CACGCTCTAC GCTCGTTACG 190140
ACGAGCGCTT CGTCGTTAGT GCACCGTGAG CGTCACGAAC GCCCCACCTT TTATCGGCGT 190200
GATTTAGGCC TGAGATTCGG CTCTTTGATT TGGAAGGGCT GTTTTGAATG GACGAGCGGA 190260
ACGATAGTGC CAGGTCTGAA GCCATGTTTG AAGCCAGGCA CGAAGGGCGG TATCGCCGTG 190320
TTGAGGTGAT CACGGGGCCA GTCCGCCGAC GGAACTGGAC GGACGAGGAG AAGGCTCTGA 190380
TGCTCGCGGA AAGCGCTGAG CCGGATGCGA ACATCTCTGC CATTGCCCGG CGCTTTGGCG 190440
TCAATCGCGG CCTTTTGAAC ACGTGGCGGC GGGCGGCAGG CCAAATTGGC CCGGTTTTGG 190500
GAGAGCCCGC GCTGGAGCAA CCCGCTTTCG TTCCGATCAG GATCGCTGAC GATCAGAATG 190560
AGCATCAAGC TGATGAAGCA GGTATCGCCG AAGGCGCCGC CGGTCGGATC GAGATCGAGC 190620
TTGGCGGTGG GCGGATGATC GTGACAGGCA ATGTCTCTCC CGACTTGGCG CATGCTGTGG 190680
TGATGGCGCT GCGGGGTAGA CGGTGATCGG GCCATCGAGG GGGCTGAGGA TCATGGTGGC 190740
GACCCAGCCG GTCGATTTCC GGCGTGGCAT GAACGGGCTC GTCGCACTGG TGGGGTCGGC 190800
CCTTTTGGCC GACCCCTATT GCGGCGATAT TTTCGTCTTC CGGGCCAAGC GTTGCGACCG 190860
GCTGCGCTGC ATTTATTGGG ACGGGTCAGG CATGATCCTT TCGACGAAGT GGCTGGAAAG 190920
CGGGAAGTTC ATCTTCCCGC CGGTCAAGGA TGGCGCTCTT CACATGTCAC CCGAAGAGTT 190980
CAGTCTGCTT GTTGCGGGCC TGGACTGGAC ACGCGTCAAG CGCAAGCCCG TGAAGCGACC 191040
AACGAAAGTG GCTTGATCTC ATTGTATTTC CTTGCAGATT CACGCCCGTG TGGTAGCTTT 191100

TCCCGAGTTC CTCGGCAATC AGCTGATGCT CGTCCTTGGA TTTTCCAGAC GGCATGTACA 195300
TGCAACAAAG GCATGCCTGG CCGTCATCGA AACCATGCCG CGAGATGCCG AGGTCGTGTT 195360
CCTGCGTCCA CGCATTCGCG ATCCATCGGG GCAGAGCACC TTGGACAGCG AGACGGTCGG 195420
CGGCTGTGTC AAGCGCCACA CCCACTCGAT CGAAAATCCA GTCTCCCCGG CGCGCGACAT 195480
GTTCTGCCCA CTTCAGCGGA TGCGCTTCGA CCTCTAAAGC GGTCGATCGA AGGGCGGTGG 195540
TTGCAAGAAC CGCCTTGGAC ATGCCGATCT CCGCTTGGCC GGCCAATACG TAGCGCTGCA 195600
GGTTTGAGAG TTCGACCGCT TCGTGGTCGA CGACATGCAG GCGACCCGAG AGGCCGGATT 195660
GTCTCGCCAG AGCCCAAAGC GCGCCATGGC CTATCGCGCC GAGCCCAACG AGGTGCGTTT 195720
CGCCAAGGTC GACAGGAAGG TCAACCGGGC GAGCATCGCG AGCTTTGGTC TTGTTGTAGC 195780
TGTATAACGA GAGGTCAATG TCCGGGTCCA ACTCGGCGCC GGTTAACTGG GCGGCGAAGA 195840
TGGTTCGAAA GACGTTGGCG GCGCCGAAGC AACTGGCGGC GCCAGCTCCA TAGGGGAGCA 195900
AACTCGAGCC AGAGCCCACC GGGTCCGTAC GCGACAGCTT TGCCGCCCAA CCGTCCGATC 195960
CGATGAAAAA GGTTGTGCAC CGCAGAGATG GCCGCGTCGC GCCTGCAACT AGGCAGACCA 196020
TGGCGGACTT GCCGGAACGC CGAATGCCGA TTTTTGGATT GATCGACTTT GCCAATCGCT 196080
CCAGCGCCTG GGCTTGAAAG CTCGACGCAC TGTCCAAGGG GAGTATTGCC AGAACCGGAT 196140
ACAGTCTGGC AAGCAACCTC ACCGCAAGAT CTAGCGTCGC CTGGCCTTCG GCGCAGGAAG 196200
CGGCCTGATC GTCGAAGGCG ACGGCCACGA CCTGCTTTTC AAGAGCCGCT TTGAAGTCGC 196260
CCAGATGAAA ATCGGTCAGG ACCTGAGATG CCGCCGTCGC GGCGCGGTCG ATGAAATTAG 196320
CAAGGGCCAT CGTCAACTCG TTATCGTGAT CATTCGCGTC AGGACCGCGG ACGGGACCTC 196380
ATTCCAATTC GCCTTAGCGT CAAGTCGGTA GGCGGCAACA TGAGCAAAAT CGAAAGGCTC 196440
CCGAGCGAAA TTCGGCACTA CCAGCGACAG ACACCCAATC GTGGTAGCGA CCGCATAGGC 196500
GTCATCGGTC GTCGAGTGAT AGGCGCGGCC CGGATGACTA TGGATCTGCG CCAGAAGCGT 196560
CAGGCCACGT TTGTAGAGCC AGACATTGAG CCGGTGCAGT TCTTCGGCAG GGACCATCAC 196620
ACAGACGCCA TCACTTGTTC GGATGTGGCG CTGCGCCGGG ATCACTGTCT CCGCTATGAC 196680
AAAATGCTGG TCTTGCTGGA CGCCGACCCA GAGTGCCATC CCTTCATGGC CCTGCTGTCC 196740
AACCGACCGC AGATGCGCCT GTACTGTGGA CACGCAGTCT CGGGGGAGGG CGACTGTCCT 196800
GACATCCTCT AACGCGCTCA TTCTGGAATG GCCTGAGGTG GTACTAGCAG TCCTCCCAAC 196860
GCGACCTGAA GGTGAATCTG CAGTTGCTCC GCAGGGCCGG TCCCGTATTT GATGATCTTG 196920
TCCAGGATGA AGGCCAAACA GCCTTCGCCA GAACCCCGAT GAAGTAGCCA TGGGTCACCT 196980
GAGTGCGCTG GATTATCGTG GTATTCCCGG ACTCCAGCCA TGCATAGGAA TGGTTGGTCC 197040
TCGAGACTGT TGGCTTGAAT GAAATCGGTC AATGATACCG CGTTCTGCTG GATAAGATTT 197100
GAAATCATTT CCGTCGGGGT TCCGGGCAGT TGCGGACGCC TTAACATATT CAGGGGAAGG 197160
TCCTTCCGGG CAATCGGCTG ACGTGTAAAC GGATCGACGA AGACCACAGA CAGTGGTCTG 197220
AGATCGTAGT TGGTGAAGTC GATCTCGATG GCCGCGCCGA TAACGCGCGG CTTTACCTTT 197280
GGGCTCGCAA ATATGAAGAA AGCGCTCGGG AAGCTCTCCT CGATCAGAAA GCAGCCTTGC 197340
ATCCTATAGG CATCTGCATA TGCGCGGAAC CGGCTGATCT CCCGATCAAA CTTCGCTCGA 197400
GAGACCTTCG GGTCCACAGT TTGGAGTTCA GGCACCTGCG ACACCCGCCT TCAAGCTGAG 197460
GAACAGGGTT CCAGTGTTGG CGAATCCGAA ATCACCGATT TTCTTGTCGG CGTCGAGCAG 197520
CGTGCCGGCC TCGTCCTTGA ACTCCCAGTT CTCGGCAGGT TGGGCCACGT TTTGCGTGTT 197580
CTCAAGGGCT TTGGTGCGAA CGACGTGTAG CGGCTGGTTG GGATTGGCTT CGACCTGCGT 197640
AGGCTGGCCG TTCACCACGA CGATGATTTC GATCTTCCCG GGGCCGCCTC CGTGATTATC 197700
GCCCTTACCC GCTTCCTTCG ACATTGTCAT ACTCCAGTGG CTTGCCAACC GGCCCCAAGG 197760
CACTACTGCC GACCTGCCGG TGAGCGGGCT GGCGGTCTTT GATCGTCCAC TCGGCGCCAC 197820
GAACCGCGAG CACGTGAAAG ACAGGGGATA AGCTACCGAA TCGGTGCTAG GGCTTTTTTA 197880
CGCGGTACTT GAGAGGCAAG TAAACCTCTG GAAAACTGCT GATCACAACT ATAATCGATT 197940
AGAGCAGCTA AAGTATTGGC GAAATCTTCC ATGAGATATT GCGGTATGAT AGCGCTGGAT 198000
ATATAATGCT ATCTTTGATA TCTTCATTGA AGGGAAGCCA TCATGGCAAA TGTGACCGTT 198060
AGGAACCTAC CCGACGAGGT GCACCGAGCG CTACGCGTAC GAGCAGCAAT GCATGGCCGC 198120
AGCACCGAAG CCGAGATCCG CGACATCCTA GAAACGACTG TCCGGCCCCC GGAGCGGGTC 198180
AAACTCGGCT CTTTATTGGC GTCTATTGCC CATGAAGCTG GAGGGCTGAC GGATGCCGAA 198240
GCGGAGCATT TCAACCAGCT CCGTGACAAG ACTCCTGCTG AACCGATGAG CTTCGAATGA 198300
TCGTGCTCGA CACCAACGTC ATATCCGAAT TGTGGAAGGC CGAGCCGGAC CGCGCTGTGT 198360
TGGCCTGGAT CGATGCGCAG ATGATTGAAA CACTGTATTT GTCCGCGATT ACGGTGGCCG 198420
AACTGCGTTT CGGCTTGGCG GCCATGCCGG CAGGCAAGCG ACGCACGATC TTTCAGAATC 198480
GCTTGGAAGG AGAGGTCTTG CCGGCCCTCG CAGGGCGCGT TCTTCCCTTC GACTTGGACG 198540
CGTCGCGAAG TTATGCGGAC CTGATGGCGC AAGCGAAAAC GTCAGGCAAG GTCATCGGCA 198600
AGGCGGATGG CTATATCGCC GCAACGGCGG TTGCGCATGG CTTTATGGTT GCTACGCGCG 198660
ATACCAGTCC GTTCGAGGCA GCTGGACTAG ACATCATCAA CCCCTGGGAG CCGGCATGAT 198720
CCCGCCGATC TCATTTCTCT ATGCGGTATG TTAAACCATA ATGTGGCGGC CAGGTTGAAC 198780
TGTCCGCCGT TGGGCAAAGT TAAAATGTCA CTTTGGATAC GTCTTCAGCC ATTTAGAAAT 198840
ACGACACTCG GCATGTCCAC TTGCGCTGAG GCAAGCCCCC GACCGGGCCG GCTGGGCCGG 198900
GTTGCAAGGG AAGGGAGGGG GCGGGTGAAG GGCACCCCTT CGGCTTTAGC TTTCTCGGTT 198960
TCAATTATTG CGTTCCTGGT AGATGTTGCA GGCAGCTGAA TGTGCAGGGC CAGGATCTCG 199020
CGATGCTTCC GGAAATCAGG CTGATGGGTG ACGTCGATGT AGCCGCGCTG TCGCCACTGT 199080
TTCGTGGCAT GGTCATGACT GTTTCCTACG CCGAGACCCA AGGAGGGATC GGTCTGACCG 199140
CGTCGGGGGC GATGAACCGC AAGTTCGTCC ACTGGGCCGC TGTTCACTTC GATTGGCCCG 199200
GCCACACGTC GGACGACTTG TACAGCGTCA GCAAGGTTCT GAACGAAGCC GATATGCCGC 199260
CGCTTTTGGT CGTCCGCGAC ATGCTCAAGC ACTTGCGGCT TCTTCGGCGG CGAAAGGACG 199320
TACTGGTACC AACCCAACGC GGCCGAGACT TTCTGGTGAG ACCGCAGGCC TTCTTCGATC 199380

TGATCGCGAC GGATTATCTT TATGCTTATA TCCACTACGG GCAGACGCGG GAGGCTGTCC 199440
GCAATCGGAT GCGTTGGTGG CACGTCTTTC TCAATCTTAT CAACATGAAA GCAGAAACGG 199500
GCTGTTCGTT GGACGACCTG GCGAACGAGC TATACCCGAG CGAATCGTAC CCGGAGCCAG 199560
CAGAAATGAC TGTCGAAACC TGGGCGGAAA GGTCGGCGCT CCGCTACGAT TCATTCGCCC 199620
GCTGTGCTGG TTGGGATTGC TGCACGAAGA GCGCGAGGGG CTGACCATTT GGCAGGGCGG 199680
AACCTACCAC AAGACACCCT TGTGGGCTGC CTGTTTAAAG CTGGAATCAG ACAGGCAATC 199740
CGAGTTTATC CTCCATTGAA CTGTGCCGGG TAGGGTTGCC CTTGCTCCAC CGCACCTTGC 199800
TGGGAAAGCG CCTGTCGACG CCGAGCAATG ACGGCCGGAT CGTTCATAAA ATCCGTCCGC 199860
TTGCCCGGCT TGCGGCGCCG CGGTTTGTAG CCAATCTTCT CGCTGTTGGT CTTCACGGTG 199920
TCAATCGGCG TCCAAAGACG AATTCGGCAG TGCGACAAGT GCCCGTGGAA AGTATCAACC 199980
GACCCTCACG ATATTCCGCA TGGCTACACT GAAACGCTGC ATCACGCACT TGCGAGGACG 200040
ATTGCCGAGC CGGGATCCCT GCACGACGCA GGACATGTGA TGGCTTGTCA TGAGCACCCA 200100
CCGGGAGACG AGGCCGACTG CGTAGGCTGG CTCATGAACC AACTTGGTCC AGGCAACAAC 200160
ATCCCACTGC GCCTCAAGGT TCGGTCCTGC GAGAACATAG ATGCGGTCAC ATTGGACGGT 200220
CCGCAGCATG AACGGTTTGT GGATACGTTG CCTAAAACCC GCAAGGAACC AGCCGATTAG 200280
GTGGTCTGCG GCCGCGACGT GAGCGCTTCG AGATGACGAG AGATCGTAAC CGTACCGCGC 200340
CGCCGCTTTG CCGCCAACGT GTATGCGATC GCCTGATCCA AATTTCCGAT TGGCTAGGTC 200400
AGTTGCTGAC AACATGGTGG TTCATCGCGG TCGCCACTTC CATTTTCGAG CTAAGCACAT 200460
AATCTGGGCG CGACGCCGGA GACCGCCACT ATGACGACAC CACCGACCGA ACCCACCTAC 200520
TGCCTGGCGT TGGTAAAGCC CGGCTCGCGG CTCGCTCGCG AATAGAAGCT GCTGAAGCCG 200580
AGCTACGGAA TCTACGAGTA CGACCGAGCC TTCGGGACGA GGGAGCTGCG TTGGGGTGAT 200640
GGGTCCTGGC AGGAACTCAC CGATGAAGAC CATGCCGATC TGATTTTGCT TCCGCGGCTC 200700
GGCAAGAAAG ACATTGAAGC GCTTTTTGAC TACTGAGAGT TCATGAAGCA GCGCGGATCT 200760
ATCTTAGCGT CAGCTCGCAA GAACTCGGTG CGCCAAACCA ACGCTCCGCG CCTTTCGCCC 200820
CAGGAGGTGA GCTGGTTTTT CAAAGACCGG ATGCCTTGGT AAGATTGACG CGGAAGCGGT 200880
CGCGCCGGCG CTGATAGTTG CGGGTCATTT CTGCTGTGGC TTTTGCTTCC CGATTTCAAT 200940
TGAAGCGGTA AGCTGAAATC TCGGCCTTGA TTTTGACCTT ACGCTTTTAC GCGCCGAGGA 201000
AGGGGAGGCT TTCGAAGCCC GGATGCTTGG TGCGATCGAT CCGAAATTGG CGCGCCGGCG 201060
ATGGGATTTG GCCGTACTGG CATGCGGAGC TCTGGACCTA ACGCTCGTCG ACGGTCGCTT 201120
GTCCCAAATT GCTGATGCTG CCGTTGATGT TTGATCTCGC AATCGCCGTG GCAAAAGTGG 201180
TTTCACTGAA ACCCTTAGGG GAAAATCGTC CGGTGCCTAT CGTCTAAACC CAGCCGGCAT 201240
ACGACAATTT CCGGTTTGAA GTGGCAAAGC AAAACCTACA TCGGGTGGCA GTGCCGCTTT 201300
GACGTTCGAA AGCATTGCGA TGCGAGCCTA TCGGCCGGCG CAGAAGTGCC GATGACGCCG 201360
GCGCGCCAGA GGCGGCGGAT GGCAAGACGA AGGCGGTCGG TCCGACGGGT TCGATGAAGC 201420
CAGCGCGAAC GTCGTTTGTC GCCTCAACTC TCATTGCGGT TGGTGACGGA CAGCCTGACG 201480
CCGTGTGCCT GCGAGGGGAA GGGGAGAGGC GCCTTAGGCT TTTGCCGCGA CAAGGGCGGC 201540
CAGCGGCGCA ATAGCCTGGC CATCGAGGCC GAGGCGATCA CCGAGATAGT GCCAGAACGA 201600
CAGGCCGAGC TTTTTGCAGG TCTTCATCAG CCCAAGCATG GTGTCGCGCG CCACGCGCCC 201660
ATCCCGGCTC ATCGTGCCGC CGGAGATCTT TCGCTTGGTG ACGAAGCTGC GCAGATCGTT 201720
CTCCGACGCA TTGGTGTGCA GCGGGATATC CGGCCGTTCC AACACCTTGA GCAGTTCGTC 201780
CTTCCGGCGA GAAAGACGAG CCAGAAGCTT GTCGAGGTCT TCATAGCCGG TGCGCAGCGA 201840
GAAGATGCGC GCGAAGCGTT TGCGGAATGC GGCGGCAAGG CCGGGCGGGG GCTTTCGTTT 201900
GACGCTCTTC AGCGCCCGGT AGAACCGCCA CACAAGATCC CGCACCGCCT CGACCTGTCG 201960
CACCTGCCGC GGCGTTGCCG GCATCAATTT TTGCAGCAGC CGCTCCGCAT AGACCCAACA 202020
CAGAGCATGG TTGCCGACTC GGAACTGGCC GGCATCGTCA GAGACGATCA CCGTGTCGCC 202080
GAGAAGACCG TGATGGCGGA TGGATCCCCA GATGCCGGCT TCTGCGAGCA CGCGGACCGC 202140
CTGCCTGTCG AAAATGTCGA TGCCCTTGCC AGCCAGATAT TCGAGGAACG GCACCTGGTC 202200
GCAAAAGCGT CGTGGTTCGT GGCTGCGGAT TTTGGCCACA AGCGCCGGAT CGGCACGACG 202260
GCCGTCCAGA TAGTCGAAGG CGGCGTCGTT GAGGACATAG TCTTGATAGC TGCCTCTGAG 202320
CAGCGACAGG AAGTTCAGCC GGGATTTTGA TGCGGTTGTG CGGAAAGCGG TAAAATGCTC 202380
AGCCCCGATC TGGGTGGTGT GGAAGCTATC GCGCGCATGC CGGGCACCGG TATCGTCGAC 202440
GGTGATGTAG GGGGCCGAGA CAAGGCCGGC ATGCAGCACA GCCGCATCCT CGGCAGCAAA 202500
GCCGTCCAGT CCCTGGGTCA AAAGTCGCAC GACCTGGCGC TTGGAGACGC TGACGCCGAC 202560
ATCGTTCAGT CAGCCGCGCC ATCGTCACCT GTCCTTGCGC GTGCAGCATC AGGCAGAGCC 202620
GCCTGAGGTT TGGACCGTAG CCGCCGACGA CCCCTTGCGG CAGCGGCGCG AGCACTGTCT 202680
TGCCGTCCGG CGTGATCCAA TATTCGCGCC GGTAGTGAAC GAGCTCAGCT GTGACAATCA 202740
GGTCCCGCAC ATGGCAGCTC TTGGGCGGAA CACGGGCGTG CAGGATTTCC TGCCGGCTCA 202800
CCCGCTTTAG ATCGAGCTTC GGGCCGCGCG GTTTCTTCCT GGTCGCCCGC GGCGCGTCGC 202860
GTTTGTCATC CGTCGCCTTG TCCATGCCCG ACGGGCGAAA CGGCGGCCGT GGCGGCAGGT 202920
TCTTCAGCCG TGCGATCTCG TCGCGCAAAA GCTGGTTCTC GACCTTCAGG CGGGTGTTTT 202980
CCAGCCGAAG CGCCTCATTG TCTTCACGCA GGGCGGCGTT CTCGGCGTGA AGGGTCGTGA 203040
CCTCCGCTGA AAGCTCCTTG ACCTCATCGA CCAGACCCGT CACAAGCACG CGCAACGCCT 203100
TCAGCGAAAG CGTGTCGGCA TGTTCGGGCG AGGCAAGTCG CTTCTTGGCC ATGATCGAAA 203160
GGAAGCATGA TTTGCCCCAA GCGCGGAATC CCACCCGCGG CTCCGACCCT CTACATCGCC 203220
CGCTTATGCA CAGTTCTCGC AGCGCGTCGA TGAAAAGTCC GCCACCCAAT CTGCCCCGGT 203280
TACCCGCGAC CGGTCCAAAA TCTCCGCCTG GCGGAAAAAT AATATCCGGA TCGAGAGACC 203340
CAACTACTCG TCGTTGCACA CGGGGTGAGC ATGACCGCGA AATCGGACTG AAGATGACCG 203400
GGCCGGACCT GGCGCGGCAG ATGGTGGGGC GCTAGCTCGC GGGGCGGCGC ACTTCGTCAA 203460
AGCTGCCGGA AGTGATCGAG TCATGGCAAA ACCGATCCCT TCGGCCGGCA TTGGCCACCA 203520

AGACACCAGA GGTCACGCCG CAGGCGGCGC CGCGGATTGT CGGCGAATTG GGCTTGAGAG 203580
AAGGGGGAGG TTTCGTGGAA AGGTGCGCTT TAGCTGTTGA TAGGTTCACG TGCGCCGGTG 203640
AATATCGATT AGAAGTTGAA AAGGCTCTTG AGCGTTTCAG ATGTCGTATG TATTCGAGAT 203700
ATTTATAAGC GATCGGCGAT AAAGCGGAAT GAGTCTCTTA ATGGCACATC GGAAGTCAAA 203760
ATCATCTCAG AGAAAACTTC GGAACCGGTC GTCATCTCTA ACCCCGCAAA AGCGCCGTAT 203820
TCGGGCCTCC AAGGGCTCAC ACGACACTAT CCTGAGTATT CGAAATGAAA CTTCCTCATT 203880
GGAAGCACTT AGTCGTACTA TTCAGTGGAC CCTCGCGAGG TACAAGTATC ATCGTTACTT 203940
GGTATCACTA TCCCCGCGGA CCCCATCGCA GCTCCCGGAC TATGTCCTAG CCGCGAATGA 204000
TAGTCTAGAA AATGCGATGA AATGGCAGAT TGCTTCAATC TCCGCCGTTG GCGGCAAGAT 204060
CGCCTCTCAC AATAAAAATA CTTATACCAG CATTGACCAG ATCACTGATT ACTCCGCCTC 204120
CTTGGAAAGG GCAAATGCAG AGATTCGAAC GTGCATGTGG AGCTTTTCGT CAGCCTCTCA 204180
AGCTCTATTT TCGCTCGCGA GAACGAAAGG CCTCGACGCT CAACGTCGAT GGATGCAAGG 204240
TAGCCTTTAC GTTGCAAATC CTTCAATATC AAACATAATT CTCTATAGCA AAGGAATAGC 204300
GTCAGAAGGA GATCGGCATC CATCGGACGT ACTTGAGGTA TTGAATAGAT TTATATTCTC 204360
AAAATTTGCC GACGAAGAGC TAAATTTGCT ATTGTACTAC CTGACATTTA ACCCTCTTCT 204420
GAATGTCGAG GTGGCATCAA AGCTTTCTCC TCTCCTTCTG TATTTTCCTG TAGTCGATCA 204480
GTATGAGTTT CTAGCTAATT TATTCACGTC AGATCAGAGT CTATCCAAAC CAGATGTTCT 204540
GCCTTTTGGG CGAGATTTTG TCGAGATTCT GTCATCTACT GGAGATTGGC GCGCAAGCCC 204600
AAAGGCAGCG CTCGCAGAAA GGGTCGAACC AAGTCGTTTC ACTATTCCTT TGCTGAACAG 204660
ATGGTGCGGC TACCTTCTCG ATAGTATTGG GATACTGGAT GGAGTTTCTT CGGCGCCCGA 204720
TCGCGAATTT GACATCGCCT TAGCAAACGA GTTTTTCCAT CAGCCACAAT CTCCCCGCGC 204780
GTATCTGGCG AGCTCCATTT TTGCGATGAA ATCCGCTCAG ACACTCGCCG ACGTCAAGTC 204840
GGCATTATAT AAACGGAGA TAGCCAACGT ACACTTCGAG AAAGTAGAGC AACAAATTGC 204900
TCCGCCCGAT CAGCGCTTCA TTGACTATTC TTTTGCCGCA GTTGCCGCTG AAGTCGGAAT 204960
TTCGGAGTCG GTATACGAAA ATCGGGAACT ATTCAGAATT GCTTGCATTT GCGGGATTAG 205020
CGAGGGACGC ACGCTCGATA CGCTCATTCT TCTTTGCGAA TATACGAATG CCGATCCATT 205080
TGGATCAAGT TACTTTCCTG CAGACCTATT CTCCGCGAGC GTAACTGAAG ACGAGGTGGC 205140
CAGGATTGGG CATGATCCGC GGGTTGCTAT CGGGCTTTCG CGCGTGGCCG CTAGCCTCGG 205200
CGATGAAGGC CAAAATCTGG TCTACATCGC AGTTGAACAA CATCTCAACG TTAGAGGAAT 205260
GAGCAAGCCG AGTGAAATTG TGGCTGACAG TGCTGTCGAC GTCGCTTTCC TTCGTGAAGC 205320
TTGTACCTCG AGCTCCTTAA GGCAATCGCT AGAGTTTTTG TCAAAGGCCG AGATGGAGGA 205380
CGAACGGATC CAGGTTCTAT TCAATTTGGC TCAGGTGGAT CCTGCAAATG AAGACGATTA 205440
CATCGAAGAA GTTCACACAA TCATTGGGCA GCAAACGATT GAGGAGCTTT TGCAACGATT 205500
CCATGTAGGT AAAGTGCAGT GCGATGAGCA AGCGCTTTCC ACATGGGCGC TCATAGAATT 205560
GAGTCCGAAG TTCAACCGTC TGAAAGATTT TATCGATGCC GGATTGCCGC CCGTAGAGAA 205620
GGACGCTGAC ATTCAATTTA TCGCTCATTT GACGTCGGGA AAATCGGAAA CATTTACTTT 205680
TAAAGTTCCC AATAATGAGT CGCTCGACAT TGCCCGCACG ATCTTGGCGG AGTTGAATTC 205740
GAAATATGCG TTAGACCCGC GCTATGGCGT AGACTCGTAC TTGAGCCTGG GGATGCGGCA 205800
TGGCGCGGTC GAAGCTCATC TTCAGAGCCC GCTCAGCGCC GAGAATATTT TGACTGCGAA 205860
GGAGCCTCTC GGATACCCGG ACGATTGTTT CTGGACGAGG TATTTCATCG ACAATGGTTT 205920
TGAGTGCTAC GGCGAGAAAA TTGGGCCGGT GCTAGCAACA TTTTCGGAGA AGTTTGACAA 205980
TAAGCTGGAG GCTATCAAGA ACGATCTTTT GCAGGTTCGT CGGCCGGACA AACCAGAAGG 206040
GCTAATAGTA GCAGATTGGT CAGAGGCTTC GGTTTTGTCG GCTTGCGCAA GATTTGCCGA 206100
GGTACCTGAC TTTGAAGCCT TCATTGCTGA GTTCACCTTG ATCTTTTGGG CGAACGTGGA 206160
AGGTAATCTG GCTGCCGCCC GTGAATTCAT AGAAAACGTG CTTTCGAACG AGCTAAACGA 206220
GTTGCTAGAC GAACTGGAGG CAAACGTTCG TCAAGCAACT GGGCAGCAGC GACTGGCGCC 206280
TTTCTCAGAT GCACTCATGC GCGCACGCCA GGAGCTCGGC AACGCTGTCA ACGACGTATC 206340
GACATGGCAC AATGTGGCTC GTTCTACTGA CGTTGAACCA CTTGGTCTGG TTGAGATTAT 206400
TAGCGCTGCC CAGAAGATTG TTTGCCGCCT GTATCCCGAT TTCGAGCCAA GAGTAACGTT 206460
TTCGGGAGAC ACCGGCATAA CCGTAACTTA CTCGCTCCAT ATACTTATTG AAGTGTTTAA 206520
GGCCTTGTTC ACGAACGTCT ACGCCCATTC AGAAGTGGAG AACCCAGCCG TGGACGTCCA 206580
CATGGCAATG ACGGTTGAAG ACGCACTGGA TGTTGAATTC GTCAGCGACT GCAATGATAT 206640
GGGCAAAGCT GAGCGGGCCG CCCTTGATGC CAATGAAAAA ATTAGGACTG GGGAATACGA 206700
GAAAAAGCTG CCGAAAGAAG GAGGTTCGGG TTTAGCGAAA GTGGCTCGCT CTACTCTGCG 206760
AGACGGAAAA CCAAACACCG TGATTTCAGT TGATCATTCC ACGTGCAAAT TTCATGTAAA 206820
GATGGCCTTC AAAATAATAC AGATATGAGG CTCACCCCGA TGAAAACCTT AATCGTCGAA 206880
GATAACCCTA AAAAGAGAGC CTCTTTGGTT GAATACTATT CGTCCGAGTT TCCTTCGGAT 206940
GACCTGGAGG TGACATCTGC TTTAATATCA GGACTGCGGG TCGCGCGAGA TACTAAGCCG 207000
GAGTTCATTA TTCTGGACAT GACTCTTCCC AATTACTCGC CCGATGAAAA CAAAGGCTCG 207060
CGTATCGAGC TGATGCCTTT CGCTGGCCGC GAGTTTGTGA TGCGAGTAAA TCGAATGTCT 207120
ATTAAGACGA AGGTGATAAT TGTTTCAATG TTTGAGACTT TCGGCGTGGC CCCTCGCTTG 207180
ATAACGCTCA ACAGTTTGGA CGCTGAGTTA AGGGACAGAT ACCCCAATGT CTTTGTTGAG 207240
GCGGTTCATT ATTCCCAGGC CCAAGCAGAT TGGAAAACAG CAATTAAAAA TGCACGGCTT 207300
AGCTTGGATA GATAAATGCG AATTCTGATC GTTGATGACG AAAATACCAA GGTTGTTGAA 207360
ATCTGTGGCG TTTTACGAGA CGCCCAAATT ACAGAGGAGT CGATTACTGT CGCTACGACA 207420
GCAGCTAGTG CCCTTAAAGA GCTCAAGGAG AGCTACTTCG ACTTGCTAAT CATTGATATG 207480
TACTTACCTA ACCGAATTGG TGAGGCCCCA AGCCTCTCGG GCGGGGTTGA TTTATTGAAG 207540
CGGATCAACC GTGGGAACGA TGTGCAGTTA CCGGAACATA TCCTTGGTTT AACTTCTAAT 207600
CTCGAAGCTT TGGCGGCTTC TGAGGAGGAT TTCTCCGCGA GATCGTGGTT TGTCGAAGAA 207660

GTAGGGCCAT CAAAGACCAC GTGGAAGCTC CGGCTGATGG AGAAATTGCA GTATCTTAAA 207720
GCGCGTGAGG AATATCAAGG CAACCAGTCG AAGAGCGAGA TTATCTCTAC TCGACCCGAA 207780
TGCGAGGTTC TGTTTGTTTG CGCGCTACTC GATCCCGAAT TGACAGCGTT GCATGCAGTT 207840
TCTGGATGTG ACTGGGAAGT CGTAACATTT CCCGGTGACC CAGGAATCTA TTGGTCAGCT 207900
AGCTTGAAAT TCGGTGCAAA TACGGTGTCG GCGATCTGCG TTTGCTTGCC GCAAATGGGG 207960
CTCGTTGCAG CGGGTGTCAC GGCCGCGAAG GCAATAACGC TATTCAAGCC CAAATTGGTG 208020
GTTATGACCG GCATCTGCGC TGGTCGAAAA GGCGATTGTG ACCTCGGGGA TATCATCGGT 208080
GCGAATCTGA CATGGGACTA TGGCAGCGGA AAGTTTACTG AAGTTGCCGG TGCGGTTGTG 208140
TTCGAGCCCG CGCCGTTTCA AGCCGCCGCT ACCGCACGAG TCGGGGGTGT GTTGGCGGAA 208200
CTTTCCAACG ATAACGAGCT ATTGCAGAAG CTGTATAAGG AAAGTCCGGG CTATAGACCT 208260
GCTAAAGTTC CGAAGTTTCA TGTGGCGCCG CTCGCGTCAG GCGCAGCCGT CCAAAATCAT 208320
AAGGAGTTTT TCTCTGGCGT AGTTACGCAA CAGCGCAAGA TGTTGGGGGT CGACATGGAA 208380
GCGTTTGCCA TAGCCTGGGC GTGTCACGAG GCATTAGAGC CTCAACCAAG TTGGCTAGTA 208440
ATAAAATCGG TCGTGGATTT TGCTGATGGA ACAAAAGATA GCCAAATACA ATCGTTTGGT 208500
TCGTATATCA GTGCATCACT GGCAATTTAC GCAGTCGATA GACTGATAAA TCGTTGATGG 208560
GGTGTCAGTT GCCGGTCAGT CGTCCGGATT CGGTGGCTTC TTCCGGTTCA AGGCGGTCGC 208620
CGGTCAGGAG ATCGTGCCAC CCTAGGCGGG TGCCCCGCTC ATTTCGATGG GCTGGCTTTC 208680
CTGTTCGCGC GAGGCATTCT CGTGCGGATG ATACATAATC TGGAAACAGG GCTCAAAGCT 208740
AGGCGATCGT ATCCGCTAGC CGCTGCGCAA CACCTTGTAG CTACCGGCCG GAGGAAGAAG 208800
CGTTGCGCAG AGGAACATCG AGAGCGAGCA TTTGGGGGAT AGTCCGCGTC ATCTTAAGGG 208860
AACTCATAAA GCCGGATCAT TCTAACTTGG AAGGCAAGGT GAAGTCCGTC GGACTGAGGT 208920
CTAGTCGTTG TTCGAAGTTT TCGAACTCGG CGGGGCTAAG AACAATACCA TTGCCCCCCA 208980
CCAGGCCGCC CGTCTGCGGA AGTCCCTGCG CTTGAACATC TGTAAAGCTC GGGATTTGGC 209040
CGTCGGGCCA GGCGTCGATG CTATCGAGTT TGATCTGTTT TGGTTCCAGA ATGACGCCGA 209100
GCAATTTCGG GTCAAGCCGG TAGTCTGACG TGGCAAACAT CTCGCGGGCA TTTGTGGCAT 209160
TTGTCTCAAT GCTCTCGAGC GAGCCACTCA CAACGCCGGT AGGCAGTAAA TCCAATGTTT 209220
CGCCGTCCGA AGTCTTGAGG TTGCGTGCGA TCGCGTTGAA CTTCCCGACC GCGACAGCCA 209280
ATGCCTTAGG ATCTTCGAGA TTTATCGCCT GCTTCTCTAC TGTTGTTGTG ACCATCTTCG 209340
GCAATGACGA GCAAGTTCTG CCGCCGAACG GCGTCGGCTT GCACTGGAGC TCGTTGACGA 209400
CCTTAGTCTC CAACGTCCAG TCGGGCCCAT AGGTGGCGAC AGCCCAATAC ATGGCCTTTG 209460
CCTTCCACCC CGGCACTCCG TTTGCGCGCA TGCCGTAGTA GAAATTTCGA TGTGTGTCGT 209520
GGGCCGTGCG GGATTTGGTC TCGCAGAAGT AGTCGTGGAT CACCGATGCC TTGAGATAAG 209580
CGCCCTCAAA GGGACCCCCT ATAATCGACC AAAAAGTCTG CGGGATCGAC GCTCCATCAA 209640
CTCTCGTCCC CGTGGGCACT GGCCACTTTA GGCCATTAGG GTCATCGAAG CTGAAGTCGC 209700
TCGACAGCTC GAACAGTGGA CGCGGATCAG CATCGATAAA CTGTCCCGCG GGTCCGCTCG 209760
AGAAGGTGCC GAAAAATTCG GCTGCCTGGG CCTCGGCGAC GCCGAAGCCC AGAATAACTG 209820
TGGCCCCCAA CAATCGCTTA GAAATTTTCG CGATTTTTCG GATCTCTGCC GGCCAAGCGC 209880
TTCCCATGAG AATTACCCTC CTGTCAGGCC GATGTTTCAC AAGTTTGGGA GTAGTTGTTG 209940
CAGGTCGCTT CTGTTGAGTA TCTGGATATT GCGCAGTTGC AGCTCATTGA TTGCATCGTT 210000
CGCGTTCTTG ATCGCGCTTT GCGCCTTGCC CAGACGTCGC CTTGGTCTGC GTTCGTCGCG 210060
AGGCTCTTCC TCCTCGTCCT GCACGGCGAC GCCAATGGTG ACGGTGTGTA TGTCTTGTCT 210120
CCAATGGGAT GACTCTGCGT CAACAGCCAC GATCTTCAAA TGGTTAGCGG GATCCAGTTC 210180
GGCACTGGGA GTGGCCGAGA AGCTGTTTTT AGTCGTGAAC TGAATCTCAT CGGCAAGTTT 210240
GTTGACCTTT GGCTGTCCAC TATCAGTGCT GCCAAGAATA TGGTCTTCAT TCAGGCGCCA 210300
AAACGTATCG ATTATTTCTT GAAGCCCGAT GTATCCGGTT ATGGGATAGG CGTAATTTTT 210360
TTCGCGCTTG AACTTTCCGT TCTCGGAATA GGGCTCGCAC CTCGGGGGAT CCGTGAATAG 210420
CTTGTAGAAA TTATCCTCGA CTCTAAAACG CCGCTGATTT CGGCGCGTTC GCTCGTGGGC 210480
ACCGGAAACA CCCAAGTTGA ATATTCCACT GCTCCAGGTA TCGTTCAGAG TAGCGCCAAG 210540
ACTGTTTCTG CTGTGCTGCG ACATATCGAA CTCGAAGTCA TAGACAATCG CAGCATTTGC 210600
GTAGAGATCG ATCTTCTTTC GTGTTGCATT TGGAAGTCGG CTGCGATCGA AGTTCGCGAT 210660
TGTGTTTGGG TCTTCCTCTA TCTGGGTGAC AATTTCCGGA GATACGTTGC TGCGACTGTT 210720
TTTTAAGTAC TCAAGCAGTT CGGACAGGAC GGCATCTCGC GCTTCGCACC GAATTCTGGT 210780
GACGATCGCA TAAGTATTCA ATCGGGTGAC ATCTTCCGGA AGTGGCCTGA TAGCGCAGTT 210840
CGGCAACAAA GCCGCTGCTG CTGTCAACGC GATCGCATGC CTCATCAGTG CCCCCCGACA 210900
AAGACCCGCT GAATTGGTCA ACGCTGAGAA TCGGCGGCGC TAGCTTTTGC CGCATAATTC 210960
CCTCATTAGT CCCCTCTAAA TTGTCCGAAA TGCCGGATGA TGGCAACTTT AAAGTTTAGT 211020
GATCAACCTA TGATTATGTT TCGCGTGCCG GTCGCCGGCT CTGTTGACAA TGAGTTAGTC 211080
CAGGCTTGCA TGTCGCGTTT TATTGGTGTC TGATGGGTGA TCTTACCAGT CCTGCGGGTA 211140
TCTTGCCCTT CCCTGTAAGC ATGGGGTTGT GTCAGTGGGC GGAAGGACCT GGGCGACCAT 211200
CGCCACGCTT CTGCAGACGT CAAAAATGAA CAACGTCGAT CCGCTCGCTT AGCTCACGCA 211260
AAGGCCCCGA TCTTTTCAGT GGCGAGGAGC GATCTAGTTC AGCGCCAACT CTAGCAAAGC 211320
ACGCCTGGGT TCGTATCGGC TATGTGTCGT GAGGTCTCGA GGCGTGGGAT GTGCAGCGGG 211380
TGGCCGTCGC AGTTGGTGAC GTCAAGGAAC TTAACGGATA GATTGTACCT GGTTCCACAA 211440
AACACGGGAT CGCGCTGCCG CGGCTTGAGG CCCAGTGGCC AGGCACGAGT TCAAAGTGAA 211500
GGAACCAACG ATTCCCACCC AGAATTTCCG ACAGCTCGCG CGATGAGTTT TCTGTAAGCG 211560
TCCGGTGAGG CAGCTTCGCG CTGACGGCTC GCCTTTACTT TACCGAAACG GCTCGTGCGA 211620
CAACTTGTAG TTTGATCGCG TCAAAACAGC CAACACCGGC GAATGCCCAG AACAACGAAA 211680
CGGCTACTGG ATAAACTCCG CGAGGTCGAA GCGCTCCGAG GTGACGGTCG CTCACCACCT 211740
CGGGGGATGC TGTCAAGATT CGGAAAGGGT ATGGTGGCCT TTCTGCGGGA ATATGTCCGT 211800

ATTCTCGCGT ATCATCTCTC CGTGGGGCCG TTTCCCCGCC TCAGTAAAAT CATTGCTGCA 211860
GTCGGTTTAG CGGTGTCTGG TCCCGGAGTC CTCTACAAGG TTATCGAGGC GATCATTGCC 211920
GGCGAAGTCC GCAATCGCGG GGCGGTCATT GCAACCGCAA AAGAAGAACC TTTCGAATTC 211980
TACACCATGA CGCTCATTGT TGGCTCGGGG GCTGCCTTCG TTACCGCCTT GGGTGTTGCC 212040
GCCTTCCTGG TCCTGATATT GAAGGGCCGC CCATCGCGCA GCGAGTGATC TATTGCCGGC 212100
GCGACCGATG CTATCGATCT TGATGCCGCA TGTTCCACGG GAGCAGTTCT CCGATCCTGT 212160
TGATCGGATG GTCGGCGCTG CGGGTGAGGA CGTCGCGAAG ATAGGCTTCG GGATCGAGAC 212220
CGTTGAGCTT TGCGGATTGG ATGAGCGACA GGATCGCCGC GGCACGCTCG CCGCCCTTAT 212280
CCGATCCGGC GAACAACCAA TTCTTTCTGT TATGCATATC TATACATAAC AGAAAGTCAT 212340
GGCTCTTTGC CGGCTCTGAT CGAGGTGCCG ATCGCGCCGC CTTCATGGCG ACACTGAGCT 212400
CAACAACGTC GATCCGCAGG CCTGGCTCGC CGACGTCCTC GCCCGCGTCG CCGACACGCC 212460
GATCACTACG CTGGAGCAAT TGCTCCCGTG GAATTGGCAG CCGCCGAAGT TCCTGCGAGA 212520
TCAAGCTGCC TAACCTGCGG CCTTGGCCGG ATGGTTACGT CTCAGCGGCT GCAATCGCCG 212580
CAGGATGGCG CCCTGACGAG GTCGCCTCAA CGTTGGTTGA GGTTGCCGAC CACGACATGT 212640
TCGCGCTGAT CGAAAACCGC GATCTAGCAG CCGATCTTCC CATCCTCCGG TTCCGACCGC 212700
GGTAGCGTCT GTCGCCAAGT GCCGGCAGGC GGCCCGAGCC GTCACTGATT TATGGTGGCG 212760
GCAGCAACAG AGTTGGCTGG CCTTGATCGT CAAAGAATGA AACCGCGTTC GAGGCTCGTT 212820
GCTCTCGCCA GTATCGTTCG GATTGTCGCG GTGCTCCAAC GGTCGCGCCG ACTGTCTGAG 212880
GGCTGGCAAT CCGTTGCGGC CCGGACTCAA TCTGCGGCTT TGAACGCGCG GTATCGCGAA 212940
CTTCCTGCGG AATCGGAATA ACGCGCTGAG ACAGGTGACT AAGGAAAGTG GTCTCGCGTA 213000
TCGGAAGCCC TCGGCTATTC TCTCGCCTTG TCAGTCTGCG TACCTCAACA CTCGGAGCGT 213060
CGTCGCCGGA GAGTTGGAGG GGTTGCCTGC GCAAGAACTC ATCCATAACC AGCCTGGCAC 213120
TGGCACTCAC CGGCGCTCGG GGCAGCTGGA CCTTCGTGTG ACCATACCGG CCGCTGACAC 213180
TGGCAGGCCG CGCGAAAACC AAACGCTCGT TTTGCGATCC GTCGGGCCGG ACGCCGAAAC 213240
CGGGATTGTC TTCCACTCCC ACGACGGTAA GCCACGTCGG TTCGGCAGGG CTTAGGCCCT 213300
TGAACCCGAA ATAGGGCCCG ACGGCGCTGC CAGCGCGAAC ATACGCTTGA AGGGCAAGAT 213360
AACTCCGGTG AAACTTCTTT TTCTCGCGCT CGCCACGATG TACAGAAGAC ATGGCTTGAT 213420
GAAGCAGCGA TGCCCCTATC GAAAAAACGG GAAGCCCGCG GCTGAGTGCA ACGTCTATGC 213480
TTTTCCGGAT TTCGGCTTCT GCCACCCCGT CCGTCATTGT GCCGAGTGCG GCGTCGAGGA 213540
TCGCACCGTC GGCGATCTGA GGGAACCAGT TCATGAGGTT CCGCACCCAA CCTCGGCGCG 213600
CGGCCAGCTT TTTGTTTCGC AGGAGGAAAT AGCCCGCCGC AACGGCAGAT ATAGGATCTC 213660
CGACTTTGTC CTGGAGGAAC CGATCCGCGA TCTGAAGGGT CTCGGCAAGT TGATTGGCCT 213720
CTGCAATCGA TCCATCCGAC AGGTAGGTGA GGATGATTTC GTTGTCCGGA ATGTCGCGCG 213780
CTATTGCTAT GTCGATGGTG TCGCTGTCGT TGCCAGCGCT TCGGGTCAAT GCCACGCGGA 213840
CCCTCTGCCC AGGAGGCAGG GCCACCAACC GCCATCCAAC GTGTTCGCCG CCGACCTGAA 213900
GCAGATGGGG GTTGTTGGGG ACGGGGATCA TGATTTGCTG GACGCCGGTC CTGTCGCTCA 213960
ACCGTTCCTC CACCGGTAGC GGGCTACTTT GCCATCGATC GTCCACCAGC GACCACAGCG 214020
TCATCCAAAC TTTGCCGATC GGGGGAGCCT TGGCAGGGTC GTCGCCAGAC ATCTCGACCA 214080
GATGGTCGAG TGACTGGAAT GGCGTGACCC ACTCCAGCCA ATCCGTTGGA GCCGTCTTGC 214140
CGATATCGAA AGTCGCATCT CCTTTATCAA CTTGCCGATT GGCGATGGTC TGCATCGAGC 214200
GACCGTCGGG GAGCATAGCT ACAACGTGGA GGCGATCGGT TTGATCGCGA CGGCGCTCGT 214260
CAATCGTTAC TTTCGCGGAT CGCTCTGGCG AAGCAATGCC AGAGGCCAGG AGGTCACCCT 214320
TGGACGAGAA CACGTTGAAG GGAAGCGTTA GTAGGTTAGT ATCCTTCTGC GACCCGGAAA 214380
CAATAACCGT TAAATCCATC CGACAATCCC CTCAGTCGAC TTTGATGAGG CGACGAACTG 214440
CAGCGTTCGT CAGTAGTTTT CGATTATAAA TCTTCTCCGG AAAGGACGTG GGATCGGTGG 214500
AATTCGTTCC ATAAGTCCAT TCTCCGTGCT CCCTGACGAA CCGGACCGGG CCGGTTGCGA 214560
GAGGCTTTGT ATCCCTGACG AGAGCGGTGT CTTCCATGAA GGCTTGCGCG ATGGGGCGGA 214620
AACCTTGCGG TAGCACGTCC AATTCAAGCA TCACCGACGG GCGTTTTGAC AAGACGTGGA 214680
GAGGCACGGT CCAGTTGCCG TCACCCTCGG TTTTTCCGAT TTTTTGGCGA TCTGCCATGC 214740
CCTCGGGTTG GAGGAACTCG AGAAATTCCG ATATTGCGGC ACGTAATCTT GCCACGTCGA 214800
CGGAATAGCC GTTGCCACTC GGATGCTCGA CCCCGCAATG TCCTCGAAGC GCTGACAAAA 214860
GGGCCTGTGT AAAGTGGGCA ACTCCTCCGT CGGGCGCGTA GGCCAAGCGG TTCGCTGTTG 214920
TCGCCCGGAC GATTGCCCAG TCGGTCGTTG TCAGCGCGCC TCTCCGCGAC CCGCCGATAA 214980
GCGCCTTCGG ATCCTCAAGC TGGTTTATGA TCTCCTCGCT CAACTCCATG CAAGCGTCGA 215040
TGAAATAGAA GAGGCTCGCC TTGGACTTTC GGAGCGTGGC CTGCACCGTC ATCGTCAACT 215100
GAAAGACGGC ATTCCAAATG TCGGCTTCAT CTTCACCGAA GTCATCTGCG ACCAGATATT 215160
GTGTCTCGCC GTCGCTAATC CCATGGCCGC AGAAATAAAA GACCCCCCGG CTCTGGGGAT 215220
TGAGTTTAAG GCGTTCGAGC CAGGCGACAT AAGCGGTCTT GAGGTTTGCG ATTGACGGGC 215280
GCGTGGTCTT AACCTTGGTG CCGAACGGTG TGTCGTAGGA ACCATTTGGC GATGCGAGCA 215340
TCACGAGTGA GCCGAGAGGG GCATCCGCGT TAGAGAACGC TTCGCTCGGG TCTTTCGCCT 215400
GACCGGCTGG CATCGCATCG GATCCGGTCA GAAACCAGTC AGCGACGGCG ACCACGGAGA 215460
TCGGCGGCGA CGTGAGAGAC GGCAGGCTTC CATATCCCGC AGCCGCAAGG CTCGGATACT 215520
CGCCGCAACC GATCAAGAGG ACATGCGTCG CGGCGACGGT CGGATCGGCT TTCACTGCCG 215580
TGGATTCGAA AACGATGCTC ATCGATATGT CTATTCCGAC CGCCATGCGC CCCGAAACCG 215640
CCATAGCCCG TGTCGTACTG GAAATCCTTG ACGCTCTTGT AAATACCCTC GGCACTGAAC 215700
GAGAAAACGT CATTGGTGTC GAAGACGTTC AGCCACTTTC CGACATTCGG ATTGGCGGCA 215760
ATGCGATCAT TCTTCGGATC AGTCGGCATC CCGGGAGGTA GCGGTCGACT ATAGAGCGCC 215820
ATCTCATCGA ACAAACCGAC CTGTGAACCG ATTGTTGCCA GGTAGTCGAC TTTCAGATCC 215880
GGAGCGAAAT AGGAAAGAAT ATCGTAGGTG ATGACCCCAC CAAGGCTATG ACCGACGATG 215940

ATAAGCTTGT CGTCGCCCTC CTGCCGGGCG GCATCAGCGT TACGGAACGC CTGTAGAACC 216000
GCCCTAACAA TCGGGCCGGG CGCCGCCGCG GTCCCGCGAT TTTTGAGGTA GACGAATACG 216060
TCGCCCAGGA ATCGCGACGC TTTCTGATGC GCGGATTTCC GCCCCAGCGC CAGCAACCCT 216120
GCTGTGGTTG CGTCGGCAGG TGCCGACCCC AGCCTGGCCA AGGCCTCCTT GGTCGAGGCG 216180
AGCCATCCAC TCAAACCGAA CGTTTCGAGC TTTTTCTCGT CTGCCGGCAG TTCCGCAATG 216240
CCTCTCAGCA GTGCATCGAG AAATTCCTCA TTGGATAGCG GCTCGGCTTT CAACGCCCAT 216300
GCCGGCGCGG GATATTTCTC GATGTAACGC TGTGACGCCT GATACGCCGC TGCGATCTTC 216360
TCAATATTCT CGGGAGAATT CGCGGTCGCG GCTGCAGCGT CCCAAATAAG ATCGACGGCA 216420
TCCGCAAACG ATTGGTCGCG GCTCACCTTG CCAAGAATAA CGGACTTTGC GCCAAAGGAC 216480
TGTTGAACCT CATGAAGCCA GAGGTCGATT TCATTTTTCT GCGCTCCCAG CGCCAAGGTC 216540
TCATAGCCGC CACCTGGCAG CGACACCTGG TTCCAGAGGA ACTTGACGCC GTCTCCGCCC 216600
CAATAAGGAA AGCTGATGTG CACCTTGGCC GGGTCGAGCC CGAGCGGCGC GGCAAGCAAC 216660
GTCTTCAAGA ATTCTTTTTT GCGCGCGACG TCGTTGTCAA AGCCAGCATC TTCCTTGCGA 216720
TTGTTGACCC CATGGACGAA CACTATTGGC AAAACGACCT CCCCAACCAA TTCTGATGCC 216780
TTGAATCAGT TGTAGCCGAG GCCGCGTTCG CCCTCAAAGG GAAAATTTTG TGGTTTTTTG 216840
CGTAAGGGGG CAGCTTACCT GTCAACTTGC GGAGTCGCCA AGGCGCTCAT GAGGTCATGG 216900
GAAATCGCGG TGACGACAAA CAATGTTGGA ACGCTTGCGA CTGCGACAGC CACATCGATT 216960
GCGCCGAGCA TCTTCAATAT GTAACCGCGC CTTGCATTGC ACTCGTCCCA TTCGTACACC 217020
ATCGAGAGAT CCTTCACTCG CTGAGCGAAT TGAAGCAAAA ACATGTGCCA AAAGTCGAGT 217080
GCACAAATGA CACTCGCCGG GGGATCAAAC GATCTCGAGT GAGAAATCGT CTAGGATTTG 217140
TGCGACTTCA TCGCGGAAAT CCAGCAACGG TCCGGAAGAC TTCACGTTGA GCTTGGGGAG 217200
CGCCCGATCG AAAAGGGCGG TATTGGCTTC GCCATTTAGG CGGGAGTCGT AGATGATCCC 217260
GTCCGGTTTC TCGTCGTGGA GCCAGAGTGC TCGTGACCAT TGCTTCCCGA GTTCATGCGA 217320
AGCGGCACGG GCAGCATCGG TCGGTATGCG CATGCGCAGC ATTCCGTCGG AGCGCAGGTC 217380
CGCGAGCAGC AGAGGTTGGT TGATTTCGAC CGACGCGCAA GAAACCTCCT GAAGTTCTGC 217440
AATACCGATA GGAAAAGAGC CTTTTGAACC GACGGCGCGT TCGCGCAAGA TCGCTTCGGC 217500
AAAGCAGACC TTTATGCTGC TGCCAAAATA AACAACGCCG AACCGGTCGG GAGGAGACAG 217560
GGTTGCGCGC TTCGCAAAAA CTGGACATTG ATTCCGCTAA ATCCCGGACA GCAATTTCAT 217620
TAAAGTCCGG ACAGTTTGAT GCGGTTGGTC CTCGGCAGCC TGGTTAGCAT CAGGGCTGAT 217680
TGATTTCGCC GTTTTCTGCC CCAGTCAAGA GGGGTGCGCT TTTTTGCTTT CGCATGCTCT 217740
CGCCCCGGAG GGTGATGCGG TGAGCATTGT GGACGATACG GTCGAGTATG GCGTCGGCGA 217800
CCGTGCTGTC GGCAATAAGG TCGTGCCAGC TTGCCACGGG AACCTGAGCT GTGATCAGGG 217860
TGGACTTTCG CTGATAACGC TCCTCGACGA TTTCGAAGAG GTGAAAGCGC TGCTGATCGG 217920
AGAGCGTATG GGTTCCAAAG TCATCGAGGA TGAGGAGCTG GACGCGGGTG AGCCTGTCGA 217980
TGAGGCGGGG GAAGGAGCCG TCGAGGCGGG CAAGCGCGAG CTCCTCGAAC ATTCGGGGCA 218040
CGCGCACATA GAGCACGGAG TGACCGAGCC TGGCCGCTTG CCTGCCGAAG GCACAGGCCA 218100
GCCATGACTT GCCGGTGCCG GTGTGGCCGG TGATGATCAG GCCCTCGTGG GCGGTGAGCC 218160
ATTGCCCCTG GGCGAGCGCC ATGGTGTTGC GCCGGTCGAG ACCGCGGGCG GCGGCGAAGT 218220
CGATATCTTC GATACAGGCC TGGGCAAAGC GTAGCTTGGC GGAGGCGAGC CGGTTACGGA 218280
TGCGCTTGTC GGCGCGCAGG GTGACTTCGC GGTCGAGCAT CAGTCCGAGC CACTCATCGC 218340
GGCTGAGCTC ATTGGTGCCG GACTGCTCGG TCAATTCGCG CCAGGCGGCG GCCATGCCCG 218400
TCAGCCCGAG GGCCTGCATC TGGTCGAGGG TGGGGTTCGT CAGCATTCTT GCTTCCTTCA 218460
CTGGTAGTAG GATCGGCCAC GGATATTGGT ATGCGCAGGC GTGGGGGCCG CGTGTTCAGC 218520
CTGCGGTCTT TCCCGGTCGA GGCCGGATTT GAGGATGGAG GCGACGGAGG AATAGGTGAT 218580
GGCATTAATG GTGAGCGCCC GCTCACAGGC CGCCTCGAGG CGCTGGGATC CATAGCGCGG 218640
CGCCAGCGAC AGAATGCCCA TGGCCGAGCG GTATCCCTGT TCCGGATGCG GCCTGTCGCG 218700
CATCATGCGT TCGACCAGGA TGGCGGCATT GGGGCCGATC TGGGTCGCAC GGCCGATCAG 218760
ATTGGCCGGC GTGGTGTTGG CATAGCGCTG ATGCGCCTTG GGCATATGGT CGTTGACGGT 218820
GACGTGGCCG GAACGCTGGG AGCGGCGAAC ATGGCTGGCG ACACGCTGGT GGTCGTGGAA 218880
GATCTCGACC ACCCGGTGGG TGAGCCGCAC CTGGAGGGTG CATCCGATCA GCCGATGCGG 218940
CACCGAGTAG AAGGTCTTGT CGACCTCGAC ATGATAGTCC GGATGGACCT TCGCCGACTT 219000
CCATTCCGCA TATTCGAACG GTATCGCCGG CAAGGGCTTC AAGGCTGGCC GCTCGATCTC 219060
CTCGAACAGC TCGCGGCGGC TTTTGCCGAC ATGGCGCATC GTCCGGTTGT TCAGGTCCTC 219120
GAGCAATTCG GCAATCGCCG TGTTGAGGGC GGCAAGCGAG AAGAAGGTAC GGTTCCTGAG 219180
CCGGGCCAGA ATCCAGCGTT CCACGATCAA TACCGCGCCT TCGACCCGGC CTTTGTCGCG 219240
CGGTTTCCTG CTGCGGGTCG GCAGGATCGT GGTGTCGTAA TGCTCCGCCA TGGCGGCGAA 219300
CGTCGCAGTC AATGTCGGCT CGAACCAAAG GGCCTTGGCC ACCCCCGATT TGAGGTTGTC 219360
GCACACGATT GCCTTGGTGA CCCCACCATA GAAGGTCAGA GCACGCACCT GACCGTCGAT 219420
CCAATCCGGC AACTGCTGGC TAAAGCTGGC ATGGGCGAAG GTCAGGTTGG AGGCGCCCAG 219480
CACCGCGACA AAGATTTGGG CCGGATGGAT GACACCGGTT GCCGGATCGA TCACCGGCAC 219540
CGTCGGCCCG GCATAGTCGG TCTGCATCAC GGCGCCCGCC GCGTGCCGAT TGCGGAACGC 219600
TACACTGGTG CGCTGCCGAA AGGCGGCAAC CTGCTCGCAG AACCAGGTGA AGCCGTAACC 219660
ATCAGGATGG CTGGCGCGGT ATTCCTGCCA TAGAAGCGTC AGCGTCACGC CTTTGCGCTT 219720
CAGCTCCCGA ACCACCAGCG CCCAGTCCGG CTCGCTGAGA TCGCGCGGCG GTCGACCGGC 219780
TCGGCCGAAC AGCCGCCGCT CCAGCTTTGC ATCCTCGTCC GCGCCGATTG GCAACGGCCA 219840
CGAAAGCCCG GCTTCCCGAG ATCGCAGCAA ATAGGTCGAT ACCGAGCTCT TGCCGATCTT 219900
CAGCCGCTCG GCAATCTCGC GCACCGAAAG ACCCTCTTCA TGGGTCAGGC GCAGAATAGT 219960
CCGGATATCC CTCACTGTCG TTCGTCTTGC TTGCTTCCGT CTCGGCATCG GCCCCTCTCA 220020
ACGTTGTCGT GAGAGGCCTA ACCAACAAGA CGGCGCAGCC GCGAACCAAC CCGTCAAACC 220080

GCCGCCGGAA ACTGTCCGGG ATTTAGCGGA ATCGCTGTCC GGGAATTACT GAAAACTGTG 220140
TCCGGAGATT ACCGGAAATC CTGTCCGGAC TTTGCCGAAA CCCGCAGCCG GCAATGATCC 220200
CATCAAGGAA GGGATGAGCG TTGAGGCCAC TATCTATAAT GCCTATAACG GGCGCCGTCT 220260
GGTCGACCGT ATTGAGGGGA GGGGCCTCAG CAAGGACCAT GTCCATCGCT TCGGCCGTGA 220320
CAATGCCAGG CGTTGGCGGA AGGTCCACAG ATGCTACTTC CTCGATCGTC AACAAAGTCC 220380
GGACCAACAC GCCGTTCAGC CGGCCGCGGA GCATTGTGAT AGAAGGACCG ACATACTGGT 220440
CGAAAACCTC AGCCCCACGC GCCTCGATGT AGCGAACGAT GTCGTCGAGC TTGCGCTCGC 220500
GAAGACGTCG CTCACCGAGA TCCCATATCT CAATATCAAT GAGATAGGCT TGTCCATCTT 220560
GGAAATCGGC CATTTCAACA AGGCCGTCTT CGCGGAAACG AATCCCAATG CGGTCACGTG 220620
GCTCGACCGA CCCAATGGTT TCGATGCCGC CTATGAAATT GTTGTAGGGG GCGTTCTTTT 220680
GGCCCGCGGG CGCGCCGCGT TGGTAGGCAT CCAGCCGCGT TCTGAACTCT GCCATCTCGT 220740
CGTTGGATGC GAACAACACT AGCGTTCGGT CGGCGTCGCT GGATAGGACC GTAAGTCCGA 220800
GTTGCTCCCA GTCTGCTTCC TGCAGTGCCC CCGTCATCTG GACGCGGAGG ATCAGCGCTG 220860
GATTTACGAA TTCCGGCTTG CGTCTCCGGC GTTGCTCGTC TCGGGCAGCG TTTAGCTCGT 220920
CTCTGAGCTT TGCACTGTGC CGGGGACCAT CTCTGTCAGG CGGCGGACTA CCTCCGCCGT 220980
GCTTGCGTCG CTCAAGCTGT TCTGGAAGTC TGACGAGCTC GAGATGGTCG TACTTGGCCA 221040
CGGCGGCCTC AGATCATTGG TGCAAGACGG GCTTGTCCGG CACGCCGCCG CGCCTCGTCC 221100
TGAAGTGCAC GGTTGAAATC CCGCTCCTGG ACTTGCTTGC GACGCTCTAT GATCATTGCT 221160
TTGATCGCCT GAGTACAGAC ACGTTCAATT TCGGCGAAGG AATAGCCCTC GAGTGCAGCC 221220
GAATTTCGGG CCGGGTCGAA CGCGACGGGG ACATTCTTGA ACTTGAGCTT GAGGAAACGA 221280
CCGATCATCG CGGCTTCGGG CCTGTCAAAC CAAAGGACCT CATCGAAACG GCGCCAGATA 221340
GCCGCGTCGA GAGAGCGGTC GAGGTTCGTC GCTGCGATCA GAAAGCCCTT CGGCTGCATG 221400
TTGTCGATAA AGAGCAGCAG GCTGTTAACG ACGCGACGAA GCTCGTTGTG CTCGCCACTG 221460
TCGTCTCGGG TCCTCGCGAG GGCATCAAAC TCGTCGAAAA ACAGGACGCA CGGCTGCTTC 221520
CGTGCGAACT CGAATGTCTT TCGGATATTT GTCGCCGTCT CACCAAGGTA GGAAGAAATG 221580
AGGCGATCAA GCTTCACCCG GAACAGTGGG AGTCCCAGTT CGGCGGCGAA AATTTCCGCG 221640
CAAAGCGTCT TGCCGCAGCC AGGCGGTCCG CAGAAAAGCA TTTTCGAGCG GACCTTGAGA 221700
CCGTGCTGGC GGATCTCATC TGCCCGACGA AATTCTTTTA CCAGGCCAAG TAGCACCCTC 221760
ACGTTGGCGG CGCTCAGCAC GATGTCGTTG CGATTATGTT CGGGTTCAAT ACGCTCGACG 221820
AAATCTGCGG CAGCTTCGGG GAATGGGATG AGGGGGGCGA GAGCTTTCGG CGCAGACTGT 221880
GTCCGCTGAG GACCAGCCTC AAGCGTTTTC CGCAATGTCC GTGCCAAGAC GCGGTTGTTT 221940
TTTTTCTCTT CCTCGTCGAT GATCTGCTCG GCAACGGCGC GAAAATCCTC ATCGCGCCCG 222000
TAGCTAGCCA GCAATTTTTT CATCAACTCG CCGCGTGCCA TCTGTCGTCC GTCTATTCCC 222060
TTATGCTCGA ATCACAGCTC GACATTATCC TGTCAGGATG ATGCTACCAC ATGATTTAGA 222120
CAATAACCTT GGTACGGATT TAGTTTTGTC GCCAGCGAAT TCAGCTTGTG CGAAGGGATT 222180
GTCCACGTCG TTCAACAAGG TCGAGGGCAG CTTTTCGGTT TTTCGTAACC GGCTCGATTT 222240
GATGAGCGAA ACGCCCGGAG GGGGCGCAGT CGCTCCCGCG GCTCCTTCGG GCCACTATAT 222300
TGCCGCTCCT GCGGCTGCTT TTTTCCCAGC CTTTGTGTGA CGCGAATTCC CGCTGTCGCT 222360
TGGCAGTTAA ACGTGAGAAT GCGGCTGTTT CGGCCAGGTT TGGCATTTGA AGATGAGAAT 222420
CCGACCGACC GGATTCTCAA ATTAGCTGCA ACTGCGAAGA CGTCATCGAT CGGCATGTAT 222480
GGGTCATGAT CTCGTCGATA GCGTTGCTGA ATCGCGCCTT GTTCCCACCC ATCATTTGGC 222540
CACGCAGCAT TTGGAGCATC TCGGGTCCGG CCCGTTCGAC GATGGCCACT CCGGCCAGCA 222600
GAGCTGGTCG TAGATGCAGC GGTAGGATCG GGCCTCGAGA GTGCCTTCGT CTGGGAGCGG 222660
GAAGGCGATT GGCCAGCTCT TTGAGAGCAT GAGCGACAAC TCTTTCACGT GCACTGTGGT 222720
AAATCGTGGC TACAAAGGCG GCCGTGATGC GCACATCACG GTTCACAACA GCAAGGGCAG 222780
CCGTCATCAC TTCGGCGACA TCAACTACTC ATGGCAATCC CATAGCGAGT CAAACACGTC 222840
AAACGGTCAC GTCAAAGTCG ACGCAGACGA GTACAACATG TTCCTCTCGC TGAACGATTT 222900
CATGAGCAGC GAGAAAAAAC GCTATGACGC GAAACAGGTC GCCGACGTCC TCTGGCTCGA 222960
ATTCACCAAT CAGGCAGGGA TCGAATATGA CTGAGGTGAA GCGTTTCCAA TGTCTAAACT 223020
GCGGTCATCG CTTCGAGATT GACGTGTTGA CGCCGGATGA GAAAAAGGAG GCAAGGAGGA 223080
AAGACGAGCC GGTCTACCCA GTGGCTTGCC CGCAATGCCG GCGGACCGAT GTAAGGGGTG 223140
GCTGGGGCTA GGCGCCGTTC AGGCCTCGCC GATAAACTGG ATTGGGAGGT ATAGCTTTGG 223200
AACCCCCGAG GAGGAGGTCT CATTGTCGAA CGATGCGGAA CCTGAAGGAA TTGGTGCTAT 223260
CGACCAGCTC TACGACATCT TAGAGATGCT CGAAGAAGAT ATAAAGGATC TAGAGGGAGC 223320
GAGGCGAGAG CGCGTAAACG TCTTCTTGTA CCAGGGCATC TTTGAGGCGA TCAGTTTTTT 223380
GGACGAACTC GGGATGGCTG GTCGGATAGG TCTATCTGCG ATGGAACTCG ATAAATTCCA 223440
ACGCATGTCT GGCACAATCG ACAACGCGGT CCTTCGTCAA ATGACCGGAC GCTTGAAGAG 223500
TTTATTGCGG GAGGCAAACT GCCTCTATGA CCGATAATGG GGGCGCAATG CTGCCGTTCA 223560
CAGACTTAAG GGACTTAAGA GTGGACGTCG CCTTCGGGGC GGACAGGATC CTGCTCCCAC 223620
GAACCCTGCA CGGCTTGTTG CTGTGATGTT CACCTTTTGG GCCAGGAAGA TCGCATAATG 223680
TATCGACTGG CACTTGCCCA GCTTCCCCGG GGCTGGCCGC TCTTTTTGAA TAACTTAATG 223740
TTAGCCGTTT GGCGATCAGT AAGCTGGTAT GGTGGGCCCG GCGGCAACCA GTCGGTCATG 223800
ACGCCGACGG GGCGCTAACC AATTAACTAC AGGTCCCACC AGGACGTTTC AAGCACGGAA 223860
AAACCGATGG ATCAGTCGCG TAGCGGGATG CTTTGAAGAA GTGTTTTGAA CCTTGACGCA 223920
CCAGTGCGGT TCATCGATAA TCAAAGTGAA CCAAGGAAGG TTAGATCCCA TGACCGCTGC 223980
GTTTACCGTG CGTGTAAAAG ATGAAACCGC GAGCAAGCTG GACCAGCTCG CTGAGAAGCT 224040
GGATCGGTCC CGCTCTTATA TGGCTGCAGA GGCCATCGAA GCTTTCGTTG AGCAACAGGA 224100
ATGGCAGCTT GCCGAGATTG AAGCCGGGTT GACCGAGGCC GACCGGGGTG AGTTCGCCAG 224160
CGATGAAGAT GTGGCGAAAG TCGTCAGGAA GTACGTCAAG TCTGCCCGCC AATCATGAGT 224220

CGGAAGCGTA TTCGTTGGAC GCTGCGGGCG TTGCGGCGAC TTGATGAGAT CGGCGCGTAT 224280
ATCGAGAAAG ACGATCCGGA TGCGGCCGCT CGCGTCGTGG CGCGGATTGG CACTGCCGTG 224340
GATGCGTTAG CGGAGTACCC TGCCAGTGGG CGGCCTGGTC GTATCAAGAG CACGCGCGAG 224400
CTCGTGCTGG CCGACATTCC CTACATCATT CCCTACCGCA TAAGTCGAGA CGTCGAGATC 224460
CTCACCGTCA TGCACGCTCA CCAGCAATGG CCGCAGGTTC TCTAGTCACG ACCAACAAAA 224520
CGCGTCGGCA CGATGAGCGA TGAATCCGCT CATCAGCCTG CCCGTTTATG TAGAAGCAAT 224580
TTCTGCGGCG GCGAGCGCAG TGGCTGCCTT TTTAGTGATC GCTTATGCCG TGTACCAAGT 224640
GTCGGAAAGC AAGCGTGTGT GTCGACGCCA GCATAACAAT GTCTTTGATC GGTCATGCCT 224700
TCTCCCCGTT TGAGCCTACG AGCCACCGCG ATCTTCGCCG ATCCCTGTAC TGGCGCTCGG 224760
AAAAGGCACA GGTCCGGCGC GAACTCCCCA CGGGGCATCG ATCACGGCCA ATCCGACGGG 224820
GCACGCGTCC CCCAGGTGCT CGAAGCACGG GGCGATGGGT CGTTCCCGAC TAGACTAATC 224880
GGCTCGGTTC GGGAAGGCTA CGCCAATTCG GGCCGTCGCC CATGGTGCAG AGAAGTTCCG 224940
CTACGCTTCA CGTGGCCACA CACTGCGAGC ATCCCAAGAA CCGCATGAGA AGGGTGGGGT 225000
CGGCGGTTAT GGTGTGGTCG CGACGCTCCG GCTGGGGCAG GGGGCAAGCG CATCGGTATC 225060
CGTGCGGATA TCGGTACGCA GCCGATCGAA GAGGCACCAT CCTGGTCTTC TCGAGCCGAA 225120
ATCCGGGCGC CATGCACGCC TAGGGCCATG ACGGGCACAC GACGATCCTG TTCGCCGCGG 225180
CCCGCCACCT CGCCGAGCAC GGCAAGTTCA ACGGCACGCT GACGCTGATC CTCCAGCCGG 225240
CCGAAGAAGT CGGCGCCGGT GCCCGCAAGA TAATCTAGTA CCGGCTGTTC GAGCCGTTTC 225300
CGGTAGATGC AGTCTCCGGC CTGCACAACT GGCCTGGCGT CCCGTCAGGA CAGTTCGGTT 225360
TCGTCGAGGG GCCGGCGATG GCGTCAGCGG ACCAGGCGCT GATCCGCATC GTCGGACGGG 225420
GCGGGCAAGG TGCGGCACCT CATGAGGCGG TCGATCCGGT GCTTGCGCCT CCTTAATCAC 225480
CGTCATGGCT GAGCCGGAAG GGGCGCCACT GGGATATCGC GTTTCGCCTC ACCTCTAATC 225540
GCCGTCGGAT GAAAGTTCGA AACGGATACA CTCCCGGCCG GCGGCGACCG TGGCCGGTGC 225600
CGTTCGCTCA TCCAGCACGA TGTCGAGATG GTCGTCCTTG CGCTGGCTCA TGGCGGTGTC 225660
TCTCATGCTC GCTCCATCCG TCGCATCGGT CGACGGCCTT GCGTCGGATC GGACCGAGGC 225720
AGCGCGCGCA CGCCGCCGCC TGCCGCGCGT TCAGGCGGGC GCATGCTGGC CTCCCCCCGC 225780
AGCGTCGCTG CGGTAGCGGC TGGTCGAGGT CTGGTAATAG TGCGCGCGGA TGGCCGCCAT 225840
GGCCTCGATC AACGGCCCCC ACGGCGCGGG AAACCGTTCT TCCGCCATAA CCCGGTGCAG 225900
CATGCGCGTA TGGCCGGCCA GTTCGTCGTT GAGGAACTCC ACCACAGGCA TAGCCGGATA 225960
GCGCTCCAGC AGCAGGATCG CCGCGTTGTC GGCCTCGCCG GCCGACCTGT CCTTGTCGCG 226020
TCCATGCAGA TCGTTCTGCA GGCGCCCTAT CGCCGAGATG AGCCGCAGGA CCTGGCGAAA 226080
CGCCGGACGC GCGCGCAAGG TCGCCATGTC CAGCCCCCAC AGCAACGACA GGCAACAGAA 226140
CACGTTTGCG TAGGCGATCG AATCGATGCC GTTGTGCAGG TACTCGGCGT AGGACCAGCG 226200
TTCCGCCGCC CCTGCCGCCT GCGCGTGTCC GGCGCGCAGC GCCGCGCAGT AGCACCGGGT 226260
ATCGTCGAGA AGCTGAGCAT AGTCGCGACG ATCGTAGGCG AGGGCGGCCA GCGAAGCGCG 226320
CAGCACAGCG CAGCCCTCGA ATCCGGGGAG CGCGCACGGC ACGCCCTGCC CCAGCGCCTG 226380
CTCCACCGCG GCGAGCTGCT CCGGCGCGAT CAGGTCAAGG TCGTTGCAAT CGTCGAGCCA 226440
GAACAGCAGC GCCAGTTCGC GATAGAATGC CACGATCAGC GTTTCGTCCT GCGGATCGCG 226500
ACCGGTGCGG GCGCTGGTGT CGCGCAGGCT CGGGTGGATG CGCTGCAGGA TGTACTGGCC 226560
GCCCCTGACG GCTTCCGCGG CATGCTCGTC GGCGAACCCG GTCAGGGAAC GCCCCCACTC 226620
CAGCACCTGC TGCAGCCCGC GTTCGGTCGG GATCATGGCG CCGCTCCTGC TCCCTCGGCC 226680
GGGACGCGCG GCCCCCAACG AAGCGCCAGC CACAACCCGG CGAGTTCGGC CACGCGCACG 226740
ACCCGGATGG GGCAATACAG TTCCTTGCCG ATCCACAGCG GCATCTGCGG CAATGCAGGC 226800
GCCGCATGGC GGGCGAGCAT CCACTCCAGC GCACGCGCCA CCGCCTGCGC GATGCGCCGG 226860
CGCCCTGTCG GCTCTTCGCT CCCGTCCATC ACGTGCAACG CGAACAGCGC ATAGGCGGTT 226920
TCCTCGAATG TGGACGCGCG ACCGGCGCCC CAGCCGCCGT CGTCGCGCTG CGCCTGCAGC 226980
AGCGCCGCCA GCGCGCGCTC GTCGCGCCAC TGGGGCTTGC CTTGCGCCAG CGCAGCGACG 227040
GCATGCGCGG TGGGATACAG CCACGAAACG TGCCATTTTT CATTGTCCCA TAGACCGTGA 227100
GGGTTGCGAT TGGCCTCGAC GTAGGCGCTG GTGCCAGCGG CAGGCTTTCC CAATAGTCGC 227160
AACGCATGCA GGGCATGGAT GTTGGTCGAC ACCGAGGCGT TGCGCTCGCC GGGGAAGGTG 227220
ACGAACAGCT CGCCGATTTC GAAATGGCGC AACGCATCGA CCGCCGGGTC GCGGCCTGCA 227280
AGGCGCAGGA CGCACAAGGC AACGGCGGTG TCGTCCGCAT CAGCCGCGAA GTGCAAGGCC 227340
GGGCCCAGAC CGCGCACGCC CAGGCGGGCG TCGAGCTGCG CGACGATCAC GCGCACCGCC 227400
TCGTCGAGCG CGGGATGCGC GAACAGCCCG GCCAAATGTA GAGTGTACAG CGACCAGCAT 227460
GGCTCGAACA CATTGATCGG CCAGACGTTG GGAACGACAC CTTCGATGCC GCTGCGCGTC 227520
GCCCGCGATG CCGCCTGCAG ATACGCGTCG GCGCGCCCGA CCTGCGGCGT GCTCCCCTGT 227580
GTCACGGCGT GCGCACGCCA CGCAGCGGTG GCCGCCGGAC TGATGCCGAT GCTGCCGTCG 227640
TCATCCGGGC ATGCGGCGGT TGGCCACGTC CCCCAGGCCT CCCAGGAGTG CAGCAACGGA 227700
TGGCCGCTCG GCAACGTCGC CACCGCCCCC AGCTTGACCA AGCACGCTTG CCGCAACGGC 227760
AACAGCGCGG GGTGGCGCGG AAACGCCACG CCGCCCAACA AGGATGCGGC CTCGCCGCAC 227820
AACTGCGGCA GGATCAGCTC CGCGCCGATC GGCGCGTCTT CCGGCACCGC ATGCGCGTAG 227880
GGATCGGCCT GGCGCTCGAG GAACCGGGTT GCAGCCTGGA CTGCGTCGGC AGCGCCGGGA 227940
AGAGGATCGG CACGCTGCAA TGCCAGCAAC GCCGCCCACG TGGGCGCATG GCGGAACAGC 228000
GGGAAGTCCG CGCTTCCCCA TCCGCCATCG GCCTGTTGCT GCGCGAGGAG CCACGCGTAT 228060
GCGTCCTGCC GACCGGTGAC GTTGCCGCCA AACTGCAGAG CCCGCGCCGT GTCGTAGACG 228120
GACGGACCGA CGCTGCCGCC GTCGCTCATC TCGCTCAGCA GGTGGCGCAA TTCGAAAAGG 228180
ATCTGTTCGG ACAGCGCATT CACGGGGGAT GTTCCTTCTG CAGACGGTTG ACGAGAACCA 228240
GGATCGGGCA GACGGCGCGC ACGTCGCCGC AGGCCCGTTG CGGCCCGCCG CATGGGCAGC 228300
GCCGGACGGC CGCTCTGCTC CGGCGCGGCG ATGAGCCCCG ATGCGGGCGC CGGTTCTCCG 228360

CCGCATGGGC TTGCGCGCAG CGTGCCGCGT GCGCCGTGAC CGTGCTGGGC AACCGCGGCG 228420
ATCGGCGCCG CGGACTCGGA CACAGTGCTG CATGCATGGC TGCCGCCGGC CGATCGCAGC 228480
GGCACAGGGG CGACTCCATG CGGACGGGCG CGCTCATGCG GATGGCGCGT GCTTGAACAG 228540
GTACGCACTG GCCCGCTGCA GCATCTGCGC CAACCGTTCC GCACGCGGCC CCAGCGGGGC 228600
AATGGCCTCC CCGGCCTCAC ACAACAGATC CAGCGCGAAC TGGCGCGCTG CCTGCAGCCC 228660
CATGATCGAC GCGCAGGTCG GTTTCTGCGC CGCCGCGTCC TTGCCGGGGG TCTTGCCCAG 228720
CGTCGCGGTA TCCGCTGTCG CGTCGAGAAT GTCGTCGACC ACCTGCAACG CCAGGCCGAA 228780
ACAGGCGCTG TAGTGATCGA GCGCACAGTA CAGCGTAGCG TCCGCGGCAT CCTCCGCGAT 228840
GGCGCATAGG GCGCCCATGC GAACGGACGC ACGCACTAGC GCTCCGCACT TCATCCGGTG 228900
CATCGCTACG ATCCTGTCCA GCTCGACGTG CTTTCCGACC AGCGACAGAT CCATGGCCTG 228960
CCCGCCTGCG GCACCCTCGG CAGACACCGC CTGCGCCAGT TCGCGCACGA GCGCGATACG 229020
GTTGTCGCCC GGCGCATCCA GGCTTGCCAG GGTCAGGAAG GCGTGTGCCT GCAGCGCATC 229080
GCCGACCAGG ATCGCAGTGG CTTCGCCGAA CTTGACATGC ACGGTCGGAA GGCCGCGGCG 229140
AAGCACGTCG TCGTCCATTG CGGGCAGGTC GTCGTGGACC AGGGTACAAG CGTGCATCAT 229200
CTCGATGGCG GCGCCGACGT CGTCGAGTAT GTGCGCCGGC GTGTCGGCCA GTGCTCCAGC 229260
AGCCAGACAG AGCAGGGCGC GGGTGCGCTT CCCGCCATGC AAGGTGGCGT AGCGCATCGC 229320
CGCCATAAGC TCGGTCTCAC CGTGGTCTTC GGCGCAGAGA AGACGCGCTA GCGCCTGTTC 229380
GACCCGCTTT GCGCCGTTCT GCATCCAGAT CTCCGGCAGC AGCCTGCCGG ATGCGCCGCG 229440
CGCCCGCGCG CCCAATCCGC CGAGGGCGGA AACGCTAGTT CGGTCGTCGT GTAGCGTGGA 229500
ACCGGTCTGC ATGTTGTTCA TGTCCTTGTA CCTGACAGGA GACGGCCACG GCGAGCGTCG 229560
AGCCGCCGCG GTGTTGCCGC GTCGCACCGA ACGCGCGCGC CGAATGCAGC AATGCCGCGC 229620
GTCGCCGGTG CCGCTGCCTC GCCACACGGG GCATGCGATG CCAGCTCATG AGAATCCGAT 229680
GCGGATTGTC ATGGACGGAT GTGCGGTCGG GTAATAGCGC CGGCCTTTTT CGACGCCGCT 229740
CAGCAACAGC GGCCGCACCC CGGCCTTGTG CATGGTCAGC GCCAAGGCGA TGGAGAACTG 229800
CACCAGTTCC AGCCATACCA GGTGATAGCC GATGCAAACG TGTGGCCGGG TACCGAACTG 229860
CAGCATGTCC ACCGGCCGAA TCGGCTCCGT ACGTTGCAGC CACCGCGCCA GGCGGAACTG 229920
ATCAGGCGCC TCGTGCAGCA GCGCCGAGGT CGAGAAATGC AGCAGCGGGA TGCACAGATG 229980
GGTGCCCGCC GGAATGCGCC GTTGGCCGAG TTGCAGTTCC TGCAGCGCGC GACGCGGCAG 230040
GAGCGTCGTC GCCGGATGCA CGCGCAGCGT CTCGCGGAAC AGCGCCTCGG CGACCGGACA 230100
CTGCTCCAGG TCCGCGTGCC GGGTCGGCAC CGCGCCTACG CGTTGTGCCT CCTCGACCAG 230160
GGCATCCCAC AGCATAGGCT GCCGCGCCAG TTCGATCACC ATCCAGGCCA TCGTCGAGGC 230220
GGTGGTGTCG TGACCGGCAA GCAGCAGCAA GCGGATATTG GCGACCAGGA GGTCATCGGA 230280
GAGCGCATCG TCGCTGCGGT CGAAGGCACT CACCATGTCG TTGATCAACC CGGTGCGCGC 230340
GGCATGCGCG CGTGCGTCGC GGACGAACTG GCGCAACTGC GCGTCGATCC AGTCGCGGGC 230400
GGCGCGGCCG CGCCGCAAGG GCAGTCCAGG CAGGTCGACC GAGGGCGCGA CGATCAACTG 230460
CAGTAGTTGC CGGTACTTGC GATGCCATCC CGGCAGGTCC TGCGCAGGGA CTCCCATGAG 230520
GGTGAAGATG AGCTTGAGCA TCAGGTCGCC GGTTTCGGGC AGGATGGTTA CGTCGCCGCG 230580
GTCGCGCCAC GCCTGCACCC GCGCCCGGAT AACGGGCGCG AACAGGTCGC CGATGCCGGC 230640
CTGGGTCAGC CCCTCGGGCA GGAACGCCGC CTTAATCGCA TCGCGCGCCT GCCGGTGCGC 230700
GCCGCCGTCC TGGGCGACCA ACGTTCCGCC AAGCAATTCG GGCGCGATCT CTTCGATCAG 230760
CGCCGAGGAC ACGTCCTTGT GCCGGAGCAG TGCGAACGCA TGCGGATCCA CGCAGGTCAT 230820
CAGGTGTCCG GCAGGGCCGA AATCCAGCCA GAAGTGGCTG CCCAGCGTCC GTTCCGCGCG 230880
CCGCAGCAGG CGCGGCAGGT CGCAGACGAT GGCGGGAAGA TGCCCGACCA GGGGGAAAGC 230940
GCCGGGCATG ACGGGGATGT CGTACCGCAG CCGGTGCCGA CGGTTTAGGG GGTTGAGCAG 231000
CACGTTCATC AGCAGCGCGC CGTTTCGGCG GTGTCGCCCG CAGTCCGCCC GGCGCAGCTG 231060
TTGCCACCGT CGGCGTACGT CGGCATATGC GCCAGCATGC CGCCGTCGAT GCACACGACC 231120
TGGCCGGTGA TGAACGAAGC ATCGTCGGAG AGCAGGAACG CCACCAGCGC GGCCACGTCC 231180
TCGGGGTGGC CGACGCGCGG CAGGAGCTGG TGCCGGCTGA GATGCCGTTG CATGCACTTG 231240
TCCAGCTTGG CCAGGAGACG CTCGGTCATG ATGAGACCCG GCGCAACCGC GTTGCAGCGG 231300
ATCTGCGCAT GGCCGTACTG GGTGGCGAGC GAGGCCGACA GCATGTTCAT CGCCGCCTTC 231360
GACACGGCGT ATGACGTCTG CGCGGTGTCA CCGCTGAGCC CCTGGCACGA CGACATGTTG 231420
ACGATCGCGC CACCGCCGCG GGCGATCATC CGTGGGATGG CCTGTCGGCA GCAGAGCAGC 231480
GTGCCGCGCA GATTGGTCGC CATGGTCTGA TCCCAGACCG CCAGGTCCAG GTCGAGGATC 231540
GCGCGGTCGC GCGGGGTCAG ATGCATGGCG CTCGCGTTGT TCACCAGCAG GTCGACCCCA 231600
CCGAAGTGCC GCTCCGCCGT CTCGAACAGC GCTGCCACTG CCTGCGCATC GGCGATGTCC 231660
ATGGCCATGG CCAGCGCATT GCCCGCTTCG GCCGCGATCT GCGCGGTGCA GGCGATGGCC 231720
GCCGAGCCAT CAAGGTCGGC CACCACCACT CTGCCGCCCT CGCGCGCGAT GGCGAGGGCA 231780
CATGCCTTGC CGATGCCGGC GCCGGCGCCG GTCACCACGG CCACCTTGCC TTCAAACCGT 231840
TCCATCGTGT CCTCCTGGAA TGCGTTGGTC TTTCGCCGCA TGCCGCTCGG CCTCCGCCGG 231900
AGAACGCGCA GGGTCGGCGC TGGCGCGCTC GTCATCGCCA GCGTCGCTTT CGATGACCCG 231960
AATGGCGGCG ACCGGGCACT GGCTGGCCGC GAGCCGCACG GCGGCGTGCA GCGCCTGCGG 232020
TACCGTCGCC ACGCACACTT CGGCCACGCC GTCCGGTTCG CGCTGGCGAA AGGTGCCCGG 232080
CAGCGTCAGC ACGCACTGCC CAGTGGTTCC GCACAGATCT TGGTCAATCA CGACGCGCAT 232140
CTCAGCCCTT GTACCCTTTT CATGCGGTTC TACGGTTGCG CCCTGCGATC AGTCGCCGAC 232200
CGCCTCTCGC CGTCGCCATG CTCAAAACTG GCACCACCTT CGACCCCTCT CTGGCCGTTC 232260
AAAAATGCGC TTGTTAAACG GGGGGAGTCC CCCTGAGCAT GCAGCCGCAC CGGCAGCGCG 232320
CGGAACGTCC TAAGGAACGC GGAGGGCTCC CGGGTCGGCT GCTCGGCCAC TGTCAGCGTG 232380
GGGAAGCGCG CCTGGATTCG CGGCAGGCTC TCGGCCAACT GCACCCGGGC CAGTTGCGCA 232440
CCGAGGCAGA AGTGGATGCC GTGGCCGAAG CTAAGCATGA TCTTCCCGTC GGTCGACATG 232500

CCAGGACTGG TGCCGTAGAA CCGAGCGGGA TCGAAGCGGT CGGGATCGGC GAAGGCGTCC 232560
GGGTCGCGAT TGCCGGCCGC GATCAGCACG CGCACGTCCG CGTTCTTCGG GATCACCACG 232620
CCGCTCAGTT CGATGTCGCT CTGGGCAATA CGCGGAATGG AGCTGAACAT GGCGGGGGCG 232680
TCGCAGCGCA GGACCTCTTC GACGAATGCC TCCACCCCCG CGGCGTCTCC CTGCAGCCAG 232740
TGCCGCTGGT CGGGATACGC CAGCATCGCC AGGACGGCAT GGTCGATGGT CGCAGCAGTG 232800
GTGGCGAAGC CGCCCAGCAG CATGCCCCAC AGCATGCTGA TCAACTCCGC ATCCGATAGC 232860
GTGTCGGCAT CGTCGTCGTG TGCGCCGACC AGCGTGGCCA CAATGTCGTG GCGGGGATCG 232920
ATGCACTTGC GCTGTATCAG GTCGCCGAAG TAGGCCTTCA TCCTGGCGCT GGCCGCGTCT 232980
GCCGCGGCGA GCTGGGGATC GCTGGCGTGC GGGCTCAGGC CTTCCAGAAT GGCGCCGATG 233040
CCGGCGGCGA GGCCGAACAT GTCGTCCTGT GGCATGCCGA ACAGTTCGGC GAAGACCAGC 233100
ATGGGCAAGG CCAGCGCGAA TTGCCGATGC AGGTCCACCG CCTCCCCGCG CTCCAGCGCG 233160
GGCGCCATGC CGTCCAGGCG CGCTGCGACG AAGCGCGCGA TGCTCGGCCG CAGGTTGTCG 233220
ATCTCGCGCA TGGTGAAATC GCGCGAGATC AGCCGGCGCA GACGCGTATG CGTCGGTGGT 233280
TCCTTCATCG CTAGCGTGGA CGCCAGCAGA TTGAGCGACA GGCTGGTCGC CGCACGCGGG 233340
AAATAGCGCG CCAGTTCGCC CGGCGCCGGT CCCCGAAACG CGTCGCCCGT GGCCTTGAGC 233400
GCCCAGCAGA TGTCGGCGTG GCGGCTCAAC AGAAAGAGGC CCGACGCCGC GCGATGCACC 233460
GGATCGTGCT CGCGCAACCA GCGCATGAAC GGATACGGGT CGTCGATGCA GGCTGGCGAC 233520
GCCAGTTCGG CAAAGGCGTC CGCGCATGCT GTCGTGGTTT CTTGCATGTC CATCTTGGTT 233580
ACCTGTCGGC TGGCTGATCT GACAGGCGCG CCAGGCGCGC CTGCAAATTG CGGAGAAGAT 233640
CGCGATTCCC GGCGGCGTCC GCGCATCACC AGAGCACCGG GAACTCCTCG AATCCGCCAG 233700
TGATGATCTC CTTGCGCAAC TTCAGTTCTT CGGGCGCCAC GGCCAGGCGC AGCGCGGGAA 233760
AGCGCTGGAA GATCGAACCG AACACCACCT TGAGTTCCAG CCTGGCCAGC GCCATGCCTA 233820
TGCAGTAGTG CGGCCCGTAG GAAAACGCCA GGTGCGGCTT TGCGTCGCGT CCGATGTCGA 233880
AGATTTCAGG GTCCTCGAAA TGGCGTGGAT CGAACGATGT CGCCGGCAGG CCGACCAGCA 233940
CCTTGCTCTC CGCGGGAATA TGCACGCCCG CGATGGTCAC GTCGGTCCTC GGATAGCGCA 234000
TGATGCCGTC CCAGCCCGCG CCCGGCGGGT ACATGCGCAG GATTTCCTCC ACCGCCTTGT 234060
CCACCAGGGA TGGATTGCCG ACCAGACGTT CGCGCTGTTG CGGATGGCGC AACATGGCCA 234120
GGAGGCCGAA TTCGATCTGC GCGACGGTGC TCTCGTGTCC TGCCACCAGC ATGCCCGCCG 234180
CGAGGCCGAT CGCCTCTTCC TCGGTCGCCT CGCCCCGGTC GACCGCCGCG AGCAGATCCG 234240
TCAGCAGGTT GTCGCCCGGA TCCTGGCGCT TGTCCCGCAT CTTGCCGCGA ATGTAGGCGC 234300
GCAGTTCTTC CCAGGCCAGG CGCGACGCGC TGCGCGGGCC GCTTTCATGC TGGTGCGTCA 234360
TCACCTCGTC GGACAGCCCG GCAAAAAAGG CGTGATCCTC GTAGAGCACG CCCATCAGCG 234420
CGCTGATGAC CATGGCCGGA AGCGGAAAGG AGAGGTGGCG CCGCAGGTCG GCGGGCTGGG 234480
GCTGGGCCGC CAGAGTCTCG AACAACTGCG CTGCGATCGC CTCGACTTGC TGCGCGAGCA 234540
GCTTCACCCT GCGGTCGCTG AAGGCCGGCG CAACGATCGT GCGTAACCGG GCATGCTCGC 234600
CCCCTTCGTG CGAGACCAGC CACCCCGGCG AACCGAGAAT CACACAATCC GGGGTGAATG 234660
CCGCCGGCGG CATTCCCGCA GGCCGGAACG CCGCGTCGGA CAGCACCGCC TTCGCCTCGT 234720
CGTAGCCTGT CACCCACCAG CCTTCGTGCC CCCGCGGGAA GCGCACGTTG TGAATCGGAC 234780
CGTTGGCGCG TAGCGCCAAC ATCGTGGGCG AGGGCTCGAT GTGATCAACC CGCCACATCG 234840
GCAGCGTCGG CAAGGGTTGT TCGGGCATGG TGGCACTCCA CTCTCTAAGC GATGGAAGGG 234900
CATGCCGGCG TGTGCCGCCG TACAACTAGG GCAGCAAGAA CCGCGCCAAC TGCGCAGAGA 234960
GCGCCTCAGG TACACTGCTC TGCAGATCAT TCAATGTCTT AACTGAGCGC GGCACGCCCG 235020
GGCCGAACAT CACCATGTCG CGGACGCGAC AAACCCGACA GTAGATCGAC GCAAAGTATG 235080
TGGGTCGCCT TACCTTGGCG CAGAAGTCAG GGTTCGTCTG TCACGCATGA TCGGACCTCC 235140
AGTAAAGCGT TCACGCTATC GTTTGGTAGC TAATCATGAG GATGCGGCTG TTTCGCCTTA 235200
GTTCGGCAGG GTAAGTTGCG AATGAAGCTC AAACGAAATT TGCCGACGAC AGGGTTTTTT 235260
GCTTCCAGCT CATCATCACC GAAAATCCTC CGCTGACGTA ACTGATCTTC CTGCCTTTCG 235320
ACGGGTTCCA CTTGCCGAGA CTGACGACCG TTGCGGAATT GTGCGTGGAG CCCGTCGCGC 235380
AGAAAGACCG ACGGAAAGTA TGACAAGGGT GGCCAGGCGA AAAGTGGAGC GCGCAGGCCA 235440
CATCCGATAC AGGGCGCTGC GCTTCAAATA GTCACTTGCG TTCATGCGTC CTCTCCTTCG 235500
CGTCAATTGC GACGGAGAGA CCCTACGCTA TGCCGATTAG GCTACCGCTA TGCTACCGAA 235560
AACGTTGGCA AAAACTTGCT TAATCGGCAT AGTTGGACGG TCGGCATAAA CGGCCAAAAT 235620
GAATGCCGTC GATCCGCTCG ACGGGCTCTC GCAGACCTTG AATCGCATTG CTCAAGGCTG 235680
GCCAGCATCC GAAATTGAAG CCCTCATGCC CTGCACTTCA AGCCTGACGT TATCGGCTGA 235740
CCGCTTACGT CGAAGGGCAG GTCATGGCGG AAGAACTCTT CGCCGCAGAG ATACTGGAAA 235800
TACGGGTTTT CCACCCAACG GGCGCACAGC TCCTCGTCCG ACAGGTTGAA CGTATGCTTG 235860
AGGATCGCCA GCCCGGCCAT CAGCCGCGTT GGCGGTGGTG GCATGCCGGG GTCGGAATAG 235920
ACCGCGCCGA AGCGCCGCTC CAAAAGCGGC TAGTCGATCG CCTGCGCCAG CCGCACCAGT 235980
TCGAGCGTCA TGTTGATGAT CTGATCCAGA CGGGCATGGA ACAGGTCCTG CTCTCCCGTA 236040
TCGCGCCGCT CGCGTGGTCT TGACATCGCC GCTCCTCGAT TCACCGCCGC TAGTACGAGT 236100
GAATCACGCC GCGCCGAAGC AGAAAACCGC AAAATCATAT TGCAAGAAAA GCGGACTCAG 236160
CGAGAGCCTT TCGGCAACAC TGCGTCACAG GAAAGCGAAG AATTACCGCA GAGAGGCGTG 236220
GGATAGTTCA CGGACGACTC CTCAAGAGTA GTTGCAAATT ACGGCTATCC ACGCGCGGAG 236280
AAATCGTCTG CTGTCCGTTC TAATAGCAAA ACTTCAGACA ACCGGCTTTA GAGGTGCTTG 236340
ACGTTTAACG ATCCGCCCCA CTTGTCATTT AAGTATTCGC ATACCAATCG CCTATGACAG 236400
TTATGGGGGT AGCCTCTGAA CAAAGCAAGC ACGTGCCCTC AAACATCTCG GGCTTGAGGC 236460
GATTCTCAAT TTGTCGCGTT GCCATTAAGC GCATGAATTG CCCCTCGTAG ACTTTCCAGT 236520
CGCCTTTGTC TCGCTTCTAA GCCTTCAAAA TATCATCGGT AGGAGCCAAA AGGGGCTGAT 236580
GCACGTACTG GGCGCCGCAT ATCTCTGTTA GGAAGTATGC CAGGTCATCA GCCTTCGCGA 236640

ACCCTGCGAG CTGAAAACGG CAACATAGCC GAGGTCCTGC AGCATCGCAG CAAGGACCGA 236700
ACCCAACACC GGCCCATCGC CATCGTGGGG AGTTCCCAGG GCATGGGCGG CAGCAAAAGC 236760
CGCGCCGCAT CGCTCGCCAG GCTGTCTTTC GCAACTCCGC GCCTGCGAGA CGATTGCCTG 236820
CGAGGCGGCG AGCGCTTCAC GCAACTTCCG GCCGGCAAAG ACCTTCTTAT TGAGGAGCGG 236880
CGTGGGCCAG CCCAAGACCA GAGGGCAGAG GGTCTCGAAG ATCCGCTCGG CTAACAGGCC 236940
TGCCCCTGCG GCCATGCGCT CCTCGTCGCT CGTCGGCATG TCCGTCGCAT TGAGGACGGC 237000
AATTCTGGAC GCAAGCGATT GCGAAAAACT GCAAAGCGAA AAGACCCGTC CCGCCAGTGC 237060
GGGCGCCTCC TCGCCTGCGG CCGACGAACA TGCGGGCGAG CCGCCGCGGC CGGTTCGGCG 237120
ACCGAACAAG CGAGCGCACG CGACACTGTA GCGTCGATCC GTATCGTGCC TGCACCGACG 237180
AGATAGCTCA TTTCATCATG CTCCTCGTTC TAACGGAATT TGCCGCGAAA CAGGTCGCGG 237240
CGATTGAGAG GAGCCGTAGG GGCCGGGGGC GGTTCGATCG CGGACGGATC GAAGAAGGCC 237300
CGTATGGTCT GCTCGGCGGT TTCCAGCGCC GCCTCCGTCG TGGCGAATTC GAAGACCGGC 237360
GAAAACAGTG AACAGAGATC GACACGCCCG ATCGTATCGA GCTCGCCGGC GTTGAAGCGA 237420
CCGAACGGGG AGCGGGAAAA ACACAAACAG ACATCTTGTC ACGCAGATGC GAAGCGTCCC 237480
ATAGTTCGCG CAGCAAAAGG GGGAGACATG CCGAGCGTCT TTTTTTCATA TTCGCACGCA 237540
GATGAGGGGC TGAGGGATCA GCTCGAAAAA CAGCTGTCGA TGCTCAAACG ACAGGGCGTG 237600
ATTGAGACCT GGCACGACCG GCGCATCGGC GCCGGCGAAG ATATCCATCG TGCGATTGAC 237660
GATCACATCA ATACGGACGA CATCATCCTG CTGCTCGTCA GCGCCGACTT CATCGCATCG 237720
GATTACTGTT ACGACATCGA AATGCAGCGT GCGATGGAGC GCCACCACAG CGGCGAAGCC 237780
ATCGTCATTC CCATCATTCT TCGGGCATGC GACTGGCACC ACGCGCCGTT TGGAAAGCTG 237840
AATGCGGTTC CTCGGGACGG CAAACCGATC ACGCAATGGC CGGATATCGA CGAGGCCTTT 237900
CTGCAAGTCG CCAAGGCCGT GCGCGAAGCA GCAGGGCGGG TAACCCGAAC GGCCTCGGCT 237960
CCGCCTGCAC GGGCCGCTGC GGCTGCGACG CCTGCGGCGT CGCCGGCTCC TCAATCCCTC 238020
GGCCCGCGGT CGAGCAATCT GCGGCTCGCG AAGAGCTTCA CCCAAAGGGA CAAGGACCAG 238080
TTCAGACACG ACACGTTCGA ATACATTGCG CGTTTCTTCG AGAACTCCCT TAAGGAGCTT 238140
GGCGAGCGAA ACGCCGGTTT CGAGGGCGTT TTCCGACGGG TCGACGCGAA CAGGTTCTTC 238200
GCCACCATCT ACCGCGACGG CAAGGATGTG TCACGGGGCA CCGCCTATCT GGGTGGGGAG 238260
ACGTGGGGAC GAGGCATCAA TTACGTTCAC GGTGAGACGA CCAGCAGCAA TAGCTCGAAT 238320
GAGTCCCTGA ACGTCGAGGC CGACGATCAG ACGCTCTTCT TGACAAGTAT GGGCATGGCG 238380
TCGTTTGGGC GGGATCGCGA TCAGAAACTT TCACAAGAAG GGGCCGCCGA ACTGCTATGG 238440
GCGATCCTTA TCGCCCCTCT TCAGGGGACG CGCTATTAAG GGAAAGCATA TGCTGTCACC 238500
GAGTGAATTC ACGATCGGCA CGCTGGGGAG CGCCGCGCCT CTCAGCCTTA TTCTGCCACG 238560
AACGAAATAC GAGGCGACCA TGCTGGTCGG CCATGTCGAC AAAGCGCCTG CGGCTGTCTT 238620
TCTTTCAGGA GAGTTTGCTT TCCATTACTT TCCGAGTACG GACAATGACA GTTGGCGAGG 238680
CCTTATTGTT CCGGGCGTCC GCGTAGAGGT GGACGAGACC AGTGTCTTCG ATCAGGGCCA 238740
AACCATGGCT CCCTTGGGCG CGGCTATCCG AATCGATACG CGTCTTGCCA TACGGGCAAA 238800
GAGCGAGCAT TCACTCAGCG GATCATCCGC CCTTACGATT CACGATAAGC TGGTATCGGC 238860
CGGAGACCTT CGCGCGGGGT TCACAAGGTG GCAGATCGTC ATCGGGGAAG GACAGACGAA 238920
GCGGGTGCTC TGGCAAAGAT CCGACGAAGA CGAAGCCGCT AAATCATCCG CCTGATTTCG 238980
GCCGCCAACG GCGCCAGGTC GCCGCTGAAG CTCCGGATCA TGGCGTCCAG AAACCGCTTC 239040
GGCTCCAGCT TGCTCTCGTC GAGCTCGTAT CCCGCATAGC GAGTGAGCAT CGTCAGGAAG 239100
ACCAGTTGGG TGCGGCCGTT GCCTTCGCGA AATGGATGAA TCGCGTTGAT CTCCGCGAGA 239160
AACCAGCTTG CGCCTTTGGC GAAGGCCTCC TTGCTTTCCG TCCTGGCAAG GTGGTCGCGA 239220
GCGGCAAGTT CGGCAAATAG GCGATTGGCT TCCCTTTCGA TATATTCCGG ATAGCAAAAC 239280
CAGTTCTCAC CCTTGCCGGT GCGGATAATC CGTGTTTGCC CGGCCCATTC ATAAACGTCC 239340
TGAAAGAAGT GTCGGTGTAA CGCGCGATAG TGGGCGAAAT CCAGATCGCC GTCCGGAAGC 239400
GCTTCCCGCG CACGGGAATC GAACATCAAT TGCTCGAACT CATCCAGCTC ATTCTGATCC 239460
GGGATATTCG CCCTGTTGCG CAGCACATGG GTGCCGGGAT AGCACAGCGG ATCTTCGACG 239520
GCATTGTAAC GGACCACCCG TCAGCCCTTC TTAAAGGCCT TGACGATCTC GCTACGGTAG 239580
GCGTCACCCT TGAGGCCGCT CGCGGTCAGC TTCTCCGAAA GCGCCTTTGA CGCGGCATTC 239640
ATCTTCAAGC CTTCGACCTG GGCGAATTTC GCGGCCTTCT TGCCGCCGAT GGGCTGCGTC 239700
ACGAACTTGC CTGTCACGGC CGAGCGGGCT GTTTTCGTAT CCTTTTTCAT GACTCACCTT 239760
ACCATCGACG CGGAACGGTT GCCTACTTCT TTATGGCCTG CCCAGGAACA GGTCCTGCCA 239820
ACTGCCTTGG CCGGTATTCC GCCTCTCTCC TGTTCTTTCC GACGCAGAAC ACGGCATGCG 239880
TGAGGGAAAG GGGAATTGCC GAGAAATCCG TCTGTCACCG GCGGCTCACT CGATCTTCGA 239940
TCGAAGGCTT GTTCTCACCC CCATGGGATT TTCGTCTGGA GCCTCGGACG GGTCACGCCG 240000
TGTTCCCGCC CTGGTCAATG CCCGTACCCC CGCTGGCCGG ATTGGCGCCA AACTGGGCTG 240060
ATCCCTGCCG CGCTCAATGG ACCTTCCGCC GCAAGCGGTC TCCCGAGAAC GCCATTGCCC 240120
GCGCCCCGTG GTGATCATTC GGCGACCGGC CGCAGAGGTG GCCGGATCAA CGAAGAGAAA 240180
AACACCAATG GCGAATACCA ACACACCTAA GTCACCCACG AATCTTCCAT GTCGTCGGCG 240240
AGAAGGACAA GGCCCGCTGA ACCAAGATCG GTCGGCTGGC AGCACGGCGA TAGCGACGGA 240300
CTCAACCTCG TTAATACTAC ACCCCACTGG TCGCAAGCCG TATGGTCGCG CGCAGGATCA 240360
AAACGCAGGA GCCCGATGTA TGAGCACGGC CGGTTTCAGC GGGCTGGCGG AGTCAACGAA 240420
CTTCGCCAAT ATCAACGCCA TCGGGTCTAC TTCACCGATT TTGAGGATTA TCAAACAAAA 240480
GTCGCTACCG CCGTTTATGC TTTCGGTCAG CCAATCGAGG AGCTCGAGCT GCAATACATC 240540
GACGGCGAGT TTTCCCGCCT GTTCGAGGCC GTCCGCGTCT CTCAAGCCAG CCTGATCGCT 240600
TGGTTTGACC TGCTTGAAGC GCTCGGTGGC GACGAAGACC GCCACCTGAT CGCCCGCCAC 240660
CTTGCCGGTT TGGGCAATGC GGTCGACGAG CTTGCCGACC GCTGCGATGA TTACACGCTG 240720
TTCGGTGGGA CCGCGGCAGA CTACGTGGCT GAAATCGTCG AGAAATGCTA CGACGTTTCG 240780

ATGCTTCGGA ATTTCGTTTG ATCGAGAACC TGTGCCCGGT TGCGAGTGAC CTCGCCACGC 240840
TCGGCAACGC CGTAGCGGCA TTGTCGCGTT TCACGACATT CGGCTTGCCG ATCTTCTCGG 240900
CCCACCAGCG CAACCAGCTG GTAGTTTTGA CGCTCTGGCT ATCGATGACC AGCCGTCGGG 240960
CTTGCCTCTT TGCCCTCCAA CTCCCGGGCT TCTATGACGA GACGATGGTT GATGCGTGGC 241020
CAAAGTCCAA GCACCCTCCA AGGCCCGGAT GCTAGCGCTG AAAACTGCCT TGTCGCCGTC 241080
TCCGACGCCG GTTCAATGGG CGGTGGAGAC GATAGCGGGT ATCGCGACCG CCAGCGCCGC 241140
CTCCGAACGC ACCTGGTGTT TTGCGGTGCG ATCCCGGCGC GGTAGCTGTC GTCTTCATGC 241200
CTCCGAGTGA AGGGGCGTGA GTGTGCACGG AACGAAGCAT CACGATCGTG GTTCGGAGCT 241260
TGCAGTTTTG CAAGTCTACC CGACCAGGCG GTGCCTGCCT GCTCACTCGG AAGCGGAGAA 241320
ATACCGATGG CGCACGGTAC GAAGCAAAAG CTGATGAAGG CAAGCGACAT CCCAGCGTTC 241380
GTGAATGAAG TCATCGAGGC CGGATGTGAC ATCTGCGCCG TTGGCCACGA AAAATACGTT 241440
ATTGGAGATA CCGACCTGTC ACGGGGCGCC TATGGAAAAA TGAGACAGAG GCTGGGTAGG 241500
ATCGAAGAAG CGTACGGCGA TCGTGATTTC CTCAAACTCG AAATCGTCGC TTACCTGCGC 241560
TCAATTGGCA GATATGTCGA CGTCGGCGCA GACGGATCTG AATAGAAAGC GCGCCACATG 241620
CTCCCGCAGA TGTGCGGCGA AATGCCGGGT AGAGCGTTAG TCTAATGGTC CAATTGCCGG 241680
AGCATCTTCG AAGCCGCCGC CCGTGAAGCC GCCGCTTCGG TTTTCTGATA GCGGTCTGTT 241740
TCGTCCTGCC CGATCAATTC CGCCGAACGG TTACCGGGCA ATCTCGGCCG CACTGGGCGC 241800
CCGCCGCGAG ATGATAGTCG ATGTGACGAG CGGCCTTGCG TGCATCATCG CTCGGCTGCG 241860
GAAATCTCGC TGCGAGATTT AAGGAGTGCC ATTGCCTGTG AAGCGGGCAG GGGGCCGCCA 241920
AACAGATAAC CCTGCGCCTC ATCGAAGCCT TCGGCCTTTA TCGTGTCGAG CTGGTCCTGG 241980
CGCTCGACGC CTTCCACTGT GGTGATAATA CCGAGTGAGC GGCCGATGGC GGCCACCGCC 242040
CTGACAATTG CCAGGGATTC CTTGCTCTTC GGCAGATCGG CGATGAAGCT CCGATCGACC 242100
TTGATTTTAT CGAAGGGAAA GGTTCTGAGA TAACTCAGCG ATGAGTAGCC GGTCCCAAAA 242160
TCGTCGATGG CGACTATCAC ACCGAGCTGC CGGATCTCCT TCAGCGTCTG AAGATTACTG 242220
TCGCATTCCT CGAGCAACAC CGACTCGGTG ATCTCGAGCT GCAGGCGCGA GGCGTCCAGC 242280
CCCGTGTCTT CCAAACTGTT GCGGACCGTC GAGGCGAGCC TCTGGTTCCT GAATTGCAGC 242340
GGTGAAAGAT TGACCGCCAC ACTGACATAG GGGGGCCACT TGACGGCCTC GGTACAAGCC 242400
TGACGAAGGA TCCACTCGCC AAGCGGGCCA ATCAGGCCGG TTTCCTCGGC AACCGCAATG 242460
AACTCTGCAG GAGACACCTG CCCTCTCTCC GGATGTGGCC ACCGGGCCAG CGCCTCAAAT 242520
GTCGTGATCT GGCCTGTGCG AAGGTTGGCC AGCGGCTGGT AGCGGACTTC CAGCTCGCCC 242580
TTTGCGAGCG CGTGCCGCAG TGAAACCTTC ATCCGCTGTC TTGCCCGAAG ATCAGCGTCC 242640
ATTTTGGGCT CGTACTGAAC GATTGTGCCA CCGCCGCCGG TTTTGGCCCG TTCAAGCGCT 242700
ATATCGGCGG TCTTGATAAG CTCGTCTAAC GACTGGTCAC TCTTCGAAGC CGCGGCAAGT 242760
CCGACAACTA CCTGAAGATC GACATGCGTC CCTGCGAGTT CATAGGGTGC TTCGATCACA 242820
TCGATGAGCT GCTGCGCCAA TAAACGAGCC TCAGCATTGC TGTTGATGCC AACGCGCAAG 242880
ACGACAAATT CGTCGCCACT GAGGCGACAG AGGATGTCGG ATTGCTTGAG AAAATTTCTG 242940
AGCCGCTCAG TCACATGGCG CAGCAGCAGG TCGCCGGTGG CTCGCCCATA AGCGTCATTG 243000
ATCGCCTTGA AGCCATCGAG ATCAAGGCAG AGAATTGCCA GCCTCGTGCT CGGCGCAATA 243060
TGGTCCGACA GGCGCTCGAA CTCCTCGCGC AGGAACTGGC GGTTTGGCAA GCCTGTCAAC 243120
GCATCATGCC GCGCCATGTG GTGAATTTGC TTCTGCGCCA GCAATCGCTC CTGTTCGGCG 243180
TTACGGCGCT CGGTGATGTC GAGCGCAATG CCAACGAAGC CGAGAAACGT TCCGTCGCTT 243240
GCGAAGCGGG GCTGTCCTAT GTCGATGACC CAGGCCGAGC CTCCGCCGGC ACGCTTCAGA 243300
CGATATTCCA TCCGGACTGG CTCGCGAAGG TCGAAGGCCT GGTAAAATAC TCTTTCGACA 243360
GCGTTCCGGT CATCGGGATG GACGGCATTC AACCAGCCTT TCCCCGCCGC CTGCTCGGCG 243420
GTCTGTCCGG TTGTCTCGAG CCAGAGGCGA CTGTGGTAGT CGTCGGCGCC GTTTTCGCCG 243480
GTCACCCAGA TCATCACCGG CGCGTCGTCG GCGATTGCCC GGAAACGGGC TTCGCTCTCC 243540
TTCAGGGCTT CGAACATCTT GCGGCGATCA TCGATGTCCT CGACTGTGCC GTACCATCGA 243600
AGGATGCTTC CGTCTTCGGC TCTGCGTGTG GCGGCACGAC TGCGATACCA ACTGTAGCCT 243660
CCCTCCGCAG CCGCCAGGCG AAACTCCACA TCGAGCGGCT CTCCGGTTGC AAGCGATTTC 243720
GCCCACTCTC GCTGAACCTC GCCCAGGTCA TCAGGGTGCA TCGCCTTCGC CCATCCTGCT 243780
CCGAGAGCTT CCTTTGGGGC ATAGCCTGTT TTCTCCCAGC GCGGACCGAT TTCCAGGACC 243840
TCGCCCGATG GATCCGCTGT CCATGGCACT TGCGGATGCA GTTCCGTCAG CGATCGATGA 243900
TGCTCGACGC TCGCCTGAAG CGCCGCTTCC GCCGTTCGGC ACTCGGTGAC ATCCAGGGCG 243960
ATCGCCACAA TAACCTTTTC GCTTGAGCTC CCTCTCGTCA GGATGGCACG ACTCTTCCGA 244020
GTTACAAGGC GCCTTACTTC TCCGCCCGGT ACGGCAATCG GCTCCTCGAC GGAGACCTCC 244080
TCCCCGGTAG AAAGCACGCC AATGTCCATG TCCCGATAGC GATCGGCCTC TGCCGTCGGA 244140
AGAATGTCGT AATCCGTGTG GCCGATCAGG TCGCATCGTG CTCTGCCGAC AAGCATGCAC 244200
GCGGCATCAT TGAGGAAGAG AAAGCGGAAA TGCTCGTCCT TGACGATGAT GGGCTGTGGG 244260
ATCGCGTTGA GGATTGCCTC AAAATCGGCG TCGAAAGTCA ACTGGTTCTC GAATGAAGTC 244320
CGTTGCATCG TAGGGCTCGG TGTTGGAGAG GTAAGTTAAT GATGTCGGTC CCGGCCGAGG 244380
CGGGCCTTAA CAATCGTTCA GATGCGAAAT GGCGCATAGA CGCCGCGTCA AACACCCTAG 244440
AGTAGCAGCG GTTAACAATA AATAGGCCCT GTCGGCTCGA AGCTGGGATT TTGGCCAAGT 244500
TGAATAACGT TGTGGGAACA CTGCCGCGGT GATTGACGTG TGATTTGTCG GGGAACGACA 244560
TTTCGCTCCG ATTTATGGAG TGGAGTGGCG AGTTTGAGCC TTTCGCATGA AGATTTGGTC 244620
AGCCGCTGGA GCCTGAGTTT TTCCGATATA GAATTTGTAA CCGGCAAGTC GGTATCGGCG 244680
CGCCTTGGGC TGGCGGCGCA GCTGAAGTTT TTCATGACCT ACGGCCTTTT CGTACAGGAT 244740
CGCAGCGCAA TCCCTGCCGA TGGTCTCTCA TACCTGGCCG AGCAGCTTGG ATTTGATGAC 244800
GATACCCCGA GCGAATACGA TTTTGGTGGT CGTACCGCGC GCCGGCATTG ATAAGCGAAG 244860
CGTCCTCAAG GCCGGGCTTT ATCACTGCGC CCGCTGATCC TGTCGGTGAA TATCCTCGAG 244920

CATCCCGCCG CTTTGAGAGC GCGCTGTTGC ATATCGAGGT TCTGTCAGCA ATGAATATTG 244980
AAAAGCTTCT CGGCGGCTTC GCTAACGTCG CCGCCATCCT TACACCTCTG GTCGCGGTTC 245040
TCGCTTATAG TCGCTTTCTT TGGGAGCGGC GTCAGAAGCG CTTGCGCCTG GAGAGCTATT 245100
TGCGCGAACA GAAATTATTC GAGTGCACTG GCCAGCATTC GTTCCTGCAC TTGGTCGCCA 245160
CTCTCGGAAT GTTCGAAGCC GATATCATGG ATGCGTCGTA CCGCAGCAAG GTTATCAGTC 245220
GCAACGTCGC CGTAGATGTT GCGGGAGAGC CTGTGAGGAT CGTCCTTGAA TACGAGCCAG 245280
ACGACTTGGA AAAGGAATTA CCCAAGCGGC CTGGCCGCGG ACAGTTCTAA AAGGAAAAAC 245340
GGTTCGTGAC GTCAATCAGA CCATCTGTTT TCAACCAACG GGTAACGCCC GAAGGCGGAC 245400
CATTTCTGCA TTTCTACGAT CATGGCGAAA CAGCGGAAAC GCTTGACGTT TACTTCAGTG 245460
ACGTGAATCC CGAGGGGGAG GAACACCAGA GCAGACTGCT CCTCCGAGTG TGTCCAGAAG 245520
CTATAGTGAT GTGGCCGATT AACGTTGGGC GGGGTAGCCA AAACTACTTA GAGCCCAAAT 245580
ATGCCTCTCT CACCGATATC TGGATTCCTC GTCCGGCCTC CGGCCCATAT CGATTGCCGA 245640
AGACGTCTGA AGACGTAGAC GAACTTTTAG ATCGCCGCCT CCCGAATGGT TTTGAGACAA 245700
ACGGGCGACT CGGCTTACTC CTGTACAAAT ATCGATCGAT TTGCCGCTCC ATCGCAGGCA 245760
TTGACGGTAT CACCACTCTG GTTGTTCATG GCCAAAGCGG AGAAAGGGAC GCAATTATAC 245820
GCCCCCTAGA ATACGTTCTC GGCACCAAGC GGTTTCATGC CCTGAAGCGC GAATTGACCA 245880
GGACCACGAA TCGTTTCCGC CGCGAGGCCA GAAACGCGAG GCAGGACCGT ATTTACGAAG 245940
CTCTTTTGCA TTCAGCGGAT CGCGAACGCT TCCCTCGGAA AACCAGAAGA ACCAAACCGG 246000
ATGCCATCTC CGAGCTCACT CAGGGAGGCG CGACCGACGG GATATCGAAG CGAGATCGAC 246060
GCACCGCCGT CCGACTCGTA CGACAGAGCG TTAACACTCT GGCCAAAGAC GAGCCCCATG 246120
TATTGCTGGC GCTATAGAGC AAGATTGAGC TCATTACGCT GAAGGGACTG ATTGACCGAT 246180
TTAACGAAAT GCTCGCAAAG GAGTTGCCCG AATCGAGATG GCAAGCGTTC CTTTAAGATA 246240
ATCCCTTCAT TCTCAGCATG GCGTTTTCCG CGCCGGCGAT CATGATCCAG GATAATGCTT 246300
ACGTCGGCGG TAAGCGCTTC AATGGTAGCA ACGGCAAATT TGCCGATTTC CTGATGGCGA 246360
CCGCTTCGAC AGGCAATCGT AACCATCCGG CGAAGTCATC ACCGGAGGGG TACAGGCAAT 246420
CTGGCCCTGA TCGAAATCAA GAGGCCCCAA ATCGACTTGC TGACCAAGAG CGCCTATCGA 246480
GGGACTGATG TCTACGGCCC ATCGGCCGAG TTGAACGGAG CCATATATCA GGTTCTGGAC 246540
TAGCGATTCC GGCTTCATCA GAATCTGCCC ATCTTCAAGG ACGAAACTGA ACGGAATGAC 246600
ATTCATGTCA TTTTGCAGTT AATCGTAAGA CTCAGATATA GCGATATCAA CGACTTGACC 246660
CGGCACATAA ATCGAGAATA GGCAATTAAA TTGAGAATCC CACGGGATTT TGGTACTTAA 246720
TCTGCGAACC GCCGCGATTA CTAAAATGGC GATTTTGGCT GAAGTCCGCT TCCGCGTATC 246780
ATTCGTTTGA ATCGAGGCTC AGCGCCACGT TGCGCTCCGA ATGAGCGATC GCACTTTATT 246840
ATCTGTCATG GTGAAATGCT CACAGATGGC GCAACTCAGT CGAAACAAGG CTTCGGATAC 246900
GCACTGAATG CAAGCAGAAC CAAAAGCTGC CGCATCGGAA GCCGGAGAAA TCCTATGAGT 246960
CATCGTGCAA TGTCTGGTTT AGTATGGTCT CGGATCTTGG TGGCCCTCGA CGTTTCGATA 247020
GATGCGCTGT ATCGGCGGGA GTCTCGCTGG ACAGCGAAGC CTTTGAGGCG TTCGGCTAAT 247080
GATTCGGTTT TGGGATCGCG CCTGCTGGTG CTCAAGTCTG TCTCGTTGAT GCCAGGTTGG 247140
TGGCCTGCAA TCTCGTCAAT TACACTGCTG GAGTGAGAGT GCTTGTAAAA GGTCGCTGGG 247200
AGGAACCTCT CCCCATGAAA AAGGCTTATT TGCGCATATT CCAGCATGGC GTCGGGGCAT 247260
GTAGTCACTG GAGTAGTCAC CTTCGTGGGC TATCGTTCCA TGGTGGTGTA GATTGTCATG 247320
CCAGGCATCG AACGTCGGTA ATTCTCGATA TGCGGCCAGT GCAAATTGTA CTCCGAACAT 247380
CACGCAGTCA GCCGCAGACT TCTGCGCGCC AACCTCAATA AAAGCCCACT TCGCCTGCGT 247440
TCCCAACTGC CGAAACGAAT CCCCCCGCAA TTTGAAATAC GAGGCGACAA AGGTGTAAAA 247500
GTTGGCTCCC TCCATCACGA TAATGGTGGG TGCTGCTCCC GCGCGTGTCC GAACATCAGC 247560
GGCGACGTGG TGCTGCTCGC CGTCCGCCAA CCGCACGACA GCACGCCAGG CACCGTCCGA 247620
TGAGCGGTCA TTCAGCGCGT CGAAAAATCT AGCAGGCGAG TCCATGTGTC TAAGGTCAAG 247680
GTCCGGGTAG CGCCGGTTGT AGCTGGCCGC CAAGAGAGGA AGATTCCTGA TGTCCAACGA 247740
TAAAATTTTC TCGTCAGGCT GTACGTTGGC AGACAAATGG CGTGCTACCT GCCGACCGTA 247800
TTCCATCAGA CTGGATGACA TCCCAGCCCG CCTGGCATGC TCAAGTGCTT GGCCAAGCAA 247860
CTCGACTTTC TCGTTGATTC GGGGTGAGAG ATTACGGCTC GACGGATTTC CAATCTCGGC 247920
CCGCCGAGAT AATTGCAACG AATGCTGGCC TTCAAGATGG GGTTGAGGTG GACTTGATCG 247980
TGCGGCCTCA ACGTAAGCAG CAAACTAGGT TTCTTGGCCG TGTCCATAGT CCTGATGGCT 248040
CTGGCGCCGT ACGCTCAGCC GGGAAATGCA ACCACCCATA TGCAAGCTCC TGTGATACGT 248100
GTTTACCCGA TCTGGCTCTT CAAAAAAATT CATCCGCGAA AACGGATTAA GGAGTCGCGA 248160
TCGCATGCAA CCGACTAACC GCATTCATGA TCTTCCGGCA TTAAACTTGC ACGGCCTCAC 248220
CGTGATGCAG TCCGCTAAGA TGCGGCTCAA AAGTTCGCTG ACGCGCCTGC TGCAACGGCA 248280
TGGTGCAGAA TGGCGAAATC TGGACCATCC AAAGGGTTGA TATCATCAAA TAGGCAGTCC 248340
CAATTCGTTG GCGTGGTGAC GTTCGCAGAT CTGCGTATTT GGATCGCTAC GTTCTCCAAG 248400
TTAACTGAAT TAGCTTTCGA GAAGCTGACG GCTGAACTGG CTGCTGGGCC GTGGGCTTCG 248460
TGCAGCCGCT GGAGCCTGAG TTTTTCCGAT ATAGAATTTG TAACCGGCAA GCCGGTATCG 248520
GCGCGCCTTG GGCTGGCGGC GCAGCTGAAG TTTTTCATGA CTTACGGCTT TCTCGTACAG 248580
GATCGCAGCG CAATCCCTGC CGATGGTCTC TCATACCTGG CCGAGTAGCT TGGATTTGAT 248640
GACGATACCC CGAGCAACAT CGCCATCGCC TCGATCGACG AACCGGCGGG GCGGGTCTGC 248700
GATGTTATCT TCCAAACGCG ACACTGTCGA TCCGCAATGC GGACGTCTGG GGTGATGCGG 248760
GGACGGCATG CGCCTCCGAT TCGACGAAGT TCGGCGCATG GGACCGCAAT CTGATGACGG 248820
AATGGCATGC CCGCTACGGA GGCCGGGGTG TGATGATCTA TTGGCATGTC GAACGGCGGG 248880
CAACCTGCGT CTATTCGCAG CTCAAGCGAT GCTCGTCCTC GGAGGTTGCC GCGATGATCG 248940
ACGGCGTGCT GCGCCATTGC ACCGACATGG AAGTCCAGCG TCAGTACGTC GATAGCCACA 249000
GCCAAAGCGC CGTCGGCTTC GCCTTTTGCC GACTTCTCGG CTTTGAGCTC GCCCCACGCC 249060

TGAAGGCAAT CGCCCGGCAG AAGCTCGCGC TTCCGGATGC AGGCATGCGG ACGCGGCTAC 249120
CGAACCTCCT GCCGATCCTT TCCAGCGTGA TCAGCTGGGA AGAGATCGAG CAGCAATATG 249180
ACGAGATGGT CAAATACACC GCCGCCATGC AGACCAGAAC GGCGGACCCG GAGGCCATCC 249240
TGCGGCGGTT TGCCCGCGCC GAGGTGATGC ATCCCACCTA CAAAGCGTTG AGCGAGCTCG 249300
GCCGCGCGGT CAAGACGATA TTCCTGTGCC GCTATCTTCG CCAGGAATCG TTCCGTCGAG 249360
AAATCCACGA GGGGCTGAAT GTCGTCGAGA ACTGGAACAG CGCGAATGGC TTTGTGTTCT 249420
TCGGCAAGGG CGGCGAGATT GCCACCAACC GGGTCGAACA GGAAATCTCC GCCCTGGCTT 249480
TGCACCTGCT GCAAGCTTCA CTGGTCTACG TGAACACACG CATGCTTCAG ACCATGCTGG 249540
CGGAACCGAA ATGGGCCGGC CGGATGGCAC CGGAAGACTA TCGCGGCCTC ACGCCCCTCA 249600
TCTATAGTCA CGTCAATCCT TATGGCCGTT TCGATCTCGA CTTGAATAGT CGGATCGACT 249660
TTGATCAGCT TGCGGCATAA TTTTTGGAGA CGATAATGGT CAAGATTGCT GAAGCATGCC 249720
TGAATGTTCT CTACCAGCAC GGGTTGTCGT CGGCACAGTT CCAATTTTGG TTCGAGAGAG 249780
CAAAGCATGA TCTCTTGGCC GATGGCATGG CCTGCGACGC CATTGTTGCT GAGGTTATGC 249840
AGTCCATGGA CGATCATCCG GACGCGGCAA CCTTGTTCGG GTTGCTGCTG GATGAGGCCC 249900
GAATGGGCAT CGAGAATGAC AGCCCATATG GAAAGGCCTT CCTGGAAAAT GCGGAGAAGG 249960
CGATCAGAGC TCGCATTGCC GCCGACGCTT TCGAACCGCT TCACCGCTTG AAAATCGCTG 250020
GCTTGTATCG GCGTGCCGGT CTGCCCGTGC CAGACATACT GATGCTTGAT CCGGAGGGGG 250080
AAGGTGCCGC CGATGAGATC GCGATGCCGG ATCTTGGTGG CGTGCTTGCC GTTTTAGCGG 250140
CAGAGGTTGA GGCTGAAGGC GGCGGCGCGT ATGAGTTCTT CAGTGGCCTG GATGAGATGA 250200
CGGCAGGAAT GCCGGAGGGG GCCAAAGCTG GGTTTATTCA TCATCTTATG AGCCTCGATA 250260
ATCCCTTTTT GGAACGCTGC GCGCTGTATT GGCTGGTGTC TGGTGCAGCA TTGACCCGGG 250320
AGGCCGTGGC TGACGGCCTA CGAGAAAGGC TCATGCGCGG GGAGCTGCAG CCTGAAACTG 250380
CATCCTATTT GCCGATTATT CGTGGCTGGT TGCCGGCGAG TGCCGCCCGG GCGGCTATCG 250440
ATGACATCGG CAAGCTGTCT CGGCGACAAG GTCTTGCAGA TGCTTCCAAT CAAAACAGAG 250500
CGGAGCCGAT TGTCAGCGAC ATCATGGCGA CCACCGCTGA CGGTGTTGGA GCACAAGGCC 250560
TGACGATTGT CGGAAAACTA CAAGCCCGGA CATTTGTCGC GATGATCCTG TTAAAGACCG 250620
GATACGGCAT CAAGGATGCC TTTGTCATTC GTTGCCGTTC AAAGAGCGAA GCAACCAACA 250680
TCGTCTCTTA TGCCCGCCAG GAAGCAAACA GCGTCAAAAT AGATCGAATG ACTGCCGAAC 250740
TGCTGCTGGA GGCGGCTCTG GCGGATGGGA TGAAAAACGG CCACCCACCA GCGCCAGGCT 250800
TCATTGACGT CATAGAGGCT TGCAGCCTGA GCCAATTGCG GCCCCAGGAA AGGGATCTGC 250860
AGGCTTTGCT GGAACACGTC GATCCGCAAA AGGAAATCCA GAATGCGACA GCGGCAGAGC 250920
TGAATCGGAT GCTCCGCAAT GCGTCCGCAC TGGATGCGCT CGTCCCGTTC ACCGACAGCT 250980
GGTTTGAAGA CACAGGAGAA ACACGCGGCA TCATCCAGGG ATCTCGCTCA GTGCGGACTG 251040
TTGAAAAAAG GATCTGGGCC TTTCTCGAAG GCCGGCGAGA CATTTGGGCG AGGCGATTTC 251100
TGCAAACCGC GATCATTCTC AAATCGGCTA AGAAGGAGCG AATGTCAAAA GCACTGGCAG 251160
CCGCAGCCTT CGCCGTGATG CACAAGCATC CTCTACAGGA CATCCCGCTT ATGAAAGATA 251220
TCGCTATGAC CACACTGGAC GCAGGCGGTG GATCTCCGTG GTGAGTATTT TGCGTATACC 251280
CATCGTGAAC TTCGCCAATG AACAGTTCGA GGCTGTGGAT TCCTATTTAT TTTGGGTCCC 251340
GACTGAGATA TCGAACAAAT CGACGCGATG GCACACGCAA ATTGAACAGT TTGCATGGTA 251400
TAGGACATGG CTCGTATCGG ATATGCCCGG ACATTTACAA CAGACCAGAA CCTTGCGGCG 251460
CAAATCGCCG CGCTCAGGAA CGCCGGCTGC GAGGTCATCC GCGAGGAGCA GAAAAGCGGT 251520
GCATCGCTCG AAGGTCGGCC GCAACTGATG ACCATTCTCG ACTTCATCCA TGCCGGCGAA 251580
ACCTTTGTCA TCACCCGTAT CGACCGGCTG GCCAGATCTC TGCGCGATCT TCAGGTGATC 251640
GTCGACCGGC TCAAGGCCAA GGGCGCGCAT CTGGTTGCCA CTGAGCAACC CGCCGATACC 251700
TCCACAGCCG CCGGGAAGGC GTTCTTCGAC ATGCTCGGCG TCTTCGCGGG GTTCGAAACC 251760
AACCTGCGGC GCGAGCGGCA GGCTGAGGGT ATTGCCGCTG CCAGGAAACG TGGCATCTAC 251820
AAAGGCCGCC CCCCGAAGAT TGACCGCGCA GAAATCCTGC TTCGGCTGCA ACAGGGCCAA 251880
GGCCCCAGCA AGATCGCTCG CGATCTCGGC ATATCGCGCG GCACGGTCTA TCAGGTGCGC 251940
AAAGGTGTTT TCGAATGAGC AAGGCCATCC GCATCGCCCG TTTGACATCC GCCACCTGCG 252000
AGCAAATCCA GGCCCGCATG CTTGAGGCTT GCAGGCAGAT CGCTTCAGAT CACGGCCTGG 252060
TCATCGAAAG CGCCGGGTGG CGCGGCCTGG AGCCTGGCTT CTCGTTCGAA CCTGCATTCC 252120
GCATCAGCAT TCCCGCGCCA GATGGCAAGC CCCTCAATCT GGATAGGGAG ATGTTCGCCG 252180
TGTTGGCCGA ACAGTACGGT CTGGAGGCTG CTGACTTCGA ACGCGAGTTT ATCGCTGGCG 252240
GCGAACGCTT CCGGATCACC GGCATTGATC CGCGCCGGCC GAAATACCCT ATATCCGTCG 252300
AGCGTATTCC CGATCACAGG GGCTTCAAGT TCACCGCTGA CAACGTTGCC ATGCTGCTCA 252360
AAGCTCAGGC CAAACCCTGA CGTTCACCAA TCGTTCTTCA ACTTGGCCAA AATCACAGCT 252420
TCGAGCCGAC GGGGCCAAAT AGCGGGCGCA TGTCGAAAGA ACGCATCTCG AATCGAAAAA 252480
GTGACGGGAA TTGCGCCAGA TCAAATGAAG GTTAAAAGTA CGAGGTTGAT ATGCGAAGAG 252540
CTGGCGCAAG GAGGAACGCC ATGGCACATC ATCGCAATTG GCGGTCCCGG TTCGATGCAG 252600
TTCTTGTTCG GGCGCTAAAA AATGCTGTTA CGCCGAGCGA TCCGGCGAAG GCGACACCGC 252660
AAGCCACCGC GTCAGGATTT GATCCACCTC CGGCGGGGAG TGACCCGGGA GTCCCCTATC 252720
TTTTGCTCAT GCGGATGGAG GTGCTTCAGC TCGATCCCAC CCGAGTCAAA GCCTTGATGC 252780
CAGCCGACTT TCGCGTGCTG GCTCACGTTT GTGACTATTG TCAAGGTGCG AGCGGGATCT 252840
TCTCTACTTC GCAGCTGGCA AACCGACTGT CTATCCGTTG AGAGCGATGG GTTTACTTCA 252900
AACAGTTCGC CCCGATCGAC GCGTGGCGAC CGCCTAGGGC CAGCAACTGG TTAGCAGGGC 252960
GGTGCGCCGG CCATGTGCTC GCCTCCGCCG AACGACCTCG ATAGAAGTGC CAGTATGAAG 253020
TCTCACTGGA TCATTGTGCC GTCATGGTGG GCCAGAGGTC TTAGAGACCG CAGAGAGGCG 253080
CGCCAAATGA TCCGAGCGAC GGCAATGTCG GCAATCCACC GCAGCAGATG CAAGTGCCAC 253140
GCTGCACCTA CAAGGCACGC CCGCCGGTCA TGAGACGAAG CCAGCGACGA AGGTTATCAA 253200

CAAAAATCGG GCATTGTTTG GTGCTCTCTA ACGGTACGCA CATCCTGCGG GCGCGCCCTT 253260
CATCGCCTAT TTGGAAAACT TCCGGATCCC TCGAAGAAGC GACTTGAGTT CCGGTTCGTC 253320
TACTGCGCCG GCTGCTTCCT CTGGCGAGAA CCAATGAAGT TCGCGTTGCC CCCTCTCCGG 253380
ATAGTCTCGT TTGAGCTCCG AGACATTTAG CAAGTGAACT TGGACGAGAG CGGGCGCTAT 253440
TCCTGATCCC TTCAGTTTTT TGACATAGGT GTAGTATCCG AGAGGTTTCC TTTTCACCTG 253500
GCCCCGAACT CCGGCTTCTT CCCAGGCTTC TTGTCGGGCA ATCTCGTGGG GCTTTTTCCT 253560
GGGCATACTC CAGCCTTTGG GAATGACCCA GCGGCGACTG TCTCGGCTGG TGATGAGCAG 253620
AACCTGAACA TCGTTCTCTG CTCCCGGATT CGGCCTCACA CAGGCAGCGG CGTATTGAGC 253680
CATGACCTTA CACCTTCATC AGCTTGGATA AAGCGGAACG GTTCGTACTG TCAGCCGGCA 253740
TCACTCAGCC CGATTCCGAT TAGCATCCCG TCGAGGATTG GGTGTTCGAA GGAGACGATG 253800
TGAGCGTTGA TGCGCATAAG ATCGCGCAGC ATGTCGAGAT GCAACGAACT CGTCTGAAGG 253860
CTGTAGGCGC GACCATCGCA CAAGCGTTCC AGATGTGGCT CCTACCATTG TTTCTCCCTT 253920
TCTGCGAACC TCGACGCGGC GATAAAGAAC GATTGCGGCG AGGATCACCA ACGGCGACAG 253980
CCATTCGACT GCCGGTCGCG ACAATCCAGG CAGTGACCGC GGTCCCGGTA TTGGCGCCGA 254040
GCAGCACAAT TTCGCCATGC GCGGCTTGAC CAGTTCACGC TCGACGAAAG ATGCGGCCAC 254100
CAAGGCCGTT GCCGTTGATC TCTGCAGCAC GACTGTCACG ACCAGCCAAA TCCGCCGTTG 254160
GTGCCGTTCG GGAACCCAGT TTGGAGCTTC GCTCCAAAGA GGCTAGACAC GCCTTTCACC 254220
TGCGCAAGGC TGAATAGCGG CAAGGGCTAC AGCGCCGAAA AAAACCAGTA CGAGAATCGT 254280
AAGTTCCATG TCTCCCTCAC ACTGAACAAA AAGCGCTCAG CGCATTTTTG GACTCCAGCC 254340
AGACGCAGCA TCCCAGTGGG CGAGCGTCGA TCCGCGTCCG CCGTCATGGA TCCCGCACCT 254400
CCGAAAAGGC TGCGACGTTC GATCTCGGGG GGATAGCTTG GCAGTTCCCG AGAGGATGGT 254460
CCGCGATGTT CAAGAAACGT GCCAACTGCC AGGAAGGAGC TCGGAAGAAC AGTTCCGCTA 254520
CAGTTCAACG GCTTGCGCTG GAGAGTGCGG CGTCGCGCGC CTGGCCGAAC ATTGTGTCGC 254580
GCACTTCACA GGAATAGCGA ACGGGTCGCC GTGTTAGCGG CAACGCGCAG ATAGTGCAAA 254640
TCCAGCCGCC GGCAAGGGCC GCTAGCGCCA CCAAGATGCA AATCGCTCCG ATGGATTGCG 254700
GCGACTACGC CCCGCACGAT ACCCGCCTGT CCGGCCGGTT GCATGCCGAT GGCAAAGGGC 254760
TCGGCATGAC GCCTTCCGCG AAGGAGGCGT TGGCGGGGCG ATAGGTGAGG GCGGCCGGAT 254820
GTACTGCATA GTAGGCGAGT ATGCCGTGCA CCGAAGGCCT CGCCCAGAAG CGCACGGCAG 254880
CCGAGCTTGT AAATGCCGAG CTTGTGGGTC TGGCAGGCTG CCACGGACAC GGTCACCGAC 254940
ATGAGCGATT TGTGGAAGGT GGCGGATGGC CGCACTCCTG TAATAGCCTC TGTCTTAGAG 255000
TCATCCGGAG GCGACAAGCG TGTCGCAGTT GCGACATTTC AGTGCCCGCC GCGTCTCGTC 255060
GGGAAAGATT TTAGAAATAG GCCAGGCGGG AGTGGTGGTT GATTGGCCGT TACACGTATT 255120
TCCAACTGAT GGCCCGGCCA CTGCCTTGCA AAGTGCGTGA CGGCGCGACT CCTTGATCGG 255180
GCGCCGGCAT CAGGCGTCGG GCGTTGAAGG CGGCCGGGAA GCGCGGTCAT TGAGGCCAGC 255240
TTCGTCCAGC CTGTCTGTAC TTCAACGTGA GCCAAATCTC AGCTTCCAGC CGAAAGCCTC 255300
CGTGCGACAC ATTGCCCTCT CCGCGCGATA AAGCGTTTCT TCCTGACTAC GGTTGATCTC 255360
GATCAATATG CCGCCGCTGC GCACAGTCTA CCGTTCGCCT TGCAACGATT GGTTCCGATC 255420
CAACTTATGC TGGTTCAAGG AGAGGTCAGA TGAGCAGGAA TGATGCACGG TATCTGAGAT 255480
GTACCGCGGC ACTGGGTGCG GCTTTCTTCG CATGCGGGGC AGCCGCGGCG GAGCTGACAT 255540
CACCGCCTGT TGCCCCGGCG ACAGAGGGTG TAGCGGCCGG GCTAAGTCCC TGGCAGATCC 255600
GCGTGCGCGG TCTGGGCGTC CTCACTGAGG ACAGTGGCTC AATCAACGGC GTGCCTGGCT 255660
CGGACCTTTC CTATTCGGAC TCGTTGATCC CGGAACTCGA CATCAGCTAC TTCTTCACCG 255720
ACAATATCGC CGCTGAACTT ATCCTTGGCA CGACCTATGC CAACATCAAG GAAGTTGGCG 255780
CGGTCGGGGT TCCGGTCGGG AAGGCCTGGC TGCTGCCACC GACGCTTACG CTGCAGTACC 255840
ATTTCACCGA CTTCGGCGCC TTCAAGCCCT ATCTCGGCGC GGGGATCAAC TACTCGCTGT 255900
TCTACAACCA GAGCGAAAAG GCGGGGTTCC ATGACCTCGA CGTCGACAAT CATGTGGGCG 255960
CTGCGCTGCA GGTCGGTTTC GACTACATGG TCGACGAGCA TTGGGGCGTG AACTTCGATG 256020
TGAAGAAAAT CTTCCTCGAA ACCGAATGGA AGGCTGATCA TGACGTTCTC GGCCCCCTGA 256080
GCGGCAAGGC AAAAATCGAT CCGTGGTTGA TCGGCGCAGG CGTGACCTAC CGTTTCTAAT 256140
AGACCATTAC TGCCGGAGAG GGAGACGCAG GCACCACCCG TCGGGCGCGT CCCCAATTGT 256200
AAACGGTCTC TATGCAGACC CTGAAGGCGG GTCACGTCGC CCTCTGTTCC CTGGAAAATC 256260
CAGAGTGACA GGTGCAAAAG ATCGCCGACT CCGTGCAATG TCCGCGGCGC TTCCGTCGCC 256320
TCCGGATGTA CTCTCGATAA CCGGTGGACT TTCGTCCCGC TGATGATCCG TTAAAAGACA 256380
CCGTTTCGCC GGAACCGTAC GTCAGTCAAC GATACCCGAC TTTGAAACGC ATTCATCTAC 256440
GCAGTGGATC TTTGCTATCC ACACAACCGT TTTGGCAATC TTCCGAACGC CGGTATAGAG 256500
AGTTGACTTC AACTGGAGGC AAGGGCGAAT GTCTATTGCG AGCCGCTGTT AGAGGTGGTC 256560
TGATTGTCGA ACTCACTTTC AGGCCATCGG TCTATGACGA TCCGCCGAAC CACTGCGAAT 256620
TTGCCCTTTT GTAGGCCAAG GATGTGATGC GCGGGAATTC CGATCATCGA GATCGATCCG 256680
AAGCGCGCGG CCGCAGGCGG CGCAATCGCG CATGTCGCTG CTTTGCGCGA CGAACGGCTG 256740
GCTTGCCTGC AAAAGCTCAG AAAAAAAGGC CACGAAGTTC GTAAGTCTGC AGTAAAGGAG 256800
TTTCATTTCC ATGGTTTTTT CATTTTCCAC GTTCAATCGG CTCGTCACCT TCACCGTCAT 256860
GGCCGCCATC GTCAGCGTGC GGCCTTTGAC GGCGGAAGAC CTGTCCTCCC AGCAGCAGTG 256920
GCCGGGAACC CCCTTGTCAC GAGTGGAAGC CCTTGCGGTT CTTCAGACTC TAAACGCGAA 256980
TCTCCTCAGC AATGCCAGTG CGACGCTCAC GCTCGATCGC TGGTGTGAAG CGCATAGACT 257040
TGCACCACCC GGTTCGAAGA TCGTAGCAAA ACATGTACAG GGACACGACA AGCCAGCTAA 257100
CGTTCATATT CGTAAATTGC TGCACGTCGG GCCTAACGAG CCGATCGTCT ATCGCCATGT 257160
CCGCCTAGCA TGCGGTGATC GCATCCTTTC GGAAGCAGAC AACTGGTATG TACCCGCAAG 257220
ATTGACAGCG GAGATGAACG AGGTGCTGAA TACGACTGAT ATCTCGTTTG GTCGAGCCGT 257280
TCGATCCTTG AACTTCACCC GAACCAACCT AACGGCCAGA CTGCTTTGGT CCCCTCTTCC 257340

GGAAGGCTGG GACATGTCAG CCGAATTACC AGTGTCGAGC TCAGGGCCCT TGATTCCACC 257400
GCCATTCCTT CTCGAACATC ATGCGGTGCT AAAGTTGCAA GATGGCACGC CTTTTAGTGC 257460
CGTGGTCGAA AGCTATACGA ACAATGTGCT CGATTTTCCG GCTCCGCTGC TATATCCGCA 257520
ATAAAGCCTA GTGGATTGGC TGGCGACTCG GCGTTAGCAC GGAATCCGAT TGAAGCCGGA 257580
CGCCATTCGG ATCGGACACC GGACAGGATT CGGATTGTGC CGAGCAGTTT GGAGTGATGA 257640
GGCCTCCGCC ACCGGCGTCT TAATCGTTGA GCCGATCGGA AGGTGCGTGA ATCAGCGCTC 257700
GCTTGCAAGT TTCCCACGCC TGCTCAGCGA ATAGTCAAGC GTGTCCACGC TGCGCTCCTG 257760
CCCAGTCCTG ACATTTCGAT CTCTGCCTAC TGAACATCAA TGAAACCACC TTTGCCCCCG 257820
CTATCGACTT CGCGACCGAA CGCTGGAGCG CCGACGGGGT CTTTGAGGCA AGCGGCAGCG 257880
CGAATGGCTT CGAGGGGCTC CTCGATCTGG TGCGGCCGGG CGCGTGCCGT GGTCCTGGTG 257940
GGCCTGCCGG TCGATGTAGT GCGTTTCGAA TTGCCGGGCG TGATCACCAG GAAGGTACGC 258000
ATCGAGACCG TATTCCGTTA AGCCAAATCT TCGACGCCGT GGATCGCCGG GCGCCCCCCT 258060
GAAGTCACCC CGCATGGCCC AGATGCGGAA CAATTATGAT TGCGGCGTGT TCGTGGTGGA 258120
CGGCACGCGT GCGCTGGTTA GCATATTGGC GCAAGGACCA CGACCAGCGC ACGAGCCGCT 258180
GCACCTCGAC AACCTCGTCG CCGATCGGCA GGCACTTCAG GACCGACTGA GGGCTCATCT 258240
TGGACCGATA GAATTTGGGG AGCGGCTTGA GCGGCTGTCC TGCGGCCATG GCGCCCAGCG 258300
CAATTTCGGC GGGACGCAAC AGCCCGCACC ACATGACCCT GTTGCGGGAG GACTCCCACC 258360
GTTGATCCCG TTTTGAGTAT GGCGCAAGGA GGCGGTCGGC CCAGATCGGT GGGCCGCCGC 258420
ATGATGTTAG GGAGCCTTGG CCGCATTCTG ACGACGATGC AGCTCTTGCC GGCGCACTTG 258480
TTGGTCAGCG CGCTCCTGTC AAGCTGCGCG AGGCGGCGTA GTCACGCCGC TGCTGGCATT 258540
AAACGCTTCT GCGAAAACGA AGACGTTCCT TCATCACCGA ACACTCTTCT CCACGGCATC 258600
AACACCTCCG GCCAACAAGT GTTACTCCTT GTGTCCGGTA CGACACGTCG CCTATGTTCT 258660
TGCAGCATCT CCGCGTCGAA GCCTGACTTA TCGGCGAGCG TCGGACGCGG GGGGAGAAGA 258720
AATACTATGT TGCTCACCTA CCCGCCAAGG CAGACCTGCG CACGTTAGCT GCCACAATCA 258780
AGTCCTGACG GAGTTGCTAA CAGGCCCATC AGCAGCTCAA AGAGGAATTC GGACTTGATC 258840
AACTTCGAAC CGCGATCCTG GCAATGGCAC GGCCCTCATC GTCACGCATT CATGATTATA 258900
GTTGATTTAC CTTCCTTCAA CATCGGCGTC TTGCAGCGGA AAAAAGACCA CTGAGCCGCG 258960
GCATCCCGAT CTCGCCTCCG GAAAGCCATG GCCTATTCTT GTGCCCGATC CAGACGTGGA 259020
AGACAGCGGC TAAATCGACA ACCTGACGAG TTTGGCGCGC CCTGCAACCG AGTCCGCAAA 259080
GTGATTTATC CTAGCTGAAT CGATGTAGAG GTCTGAGGTG TCTTCCAATA CTCTCGCTAC 259140
GGCTTCAGCG GTGGAGTCGA CCAAGTTTGT AATTCGGCGG CGCACCAAAG GTAAGCCAAC 259200
TCCAGCTTCC TTTGCAAAAG TCTGCCACCC ATCAGCGTCC ATCTCGGCAA GCGTGACCCG 259260
TTTCCCGATC CTCATGGCCA TTTTCGGTGA AAGGTCTGGA TAGGCGACCG TAGAAAGCAG 259320
ATCGTACAGC GGGGCCATTT TCGGTCCCTG ATCGTCGTAG AGGATCGAGA AATTCTTGCC 259380
GTGGGCATCG GCATTTCCGA CAACCAGGTT GAAGATCGCG GCGTCCAGCA GCTTCAACAC 259440
ATCTGTAGCG GGCCGCGCTG ATACTCGCCG CAACAGCTCA AAGCAGTCCT TGAATGTCGG 259500
CCCGCCCTCG CTGGCGTATT TCGTTTCAGG CGGCACACCA AGTGCTTGGC AAAAGTCTTC 259560
CTGGTGGATG CGGTGAACCA CACCGTCGGC ATCGCGATAG CGATCGTATC GCTCGACCAC 259620
AAGAAAAGGG CGCCCATTCG CAGACCTCGG TTCGACTGGT GCAACGTCGA GGCCGATCGC 259680
TGCAGCGAGT CTCATCACGA ATGCTTCGTT TTCGGTTGTC CCTGGAAAGC GCGCGATTGG 259740
TGGTTTCAGA ATATGTGTTG TTGCTTGTCC GGAAACTGGA AGCGCGAGCT CGCCGTCAAT 259800
CAAAACAAGC GGCACCTTGG ACTGCGCGCC GGCTAGCGAT AGCCTCAAGC CTTCCTGGCC 259860
GGCCAATAGC GGCCGCGTCG GAAGTGCGTC CAGTATCCGA ACGATTCCGG CCTCATCCAG 259920
AGGTGTCGGC TGCTGGTCTG GAAGCGGCCC TGCCTCTATG GGCTCCTGAT CTTCCGGCAA 259980
AAGCTGAAGT GCGCCGGCGA CGTCGCCGCC CAAGCGATCA AGGAGCGCAA AGTCGTTCGC 260040
AGGTGAAACG CCCAATGCCT GCGCTGTCAC AAGACGCTGG CTTTCCTCGG GCAACAGGCC 260100
GCCGAAAAAT GGTCGGCATT CACGACGAGA AAAAGGTTCT GCGCGCTTCG GGAGGGAGGC 260160
AGACAGCGGA AGCGTGTTTT CGTCGTCGAG CCAAGCTTCG CTGTAGGCAA AGCCGATATC 260220
ACCGTGCCTG TCCTGGGTGA ATTGCCCGAC AATGCGACCA TCCCACCAGA CGTTGAGAAC 260280
CCTTACCGTC ATTTTCTACG TTCACCAGGA GCCAGAATAT CGATCGAACA GCCGAGTGTT 260340
TGCAAAACCT GCAACACTTT TCCTATCTGA GCTGTCGGTT TGCCCGCTTC GAGATCGACA 260400
ATGAACCGGA GGCCAACGCC GGCAACGCCC GCAAGCTCAT CCTGCCGCAA GTTTTGCTCC 260460
TTGCGCACGA TGCGGACTAG AGCGCCGATA TCGGCTGGAG TTTTGATGCG CTTCGCCATT 260520
TTTTTGTTTT CCCGCTCGGG AAAGTAGCAT CATATCGGCC GCCCTGTCGA GTGAAATTTC 260580
CCGGTCGGGA AAATTTATGC CCGAGAAGGC GTATCGCCAA TGAAATTTAC CGCTCGGGAT 260640
TACGAAATTG AGTGGCAATT TTGGTGGCAG GCTGAGTTTT CGTCCCGTCC TCAAATTCTA 260700
TGAAGCGCAG TATAAGCCTG GGCGGCGTGC GGGCTCAGAT TCCGCCGCGC CGTTTCTGCC 260760
TGGACTGCCT CCCTTATCTT TAGGCCGCTG AGGGGCGGCG CCTCACGTGC GGCCTCATCG 260820
ACGACGGTAG CCTTTCTCGC GCCCGCAAGT GCAGCTCAAA GGCGGCTTGC GCCTGGCCAG 260880
CTTGCTGATC GTCGGGGAGG GCATGCTGAA CCTGCATCGA AGCGCCAGTT TGGTTGGCGA 260940
AAAATTGTCC ATGCTAGTCT CATCTCCGCA GATCCCGGCG GCACGCTACG CTGGCAAGCT 261000
GCCGAGAAGC TGACGAGCCT CGCCGCGCAG TGTAAAAAAG CCGCGTGCCA ATCCCATCGC 261060
GGACACGAAA AAAGTCCTGG ACGCAAGCGT GCCGCTGAAT ATGCGCATCA AGCCTGCGGT 261120
CCGCAACCTG AGCCCGCGCT GCCGAGCTGC TTGGGAAGAC ACGCACTAAT TTCATGCCGG 261180
AGGTCTCTGA GCGTCGCGCC GAGGAGGCGC TGCTCGTCGC GCACTTCGTT GCCTGCGAGG 261240
GCAACCGCGT CATCGCCTAC TGCGCACTCG CTTCCGGTGC GGTAAAGCAG CCGAAGCGGC 261300
TGGCGGTTTC CGGCGCAATA TGCCTGACCC GGTTCCGGGT GCCGTCCTCG GCCGCCTCGC 261360
AGTCGACCCT CCTATTAGGG TCGCGGCATC CGTAGGGCAT TGGTGCACGA CGCAGGCCTG 261420
CGCCTGCTCC ATGCTTTTGA AATCCTCGGC ATTCGCGGTG TGCTGTTGCA TGCGATTTCC 261480

AGTGAGCGCG AGCGTTCTAG AGGCGATCGG CTTCCTGCCT TCGCCGTCCG ATCCCACGAT 261540
GCTGACGGCC GGGTTGCATG ATCTCAGCAG TGCGCTGAAT CCCTGACGAT CTTTAGGCCG 261600
CCCTCACAAT TCGGATGTGG TTTTGTAATC GTCATTAGAA TGTGGCGAGC ACTGTAGCGG 261660
TCTACCCCTT TGCCGAATCA ACGCTGTCGC CGGGCACGGG CGAAGCCTGT AGCACCGCTG 261720
CGGAAGCGTA AGCCTCAGTC CCCGCGTTAT ACTCGGTAAA ATGTGGCCGC GTTGTCGTGG 261780
AACAGTGCCG AGCGTTCTGA GACCGAGAAG CCCACGGTGA TCTTCTTGAA GGCATTCCAA 261840
ATGGCGTCGT AACTTGAGAA CAGCTTATCG ACGGGGAAAT TCGAGGCGAA CATCGCCCTG 261900
TCGACCCCGA AGGCGGCAAT CGCCTCCTCG ACATATGGGC GGATGCTCTC GCTGGTCCAG 261960
TTCCAGTTCC CCATACCGAG GCCAGAGATC TTGCAGGCGA CGTTGGGCGC TTGTGCGAGT 262020
GTCTTCATCG CTTTCCGCCA GCCCTCAAAA TGCGACGGCC CGTCAACCTG CATGCCGGTA 262080
TGGTTGAGGA TGATCTGCAC GTCTGGGAAA TCACGGGCGA GTTCAAGGAA TTCCTCCATC 262140
TGCCAGTAGT AGAGCTGGAG GTCGTAACTC AGGCCGCGAC GAGCCAGCTC CTTGAAGCCC 262200
TGCCGCCATT CGGGCGTCCT GCTGACCTCG GGCTCATTCA GGTAGGTCTT GGCGCCATCG 262260
GTGTGAAAGT TCATCGACTG GCGGATACCG CGGAAGTTCG CGTATTGCAT ATGCTCATCA 262320
AGCAGGTCGC CGACATCGGG CTTGCGGAAG TCCGCATAGC CGACAATGCC GTGAGGGAAG 262380
CCATGCTTGT CCGCAACGCC TTGGAGCCAT TTCGTTTCGC CGGCGGGGTT CGTGGGGTCA 262440
AATCCGACAT CGAGATGGAC CGCCTTGACG AGGTTCTGGT TCTTCGCGTC CGCGAGGAAA 262500
TCCTCGATCA GGTAGGTCTT GTTGATAGCT GTATAATCGC CGAAGGCTGA AGGCTTAACA 262560
CCGTCGGAAA GCCAGGGGTA GTAGTTGGTC TCCAAATCCC AGAGGTGGAA ATGGGGGTCG 262620
ATGATGGCGA TGTCGGACAT GGTATCTCCT CGGGGTCGCG GGAAACGGCA GGCACCGCCG 262680
TTCCCGCGAG TCTCCTGTCA GTTGCTCGGG GGAAAAACGC GGGCGATGAC CGCCTTCGCT 262740
TCCGCCGTAT CGATCTTGTC GGCGGTCACG AGCGGCATGA CAAGCGCAAG ATCCTTTTCG 262800
ACCGGAGCAT TGGTCAGAGC GTTGAATACC TGCAGCGTGC CGTCGATGCC CTGTCCCACC 262860
GCCTCCTGGA AGAAGATCCC CTGGATGGCG GTGGCTTTCA ACAGTGCAAT GGTCGTCGGG 262920
CTGCCGTCTG CGACCGCGAT GCCGATCTTT CCGGTCAGGC CGCGGGTTTC AAGCACCTTC 262980
GCCGCTCCGG TCCCGGCCTC ATCATACATA CCGTAGATCG CCTTGATGTC CGGATGGGCC 263040
GTCAGCATGT CATTGGCCTG CGTCACCGCC TCATTGACGG TCAGCCCACG CGTCTCCAGC 263100
ATCTGGACAA GTTCGCAGCC GTCGGCTGCG AAGGCCTCCT GAGCGCCCTT GAGATACTTC 263160
TGGGCGTTCT CGCGGTCCTG CGGAAGGGAA AGCATACCAA CCTTGTTGCC GCCGCGCTCC 263220
TTGGCGAGGG CGCAGACGAA GCTGCCTTGG GCTTTGCCAG TTTCGTAGTT GTTGGCGGTG 263280
ACCGAGGACG TGTAGTCCGT CTGGCCGGGC TGCGGGCCGA TACCCGCGAA AGCGATCGGT 263340
ATGTTCTGGG ATTTCAGATA GGCGAGCAGA GGCGGGGTGC TGGTCGAGCT CACCGGACCG 263400
ATGACGATCG CGTCGACGCC CTTTGTCACC GCGGTCCGCG CATTATCCAT CTGCTTGGCC 263460
GGCGAATTCT CGGAGGTGAA CTCGACATAG TCCATACCTA GCTCTTGGGC CTTCTGCTTC 263520
ACGCCGAAGC CGACCCACTG CCAATAGGAA ATGTCGAGCG ATGGCGCGAG ATAGGCGACC 263580
GTTTTCCTGT CCTCCGCATG CGAGACGCTC GACAACGCTG CCAGCGCTGC ACCCGCTGCC 263640
ATGGCGAGCC ACCCGTTCAG TTTCTTCCTC ATGCCTTCTT CCTCCTCTTG ACGTCCAGGT 263700
CTCCCCCGGG ACGGATTACA CGGCACCGCG CCGCGATTTG CTGAAGCGAT CGATCAGGAC 263760
CGCGATCAGG ATGGCGAGGC CGGTGACCGA TCCCTGCCAG AAACTATTGA TGCCGATGAG 263820
ATTGACGCCG TTCTGGATGA CCGTGATCAT GAGCGCTCCG AGAACCGCGC CCACCGCCGT 263880
TCCCGTGCCG CCAAGCAGGC TTGCGCCGCC GATCACAACC GCGGCGATCG CCTGCAGCAT 263940
GAGGCTCGAA CCGGCCGTCG ATTCCGCATT GAGAATGTAG GAAATCGTCA GCAATCCGGA 264000
GAAGGATGCG AGAAGTGACG AGGCAACATA AGCGAAGAAC TGCGTCCGCT TGACGGGAAT 264060
TCCCAGAAGA CGTGCCGCTG CCGCGCTGCT GCCCACGGCA TAGAACCAGC GCCCCGCGAC 264120
CATCTTCTTG AGAAAGATCT CGATCACGAC GAGCAGGACG ATGCAGAACA GGATGTAGTT 264180
GGGCACGCCG GGTATCAGGC TGCCGCTGTT GAGCAGCCAG AAATCCGGAT CGCCGATCGG 264240
CATCGAGCGT CCATTGGTGA CGATGAAAGC GAGTGAGCCG GCCACGGCAA AGGTGATCAG 264300
CGTGACGACG AAAGGCGCGA GACCGGCCAC CGTGACGAGA AAGCCGTTGA TCGAACCGAA 264360
CACAAGCCCG ACCCCAAGCC CAATCAGCGC GGCGCTGACG TCGAGACCTG AGGCCATAGC 264420
CTGTGCTGTG ACCATGCCGG TCAGTGAGAA GACCGAGCCG ACAGAAAGGT CTATGCCGCC 264480
GGTAATGACG ACGAGAAGCA CGCCGAGCGA CATGATGATC AGCGGTGCTC CGGCCTGTGT 264540
AATATTGGCG AAATTGCCCC AGCTCATCGC CTGCGGCACC TGTGTTCCAA CCGCGAGAAC 264600
GAGAAGGACA ATGGCCCCTG CAATCGCCAA TTCCGTACGG TAGGCGGAAA CGAGGCCTTC 264660
CCGAGTTCGG ATTGCCCCTG GCGTAGCCCC GGCCTGGTCA ATAATGGTCT TCGTCATGCC 264720
GCCATTCCTG TCAATGTCGC AAGCTTGTCT TCGCTGAGTT CGTCCCGCTC GATCACACCC 264780
GATGAGCGCC CCTCTGTATC GAAAGCGAGC ACCTGGTCGC AGATCTCAAG CAATTCCGCG 264840
TTTTCGGTCG ACCACCAGAC GATTGCCGTC CCCGTAGTCG CCATGTCCCG GATCAACTGG 264900
TAGATTTCCC GCTTGGTGCC GATATCGACG CCGCGGGTGG GTTCTTCGAG CACCAGGAGG 264960
CGGGAAGGAA CGTTCAGCCA GCGGGCGAGA AGAAGCTTCT GCTGCGTGCC GCCGCTCAGC 265020
GTGTTCGGCA GGTGCCAGAG CGAACCGGCT TTCACTTTGA GCGCCTGCAT CAGGTCCAGA 265080
CACTCGACCT CTTCCCGCTT GGTGGCAAAC AGGGAACCGC GCGTCGCGCG GCGCGATGCG 265140
AGCACATTGT CGATGATCGG CAGGGAATGG AGGATGCCGC GGTGCGACCG GTCCCCGGTT 265200
ACGAAGCCTG CTCCGAGGCG CGCGGCCTCT CGCGGCGAAC GGAAACGGTC GGGCCATCCG 265260
GCGCGCGTTA CCGTCCAGCG CCTTTCGTGC GCGGCGCCAA TGAGCGCGGC GATCAGGGTT 265320
TCGGGGCCCG CAGGAGCGCC GGCAACACCG AGAATGGTCC CAGGCTGGAG CGTTACGGAA 265380
AACCCTGTTT CCGCGATCGT CAGCGGTTCA TCGGACAGAG ACGCCACCGG TGCCGGCCGA 265440
GCGGAATGGG CAACGGCTGT GGCTTGCGCC TGCCCCATAT GCTCGATGAT GGCGAGATCC 265500
GACAGCTCTG CCGTCGGCAC GTTCCCCACC ACGGTTCTTC CGTCCCGGAT AATCGAGCAA 265560
ACATGGGCGA TCTGGCGGAT TTCCTTCATG CGATGCGAGA CAAAGACGAC CGAGAGGCCA 265620

TTTCCCCGTG TCAATTTTCT CAGAACCCCG AACAGCCGCT CCGTCTCGGC CGCGGTCAGA 265680
TTGGCGGTCG GCTCGTCCAG GAGGATAAGT TCGGCCCCCG ACGACAGCGC TCGCGCAATC 265740
TCCACCATCT GTCCCTCATG GAGGCTGAGG TCGCCGACGA GACGGTTCAG CGCGGTACCC 265800
GCAAAGTCCC GATCAATCAT CGAAAGGGCG GCATAGGCCT GGCTGCCGGC CGTCCTTCCA 265860
TCGTAAACGC GCGAGCCCTT GCGGAAATGA GGCAGTGCTA TGCTCTCAGC AACCGTCAGG 265920
TGGGGAAGAA GCGCGAGTTC CTGGTGGACG ACGGCAACCT TGCGGCGACC GTCCGCTTCG 265980
GTGAGGATGT TGCCGGCCAT ATCCCGGAAG ACGAGTTCGC CACCATCCTT TGCCGCCGTT 266040
CCCGTGATGA CGCGGATGAG CGTCGATTTG CCGGCACCGT TGCCGCCGAG GAGCGCGTGT 266100
ATTTCGCCGC GCGCTACCGA AAAATCCACG CCTTTGAGCA CGACTGTCGC ACCATACGAC 266160
TTGGTAAGTG ATCGGACCGA GACGATGTCT TTGCTGGCGT CGGTCATAGT CCTGTCCTCA 266220
GTAGAGCGTG AGCGCCGGGA TGAAGCTCAC CGCAAGAAGG AGCGTCATCG CCATGGCAAA 266280
GAAGGGCCAG AGTTCACGGG TGGTCTCGCC GAGTGGTGAT TTGGCGATCG ACGAGGAGAT 266340
GAAGAGCGTC GTGCCGATCG GGGGGGTGTA GAGCCCGATT CCAAGATTGA TCACCATCAT 266400
CAGCCCAAGC TGGACCCGGT CCAGACCGAT CGTGTCGGCA AGGGGAACGA AGATGGGTCC 266460
GAGCAGCAGG ATGGCGGGCG GCATATCGAG CGGCATGCCG ACGATCAGCA TCAGGATGTT 266520
CATCATTAGG ATGATGACGA TCGGGCTCGA GATGTTGGCG GCGACCCAAT CCGCCATCTG 266580
CTGCGGCGCC TGGTCGAAGG TCAAGACCCA ACCCACGGCG GCACTGCCCA TGATGACCAG 266640
CATGACCACG CCGGTGGCCA TGCCGGCCTC GACCACGTTG TCGCAGAATC GCTTCCATGT 266700
CAGGTCGCGG TAGTAAAGAA GGCTGAGTGC GAGCGAATAG ACGACCGAGA GCACCGCGAC 266760
TTCCGTGGGC GTGGCCAGGC CAAACCGCAG GAATAGGATG ATCAGCACCG GCAGCAGCAC 266820
CGCGGGCAGG CTCTTCAGTG CCAGCGTGGC GAGCTGCCGC CTGCTGGTCT GCGAGGGCTG 266880
CGAGGCGTAG CCGCGCCTCA CCGAAACGAA CCAGCAGACG AAGACAAAGC TCGCGGCCAT 266940
CAGGATGCCC GGCAAGATGC CCGCGACGAA GAGGTTGGCG ACACTGACGC CGCTTACCAG 267000
GGAGAAGAGG ATCATGGGAA TCGACGGCGG TATCAGAACG TCGATGACCG CCGAGGTCGC 267060
GTTGTTGGCC GCGCAGAGCG CGGGCGGAAA GCCGTGCTTT TTCTGCCACG GGATAAGAAC 267120
GGATCCGAGG GCGCTGGCAT TCGCCACGGC GGAACCGGAG ACGCCGCCAA ACACGACGGA 267180
AGAAACGACG GTGGTCGACA GGGGTCCGCC CCGCCAGCGC TGCATCGCAT GCGATGCCAG 267240
CTCCAGCAGC TGCCGGCCGA GCTCACCGCC AAGCATCAGC GTGCCGGCCA GCATGAAGAA 267300
CGGCAGCGCG AGCATCGGAA AGGATTGGGT CTGGTCATAA ATCTGCTGCG CCACAATCGA 267360
AAGCGGCAGA TAGCCGTTGA ACAGGACCGC GACGCCGGCG GCGATAATCA GGGCGTAACC 267420
GACCGGCACT GCCAGCATCA TCAGTGCGCA GAAGACAATG ATCATCACGA TCGTCATAGC 267480
GCCACCTCGC CGCTTTCGGT ATTCGTCCGG TGGTCCCAAC CGAGGCGCAG CACGCGCAGA 267540
GCGACAGCGA GCGTGACGAT CGCGACCAGC ACGGATCCTA CGGCAAGGGC GAAATACCCG 267600
ACGCTGTTGG GCAATTGGAG GACCGGGCTG TGTTCGATCC CGGCGATCTC GCCAACAAGA 267660
TAGGCCTGGT AGCCGAGCAT CAGGAAAGCT CCGGCGCTGA CGAGATTGGC GGCGACAAAT 267720
GCAGTTGAAC GTGCGCCATC CCGGAGTTTG TCGTAGATCC ACTCGACCGC CATATGGCCG 267780
CCGGAATGGG CCGCCAGCAC GATGCCGCCG AGGATGAACC AGGGAAAGAT CAGCACCGGT 267840
AGCTCCTGAG CGAATGAAAA GCCTCCGCCG GCCAGCACGT AGCGGGCAGC GACATTCGCG 267900
GTCATGAAGA CCATGAGTGC GATGCCGCTG AATATGACGA CCTGCCGCGC GAACCAGACG 267960
ATGGCCCGTG CCGTCAGCTC AACGAGATTT GAGAGGCTTT TCATGGGATG CTCCCGCGCC 268020
GACAAGGCCT CAGCCTTTGG CTGCCGATTC GATCTGGGCG ACGAAATTCC CGAAGGGCTT 268080
CTTCTGCTCT CGACAACAGA AGCAGTCGCC TTGGCGAAGC CTTCGCGGTC CACCTCGGTG 268140
ACCTCGATCG CGGAAATGCG CCTGAAGGCT GCCAACACTT CTGCGTCCTT GTCGGCGGAA 268200
AGCTTGCGCT GCAGTTCGCC GGCTTCCTTG GCCGCCGCCT TCACTGCCTC GAGATCGCCG 268260
CCTAGACGGG CTTGTGCAAT CTGCGACATC AGGAACGGCG TCGATTCCCA TTTATGGCCG 268320
GTGAGACTGA TGTACTTGTT TACCTCGTAG AGCTTGGAGC TATCGATATT GGCGAGCGGG 268380
TTCTCCTGTC CGTCGACCAC GCCCTGCTGC AAAGCCACGT AGAGCTCGCC GAAGGCAATC 268440
TGCTCCGTCG CCGCACCGAG AGCCTGAAAA ATATCCATGG TCATCGGGTC GGCGGGCGTG 268500
CGGATCTTCA TTCCGCTCAC CTCGGCGGGG GCTGCGACCT TGCGCTTCGA ATTGGTGAGG 268560
TGGCGAATGC CATTATCCCA CCAGTCGAGC GGCACCACGC CGACCGCTTC GAAGCGCTTG 268620
ACGAGTTCGG CGCCAACGGG CCCACCGAGA ACCTGCATCG CCTTCGCCGT GTTCTCGAAC 268680
AGGAACGGAA GACCGAGCGC GGCAAGCTCC GGAACGAGCG CGGACGTCGC TCCCTGGCTG 268740
TTGGCTGTGA CATCGAGCGC GCCGGTGCGC AGGCTCGTCA GCATCGCGGC GTCGCTGCCG 268800
AGTGTTTCCG CGCCGGCCAC ATTGATGGTC ACACGACCGG CTGTCTTCTC GGCTACCAGT 268860
TCGGCAAACT TCGCGGCCGC GACAGTTCGC GGATTTCCCG GTGCGGCACC ATGGCCTAGG 268920
GTCAGTGTCG TGCCGGCCCG TGCGGGCCGA ACCATCGTGA CGATCGCAGG GGCCGCGAGC 268980
ACGGCCGATG ACGCGGCCAG GAAGCGGCGT CTGGTTATCT CGTACTTCAT TGTCTCTCCT 269040
CCACTTTGAA ACTGTTGCGG CCCCGATGCT CCTCCAGCGC GGGTGACCGT TTTCGTCACT 269100
CGTCGAGCCA CTCCTTGATC TGGGCCACGA TTTTCGTGGT CGATATGCCG TACATGTCGT 269160
GCAGGGTCGG CAGGGCGCCC GCTTCTAGAA AGGCGTCGGG CAGTCCGATC TGCCGGAATG 269220
CGGCGGGACG CACAGCGGAC CGCATCAAGG TGGCGGCGAC CGCTTCGCCG AGGCCACCGA 269280
TAACGGTGTG GTTCTCAGCC ACGACCACAA GCCGTCCCTG CCTGGCGCAC TCAGCGAGGA 269340
TCGTTGCCTC GTCAAGCGGC TTGATCGTCG GAACATGAAG AACGCCGGCA TCAATGCCGT 269400
TCTTGCGAAG TTCCTCGGCC GCTTCGAGCG CCCGCATCGT CATCAGACCG GAGGAAATTA 269460
TCAGTGTGTC CCTGCCATCC CGCAGCAGCT TGGCCTTGCC CAGTTCGAAC CGGTAGCCGT 269520
ATTCCTCAAG CACGAGTGGA ACATTGCCGC GCAGAAGCCG CATATAGACC GGACCCTCGT 269580
GGGCAGCGAT GGCCGGGACC GCCTGCTCGA TCTCGCTGGC ATCGCAAGGA TCGATGATGG 269640
TGAGGTTCGG CATGCCGCGG AACATGGCAA TGTCCTCGGT CGCCTGATGG CTCGGGCCAT 269700
AGCCTGTGGT CAGTCCCGGC AGCGCGCAGA CGACCTTGAC GTCAAGCATC TCCTCGGCGA 269760

TCGCGAGGCA GATGAAGTCA TAGGCGCGGC GCGATGCGAA GACGGCATAG GTCGTCACCC 269820
AGGGCTGCAG CCCTTCCCGT GCCATCCCGG CAGCCGACAT CATCAGCAAC TGCTCGGCCA 269880
TGCCCATCTG GTAGAAGCGA TCGGGATGGG CCGCCCGGAA GACATGCAGA TCCGTGTATT 269940
TGGCGAGATC GGCCGAAAGA CCGACGACAC GGTCGTCCTT TTCCGCCAAG GCGGAAAGCG 270000
CATGTCCGAA AGGCGCCGGA CGTGTCGGCT GGTCGGCGCC GGCAATGGAA GCGATCATCG 270060
CCGACGTGGT TAATCGGGGT CTCTCCGCAT TGCCGATCAG ATGGGCGGGG CGTTCGTATT 270120
TCGAGCGTCT CATGCCGTCA CTCCCGCGTC GATGATCCGG ATCGCCTCGG CCCATTCCTG 270180
CGGTTCGACG CGCAGGAAGT GGTTGCGCTC ACGCGCCTCC AGGAAGGGCA CGCCCTTGGC 270240
CATCTTCGTA TCGCAGATGA TGATTCTGGG CTGCGGATGC CGGTGCTGGC GCGCATTGTC 270300
GAAAGCGTCG ACGAGCGCAT CGATGTCGTT GCCGTCGATG CGTTGGACGT ACCAGCCGAA 270360
GGCCTCGAAC TTTGGACCGA GAGGCTCGAA GTTGAGCACC CCGAGCGACG GGCCGTCCGC 270420
CTGCATCTGA TTGACGTCGA CGATGCCGAT CAGGTTGTCG AGCTTGTAGG AACCGGCCGA 270480
CATGGCCGCT TCCCAGGTAG AACCCTCGTC GAGCTCACCG TCGGAGAAGA GGTTGTAGAC 270540
GAAGGACCGC GAACCTTTGC GCTTCAGCGC GAGCGACATG CCGACGGCTA TGCCGAGGCC 270600
GTGACCCAGC GATCCTCCGG TAATCTCCAT GCCCGGCGTG TAGGCCGCCA TGCCGGACAT 270660
CGGCAGGCGG CTGTCATCGG CGCCATAGGT TTCCAGTTCA TCTTCGGGAA TGATCTTCGC 270720
CTCGATCAAG GCCGCATAGA GTGCGATGGC ATAATGGCCG ATCGACAATA GGAAACGGTC 270780
GCGACCTTCC CACTCCGGAT CGTCCGGCCG GTAGGTCGTC GCATGGAAAT AGGCGACCGC 270840
GAGAACGTCT GCGATGCCGA GCGCCTGTGC GATATAGCCC TGGCCCTGAA CTTCGCCCAT 270900
GCGGAGCGCA TGCCGGCGAA TGCGGCGCGC ACGCTCGGGC AGACTGATGT TGTGACCGAT 270960
CTGAGCCATG CCGTCCTTCC TTTCAGGCTT CCTCGTGGAT CAATGGATCA GCATGCCGCC 271020
ATTGACGTCG ATGACGGCGC CCGTGACGTA GGCGGAGAGA TCGGATGCGA GGAAGAGATA 271080
GATGTTGGCC ACGTCGCGCG CGTCGCCCAA ACGACTGAGC GGAATGCCCT TGACGATGTC 271140
TGCGCGCATC TCGGCGGACA GCTTGTCGCC GGTGATGTCG GTCTGAATGA GCCCCGGTGT 271200
CACGCAATTG ACGCGAATGC TGTCGGGGCC GAACTCCCTG GCCATGGCCT TGGCAAGGCC 271260
GAGCACGCCG GCCTTGGCCG CCGAATAATG GGGGCCGCCG AAGATTCCGC CGCCGCGCTG 271320
CGCTGAAACC GACGACATGC AGGCGATCGA TCCACCGCCA TTCTGCCGCA TGTTTGGTAT 271380
GAAAACCTGA CTGAGATTGA GCACGCCCGT CATGTTGACG GCGACGATGC GCTGCCAGTC 271440
GGCGTCAGAG ATATCGAGGG TCTTCACCGG CTGGGTGATG CCGGCATTGT TGATGAGGAT 271500
GTTGGCGACG CCAAACGCGC TCAAGACCTC ATCCGAGGCG GACGTGCAGG AGGCACGATC 271560
GGCAACGTCG CAACGCAGCC CGATATGCGC ACCGTGTTCG ACCGGCGGCA GGTCGCCCGC 271620
TGCAGCCTTT GCCTCATCGG CGTTGATGTC AAGAATGGCG ACCCGCGCGC CGTGCGATGC 271680
AAAAAGCTCC GCCGTTGCCC GGCCAATGCC ACGCTTGCTC GCCGCGCCAG AGATGACGGC 271740
GGTCTTTCCC TTCAAAAGCA TCTTTTCTTT CCTCCCGACA CCGACTATGC GGCGCCTGGT 271800
GTCTCCTTCC AGGCCTCTGC CTGTCCTCGT GGAAGAGTTG TGGCGTAGCG CGCACCGTCC 271860
TTCAAACGCC AACTTTACAT CTGTGGGTGA ATCTGATTCA CTAATCCCAT GCTGCAGACG 271920
ACGTCACTCT CCGCTATGCG CATTTTTGAA GCTGCCGCGC GACTCGGTTC GTTCCGGGCA 271980
GCCGCGGAGG AGTTGAACCT CTCGCCGAGT GCCGTCAGTC ATGCGATTAT GCGGCTGGAA 272040
CGGGATCTGG GGGTGGCGTT GTTCGAACGG ACGACGCGCA GCGTTTCGCT GACTGTCGCG 272100
GGGCAGACAC TACTCAATCA TGCGTCCAAT GCCTTCGAAG AATTGCGGCG CGGTGTTGAA 272160
CAAATTTCAT CCAACAAGGC GCAATTGCTG CGACTCCACT GCGCGCCGAG CTTCGCGGCA 272220
CAGGTGCTGT CGCCCCGTCT GCCGCAGTTT TTGAAAGAGA ATCCCGGCAT AGAGGTCCGG 272280
GTGGCGGCCA GCACCAACTA CGCCCGCTTC GTCGATGGGC TCTTCGATGC CGATATCGTC 272340
TACGGCGAGC CACTCAATCG TGAAGACCTG ATCGTCATCC CGCTTTCTGA AGAAATCGTC 272400
TTGCCGCTTT GCGCGCCGGA TCTGGCGCAC CAAATCAAGT CCCCGCGCGA CCTGTTTCAC 272460
CAACCGCTGA TCCGAAGCGA TCTCAAGCGC ATCCAGTGGA TCGACTGGTT CGAAGCCAAT 272520
GACCTTGGAC CACCACCATC ACCATCGATG AGTTTTGACC GGAGCTTCCT TGCCGTCGAT 272580
GCTGCGGTCA ACGGGCTCGG AATTGCGCTC GAGTCCGATG TGTTGGCCAG GCGCGAGCTG 272640
GAAAGCGGCA AGTTGGTCCG TCCTCTTCAT CGGATCTGTC GCGACAACCG CTATATCGGC 272700
CACTATCTCG CCTATCCAAA ATCCGGCAGC CAGCGGCGAC TCGCCCGCGC CTTTGCCGAT 272760
TGGCTGACGC GCGAATGCCA GCCGGCCAGC ACTGGCTCAG AGTAGACATT CCGCCAGGCT 272820
TCTCGGCCGC GACGTCTCAC TCGTTTCCGA GTGAAGCGGA CGAGAAATCT TTCAAGGCCG 272880
GTCGCTCAAC CGTATCACTG AAGGGGCCGC GTACCCGAGG CGTGATTGGG ACTAATAACC 272940
GTCATTGCCA GATAGGAAGC ATGTGAGCAG GTCGATCCGG GGAAACTCGA CTGATCAAAG 273000
AACGGGTTTC GCTCATTCCC AGATTCGAAA CCACGCCGGC GGCAATGGCA GGGACCAGGG 273060
TATTAAGGGT TGATTTTTGG CGGGCTGCAT GGAGGTACAT CGGTGGGGCG AGAAGAAAGG 273120
CGAAACTTCG CCTCCTTCGT CGGTTCTCGC CACTGGGTTC ATTTTGCTCA GATTTGCCCG 273180
ACATGGCCTA AGGCTTTACG CACTCACTTA TTATCCATGA GTTTTTGAAA CATGTAGATG 273240
TATTCGAGGG CGTAGCGGCG GGCGTGTTCC TGCAGGTTGG CGGCGGCGGC GTGACCGCCC 273300
TCGATATTTT CATAATAGTA GAAGGGCAGG CCCATGTCTT CGAACAGCGC GGCCATCTTG 273360
CGCGCGTGCA CTGGGCCGAC GCGGTCATCC TTTGTCGAAG TTTCAAAGAA GGGTTCCGGA 273420
TACGCTACGC CCGCCTTGAC GTTGTGATAA GGCGAGATCG AGCGCAGGAA GGCGCCTTCA 273480
ACCGGATCGT CGGGACTGCC ATATTCGGCT TGCCAGGAGG CTCCTGCCGA CATGCGGGTG 273540
AAATTGACCA TGTCGAGAAG CGGCACCTGG ATCACGACTG CGTTCCACAG GTCGGGGCGC 273600
TGGATCATTT GCACGCCCAT GAGCAGGCCG CCGTTCGAAC CCCCCATGAT CCCGAGATGC 273660
GGTGTCGAGG TGACCTTCTT GGCGATCAGG TCCTGCGCAA CGGCCTGGAA GTCGTCATAG 273720
ACGCGCTGAC GATTTGTCTT CAAACCGGCG TCGTGCCATT TCGGGCCGAA TTCGCCGCCG 273780
CCGCGGATAT TCGCGAGCGC GTAGGCACCA CCCTTTTCCA GCCACAGTTT GCCGAGCACC 273840
GCCGAATAGC TGGGCTGCAT TGGAATTTGG AAACCGCCAT AGGCATAGAG GATGGTCGGG 273900

TTCGTGCCGT CCAGCTTGAC GTCCTTGCGC GCTACGAGGA AGTATGGAAC CTTCGTTCCA 273960
TCCTTCGAGG TCGCCCAGAA CTGCTGTGCT TGCAGGCCTC CGGCATCGAA GCGTGCTGGG 274020
GTCGAAGTGA TCTTCTCGAC TTGGCCGGTT GCAGCATCGG CGCAAAACAG AGTTGAGGGT 274080
TCGAGGAACC CTTCTGAGAA GACGAACAAC TGATCGCTCT CATCGTCGCT AGACGTCAGG 274140
GACAGCGTCG AATTTTCCGG CAGAGCCAGC TTGAAAGACG ACCAGCCGCC CTTGCCGAAA 274200
TCGAAGCTGC GCACCTCGCT GGTGACATTC GATAGGATCG ACAGCACGAG ACGATTTTTG 274260
GTCTGGGTAG TGCCTGCGAC AGATTGATGC TCGTTTGGCA TGAACAGCAC AAGCGGTTCA 274320
ACGCGCGCTG GATCGGCGAG CGCCGCCTTG AGATCGAAAG CGATAATCGC ACCATTGTGG 274380
AAGACGGTGC CTTTGGCCGA GGTCCAGTCC GACTTCAACC AATAGATTGC CTGGCCCTTG 274440
TAGTAGCCGC TAAAGACGGC CGTGGTCGGT AGGGGCAGGA CCACCTTGCG CGTATCGGGG 274500
TGGCCGTTTG GGTAAAAGGC TAGTTCGGTA TTGAAGAAGT CGAGGCCGCG ATACGAGGTG 274560
TCCATAACAT ACTTGCCATC GATGTCGCGT AGCACGCCGC GCTCAGCCGA GACGTCCTTC 274620
TTTTGGCCGC GGAAAATCTC GACCGCCTGA TCCAAGCTCT GGCCGCGCTT TACCACCTTG 274680
GTGACGTAGG CATAGCCGGA AGAGGTCACT TCACCCGGCG TCCATTCCCG GGTCACGTAG 274740
ATGGTGTTCT CGTCCACCCA GGTCACGGAC TGCTTGCCTT CGGGTAGGAC GAAGCCTTCC 274800
TTAACGAATT CACCCTTGGC AATGTCGAAT TCGCGGACCA CATCCGCATC CTTGCCGCCA 274860
TCGGACAGGC GGATGAGGCA GAGATTGCTC GTGGGCGGCA GGCAGTCCCC GCCTTCGAAT 274920
ACCCAGGTCT TGCCCTCGGC CTTGGATAGC GCATCGACGT CGAGAATTGT GCGCCATTGC 274980
GGGTTGCCGG ACCTGTAGGA CTCCCAGGTC GTGCGCCGCC ATAGGCCCTG CACGTGCGTG 275040
CCGTCCTGCC AGAAGTTGTC GATCATCCCG TCGCGCGCGA AGCTGGGCGA AGCAATGCGG 275100
TCGGTCGCCT GTAGGATCGT CAGAGCGTCG GCCTGGTACT CGCTGTAACG CGGATCTTTC 275160
GACAGCTTAT CGACGGTCGA AAGGTTATGC GCCTCGACCC AGGTCATCGC CTTGTCGCCG 275220
TCGATCTCGT TCAGATAGGC GCGAGGATCG GAGGCGTCCT TGGAAGCTTT GTTGGGGTTG 275280
GAATCAATCG CCGTGGCGAC GGTGGTCATC TGGGGCTGGG CGGGAGCCGT CATGGTGACC 275340
ATAGTCATCG CCATCATCAA GCTGTTCAGA AATGTAGTGA GCATGGTTTG CCTCTTCGAA 275400
AGCCTCGATA AACGTATTGG AAGCGTTTAA AATCAATCGC TTCGCACCCT TCTCAGCAAA 275460
TGCTGTGCCA GTCTGTGTGG GCAAGAGCTG TGCCAGCCCG GTTTTTTCGG GGTCATTGGG 275520
AGGCCGTTGT AGGAGAAGTG GCACTGTGTG TCGCAACCGC GAAACGGCGT TGGACGACGT 275580
CGGGCGCGGT CGGATCCGGC ACTTCAGGGA GTTCGCGCTA TAGGGTTGAC TCGGGATTGT 275640
CGACACCCAG CCTTGCTTTG TGGAAGAACC GCGCGTCTGC GTATGAAGAG CCGCGCCGTC 275700
AGCGGCAATT TGGTCAACAA GCTCGGGGGA TTTTTCCGAA AACCTGGTAG GCCGGTCAAT 275760
TCGTCATCCC GGACTGTCCA CCCAAGGGCG CACTCCAATA AGGGTCAAGT TCAGTGCTGA 275820
TTGACACCTC CGCCAGACCG CCGTCGCTCA TCAGGCCGTC GGGGACCCCT CAGGAGCTTC 275880
CGGATATCGG ACCTATCATT GGCGAGAATT GGCGCCACGG CTCCGAACCA GCCTAGAATG 275940
TCGTGATCGA CGTACTGGAA GATATCAATC TACTGCCAAA CCAGTTCGGT CCAAGGCAAG 276000
CTTCCACCTT ACCTTGACCG TGCACTAGAG AGCGAGCGCC TCATGCCGGA AGCCGGGCGC 276060
CCGACATATA TCAACATCCG CGGCGTGCCT TATAGGGCCG AGTTGCTGGA AGAGAACGGT 276120
CGCTCACGGG TTCGTATTTA TCCTGTGACT GGCTGAGGAA TGCGCTTGAG CTGCAATCTC 276180
GGTTGTAAGC GGAGCGGCGC ACCGCGGGCA AGCCTTTTAC CGCCCCAGCC ACTGTCATAG 276240
GCCTGCTCGT ACCAAGGGCG CTCATGGGAA CTGCCCTGCG CAATGATATA CGAGCCTTTG 276300
GTTAGATCGA AAACGGCGAA CGGAAGTTCA ACTTGTACAT TCGATCCAGG AGTTCGCTGC 276360
TCCCCAGTGG AAGAGTGACC TTTCGCCGTG GGGCAGCAAC CTTATGCGTT GACATACACG 276420
TATCTTGCGC GAAGCCCGCG CCCGATAGGA TCACGCGGTT GCGGACGGTT TCCTGTATAG 276480
CATGCCGTCG CACCATACGT GTTCAACGCG GCCCCACTCG CAGGCTGCGG CTGCACTTGG 276540
GAAGAAGCCA CGCCCATGGT AATTGGCACT TGTGTTGCAA AGCAACGGAA TGCCCGTGAG 276600
CTTTTCATAT TCGACAAGTA CCTCGGCGAC CTTATGCTGA GAGTTCCTGG AAATGGTTTG 276660
CAGCCGCGCA GATCCGTCAA GATGTACAAC TGCAGGAACT TTGTCCTGCC ACGGCATCTT 276720
CGTCTGGTGA TCGAACAACA TGTATGGATC GGGCGTTCCT GGACTAAATA TATCCGGTGC 276780
ACGGTCCTCC AGACATATTG GCGCAACTGG CCGGAAGTGT TCGCGGAATT TGATTTCGTT 276840
AAGATGATCT TTCATTTCCG GCGAGGTCGC AGCCGCCAGA ATGCTTCTTC CTCCCAGTGC 276900
CCGTGGTCCA AGCTCGCTGC GCCCGGAAAG GAAAACCACG GGTTTGTTGC TGGCGAGGAT 276960
GGCGGCCACT TCCGAGATGC TGCACGGGCT AGCATGCCAC CCAGCTGGTA CTTTGCCTGC 277020
TTGCAGGCTC GGGCCGCTGT AGACTGACCA GTCTAATGGC ACGAACCCTT GCTGGGCCAC 277080
TATTTCACAG CAGGCTGCAC CAATCGCAGA GCCGCTGTCA TTAGGAAAGG GCGGTACCCA 277140
AACCGAATCG AACAGGCCTG TCTCGCGCAG CGCACTGTTC CATTTGATAT TGAGTCCACA 277200
GCCGCCAGCT ATGCACAGAT TGCGTGCTCC CGGCAGGGGA TGGTGCTGCA AGGCGTTCGC 277260
CATTTCGTCA ACGAGGAGAC GTTCGAGGAA AAAATGAGAC GATGCAAGCA CGTCTTCGGG 277320
CGCCTCTGCC CCCAATTGGA GCGCGCTGGC AGCGAAAAAA TCGTGCACGG CCGCAAGTGA 277380
GGATTCCGAG TTGTTGATGT TCGCACGGAA GGCGCAGGCA AGCGCAGTAT CGCCGGCAAA 277440
GTGTTCTTGA TAGAGCTTTT GGAACACAGC AACGATGCGT TCGTGAACTG ATCCCAGTGC 277500
GATAAAGGCC ATCAGCTTGC CGGCAACGCC GAGGTCCCAG CCCCCGCGGC TCGTCTGCTT 277560
ATAGGGGCCG AAGTAGTGGC CCGCGGCAGC GTAGGCCTGG CCTGTTACCG GGAACAACGA 277620
TTTGACGAAG CTGGCTCGCT TGCCTTCCAC ATGGTAGAGC CGCGGAAATA TGCAGCCGTC 277680
CCACACCAGG CAAAGCGCAG GGTCTCCGGA TTTGGCAAAG GGGCTGGTGC AGTATGCAGA 277740
GGCGACATGG CTCGTAACAT GCGGATAGCT TCTGTAGCTA AAAACCCGAT CACCAAGTGT 277800
GAGGCCGGAG CCGCCGATCC AATCGAGAAG TCCCTCGGCG TGGCGTTCAA CGTAAGGCGC 277860
GCCTCTGAGG ATAACAGGAG TCTCCCCACT GAGGACCTTG AACTGCGACT CGGCCTCACC 277920
GTCCCAGCCG TCGATGACAA ACTGATCAAC GTCTCGTGCA TTGACCCCGT TTTCCGCCAG 277980
AGCCGCCACA ACAGCATCGA GATTGTTGAT TTCTTGATAG CGCGAATTGT TATTCCGCTT 278040

CTCCTGCTCA GTACAGAAGA CGAGCCTTCC ATCTTCGACA ACGGCGATAG CCCCGTCATG 278100
GGTTAACTTG ATGCCACAGA CGCGCATAGT GTCTCTATCC AAATCAGGTA GTCGATTTCT 278160
GAAAACGGGA AAGCAAGCAG TCTTCATTGA CCGATGCGCC GCGGCAATAA AGCCGCTCCA 278220
CCTCGATCAA CGTTTCGTTC AACATCGCGA TGACGGTCTC GGCACCGGCC ACATGCCCCC 278280
AGCGCCGGCA ATTAGCATCG ATTGCGGATC CAAAAACCAT GTGCCCACTT GGCGCCAGCA 278340
TGCTCACCAG GTTGTGAACG GCGGTGCGTA TCGCGGCTAC ATCCTCGAGA TAATAAAGAA 278400
CTTCGGCCAC GACGATCAGA TCGAATAGCT GCTGAGTCGA GAACTGTCGA ACGTCAGCGA 278460
CTATCCAGTT GATGTGAGAC GATTCCTTCA TCCGTTGCCT CGTTCGAGCA ATCGCTCGCG 278520
GCACCACATC AATCACAGTC AGCCGCTTGC AATAAGGCGC CAGTTTTTCC GTGAATGCGC 278580
CGGCCGCGCA CCCGACTTCG AGTGCGTTGG TGATAGGACC TTGGGAAAGC GACAACCGAA 278640
GCATCTGCGA ATGTCGCTCC TGCTCGAACG CATTGGCGTC GAGCCGCCAT GGGTCCTCCG 278700
CCGCCAGCTC ACGGTGCAAT AAATGATAGT TGTTGACCTG CGTCAAATTC GCCTCCGAGA 278760
CGCCGTGCAC GGATCTGCAA AGACTCTTGC ACATGGGTGG GCAGAGCGAG GCGCGAATCC 278820
GGAGGCGTGG AATGCACGCG AATTCCCGAA AAAGCGGTCG CCGGCAACGA TGGCTCCGTC 278880
AATACTGCTA CTTGTGGCCC CTTGAGTTCG CTCGAAAGTC ATACTTTTCT GATGCAGCAT 278940
CAAGCAAAAT TCGTAAAATC GATTTTTGTG ATGCCAAACA TCCAGGTTAT GGAAGTTTCG 279000
CCAGTTCCGG TCACTCCAAG GGCGGGCGTG TTGGGGCTGC TCGGCTTGAT CGCATTCGTC 279060
TGGAGTGAAG AGTTGGAGTA GTCGCCCGAT CGTTGAGAGG CTCTCGATGG ATCGTTCAGC 279120
GGCTTTTCTC AACAAGGCTT TGAGCTTTGA GAGTGTGTAT TCTCGATCGG GTGAAATCGG 279180
GCCATACGGC GGGAGCTATC CAAGGGAGGC ATCCATGTCG AAATCACAAT TGGGTGGATG 279240
TGATGGAGAC GTGTTCCAGG CGCTTGTGGC AGATGCCTTT GGGATTCTCA GACGGCCCGA 279300
TCGATCCTGC GCCTGTTCAG GGAGCAGCTG TTGTTGCTGG ACGGGAGGGT GGCTGCTAGA 279360
CTGGGAGGTT GCACCAGCGC CTATGAAGGC CGTCGCTTTC CAGTGACCAT AAGGGACGCT 279420
GATCCGCGAG CGTTCTCCTA TACGGGATCG ACCGTAGCGG TGCGCCATAC TGATGAAAGC 279480
CCAGGGTCTC ATCGTTACAA TGAAACGTTC CGGATCAAGG TCAAGTTGGC TGCCGAACCA 279540
GACTTGCCGC CGCTTCAGGA TGTCAGGATA CGAAATCCGC GTACTCTTGT GTGCTGCTGG 279600
CGATGCGCCT GATGTCGCCA CCCAAGAATC GCCTCACTCC TTCCTTGAAG GAAGCTCGGC 279660
CGATCTCGAT AATTGACGAC CAGACAAAAC AATCGAGGCT CAGTTGTCTG AGGAACGCCG 279720
GTTCCTTTGA CCCGGCCATT TCAAGCTCTC CCAGCGGCCG TGTGCGCTCT CATGCGCCAT 279780
GTCTCCAGCA TCATCCACCG TACCCGACTT GATACGCATT CATCCATGGC GTGGATCTTT 279840
GCCATCCAAA TAAACAGTTA TCGCAATCTT CTGAACGGCG ATAGAGAAAG TTGACTTAAA 279900
CTGAAAGAGA GAAGGCGAAA TTCTTTCGCG ATCGCTTTTA GACTAGAGGT GCTCTGATGC 279960
CGGGAACTCG GTAACTTGAG CTTTGGACCG ACCCGGATAT GAATGACGAG CGGAACTACC 280020
GATCAATTTC TCGAAGTCCT GGAACTACAC GATTGGACTT GGCGGAGGAT CGAACTGTTG 280080
CTTCTCCCAG ACGCCGCGAC CGCATCTGCG TGAGACTCTC AAGAGCAGGA TTAGGAAGTC 280140
GGCCTGACGG ACATATGCAG AATGGTTCTC GATACTGGAA CAACTCAAAC GTGGCGGGAT 280200
CTGCGCCAGT TTGCGCCGTA GCCGGTCTTC CGAGGCGGGG TCCGGAGTAT CCTCGGCGCG 280260
TCGTCACTGA TACTACTCCC GATGGTCGGG AGGGGATTTC GCCAATCAAG CTGGGGGAGA 280320
CGACGATAAT CCTGGATCTA CACGGGCAAG GGCTATCGGT GTCAGCGACC GCCAAACAGA 280380
TTGGCTACGG ATTGCAAGAA TGTATAGCGC GGCGATCAGG ATGAGACGGC GGCGACAATC 280440
TGGATGAGAC TCTTATGTCG CGGTCGGCAT GAAGGTATCA TGTTGATTGT CGCTGGCAAG 280500
GGTCTCATCG TGTTCTGATT GTCGCTCCGC GACAATCAGG GAAGCACTTT TGGTTGTCGC 280560
GAAGGCGGCG GGTCTGCCGC GGTGGCGTTT GGCTTCCATG GCGGAACGTC GCCGATAGCT 280620
TTCGACGTTC ATTTCGAAGA TCGTTGCGTG ATGAACAAGT CGGTCCACCG CGGCAAGCGT 280680
CATGGCCGGG TCCGGAAAGA CGCGGTTCCA TTCTCCGAAG GGCTGATTGG CGGTTATCAT 280740
GATGGAACGC CGCTCATATC TTGCGGAGAT GAGTTCGAAC AGCACGCTGG TTTCGGCCTG 280800
GTCCTTGGTG ACGTAGGCCA GATCGTCGAG GATGAGCAGA TCGAACTTGT CGAGCTTTGC 280860
GATGGCGGAT TCGAGCTGGA GTTCACGCCG TGCGACCTGA AGCTTCTGGA CGAGGTCGGT 280920
CGTGCGCGTG AACAGCACCC GCCAACCATT CTCGATCAGC GCGAGGCCGA TGGCGGCGGC 280980
GAGATGGCTC TTTCCGCCGC CCGGCGGACC GAACAGGAGG ATATTGGCAC CTTTGGCGAG 281040
CCAACTGTCG CCGGCGGCAA TGGCCATGAC CTGGGCCTTG GAGACCATGG GCACGGCGTC 281100
GAAAGCGAAG CTCTCGAGCG TCTTTCCGGG CGGCAGATGC GCCTCGGCGA GGTGACGTTC 281160
GATCCTGCGA TGCGCCCGTT CAGCCAGTTC ATGCTCGGCG ATGGCCGAAA GGAAACGAGC 281220
CGCAGGCCAC CCTTCACGAT CGGCCTGTTC GGCAAATTGC GGCCACAGCG TTTTGATCGT 281280
CGGCAGCCTC AGGTCATTGA GCATGATGCC GAGGCGGGCT TCGTCGATGG TGTTGTGGAC 281340
GTTTTTCATG CGGCCTCTCC CGCATAAGCA GACCCCATCA GGGCTTCATA GCTATTGAGC 281400
GATGCGAGCT GCACATGCAC GGTCGGCAAC TGATCCGGGT CCGGACCGAA GATGGCTCTC 281460
AAGGCCATCA GGTCGGGCAG TTTGCGGGCG TCGAGCGTTC TGGCAAGCTC CTCGGCCAGT 281520
TCACGCTCGC AACCGCGATC ATGAGCCAGG GCCAGCAATT CGACGGTGAT CTTGCAAGCC 281580
TGCCGGTCAG GCAGTTGCTC GATGAGAGCG TCGAAAGCCC TGCGATATTC CGGCCGGGGG 281640
AAGAGCTTGT CGCGATAGAC CAGGTTGAGA AGCGCCATGG GCTTTTTGCG CAGAGAGTGG 281700
ATGACGTGGT GATAGTTGAC GACCTGGTCA TGCTTGCCGC TCGCGTGGGC GCGACCTCGT 281760
GGCAACGTCA GCAGATGCGT GCCGCCGATG AAGACGTCGA GACGATCGTC AAACAAACGC 281820
ACACGCAGCC GATGGCCGAT CAAACGGGAG GGGACGGTGT AGAAGACTTT GCGCAAGGCG 281880
AAGCCGCCGG TGCGCGACAC GGTGACGACT ACCTCCTCGA AGTCGGTGGT GCGGCGCTCG 281940
GGAAGCACCT GCAGATGCGG GCGCTCGGCA TCAATGCGCT TGCCATGCGC GGCATTGCGG 282000
CGGCTGACGA TCTCGTCGAT GAAGGCGCGG TAGGAGCGCA GATCGTCGAA GTCTCTGGTG 282060
CCGCGCATCA GGAGTGCATC GCGGACTGCG TTCTTGAGAT GGCCGTGGGA GCTTTCGATT 282120
GAGCCGTTCT CGTGGGCGAC GCCCTTGTTG TTGCGCGTCG GCGTCATCCG GTAGTGAGCG 282180

CACAGCTCCT CATAGCGGTT TGTCAGATCG ACCTTGGCAT CGGCATCGAG GTTGCGGAAG 282240
GCAGCCGACA GGCTGTCGCT GCGGTGATAG AGCGGCGAAC CGCCGACCGA CCACAGGGCG 282300
TTCTGCAGGC CCTCCGCCAA GGCGACGAAG CTTTCGCCGC CAAGGATGAC ATGGGCGTGC 282360
TCAAAACCCG ACCAAACCAG CCGGAAGTGA TAGAGCAGAT GGTCGAGCGG TTGGCCGGCG 282420
ATCGTCACGC TGAGGCTGCC CATGTCGGTA AAATCCGACA GCCCTAGTCG GCCGGGCTCG 282480
TGCGTCTGGC GGAAGATCAC CTCCTGTGCT TCACCGTGAA CCGCCCGCCA TGACCGGATG 282540
CGCCGCTCAA GTGTGCGGCG AATGCCTTCG GGCAGTTCCG GATGACGCCG CAGCATCTCG 282600
TCGTAAACGG CGACCGCACG AATGCCGGGA GCGGCCTTGA GGAGCGGAAC GACCTCCGCA 282660
TCAAAGATAT GCTCAAGCGG ATCGGGTCGA CGCCGACCGC GGGGCGGCTT GTTCTGCGAC 282720
GGAAGGCGCT GCTCTTTCTC CATGCGGAAC GCCGTCGCCC GGCTGATCGA CGCCTTCGCG 282780
GCGGCGACCT CAACAGAATG CGTTTGTCGG TACTTCATGA ATAATCTCAT CTGATGATCG 282840
GTTACATGGC GACCCGGCAC AAAGGTGGTT CTCCATTCCA GAAAACCGCC ACCGTAGCGG 282900
GCCGACCGCG ATCATGAGAC GCCTAAAAAT TGCGCCGCGG CGGGGGTGTA ACTCCGGTCG 282960
GGCTACGCCC TCCCTTCGTC ACACCCCCAC CGCCGAGTCT CATCCTGATT GACGCTGAGT 283020
CTCACCTTGT TTGTCGCCGC GCAAAGAATG CGCGCAACTA CGTCGAGCGG GGGCTAGAGG 283080
CTCCGAAATA TGGCCAACGC AAGCACACAA TGTTTGGCGA CCGGCTCGCG ACCTATCTTC 283140
GCGAACGCGT GAAGGCTTGT CCCGTCCGGA CCGCGCGGCG GCTTTTCCGC GAGATCTAGG 283200
GTCTCGACCG TTTCTGCAGC TATACGGCGC TATCCGGGCT GACAAGGTGA CCGGGCTCGA 283260
AACCGGCGCC CGCAACTACA TCACCAAGCC CTTTGCCTCG CCGGAGTACC TGGCCCGCGT 283320
GCGTGCCGCG TTACGCGGGC CGCCGGAAGC GTCACTGCAA CAGGATTTTC CATTTCGATG 283380
TCTGGCGAAT GGGCGCCGCT TGCATCGAGG AACGGATTGT AAACTCAAGC TCTCTGCGAG 283440
TTCACTCTTC TGATGGCCTT TCTCAAAAAT CAGTGTCCTC TCGCATGAGC AGCTTCTGGG 283500
GATGATGCGC GTCAATTTTC CCTTAATTCG CGTCTTCGCC AGCTCGCCAG CGGGCTGGTG 283560
GATCTGCATC GCCTCCTTTC GTCATATGGA GGGGACCATT TCAACCTTCG CGGTGCTTGC 283620
GGTGCGCTCC CCTTCACAAC CAGTGATCAG AATGCTCCAA GCACTCCATG GGGCGGTCCG 283680
GAGCCGAGGT CCACACGCCG GGACGATACC GCAATGCGGC TGTGTATGCT GGCACGCTAT 283740
CACCAGCAAA AAGGCGCTTG TAGAGACAAT GTCCCCCAGT CCATGTTGAG GAGGGGAGAT 283800
ATTCGACGAT GGCACGTTAC GATGCCCGGC TGAGGGGCAT TGGAAAGGCA CATGCCTGCA 283860
GCGCGTTCGC AGGCCACGAT TTCGAGGGTA GGAACATTAT GAAGACACCG TGGAAATTTC 283920
TTGCTCAATT GGCGTCGCGG CGACGATCGG CAAGCGTGCA AGAGAATTTT ATTGCGCCTG 283980
ACACTGATCC CGAAGTGATC GAAAGCGAAA CGGAAAACAA GTCGCCACTT CCGTTCGACA 284040
AGCCAACGCA AGCTTCTGGC ACTCCTGATC ACGATTCCGC GGTTGATCGC GGGTCGATGT 284100
TATCGGACCA GCTGGAAAGC GACCCCAATC CCCTGCAGGA AATGAACCTC ACAGCTGACA 284160
TTCAAGAGCC TGGAACCCCT GCCCGAAGTG AAACCAAACA ATCGGATGCG GCTGGGAAGA 284220
GGCTGGGGCC GCAAAGCCAG ACAAGCGCAA ATTCCCAAAA GAAGCCGGAG ATCAGATGTC 284280
GAGAACGCTC CAAGAACGCG CGCGCCGAGG GGGTTGCGCA GAGCGCTGTC GCCACAAATG 284340
AAGCTGAGAC CGTACAAACC TCGTCGTCCG GGATCCTTTT CTTCAACGAA GTGGCAATCC 284400
TCGACGAGGA AATCAAAGAG CTGCGACGTC TGCTGGCTCA AAAACTTTAT CTGCAGAACG 284460
CTCAGCTCAA AAAGATGCTT GAGCGTTTCG ACGCCTCGTG AGTTCGCGTC AACTGACGCC 284520
GGACCGAGAA AATGCTGCAG GACGTTATTC GCCGCGAGTT CCGAGGCCTG CTCGAAAGCG 284580
GGGAACCAGT GGTGTGCCCA GCCGACGGTG AAGGTGGAGC AGTGTCCCGT CACGGCCTTC 284640
GACGATCTCG AGGAGATTAT TTTCGCGCTT GATCAAAAGA GAGGTTCGGG GCGCCAATCG 284700
TTCAGGATCA ACAGCTGAAA ATGGTGCGCG GCAGGCGCGC ATATCGCCCT CCACGAGCAA 284760
GCGTCAGTTG GGCAAACGGC CTTGCGCCGC ACTGGTAAAG GACGGCGCAA CCATCGCATC 284820
AGGCCTTGTG CCCGAGCGCA AGCCAACCAG CTCTCTCCGA CGCCGGACGG TCCTCGGATG 284880
GCCAGATCGT CGTGCGCATC GATCCAGTCG CCGCCAATCA AGCCTCATGA ACAACGCGAT 284940
CCCGGCTGTG TTCGCTTCGG CAGTGGCCTG ATGGCGTAGC CCGGCGAACC TGAGGCGTGC 285000
GGCGAGCTTC CGGGGCGCGA AATCCAGTCC CGTTCGAGAT TAGGCGCCCA AGCATCTTTC 285060
GTTGTCCTTA CTGGCGATAG CGGGTCCGCA GATGTTTGTG TGATAGATCG GCTTATGCGC 285120
GCCACTCCAG CTGACACAGC GCTTCAGTCG AGATGATTGG CGACGATCGG AACGGACCAG 285180
ACCGTGGAAT TCGTGCCCTG ATTCCCAAGG CACGAGCGCA GGCGTGTGTC GAAGTCGACT 285240
GGGCGCCTAC TGTGCCACTG GATTTGTCGA GTTCCCGACA TTCGGGAGGC GAGACATAGC 285300
GCCACCGAAG TGATCGCGTT GCTTTGGAAC GATTTTCAAG CTTGGCACGA CACATGCATA 285360
GCCATCCACA TACACGCTGC GCCGCCGCGC TGGAGATTCC GAAGCAATGG TGAAGAACTT 285420
CCTTGGATCA TCGGTTACAT CAGGCTCGGC TGGAGCGGAA CATGGAAAGA CTTTGCAAAT 285480
TCCGACTTTA CGATGGGTGC GCCAGTCGAA GACTTCGATC ATGGCACTTG TAATTTGTCT 285540
AGGCAGGATA GTGAAAACAG CACTCGTTAC CGGCGGCTCC GGATATTTTG GGGAACTGTT 285600
AAGCAAGCAA TTGCTTAGAC AAGGAACCTA CGTTCGCGTG TTCGACCTGA ATCCGCCCGG 285660
CTTCAGTCAT CCTAATCTTG AATTTCTCAA GGGCACAATT CTCGACCGAA ACGCGGTCAG 285720
ACAAGCACTT TCCGGGATAG ACAAGGTGTT CCACAACGTT GCGCAGGTAC CGCTAGCCAA 285780
GGAAAAAGAC CTATTCTGGT CCGTTAACTG CGGAGGCACT CAGATCATCG TTGACGAGTC 285840
GGTCGCGACG GGGATCGAAA AGTTCGTCTA CACGTCCTCG AGCGCTGTTT TCGGTGCGCC 285900
GAAATCGAAC CCCGTAACAG AAGAAACAGA ACCGAACCCG GCGGAAGATT ATGGTCGCGC 285960
GAAACTCGCA GGAGAAATTA TCTGTAAGGA AGCAATGCAG CGTGATGGCT TGGATGTGGC 286020
GATTGTGCGC CCTCGCACAG TTCTCGGATA TGGCCGCCAA GGCGTCGTCC AGATTCTCTT 286080
TGACTGGGTT GAACGGGGGC TCGATATTCC AGTGTTGGGT GGGGGTAATA ACAAATATCA 286140
GTTCGTGCAC TCGGATGATT TAGCATCGGC ATGCATAGCA GCCTCGAATG TAAAGGGGTT 286200
TGCCACATAC AATATTGGTG CTGCCGAGTT CGGTACGATG CGTGAACTTT TGCAAGTGGT 286260
AATCAAGCAT GCCGAAACCG GAAGTCGAAT CAAATCGATC CCGATGGGAC CGACTGCCCT 286320

TGCAGCGAAT CTGGCAAGCG CGCTCGGCCT CTCTCCCTTA GGGCCATACC ATTCGCTGAT 286380
GTATGGGAGA GCAATGTATT TCGATATCTC CAAAGCGCAA AAGGAACTTG GGTATGCACC 286440
GAGATACTCT AATTCTCAGA TGATGATCGA GACCTATAAT TGGTACCAGG CTAATAGGGC 286500
TTCTCTCGGA AAAGGCGGAG GGTCACGTCA TAAGGCGCCC GTCAAACAGC AGCTCCTGGC 286560
ATTGCTTCCC TATGCACTGC GGCTGATCCC AGGATGAACG CGGTTCCGAT AATCTTGGTT 286620
TTTGCTGCGG GCCTGAATTC CTGTATTGGC AATATATTGC TGAAATGGGG CCGGGCCTCA 286680
CTGCCTCCCA GCGCCGGACT TGCCGATACG TTTCTCACTC CCGGCTTTGT AGGCGGGGTG 286740
GTATTTTATG GCATTAACGT TCTACTGTTT GCAAAGGCTC TGGACTCCTT GGAGGTATCA 286800
GTCGCTTATC CGATTCTGGC AGGCTCCGGA TTCGCGATGC TTATCATCGC GTCGCATTAT 286860
TTTTTTGGAG AACCATTCCA CCTTCACAAA TGGATCGGTG TTGCCCTCGT CCTGGTGGGC 286920
ATTATTTTTC TGGCGCGAGG AGGCTAAGTT GGCATTCGAT CGAGTGATCG TGGCAACATT 286980
GACACCTTTA TGGCCATTGC TCGACGCGGA AGAAAGGCCA GCGGTGGTGT CAGAAGTCGC 287040
ACGCAGTGTG ACCCGTTCCA TCGCACTTGC CCCATTTCAC ATTCGCTTTG CGGTCGAAAG 287100
CGTTTCGATC GTCATAGGTC TCTGCACTGT CTTGATTTCA GCGGGTGCAG GCGGTCCCTT 287160
GGCGCGCACG CTCCGAACAG ACAGATTTTA TCGGCTCCTG CAAAGAATGC CCGGTCCCGC 287220
CGGTTCTGTC ATACGCCTTT ATCGCTCGAT GACGCTGCTT GCATTTTATG ATGAAGCGCC 287280
GGTAGCCGAA AAGCTGTTGG CTGCACGGCC TGCTCAAACC TCATAGGCTT GAATATGAAG 287340
GGCGGGTTTT CGTTGAACAG CGAACTGCAA CTGGAAGCCG ATGCGGTGAT AATCGGTTCC 287400
GGCGCGGGCG GTGCAAGCGT TGCTGATGTA CTTACAGCTG CCGGTTTATA TGTGATCATG 287460
CTTGAAGAAG GTGGGCATGT TCCCTCATCA TCGGCTTCTC CTTTCGCGAG TGAAGCATTC 287520
GCGGCAGCCT GGCGCGGCGG AGGATTAACC GCGGCGATCG GCCGCCCCCC TATTGCTTAT 287580
GCGGAGGGCC GCTGCGTTGG GGGAGGGACA GAAATCAACA GCGCGATTGC CCAGCGCGCC 287640
GATAGCGACC TCCTTGATCA ATGGCGCAAG CTTTACAAGA TCGAGAATTT TACCCCGGAC 287700
GAACTGTCGC AATATTATGG TCGCGCGGAG ACAACGGTGA ATGCGAGCCT GACCCCCGGC 287760
CCACTCGGGC GTCCCACCGA CATTCTGCGG CTGGGTGGGG AAGCGCTTGG CTGGAAAGTT 287820
TCGGAGCTGA AACGGGGGCA GCGTGACTGC AAGGGCGCCA ATCGGTGTTC TTTCATTTGT 287880
CCGAATGGTG CCAAGCAGTC GATGGCGGTG ACCCTTCTCC CAAAGTCGAT GGATCGCGGC 287940
ATGCGGCTGC TTGCGCGAAC GCGCGTAGAC AAGATCCGTA TCGAGAAAGG ACGTGCCGCA 288000
GTGGTCGTCG CTCAGTTGCA GGACGCCGGC GGACAGGGCG TGCACGTGCG CGTAAAAGCG 288060
GGACTTGTGT TTGTCTGCGC GGGAGCAATT CATACGCCAG CGCTTTTGCG TCGTTCTGGC 288120
CTGCGTAAAC GCATTGGCGA TACCCTGCGC ATTCATCCGA CGATCAGAGC GACGGCACTT 288180
TTTGATGAAC CCGTTGATGC GCACCAGTCG CGCCTTCCGC TGACAGCCGT TACCGAATTC 288240
ATGCCCGAGC AGCGCATAGG CGGGTCGGTG TTCACGCCGG CTGTCTTTGG CCTTTCACTT 288300
GCGGAAGACT GGACCAATCG CGGCGATCTG ATGCAGGCTT GGCGTTTATG CGGCTCTTAT 288360
TACGGCATGA TACGACCGCG CGGAGTGGGT TCAGTGAGAC CTCTTCCCGG TATTAATGAA 288420
CCGCTTGTCA GTTTCAAACT CGCGCCTGAG GACTGGATCT CTTTGGGCCA GGTTCTCACC 288480
CTCCTCGGAC AGGCGATGTT TGCGGCCGGT GCCCGGAAGG TAATCCCAAG TATATCGGGC 288540
CACGAGGGCT GGACAAACCC TGATGAGGTC GATGAATTCC GGAACAAGCC TCTGCCTGAA 288600
AAAGCGACCA ACCTGATGAC CATACATCTG TTTTCCACCT GCCCACCCGG CGAGCATCGG 288660
GACGCTTGTG CAGTCGACAG TTACGGACGT GTGCGGGGCG TCGAAAACCT CTTTGTCGCC 288720
GATGGCAGCG TCATTCCTGA GGCGCCCGGC GTCAATCCCC AGATGACGAT CATGGCTCTG 288780
GCGTTCCGGA TAGCGGAAGC GGCACTCAGT CATTCCTCTC GAGAACGCGC CCAATCGGCC 288840
GCAAGGGAGT AAGTGTTATA CATTAAGGTC CAGTAGTGAG GACCGGGAAT TGCAGCCCAG 288900
GGGAACTATC GATAAATGCG CCACTTGCCC TTCTTCTCGG AAACAAGCTT GTAGACTGAA 288960
TTGTTGTCCA GCACCGGTTT GCCCCACAAA CCATCTCTTG AAGTGACTAC AAGAGTTCTA 289020
CAACGATATT TTTCCTTCAT AATTCTAAGA TGGTCTTCTG TTGCCAGGCC GGCGAAAGCG 289080
CGTACGAAGA AATTATAGAT CTCTGATGCT TTTTTAGGCG TCAGTTGAGC AGCATAGGCG 289140
CGCAAAAAAT CAAGCGTGGT TCCGCAATGT CGTCTCTGAG ACAAAATTGC CCAGGAGATA 289200
TTTGCCGGTG TATAGGTCAC ACTTGCTAGA TCAAGCGGGT TGTTGGCCAC CGCTTCATTC 289260
GGAGGCGTTA CCTCGCGGAC AGCTTTCCAC ATCTCAGGGG ATGCTTTGAA AGCAGTGCCT 289320
TCTTCTGAAT CAGGTCCTTC GACCTGGAAT TTGAAGGTGT TCGAATAGAC AGACTTGGCG 289380
CCAACCAAAA TTGAGGGGGC GAGTAAAATA ATAGCTGTTA TGGTTGTGAG GCGCCCAACT 289440
AGCGTGCCTT TACCGATAGT TGTGGAGAAA AGCCCAGAAG TCAGCGCAGT CATGACAAGC 289500
ATAGATGGAA TGAGTACACG CCATCCTAAA TCATTGTACA TAATTACGCT ATGGAGTATC 289560
TGTGTACAAA ATAGAGGCGC CAGCACCGAG ACAGTTAAAG CGCAGTCAAT GTGTGCGTAT 289620
CGCTTACTGT CGTCAGAGGG TCTGGCAAAA CTCCAGATGA GGAATGATAA ATATATTATT 289680
CCGAACTCAA GAAAGACCAG AACAAGCCAG AATCCTGGAA TGTCCAAAAA CCAATAATCG 289740
CCGGCAAAAA TGGGAAAAAC CCAAAACTCC ACGACCTTCC TAGTGTGTAA TATAGCGGAC 289800
TGTTCGCGAA TAAGCACGGC TGCACAGAGT AGGGTTATGA CGACTGTGAC CGAAAGTGAA 289860
ATGAGAACCT CCAGAAGCCG TTTGACCTTT AGCACATGTG AGACCGACAG CGCCCCCACA 289920
AGGGGTAATA TCAAAAGCAG CGATAAGCTG CCCGCCCACA TCGAGCTCCC ATAAGCAGAA 289980
GCCAAGATTG CGCCCATAAA TACTGCCAGA GCCATATTGC GCCCGGCGTT GGAATATAAA 290040
ATTCGCAGGT ATGCCATGAT GGCAATCAGG GCCAGCGTCC CGGAAAAGAC GTGCTGGGGT 290100
ACCCAGGGGG CCTGGATAAT CCACGTTTGG AGGCCATGTT CGGGTGCCAT CCATTTCTCG 290160
AGCCACTTTC CGAAGACAAA TCGCACGGCT GGCTTAAGCG AACCGACGAA TAAGAGCGGG 290220
AGTGACCACC AAGCGGCATA TGCACTTCTG CTTCGCGCCA CAGCCAGCCA AGTTACGACA 290280
AACGTGGAGA AAAGCGCGGT CATGCCGGTA AGAGCGATGT CGGCCTCCCA GCCGGTTGCG 290340
CCTGTTATCA CGGATGAACA GGCGGCTATG AAGTGCCAGA CGTAGTAGTA AATTAGGGTG 290400
TTGGGAGATC CGGCTTCGGA ATGGAATGGA TTGGCGGGAG GCAATCCGTT TTGAGCGATT 290460

TCATTTACGA TCGCGATCTT TGCATGGTCC CAGATCGGGT GACCGACGCC GACGCTACCT 290520
CCATAATGCT GCGGAATGAT CTCGAAAGCT GGCAAAAGGC AGAGGAGAAA CGCAACAGTG 290580
AACCAGCAAA AGCCGGGACT CGCAGGCGAT CGCAGGAGCG GGTCTATGGT GCCTTTCGAG 290640
AGCCAGAATG CGACAGCGGA CAAACCAAGG ACGATCAGCA TCAGGTTGAT CGCTGTAATG 290700
GGTAAAAAAT GAAAAATGGA AACGGCTACG GCGCCGAATA TCCCAAGTCC GATGACCGGC 290760
GACAGAAGCA GGCGCATCGT GGTGTTGCTG TCAAAATACT TCGAAACAGG CCAACCTATG 290820
GCATATCCTA TAGCACTTGA AACAAGTGCG CTGGAGACAA TTGAAGAAGT TTGCACCAGC 290880
ACTGACGGCC TCACAGCTCC TCGGCAAGCT TGACTAGAAT GACTGACCGT TTTTGACGCA 290940
CCAATCGACC GAACGTTGCA TGCCCTCGCG CAACGCAATT TTCGGGGCGT ATCCGAGCAC 291000
TTTTCTGGCT TTGGTGATAT CGCAGGCAAT TGTCTTGTTC ATTTCCGACA GCACATGGAT 291060
CTTCTGGTGA TAGATCCCGG CATATTGGAG GGTAGCATCA AGTATCGTTG CGGCGCCGCC 291120
TACGATGTCC GGCAGACGGA AAGGGTTGGG CTTTACCGTC ATGCCGAAAT CTTCGTGCAG 291180
GACCATTCCA ACGACTTCAA TGATTTCGTT CATGGTGTAG GGCGTCTCGT CTGCAAGCCA 291240
GAAAATATCT CCCGCAGCCC GCTCGTGAAC TGCGGCCAGA AGAATGCCCT GAGCCAGGTT 291300
GTCAGTGTAA CCCATGGATC GGCGGTTCCG CCCTGATCCA ATAATGGGAA ACTTGCCTTC 291360
TTTGACCATC TTGAAAAAAA GCGTTTGCCT TGAAGGCTGA TTGGGACCGT AAAACCAGGG 291420
CGCGCGCACG ATGACAATCT CCATAGTGCT GCCAGCAGCC ACTTCCGCAC GCAGTGCCCG 291480
TTCCATCAGC ATCTTGGAGC GGCCGTATCC CGCATGGGGA TCATAGGGGC TTTCCTCCGT 291540
GAATCTGTGG TCCGAGTGAG GATTGAAGCC GACCGGCGAA TTCGACGACA TGACGACAGC 291600
GCGCCGCACA CCTGCCTTTT GCGCTGCAGT AACGAGGTTG ATCGTGCCCT GTGTGTTGAT 291660
TGCTTCAAAC TGGGCAACAT TTTTGGGATG AATGATACCT GCCATGTGAA TGAGAACGGC 291720
GCCTTCGGCG CCGGCGACGA ACGCGCGGAC GGACTGCATC TCCCGAAGGT CACCTGTCGC 291780
GATTTCCATG CCCTGTTTGC GCAACTCAGA TATGTCTTCT CCCTTAGGCA CGAGGGCCCT 291840
TACCCGGAAG CTTCCGTTTG CGAGAAGTCC TGCATCCGGC AGGCCCGTCG TCAACGCGGC 291900
CGCCACCCGC CCGCCCAACC AGCCGGCAGC TCCAGTTAGG AGTACGAGCG GCGCATTCAA 291960
AGTTATAGGG GTTATCTCTT GCATATTGGC ATTTGCTTAA TTTCACATGG CCATCACGAA 292020
CGCCGACGCG ATGAGGGCGC CGATAAACCA GCTGGTCTTG TCGTTGAGCG CGAAGACAAT 292080
AGGGTCGTCG TGCAATTCCA TACGATGACT GAGGAGCCAG ATCCTTTGCA GCCAAATGGA 292140
AACGCCGAGC GGCGCAACAT AAAGCCATGC TGGATTATGA TATAGGTGCG TCACTCTGGG 292200
CTCGAGTGCG AGGAAAAGCA GCATGATGAC GATCGAGGCC GAAGCGGACG CAAGGCCATG 292260
GCCCAAGGTT AGCGGCCAGT CGCCAGGCAA GTATCCGCGC CCTTCGATGG TGTCCTTGCG 292320
ATTTGAGCAC GCGCGCATTG CTTCCACATG ACGCTTTGCC AGAGCCAGCG AAATGAAGAA 292380
CATTACTGAA AACGAAAAAA GCCAAGGGGA GAATGCCAGG CCGTTCAGCA CCTGACCCAT 292440
GACAATCCTT AAGGTGAATA ACGCCCCAAT AACTGTCACA TCGAGTAACG CAACACGCTT 292500
CAGGCGGAAT GAATAGGCCA GTGTCAGCGC GAGATAGACA GAAGCAACCA GCGCGAATTG 292560
ATGGTCCAAA AGGAGGGCCG TCCCAATGCC TGTAGCAAGC ATAACCAGAC ACGCCAGAAA 292620
GCCGTTCATG ACACTGATTC GGCCTGCAGC GATTGCCCGG AAGCGTTTGG TCGCATGCGC 292680
CCGGTCCGCC TCCAGATCAG CCAGATCGTT GATAATATAG GTTGCCGAAA CAATCAGGCC 292740
GAAAGCGAGA AAACCGAACG TCGTTTTCAG TACAGCATGG AAGTCGTCCG CAATGCGGCC 292800
GAGAAACAGA GGCACGAAGA TCAGCACATT TTTCGACCAC TGGTGTATCC TGAGTTCAGA 292860
AAGCCAATCC TTCACCGGAT GACTTTTTTC AGGACTGAGC GTCCTAACCT CGACGCCAGC 292920
CTCACGGAGT TTGCTTTCGA ACTTTACTCC GTCGCCAACC AGAACGGCTC CGGAAGCCGC 292980
CTCCCAGATC GGCAGGTCGG CGGCACTGTC GCCTGCATAG ATGAATCCCT CGGGAAAGCT 293040
CTCCTGAAGC CATTGCAGTT TGCGCCGGCT CTTGAGGTTC AGTGAGCCAC TACTCCCCTT 293100
GGCACCGTCG AAACCGCCGA GATGAGAAAT GATATCATCT GCTATGCTCT GATCAGCTGC 293160
CGTAACGAGA TAGGTTTCAC GCCCGGCAGA TCTCGCGCGG CGTATCAGGT CAAGCATCTG 293220
TTCACGGTAG GGAAGCGCCT GCGGCTCAAC CGAACGGACT TTCGCCAGAG CTTCCTTCAG 293280
GGCCGGGCGC CCCTTAAACC AGTTTGGAAT CGTGCGAAGC AACTGCTGCG GGGAATGGAA 293340
GAAGGCATCA AACAGGTTCT CGTGAAGACT GTCTGATTTT ATCAGTGTGC CATCCAGGTC 293400
ACAAATCAAA GGCAACTTAC GATCAAAGCA GTCCAGATCA AGCACCTTCA TTTCATTTCG 293460
CCGCTGTTTC ATCAGTAGCA TCCTCCAGCC GGACGCCCCG AAACAGGTCC AAAGTACGGC 293520
GGGCGCATTT TCGGGAGGTG GTGAGCCTGG CACGCGCGTT CTGGTATATG AGCTGACGAG 293580
GACATCTGCT TTTTGCCTCT CTCAAACGAC CGCTAAGCTA TCCATCCCCA CCGGGGTCGG 293640
TATCTCCAGA CGCATGCGAC AGAGGAGCAT CAAATGTTGT GCCAGCTTGC ACAAACTTAT 293700
GGGAAGAGAC TTTCTGTATA TTGTTCAATG ACGTGCATGC GTGGCGCCTG ATCTCAGCTG 293760
GCGAGCGCTG TGTCGCGGAC ACGACAGAAT GGACAGCAGG TGTCTCTTGT TTGACGTTTA 293820
CAGGAACCGG CGCCATAGAG TGATCAGTGA GCGGGACGAG GGAGCTTGGA TAACCGCGGC 293880
ATTCCGCTGT CCGCCAGCAG TCGCTGCACA AGTCGGTACC CAGGCGGCCA GCTTCTACAA 293940
CGACGCCGAA CGCAAGGCGG GTAGGGCGCG CGGCTTGTCA TCTGAGCGGG AGAACCGAGG 294000
ATGCTCGTCA CTCGGCTGAA AGAGGAGCTC GCGGCCATTC AGAAGGCACG GCAAAGTACT 294060
GAATTGGCTG ACACTCCGCG ACGGAACGGC GCAAGAGTCG CGGCCGCAAC TGCATCCAAC 294120
CTCCGAATGT CCAATTCGCA TTCCGGCCAG CTGCGATCAC CATTATAGGG TGGCGAACTC 294180
GTCGCGAAAT GCGTCTGAGT GTGGATCGAT CTTCGGAACT CATTGCGGCG ACCCCAGCCG 294240
GATGGGAATG CCAATCCCCA AGATAGTCGA GGTCGCCCGC GGTGTTCCTG AATGCTTCGT 294300
CTATCTGCTC GACCTGCCAT GAGTGGTCCG GAATGAAAGT ATGGCGACCA TGCAGCGCTT 294360
TCGGGCCAGC ACCTACAATA TCAACGACAA CCCGATCTTC GCCATCTCTC CAACCGAGGA 294420
GAACACCGCC GGTCTCGAGC GGAAAGAATA TGTTGGCCTG TTCGATTATT ACTTGAGCTG 294480
CGTCGCGCCC CAACCAAATT GTCATCCCTG TGACGCTCCG CAGCTGCAGC TCGAATGTGG 294540
TGCGATCGTC TCGGCTTTCC ACCGCGGGAG AATGCGGCGG CCATTTTCGG TCAGATCAAG 294600

AATGGAAAGC TGCCAGTCAC CCGAATCGTA CACGTCCGGG GCGAGCAGTC CGATCGTGCT 294660
GCGTACAACT TCCATCGACA CTTCCTGCAG GTCAAAGGCG CCTCCGGTGA ACGTTGGCGC 294720
GTTGCAGCCG GTCGGAACTA TCGTGCCCGA GCTGTCAATA GTTGGAGCGG GGAGCGTCTT 294780
GCCGGACCAA TGCTGCTGCA AGCAGACATA ACAACCCGGG GCTCCCGGTT TGAATCTCGC 294840
CACAACGCCT CCGGCCGCGC CTTCGGTTGC GTGGCCGAGT ACGTACCGCT TTCCTATGCT 294900
ACGGCACATG TAGGCCAAGG CGCCCTGACA CTCGGTCGAG GCCGAAGTGT CTACCACAAT 294960
GTCGGCGTCC TCGATCAATG CTCGAAGCCA ACGCATGGGA TTCCCCTCGA GTTTGCGGAC 295020
GTCGTCCACA TTCGAGATTG CTGAGCCCAC TTTAGCGTGT CCACGCCCGA CATTTGTCCA 295080
AGGGTAGTTG TGATAGAGGA AATCGTGCAG CGCGGTTGTC TTTGGCAGGC CCCACACCGA 295140
ACGACCCAGC GCCCATCTGA CCGTGTTGCC AGGCTCGACG AGATCGAAAT CGATGATCGT 295200
CAGCTGACCG ACGCCTGAGC GCGCAAGCTC GACTGCAGCG AAACTGCCGA TGGCGCCGCA 295260
CCCTACGAGA ACAACCTTTT TGCTTTTCAA AGCACTCGCG ACAGGCAGTC GCGAGAGCAT 295320
ATCGCTCGAC AGACGGTATC CGCGCACCAA GGAAACCTGT CGCCGGCGGC TGCCAGGCAC 295380
GCGCCGACTG ACGAGAAAGA ACCAACCGTT GCCCTTGTTA TCAGGGCCGT AGGAAAGCTC 295440
CTCCTGAAAA ACCACGGCGG TAATCGATAG ATCGTCGTCG GCAATTGATC CGAGCTTCTT 295500
CAGGTGAGCG GGGAAGAGGG CCGATTGTCG CTCTATCGCC TTCTCGGCCG CCGCCAAAAT 295560
CTCCTCCGGG GCCTCGGGGA ATGGCGGCTC CAGGCGTATC CAGCGCCCGA TGACGTTTTT 295620
CCATGGGCCG GCCCGTTTCG GGAGATCGAA GGGCTTCGCC ATCCAGGTGC CGCTCAATGG 295680
CTCCACTTTG CTCAGAATGG CTTGAAACGG TGGAGACCTT CCTCGCTCCA GGCGGTCCTG 295740
GAGCAGGAAA ACTGCGGTTC CGAGGGGGGC ATTTGGAACG GTCTGCTGCC CATCAAAAAA 295800
GACCGCCGAA TACTCCTCGG CAACATGCAT ATGATATGGC GTTACAGGGT CGGGAGCTTG 295860
TTCTTCGAGG CTGGCTGCCT CACTCCACTG CTCGTTCCGC CGCAGTGTCA TGACTTGCAG 295920
GACCTGCGAT AACCGCTCGG CTATGAAGTC CGCGACCATT TGATGGGGGT ACCACTGGGC 295980
AGAGTCCTGC GTCAGCAGGC ACAAATCCCT CGTAAAGGGA TTCTGATGCC GCTCGAGTTG 296040
CAGTTCAGGC GCGGATATCG AGGGTCGGCA GAACGGATGC AGCGGCGAGT AGCCGACATT 296100
AAGTCGGATG GACCCGGCAC TGTCCATCGG CCAAAGGACC TCGAGGATCA AGGTGCCGTC 296160
CTGTTGGATT AACGGCCGGT GAGAGGCGCC GTGCCCCTCA TAGGCCGCCA ACTCCCGGGC 296220
ATACAAGCCA GGATAGGCCT GCCACCAATT GTCCTGATCC GAGCCGCGCT TAAGAACGTT 296280
CACCTCCGTC AGCCGACCGG CCGGTGGGAC GGGGGGTGAG AGGGCACTGG CGGGCCATCG 296340
GGGCGACCCG GAGTATGGCT GGGCGGCCCC TTCGGGACGT CGGGGTGTTC AGGATGACCT 296400
TCCCGGCCCG GCTTATTCGT ATTCGACATG AGTATCTCCT TACAATACAG GCCTCCGTTC 296460
TTGACGGAGT CCAATTCCGG ATTAAAATTC TGGATGAGTC CAGATATGGA TTATATAAGA 296520
ATGGACTCAT TCGTTGTTCA AGTACACACT TGGACTTAGG AGCAGATTTT TATGACAGAA 296580
CCAGTTCGAG GGTCATCTCC AAGCAGCGAT TCTGCTCGAG TCCCTGCAGA ACTCGAAGCG 296640
TTCTTCCGCA TTGCCGACCG GTGGCGTCTG TCTGCCGACG ATCAGATCAA GCTGTTGGGA 296700
TCTCCAGGTC GGTCGACGTT CTTCAAGTGG AAGAAGGCGG GCGGCACGTT GCCCGCCGAC 296760
ACGGTTGAGC GCCTGTCGCA CATTCTCAGC ATTTGGAAGG CACTGCGAAT TCTCTTTACC 296820
ATAGAGGAGC GCGCCGACGA TTGGATTCGC CGACCCAACG ATTTCTTCGA TGGAGCAAGT 296880
GCACTCGACA CCATGCTGCA GGGGAGGGTT GTCGATATCT ACAGAGTGCG CCAGTACCTC 296940
GATGCTCAGC GTGGCGGGTG ACAAATGGAT GAGGTCGCCA TCAAGGACGA GTTTTATCGG 297000
CTAATTCCGT CACGGTTCCC GCCAGTCGCG GTGTACGAGG GATTGGTCAG CGAGGACAAG 297060
ATCGAGGCAC TTGTCGAAAT AGAGAACAAG ACGAATCCGC GTCTCCAGTC GGAGGGCCGG 297120
CTGCTATCCG CTCATACCGA TCCGAGGGCG CCGAGGCTGC AGAACTGGAA CCTGGCTCCC 297180
TTCAAGTACC TGAACCCGGA GGGAATCCGC TTTTTCGACG GTAGTCGCCC GGCACTGGAA 297240
TTGGCAGACG ATCGTCAGAC CGCGCTCGCT ATGTCTGTCG AGCGTCGTCA GGCCTTCTTG 297300
TCGCGAACCA AGGAAGCGCC AATCGGCCTC GATATGCGAT TGCTCAAGAC ACCGGTGAGC 297360
GGGCGGTTCA TCGATTTTCG AAAGTATCCG ATCGATCTAT CGTGCGAGGA ACGCTGGAGA 297420
TTGGGAGGTT CGGTGCCCGA AGGCGCGGAC GGCGTGATTT ATCATCCGCC GGAGCGGCCC 297480
TCAGCCATTT GCATTGCGGT ACTCCGAGGC GACGTTCTTG GCCGAACGAT TCAGACGGTC 297540
CATTACCGCT ACGTTTGGAA TGGTACCCGA ATATCCCTCC TTTATGCCTT TGATGATGCA 297600
GGCAACGAAA TTCGCCCAGA GGTATTGGGT GGCGCGGATG ATGCATTCGC CGTGTGAGCA 297660
TTAAATCTAG CAAAACACAC CTACATTCCG CTTAAAATCG ATTTCGCCTT CTGCTTATTC 297720
ACAGTGATAT TCTCAGCAGG CAACTCTACC CTTCTCGTCG CTTTGTGACC TCCTCTCGAA 297780
ACCGGGAACG GAGTACTCTC ATCTCGGCCT TCTCTAAACG CCTGCTGATC TTCTCAGCCG 297840
TCTGGGCCAG GAGCCTGCCG TCGAATTCGG GGGGAACTCC CCTGTTGTCC TTCCAGAAGA 297900
TTTTAATTGC CTGAAGGTCG GCCTCTTGGA GATCTGCGAC GAAAGAGCAC GCGGCAGCAA 297960
TGATAAGTCC ATTATCATCA TCGGCAGTTT CTGCGGCGGG TACCGCCGCT TTGGCGGGCG 298020
ATGCCGCTAT AGCGGTCATT GCAGGCTGGT GAACGGACCG CAGCGACAGA AACTTTTCGA 298080
CGGTGAGCTT GTCCCATTCA GAGGTGGTGT CGGCTTCCTG TCTGAGGGTC GGCGACTGCG 298140
ATCCTCGCGC CGGATGAATG CCCAGCAAAT GAACCCGAAC CCCATAGTTC TGAGCAATCT 298200
GCACGCCGAC TCGGACATCT TCATCGCCAG ACAGCAGCAC CACCTCAGAG ATAGCCCTTT 298260
GCCGTGCCAA CTCTATCAAG TCAGTCACGA TAAGCGAATC CACGCCCTTT TGCTGCCCCT 298320
GCCCATTAAT GAACCCGAGG CGCAACTTGA TGTCATCCAT GTCGGCGATG GCAGTCTGAT 298380
CAGCGCTCAT CGCTGCACCT GGTCTTATTC CATCGTACTA GTAAATGCGC ATCAGTGACC 298440
CAGTAGGGGC TTTCGTGGAA CAGAACGCCT TCAGTTCCTC AATGACTGAC CTCGCCGAGA 298500
GTGACAACTT CGCCCTAGCC ACTTTGTTAC CGGTGAGTGC AGTGCTCCCC TGCGGGAACA 298560
GATAGCCCGC GTCGACGAAA ATAGCAGAGC GCCCCAAGAT CCCCCAACCC TAAAAGTAGC 298620
AAGGGCTCCC GCGGGAGCCC TTTGTATGCC GGCGCAACCA TAATATCTAA TCGGTCACGC 298680
CGGAGTAATT TATGTATGCG CCACGCATGC GCACAAGTCA ATACGAAACG ATAAACGCTG 298740

CAGGAAATGG ACGCTAATGA GACGCAACGC ACGCCAATCA GACCTAATCG CGCTCAATTA 298800
GATATTCTGC ATATACGCAG CTGCAACAAG TTACTACGGA TATAATGCCG CGGGCGCTGC 298860
AACGCAAGAG GCCGAAGCAG CCTGCATCGC GCAAAATCAA TGGCTATCGA CGGACAGGCG 298920
CCGCACCGAC CAGTCCGCTT AATTTCCGGC GCCGGCCGAA GGCATCCCCG TCAGACTGCG 298980
GAAAATGTCG ACCCAGAATG CGAAATCGTC TGCTCGCCCG TCGGTCCAGG CCTCAAGACC 299040
GCAATGCGCC ACGGCAGTCG CTGCCTCGTC GCCGAACAGG TCAATCGCGG CTTCCATCAT 299100
GCTTTCCGGG TCCGAAGGTT CTGGCCAGAT CGCCTTCATC ATAGTCATAC ACCTAATCGC 299160
TGCTTCTCGG GCGTGAGGAT ACTACGCGCA TTTGAGCGTT GCGCAAACGA TCGTTTATAC 299220
ACGCGTTGCA CAGGCCGCAG AAAACGCAAT GAAATCAGCT GCCTAGAGCC GCGTATCCGG 299280
GCAGGATTGC ATAAGCATGC TCCATTACGC AACGGGGTAT GATGGGCGAG AAGACCGGAT 299340
ATGCAAGGGT TTCCACCCTC GAACAGAACC TCGATATGGA ACAGCGCGCT CTCAAAGCGG 299400
CGGCAATGGT CACGTCCGCT GCGCCCCTGG TCGCCCCTCG ACGGGATCAG GTGAACAGCA 299460
TTATCCGATT TGCGCGCAGA TGCCCTATCA ACTCGCGATC GATGTCGCTT GAGGCCGCCG 299520
ACGGCCACAG AGACCTTAAC GAGCGAACCG TTGTCGCGAT ACTCCTCGAG CGTGAAGTTA 299580
TTGACCACCT GCTTGCGGAT CGAGATCGAG CCACCTTCGG GTGCCGCGCG GCGGCAAGAC 299640
GATCTGGATA CGCTCGCCGG TCGGAAGCGC GGCGCTGAGG ATCGGTTTCG AGCGGCTGAT 299700
GAACTGGTTC GTCGCGGCCG CAACACGCTC GCCGACGTTC TGGATTTCAT CACCCATCAG 299760
GGCGACGATT AGATGATGCT CCATGTAATC GGCGCCAAAC CGTTCCACAT ATACATCGCC 299820
CGGTTTGTTG ACGGCGATTT CCACCACCCT CGGATCGTCG ATAAATTCCC TGATCGGTTC 299880
AAGTGCGGAT TCCGACGTGA AGCCGGCCGC ATTCCGATCA ATATCCGGCC ACCGTTCCGA 299940
TTTGAAGCCG GCCACGATTC CAATCAATTA CCGGCCGGTT TTCGGCTCTA ACGATACCCC 300000
CTGGGTCAGC AACTTTGGCC ATCAAGCCTC CGGGTACACG AGGAGATATT GATGCCGGCG 300060
AAGAGAAGGC TGACCATGAG ACAATTACGA CAAATGCTCC GGCTTGGCGG AAGCGGAAAA 300120
GCAGCCGCGA GATTGCGGTG ATGCTTGGTG TTGCGCGCAG CACCGTGCAG GATAACCTGA 300180
AGCGGGCGGC GTTGGCGGGC CTGACCTGGC CACTGCCTGG CGAACTGACC GACGAGGCGC 300240
TCGAGCATCG GCTGTTCGCC CGCGCCGGGG TCAAGCAGGG CCAACGGCGA CGCCAAGAGC 300300
CTGATTGGGC CGAGCTCTCC ATCGAACTGA AGAAGCCGGG CGTGACGCTT CTTATGCTCT 300360
GGGAGGAATA TCGCAGCGTC CATCCCGACG GATACTGCTA CAGCCGCTTC TGCGAACTGT 300420
TCCGCGGCTT CGAGCAGCGC TTGTCGCCGA CGATGCGCCA GGAGCACGCC GCTGGCGACA 300480
AGGCCTTCGT GGACTATTCC GGCAAGGGGG TGCCAATCGT CGATCGCAAG ACCGGCGAGG 300540
TTCGCGAGGC GGAACTGTTC GTCGGCGTGC TCGGCGCGTC GAGCTATACG TTCGCCGAGG 300600
CGACCTTGAC CCAGACGCTC CCGGATTGGA TCGGCTCGCA CGTGCGCATG TTCGCCTTCT 300660
TCGGTGGCGT GCCCCGGCTG ATCGTGCCGG ACAACCTGAA GTCCGGCGTC AACCGGGCCA 300720
ACTTCCACGA TCCCGAGATC AACCGCAGCT ACGGCATGAT GGCGTCCCAC TACGGCGTCG 300780
GCGTCCTACC GGCCCGGCCG CGCCGCCCGC AGGCCAAGGA GCAGACATCA CGATGCACCT 300840
TGTTGGAATT GTTGAGCGTC AATTAACCTA GGTTTGCGGC TACCATAGCG ACCGCCTACG 300900
CCTTTTGCGA GTCTAACGTT AGCCGGTGGC TCATACAGGT AGCGGTGGTT GGTAGCGTAG 300960
CGATGGCGCT TTAGAACACC CTCGCGTCCG AGTTGGTGTA CGGTTGTGGC ACTTACGCCA 301020
AATCGACGAG CCACTTCCTC GCCAGTTAGC ATGCCACTCT CGCGCAACCG TTCGAACCGG 301080
GTCTTCAGCC CATAGGCTCG GCGAACGAGC ATGACTTTCT TCAACGTAAA AGCCTCACCG 301140
CGCCAGTTAC GATGCCCGAG TGCGTTGAGA CGACTGGCAA TCTGCTGGTC GTTGTCGGTC 301200
TCCAGAAGTT TGTTGATCAG TGCCACAACC GCTTCCGGGG TCTTGCGGAT CACGGCCATG 301260
GGCCGGGGAC GAGCTACTGC CAGGCTTTGC GTGCGACCTC CGCAATGTTG ATCTGCTCGT 301320
CGACCAGCAG TGTCACGTCT TCGATGAGGA GGCCAAGCAT GCGTTTACGC TCGACCGCGC 301380
TGGTGCGCTC GTTGTTCCAG ATGCGCGGAA AATCTGCGGC CAGCGCTCGG ATCCGTTCCT 301440
GCATAGCGCC GTCCAGCAAT GACTGGTCTG TCTCGTTCTG ACGGTCATGT TCGCGCTGAA 301500
GCGTATCGAG GTCGCGTAGC TTGCCATTCC AATCCGCTTC GAGTGCGTCG GCAACCAAGC 301560
GGTTGTCGGG ATCGACCTTC AGATAGCGGC GGCGTGCGAG TTCAGCCTCA TACCGGGCGC 301620
GCTGTAACTG CTTGTCGCGC AGCGAGGCAG CTTGTTCGAC ACGTTGTGAG ATCTCCTCCT 301680
GCACCGCAAG CGCGACCTCT ATCGCCGTCG GTGCCATTTG CTTCGAGCAG CAACGCGCTG 301740
ACCGCTTCAT CGATCGCTGG TCCTCTTGCC CATTGGCAAG CCTTGCCCGC GTGGCGCACG 301800
ACGGCCTCGT TGCAGATGTA ATAGGGACGA AGCTTGCCCT CGAACTGTTC ATAATGGACC 301860
CGCATGCGGG CGCCGCAGCG ACCGCACAAA ACTCTTCCTT GCAATAAGCC GTTGCCCTGA 301920
CGAGGCATAC GACCCCGCAG GCCCGGAGAA AAACCGACTG CATTTTGTTC GAGACTTGTT 301980
TGATTGCGTT CGAACTCGTC CCACGAGATG TAGCCTTCAT GGGCCTGAGG GATGAGCACC 302040
TGCCAGTCGG ACCTCGGCAT CTTCTGCTGT ACGGACTTAA GCTTGGCATT GTAGATCGTG 302100
CGTGTGCGTC CGTATGCGAA AGCACCAGCG TATCGTGGAT TGTGCAAAAT CTGGATCACG 302160
CGAGAATGGT CGATCTGGCT CCACAGGACG TCGCCCTTGC CGATGCCGCG ACGGATACGA 302220
CGGGGAAACA GGATCTTCTC GCTGCGCAGG CGGCGCACGA CCGCACAGGC TGAGCCCACC 302280
TCCCGGAAGG TATCGAACAG CATGCGCACC GTGTCCTGGA TCTGGCGATC CGGATCGAGC 302340
ACCACGCGCA TATCAGGCGT GTAGACCAGT CCGATCGGCA ACGGTAACTC GAGCTCGCCA 302400
CGACGCGCCT TGTTGAGAAT GCCGCCTTGC AGTCGAGACT TCAGGATGTG GAGTTCGGCC 302460
TCACTCATCG TGCCCTTCAG GCCGAGCAGC ATACGATCGT TGAACGACGC GGCGTCGTAA 302520
ACGCCATCTT CGTCCATGAT GAGCGTGCGC GACATGGCGG CCAGTTCCAG AAGGCGATGC 302580
CAGTCAGCAT TGTTGCGAGC CAGGCGAGAT ACCTCCAGCC CCAGCACGAT CCCTGCGTGC 302640
CCCATCGCGA CCTCCGTCAC CAAGCGCTGA AATCCATCGC GGTCCTGCGA CTGTGCGCCG 302700
GAAAGGCCGA GGTCGTTGTC GATGACGTGG ACGCGCTCAA TCGGCCACCC CAGAGCCACC 302760
GCGCGGTCCC GTAATGCATA TTGTCGCTTC GTGCTCTCGG TGTTCTCGAA CACCTGACGA 302820
AGGGAGGATT GGCGGACGTA GAGAAATGCA TCGCGCCGCA GGTGATCGGC GCTAATCTTC 302880

ATGGCATTAT CAGTAGACAT GGCTTCCTGT CTCTGCTGCC AGCACCATAT TGGCGAGCAT 302940
GTGCACGAGC TGGCGGTCGT GCGCGACCAT TGAAGTTGTC TTGAACCGAG GCGCTGACTG 303000
CTGTCGCGCT ATTGCGGATT GATGTGGTGC GATCAGCACT GCCAGGCCGC GCCACAGTCC 303060
GTGAAAGACG ATGGCGCCCA TGCGCCTCGC GCAAGCCGTG CCGCAAATAA CATCGGCGCG 303120
GAGCGCTTCG TAAACATCCT TGCCGTTCTC TGACATGGCG ATTGCGTGGG TCGGCTCTTC 303180
ATCGTTTTTT TTTCCGCGCC ACCGCGCGTT CGATGCTGCG AGGATGGACG CTGATGCCCA 303240
GTCTCGAATG CACCAGGTCC GCCAGAGAAC GGGCCTGCAG TTGGCCGTTC GCGTCGAGAT 303300
GCTCCTCGAC GAGACGCATG ACCTCGCCAG TAAGCTTGTG GGCTGACTTC GGCCCACGGG 303360
TGCGCGGCAA GAGGCCGGAC ATGCCATCGC GCTCGAACGC GGCCTCCGCT TGATAGTAGG 303420
TCTGCCGAGA AAGCCCAAAA AGCGCGGCCG CATCCGCTTT GTTGGTGCCG TCTTCGCGGG 303480
CGTGCCGAAG CATCTCATAC TTCACTTGCA CGAGATCGAG CGGATCAAAG AAACCTGCTT 303540
CCCGGAACCA GGGGGCGCGG ACGCTCTCGG GCTTGGGGTT CAGAGCGCCC ATCTGGCGCA 303600
GGCGATCGGG CTTGCTTTCT TTGGTCATGG GAGCCTCCTT GTGTAAGATA AATTATGCCG 303660
CACTTGGCTC GCACGTCAAT TAAATTATAT CGCTAATATC GCCGATACGC ATGCATCACA 303720
GGGCTTCACT CCTTGCTTTG ATGATCAAAG ACATCGTACG CCCGGCATAA TTAGCCTTAC 303780
ATGTGCGGCG TAGTTTACTT GACGCTTCGT CACTCTTCCT GACGGCGCTT AATGCGATCG 303840
ATCAACGACG CCAGCGCCGA CAGATCGTCT GGGCGGGAGA AGGACCGACC TGCAGCAACC 303900
TGACTGAACA AGTCGGCCTC ATTAACATCG GTCCACGAGG CCGGCAGCGA ACGCAACCGT 303960
CCTTGAGCGT CGTAAAACAT GACGCGGTCT TCGCCCCAGT TCTGTTTGCG TGTCGACAGC 304020
ACGAAGCGCT GTCCGCGCCA AGGATGGAAC GGGTGTGTGA CCTCGAACTC TATGCGGGCT 304080
TGAGTGTCAG TACGAACTGC AGTCCTGGAC TTGAAAGAAG GCGAAGCTCG AGAACGCCGT 304140
GCGCTTTGCC CAGAGCTGCA TTCTCGGACG CCTCCGCAAG CAGACCTTCT TCTCGCTCGC 304200
CGAGGCTAAT GCCGCGATCG CCGAGGCGCT CGCGCGCATC AACGATCATG TCATGCGCCG 304260
GCTCGGCGTC AGCCGCCGGC ACCTCTTCGA GACGGTCGAA CGCCCGGCAC TCGCAAGCCT 304320
GCCCGCCGAA GACTACGAGT TCGCCGAATG GCGCTTGGCG CGGGTCTCGA CGGATTACCA 304380
TGTCGAGTGC AAGAGCTTCT TCTATTCCGT CCCGCATGCC CTCATCCGCC AGCAAAAAGG 304440
CGCTGAAAAG TGATGCGATA AGCGCCCTTT ACGAAGGATG ATCGATCAGC CGTTTCAATG 304500
TCGTCAAGAT CGAAGAGACG CTGACGATCG AAAAGTTTTT CCCAGCTACG CCGCACACGA 304560
GCGCTCCTAT GTTCGGATCC GTTGCTCCCC GTTGCTATTC CGCGCAACCC ATTTCGCGCG 304620
CCCACTCGAT AAGTGTTCGG CTCTCGCGGA TGAATGCGCA TCTCAGACGC TCGCCTTCTA 304680
CTCCGCCTGC CTTGTCCTTG GTGATTGAAC TTCCTCAAAG GGCCTGACCG TTACCCAATA 304740
TTACTCAATC GTAGGGCTCA TCGGCGTTGA ATGCGTCATG GGGGTGCCGT AAGCCATCAT 304800
ACATGGAGCG AGAGGAGAAG GCGGTCGAGG GGGCCGCAGA TGCTTCAGCA AGGAGGAGAT 304860
ATGAAACGTC GTACATTCAC CGCTGGGCTG GCGGCTTTGC CGTTCCTCGG CTCCAGCCTC 304920
ACCCGCGCAT TCGCGCAAGA TGCTGCCGCC TCGCTCCCGA AAACCTATTC GGGGCAGAAG 304980
ATCCGAGCGC TGGCCTCGAC CGGTGCTGCC TTCGAAGCGA TGGCTACGGT CAGCCGAGAC 305040
TTCACCGAGG CGACCGGCAT CGCCGTCGAA TACGTCAACC TGTCCTATAA CGAGCAGTAC 305100
CAGAAGCTGA TCCTCGATCT AACCAGCGGT GCGGCGTCTT TCGACGTCTT CAACTTCGCC 305160
TATCAGTGGA AATTCGAGAT CGAGCCGTAT TGCGCCGATC TTGCGAATAT TCCGAAGGAA 305220
ATTCAGGGTG CGCCAGATCT CGCGCTTGAC GACTATCCGC AACGAGCACT CGAAATCTAC 305280
GGCCGTGCCA ACAACAAGCT CATCGGTCTG CCGACACTCG GCGATGTGAC GCTGTTCGTC 305340
TGGAACAAGG AGGCCTACAA GGCCGCGGGG TTGGATCCTG ACGCCGCACC GAAGACCTGG 305400
GACGAAGTCG TCGAGCGCGG CGCCAAGCTC GTCAGCAACG GCCAATTCGG CTACGCCATG 305460
CCGGCGGGCA AGGGCATTCA AACGACCGTG ACCTGGATCA TGGTGTTCAA GTCGATGGGC 305520
GGCGAATATT TCGATGCGTC CGGCGCCCCG ACCTTTGCCA GCGAAGCCGG CGTCAAGACG 305580
ATGAAATTCC TGGTCGAGAA GCTGGCGGCG GTCAGCCCTC CCGGCAATCT TGCCTGGGAT 305640
TTCCCGGAGA TGTTCAACAG CCTGTCGACC GGTCAGTCGG GGCAGTCGAT GATGTGGCCC 305700
GGAGCTTTCG GCGATCTGCT CAACCCGAAG AGGTCCCAGG TCCACGACAA AATCGGGTGG 305760
TCGCCGATGC CGCAAGCTTC ACTGTTGGGT GGCTGGTCGA TGGGCGTCAA CGATGCCTCT 305820
CGGTCAAAGG ATGCCGCCAA GCTTTACGTG GCATGGCTCA CATCTCCCGA CATCGTTCGC 305880
CGCATGGGTC TTATTGGCGG GGCTCCGGCA CGCATCTCGG CGCTAAAGGA TCCGGAGTTG 305940
ATCAAGCAGG CTCCGAACCG ACCGGCTGTG CTTGCCGGTC TGCAGGGCGA CGTCGCCGAA 306000
TATCCGCCGA TCAAGGAAGC GGAGCAGGTC CACATCATGA TCTATGATGA GGTGAATGCG 306060
GCGGTGGCGA AGATCAAGAC GCCGGAACAG GCGGCCAGCG ATCTTCAGGG CAAGGTTGAG 306120
TCTTTCATGC GGCGCCGCGG CTATCTCAAA ACCTGAGGTC GCGCTCCGTG GGGGGCAGGC 306180
GTTCCTCCCC GTCTGCCCCC CCTTTACAAC GGCAAATCTG CAAGTAGTTT ACCTGATGAC 306240
AATCTCTCAA ACAGTTCCAC GGGGCGCAGT GGCGGTTCGC CGGCGTCGCC GCATCCGCAT 306300
CAGCACTGTG GTGTGGTTCA CGATGCCTGC TGCGGCAATC ATGTTGCTGG TGCTCGGCGT 306360
CCCGCTGGTC TACTCGTTCT ACTACAGCCT CACTGGCTGG TCTCTCGTCG TTCCCGGCAG 306420
CGATCAGGAC TTCATCGGCC TTCTCAATTA TACCGATGTC TTGAGGAGCA GCGAGTTCTG 306480
GGCGGCGATC CGCGTGACGC TGATCTATGC CGTGGTCGCG GTCTCGCTGG AATGCGCGCT 306540
CGGCATCCTC TTCGCCGTGC TTTTGAACCT AGAATTCTTC GGCCGCGGCC TCTTTCGCTC 306600
GCTGATGCTG ATCCCGATGG TGATCACCCC GGCGGTCGTC GGCATCTTCT GGAAGCTGCT 306660
CTATGAGCAG GATTCCGGCG TCTTCAACTA TCTGCTTGGA ACCCTCGGCT TCGAGCCGGT 306720
ACCGTGGCTG AGCCTCACCG TGGCGCTCGC CTCCGTCATC ATCGTGGACG TGTGGCAGTC 306780
GACGCCGTTC TTCACGCTGA TCATTCTGGC GGGGCTGCAG TCCCTCGACC GCGACACGGT 306840
GAGCGCCGCG CAGGCGGACG GCGCCAATGC GCTCCAGGTC TTCCGCTACC TGACGCTGCC 306900
GCATCTCGTC CCCTATATCA TGATTGCCGC GGCATTTCGC ATTATCGGCG TCATGGCCGA 306960
CTTCGACAAG ATCTTCCTGT TGACGCTCGG TGGGCCGGGC AATGTCACGA CGACGCTCAG 307020

CGTCTACGCC TACAACACCG GTTTCAAGGT CTTCGATATC GGCAGAACCA CGGCGATCTC 307080
GTGGATCTAT GTCGTCTTCG TTCTCGCGAT CAGCGCGCCC CTGATCTGGC GCCTGTTCCG 307140
CGGCGCAAGC GTCAACCGCC ACTAGGGGAG GTGAGGCATG GCTATTTCAT CCAACACCAG 307200
CCGCACCACC CGCAAGCTGC TCTGGACCCT CTTCTGGTGC GTGCTCGCCT TCGTCTATCT 307260
GTTTCCATAC ACCTGGATGG TGCTGACCGG CTTTCGTCAT GGCGTCGACA CGATCTCGAT 307320
GCCGCCACGC TTCATCTTCG AGCCGACGCT CGCCGGCTTC AAGCACCTGT TCGAGGTGAC 307380
CGGGTTTCAG AAATACATCA TCAACAGTGC GGTGGTGACG ATTGTCAGCG TCGTGCTGGT 307440
GATCGCAGTC TCCGCGCCGG CGGCTTACGC GCTTGCGCAT GTCACCAAGC GGGGCGGTAC 307500
TCTCCTGGTG GCGATCCTGG TCGCCCGCAT CATTCCCGGC ATTGCGATCG GCGTGCCGGT 307560
CTACCTGCTG GCGACGCGCC TCCACCAGCT CGATACCTAT CAGGCGCTGA TCATCATCAA 307620
TGTGGCCGTC AACATTCCCT TCGCGATCTG GCTGATGCGC AGCTTCTTCA TGGAGGTGCA 307680
TCCGACGCTC CGCGAGGCGG CGATTTCGGA CGGTTGCAGC GAATGGCAGG TCTTCACAAA 307740
GATCATGATG CCGCTGGTGC TTGGCGGGAT GCTGGCGACC GCCGTGTTCG TGTTCATCGC 307800
GGTGTGGAAC GAATTTCTAT TTGCTCTCAT CCTGACCACC TCCGTTAGCC CGACGGCACC 307860
TTTGGCGATG CTCGGCTTTA GAACCCAGTA CGGCGTGCAG TGGGACGCTG TCGGTGCTGC 307920
GGCGTTCCTG GTGTCGACGC CGGTCATCGC CTTCGCCTTC ATAATGCAGC GCTACCTGGT 307980
GCAAGGCCTG ACCATGGGCT CCGTCAAGTG ACAGGCATGG TTGGTACAAT TCTTCGCAGA 308040
AAGGCCGAGC AGAATATGAC TGATGTAACT ATCCGCAATG TCACCAAGCG CTATGGCGCT 308100
CTGACCGTCA TCCCTCAGCT TTCGTTCAGG ATCGAGGACG GGGAGTTTGT CGTGCTGGTC 308160
GGACCGTCTG GCTGCGGCAA GTCCACGCTT CTGCGCATGC TGGCGGGACT GGAGGAGATC 308220
TCCGGCGGCG ACCTCCTGAT GGGAGCCGAC GTAATCAATG ATCGCCCCGC CAAGGAGCGG 308280
GACATGGCGA TCGTCTTCCA GAACTATGCG CTATACCCGC ACATGACAGT CGCGGAAAAC 308340
ATGGGCTTTG CGCTGAAACT GCGCAAACGC CCGCGGGCCG AGATCGATGA ACGCGTCGAC 308400
AAGGCGGCGG CGATCCTCGG GCTCGGCAAG TTGCTGGATC GTTATCCGCG CGCGCTTTCC 308460
GGTGGCCAGC GACAGCGCGT CGCCATGGGC CGTGCGATCG TGCGCGACCC GCAGGTCTTC 308520
CTGTTCGACG AGCCGCTGTC CAATCTCGAT GCCAAGCTAA GGGTGCAGAT GCGGGCCGAG 308580
ATCAAGGCGC TTCACCAAAG GCTGAAGATA ACTACCGTCT ACGTGACGCA CGACCAAATA 308640
GAAGCCATGA CCATGGCTGA CAAGATCGTA GTCATGAACG AGGGACGGGT GGAGCAGATG 308700
GGCACGCCGC TCGAACTTTA CGACCGCCCG GCCAATATCT TCGTCGCTGG CTTTATCGGA 308760
TCGCCGTCGA TGAACTTCCT GCCGGCGACG GTCGCTGCGA CGAACGGCCC TCTTCTGAAG 308820
ACGCCGGAAG GCGTGGCGCT GCCAATCGAT GGTGGTCCGA CGCTTACCGG ACGCTCGGAG 308880
GTGACCTATG GCATTCGTCC GGAGCACCTG CAGCTCGGCG AGACCGGTAT TCCTGCAGAG 308940
GTCGTGGTCG TCGAGCCGAC GGGTTCCGAA ACACAGCTCT ATGTCACGGT CGGAGGGCGC 309000
GAAGTCGTCG CTGTCCTGAG GGACCGGGTC GACGTGCGGC CGGGCGAAAA AATCTGGCTA 309060
ACACCAAGGA AGGGCTGCGC CCACCTCTTC GACCCGAACA CCGGCGCACG GATCGCGGGC 309120
TGATTGCCCG CTCACGTCCC GCCGACCGCG TCTGGAAGTC GAAACAGCCC GAGGGAGGTC 309180
TCATGCGCAA GCTCCGAGAA CTCGGCGAGC ATGGCGTCGT TGGCATGCCG TGCCGCGATC 309240
AGTCCGCTGA CGCCGATCGC GACGGCTTCC TGCGCCATGC CGAAACTGTC GACGACGGCG 309300
AGCGTGCTGA AGCCCTTGGC GATCGCGGTC CGCAAGAGAT TCATCTCGGC GGTCACGCCG 309360
TAGCCGAAGT CCTCGAGGCT GGCGCGCATT TCTCCGTCGA TCGCTGTCAC GCTCGGAAAG 309420
TTGACCACTC TGTGGCAGCC GGCGGTCTCG AGGGCTTTGA GGATGTCGAC GTGGCGGCGG 309480
AAAGGATCAA GGGCGAACAC GCCCGCAAAA ACCTGGCTCT CGCTCAAAAC GCCCTTCCGG 309540
AGCTCGGACA AGAGACGTTC GTTGGCGTCA TGGATCGGGA GCAGCACCAG GCTGTCATAG 309600
GCGCTGCCAC CCAATCCCTC GCAGGACGGG CAACAAAGCG TCACGGATTG TAGATGATCG 309660
CCGGGAAGGG CGGCGAGATC CGTCACGATC GGAATGGAGG GAAGTGTTGA GGGAGAGTGC 309720
ATTGTGTACC CGAGAAACGT TTACCCAATT CTACCCAAAT CGGCGGCCTC AGCCAATTGA 309780
TTTGCTGTGG CGCGCCACTA CCGTTTCTTT GAGGCGAGGA AAGCCCGCTG AGGAAGGGCC 309840
GGGAGGAGAA AGATGAATAC AGTTTATGTG GTCGGGACCT GCGATACCAA GGGCGGCGAA 309900
CTGCGCTATC TCCGCGATCT TATCCGGCAA GCAGGGTGTG ACGCTGTCCT GGTCGACGTT 309960
TCCGTCTCGG AGTTCCACAG CCAAGCTGCG GATGTCGACG TTCAGCCGTC GGAGGTCGCG 310020
CGCTTCCATC CGAACCCGCC GAAGCCCGAG GATCTCAAGG ACCGCGGCAA GGCTGTCGCC 310080
GCCATGGCGC AAGCCCTGGT GGAATTTATC CGGTCACGAC CGGACGTCGA CGGCATCATT 310140
GGTGCCGGCG GTACCGGCGG TACGGCGCTG ATCGCTCCTG CCCTGCGGGC GCTGCCGATC 310200
GGCACCCCGA AGGTGCTCGT TTCCACCGTC GCCTCAGGCA ATGTCGCCCC CTATGTCGGG 310260
CCGACCGATA TCAGCATGAT GTACTCGGTC ACGGACGTCT CCGGGCTTAA CCGCATCTCT 310320
CGGGTGGTCC TCGCCAACGC CGCCCATTCC GTAGCCGGCA TGGTCCTCAA CAAGGTTAGC 310380
GCCGCCAAGG ACGAGAGGCC GGCCATCGGC CTGACGATGT TCGGTGTCAC GACCCCTTGC 310440
GTCCAGGCGG TCACCCGTGC GTTGGAGGCA GACTTCGACT GCTTGGTGTT TCATGCCACC 310500
GGAACCGGCG GCCAGTCCTT CGAGAAGCTC GCCGATTCCG CCCTGCTTGT CGGCGGGATT 310560
GATGTCTCCA CGACGGAGGT CTGCGACTAT CTGGTCGGCG GCGTCTTCCC CTGTACGGCC 310620
GATCGCTTCG GTGCCTTCGC CCGGACCAGA CTGCCCTATG TCGGCTCCTG CGGTGCGCTC 310680
GACATGGTCA ATTTCGGGGC GATGGACACA GTGCCCAGCC GATTTCGCTC GCGCCGTCTG 310740
CATGTCCACA ATCCCCAGGT CACGCTGATG CGCACGACCC CTGAGGAGTG CAACAGGATT 310800
GGCGAATGGA TCGCGGAACG GCTGAACCTC TGTGAGGGGA CGGTCCGCTT CCTGATCCCG 310860
GAGCTCGGTG TCTCGGCGAT CGATGCGCCC GGTCAGCCCT TCCACGATCC TGAAGCAGAT 310920
AGCGCGCTCT TCGCAGCGCT CGAGCGCACC CTTCGCCGGA CGACCAAGCG GCAACTCATC 310980
CGCGTTCCCC TTCACATCAA TGATCCGCAA TTTGCGGAAC TCCTTGTCAC CAACTTCAAA 311040
GAGGCCCTTC GTGAGCACTG ATACTCTTGT CAAGCGTCCC ACTCGTTCTG AGTTGATCGA 311100
CCGTTTCCAT AGTAAGATCC GCGCCGGCGA GCCGATCATC GGCGGCGGCG CCGGCACGGG 311160

CCTTTCGGCC AAGAGCGAGG AAGCCGGCGA TATCGATCTG ATCGTGATCT ACAATTCCGG 311220
CCGCTATCGG ATGGCCGGCC GCGGCTCGCT CGCCGGGCTT CTCGCCTACG GCAACGCCAA 311280
CCAGATCGTC GTCGACATGG CGAGCGAGGT TCTGCCGGTC GTCAAGCACA CGCCGGTGCT 311340
TGCCGGTGTC AACGGCACGG ATCCGTTCGT CGTCATGCCG ACCTTCCTGC GCGAACTGAA 311400
GGAGATTGGC TTTGCCGGCG TGCAGAACTT CCCGACGGTT GGTCTTATCG ACGGGCTTTT 311460
CCGTCAGAAC CTCGAAGAGA CCGGCATGAG CTATGCCCAA GAGGTCGCGA TGATCGCTGA 311520
GGCGCACAAC CTCGATCTCC TGACGACGCC TTACGTGTTC GGCCCGGATG ATGCCGTGGC 311580
GATGGCGAAG GCCGGTGCCG ATATTCTCGT CTGCCATATG GGGCTGACGA CCGGTGGAGC 311640
GATTGGCGCG CAATCAGGCA AAACGATGGA GGACTGCGTC GCCCTCATCA ACCAGTGCAT 311700
CAAGGCGGCG CGGGAAATTC GCGACGACAT CATCATCCTT TGCCATGGCG GCCCGATTGC 311760
CAATCCTGAG GACGCGCGCT TCATTCTCGG CGCCTGCCCC GGTTGCCACG GTTTCTATGG 311820
CGCCAGCAGC ATGGAGCGGC TGCCGACCGA AGAGGCGATC AAGTCGCAGA CGCTCGCCTT 311880
CAAAGCGATC CGTCGCAAGC CGGCCTGACC GTGAGAAGGG AAAGCAAGCA GATGACCATC 311940
TTTCGAAGTG GAGACCAGCC GCCGGGTTGG TGCGAACTCA CCGACTTCGA GTTCGTTGAC 312000
TTGACCGATC AACCCTTGCC GATTCCGCTT GCCGCCGAAA AGCAACGTCT GCTCGTCACG 312060
AGCGGATCGT GCTGCGTGAG GAGCGCCGAG GGAGCGCAGG TTCTGAGTGA AGGCCAGTTC 312120
CTCGACATGG ATGGGGCCAA TGGTCCCTTC ACCGCGGATG CCGGAGAAGG GGCCGCACAA 312180
GTGCTCGTTT TTTATGGCAG ATGGGGCAAC GAACTCGGCG GATGCGGGGT GTTCAAGCTC 312240
GGCCCCGACA CCCCGGTTCC GGTCAGGGGG GATCCGGTTA ATTATCCCAA GTCGACGAAC 312300
TTCGATTCGC ATTATCACGA CTGCGACGAA TACTGGGTAA TTATCGAGGG GGCGGGGACG 312360
GTCGTCGTCG GCTCACGCAG CTTCGAGGTT GAGGTCGGCG ATTGCGTCGC CATCGGCATG 312420
GGGCACCATC ATGATCTTTC CGAAGTGTGG TCCGACGTGA AGGGCGCATA TTTCGAGACG 312480
ACGCTCGAAG GCAGGAAGCG CTTTGGTCAT CTTTGGGAGC ACACGCATGG GCCTGCCGAT 312540
GTCCGTCCCG AGCGGGTTTG ATCCGTCGCG TGCGCCAAGT CCTGCCTACC TGGAGGTGAA 312600
CATCAATGAC GTATAAGGTC CTGCATATCG GCGCCGGCGG TTTCGGAGAG CGTTGGTGCG 312660
ACACGTTCCT GCCGCAGAAC GTGGCGGACG GGACGATCGA GGTGGTCGGC CTCGTGGACA 312720
TCGATGCCAA AGCGCTCGAT ATCGGCCGCA AGCATCTCGG CTTGAAGGCC GAACAGTGTT 312780
TCACCGCGGC CGCACAAGCC TTCCAGATGG TGGATGCCGA CTTTTGCACC ATCGTCATTC 312840
CGCCAGCTCT GCACGAGGGG ATCGTCGACC TCGCCCTTGC GCGCGGCATG CACATCCTGT 312900
CCGAGAAGCC GATCGCCGAC ACCATGGAGG CCTCCGTCCG TATCGCTGAG AAAGTGAGGA 312960
AGTCCGGCTT AAACATGGGC GTGACGATGA GCCACCGGTT CGATCAGGAC AAGTCGACGC 313020
TGAGGGCGCT CGTCGGTGCC GACGCCATCG GCCGTGTCAA TACGGTATCG TGTCGGTTTG 313080
CCGGAGACTT CCGCCTCTAT GATTCCTGGG GGCGCTTTCG CCACGAAATG ATGCATCCGA 313140
TGCTTATCGA GGGCGCTGTC CATCACTTGG ATATCATGGC CGATCTCGCC GGCGCACCCT 313200
GCACGTCGAT CTATGCCCGC ACGTGGAAGC CGGAATGGGC AGATTACAAA GGCGACACAG 313260
ATGCCATAGT CTTGATGGAC TTTGCGAACG GCGCTCATGG GGTCTATGAA GGGTCTTCGG 313320
CACAAGCCAC GGGTCTTAAC GATTGGGCTT TCGAATATGT TCGGGTGGAG GGGGAAAGCG 313380
GCACGGCTAT CCTCGACCAT CGTGAGATCG AGGTATTTCA CCGTTACCCG ATGCGGCTTC 313440
GACAAGCGAG CCGGCAAGGA AAAGGTCAGC AAGTGTCGCT GCTACCCGGC AGGAAGTGGC 313500
AGAATGCCCT GTTGATCGAG CAATTTTGTC AATGGCTCGA CAGCGGCCCG CCGATGGCAA 313560
CCAACGTTTG GGAAAATCTG CAGTCTGTTG CGCTCGTATT CTCAGCGATC GAAAGCGTCC 313620
GGCTGGGTCA ACCGGTGAAA GTGCAGGAAT TCCTCCAGTC CTACCGAGTG GGGGCTTCGA 313680
TCGAGTAACG ATAAGATGTA GGTTAGCCTC ACAATGGCTG ATCCGATCGT TCATCCTGGG 313740
GTTCATCGTC CCGAATTCTC CGCTCCCCTC CACGCGAGAG CGGAGATTAT CGCGACACTC 313800
AGGGCCGCGC TTGGCAAGCC AAACACGACT CTCGTTGGTG CTGCGATCGG CACGGGAATG 313860
GCCGCCCAGG CAGCCTCGCG GGGCGGCGCA GACTTTATCC TTGCCCTGAA TGCGGGACGT 313920
TTGCGTAGCA TGGGCGCCCC TTCTATCTTT TCCCTGCTCG CATTGCGCAA GAGCAACGAC 313980
TTTGTCCTGG ATTTCGCTCA ATCAGAGATT CTGCCTTTCG TTAAAGTTCC AGTCTTTTTC 314040
GGCGCCAGTG CTTTCGATCC CCGGTGTTCG ATAGAAGCTG AACTGGAAAG AATTGCGGAT 314100
GCTGGTTTTG GCGCAATTGT AAACTTCCCC ACCTCCATTT TTCTCGATGG ACGGTTTCGA 314160
GCCGATATCG AGGGAGCAGG GCTGGGGTTC CAGCGTGAGC TTGAAATGCT TCGGGCCGCT 314220
CAGAAAAGAA ATATGGCGAC TCTCGCCTAT GTTCGAACTG TGGCCGAGGC TCAGCAGGCC 314280
GCGACCGCCG GCGTGGACAT CATAAATCTC AATCTCGGCT GGAACGTCGG AGGGACTGTC 314340
GGAAGCCGCA CAGAGCTCAG CCTGAGGCAA GCGGCTGAGT ATGCGAAGAT CATTTTCCGT 314400
CAGATACGGG CCATCTCGGA GGATACTCTT TGTGTCCTCG AGGGCGGCCC CATCGTCAGC 314460
CCCGACCAAA TGTACGAGGT ATCCGCACTG TCCAAGGCAG ACGGATACAT CGGCGGCTCC 314520
ACCATCGATC GGGTGCCTCT CGAAGCCTCG ATGGAGCAGA TTACATCGGC TTTCAAATCC 314580
GTCGGCACCT TGCAAAAGCG AATTGATGAA CTGGAGCGCC AGCTCGAGCA TGTGCAGCGC 314640
GAATACTCGA TCGTTGGTCG GTCACCTTCG ATCCAGCAAA TCAAGCAACG TATCGAAAAG 314700
CTTGCCGCTT CTAGTCTCCC GGTCATGATT ACCGGACAAG CCGGAACCGG CAAAAAACTT 314760
CTGGCACGGG GTATTCATGA AGCTGCCAGA CGTTCCGGCA GCAAGCTCAT CAGCTCAGAA 314820
GACGCAAGTG GGGAATCGCT TTTTGGCTTC GCCCCGAGCG AGGGCGGCAG GAAGGTGCTT 314880
GGGCTACTCC AATACCATCC CAAAGCAACC CTGCTGATCG AGAGCGTCGA ATGCCTGTGC 314940
GTCGACGCTC AGGAACGTCT GATAGAAGTC ATCGAGACAG GCGCTTATCG GCGCCTGGGC 315000
GACAACGAGA GAGGGCGGTT CGAAGGCCGC CTGATCCTGG CATCGACGCG GCCGCTTCCT 315060
GAGTTGGGCT CGAGCGGGCA GCTGATACCT GCTCTTGAGT CGCGTCTCGC GCCGGGTCAC 315120
GTCTTTCTTC CCCCTCTATG CGATCGCCTC GAAGATCTTC CACTCCTAGC TGAACACTTC 315180
CTACAAGCGC TCCGAAAGGA TCGTCGTAGC CGGAAGCTGT CGGTGGACCA CTCGGCCTAT 315240
CGAGTTCTGA TGACCTATGG CTGGCCAGAA AATATTCGCG AGCTCAGATC CGTGCTCGAA 315300

ACGGCCGCCA TCCGCTGCGA AGGCGACTGG ATCAAGTCCG AGCATCTGCC GCCGCTCGGT 315360
GACGCGAACG CGGACGCGCC GCATCCGCAT CCGGGGGAAG AGCGGGAATG GATCCTGGAT 315420
GCGCTTCAGC GCCATCGGTT TCGCCGCGGT GAAGCCGCCC GATATCTCGG GATATCTCGA 315480
AAGACCCTAT ACAACAAAAT GCGAGTGTAC GGTCTCCCGT TGCAGCCAAG AGAAAGATCC 315540
TAGGCGAGGG CGCCAAATAA CTCCAGGTAG CTTGTTCAGC GACTGCAGCG CCGCCGCCGA 315600
ACGACCGCAG CCGGAGCGCC CAGACCGGTT ATTGGGGTCG CGAGGTAGTG GGGCGCCTAA 315660
GCTCAACACA AGCTCGGACG TGCAGGCCTT CACTAAAGCG AAAAGAAGCC TCTCGCCGAC 315720
TTTCACAGAG GGGGGAGCGA CAGCACTGTT CGGGGGTAAT TGAAGCGGCG GCGGGGAAGT 315780
AGCGAGGTGG CAACACCGTC AGTCGCTGTC CGGCTTTAGC CTCGCAAGAA GTGCTAAGAC 315840
CTCGGCTTGA ATGTCTAGGC AAAGGGCTTC GTATTTGAGT ACCCCTTCCT CCAGGTACGG 315900
AGTCTCTCTC CGGTGACGTT CAATAGCCTC GGCCGCGACG GCGTAGGCTT CAAACAGGCC 315960
ATCCAATTCA ATTCGCGGCT CCTGATGTGC CGCAGCAGAT CTCTGTGACG AGGCAGGGCT 316020
AGCGCCAGCC GAGCTCTGCC GTTGCGAACC AGTGACATTT CACTCGATCC CCAGCACGGC 316080
CCTCGGCAGC TCATCCTTGA TCAGGTACGA GCAAATTATG CGGAGGATCG CATTTTTATG 316140
GTAAGGTAAC GCTAAAGCAG GCTAGGTGTT CCGAGTTTGA TGGTGTGGAA CGGCCCTCCG 316200
AGCCGTGGCT CAACAGCGAT CTCAAGGTGT CAGCGAAAGC ATCTGCTTTT GACTGTCCAA 316260
AGGAAGTGCT GCGCCTCTCA ACTCGCCATC TGACTTTAAA TGGATGGCAG GGGTGGCCGG 316320
TAACGGGCAT CTGGTATAGA CATGGCGTCA TGTCATGCCA GACGCAGCTT CTCAGCGCCT 316380
CGGAGGCCGC CTTAGGAGAG GACTTCCTCT CCGCTCTCTC GACGGATCTG GACAATTGGG 316440
CTCTGTTCCT CGATATCGAT GGTACTTTGC TTGACCTGGC CGAAACGCCG GACGCCGTAG 316500
CCGTTCCGCC TTCGCTGCCT GCAAGTCTCG ATCACTTGTC GAAAAAACTC GGCGGCGCTT 316560
TGGCACTCGT GACCGGTCGC GGCCTTGATT ATGCCGACCA GCTTTTTTCG CCGGCCAATT 316620
TTCCCATTGC GGGCCTTCAT GGTGCCGAGC GCCGCGACCC GGATGGCCGT GTGCACAAAG 316680
CCGCAGAGAC GGCGGATTTT GAGCGACTGA AGGCCGAGCT GGTCGCCGCT ACTGCCAGCT 316740
GGGCGGGCGT TCTGATCGAG GACAAGGGGG CGGCGGTCGC TGCCCATTAT CGGCTTGCGC 316800
CGGATAGACA ACTCGAACTT GAACAGCTGA TGGAGTGGGC GTTGTATCGA GCCGGACCAG 316860
ACTGGGCAAT CCAGCACGGC AAGATGGTCG TCGAAATCCG TCCAGCCAGA GCCAACAAGG 316920
GCGATGCAGT TGCGGCATTT CTCGGCCAGC CGCCCTTTGC CGGCAGACGC GCGATCGCGA 316980
TCGGCGACGA TGTGACCGAC GAAGCGATGT TTCGCACGGT CAACCGGCTC GGCGGCCTTT 317040
CGATCCGCAT CGGACCGCCT GTGCCTGCGA GCGAAGCGCT CGGCTCCATC CCTTCTGCCG 317100
AGGCCCTGCG CGGCATCATC GCCGCATTGG CCCTCCTGAA TATTTGAGCA CCTGACATAT 317160
TTTCTTGCAT GAAAGGAATT CGGCATGAGC CGTCTCGTCA TTGTTTCCAA TCGCGTACCT 317220
GTTCCGGACA AGGGTGGCAT TGCGCCGGCC GGTGGGCTGG CGGTCGCGCT GAAAGTCGCC 317280
CTCGAAGAGC AGGGCGGCGG CATATGGATG GGCTGGTCGG GAAAGTCGAG TGGCGAGGAC 317340
GAGCCGGCGC CGCTTGCGCA ACTGCAGCAG GGCAATATTA CCTATGCACT GACGGATCTG 317400
ACCGATACCG ACGTAGAGGA ATACTACCAC GGCTTCGCCA ACCGCGTTCT CTGGCCGATT 317460
TGCCACTACC GCCTTGATCT CGCCGAATAC GGTCGCAAGG AAATGGCCGG GTATTTCCGC 317520
GTCAACCGCT TCTTCGCCCA TCGCCTGGCG CCGCTTGTCA AACCCGATGA CGTCATTTGG 317580
GTGCACGACT ACCCCTTGAT TCCTCTCGCC GCGGAACTGC GTCAGATGGG CCTGGAGAAC 317640
CGCATCGGCT TCTTCCTCCA CATTCCCTGG CCGCCTGCAG ACGTACTCTT CACGATGCCC 317700
GTTCACGAGG AGATCATGCG CGGCCTGTCG CACTACGACG TCGTCGGCTT TCAGACCGAT 317760
CATGACCTTG AGAACTTCGC CAGCTGCCTC AGGCGGGAAG GCATCGGCGA CGCACTTGGC 317820
GGAGGCCGCT TGAGTGCCTA TGGCCGCATA TTCAAAGGCG GCGTCTATGC AATCGGCATC 317880
GAGACTGCGG CCTTCGCCGA ATTCGCCAAA AAGGCATCGA CCAACAGCAC GGTCAAAAAG 317940
GCGCGTGAAA GCATCGAGCG CCGCAGCCTC ATCATCGGTG TCGATCGCCT CGATTATTCC 318000
AAGGGACTGA CGCAGCGCAT CGAAGCGTTT GAGCGCTTCA TCCTGGCCAA TCCGGCACAG 318060
CGGGGGCGTG TCACCTATCT GCAAATCACG CCAAAGTCGC GCTCCGAAGT GCCGGAATAT 318120
GAAGCCATGC AACGCACTGT TGCCGAACAG GCCGGCAGGG TGAACGGCGC GCTCGGCGCC 318180
GTCGATTGGG TGCCTATGCG CTACATCAAC CGCTCGGTGG GCCGCCGCGT TCTTGCAGGG 318240
CTTTACCGGC TTGGCAAAGT CGGCCTCGTG ACGCCGCTTC GAGACGGCAA GAACCTCGTC 318300
GCAAAGGAAT ATGTTGCCGC GCAGGATCCG GACGATCCGG GCGTGCTTGT TCTTTCGCGC 318360
TTCGCGGGCG CTGCCCGCGA GCTACAGGGA GCACTTCTTG TCAATCCCTA CGACATAGAG 318420
GGCACCGCCA ACGCCATGGC GCGCTCGCTC AGCATGCCGC TGGAAGAGCG GCAGGAACGC 318480
TGGACGACGA TGATGGATCA ATTGCTGGAA CACGACGTTT CGCGCTGGTG CCGGGATTTT 318540
CTCAATGATC TGACGGCATC ATCAGATCGA TGTGGTTAGG GCTCCTTCAA TCAAAGATCA 318600
TCATCGTGCC ATTAATGAAG TCCGGCGACC GGCTGCGGCG TTATCGCCAT AGCCGCCTGG 318660
CCGCATTTGG ATCGTCGGCA CGGGATTGAG ATTCCACCGG ACCCGGTGTC CAAACCCGAA 318720
ATCGGCTCTC GCTGCCTCCG AACCGCACCC GAATACACGG AATTACCGTG CGCACCTCGC 318780
ACGCGATGCG ACTGTTGTTT CGAGGGTATC AACCGAATTT CAGACTGACC GTCTTGCGAG 318840
CGCCAGGATC TAGGGTCAGG ACCTATTAAT TTCGTTTGGG ATGTGATTCA GAGTCTCCAT 318900
TGAAGGAGGT CCTGATGGGT GATTTGTTTC TGCTAAGCGA GCGCCAAATG GCTCGCATCT 318960
CTCCGCATTT TCCGCTTTCT CATGGGGTTG CGCGTGTCGA TGATCAGCGC GTCGTCAGTG 319020
GTATCGTCTA TGTGATCCGC AATGGTCTGC AGTGGAAGGA TGCACCTTCG CAGTATGGGC 319080
CACATAAGAC GCTCTACAAC CGCTTCATTC GCTGGAGCCC GGCTCGGTGT GTTTGACAGG 319140
ATTTTCGCCG CTTTCGCCGG CGAAGGCCCC CAACCTGAGC GCATCATGAT CGACGCAACA 319200
CATCTGAAGG CCCACCGCAC TGCGGCAAGC CTTCTAAAAA AGGGGATGTT CCCCGTCGTA 319260
TCGGGCGCAC CAAGGGCGGA CTGAACTCGA AGCTCCATGC CGTCTGCGAC GAGGACGGCC 319320
GGCCGATCAT CATGTTGCTC TCTGAAGGCC AGATGAGCGA CCACAAGGGC GCCCGGATCG 319380
TGCTCAACGC GTTGCCTAAA GCCGATTGCC TCATCGCCGA TAAGGGCTAC GACAGCACCT 319440

GGTTCCGAGA AGAGCTCCTC GCAAGGGGCA TCGAGCCCTG CATCCCGTCC TCTAAAAGCC 319500
GGAAGAAACC GTACGTCTAC GACAAAGACC TCTATCGTCG CAGACACAAG GTCGAGAACC 319560
TTTTCGCCAA GCTCAAGGAT TGGAGGCGCA TCGCAACACG CTATGACCGA TGTGCCCACA 319620
CCTTCTTCTC TGCAATCTGC ATCGCAGCTT CTGTCATCTT CTGGCTGTGA TTAACGAGTC 319680
CTGACCCTAG CTCAGGTGCC GTCCGTTTCT TTCCCAGACC CAAAGCGCGC GCAAGCTCGG 319740
AACGCTTTTG AGCATAGCTT GAAGCAACCA TGGGGTAATC AGCCGGGAGA CCCCATTTCT 319800
CTCGATATTG CTGCGGCGTG AGGCCGTACT TAGCCATCAG GTGTCGCTTC AGCGATTTGA 319860
ATTTTTTACC GTCCTCCAGG CATATGATGA AGTCTGCGGT CACTGACTTC TTGATCGGGA 319920
CGGCGGGCCG CTGTTCTTCG ACGGCAGCCT CCTCAGCCTT GTCTGCCTGA GAGGTGCTGC 319980
ATAGCGAGAG ATACGTCTGC TGAATAAGAC TGGCCAGATC GGCGGCAGGA ACGATATTGC 320040
GGCTCAGGTA GGCCGAGACA ACCCGGCTTG TCAGCTCAAG ATTCCGTTGC CCGCTGGCTA 320100
ACCGCGGTTG GTTCAATGTT CTTCTCCTGC ATCTTCCGTC ACTTAGATCC GTCCGCGCAA 320160
TTTGCAGATA TTTCGGTCAG AAGGAAGGCC GACAGGGAGC CGGTTGATTT TTGCCGAGAG 320220
TTTTTCTTTT TGACGCGTTG GTTTCACCGA GATCATTCTC GTCGCGTAAG CAGGCCGCTG 320280
CTCGGAACTT GAACCACGGC CGAGCAAACG GCCATCCGGT CGAAAAACCC GCGTGACTTT 320340
AGATACGCAA AGTTCGAGGC GACGCTCGCT GGTCGCATTC GGCTTTTGGC TGCTCTCGAT 320400
GATCACGGGC ACCCTCACAG TCACCGAGTG CCTATCCGCC TCCCAGAACC ATCTGTGCGG 320460
ATTCCGAGGC AAGCCGCCCC CGCATTCCGA AATCATTCCG CCCCCCAATT CCGAGAATTA 320520
GTCGCCCCCT GATTCCGAGA TGATGCCGCC CCCTGAAAGG GGTCGTTTGT CGGGGTGTCC 320580
TGCTGGTGAT ACCAACGAGC GGTGATCATG ACCGATGCGC CCATTCGCGG AGTCCGATGG 320640
ACATGATCTT CCACGGCTGG TCGACGAGCT TGTTCCATGC GGCGCAGCAA TGGGTGACGA 320700
TGTCGTCGTA GTCCTTGAAG ATACGGTTGG AGAGCCAGTT GTCGCGCATG AACTGCCAGA 320760
CGTTTTCGAC TGGGTTGAGT TCCGGTGAGC GCGGCGGCAG GAACATCAAG GTGATGTTGT 320820
CCGGCACCTT CAGTTTCGGC GTCACGTGCC ATCCGGCCTG ATCGAGGATG AGCACCGCAT 320880
GGGCGCCGTC ATCGACGCTC CGGCCGATCT CGGCGAGGTG CTCCTGCATG GCCTCGGTAT 320940
CGCAATAGGG CAGGACCAGG CCCGCGCCCT TTCCCTTCCG CGGCAGACGG GCCCCGAAGA 321000
TGTAGGCCCA CATATGTGGA CGGCTCCCGC TTGCAAGTGT TTTCTGCAGA TATTTTTGAC 321060
CGGATCGCTT GCTTCCATAT GTGCGGCCTT TTGGTGTGGT CGCACATGAC CACTGGCCAA 321120
GATGATTTCC GCAACGCGTA TTCCTAACAT GGTCTCGACC TTTAGCGGTC AGTGGGCCTA 321180
ACGGAGTTTC ACGCGTCTTG GATCGTCCGA TCGCATCATC TGCTCTTCGC TTGCAAGTTC 321240
ACAGCATCAG CTCACACGGT AGCATTTTGT CTTTGCTCAC CGTGGTGGCT GAACTTTTAT 321300
GATGTCTGCG CTTGGCCTTC TGCCTGGCGA TATGACTCGC CGCTCGTCAT AAGCTTCCAC 321360
GCAATCCGCG CCAGCTTGTT GGCGAGCGCT ACGGCAACCA ACTTTGGCCT TTTCCGCTGC 321420
AGCAGCCCCA TAAGCCATGC AGAGGCGTGC CTGCTGCGGC CTGTTCTGAC TTGCTGTAGA 321480
AGAGATGTCG CGCCAACCAC CAAGATGCTC CGCAACATTT CGTCGCCAGC CCGGGTAATG 321540
ACACCAAGCC TGACCTTTCC GGCAGTTGAA TGGTCCTTCG GCGTCAGACC GATCCATGCT 321600
GCAAAGTCGC GGCCCGATTT AAACATTCGA GGATCCGGTG TCTTCATCAA GAGAAGGGAG 321660
GCGCCTATCG GGCCGACACC CGGGATCTCG GCCAGGCGTT TGCTGCATTC ATCAGCACGA 321720
TGCAACTTCA TCAACTTTTC ATCGAGCGTT TTGATCTGGC AAGCTAGCTC GCGGTATTCC 321780
TGTCCATGCA GCGCGAAGAG ATCCTGCGCC AGTTCCGGTA GGGACGGGTC AGCGGCAATG 321840
CGCTCCAATA GCGCTTCGAT GCGGCACATG CCTTTGGCCG CAACGATGCC AAATTCCATG 321900
GCAAAACCGC GAATGGCATT GGCGAGCTGT GTCCGATTGC GGATGAGTCT CTCTCGCATA 321960
CCGACCAGCA TTAACGCCGC CTGCTGATCA GCCGTTTTCA TTGGCACGAA GCGCATGGTC 322020
GGGCGGCTCA TTGCCTCACA CAATGCTTCC GCATCCGCGG CATCGTTCTT GCCCCGTTTC 322080
ACATACGGCT TGGCCAGTTG CGGTGCTATC AACTTTACTT CATGGCCGAA CGAACTTAAC 322140
AGCCGTGCCC AGTGATGTGA CCCACCGCAC GCTTCAAGTG CGATAATGGT CGGCGGTGTT 322200
TTCTCGAAGA ACTTCACCAT TTCCCGGCGC CACAGCTTCT TACGAAGGAT TGGCTGCTCA 322260
ACCGCATTCA CACCATGCAA TTGGAAGACG CTCTTTGACG TATCCATACC AATTCGGATA 322320
ATCTGTTCCA CGGACGGTTT CCCTCGATTG AGAACTTCAA CGAACTCATT CTGGCACATT 322380
CGATGCCGTT GGGAGCCGTC CACCTCAACA TGGTGCGCTG ATCCAAGGGC GCGGATGGTC 322440
TGGTGCCACG ACGCGCCCAT CGGCGCGTGA TCTTGTTCTT CTGGCCTATG CGCGCTTCGT 322500
CGGCCCACCA GAGTTCGATG TCCGTGCCTT GCGGGAGCCG GCTTCGGATT TCCGCCAGAG 322560
CGGCGGGGAA GTCTTTTTAA AAGCGTCTAC TTCCAGCTCG TTCTGAGCGT AGTGGCGTGG 322620
GCGAGCCGAC AGCTTGGCAA AGCCGAGCGC CTTCAACTCG CGGCCGACCG TCGTCTCGTC 322680
CATCGAGATG CGGAATTCCT GGAATATCCA CTGAACCAGG TCCTTGCGCC GCCAACGGAC 322740
AACACCGTGG ATCGCCGGGA TCGGGCCGCT CTCGACGACC TTTGCAAGAG CCTGGCGCTG 322800
AGCGGCATTC AACTTCGCTC GGCCGCCAGG CGCTTTGCCA TTCACCAGTC CATCGGGGCC 322860
GCGCGCGTTG AAACGCAGGA CCCAATCCCG CACAATCTGC AGCGTCACGC TGCCGATCCG 322920
TGCCGCATCC GAGCGCGGGC CGCCGTCATA GATCGCGGCA AGAGCCAGCA ACCGCCGGGC 322980
CTGATTGGCA TCCTTCGTCT GCCGTGCGAG CTGTCGAAGA GCAGCTCCGT CGAAATCATC 323040
ACGCAACAAA ACCGCCGAAC CCATCGCAAA CCTCCTGTTT GCAGCATGGA TTCAGATTCG 323100
CGACACCTTG GGAATCCCAT GAGAGTCAGA AACGGCGCTC GTTGGTATGA GACGTTCCTC 323160
CTTTCAGTGT CAGAGGGGAA GGAACGGGAT GCCAGCGGAG AGACTAAAGA TGCGGCGTGT 323220
CCGCGAGGTT CTGAGATACA GATTTGAGGA AGGCCTTGGC CACAAGTCGA TTGCGGTGCG 323280
CGTTGGAGCG GCCCCCTCGA CCGTGCGCGA GACGTTGCGC CGTTTGGAGC GTGCCGGCCT 323340
TTCCTGGCCG TTGGGCGACG ATGTCAGCGA TGCGGTGTTG GAAGCGGCTC TCTATAAAGC 323400
TGCCGGCACG AAGACCGGTC ATCGTCGCAG CGTTGAACCG GATTGAGCGC ATGTTCATCG 323460
CGAGCTGAAA CGCAAGCATG TGACGCTGCA GATCCTCTGG GACGAGTATA TCGGCCTTCA 323520
TCCCGATGGA TATCGCTACA GCCGCTACTG CGATTTGTAT CGCGCCTGGG CACTGAAGCT 323580

GCCGGTGACG ATGCGCCAGG ACCATGCGGC TGGCGAAAAG CTGTTCGTCG ACTATGCCGG 323640
CGACACGGTC ACAGTGGTCA TCGACAGATT GTCGGGAAAG ACGCGGCAAG CGCACCTGTT 323700
CGTGGCAGTG CTCGGGGCAT CGAGGCTGTC ATTCGCCCAT GCTCGCTGGA GTGAAACACT 323760
TCCCGACTGG ATTGAATGCC ACCTGCTGGC GCTTGAGGCC TTTGGTGGTG CGCCGGCGCT 323820
GCTGGTTCCC GATAACGCCA AGGTCGCCGT CATCAAGGCC TGCCATTTTG ATCCGCAGGT 323880
CAATCGCACC TATGCCGGCA TGGCAGCGCA TTACGGAAGC GCTGTTCTTC CGACGCGACC 323940
GCGCCGGCCG CGCGACAAGG AGCGGACATC ACGATGCACC TTGTTGGAGT GGTTGAGCGA 324000
CAATTAATCT GGGTTGACGG CCACCATATC GACTGCCGGC GCCTTTTTCG AGTCTGACGT 324060
TGCCCGGCGG TTCGTACAAG TATCGATGGT TGCTGGCGTA TCGATGACGC TTGAGAGTGC 324120
CTTTGCGCCC GAGTTGGTGA ACGGTCGATT CGCATACGCC GAGCTGCTGG GCTACTTCCT 324180
CGCCGGTGAG CATGCCGCCC TCGCGCAGCC GTTCGTACCG GCTCTTCAAA CCATAGGTGC 324240
GGCGCACGAG CATGACCTTC TTCGGCGTGA AGGGTTCGCC GCGCCAGTTG CGATGCCCGA 324300
GTTCGTTGAG ACGAGCGGCG ATCTGCCGGT CGTTGGTGGC CTCCAGCAGC TCGTTGATCA 324360
ACGCCACGAC CTGTGCCGGC GTCTTGCGTA TCACCGACAT AGGCCGAGGT CTGGCCACCG 324420
ACAGGCTCTG CGTGCGCCCG CCGCGCCAAC GGATGTTGAT GTTGACCTCA TCGTCGACGA 324480
GCAGCGTCAC GTCTTCGATG AGAAGGCCGA GCATGCGCTT GCGTTCGACG GCACCGGTGC 324540
GCTCGTCGTT CCAGATGCGC GGGAAATCGG CGGTCAGCGC CCTGATGCGC TGCTGCGCTG 324600
GCTCGTCCAG CAGCGAGTGG TCGGCCTCGT TCTGGCGCTC GTGCTCGCGC TGGAGTGCGT 324660
CGAGGTCGCG CAGCCGTGCG TTCCAGTCGG CTTCCAGCGC GTCGGCGACC AGGCGGTTGT 324720
CCGGATCGAC CTTCAGGTAG CGCCGGCGGG CCAGTTCAGC CTCATAGCGG GCGCGTTGCA 324780
GCTGCGTGCC GCGCAGCGCC GCGGCCTGCT CCACGCGCTG GGTGATCTCC TGCTGCACGG 324840
CAAGCGCGAC ATCGATCGCT GCCGGCGCCA TTACCTCCAG CAGTAGCGCG CTCACCGCCT 324900
CGTCGACCGG AGCACCGCGC ACCCATTGGC AGTGTTTGCC GGCGTGTCGC ACGACGGCTT 324960
CGTTGCAGAC GTAATATGGG CGCAGCCGCC CCTCGAACGG CTCGTAATGG ACCCGCATGC 325020
GTGCACCGCA GCGGCCGCAC AGCAGCCGTC CCTGCAACAG GCCGCTGCCC TGGCGGGGCA 325080
TGCGTCCACG CAGGCCGGGT GAAAAACCGG TGGCGTTCTG TTCCAGGGCT GCCTGATTGC 325140
GTTCGTATTC GGCCCAGGAA ATGTAGCCGT CGTGGGCGTC GGGAATCAGT ACCTGCCAGT 325200
CGGATCTCGC CACGCGCAGT TGCACGGGCT TCAGCTTGGC ATTGTAGGCG GTGCGCGTGC 325260
GTCCATAGGC GAAGGCGCCG GCATAGCGCG GGTTGTGCAA GATCTGGAGC ACGCGGGAAT 325320
GGTCGATCTC GTTCCACAGG ACGTCGCCCT TGCCGATGCC GCGACGGATA CGCCGCGGAA 325380
AGAGGATCTT CTCGCCCCGC AGGCGGCGCA CGACGGCGCA GGCCGAGCCG GTCTGGCGGA 325440
AAGTATCGAA CAGGAGCCGC ACCGTGTCCT GGATCTGTCG GTCGGGATCG AGCACGACCC 325500
GCGCGTCAGG GGTGTAGACC AACCCGATCG GCAGCGGCAT CTCGAGTTCG CCGCGGCGCG 325560
CCTTGTTGAG AATGCCGCCT TGCAGCCGCG ACTTCAGGAT GTGCAGTTCG GCCTCGCTCA 325620
TCGTGCCCTT CAGGCCGAGC AGCATGCGGT CGTTGAAATA GGCCGGATCA TAGACGCCGT 325680
CCTCGTCCAT GATGAGCGTG CGCGACAAGG CGGCCAGCTC AAGGAGACGG TGCCAGTCGG 325740
CATTGTTGCG TGCCAGGCGC GACACTTCCA GCCCCAGCAC GATCCCTGCA TGTCCCATCG 325800
CCACTTCGCT CACCAGGTGC TGGAAGCCGT CGCGGTCCTG CGACTGCGCG CCGGAGAGGC 325860
CGAGGTCGCT ATCGATGACG TGGACACGTT CGATCGGCCA GCCCAGCGCC ACGGCGCGAT 325920
CGCGCAGGGC GTATTGCCGC TTGGTGCTCT CGGTGTTCTC GAACACCTGA CGAAGCGAGG 325980
ACTGACGCAC GTAGAGGAAT GCATCACGCC GCAGATGGTC GGCGTCAACC TTTATGGCGA 326040
TGTCAGTAGA CATGGTTCCC TCGGGTCTCT GCTGCCAGCA CCATGTTGGC GAGCATGTGC 326100
ACGAGCTGGC GATCGTGCAC TGCGACCGAA GCCGTAGGCC GTGATTCCAG CCGCCGATGC 326160
GTCGGCGGGG GAGGCTGCGC GATCAGCACC GCCAGCCCTT GCCAAAGGCC GTGGAAAACG 326220
ATGGCGCCCA TGTCCTTGGC GCAGGCCTGA CCGCAAAGGA CGGCGGTGCG CAGCGTCTCG 326280
TAGAAATCCT TGCAGCTGCG CGGTAGCACC GGGGTGCGTG CATGGGGCTC ACCGTTTTTT 326340
TTTACGGGCG ATCGCGCGTT CGATGCTGCG GGGATGGACA CTGATGCCGA GCGTCGAATG 326400
CAGCAGTTCT GCCAGGGAGC GCGCCTGCAG CGGGGCGCCC GCATGCTGGT TCTGCTCGAT 326460
GAGCTGCATG ACCTCGTCGG TGAGTTTGTG GGCCGACTTT GGCCCTCGGG TGCGCGGCAG 326520
CAGGCCGGCA ATGCCGTCGC GTTCGAACGC GGCCTCGGCC TGATAGTAGG TTGGCCGCGA 326580
CAGACCGAAA AGCGCGGCGG CATCGGCCTT GTTGACGCCA TCCTCCTGGA CGTGACGCAG 326640
CATCTCGTAC TTCACCTGCA CGAGATCGAG AGGATCGAAG AAGGTGGACT CACGAAACCA 326700
GGGTGCACGG ACGGCTTCGG GCCGCGCATT GAGTGCTCCG AGCTCGCGCA GTCGCTCACG 326760
TTTGGTCTCG TTGGGCATGG CATAACCTCT TCCTAGTGGT GTAAATCAAA TTACGCCTCG 326820
ATAGAGAACG TGTCAACCTA ACGACGCCGG GCGATATGGC TGTTTATGCT GCATATAACT 326880
ACAATGTAGA GGCAATTATC GCTGTGAGCG CCCGTATGAT TCGGCATAAT TAGATTTACA 326940
TAATCGGCAT AAATTGCCTG ACACGCATTC AGCCGCCATG GCTGCGCTCG ATTTGCTCGA 327000
TCAATGCGGC TAGGGTTGCC AGATCATCTG GACGCACGAA CGACCTACCG GCCGCGATCT 327060
GAATGAAAAC ATCGGGTGCA GCGACGTCCG TCCATGACGC AAGCAGCGAG CGAAGCCGCC 327120
CATCCGCGTC GTAGAACATG ACGCGATCCT CGCCCCAGTT CTGCTTGCGT GTCGACAACA 327180
CGAAGCGCTG GCCGCGCCAC GGATGGAACG GGTGCGTGAC CTCGAACTCT ATACGCACTT 327240
GAGTGTCAGT ACGAACTGCA GTCCTGGACT TGAAAGAAGG CAAAAGTCGA AGCCGCGGTT 327300
CGCATTGTCG AGCGCTGGCT ACTTGGCCGA TTGCGCCACC GCGTCTTTTA CAGCCTTGCC 327360
GACGTCAATG CGGCGATTGG CGAATTGCTC GTCGATCTCA ACGACACCCG TGTTCTGCGC 327420
CGTGTCGGGC GCACGCGACG CCAGTTGTTT GAAGAGATCG ACCATCCTGC TCTCCGGCCG 327480
CTGCCCTCCG AACGCTACGT CTTTGCCGAA TGGCGCATAC GCCGGGCCGG CCTCGATTAT 327540
CATGTCGATA TCGACAAACA TTACTACTCC GTGCCCTACC GATTTGCCCG CGAGCAGGTC 327600
GAGGCGCGCA TCACCGCCAA TACCATCGAG ATCTTCCACA AAGGTGAGCG CATCGCTGCC 327660
CACCGCCGCT CGAGTGGCAA TGGGAAGCAC ACGACAACAC CCGAACACAT GCCATCATCG 327720

CATCGCCGCT TTGCCGACTG GACGATAGAG CGCATTAGCC GCGAGGCATC GGCAATCGGA 327780
TCGGATGTTG CCTTGCTCTG CGAGCGTATT CTCGCTGATC GGCCGCATCC GGAACAGGGC 327840
TTCCGGGCCT GCCTGGGCAT TATCCGCCTC GTCAAAGCCT TCGAGCGCGA GAGGGTCAAT 327900
GCGGCCTGCG GCCGGGCATT GGAAATCGGC GCGCGAACCT ATGGATCGGT GCGCTCCATT 327960
CTCGACAACA ATCTCGATCG GTCGCCAGCG TCAGCCGGCA CAGCCTCGCC TGAACCCATC 328020
CACCACTCCA ACATCCGCGG TCCACGCTAT TACCACTGAG GAGAAGCCAT ATGCTTGCAC 328080
ATCCCACTCT TGATAAATTG AACACCATGG GCCTGACCGG CATGGCCAAG GCGTTCAGTG 328140
AACTGATCAG CAACGGCGAA AGCGAGCAAC TCTCCCATGC CGAATGGCTC GGGCTGCTGC 328200
TCGAGCGAGA ATGGAGCTGG CGCTACGATC GCAAGCTTGC CGCACGTCTC AGGTTCGCCA 328260
AGCTGCGCCA CCAGGCCGTG CCGGAGGATG TCGATTATAG AAGTGAACGC GGTCTCGATC 328320
GCGCCCTGTT CATGAAGCTG ATCGGCGGCG ACTGGATCGA TGCCCACGAC AATCTTGCCA 328380
TTTGCGGGCC GTCCGGCGTC GGCAAAAGTT GGTTGGCCTG CGCACTCGGG CACAAAGCGT 328440
GTCGTGATGA TCGCTCGGTT CTCTACCAGC GCGTTCCGCG GCTGTTTGCC AACCTTGCTC 328500
TGGCTCGCGG CGATGGCCGT TATGCAAGGC TGCAGCGAAC CCTCGGGCAC GTGCAGCTCC 328560
TGATCCTCGA TGACTGGGGC CTGGAGCCGC TCAACGAGCA GGCGCGCCAC GATCTTCTGG 328620
AGATCCTCGA AGATCGTTAC GGACGCCGCT CGACGATCAT TACCAGCCAG CTTCCGGTAT 328680
CAGCCTGGCA CGAGATCATC GGCAATCCAA CCTATGCCGA TGCCATCCTC GACCGCCTCG 328740
TTCACAATGC CCACCGCATC GACCTATCCG GCGAAAGCTT ACGGCGAAAC CAGCGCCGGA 328800
AATCTTGACT CGCGACCACG ATCAACTGAC AACAATGACA GCCAGCAGGA CCCCAGCCTC 328860
AAGGGGGCGA GATCATCCCG GAATCCGGGG GCGCAATCAT CTCGGAACAA AGGGGCGGCT 328920
TCATCGGAAT CGGCACCATC TGATAGAAAA GACGCCGAGG CGGCGTGAAA CGCGAGGCAA 328980
GAGGTAACCC CGAGATGGCA TAGGCTGTCT TCGTCAAATA TTCGATTGCA ACTTCCCCGG 329040
TTGCCTCCTA ATTAAGCCGC CAGACGCCTG TGCCATCGGC CGTTGGATTA TATTGTTCTT 329100
TTCCAGATTT AGCGGGTTAC AGCTTTTGTC CACAGACCAG AACCATTTGA TTTTCTTCAT 329160
GGATGATTTC GGAACACGTA CGGCTTGCAA GGCGACCGAA ACTTTGCCCA TGTGGTCGCA 329220
TGTATTCAGC TTCGGCTTGG GTGGAATCAT CGTTCCATCG GAGGCCGTTC ACGAAATCTC 329280
CAGCGCGACC AATGACCTTT GCGCAAGATG GGATGTGCCC GTGCTGCACG GAAACAAGAT 329340
CAGAGGGGCG CGAGGGAGCT TTGGTTTCCT GAAGAAAGAC GAGAACAAGA AAGCGCGGTT 329400
CTTTCAAGAG CTGGAAGAAA TCCTGATCGA CGACCGGATT ACCGCCCATG CTTGTGTCAT 329460
CTGTCGGCCA GGCTACAGAG ACCGCTACCA TGACAAGCGC CCTGAGGGTG TCCGTTGGGA 329520
GATGAGCCGG ACCGCATTCG ATATATCCGT CGAAAGGGCT GCCAAATATG CCCGTTCTCT 329580
CAAGCGTAAG CTTTCGGTGG TCTATGAACG AACAGGAGAA ACCGAGGATC GATTGATTGA 329640
AGGTTACTTC CAGAGATTGC GGACAACCGG GACAGAGTTC AGCGTTGAAA ACTCCGCTCA 329700
ACACAGTCCC ATGTCATCTG CCGATTTGGC TGACACATTG ATGTCGATCT GGCCAGACGG 329760
CAAGGGAAAC CCGATGCTGC AATTGGCAGA CTTGGTGGTC CATCCATTGG GGCATCGCCC 329820
AACGGGTCTT AGGAATAGAG CATATGACCG CTTCGCAGAG TCAGGCCAAC TTCTAGACTC 329880
TCGCACCGAT GATCCGACGA TCTCGATTAA GTATTCGTGC TACGACGATC CGTACAAGGA 329940
ATACGTGGCT CCTGAAGGAA ACCCCAGAAA CACATAAGGG ACCCGAAGGT CCCTTATGGC 330000
GGTTAGCGTA AAAGCAATAA CCCCGTGGCA AAGCCATACC ATAAGGTAGA TAAGTCCTGT 330060
GCCATTTGCA AGGCGTTTGT GAATTTTAGC CAAAAATCGC TATCTCCTTG AATTGTGGTT 330120
TTAAAGACTC TTGAACGGCT TCACAGGCCG ACGGTAAGAA GCGCGTGTTG TCGATGGCTT 330180
TCGCCCTGAG GCGTTCTCCA CGCGCGGTGC GCCTCAAGAC AGTCCCTGCC GTTCGAAAAA 330240
ATGAAAGCGG GCGGCCGTTC GATATCCGGC TATCGTTCTA CCGTCCGCAG GCACTTTGGT 330300
TTCCAACCAG TGCCTTTCGC GCCCGACAAC GTCGCCGGAG GGCATACTGA TCAGGGCATT 330360
AGGCGCAGGT AGTATGGTGA TTCGCTCGGT GGGCCGAAGT GGCCAAAAAC CTGCGCGATT 330420
GTTGCCTGCG TATGCTCGTG AAACAGGTTC AGCGACGCCG AGAGCAGCGT CGAGGATGGC 330480
GCAGCTCAGG GCTACCAGCT TCGACCACCG TAAGGCAAGC TAATATTAGG TGGATTGCTG 330540
CGCGGTCGCA TTGAACCAGG GTTAGACAAT TGAATGCAAT CGGTTCCCGG TATTGGCTAA 330600
GGTAGATTCT GATTGGCTTC GCCAGGTTGA CGCCCGTTGC GCGGTCGCCA GCCGCATCAG 330660
CGCAAACTGA CGCAAGGGCA CTGGGCAAAT CCCCATCTGA CCTCGGTATA GTCGATGTTG 330720
ACGTTGACGT ATTGCGTCGA GGGGCAAGAG GATTTCAGCT CAAGGAAGTG CTCTTTGAAC 330780
TCGATCTGCC GCAGAACGAT TGCTCCGTAT TCGTAATATC CGCCTGCCCC ATTTTCGAGA 330840
AGGTAACTTT GGTAGGTGCA TGTCAGTGCC TTGTATTGCG GCGGCTTGCC ATTGCCGCTG 330900
AAGGTGAGGT TGCCCATCTC GGTGAGGAAG ATCTGGAAAT CCATCAGCAG CGATGCGCCA 330960
TCTGTGATGA TTGCGTTGGC AAGCAAGGAG AGAAAGGTGG CTGCGACAGA GGTGCCGGCA 331020
AACCTGTTGC AAAACTCTTC TGCTTGTGTC TGCCGATCGA AACGTCGGGG ATGTTGGCAA 331080
CAACGTCTGT CCAGGTGTTG ATGTCCAGTT TTTGTGAATC GATGATGGAA TAATGGGTGT 331140
GCGCGTAGTC GTGAATGAGT CCTCCTATCG CCAGCAGGTT GTCCTGGTTC TCATTCATAA 331200
TGCCGTTATA GATCGGATCG TTCGTTTTCT GGCCGATGAG GTGAAACTGT ACTGCCCCTT 331260
GATCGTTCGC GATCGTGTCG TCACTGGGGG GCGCAGCATT TCTCTGGGCC AAAACCAGGG 331320
GCTTGCGGCA GAAAAGCGCT CTCGCCGCCT CGTAGTTCTT GTCTGAATAG CCCTGTTTAT 331380
GCTCGTGAGT ATCCACTGCA ATTGCGTTCG ACATGCTTTT CCTCGTGGGG AGATCTGAGC 331440
GTTACTGTAA GATCCGTGAG GTGCCCTGAA CGCGCAAAGC CTGAGTGCGA GAACATTTTG 331500
GGCGTCTGTT GATGTATACC AGGTCATTCC GGTCTCCACC TTTGCGTCAG GCAGGCTTTT 331560
CTCTGCCCGC GACCATTTTT GCGATAGCGT AGCGGCGAGA GGCCATGAAA CGCCCTGAAA 331620
GCTGTCGAGA AGTTGTTGCT GTTGTCGTAC CCTAGTGACT CTGCGACGGC GGCGACGCTT 331680
TCGTGGCCGC TTCGCAGGCG CGCGGCAGCC ATGGTCATCC GGTAGTGCCG TAACCAAGTC 331740
ATGGGGCGCA GTTAAAGGAT GCAGCAAAGC GCTTATTGGT GCCGATGATG CGAGCCAATT 331800
TGCTGAGGGT GATCTTCTGG TCGACCTGCT GTGCAAGCAG GTCGCATGAG TCCTGCACCA 331860

TGGGATCAGC TGAGAAGAAT GGCGGAGCAG AACGCCGAGC GATCTCGGCG GCGCAGGCGG 331920
TAAGTCTGCT CAGAACTTCC TGCTCGATAG GCGGTCACAG CAGTTAGTCA AAGTACCCGT 331980
TGGAGTAAAC TGCCCTTCGA TACAGGCTGC CTTCCGCAAC CAAGGCTAGT TGCACGAGTT 332040
CGGATGAGAG CTCCGTACGA TCGTGATTGT TTTGCGGGGC GCGACAATCC ACGATGACGA 332100
GTTCCGCCGA GCGTCCTGGC CGCAACAAAC GGTAATCAAC GATATCGACC TTCGGCAGAG 332160
AGCTCGCGAT TTGGGCCAAC GACGTACATG TGACCAGGTC GGACCCAAGG TACGCTCCCA 332220
GAATGGATGC ATGCACCTTT GGCCCACCCT GAGGATATGG CCTACCAATT GGCTTGGTGC 332280
GGCGCACGGC AGTTGACGAG GGGAGACTTG GATTGTGTAG ATGGCCGAAC GTGTGAATAC 332340
GCGCTCGTTT GGCAGTTTGC CGAAAACCCT CGATTCAAAT TCCGGCTCGC GATAGGCTCG 332400
CATCGAGCTC GTTGTATTGG CGCAGCGGCG TCTTCGCCGG CCGGCGCATG AGGAGAGGGT 332460
AACCAACCGT CGCCGATATG GACCGAAGGC GGCCAGATTT CGGACTCATT GCCTGGACGC 332520
AGCATCGGTC GACTGCCCTT GCTCTGTTGC CGTTGCCGCG GCGGCGATCT CCTGGCGCAA 332580
CCACCGATGG TCCGGGTCGC GATGGCAACG CTCGTGCCAG ATCAGCGCGA CCTCGTAGGG 332640
CGGCAGTTCC ACAGGCGGGT CGACAATCAC TAGCGGCAGC AAGGCGGCGT TCCTGGTCGC 332700
GACGCGGCGT GGTACGACCA GCACCAGATC GGTCGACACG ATCGCCAATC CGGCCGTCAG 332760
GACATTGGAA ACCAGCACCG GATCGGGATC CTGGAGATCC AACTTGACCA GATCATCATG 332820
CACCCGGCCG AAACCGTCTT TGGTATCCGA GGCAATGGCG GCGTGGCGCA GGGCTGAGAA 332880
GGTGTCGATT ATCGATTCCT GCTCCAGGGC TGGGTGGCCG TTGCGTAGCA GGCAAGCAAA 332940
GCGGTCGTTG TAGAGCCTGC GCCGAAAGTA GCCCGGTGGA GCCTCACCGA TCTGGCCAAC 333000
GGCTAAATCA ATTTCGCCTT GCTCAAGGCG TCGGAGCGCT AGGAGCGAAT CGGTCACTAT 333060
GTCCAGGTTA GGCGCGCGCT CGCGCATCAG CGGCAGGAAA GGCGGTAGCA GCACGATCGC 333120
TTGATGATCA GGCATTGCCA TTCTTGCCGT TAATCGCCAT TCTCCCTGAT CAAGGCCGGG 333180
GTCCACCATC CCGCGGATCG AATCCAACAC CGGCGGTAGC ATCTGGCCCA GGCGTTGGGC 333240
GTGAGGCGTC GGAACCATGC CGCTTGAGCC GCGAACCAAC AGATCATCAT TGAAAACGCC 333300
ACGCAGCCTG ATCAAGGCCC GGCTTACCGA CGGTTGGCTC AGGCCCAGGT GCCGGGCTGC 333360
CTGCGTCACG TTGCGGTGCT CGAGCAGCGC CTCGAGAGCC AACAGCGTGT TCAGGTCAAG 333420
CGATGCCAAC ATTTGCCGTC GCCGTGCGGC GCGAGGGCCG GCGACGATCA GTTGCGGCCG 333480
CTGCCATTCG TTCGAGGTGC CATTACAAGC GGGCTCATCC ACGAGCCTCT CCTTCTGTCT 333540
TCGTACGCAG TGTGCTCAGG CTAACGTCTC GGAGAACTGG CGGTGCGGAG TAGCCCGAGC 333600
ATGCTGAGCA CATCAGGGGT TTCTCCAGCC GGATCGACAT CGTCAAAACA CGGCGTCGAT 333660
TAGAGAGGTG ATAACTGGTC AAACGCTCGA CCCTTCAGTG CCGGCGACGA GCGCATTTCT 333720
GACGTTTGGC CGGTTACCGC TCGACGCTGT TGGAGGCGAT CTCTTCGGGC CGGCGACTGA 333780
GCCGGCGCAT TTGAGAATGC GGAGAGGATC GCCTCAACGA GCTTGATTAT CTGGCGCAAC 333840
CCGTGAGTTC TCCCCGCGGT CTGCGCCATC GCCTTACTTC GTCAGCCGGG AAACTGGTCT 333900
GTCTTCGCCT TCGTGCTGTG ACCGCGACAC ATTCACCATA CTTCCGCAAG ACAGATTGCT 333960
GGCTGATTTA TTTTACCCAG TCGCGGTCAG CCTGCAACTG GCGCGGCCGC GTCCAACGGC 334020
GCCGCCAAAC CTCGGGGTGG ATCCAGGCTT GCGAGGAAGT CGTGGCACCA GCGATAGACA 334080
TCGTGCTCAC GAAGCCTCTC CATCATCGCG GTCCATCGTC TAATCCGCTC ATCTCGCGGC 334140
ATGTCCACGG CCGTTTCTAT GGCGCTTGCC ATGTCCTCCG GGTCATAGGG ATTGACGAGG 334200
ATCGCTCCTT CCAACTCACT GGAGGCACCG GCAAAGCGCG AGAGCACGAG AACGCCCGGG 334260
TCCCGGGGAT CCTGCGCAGC GACATATTCC TTCGCCACCA GGTTCATGCC GTCGCGAAGT 334320
GGCGTCACAA GACCGACGCG TGCCCTGCGG TAGACGCGCG CCAGTTCCGC TTGGCTGAGC 334380
GACTGATTGA TGTAGTGCAC CGGCACCCAC CCAATGGTTC CGAGTTCCCC ATCGACGCGC 334440
CCCACCAGCT CGGCGACGTC CTTTTGCACC GTCTGATATG CCGGTATTTT GTCGCGGGAT 334500
TTCGGGGTGA TCTGCAGCAG CACTGTCTTG CGCCTGCGAT GTTTGTTGCG CTGTAGGAAT 334560
CGATTGAAGG CGTCGATGCG GTTCTCGATG CCCTTGGAAT AATCAAGACG ATCGACGCCG 334620
ATGGCGACGT CAAATCCAGC AAGCCTGCAA AAGCGATCCT GCAGATCGTC GTCGCGGTCT 334680
GCGAGACATG CGAACTCGGC CGTATCGATG CTGATTGGAA ATACGCCGCA GCGAAATTCT 334740
TTTCCATAGG CTCGGCAGTC ATCTCCGTTG AGATCCCCCA CTTTCTGCCG GAGCAGGCAT 334800
TTGGCGAAGT TGTAGCGATC GTTTTCGGTA TGAAAGCCGA CGAGGTCATA ACACGAAAGC 334860
CCGCGCAGCA GGTTATCATA GCTCGGCAGG GTGAAGAGGA TGTCGGCCGG CGGCCACGGA 334920
GTGTGCAGGA AAAAGCCGAT CCGGTTTTTG TGGCCACGCT GTTGCAATTT CTCAGCAAGC 334980
GGGATCAGGT GATAGTCTTG CACGGGACTG TCAGGAATTC TGTGCGGGGG GCCATGATGA 335040
TGAAAAGGAG CGAATCATCA CATGGCTATC GAAAAAGAAC TTCTGGACCA GCTTCTCGCC 335100
GGTCGTGATC CATCCGAGGT TTTCGGCAAG GACGGGTTGC TGGACGATCT GAAGAAAGCG 335160
CTCTCGGAGC GCATCTTAAA TGCGGAGCTC GACGAGCATC TTGACGTCGA GCGTAGCGAG 335220
GGAACAGCCG CCAACCGCCG CAATGGCTCT TCGAAGAAGA CGGTTTTGAC TGGGACGTCG 335280
AAAATGACGC TCACCATCCC GCGCGATCGG GCCGGCACGT TCGACCCGAA ACTGATCGCG 335340
AGATATCAGC GCCGCTTTCC CGATTTCGAC GACAAGATCA TTTCCATGTA TGCCCGCGGC 335400
ATGACGGTGC GCGAGATCCA GGGCCATCTC GAAGACATCT ACGGCATCGA CGTGTCGCCG 335460
GACCTGATTT CGGCGGTGAC AGACCAGGTT CTGGAAGCCG TTGGGGAATG GCAGAATAGG 335520
CCGCTCGAGC TTTGCTATCC ACTCGTGTTC TTCGATGCCA TCCGGGTCAA GATCCGGGAC 335580
GAAGGTTTCG TTCGCAACAA GGCGGTCTAT GTTGCGCTCG CCGTTCTGGC GGACGGTACC 335640
AAGGAAATCC TCGGGCTCTG GATCGAGCAG ACGGAAGGGG CAAAGTTCTG GCTTCGGGTG 335700
ATGAACGAAC TGAAGAGCCG CGGCTGCCAG GACATCCTGA TCGCCGTGGT CGACGGCCTG 335760
AAGGGTTTCC CCGATGCCAT CACCGCCGTC TTTCCCCAAA CGATCGTGCA GACCTGCATT 335820
GTCCATCTGA TCCGGCATTC CCTGGAATTC GTGTCCTACA AGGATAGAAA GCCCGTCGTG 335880
CCGGCGTTGA GAGCCATTTA CCGTGCTCGG GATGCGGAGG CCGGCCTGAA GGCGCTGGAG 335940
GCCTTCGAGG AGGGTTATTG GGGTCAGAAA TACCCTGCCA TCTCTCAGAG CTGGCGGCGC 336000

AATTGGGAAC ACGTCGTTCC CTTCTTCGCT TTCCCGGAAG GCGTCCGCCG CATCATTTAT 336060
ACCACGAACG CCATAGAGGC CTTGAACTCG AAGCTTCGCC GAGCTGTGCG CTCGCGCGGC 336120
CATTTCCCTG GTGACGAAGC CGCGATGAAG CTGTTATATC TGGTTCTCAA TAACGCCGCC 336180
GAGCAATGGA AGCGCGCGCC GCGGGAATGG GTTGAGGCAA AGACACAGTT TGCCGTTATC 336240
TTCGGCGAAC GGTTCTTCAA CTGATCAAAC CGGCCCCCCG CACAGAATTC CTGACAGTCC 336300
CGTCTTGCAC CCATATGACG TCGTCCTCCC GGAGGGCCTG CTCCAACAGA TCTGCGAAAA 336360
GTTCGTTGAC GCGGTAATAT GCGGACTCCC GCGTCTCGGA ATATTCAGCG AGGTCGGCAC 336420
GGTTGTGGAA GATCGGCCAA AGAACCTGGT TGGCGAAGCC ATAGTAGAAT TCTTCCAGAT 336480
CCGTCTGCGA GAGATCGGTA AGCGCGTAAG TGATGCCGCC GACGTCCTTT CTGATCAACG 336540
CTGACGGTTC TTCCTCGGCT GCGACCTTGC CCGACCATCC CATCCAGACA CCGCCCTTTC 336600
GCCGGAGTGC GGCGTTCAAT GCAACCGCCA TCCCCCCGGC CGACGACGAG CCGGGCAGGG 336660
TAACCCTGTT AGACACGACG ATCAGTCTAC TCATTTCGAC CTCCCGTCGT CGCACACGCC 336720
GGCCTATCCA TGCGAACGCG GCTCTCCAGT TGTTCAGAAG TTGCTGGCCA GGGTAGCTCA 336780
TTCTGCGCGA CCACGGCAGC CATGTGCGTA AGCATATACA CATCAGTATC TCGTATTGAG 336840
AGAAGATGTT TTGATGATGG TAACAATAGC TTCATTAGTA GACTTTTCAG ATGTAGGTTA 336900
GTTGGCATGT CAACTAATAA TAGTACTGAT AGTTTGCATA GTAAGGCTCG CAAAATGCAC 336960
GGCTCCGTCA ACTTTTCCCA GGTTTCAAGT GAAATAAAGC TGAGCCGGAT TAGTATGTTT 337020
CAAAAAACAG CAATCAAGCG TGCACAGATG CCAACGATGG GGCTAAAGGA GCGGAGGCAA 337080
ACGAGACTCG TAGGAAGAGG GTCTCCCACA ATGTTGGTGT ACCGCACCAG CAATACTCTA 337140
CGTTAAATCA GCAGAACGCG ACCGCCGAAG ACCAGCGGCC TTCGAGAGCT CAGTTCCCGC 337200
GCAACAGCAG AACGCTCCGC ATAGCCCTAT TTGTCTGCGC TATTCTGCTA TGCACCTGAT 337260
GATGATTGCC CCACTCGGTG GCCGTCCTCA TTTTGGGAGT GATGGCCCAG GAGGCCAAAG 337320
CTACAGCAGG AGTGACTATG AAAAACATTC TCGTTATCAG TCAGGACGCT TCGATGAGGT 337380
AACTGCTGAT GGAGTTCTTG CCAAAGCATG CGTTTAGCGT CAGAGCGGTG GAAAACGGCC 337440
AGCAGGCCGG TAGCAAGATG GCTTCCGAGG TGATTGATCT AGCAATTATT GATATGAGCC 337500
TCCGCCGAGA AGATGGTCAT TGGCTCGTCG GGGATTTTGG CGCAAAGATA GACCTTCCAA 337560
TCATCATCAT CAGCGGTGAC GACCTTGAAG AGGGTGACAA CATCAGCCGA CTCCACACCG 337620
GCGCCCTCGA CTGTATCTCC AAGCCCTTCG GCTTGCCGGA GCTCCTGACC CGCGTGAGCG 337680
CCGCTTTACG CGATCGGCCA GAGCGTCATC GGAACAGGAT CTTCCATTTC GATGACTGGC 337740
GCGTCAGCAC GAAGCGTCGC CGTTTGCGCC GAGGGACCAA TGGTGAAGTC AAGCTCACTG 337800
CGGGCGAATT CAACCTCCTG ATGGCCTTTC TCAACAATCC GGGCAGGTCC TTTCGCGCGA 337860
GCAGCTTTTG GTGGCGACGC ACGTCTATGA TCAAGAGGTC TTTGACCGCA GCATCGATGT 337920
TAAAATCCTG CGCCTTCGCC GCAAGCTAGG TCAGCACACC GGCTCGAACC CGCAATATAT 337980
TCGCACGGAA AGGGGCGCAG GCTATGTTTT CGACAGCGTC GTGAACGTTG ACGAGTTGAG 338040
GACGCGCGGC CACTGACAGC GCACTCATTC TATTTTGCTT GAAGGTCGCA TGTTCGCCTC 338100
AGGGCGGTTG GGGCGCTGGA TTTCCATCAA ATCCTCTCGT CAGATGAACG AGAGCTACTC 338160
CAACCAAGTG TCTCTTCTGG GTGTTGCAGT GGCGCTCCGT GTCTTAACGA CGGACCAGAA 338220
TGCCCCGGTT TAAAAGGCTC CAAGCGGCGG TCCGGCGCTG GAGCTCTCGA CGTTGCCGGC 338280
CGTCAAGACC GTAAAGCCGG CGGACTGCCA GGCGACTTCA TTAAAAGTCG CTTGTAGAGA 338340
CCCACAGTCC ATGTTGAGAA GGGGAGATAT TCGCCGGCCG CACGCTCAAT TGTCCTCGCG 338400
GCGACGACCG GCGAATGTGC AAGAGAATTC GATTGCGCAT GACGCCGGTC CCGAGGCTGT 338460
CGAAGCGAAG CTGAAGACAC GTTGGCACTC CCGTCCGATA ATCCAACGCA AGCGTTTAGC 338520
ACACCTGAGC CGGCGTCAAC GACGCCGGAG CGAGCTTGCT GCACCACGCC ATTCGCCGGG 338580
GGCTGAATGC TTGCATAAGT GAGTGGCGGC AGGGCGAAAG CTGCGCGAGG AATTCACGAG 338640
ATTGAGTGGC AAGCTGCTGT CGGTCGTGCG CGACGATCAG TTTGCCGGCG GATGGGCTGG 338700
GCCTCGATGA TGGCGCAGAT GCAGAACATT ATCGACTTCC CGCCCGCGCC CAAGCTGGAC 338760
GCGTCTGGAC GCCAAGCCGG GCGAGCGAGC AGCAGCGAGA GGGCGAACGG GTGTTTATGA 338820
GAGTCCGCTG CACCGTTCGC CACGCCCCCG CAGACCGCCT TCATGTGCAA CAACATGCAC 338880
GACCCGAAGC TCGGGCGCTT CTCTAATATC AGCCCGACGG CCAACGATCT CGTGATGTTA 338940
CCAGGTGGTC CAACCAAAAC CTTCACACCG CGACGCTGCG TGGCATCAAG GACGCTCCAC 339000
CCTACTTGCA CGATGGGCGG CTGATAACAC TCGCCGACAC GGTAGAGTTC AGCTAGGTCT 339060
TGTAGGTGGA GATCGTGGCC GCGGTGGTTT CCACCCTTTC GAAGAGAAGC GCCCGACGTG 339120
TTAATTGTCC GATCCAGGCA TGAGGCAGCC AGTTCTCGGG TCCGCCACAT AGGAGGATCA 339180
TGACTGGTCT GGGCGCTCGC GCTTCTGGAG AGCGGATGAT CGTTCTGAGG CAGCGACGGT 339240
CGGTCCAGCT TGGTTCGCCG CTCTGAGCGT TCGGATGCGT GTGCCAGTCG CCAATGTAGG 339300
TGTCTACACG GCCTGACACG CGATAATGAC GGTCGATCTC GGATTGCTCC CATTCACGGT 339360
CCGGGGAAAA GCTTTTGCGT GTGCGGATCG CTTCCGATCC GCCGTCAATC ATCTTCGTGA 339420
TTACCGCGAC ATTCGCGTCC GACCAGTATC CCATAAAGGT CCCGCCTGTT TCGAGGTCGT 339480
GCCAACGGCT CGCGTCTTTC AGCATCGCTT CGACGACGGA CTCCGGTATC CAAATCAGCT 339540
TCGGCATCCG CAGTCCGGGT GTCGTTCCAG TGGATCGATC CGCCAACTCG GCGGCATACG 339600
ACGTTTTACG TTATGCAGAT TGACTGTGCG GACCTCGGAT ACCGTCCACT CATCCGGGCT 339660
AGACAGGACA TCGACGGCCA TCCGGACGGT CTCCAAGGAC AGTTCCTTCA GGTCGAATGA 339720
AGCTCCGGTG AAGGTGAGCT CGGAGCAACC GGGTGGCTGA ACGAGCGTTG ATTCGTCATT 339780
CATGCCGTTC GGATAGGGGA TGTGCCCCGA CTTTCCTTCC GGGGTCTCGT TGGCGTGCGC 339840
CATGCACACC GGACAACCGG ATTCCGGATG ATGGAATACG ACCGCCCCGC CATGCAGGTT 339900
GGGTGAGGCG AAAACGGAGA TGAGCGAAAG GCCACGCTCC CGGCAGATGT CGCCGAGAAG 339960
GTAGGTAACG CCCGGCGCGG CACTGGCGTC CACAACGAGA TCGACGCTGT CTAATAACTC 340020
GTTCAGCCGA TCTGTTTCTC CGGGAATGTG CGGATCGCAG TTTGGTAATC CAATGCGGTG 340080
CGGGACGGCC AGAACGGACG TCCATGGAAA TTCCCGTTCG ATGAACCGCT TCAAGGCGTC 340140

CTGCTTCTTC GTTCCCCAAC TACTGGCTCC AAGTTCCCAA CGGATCGTGT TGCCCGGTTC 340200
GACGACGTCG TCGTCCATCA GGTGGAGCGA GGTGCAGCCG TTTCTAGCCA GTTCGACGGC 340260
AGCGGGTGAT CCCAGCGCCC CGAGCCCGAC AACCGCAATG CGTTTGCCTC TCAGACTCGC 340320
GACCGATGGA ACTCGATAGC CGATGTCTAT CTCGCCGTCC CGAAAAACAG GCACGATGGA 340380
CGGGACCGTG TCTTTACGTC GTGGTCCCCT ACGACTTGAG TGTGAAATGT GCTCTCTTAC 340440
CGCCATTCCG CACACCCAAG CATCTCCATG CTCGTTGTGT TGAAGTTCGG TCCTGCTGAT 340500
CGTGAATGAG AGCCGGATAT CTGCTCCGTC GCATGTCACA TATGTTTTCC CGTACGCGAC 340560
TTTGCCGACC GTCTGTTCAG CAGAACCTGT TGGTAGGGAA AGTTGGTCGT CCCGCCGCCA 340620
AGGGAAGGCG CGAATGGCAG TCGAAGAAAC GGCGGCAGGA AGAGGCGCAA CACGCTCCCC 340680
CAAAAGACTA CCGTCACCGG CCCTGACGCT TAAGATCGCA GCCCTGATGG TCCGGCCGAT 340740
TTCTGTCCGG CTTGCGTTCA CGAGGACATC GACAGTCCCT TCGTCATAGC CGGAGAGCTT 340800
CCATCTGGAG TCAACGAGGA CGAAGCTTCC GCTCGCTTGG CCGAGGCTGT TCCACCAATA 340860
CTCGATCGGC TCTCCTTGTG GATCCTCCGC TCCCGTGCCG TCCAGGACAT GCGCGAGGTT 340920
TTCGTCGAGG AGTTCCGCCA ATGACATGTT GGCCTGCCAA TAGCGGGTAT CGCGCCCCAG 340980
CAGACAAAGA GCTCCTTCGG ATCCACAGTG CCGCTCAGGA TATTCGGATG GGTCGCAGGT 341040
CAGAAAAACA TGGGGGCGCA GGCGTGGAAA GGTATCTGGA TAAACTGCCC GCAGCCCAAT 341100
CGTTCTTCCG TCGTTCAATG GCCATGAAAA CTCGATTCGA AGGATTCCGG CAGCCGCCGC 341160
CTCTTCATCT ACGAGGAGGC TCTCTCCAGC AGCAGAATGA AGCTTTTGAA GTTCCGCCAG 341220
CCTCCTGCGC CCTGCTGGTG TGACAGCAAG AGCAGGAGGT GGCTCAAAGT CGGTGTATGG 341280
CTTCATGTTC AACCAACGTG CTTCGGAGGC ACTTTGGGTG GAACGCTTGG ACCCGGATGA 341340
TTCGGCGGAC CAGGCGGATG ACCGGGATGA CCAGGATGAT CGGGATTGCC GGGCTCGCGT 341400
CCCGGATTGT CAGCTTTGCC TGCCATCTGA GGCTCCTTCT TTCTGCGGTC GGTGCTAAGT 341460
TTTAGTGTTG TTCGACTAGG CGTCTTCAGG CACGGTGAAC GGCTTAAGAA GCGTATACCT 341520
GGTTGGTATT CCATGCGAAG AACTTATTGG TGTATCCGTA GTCGGCCGAT TCGTCGAAAA 341580
TTGCGTCATT CATGTCGAGC GTCCGTTTCA GCTTGGTAGC TGTGAGCCCA GGACTGTGTT 341640
GCGGCCCCTT TTCGCTGATG TCTTTGATAT TGATGGTCAC GGCTTGTCCT CAATTGACTT 341700
ACTAATTGGG CCGAGGACCT TGACGAGCCA GCCTCTCAAT ATGGAGCGAC TATACGACCG 341760
CGCCTAAGTC AGATTTCACC ATTTATAGGG TGGAAGACGC AGGATATCAG CAACTTTTCA 341820
GCTGGCCCGC AGGGTTGCTG CAAGTAAGTG CGATGGCCCC AAAAGGTGAC GCGATCCAGT 341880
CGGCGGAAAG GACCAGGGCG TTAGTACTGC TTGCCGCGAC TTGAAGGCGC AGCCCGTCGG 341940
AGGAGGAGCT CGCATTTTTC GGTCGGGTTC ACGCCGACGC GATGCCGCGT CCGGCGCTGT 342000
CGGAAGCAAT GCGTGTCTCC TTCCTTGGGA CGGCGCCGTT GCCGAACTCG GCAAAGATGG 342060
CAGCGATGAA CGACGCTTCT GGAAATACAG GACGACGCCC GCAGATACAT TTGAGCTGCG 342120
CGTGGGCGTC GCTGCAGCCG AGCAAGCTGC GAGGCCGCGA CCTTCACGTA CAACGCTGTT 342180
GTGTTGATGC TGCGATGTAC GAGAAGATCG GCGACCTCGT TGATCGGCCT TCGGATGTGA 342240
GAAGTTGCTG CCGAACGGGT ACCGGAGGTC ACGATAGCGA CACCCGATGC TCGACAGCAT 342300
CAAGGACCTG CTCAGCCGTC AACGCCTCGA GGTTTTGGTA GGGATGGTGA TGGCGGAACC 342360
AATCCAGAAA GCGTCGGCCG CGCAGAAGAA CGCCTTCGCG GGTGTTCGGC TCCAGCCCCG 342420
TACCCTATGC AGATAATCCA AAAAGGAGGC CAGAAGCGGA GCATCCGGGT CAACCTCTTC 342480
ACTTGCAACG GGGAGCCACC CGCCGTGCGT GCCCCAGCGC CGACACGGCC CCAATGCGCG 342540
GAGGATCCGT ACTAAACGCG CCCAAATAGC GTTGACGACA TGGATCGGCA TCGACCGCAG 342600
TGCGTCTCGG CAAACCGGCT AAAGCGCGCA ATTCGGCTCA GGTAAATCTT GCCGACGCTC 342660
GCTTGTAACC AAGCGTGAAA AAATATTCCG CGAGGCGATC CATCTCGCCG CGGAGCGCAC 342720
CACTACTAGC CGCTTGAGCA CCCCACCATA CGAAAAATAT GACTCGAGCA TACCGCCCCT 342780
CCGGCATAGC GGCGCAGGGG GATCCCGCGC CTACCTCAGA GGAACCAATC TTATTTCGAC 342840
CACCGCGTCC GCCGCACACC GCACCGGTGT TTCGCAGCTC GAATGCCGGT ATGGAGCAGG 342900
TCGGCCGCGC CGAGCGTCAC AACGCCGTGG TCGCTTAACT TCCCACAAAA CCCTCGTGCT 342960
CTCCAGGCAT CATACAGTTT CTCCTGGGTA TCGCCCTCCC ATGTTGCCGC TGATGCACCA 343020
GATAGTTGGC CGCGAGAGCC CGCGGCTCGG CCCAGACCTG ACATCCTGCT GTCCCTCCTA 343080
CGCTCATTCG GTTTGCAGCA CCACGTGGAA CCTGCCGCCT CGTGCCATGA GGAGTCTTGA 343140
AAAGGCGCCC TGTTCACAAG GAAGCGCCTC GGCAGGTTTT GGTCAGTGCC CATGCGTGCG 343200
TTCAGCCGTA GGTAGTTAAT CGGAACTGGA CCTGAACGCG TAATTGGCCG CTGCTAGATC 343260
AACTGCATCG TTCACCGTTC TCACACGTTG AGGTTCAGCA CACATGAGGT GCATCGGGTG 343320
TGCATGAATA CCCAAAGCGG TTTCAAAGAA CTCGAGATCG TCATGCGCGC GATACCGGCG 343380
GTTTCTAGCA ATGGCCCGAA CGAATGCAGG GTCAAACTTA TAAACACCCA TTAGATTCCA 343440
ATCTGCAGAG GCGTGATGCT GGCTTGAAGG GCGGTCGGGT AGCACTGCTG TAAGGCGCAG 343500
CGCATCGTCC GCCAGTACGC GCAGGGATCG TCCGCCCTGT GTTTTGCTGA CTACGGCAAT 343560
ATTGCGGTCT TGCGACCGCA ACAGGCGTTC GATCACATCG AAGTCTATGA CCAAATTCTC 343620
CGTGAACACC AGCACGTCGC CATCAACGTG GGCGATCTCA TTTGCCGTTG CTGCGTCCAC 343680
GAGACGTCCG GCATTCGGCC TGGGCAGAGC GCGTAAGCAG AGCTCCATCC CATTCAGCTC 343740
GCCACCGAGG TCGCCGGCTA TTTCGCCTAA AGTTTCTCGG GCCAGCACCA TCGTCCTCTG 343800
CACCCCAGCG TGTGCCAATT GAGATAATAA GCGTCGCAGA ACCGACGTGC CGCCAACCAC 343860
ACCTAGAGCT GCTGAAAAGC GCCTCTTACA CGTCGAGATC ATCTGTTGTG GGTCGGTAAT 343920
AACAATCCCC AAGCTCTGAA CTTCGCGCAT TTCGTCGATC CTCGGACTGA GAACTTCCTC 343980
AAAATCTATC TGAGGGCGAG CGTTCTATCT ACTACCAGGG CAAATAGTAT TGGTGCGACT 344040
TCGAAGTCGG ACTGTAGGTG AAGTGCGCAA AAGAGTTGAG GTGTATTGAT GGGTCAATGT 344100
TCCGTAAGAC CGTATGTCCT TCTTCGGGAG TGAGGTGGCT CGCCCTAGAC TGTTCTTCCG 344160
AGGGTTGCTC GGACAGGCCG GGCCGATCCT CATAACAGGA GGACGAGCCG TGGCTGATTA 344220
TAGAGAAGCT TTTGTCGGAA TCGATGTTGC AAAATTAAAG AACGCGATTG CGGTCGCCGA 344280

ATCCGGTCGA AACGGCGAGG TACGATACTG GGGCGAGGTC GAGGCGTCTG ATGCAGGGAT 344340
ACGTCGCGCG ATCAAGCAGA TCACGGCGAA GTTTGAACAC GTGTATTTCT GTTACGAGGC 344400
GGGGCCCACT GGCTACGGGC TCTACCGGCT GATCCGCTCA CTCGGCCACG AATGCATCGT 344460
TGTGGCTCCA TCTTTGATCC CGAGAAAACC AGGCGACCGG GTAAAGACGA ACCGCCGCGA 344520
TGCCATTTCG CTCGCCCGGC TATTGCGGGC CGGCGAGCTC ACGGCGGTGT GGGTTCCCGA 344580
TGAAGATCAT GAGGCCATGC GCGACCTTGT CCGGGCTCGC ACGAGTGCCG TCGAGACGCT 344640
GCGCACCCAT CGCCAGCAGG TCAACGCGTT CATGCTCAAG CACGGTCGTA TCTATCCCGG 344700
CAAGAGGAGC TGGACGATGC GCTACCTTCG ATGGCTGCAA GAACAGCACT TCGACCATCC 344760
TGCCCATCAG ATCGCGCTGC AGGAGATGGT CGAAACGGTC ATCGCCGAAT TCGTGCCCCA 344820
CTGGTCACTC GCTGCGGTCG TATGCGCTCT GCGGGCCTTG CGCGGCGTGG ACTTGATCGC 344880
CGCGGTGACG TTTGCGACAG AAGTGGGAGA TGCCGGCCGC TTTGAGAGCC CCCGCCAACT 344940
GATGGGGTAT CTCGGTCTTG TTCCCGGCGA ACGATCGACC GGGGAGACAA CAAAGAGGAT 345000
CGGCATTACC AAGGCAGGCA ACAGCCGAGT CCGGACTTTG CTGGTTGAAT GCGCGTGGAC 345060
GTATCGGTAT CCGCCTCGGA TCGGCAAGAG GAAGCTGTAT CGCCTCGAAG AGGTATCATC 345120
GTCGGTTCGC GAGATCGCCT GGAAGGCGCA GACCCGCCTG ACGGCCCGCT ACCGCATGCT 345180
GAGCGCCCGA GGGAAAAAGT CGACGGTCGT GTGCACCGCA GTCGCACGGG AACTCGCCGG 345240
CTTCATGTGG GCAATTGCAA GGGAGGCGCG ACAGACCACG TCATAACAGC CGCGCACCGA 345300
CTCGCGCATT GGCGGAGGCG GGACAACGGC AGGGGAATGT CCGTTAACCG CTTTGTGGCC 345360
GGCAAGTCCG ACGCCCGCAG TAAGACAGGA ACAGCCCCAG ACGCAAAATC GGGAATGCGG 345420
TAGCCAACCC GCGTATCAGA GCTTGATCAC CGACGTCTTT CAGTTCCGCC TCCACCAATG 345480
CGCAAGCTAT CCACCTCGAG CGTTCCTCTA CGGAACGAGC GACTCGACGT GTCTGCAACG 345540
TTGACAGCGG ACATCAGAGC GGCAAGCAGA CCCCATAGCC GGAATGGCGC GTCGTGCGCC 345600
CGTCGATGGC AGAGCGGCGA CCATTGATGT TCTGCGCCAC ATGGGGCGTC ACCTTCATCG 345660
ACCGCAGATC TTTAACGAAG TCCTTTGTGT CATAGGCCTT GTCGGCACCC AGCGTGATCG 345720
CTTGTGTCCG GTCGGCGAAG TGCTCGATCA TGTGCAGAGG GCGGATGATG GCGACGCTGA 345780
TCGGATAGTT GGCAAGCTCG GGACCGCGAT CGGGCGAAGT GGTCAAGCCT GATCGTTCCG 345840
ACGTCGATCT GACAAATGGG TCTGTCCTCG TTCGGAAGAA GATATTCCGC AAGGACCGTC 345900
TCGTTCCAGT TCACGCGACT CAGGCTGCCC TTCGCCACTA GATGCGTGTG CGCAACGTGG 345960
CTTTTCTTCA GCCGAAGGAC CAGGCCTTCT TTCTGAGCTC CCGTGGCCAC CGCCTCTGTG 346020
CGACCGGCCT AAAAAATGGT TTTGCTGAAG TCCGCAAGTT CGCCGGCCTT GATGACGGCA 346080
AGCGGTTGCG GCCGCACGAT CTGCGGCACC GGTTTGCCGT GACCCGCCTG AGCATCAACA 346140
ACGCGCCAAC GCCCTGGCGC TGCTGCCGTT GCTGCCACCT ATCTCGGCCA CTCCAGCTAC 346200
AGTGACACAG CCCACTATCT CACTGGATCG GTGGACCTTC TGGCGGCGGA GCGCGCCTTC 346260
CTTGATGGGG GCGGAGCGTG ACTTCGGAGC TGGTCGAGAA CCCAGGCCAT GCGAAAAGCT 346320
GCTTGTTCCG CTATGCCAGG CCCATGTGAC GGGCCGCTGT GTCCGCCCAC CATCCGGATG 346380
CGGAAGACAA GATCGGGGTC GCGATCGGTC GCGCAGGACC GACGCTGCGC CACGTAACGA 346440
GCAGGTTGGT AATAGATCAC TTGGCCATCG TCGAGCGCGG CGTCGACGTA TGTAGGTGGA 346500
AGAGATCGCT CGGGGCTGAG GTTGTAATAC GGGTCGTAGC TGCGCAGATA CCGATACTCG 346560
TAGGCGTCCT GGGGATCTCC ATACTCTGCC GTTTCCTTTA GCGTGTACGG CATTGTCGAG 346620
TCCAGCTGGG TATCTATGAT GTCGGCCAGT GGGACCTCGG CAACCACGGC GCGGAACAAG 346680
TTGGGCCGAA AGACGGCCGT TGCAAGCACC GTACCCCCGC CACCACTCTT TCCCTCAATA 346740
ACGACGCCGT CGCGGGTGGC GAACCCACGC TCAATAAGCC CCTCGGTAGC GGAGATGAGA 346800
TCTGTGTGAG TAATGCGCTT TTGGTCGCGG GTAGCCGCGT CGTGCCATGG GCGTCCGAGC 346860
TCGCCACCCC CTCGCACGTG CACGATGCCG AAAGCTACTT CGCGGTCGAG CAGGCTCAAA 346920
CGCGCCGTCA TGGAAGAGGG CCAAGCGAGG AACGAGGGCA ATCTCGGTAT CCCGTAGCAG 346980
CCGTACACAC TCAATAACAC TGGTCCAGGG CTCGTTCGAT CGCGCCGCGC GACCAACGAG 347040
ATCGGGACCT GCACCCCGTC TTCAGCCTCT GCCATTACGA CCGTTGCAAG GTATTGAGTG 347100
GCATCATACC CGGGAACGCG GGCCTCGCAC AGGACCACCG ATCGGTCGTT GGCGAAATCA 347160
TGCTCGATGA AGGTGTCGGG CGTCACAAAC GAGCTAACCG AGTAGGTCAA CTTCGAGCTT 347220
CGGAACGGAT GTCGTGCGGC CGAATAGCAA CCACCGGCCG ACAGGCCGAC CCTTATAGTG 347280
CAGCTCGGCT CGTCGGGAAC GATCACAGCG CCAACCCGCC CGCTTCGATT ACGCGAGATC 347340
AGGCGAGGAC GGAGGCCTTC TCGTTCCAGG AGGACCAAGT GCTGTTCGAG CACGTGGATC 347400
TCGTCGATCG TCACGCCCGC TCGATGCGGC ACGACCTCCT CCCACCGCGA CGGTGAGGGA 347460
TCATCTATCG GCGCACTCAC CAGCCGCCAG TACGGTCCAG CATCATCCAC CCGAAACAGG 347520
AACCTGTCGT ACCAGTGCTC GGCATAGATC TGGTGCCCCA GTTCACGCAT CACGATCCGG 347580
CGCCACTGAC CTCCCGGCTC GTCGGCGGGA AGACACCACA CCTCTGCAGC CCCTCGCTGA 347640
ACGTAAGAGG ACATGTCCGA GGTAGTAAGT ACGTCGAGGA ATAACCAAGC GCCGCTCTGA 347700
GAACGCCGAA CCAGCACCGC CAGTCGCTCG TTTGCCTCCT CGAACACCAC CTCCGAGTTT 347760
CCTCGCCCGA CGTTCAAGCG GACGATCTGA TGATGTTGCC GCCGGTCAGG CCGCTCCCGT 347820
GTGAAAAAGA GCGTATGATT GTCGGCGGCC CAGACAACCT GCCCGACGCT ACCCGCGTCT 347880
CGCCAGACAT CGCGTCCGTC GGACATGTCT CGCACCCTTA ACTCGTAGCG CTCGGCACCG 347940
ATGAGATCAA CGCTAAACGC TAGGTAACGC CCGTCGTCAC TCGGCTCGAA CACACCGAGC 348000
CAATAGAAGA CGTCGGCCCC GGGTATCGCG TTCGGGTCGA GGACAAGCTC GGCCGAACCT 348060
CCGGTCACCG GCCGCCGCCA CCATACCGGA TGAGGCAGCC CCCGCTCGTG TCCCTGGAAG 348120
TAGTCAAACG GCCCAACTTG GAACGGTGGC GTGGCGCCCT CGCAAGGCTG ACGCCCTTCG 348180
ATCTCGGCGA TGAGCTCAGT CTTTAGTCTT CTCAGGTGAG CTGTCGCTTG CTCTGCATAG 348240
CTGTTTTCGG CTTCAAGATA CGCGCGGACG TCGGGATTCT CTCGGTCGCG AAGCCATCCA 348300
TACCGGTCGA CAGTGACGTC GTCATGAAGG ACACGGATCC GTGGCTCGGC ACGCGGAAGC 348360
GGTGGCCGCA GGCTCTTGTC CCACATCACA ACGCTCCTGA AATGAGAACA AGCGTCGTAT 348420

TCATCACCGC CCTCCACTAA CAAGTCGATG CGTCGCTGCA TCGCGCTTAA CCCGCCCATG 348480
AACCGGAGTC GCGATCCGCT TTAGATGCTT GCGGCCACAG ATTTGTGCAG GATGTCCAGA 348540
CCACGTCGAA GCGTCTGCTC GTCAGTTGTC AAAGGAGGTA GAAGCTTGAT AACTTGATCT 348600
TCCGCTCCGC ATCGCTCTAC CACAAGTCCC TCTTCAAAGG CCTTGCGGAC GATCCTCTCA 348660
GCCAGCTTAC CTGTACCACA ATTGAGCCCC AACATCATGC CCCTTCCGCG TACGGAGAGG 348720
CTACGAGCTC GGTTGGTCTG AGCCACCTGC CGAAGGCGCT CCGCTATGAT GCGTCCTGTC 348780
TCCATGACAC GGGCAGACAG CGCATCATTC GTCCAGTACT TTCGCAAAGC AGCGGCACCC 348840
GTCACGAACG CAAGATTGTT GCCCCTGAAA GTCCCATTGT GTTCGCCCGG CCGCCAAACG 348900
TCTAATTCTG GCTTGAGCAG CAAAAGGGAC AAAGGCAGTC CGCAGCCACT TATAGACTTC 348960
GACAAGACTA CAACATCTGG CGACAAACCC GCAAACTCGA AACTGAAGAA ATTGCCTGCT 349020
CTTCCGCAAC CTGCCTGAAT GTCGTCAACA ATCAGCAGGA TGCCATGGCT TCTGCAAATC 349080
CGCTGGATCG ACTGCAGCCA CTCTTTTCGC GCGGCGTTGA TCCCCCCTTC GCCTTGCACG 349140
GTTTCCAGAA TGATTGCCGC AGGTACATCT ACGCCACTGC TTGCATCAGC GAGCACCTTA 349200
TCGAGATATT CCGAAGTGTC GTTATCGGCA CCCCAATACC CGTCATAGGG CATGAACGCC 349260
ACCCCGGCTG GCGGAAAGCC TGCCGCGTCC CGGTAGTATC GATTGCCCGT AACCGCGAGC 349320
GCACCCAAGC TCATCCCATG GAAGCCGTTA GTGAATGAAA CTATGCTGTG CCGTCCAGTG 349380
GCCTTGCGCG CGAGTTTCAA AGCAGCTTCC ACTGCATTAG CGCCGGTAGG ACCGCAAAAC 349440
TGAAATTTGT AAGTCAGACC ACGTGGCCGC AATATTATGG CGTCAAAACA CTCCATAAAC 349500
TCTCGTTTAG CTGGCGTAGC CATGTCCAAC CCGTGGATTA TTCCATTTGA TCTCAGGTAT 349560
TGAGTCGCTT CGTCAAGAAA ATACGGATCG TTGTGACCGT AGTTAAGAAC GCCTGAGCCA 349620
GAGAGAAAAT CGATAAACTC GCGGCCGCTC TCGTCATGCA GAATGGCGCC TGCGGCTTTC 349680
TCAAACACCA CGGGGAACGA TCGCGAATAA AATCGCACGT TCGATTCGAG GCTCTCGAAG 349740
GCGTAGAGTG AATTGGACTT ACTCTCGTTT GACATGATTT TCCTCAACAA GACGATGGCG 349800
TCAGAGCGGC GCGCCTCATG CTCTTGAAAC CGATGCCAAT GCCACATCGG AATTTTACGT 349860
GGCTCCGCGG TTTTACACGG CGATGCAGGC CCACGCGCCT ACCCAGGCTG GCAGTATCGT 349920
CGGTGCCACT TCTTCAGTAT ATTCGCGTGT TCTGATATGA CTAGACGAGA ACCAGGAGAG 349980
TTTGCCCCTG GTGCCTTTCG ACATCCGATT TCCTGAGAGC TTGAAACTAT CTGAGTCGCA 350040
CTACTTATTG CAGACGCGAG AGGACATGTA GCCGCCATGC AGCCAGATGC GGCTCCCACG 350100
ATAAAGACTT GGCGCTCCCT CCAAGATCGG TTCCGAGCCG AGTCTGACGA TCAGCCATGT 350160
GGGTAACTCA CTGCCGCGGT GCAGAGGCCG CGAGCTCTGA ATGCAGGCTC ACAAGAGTAG 350220
CAGCGTGGCC GATGTTCGAA TCACCTGTAC TCCCTTTAGA GAGTGCCCGA GCCTGACGTT 350280
GCTTGGTCGC TTCTTACAAT TCGATAATTC CAAGGCTTAT TGCTTTAATC GCTGCGACGG 350340
TTCTGCTCGA AGTACCCAAC TTCCGCATCA CGTTCTTAAT GTGAAAGTTC ACAGTATTTT 350400
CGGAAATCCG CATAATAACC CCTATGTCCC ACGAAGATTT CCCTCGTGCC ACCCACAACA 350460
CACACTCTTT TTCTCTTAAA GAAAGGGCAG GAATTTTTTG GACGGCGTTC AAATTCGCGA 350520
TTTTCGCGAC TCTCAGATGG AAATGGGTCG CGGCAAGTTG TAAATAGGCG ATTGTTCTGT 350580
CGTGGAATTC ACGCTCGCAA TGCCGAGCAA AGCTCATGAT CGCGCAGCTA CCTCTAGGGC 350640
CACGCAATGG AACAGTAATG CCGGACCTCA AGCCAAACAT TGCAGCCTCG TCAAACATGC 350700
GCCGCTCGTA TTCAGTGGTG CTTGCGTCAC TGTACATGTC GCTCCATCGA ATCGGGCCAG 350760
CCCCCATTCG ACTCTCTTTG ATGACAGGGG CTATCGTCTC GTAACCCATT TCGAGACATC 350820
GCTCCCGCCA ACCATCAGGA TAATTCAGAA TCTCTTCCGA GTCGCATTTC ACTCGGTTCA 350880
GAGCCTTATG GTCGCTTGTA AGCGGACCAT AGGCCAGCCA CTTGCAGCCA AATTTAAACG 350940
CAAAAGCCGA CAAAAGGTCG AACAATTTTT TTGGTTGAGC GACACCATCT GTCAGGTCGA 351000
GAAGCCGGCC GAGCTCGACC ACATACTCGT CCCTACCTGC GTTTGAGAAA TGGATCGATC 351060
CTCCCAAGGA CTGATCAGAA ACTCCGGTGG CCGATCCGCG GACTTGAGGG AAACTGCAGC 351120
CATCCGCGAG TCTTCCACAG TCTCGCTCGA GTGACGAATC GCCCGCCATG TAGTCACCGG 351180
TGCACGACGC GGCAGCCGAT GAGGCATTAC CGCTCTGCGC CCCCACCGGC CGGTATTGCA 351240
GGGCGCTCAA TGCAAGTCGA TGGAGATCTG GCATCTTGCT CTCCTCCTTC GTCCGTTGAG 351300
TAATGTATAT CGTTGACGAG TCCTACTCGC GTCGCCAATG AGGTTCAAAA GTCGTGCCAA 351360
GTTTGCAGAA AGGGGGCGTC AACGAAATTC TTGTTCGTTT GTCAGGGATT TGAACACCAT 351420
GCAGCCAGTA CTCAGCCGCA TGCCGCTACG TCGTGGGCTC GACAAACGCG ACAGTAGTGT 351480
CCGTTGCGTG GTTGCACTGT GAGGCTATGC ATTACGCATC GGCCATTTGC TTGTCCAGGA 351540
AGCCGTTCGG CAATCCAAAG CGGGAGCCGC AGCCTGCTGC CTCGACTGCG AACTAGTTTC 351600
AGAACGAGGC GCAGACATAG AAGCTCACAT CGGCTCACAA CGCGCTACGA TATCCGGCCG 351660
CCACCCCCTT CCGCCAGACG GGGTTCCCTT GCCGCTCTTA CGGTCGAGAT CACTTTCGCA 351720
TACCACAACG GCGACGAGGC CATACTCAGG GCCAAACAGG AGACTTCTTC AACAGCCTGC 351780
TAGGGCTAGC TGTAGGCGTA GACATAGATC CTCACAGTCA GAGAAATCGT AAGCGGTCAG 351840
CCGATAGCGT CAGGCTTGAA GTTCCAGGGC ATGAGCGCTT CGATTTCGGA TGCGGGCCAG 351900
CCTTGAGCAA TGCGGGTCAA GGTCTGCGAG AGCCAGTCGA GCGGATCGAC GCCATTCATT 351960
TTGCAGGTCT GCAGCAAGGT GGCCACCGTC GCCCAGGTTC GTCCACCGCC CTCGCTGCCG 352020
GCGAATAGAC TGTTCTTTCT CGTAATCGTT TGGGGCCTGA TCGCCCGTTC GACGATGTTG 352080
GAGTCGATCT CGATACGGCC GTCCGTCAGA AAGCGCTCCA GCGCCTCGCG CCGGGTGAGC 352140
GCGTAGCGGA TCGCCTCGGC GGTTTTGGAT TTTCCCGAGA CCTTGCCCAG CTCCTTTTCC 352200
CACAGCTCGA AGAGGCTGGC GACGATGGCC GAGGACTTTT CCTGGCGGAG TTCAGCGCGG 352260
CTTGCGGCAT CCTTGCCGCG CACTTCATCC TCGAGCCTCC ACAACTCGGT CATGGCGATG 352320
ATTGAATCCG TTGCGGCTTG CGAGACCCCG CTGATGTGCA GGTCGTAAAA CTTGCGGCGC 352380
AGGTGCGCCC AGCACCCTGC GAGCTGGATC GTTTCATTGC TGCCGGTTTT GGCCCGCGCC 352440
TTGGCCAGGT TGGTATAAGC TGAGTAGCCA TCCACTTGCA GAATGCCGCT GAATCCGGCG 352500
AGATGACGCG CCACGCAATC GGCGCCCCTG CCGTCTTCAA AACGGTAGGC CACCATCGGC 352560

GGACTGGTTC CGCCATAGGG TCGATCATCG CGTGCGTAAG CCCACAGCCA GGCCTTGGTG 352620
GTTTTCCCGG AACCGGGGGC AAGGGTGGGC AAGGTCGTCT CGTCGGCGAA GACCCTTTCG 352680
CCCTCCTTGA CGCGCTCCAG GATATAATCG GCGAGCATCT GCAACTCGAA GCCCAGATGC 352740
CCCATCCACT GCGCCATCAG TGATCGGCTG ACCTCGACAC CATCGCGCAG ATAGATCGCC 352800
TCCTGCCGGT AAAGCGGAAG GCCGTCGGCG TATTTTGACA CGGCGATATA GGCGAGCAGC 352860
CGCTCCGTCG GCAGGCCGCT TTCGATGAGG TGTGCCGGCG CCAGTGCCTG AACCACGCCG 352920
TCGCGGCCTC GGAAAGCGTA TTTCGGGCGG CGCGTCACGA TGACCCGGAA CTTCGGCGGC 352980
ACGACGTCCA GCCGCTCGGA GCGGTCCTCG CCGATCAGAA CCTTTTCCAG CCCCGCGCAG 353040
TCGGCCGGGA CTTCCGGCTC GATAACCTCC TCGATGCGTT CGAGGTGTGC GGCAAAGCCC 353100
TTGCGCGGAC GCGGCGCACG CTTCGGCTTG TCCCCGGCCG CGCGGTTGAG TTCGCTCTGG 353160
ATTTCCGAAA GGCCGGTTTC GACCTCCTCG AACGCAAAGG ACACCTGTTC GTCGTTGACA 353220
GCCAAGCGCA GCCGCTCGGA CCGCGTGCCA TGTTGCGTGC GCTGCAGAAC TTTCAGGATC 353280
GACGTGAGGT TGGCGATCCG CTCGTTGGCG CTTTTCTCGA CGGCCTTCAG CCGGGCGACC 353340
TCTGCCGCCG CGGCTGCAAG TCGGGCCTCG TTCGCAGCTC GCTCCTTGGC CATTGCGAGG 353400
ATCATCGCTT TCAGCGCATC GACGTCGTCG GGAAGATCGG CAACAGGCAA AACCATGGGA 353460
GGCAATAGAG CACAAAAACA GCCGTTTTCC CAACCGTTAC AGCCACATGA TTCATCATGT 353520
CGCAGGCCGG TTTCAGCCCG TTAACAACGG CCGCCTGACC TTGGCCGGGC GGATCTTTTT 353580
CCAATCGAGC CCGGCCAGAA GCGCCATCAA CTGCGAATGG TCCAGACGCA TCCGCGCCGC 353640
CGATATGCCC GGCCAGCAGA AGCTTTGCTC TTCCAGAGTT TTCGAATAGA GGCAGACCCC 353700
GCTGCCGTCC CACCAAACTA TCCGAACACG GTCCGCCCGC TTCGATCGGA ACACGTAAAG 353760
CGAACCATTG AACGGTTATG TGCCGCACCA CATAATGCAG AGCGTTTTTA GCGTGGCGTC 353820
GCGTGTATCC ACGTTGACGC GCGCGCCGAG GCGGATGGCG ACGGTGCGCT AAAAACGTGT 353880
TGGACTAAGC CGCGGGTGCG TTTCAGGTCC TATGGGTATT GAATGGTGCG GCTCGAAGGA 353940
AAGGCGTCGC AGGGGCGGTG ATGACGTACG CCGCCGCTGG GTGCACGGGA CGAAAGCAGC 354000
GCCGATATGG AATTCGACAG GGGTTGAGAT TGCCATTGTA ACCTGCCGGG AAGGATCGGT 354060
AGGCGCAACG GCACGCACCT TCTCGGGACC GACCGGTTTC CGGCGGAACG GTGCGACGGT 354120
GACGATGAAA GCGCTTCTTT CATGACGTGT CCCAGCATCG CGGCGGTCCG GTGACGCCGT 354180
CGAAGATTTC GATGGTGATC ATCTGCCCTT GATGACAGGC CGGGCATTGT CTGAGGGAAC 354240
GCCCGGTGAG TTCCTCGTAG CGGTCGCGGT ACTCCTTCGG CGCCTGGGTC TCCGATGGGG 354300
CGGGAACGGA CATGCCGAGC AGGTCGCGGC ATTGGGCAAG CTTTTGTTCG CGATAGCGAT 354360
TGCCGAGGAA ACCGTAATAG CGGATGCGGT GGAAGCCCTC AGGCAGGACG TGTAACAGAA 354420
AGCGGCGGAT GAATTCGTCG GTCGACACGG TCATGACCTT TTGCCGGTCG CCGTGCCGAT 354480
AATCCTTCCA GCGGAAACGC ACCGCTCCAT CTTCGATATC GACGAGCCGG TTGTTGGAGA 354540
TGGCAACGCG GTGCGTATAA CGGCCGACGT AATCGAGCAC CTGTTCGGGC CCGGCGAAGG 354600
GCGGCTTGGC AAAGACGACC CACTCGGCCT TTCGCAGCGG CGCCAGATAG CGCCGGAAGG 354660
CGTTGCGCTC GTTGAGGGCT CGCAAGTCGG AGAAGAACTG CAGCTTGCCG CCGTCGAAGG 354720
CTTTCTCCAG GTGCTCCAGA AACAAGCGTC GGAACAGGCG AGAGAGCACG CGGACCGGCA 354780
GGAAGAAGCC GGGTTTGCAG GCGATCCATT GCGTGCCGTC CGGCGAGAAC CCGCCACCCG 354840
GGACGACGCA GTGCAGATGG GGATGGTGCA GTAGATTTTG CCCCCAGGTG TGCAGGACGG 354900
CGAAGAAGCC GATCTCGGCG CCGAGATGTC GGGGGTCGGC AGCGATGGTG CGTAGCGTCT 354960
CGGCGGTGGC GCGGAAGAGC AGCCCGTAAA CGAGCGCCTT GTTCTGGTAA GCGATGGCGG 355020
CAATGGCCTC CGGCAGTGTG AAGACGACGT GAAAGTACTG TGTTTCGAGC AGCTCGGCGC 355080
GACGATCCTC CAGCCATTGC GCACGGGCAA GCGATTGGCA GCGGGGGCAA TGCCTGTCGC 355140
GGCAGCTGTT GAAGGCGATG CGCCGGTGGC CGCATTGATC GCACGCTTCG ACGTGCCCAC 355200
CGAGCGCGGC GGTCCGGCAC AACTCGATCG CCGTCATGAC GCGGCGCTGC GCGGTCGAAA 355260
GCGACGTGTG CTGGGCACGA TAGGCCTCGC CGTAGCGACG GAAGATATCC GCCACTTCCG 355320
GCCCCGAACG GGCCATCGGG AGCTCAGAAA TACTCAGGTT TGGCGGGCGG CGGCGTGGGC 355380
GCCGGACGGG GCAAAAGCTC GAACGGGCTT GAGGTGGCGC AGACTTTGTT GGTGGCGATG 355440
CGCAGGTAGT GGGCGGTGGT CGCGAGGCTG CGGTGACCGA GCAGCAATTG AATGGTGCGC 355500
ACGTCGGCGC CGGCCTCAAG GAGATGGACG GCGAAGGCAT GCCGCAAGCT ATGTGGCGTC 355560
ACCGGTTTGG ACAAGCGGGA GAGATCGTGC GCTTTCGCAC AGGCTTGCCC GACTGCATCC 355620
CGAGTGATCG GGTGGCCGGC GCGATCTCCG GGGAAGAGCC ACTCTTTTGG CCGCGGCATC 355680
CTCCAATAGT CGCGCAGGAT CTCCAGGAGC TTGGGCGACA ACATCACATA GCGGTCCTTC 355740
TGGCCTTTAC CCTGCTCGAC ACGGACGACC ATTCTCTGGC TGTCGATGTC CGTGGGCTTC 355800
AGCTGAACCG CTTCCGAGAT GCGCAAGCCA GCGGCATAGC ACGTCGTCAG GATGGCGTGG 355860
TGTTTGAGAT CAAGCACGCA GCCGAGAAAG TGCTGTACTT CATCCGGACT GAGGATGATC 355920
GGCAGCTTCT GCGGCTTCTT CGGAAGCGGC AATACCTCTG CAGGCACCCA GTCCCTCTCG 355980
AGTGTGACGC TGAAGAAAAA GCGCAACGCC GCAATGGCGA TATGGATTGA GTTAGGCGCC 356040
AGCTTCTTCT CGTTGGCCAG ATAGACCTGA TAGGTCCGAA TATCCTCGCG GCCGAGCAAG 356100
TCCGGCGCCT TGCCGAAGTG TCGTGCAAAC AGCGACACCT GCCGCAGATA TGAAAGCTGG 356160
GTATTGAGCG AGAAATTGCG CACCTGCATG TCCTCGCTCA TGCGCTGGCG AAGTGGGGTC 356220
ATGACGAGCT CCTCTGTCTG ATGGAATAGG CTGCCAAACA GCCGTCCCAT CGTCGCGCAG 356280
TTGGGGCTCC TACTGAATAC ATGACCTCCT TGCCGCTCCG CGGTAGCGGA GCGGGTTAGT 356340
CCAAATGCGA TTATGTGCCG GGCGAGATTA TGTGGCGGAG CCGTCGCGGC GCCAGCCGAG 356400
GCCCGTCTAA TCTGGAATTC TCCGCCACAT AATAGTCAGA TACTGTTCAG TAGTTGCAGC 356460
ACGGCGTCAT TTGGTCGGAA ACGGATGCCC AGATCGCCGG CGACTCCAGC CTTCTCGAGA 356520
CCTTTGCGCA TCATCTCGAC ATCGGCGGCG GCATAGCGAT GGGTTGTAGC GACCTGTGCG 356580
TGCCCGAGCC AGGCTTGGAT CGTTAAAAGG TCAACGCCCG CCTGGAGAAG CTTCATGGCC 356640
AGTGAGTGTC GGCTATGTCT TGATCGATAT AGGCGACACT ATCTACCCCG CCGGACTATG 356700

TCGTGTAACT AGATTTCGAT CAAAGATTTC AATGCTTTAT AGATAGCGTG TAGAGATAGT 356760
TCGTAGCCCT TCCAGCAATC CAGCTTGGGA AGGGAGCGAA GAATGAAGTA TTTCGTCAAT 356820
GCCGATTTTG CGCTGTCGCG GCCGCCAGAG GGCCCGGTGG CGATTTACAT TATTCCTTTT 356880
GCCGAATGGC TCGTTGATCG AGGCTACGGC CTTGTTTCTA CAAGGAACCA GGTGCTGATG 356940
GCTGCCGGCT TCAGCAGTTG GCTTCGGCAA AAGGGGATTG GACTCAGCGA CATAAATGGA 357000
GAGCATGCTG GGCGTTATTT GCTCGACCGA GTGCAGCGCC CAAAGCTTGG AGATGACGCC 357060
GCTCTTCGAC ATCTATTGGC TTTTCTTCGA AGTCAAAACG CGATCGCCGA AGAGATTGAG 357120
GTCGATCACA ACCCGTCAGC GGTAGAACAA CATGTGCAGG CATATGAGCG GCATCTGCGA 357180
GACGCCCGTG CCCTGTCGCG TCAAACGATC ATAAATTACC GACCTGTTGT CCGGGATTTT 357240
CTCAGCTTCC GCTTCGGTGA TGGCGAGATC TCGCTCGCAC AATTGCGCGC CGCCGACGTG 357300
ACCGATTTCG TGCAAAAGAA GGTATCGCGC CTCAATATGC GACGCGCAAA GATTGTGACC 357360
ACGGCACTGC GGTCATTTCT CTCCTATGCG CGTTATCGGG GGGACATCAC GTCGGACCTC 357420
GCGGCCGCGG TCCCGATCGT GGCTAATTGG TCGCTCTCAT CCATTCCTCG TGCAATCGGC 357480
CGCGATGACG TGAGCCGATT GCTCTCCAGC ATCGATCGGG ATACGCCCAT CGGATGTCGC 357540
GATTATGCGA TGATCCTCGC ATTGGCGCGA CTGGGATTGC GGTCGAGCGA GGTGGTGACG 357600
CTCGAGCTCG ACGATATTGA CTGGGTAGCC GGACGGATCC GGGTGCGCGG TAAACACGGA 357660
CGTAACGAAC TTCCGTTGCC GGCGGACGTT GGCGAGGCGA TTGCCGACTA CCTGTGGAGG 357720
GCGCGTCCGC GCAATGCCAG TCGCCGTGTT TTTCTACGCG ACAAGGCCCC GATCCGAGGC 357780
TTCGTGGGCC CGAGCGGACT CGGGTCAATT GTCAGACGCT CACTCAAGAG GACCGGCATC 357840
GACTCTCCAA CAAAGGGAAC GCACCAATTC CGACATGGGC TTGCCTCGGA GATGCTGCGT 357900
GGCGGCGCGT CGCTGGGTGA GATCGGCGAA GTCCTGGGAC ACCGTCATGT GCAGACAACG 357960
GCAATTTACG CCAAGGTCGA TCTCGACGCG TTGCGAACAC TGGCTTTGCC ATGGCCGGGA 358020
GAAGCCCAAT GAGCACATTC CGACAGGCTG TTCAGGAGTA CATCGAGATG CGGCGAGGGC 358080
TGGGGTTCAA GCTGCGAGAG ACAGAACGGG GATTGATCGA TTTCGCCGCC TTCCTGGAGG 358140
CCAACGACAC GCCACACATC ACGACGGAAC TGGCCCTTGC CTGGGCTCAG CGACCGTCGC 358200
GGGCGCAGCC TTCGCATTGG GCGACACGGC TGGGCTATGT CCGCGTATTC GCCCGTTATC 358260
GGGCCGCCGC CGATCCGCGA ACTCAGATTC CTCCAAGCGG CTTGCTTCCC TTTCGCCCGA 358320
AGCGGGCTCG ACCATATCTC TATTCGAAGG AAGACATCCA ACGCCTCCTG TCGGCCGCTC 358380
TGGAGATGCC GTGTCGATAT ACCCGCTGCA AGCTCAGGCC ATGGACATAT TATTGCCTGT 358440
TCGGGCTGCT GAGCGTTTCC GGCTTGCGGC TCGGCGAGGC GCGCAACCTC AAGCTCGCGG 358500
ACGTTGATTT CGACGCTGCG GTGTTGACGA TCCGCGGAAC GAAGTTCGGA AAGTCCCGTC 358560
TTGTACCGAT GCACGCATCG ACATGCGCAG TGCTCCGCGA TTATCTCAAA CGCCGACGAC 358620
AGCATTGTGC AGCCCAGGCG GCATCTCCCT ATTTATTCAC TTCGCAACTG GGCAATCGCC 358680
TTGATGTCGG AGACATTCAC CGAACATTCT ATGCTCTGTC TCGCCAAATC GGCCTGCGCG 358740
GCGCAACTGA CAGCCACGGT CCGCGGCTGC ATGACATGCG GCATGTGTTC GCCACGAACA 358800
CGCTGGTGCG CTGGTACGAA GCCGAGCAAG ATCCCGAGCG GCTCCTGCCC ATTCTGTCCA 358860
CCTATCTCGG TCATGTCCAC GTGGCCGATA CCCAATGGTA CCTCACCGGT TCACCCGAGC 358920
TGATGAAAGA AGCAATGCGC CGCCTTGAAC GTCGCTGGGA GGATCGGACA TGACCAAGCA 358980
CGCCAGCCTC GCGCCACTCT TGGAGAGCTT TTTCCTCCAA CGCCTGATGC AACAGCGGCA 359040
GGCAAGCCCC CATACGATCA GTTCCTATCG CGATACATTT CGGCAACTGC TGAAGTTCGC 359100
AGAACGAAGA TTGCGCAAGC CGCCCTCTCG CCTGAACTTC GAGGAGATCG ACGCGCCGCT 359160
GATCGTTGCC TTCCTTGATG ACCTGGAGAA CCGCCAGGGT ATCAGCGTCC GCAGCCGCAA 359220
CCTGCGCCTC ACGGCGATTC ATTCCTTCTT TCGCTATGCG GCCTTCGAGA TACCCGAGCA 359280
TTCCGCCCAA ATCCAACGCG TGCTTGCGAT TCCCAGCAAG CGCTTCACCC GAACCCTCGT 359340
TAATTTCCTG ACCCGTCCGG AGGTCGATGC CTTGCTGGCC GCACCGGATC GATCGACCTG 359400
GTCCGGCCGC CGCGACCACG CGTTCCTCCT GGTCGCGGTG CAGACCGGAT TGCGCCTATC 359460
GGAGATCACC GGTCTCAAGC GGGATGATCT GTTCTTCGGC ACGGGGGCCC ACCTGCGCGT 359520
CATTGGTAAA GGGCGCAAGG AACGCTGCAC CCCGTTCGCC AAGTCTACGA CCGCCGTCTT 359580
GAGAAACTGG CTGAAAGAGC CGCAGCGCGG AGACCAAGGT ATCCTGTTTC CCAGCGCCAG 359640
AGGTGAGCGG CTGAGCGTTC ATGGCGTTCA GTATATGCTG AACAAACACC GTCAGATCGC 359700
TTCTGCCATG AGCCCGTCGC TGGAGGGAAA GCGCGTCACT GTTCATCGTC TGAGGCACAC 359760
GATGGCCATG GACCTCCTAC AGGCCGGCGT CGATCGCGCC GTCATCGCCC TGTGGCTCGG 359820
CCATGAATCG GTCGAGACGA CACAAATCTA TCTCGAGGCG ACGTTGGCGA TGAAGGAGGC 359880
GGCGTTGGCA AAGACATCCC CATATTCCGG GAAGTCATCC CGATTCCGGC CCGACGACAA 359940
TCTGCTGGCG TTCTTGAACA GCCTGTAGAT CCCGCTGACT ATGTCGTGTG GCTCGGCCGA 360000
TCCTGTAGGA AATCTCGTTG TTCCTCAGAC GCTTGCGTTT TCGGTACCCG ACATAGTCGG 360060
GTGGGGTAGA TAGTGTCGGA AGATGTGCGG CGATATAGGC TTTTGGGCCA AGGTCGGCCT 360120
GATGGTCACG GCCTGGGCCA CCGCACGCCG CACGATGTGG GTTGCTCCGA AGCGCGTCAG 360180
GCGCTCGTTG CGGGCGCCGA CGAATATCGG TCGCGGCTCG TGGTTCGCGA TTCCGTGCTC 360240
GGCCAACAAG GCTGTCAGTG CTCTCGCCAG ATCCTGAGGA ATGGGGATGA CGCGGTCTCT 360300
GCGCCCTTTG CCGCGCAGCA GCACTTGCGG GTGCGACCGT TCCAACTGAA GGTCGTTTGC 360360
GTTGACGCCG GTAGCTTCGG AGACCCGCGC GCCGGTCCGT GCCAGGAACA GTAAAAACGT 360420
GCGGTCACGC CGACCACGGG GCGTCCTTGG ATTGGCGCCC TCAATGAGGG CGTCCACTTC 360480
GGCCTTGGTG AGGTGATGCG TCACCTCGAT GTGCGCCCGT TTTATGGAAA TCGTCAGCAC 360540
GCGTTGGGCG ACACCGAAGG ACGCCGGATC GGCGGCCGCG ACATGGTGGA AGAACGATCG 360600
GATCGCCGCT AGGCGGGCGT TGCGCGTGGC GATCGTGTTG TTCCGCTTCT CCTCGAGTTC 360660
GTCGAGAAAC GCGAGAATGA GGTCTCGATC GAGCTCCTCG AGCACCAGAG CCGCCGGCTT 360720
CTTCCGCAAC CGGGTGGCGG CAAAGAGGAT GAGCATGCGC AAGGCGTCGC GATAGCTGGC 360780
CATGGTCGCG GGAGTCGCGT TGCGTTGTTT GGTCAAGCGC CGGCGAAAGT AGGACTCCAG 360840

GAGCGGCGCC AGGAGTAAGT GTTCGCTCAT GCTGCGCCTC CATCGAGGAA GGCGCGCTCC 360900
GCCGCCATGG CGAGAAGATC CACCGAGCCA GTGAGGTAGT AGGCTGTGTC ACTGTAATTG 360960
GCGTGGCCGA GATAGGTGGC GAGCACCGGG AGTAGCGCCT GGACGTTGGC GCGTTGTTGA 361020
TGCCAAAGGC TCATGCGGGT CACGGCAAAC CGGTGCCGCA GATCGTGCGG CCGCAACGTC 361080
TTGCCGTCAT CAAGGCCGGC GAACTTGCGG ACCTGAGCAA ATCCGCATTG CAAGCCAGTC 361140
GCCGAGAGGC GGTTGCCACG AGAGCTGAGG AAGAAGGCCT GGTCCTTGGG ACTGGGAAAA 361200
GCGGCATCGC GCTCACGGGC ATAGCGACAA AGCGCTGTTT GGGTCGTCGC GTGAACCGGA 361260
ACGAGACGAT CCTTGCGGAA CTTCGTCTTC CGAACCAGAA GAACCCCGTT GGTCAGATCG 361320
ACATCGGAAC GATCAAGCCT GACCACTTCG CCCGACCGCA GTCCCGAGCT TGCTAACAGT 361380
CCGATCAGCG TCGCCATCGT CAGCCCCCTG AGAGGGATGC CTGGCGAAAT GCGGGCGCAT 361440
GCGTCGATGA GCGACGCCAA CTCTGCCTCG CTGAGGATAC GTGGAGGCGG AATCGCCCTG 361500
GATCTAGGAA AGGCTCTGCG CTTCAGGGCT TCGGTTTGGG CGTCATAGAC AGCGAGATAC 361560
TCGTAGAATC GGCGGAGCAC GCCGTGACGA GTGGCGCGGC TGTTGGCGGC GCCGCCGAAC 361620
GACAGGACGA AGTCCAGCGC CATAGTCCGG GTGGCGGGCG CATCGAATTG AGCGCGTTCA 361680
ACGTAGCGGA CGAAAGCCCG CAACGTGCCG GCTTGTTTGC TGAAGGCATA GCCGAGCGAG 361740
TGACGCAGGT CGATGTAACG CTCGACCTTC TCGCCGAGGA ATCGGGCAAA GGCGGTCATG 361800
CAGCGCCTCC CGGAAAGGGG AGTGCGACCT CGGCGAGTTG CGAGGCCGCA ACCTTTACGT 361860
ACAGCGCCGT TGTGTTGATG CTCCGGTGAC CAAGAAGATC GGCGACCTCG TTGATTGGCC 361920
TTCGCTGCCC GACAAGCTGG GTGGCGAGGC TGTGGCGCAG GAGATGTGCA CCTGCGACCC 361980
GCCCGAGTTC GACCCCGCCA TGTCGCAATC GCTTCCTCAC GATCCTTGAT ACAGGCGCCG 362040
CAGACTTGAA CGCTCCCACC GGCGGCGTGA ACGACAGGAA CAGATACGGA CTGTCCACCT 362100
TCGGTCGAGC GCGCAGGATG TAGTCGACGA GTGCGGCGCC GGTCTCCTCA AGGAGTGGCA 362160
CCACCCGATC ACGCTTGCCC TTGGTGCGCC GGATAAAAAC CTCGCCAGTA CGCCAGTCGA 362220
TATCCTGCAG CCGAATGGCG CGTAACTCGC CGTTGCGAAT GCCCGTGGTG GCGAGCAGCA 362280
GCAGGACGGC TCGATCGCGG ATAGCGACCG GCGTCGTTGC GCCGATCGCA TCGATTGCGC 362340
GCCGAACGTC ACCCCATGCA AGGCGCGGCG GCAGATGCGC CAAACGCCAA TAGGGCGTCC 362400
TCGGGACGAT GCGGGCAAGA TCTTGGCGAT GGTGGCCAGC CCAACATAAG AACCGAAGAA 362460
ATGTTCGAAT GTGAGAGGTC GCTGCCGTGC GGGTGCCGGA GGTCGCCGAT AGCGACAGCC 362520
GATGCTCGAC AGCAGCGAGC ACGTGCTCGG CCGTCAACGC CTCAAGATTT TGGCCGGGGT 362580
GGCGATGGCG GAACCAATCC AAAAAGCGGC GGCCGCCCAG AAGAATACCT TCGCGGGTCT 362640
TCGGCTCCAG GCCCCGTACC GTGCGCAGAT AGTCCGAAAA GGAGGCCAGA AGCAGAGCAT 362700
CCGGGTCAGC CTCTTCACTT GGAACGGGAA TAATGAACCG CTCCGGAGCC ACTCGCCGTG 362760
CGTGTGCCAG AGCCGATACG GCCCCGATCC GCGGAGTATC TGTAGTAAAC GTGCCCAAAT 362820
AGCTGTCGAT GACATCTTGA TGGATTGGCA TCAAGCCACA GCGCGTCGCG GCAAACTGGC 362880
TAAAACGCGC AACCCGGCTC AGGTAAATCT TGGCGGACGC TCGCTTATAA CCGAGCGTAA 362940
GAAAATGCTC GGCGAGGCGA TCCATCTCGC CACCGAGCGC ACCGCTACGT AGGCGCTTGA 363000
GCACCCCACG ATACGAGAAA TAGAACTCGA GCATGTTGTC CCTCCGGAAT AGCGGCGCGG 363060
GGGGATCCCG CACTCATCCA AGAGGCACGA AATATTATGT GGCGGAGAAT TCCAGATTAG 363120
GCGGGCCTCG GCTGGCGCTG GGACGGCTCC GCCACATAAT CTCGCCCGGC ACATAAGAAC 363180
GGGTCGAGGC CGCCGTCCCT CACCAGCGCC ATCAAAGACG CGGCTCCCTT GCGGAAGTCG 363240
ACCGGCTGGC ACGACACGTA AACCACAACT CCGGAAGCGA TCATACCTAG CGGACCACCC 363300
GGAGAATCTT GGCCAGGTGA TCGGGATCGA CATCGGCGCC GGCGCGCACC ACGATATCGC 363360
CCACGACGAT TTCCACCATC GAACTGCCCA CCGCTTCAAA GCGCGTGAAC TTGACTGGCG 363420
CGGGCGCTCC CTCCGTCAAT GGCGCGACCA TTCCCGACGA GAGCGCCTTA CGACGCCAGG 363480
CATAGAGCTG CGACGGGTCC AATCCCTGAG ACCGCGCAAT CGCCGACACA TTGGCCCCCG 363540
GCGAGAGCGC TTCGGCGACA AGCTGCGCCT TCTCCTCGTC CGACCAATGA CGCGGCTTGC 363600
GTCGAGACCG CACCGGCTCC GCCGTCAGGA CCTCGAATGT TCGATGATGA TGATGATTCA 363660
CACTGTCGCT CATAGGTTTC TCCGCATGAC TCATGCAGAG AATGACCGTT CAGCGGCCCT 363720
AAAGATACGT GGGGTGACCT ACCCGCTTAC GAGAAATCGA ATGTCTGCCT TATCTAGTCA 363780
CGCGTTAAGC CGGCCAGATG GGGTCTGCTG GTCGGTTTCG AATCACTGAA GCGTCGACAG 363840
CCTCAGCTTT CTCGTCCGGA TCCGATGGAT AGTCTATGGA AAGCTCACAA CTGGTTAGTC 363900
CGGAAAGTAC AGCGGAGTAT ATTGTTCCTG ATCATGAAGG TCCGAGAATG GTGAAATAGA 363960
CCAATCGATT GATTCAACTG CGCGTTGAAT TATCGTATCA GCGCTGGAGC GCGAGGGCGC 364020
TACAGCAACG ACATATCCAA TTCGGTCACA GTAATCACCA TTTCTGACTA TTGGCGTTCC 364080
TGGTCTAACA TACAATTTGA CGTCCGCGAC ACCGGGTATA GCTGCCGCCC GGTCGATACC 364140
ATCAATGTAA TCAAGTGTGC CATCGCGATC CGCAATCAAG GATCGGGCGG TCGCTGTTCG 364200
TGACTGCCTC TTCGACAAAT TGCATTGTTC ACCAATAGCA AGCTTGATGT GCTCGCCGAT 364260
GAGATCGACG TCGAAGGCCA GCTGAACCAA TTGCGGATCA GGATTACCAG CAAGTCGCGG 364320
GTTGACTTCA ATAACGACTG GACCGACCTC GGTCCACCGG AACTCAATGT TCGTTGGTCC 364380
CCAGCCAAGG CCGAGTGCCC GGAGGCAGCG TTGTACAACA TCAACGGCAC GCTCGTACTG 364440
CTCATCGGTC AAGGGTGCAG GAAAGGTCAA CTGGCGGAAC ACGAAATGCG GCGGTGGGCC 364500
ATACTCAGTG ATGCCTATTC CAATAACTTC GTCTCCCATG ATCTCGGCGA TGTAATGAGG 364560
GCCATCCACG AACTGCTCTA CCAATACCTC GGGTGAAGAT CGCCACATGT GCTTCCCGCC 364620
CAATAGATAA TCTGTGTGTT CAGCTAGCTC GCGAGCGTCG CGACACAAGC GGACTCCTCG 364680
GCTACCGCTG CCGACTGCAG GCTTAAGTAC TGCTGGCAGG CCGATCTCCG CTGCAACGCA 364740
TTGTACTTCG GCGGCTGTGG TCGCCGACCG ATAGCCAGGC ACGGGAACTC CGGCTGCCGC 364800
AAGGACCTGA CGTTGGGCGG CCTTATTGCA GCATTGTTCA ATTGATTCCG GATCAGGTCC 364860
AGGTAGATTG AAATTCCGAC AGATCTTGCC AACTGTCGCA TAGAACGACT CTGCGGCAGA 364920
TGTAACTCCG GCTATGTCGA AACTCTTACC CAGCCGAGAA TATTCGCCGA TCAGAGCATC 364980

CAAATTGGTT GTATCGACGC GAGCGACTTC AATGCGTTCC GCCGCGACAT AGTCGTAAAG 365040
TGCTGGATCT GCCGCCAGGA TTATTGGATG TAGCCGAAGA CGCCGGACGG CTTCTACGTA 365100
CGACCGGCCA TTAGTCCGCG TATTTTCAAT TAAAATGAGC GCTTTTCGCA TTTTATCTGC 365160
CTCTGCTGTT ACGTTCTGTG GGTGGCCCAG GCGATCTGCA TCAGTTGTTT CAGCCGCTGA 365220
GGACGGTGCC CACTCCCGGC TGTAATTGCG CATGCACAAT CGGCGAACGC GTACATCATA 365280
AAGAGCCAGA GTGAAGGAGC GTGCTGTCCC GAACATGATT TTCCCACTCA CGGGCGACTT 365340
CTGACGTATT CGAGAGCATC TTGAGAAGCT CGTCGCGAGT CGTCCAGAGG AGCCATTCGT 365400
CGTCGTCTAT TCCTAATGTT GGCAACCGAA GGACTGATCC ATGGGGCTCT CGATGACAGT 365460
GTCCTGATCG ATCGAATATT CTCTTTTTGA TGTACCATTC GGCCACAAGT GAGTAGATGA 365520
AATCAGGGGA CCAGTTCACC CAAGCAAGCG CGAATGCAAG GCGACCAAGC GGTGCTAGCA 365580
AACCACGACC CCTAAAGTCG TCGCGAAACC ACATGTCACC GCGATAAGCG ACTCGCCCGG 365640
TAATGCTGCG AGCGCTGGGT GCGTAACAAC GACACGTTGA GTTAAGGCCT GATTTGCCGA 365700
CGGGGTCCGC AAAGCAGGCG CGCAGCGACT CCAGATGCTC AACAAGTGTG GTTTTGGATA 365760
GATCGTATAG TCGCAATGCC TGTACATGGG CGACTCTGTC TTCTTGGTCA CGCCCTACAA 365820
TCCAGAAGGA GCTCTCAGGT GAAAGATCAG AGTAGTTCGG ATTGAAGTTC GGAGTAGTTG 365880
GATATTTGCC TGGAAGTTTG CTGGTGATCG CCACGTACTG CCGAAAATCG TCGCCCATTG 365940
CAATATTGAG GCCTGCGTTG CGGGCTACAT CATCTGCGCA GGAAATGTAC TTTGAAACGT 366000
CAAGTGTGGT CACGTTGCTT TGCATGTTGG TACACCCCGT TTTATTGCAT CGGACGGCTC 366060
TATTTCTAAA GCTCTGGCCA ACGCTCGGAT ACTGCGGCAT TAGGTGGGTT GACGGGCGGG 366120
GCGGCTCGCG GCCTCGAAAG GGAGTTAGTA CCATTTACCC CGGACCGCCC TAGCGTTTCT 366180
GAATGCCATT AGGAAGCTCG CAACGGCGGA GCAAGTGTTG GCACGAGAAC TTGTTCGATA 366240
TAGGCGCCGA ACGCTATGTC ATGCGGATCG AGGAATTGAC CCCTCCTATT GCCTCACCCA 366300
GGAATGTTAC TGAAGTTGAG GGCACTGAGA TACGGGCGCG CACTCCAACC AGTCGCCATT 366360
GCGTATGATC TGCCCGGCTA TTTCAATCCG CCAAGCCTTG ACGGCCTTTT GTACCGTCCG 366420
CAGGCTTTTC TCCGTGAAGC GTGACGGATC GGCGCTCTTT AGACGCTGCA GGACCGAGGC 366480
TGCCGAGAGC GCCGGATCCG CCTCTAGCCA CGCTCGAATT TGGGTTCGAA TGGCTCCAGC 366540
AGGCTCGGCT TTTTGGGTAA GGCTTAGTCC GTCGATATGG CCGCCGACGT GTCGGCCGTT 366600
TCTCTCCCCG CCTGCCAGGC CGTCTTCAGG CTCGAGGTGA ATCGCTGGAG ATCGATGGCG 366660
ACCGGTTCTT CGGGCCCGCA TTCGAGCCAC GGCGATCGAC CCGCTTTCCG AGCTCCTCCT 366720
GGGCAGCACG GATGCCCGCA AACAACGTCA CCGGATTTTC CCTTTCCAGC ATGCCGTCGT 366780
CTTTTGGCTT TCACGATCGG GCGATCCGTC GGCCGGGTAG CTCCGTCCTT CCAGGCCGTT 366840
CGCAAACTCG CGAGGAAAAG GTCGATCGGC TGAGCTATCC CGTCAGTGCG CATGGCCGGC 366900
GGCGTGTCGG CAAGCGCAGC GAGTCGCTCC TGCAGGGCGC GAATATCGCG CAATAGCAGG 366960
ACTGGATCGA GCCCGGCGTA GATTTCCTGC AGACGGAGCG AGCTGCATCT TACGCGCGAG 367020
CGTCGGCAAT CAGGCGCTGA TGCGGCGTGG CTGGGCGGAC TATACGTCTT GCGCACACGA 367080
GCACCGTCGC GCTGCTTCGC CATTAATTTG AACGATGGCT GAAAGAAGTT CACGAACAGC 367140
CGCGCTGATC GGTAGAGCTT CGCTAGCAGC GTGGCGGCCT CCAGTCCCTG GAACCGACGG 367200
TATCCGACCA TCCTGCGCAC CACGGCGCCA TTCTTCTGCT CGACAAAGGC CCGGTCGTTC 367260
TTGCGGTAGG GGCGGCAGCG CGTGAAGACG ATGTTGGCGG CGTCACAATA GCCTTCAGCG 367320
TCTCGTTCAT GAAGACGGTG TCGTTGTCCG TATCCAGGCC GAGAAGCGCG AAGGGCAGTT 367380
GCTTGCGCAA TTCTGTGAGC ACACTCCTCA ACAGTGTCTG TTCGCAACCC AGCAGCGGCG 367440
CACATTCCGT CCAGCCAGTC GCGATGTCGT GAGAACAAGG GTTTGGATGA AGCTGCCGCG 367500
AGCCCTAGGG CCGCAATGGG CGACGAGATC CGCCTCGACA AAACCTGGCG CCGTCATTCC 367560
AGCGCGCCGA CGCCGGCCAC CGCGGGCCAC CACACTTCGG CATCCGGCAG ACTACGATCG 367620
AATCTCATCG CCCCTTCACT CCAAGCACAA ACGCCATCTC TTGGATGGCG CAACGGCGCT 367680
GAGCTATGCC TGCTACTTCC GCTGGCCGCT CATCGGATCG CGCTTCAACG TCTCCTGCAC 367740
CATCAGCGAC AGGCCGGGAA AGCCCATGCG CATATCCGTA TAGCCGGTCG CGAGCGAGAC 367800
CTTCACACCG ATCGGGACCG GGATCATCGC CGCTCCAGCA CATCAAGAAT CCGACCGAGG 367860
GCATCAGTGT CGATGTCATT CTGGACCCCC AGGCGACGCC CACCGCCCGG CTCGATCGTC 367920
ACCATGCTTG CCTTCTTGCG CGAGCGAGGG GCCGGCGGCG GCTCCGGTGA TACCGGCCTC 367980
GGCGGCACTG GCGATGAATG TCGCCAGGAG ACGTGGTCTC AAGAGAGCGG TTGTCGCGCT 368040
TGGCCGCCGG CTGGAAACGA TCATGCATCG CATGTGGAGT GACGAAACGG AGTTTTTGCT 368100
GGAGCAGAAA AGAAGGTCTT GGCCCGGCTC AACGCTGCTG ATTGAAGGCT CCGCGCCGAG 368160
CATGGGGTCT CGAGACCGAA CGGTGGAGGT GAAGATATTC CGCCTCCGGC GGTATGGATG 368220
TCCTTCGCAG GACGATGGAT GAGGTGAGTT CGTTGCAAGC GCTGTCCCGG TCGCATCAGT 368280
TGCGGTCAGG GCGTCTCCTA AATTTAGTCT AGTGCCCGTC CATAAACGCC TGTTTTTGTG 368340
GAATTTTCAA AGGTTTCTTG CCGGCTGACG CGTGTTTCTT GGACGCGTGA TGGCGCAATG 368400
CCATGCAGAT TATCACCCGA AGAGCGATCT GGCGGCAATG GGTCATGGGG TATCCGCCGA 368460
CGCTCGCCGG GGTTTGCTCT GAACAGTGCC AGACAAGGGG AGTCAGGTGT GCCGGAGGCG 368520
GGCGAAGAGG GAGAGATTGT AAGCGACCGC CGAGGACCAG ACATAAGCCT TGAAGTGGTC 368580
GAGGCCGCGC CAGGTGCAGC GCCCCAAGCC ATAAGCGCGT TTCAGGCAAG AGATGCCGGC 368640
GTCGATGCCG GCGCGGAAGT TTCTGAGCTT GCGATAGACC CAGCGACTCC TGACCATGTC 368700
TTCGATCTTG AGACCGCGCT TCTTGTGGAA GGCCATGTCG CGTATGCCGC GGGCTTTGGC 368760
TCCGCTCAGA TTTTCGCGGC TGGCATAGCC GCCGTCGGCC GCCGCCTGAC GCGGCGGCTC 368820
GCCGTAAAAA GCGATGTGGC GTTCCAGCAT CGGCAGCAAG CGCTCGCTGT CGGCCGGGTT 368880
GCCCGCTTCG ATGACAAGGT CGAGGATCAG TCCGCTTGTG CCGGTGGTCA GATTGAGTTT 368940
GTGGCCGTAG TCGACGTCGC GGCTGCCCTT GACGATGATG TCGACATGCG GCTCGAACAG 369000
GCTCGCCAGC TTCTCGCCAG CCGGCACCGC CTCGCCGGCC AGGACCCGCC GCTCCGTCTG 369060
GGCAATGATC CGCTCGACCA GCGGCTTGTA GCGGCAGACT TGCGCCTGCC AGAGTTCGAC 369120

CGCCGGGCCC GCCGTGAGGA GCAACTGTCC ACTCGCCTGC TGCAGATAGC TCAAGGTGGT 369180
GCGCGCGATC TTGAGCAGTT CGCGGTAGTG CTTCACCCGT TTCGGGCGAC CGCGGGTATA 369240
TTCGATCGCT CGAGCCCGCT TCTTCGCCGC GCGGCAGTGA TCGTGCCATG GGATGGCGCG 369300
ACCCAGTGCA TCAGCCTGCT GCAACAGTCG CACCATCACC CGGACAGCGT CCCATAACAG 369360
GCTGCTGTCG CTCGGCTCAT GGATCGGTGC GGCGGTGACG GTGCTGTCCA CACGCACGAC 369420
CTTGCCGCTT TCCAGTTTCT CCTGCCGGGC GCTCGCCAGC AGCACCCGGT TGATCTCTTC 369480
AAAGGTTTGC GCCCGGATCG GGCTGATTGT CTTGTGCAAG ACCGACTTCT TCGGGTTCCA 369540
CCCCCACGGC AACCGGGCAA AGGCCCGGAA AGAGGCGGAG TCTTCGAAAT GGAAGGCCAG 369600
TTCCTGATAG ATCCTATGAG GAGAGGTGGA TAGCGATCCG CGCTGTGACA AAGTGATGTC 369660
GCTTTCAATC AACACCTTAC TCTGGAGAGG CCGAACATGC TCGCTGCGAA TGAACGACCT 369720
GAAGCGCCGA CCGCTATCCG CATTGATCTT GGCGCAATCT TCGTCTCCTT GGAACTCAGC 369780
CGTTCGAAAT GGCTAATCAC CTCGCTGTCG CCGGGCGGCG GCGAGAAGAT GTCGAAACAT 369840
GCGGTGGCGG CCGACGACAT CGCCGGCCTG CTGGCGCGGT TTGCCGAGCT CAAGAGGAAG 369900
GCGCGGGCCC GGACCGGGCG CTACTTTCCC ACCATCGTGA TCCAGGAGGC CGGGCTCGAC 369960
GGCTTCTGGA TCCACCGGGT GCTGCAGGCC GAGGGGATCG AAAGCCACGT CGTCGATCCG 370020
GCCCCGATTC CGACCTCGCG CCGGCGGCGG CGGGCCAAGA CCGACAAGAT CGACGGTGAG 370080
ACGCTGGTGC GCGCGCTGCT CGCCTATAAA AGGGGCGAGC CGCGCGTCTG TGCGATGCTG 370140
CGGGTGCCGA CGCCCGAGGA GGAGGACCGT CGCCGCATCT CGCGTGAGCG CAAGGCGTTG 370200
ACGAACGAGC GCGTACGCCA CGTCAATCGC ATCAAGGGCC TGCTGTTCAG CCAGGGCGTC 370260
TCCGGCTACC AGCCGCTGCG CCGCGACCGG CGCACGCGGC TCGAGGAGCT GAGGACCGGC 370320
GACGGCCGAC CATTGCCGAC GCATCTTAAG GCGCAGGTCG GCCGCGAACT CGATCGGCTG 370380
GAGCTGCTGA TCGGACAGAT CAAGGCAGTC GAGGTCGAGC GCGACGCCAT GCTCGCTGCC 370440
GCGCCGGTCG GCTCTGCGCA TTTCGCTGAT TGTGAGCAGC CGGCACCGGC GATGCTGCTC 370500
GCCTTGAAAG GCATCGGGCC GGAGTTTGCC GCTGTCTTGT GGTCGGAGGG GCTGTCGCGT 370560
CATTTTGACA ACCGACGACA GGTTGCCGCC TATGCCGGCC TTGCGCCGAC ACCCTGGCAG 370620
AGCGGCTCGG TCGATCACGA CCAGGGGGTA TCGAAAGCCG GCAATCCAAG GCTGCGAACG 370680
ACGCTGATCC AGGGGGCCTG GCTGTGGTTG CGCCATCAGC CGCACTCAGC GCTCAGCCTG 370740
TGGTTCAAGC AGCGGGGGAA GCAGAACGAT GGTCGTCTGA AGAAGAAGAA GACGATCGTG 370800
GCGCTCGCCT GCAAGCTACT CGTCGCGCTG TGGAAATACG TCAATGCCGG CGTCGTCATC 370860
GAGGGGGCCG TGATGAAGAC CCCCTGATCC CGATCGCAAC CGAAATTCTG CAATCTTCCA 370920
GGACCCGATC AGTCCTGGCG GATCTAGGTG GGACGAACCG CAGTTGAGTA TGGCTTCAAA 370980
CGCCGCTTGA TAGAATGGTC TCGTCCTCCT GAGCCCTTGT CCGCCGCAAG CGGGATGTTG 371040
GTTCCGCTGT CTCGAACAGC GACCGTATAT AAGTTGGATC TGGCTTGGCA ACGAGCCGCG 371100
TCTTGCAATC GGGCTCAGAC CGTGGATGCC GCAACAACCG ATGGAAAGCA AAAAAAGTCA 371160
CCCTGACGAC AGTTCCACAT TGACGCTCCT CATATAAACT CAACTGGCGA TGCTGCTTGA 371220
GCAGCGCGCA GCGCAGCACA GCCTCGGCCG GCAGCCCCTC GCGGCCGGTC TCCTTGAGGC 371280
CATGCCGGCG CAGGTCCCGC GTCACCAGCC CGAGCAGATC GCGATGCTCA TCCAGCCATT 371340
GCGACATCAC CTTCAGTTCA CGACCGATCT CGTGTTCGGC GAAAAGATCG AATATGCTCG 371400
CCTTGTCAAG CGAATCGAAG AATTGACCCC CCCTATTGCC TCACCCAGGA ATGTTACTGA 371460
AGTTGGGGGC ATTGAGATAC GGGCGCGCGC TTCATCCAGT CGCCATCGAG TATGATCTGC 371520
CCGGCTATTT CCATGCGCCA AGCCTTGACG GCCATTTGCA CCGTCCGCAG CGCTTTCTTG 371580
GTGAAGCGTG ACGGATCGGC GCTCACAAGG CGCTGGAGCA CCGCGGCTGC CGAGAGCGCC 371640
GGATCTGCCT CAAGCCACGC TCGAATCTGG GGCTCGAATG GCTCCAGCAT GCTCGGCCTT 371700
TTCGGGTAAG GCTTGGTCCG CCGATAGGGC CGCCGATGTG TCGGCCGTTT CTCTCCGGCC 371760
TGCCAGGCTG TCTTCAGGCT CACGGTGAAT CGCTGCAGAT CGATGGCGAG CGGTTCTTCG 371820
GTCCCGGCAT TCAAGCCACG GCAATCGACC CGCTTTCCGA GCTCCTCCTG GGCGGCACGA 371880
ATTCCGGCAA ACAGCATCAC CGGATCTTCC CTTTCCAACA TCGCCTGCAG CCGCTCCTTA 371940
TCGGCCTCGG CCACACCGGA ATGGGCAACC ACCCCGCGCG ATAGGCGGCA CGGGCGGATG 372000
GTAGCGTTTG ACGACGCGGG CGCCGATCCG TGTCTTCTCT CGCAGCTTGA ACGAAGGCTG 372060
GAACAGATTG CCGTAGAGAC GCACCACGTC GTAGAGACGC CCAAGGGCAG CCGTCGCCTC 372120
CGCGCCGACG AGCCTTCCGT AACCGACCAG CCGTCGCACG ATCGCGCCGT TCTTCTGCTC 372180
GACCCAAGCC TGATCGTTCT TTCGATACGC GCGCGAACGG GTCACCTCCA ATCCCTGACT 372240
TCGGCACCAG CAGACCACAC GTTCGTTCAT GAAGGCGCTA TCGTTGTCGA AGTCCACGCC 372300
CTGCAGCGGG AATGGAAACA GCGAGCGGGC CTGATTGAGG GCAGCGATAA CGAGACCGCT 372360
TTCGCGCGTG CGGACTGGCA CGCACTCGGT CCAGCCCGTA GCGATATCGG TTAGCACCAT 372420
CGTCTGCACG AAACTACCGG ACGAAGAGGT CCCCGAATGA GCCACGAAGT CGACCTCGAC 372480
ATAACCGGGC AGCGGATCGT TCCAGTCCCC GAAAGTGCGC ACCGGACCGT AGCGGCGAAC 372540
AGCAGAACTC ATTCCAGCGC GCCGACGCTG GCCACCACGA GCCACCACAC GTATCTCTGA 372600
CAGCAACCGG TCCATCGTCG CCGCACTGAC GGTCAGTAGC TTATCGCGAA GCTCGGTACT 372660
CAGATCGAGC CGGCCGTGAC GCTCAAGAGC TGGCAGCAAA ATCGGGATCA GCGGCTTCAG 372720
ACGTTTCGAG CAGAGGCGAT CGGATGCTTC CCACAGAGCC ACCAGCGCTT CCCGAACCTC 372780
GGGACCATAC CTGACAGCAT ATTGCCGAGC GCAAGGCGGC TCGCTCTCAC TAGGGCGGAG 372840
CACTCGGATC GCATGCTTGC GGTGATATCC GGTAACTGCG ACGAACTCAT CTAGGATCCT 372900
TTGCTTGTCC GCCCGACAGC TCGATCGATA TCGTTCGATG ATCGCCTCGA CCAGTTCCGT 372960
CCGTGTCGCC ATGCTGATCC GCCGTGCCAT CCATCCCCCG AGCCGGAACA GCGAACCATG 373020
GCCATCCCAT CGGGAGCATC AACACTATCG GTAACATTTT GCTTGAGGCA ATGCGGGGTC 373080
AATTTTTCAT TTCGCCCGAC ACGCCTGGAC GGTGCGTTCT TGGCGCATTG TCGGCTCCGG 373140
CGGTTACGGG TTTATCTTTA GAATCAGTAG CTTGATCTAA AGTATACCTG AAGCCGCCGG 373200
GCTTTGCCCG CCGAGATATC GTGATTAATA CAATAATTTC AGCAGTTTAA CGTTTATGGA 373260

CGGGCACTAG TCTAGATTAG ACCGCTGCAT TCTTCCGACC CCATGGTGGG AGGGCCAGGG 373320
TGCCGATCCC CGAAAAGAAA AAAGAACCCG CGGAGACCAT TTCAACGCTA GCCGGAACGT 373380
AAAGCTCAAA AGCGCTTGAC CACAACCGCC GAATAGAGAA GTGCTCGCAC CAGGAGTTGC 373440
AGCCTTCAGG TCATCCGGCC TTCACCGTGG GATACCGTCA GCCTCGCGCA AGCGACGATG 373500
TGCTGAGCCA GTGCGCATGC GTTTGTCACC ACTGCCCATT TATAGCGCTG AGGTTTGCGA 373560
CATCCCGCAA CGGTACGTTA GGCCACCGGC GTTGCATCGG GAGGGATGGA GAGGGTTGCA 373620
ATCGCCGCAA GCACAGATGT GATGCCCGCC AAAACGAATA TAGCCGACAA CGATTCTGTG 373680
ATCACTACCC CAGCAATGGC CGCTCCTAGT GGGAAGCCCG CTTGGTTTAC GCTTATCGAA 373740
ATGGACATCA CTCGACCAAG CTGTCGGGGA TTGGTGCGCC GTTGGCGCAA GGTCAGCATC 373800
GCGACATCAA TAGGACCGGA CATTGCGCCA GCGAGCAACA GGCCGATCGT CAGGCCACGG 373860
AAGCCGAATT CCGCTGCAAT GGGCCACGTG GCAAAAGCGG TTACGGCCAT TCCGGCGGTC 373920
ATAATGTGGC GCTCGCGCCC AGTGGTGCAC AGATGTCCAG CGAGGAGCGC ACCAACACCT 373980
CCGGCGATAC CGACCAGAGC CCATAAAAGC CCTACTACTG TACTGCCCGC CGCAACAGTG 374040
TAGTTACCGG CAACGAACAC CGGAATGACC ACGTGAAGGG CGCCCCATGT CATTTGATAC 374100
AGCGACTGGG AGACGGCCAG ACCGCGTAAT GTGGGCTGTC GGACCACCAC ATAAATGCCT 374160
TTGATCGCTT GGCGTAGCAA AGATGTGCGT GAGGATGCCA GCCCTGGCAG GCGCTGGACC 374220
TGGGATAAGC AGACCGCGGC ACCTGCACAG GCCGCAGCGA TTAGAGACAT CGCAGCTTCT 374280
GGCCCGAGCC ATCCGACGAG TCCGCCCGCC ATCGCCGGCC CCACCACATC GACGATGGAA 374340
TAGACCGCAG TGTCTAGCGC ATTCGCCTGA TCAAGCGCGT GTGGTGGAAC GAGTCGGGGC 374400
AACAGCGTGC GGATTCCAGC AATGCCTAAG GGGCCGGCTA GCGAAAATAG CATCGCCAGG 374460
GTGCATACGA CTGGAGGACT GGACCAACCA AGCCAACCGG CAAGGCTAAT CGCGGTAATG 374520
AAGGCAGTGC TAGCGATCAT GTCGATCCTT ACTGCGATTG TCGGTCCTAC GCAATCAAGC 374580
AAAACACCTG CGAGCGGACT TACAATCAGG CCCGGGACAA TTGCTGCAAA TGTAAGCCAC 374640
CCAGCCAGAA CTGGTGACGA AAAACGCGCC AGCGCGAACA GCACCAATGT GAGGACGAAC 374700
ATGCGTCCGG CCAGGCGGGA CAAGGTTGCA GCAAGAAGCA AGGAGAATAG GCCAGGTATC 374760
TTCGAAAGGC TTCTGTATGA AATTTGTCGT TCCATGGTGA CCTGCACACG GGCGCTATGT 374820
TGGTTGCAGT TTACGAGACA TCCGGACGTG TTACTGGGGG GCCGGGTCCT TTCGAGCACA 374880
CGACAAGTCC AAGAGCAGCG TATCCGTAGC CGCCGACACC TTCCCGCATC AGACAATCGG 374940
CACTGCTTTT GGCCGTGTCG CCAGTCTAAC AAGTCGAGTG TCTGATTCTG TTTATAGACT 375000
CGCTCCGACG GCGTGCCGCA TGATTAAGGC TGAAAATGAA AACCTCCTCC AGCGATCCAG 375060
GCGGAGCACC CGCTCGCCTC CTTCCTAAGA TATAGGCATT GATGGAAATC GAAGAATAGC 375120
CCAACACGAT ACTACCAACG TAAATAGGTG CACGAGATGG CGCTCTATGC TGAGACTCTC 375180
CACGAAGCTC AGTGCGATTA TGCCTTTGTT CACGTTAGTG GCGGTGCCGA CCACATGCTT 375240
ATCTTCTCGG TCAGCATGAT CAAAGTTTGC ACGTGATCTG GTAATGTTAG CCCCCGCAAT 375300
GCGAGTTGAA GAATGCCCAA CGTCCAGAGT TTAGCGATTA TTGTCACTGA CCCGCGACTG 375360
ATAATCTCGA CTTGGAAAGA TCGCTGTCCA GCGCCGCTGA GCTCGGTCGG CGGCATGCCA 375420
ATTCTTGCCC GCTTGGTCTC TCAATTGGCA CAGGCGGGGG TACAAAAAAC GATCGTGTTC 375480
GCATTAGATG GTATCGGCGA CGTTAAACGT ATTCTCAACA ACCGCATGAA GCGCATGGAG 375540
CTCGTGATAC GTATCATGCC GACCCCGGGC TCTGGTCGCC TTATAGATCT AGCAACTATT 375600
ACGGAAATCG CTGAGTTCCA CGGTCAAGTT TTAGTGTTCA CTGAGAACGC TGTCATCGAT 375660
CCCTCACTGA TTCAACGTTT ATTGCGATCA TCTGACCGCA ATATCACGGT AGTAAGCAGA 375720
TCAGAACGCA GACGATGCTT GCGTTTATTG GGCGATATTG GAGGCCGTCT TACTGCCATG 375780
TTGCCCGGTG ACTCTCCCAT TCGAGAAAGT GCTTCTGCAG ACGTTAGCCC CGTCGGGATC 375840
TATAAATTTG ATCCCGCACT TCTCCGAGCG ATTGCTCGTA TCCGCCCCCA TCGGTTTAAT 375900
GACGATCTGG AGTTCTTCGA AACCGCTTTA GGGCTTCAAC GCCATCAAAT TTACTTGATG 375960
TATGCCGACC CTCAACACGT AAGGAGAGTG AATGATGCCA CCGATCTTGA GGCGGCAAAT 376020
TTTGCGTCTA GTTCCAGCGG CGACCAGTTT AGTATACTTG AACGATTGCA AGCGGGGAAC 376080
TGGCGATATC CAGCCAGTGA GCACATCTTG CTCTGTAATC ATCATTTTCC TCCAGCCTCA 376140
GTAGTCGACC GACTGCGCGA ACGTTTGCAG GACCTGCTCT ATCTTCAGCC CTCCGACCAG 376200
CTCGATATTA TAGCAAAGCT CTCGGAAATG ACAGATCTTC CGGCTCGAAA TCTTGCAGTT 376260
GGTAACGGCG TGGGCGAGTT AATTAAAGCC CTCTACGGCT ACCTCGATCC GAGAATCGTG 376320
ATTCCAACCC CAACATCCGC GCAGTACATT GATGCCGTCG AACCAAATAA AGTGAGTCGT 376380
TTCGAGCTTC CGCCTGAGAA CTTTGACTTA GACGTGGAGG CTTTCGCAAA TTTTGCAAAA 376440
AGGCGGCAAG CGTCCGTCGC TGTACTGGTC AACCCAAACA ACCCAACAGG ACGCTTGGTT 376500
CCCGTCCAGG AGATAGAATG GTTGGCGTCA CAACTCGCTA TAGAGAAGTG CCGGCTCGTG 376560
GTCGATGAGA CGTTCATAGA GTTTTCAGTG GCAGGGAAAG GAAATTCGGT AGAGAAGCTT 376620
CTCAGCGTTT TTCCAAAGAT GGTCATTCTT AAAAGCCTAG GTGCGATAAT GGGCCTGGGA 376680
GGTGCCCAGA TCGGCTATCT TGCTTCGAAA GACGAACAAC TCACCCACGG AGTTCGCCGG 376740
CGGCTTCCGC TTGGGAACAT AAATGGCATC GCAGAATATC TCCTTTGGAT TTTGCCAGAA 376800
TTTCGCGAGG AGTGGGAAGC AAGCTTTCGT CGCACTCGAG CAGACGTTGT GTCCTTCTCT 376860
CGAATGTTGG ATACTATCCC GGAGCTTGAG GTCCACCCTT CCCAAGCAAA CTACCTCTTC 376920
TGCAGAACTC CTGAGGCTTG GCCAAGTGCG AAGCATGTTG CCACGATGCT TGCGAAGCGA 376980
TACGGCGTGT TGGTCCAGAA CTGTGAAAAT CAGTGTATGA AGTATGGTGA CCGATACCTG 377040
CGCTTGACTG TGCTACCCTA TGAGGAGAAT CGTTACCTTG TGTCGGCACT CCGGCGGATT 377100
AATGAAGAAC TGGTAGAATG GTCGACACAA AGTAAGCGCG CGGGTACGGC GTATCATTTG 377160
GGGTGCTGAT CTTGAATTTT CTGCATGAGT CATGCGGAGA ATCCTATGAG CGACAGTGTC 377220
AGTCAGCCGC GAACATTCGA GCTTTTGACG GCGGCGCCGG TGCGGGCGCG ACGCAAGCCG 377280
CGGGACCGGC CGGACGAGGA GAAGGACCGC CTGATTGCCG AGACGTTGAC CTGCCCCCTG 377340
GAATGTCCCG GGTTCGAGGT TGGTCTGTTC TCCGAAGATG GAGACAGAGA ACGCAGCGAT 377400

TGGCCATGAC CTGGGCCTTG GAGACCATGG GCACGGCGTC GAAAGCGAAG CTCTCGAGCG 381600
TCTTTCCGGG CGGCAGATGC GCCTCGGCGA GGTGACGTTC GATCCTGCGA TGCGCCCGTT 381660
CAGCCAGTTC ATGCTCGGCG ATGGCCGAAA GGAAACGAGC CGCAGGCCAC CCTTCACGAT 381720
CGGCCTGTTC GGCAAATTGC GGCCACAGCG TTTTGATCGT CGGCAGCCTC AGGTCATTGA 381780
GCATGATGCC GAGGCGGGCT TCGTCGATGG TGTTGTGGAC GTTTTTCATG CGGCCTCTCC 381840
CGCATAAGCA GACCCCATCA GGGCTTCATA GCTATTGAGC GATGCGAGCT GCACATGCAC 381900
GGTCGGCAAC TGATCCGGGT CCGGACCGAA GATGGCTCTC AAGGCCATCA GGTCGGGCAG 381960
TTTGCGGGCG TCGAGCGTTC TGGCAAGCTC CTCGGCCAGT TCACGCTCGC AACCGCGATC 382020
ATGAGCCAGG GCCAGCAATT CGACGGTGAT CTTGCAAGCC TGCCGGTCAG GCAGTTGCTC 382080
GATGAGAGCG TCGAAAGCCC TGCGATATTC CGGCCGGGGG AAGAGCTTGT CGCGATAGAC 382140
CAGGTTGAGA AGCGCCATGG GCTTTTTGCG CAGAGAGTGG ATGACGTGGT GATAGTTGAC 382200
GACCTGGTCA TGCTTGCCGC TCGCGTGGGC GCGACCTCGT GGCAACGTCA GCAGATGCGT 382260
GCCGCCGATG AAGACGTCGA GACGATCGTC AAACAAACGC ACACGCAGCC GATGGCCGAT 382320
CAAACGGGAG GGGACGGTGT AGAAGACTTT GCGCAAGGCG AAGCCGCCGG TGCGCGACAC 382380
GGTGACGACT ACCTCCTCGA AGTCGGTGGT GCGGCGCTCG GGAAGCACCT GCAGATGCGG 382440
GCGCTCGGCA TCAATGCGCT TGCCATGCGC GGCATTGCGG CGGCTGACGA TCTCGTCGAT 382500
GAAGGCGCGG TAGGAGCGCA GATCGTCGAA GTCTCTGGTG CCGCGCATCA GGAGTGCATC 382560
GCGGACTGCG TTCTTGAGAT GGCCGTGGGA GCTTTCGATT GAGCCGTTCT CGTGGGCGAC 382620
GCCCTTGTTG TTGCGCGTCG GCGTCATCCG GTAGTGAGCG CACAGCTCCT CATAGCGGTT 382680
TGTCAGATCG ACCTTGGCAT CGGCATCGAG GTTGCGGAAG GCAGCCGACA GGCTGTCGCT 382740
GCGGTGATAG AGCGGCGAAC CGCCGACCGA CCACAGGGCG TTCTGCAGGC CCTCCGCCAA 382800
GGCGACGAAG CTTTCGCCGC CAAGGATGAC ATGGGCGTGC TCAAAACCCG ACCAAACCAG 382860
CCGGAAGTGA TAGAGCAGAT GGTCGAGCGG TTGGCCGGCG ATCGTCACGC TGAGGCTGCC 382920
CATGTCGGTA AAATCCGACA GCCCTAGTCG GCCGGGCTCG TGCGTCTGGC GGAAGATCAC 382980
CTCCTGTGCT TCACCGTGAA CCGCCCGCCA TGACCGGATG CGCCGCTCAA GTGTGCGGCG 383040
AATGCCTTCG GGCAGTTCCG GATGACGCCG CAGCATCTCG TCGTAAACGG CGACCGCACG 383100
AATGCCGGGA GCGGCCTTGA GGAGCGGAAC GACCTCCGCA TCAAAGATAT GCTCAAGCGG 383160
ATCGGGTCGA CGCCGACCGC GGGGCGGCTT GTTCTGCGAC GGAAGGCGCT GCTCTTTCTC 383220
CATGCGGAAC GCCGTCGCCC GGCTGATCGA CGCCTTCGCG GCGGCGACCT CAACAGAATG 383280
CGTTTGTCGG TACTTCATGA ATAATCTCAT CTGATGATCG GTTACATGGC GACCCGGCAC 383340
AAAGGTGGTT CTCCATTCCA GAAAACCGCC ACCGTAGCGG GCCGACCGCG ATCATGAGAC 383400
GCCTAAAAAT TGCGCCGCGG CGGGGGTGTA ACTCCGGTCG GGCTACGCCC TCCCTTCGTC 383460
ACACCCCCAC CGCCGAGTCT CATCCTGATT GACGCTGAGT CTCACCTTGT TTGTCGCCGC 383520
GCAATCTCGC GTGTGACGTC ACCAATGCGC TTCTCCAATT GGCGCAGATC GGTGAACAGC 383580
TCTGCGAGCA GCTTACGCAT CGCCGACGAA AGGTCGTTTT GATTGGTCCT CGAGAACCAG 383640
TGGCAGGTCG AGCTTGAACA GACCTGCGCC CTGGCGTAGG GCGACCCCGT GTTCGAGGCA 383700
GAAGGCTCGC ATCTGATTGA TCAGACGCGT TCTCGTTCCG ATCATCTGGC CCCGCACCCG 383760
ATGCAAAGCC CGAAGATCGG CTTGTTCCTC GCTCTTGAGT GCAGCAAATC GCATCGTTGG 383820
TCGCGTGGCT GCCTCAGCGA TCGCCTCCGC ATCGATGATA TCGGTTTTGT TCGACTTTAC 383880
GTAGGGCTTC ACAAACTGCG CGGGGATCAA GCGCACCTTG TGTCCGAGCG CCTGTATCTT 383940
CCTGGCAATC CACCGAGATC CTGCGCACGA TTCCATCCCC ACGATCGCCG GTGCTGCGCG 384000
CGCGAAGAAC TGTAACAGCG TGTCGCGTCG GAAACCAACC TTCTGGACTG GCACGCCATC 384060
GCTCCGAGGC CGACGACGTG GAATATACTC TTGCCGATAT CAATTCCGTA GACAGCCGCT 384120
GGGCGCGGCA GATTGCGTTG TGGTATTGCA ACCTCCTTCG GTTTGACAGC CTCAGAATGC 384180
GGCAGGAGGA CAAGGCGGAC CATCCCATTA ATGGTAGGGC CTGCCGCCAA GCGTGAAGCC 384240
GGCGCGCATC TGAAGGCCGT CATGGGTCTG TCGGAGCGGC GGGCCTGCCA GATCATATCC 384300
GCTGACCAGA AAATGGTGCG CTATCGGTCA CGCCCTATAA CTTGAACCGC CCGCACACGG 384360
TCCTCAACGG CTTGACCCCA AGGAGCAGAA GGCTGGCGCG AAGGTGGCCC GACCTCTGCA 384420
GCATGGGATT TCAGAAGCAA CCTTTACAAC TGGAAAGCCA AATACGGCGG CATGGTGCTT 384480
TCCAAGGCGA AGCGGTCTCT TGGCCTTAGC CTTTCGTCTT GGCCCGCTAG GGACGGCGTG 384540
CCCTAGGATG AAGATCGTCC CCTCGCACTT GTGTTCAGCT TTGTCCGTTT GAGACTTCCA 384600
TGTGCGAAAC CCCAGAGACT GAGGGCGGGT ATTCCGTCGG CGTGGCGCGA AAAAACGTAA 384660
GTTTTCTCCC CTTGTTCTCA TTCGTTGGCA CGCCGATTGC ATTGGACTCC GTGTAAGCGC 384720
GTGGGATGAG ACCAACTGTT ACCCTCGATT AGCGACCAAA TTCGAGCAAA TGAGAGTGAC 384780
AGTTACCAGA CAACCAAGGA GTAGCTCATG CGAATAGCCG TCGTGTGTAA CAGTGCTTTT 384840
ACGGGTGTAA TCAATCGGTT CGGTCAGCCT TATCCGCAGC CGCCGCAGCC TTGGCCGCAG 384900
GGCCGGATAG CGGACAGCGT GGTGGCGGCC CTGCAAGAAT GCGGCCACGA GACGCTGCTG 384960
TGTGAAGGCG ACAAAGGACT GCTCACTACG CTTGAGCGGT TCATGCCACC CGATCCGCTA 385020
GCCCGTACTT CGGGAATGGT CTTCAACTTG GCCGAGGGAA TTCAGGGCGA GTACCGTTTC 385080
ACCCATGTTC CGGCTATGCT CGAGATGGCC GGCGTCCCCT ACACCGGATC GAGCCCTCTG 385140
GGGCATGGGC TGACCGACGA TAAGGTCATC AGCAAGACGC TTATGCGCGA TAGCGGCGTG 385200
CCCACGCCGA ACTTTAGTAT TATGCGCCGC GGCACCGAGA GTACCGACGA TCTCCGATTC 385260
CCAGTCGTGG TGAAACCGCG TCACGAGGAC AACAGCTTCG GATTGCAGCT CGTACATGAG 385320
CCGGCTCAAT TGCAGCAAGC AGTGAAAATG ATCGTCACGA AGTATGCGCA GGATGCGCTT 385380
GTGGAAGAAT ACATCGATGG GCGAGAAATC CACGTCGCGC TGTTGGGAAA CCAAGAAGTC 385440
GAGGTGTTGC CTATGGTGGA ATTTGAGCTT GGCGAACACG AAGCCCCTCT TCTAACCTGG 385500
GAAGCGAAGT ACTTGGCGGC CGTGCAGCCG CCAAAGACCT GTCCGGCGAA AATCGAGAGC 385560
AAGCTCGCGA CTTTGCTGCG GGATATTTCT GTTGCGACCT TCCGCGCCTG CCAGTGCCGG 385620
GATTATGCTC GCGTCGACCT CCGGCTCGAC CGTTCCGGCC AGCCCTTTGT TCTTGAGATA 385680

AACTCGATGC CGGGACTCAG TACTCACTCC GCTTATGTTT TAGCCGCGAT GACTGCCGGA 385740
CACAGTTACT CTAGCTTGAT CAATGGCATC CTCGACGTCG CCCATAGGCG GTACTTCGGA 385800
AACGGCATCC TAGGGTGAGG GTGGTTCAGT CATCATAGCC CAAAGATGGC GGCAACTGCG 385860
ATACAGATTT CCGAAAAAGG TTTAGGCGTA TCGGTCGCAG CAGCTGTCAA TCTGATCCAC 385920
GCATTAGCGA GTGTCATACA GGAATGATAG CTGCATGCGG CTCGGTACGG CGGATGCCCC 385980
TTGTCTCGAT GCTCTTGTCC CGATCAAGCC GGAAAGCATG GATGAAGTCG ACGGCTAAGA 386040
GTTCATGAAC GTCGCCGCGG TCGCGGTCGC GCACAAAACG ACACGCGCCG TAGGAACAGT 386100
GCGGGTTTGC GAGGAGAGGT GAATGTGACG ACAAACCGGT ATGGTGCGGT TTGGGAAACA 386160
CCGATGAGAC GATGCAAAAG AGCTCGTCCG TTTGACTGGC CTCCGATCGC GAATACCATC 386220
AAGGCCTGCG GTCAAGCAGC TCGCACAAAC ATGCCGGAAA CTGACCGCAA GCACCGTGTC 386280
TGCCGTGATG TTCAAATTAC ACCTGTAAAC ATGGAGAGAT TCACACTCGT CCCTATGCCC 386340
GATGGTCGTG CGGTGGCTTG CCACGTACGG GCCCCGGCTG CCGCGGCTCA TATTTCGCCG 386400
AGCGCTCTCG CGTCTCTTGC ACTGCCAAAA ACCGTTCAGC CCGGCGGAAC AATTCTCGCC 386460
CGTCGTTGAA TCAAAAAGCT AGCCCAGAAC ACCAAGGAAG AAATATGGAG AAGTGCTCAC 386520
ACGAGAGCGG TCGTCATTCG GCGGAGAATG ACGGCAAATA CGATATCACT GGTTCGACGG 386580
CGACGAACGT CGTGGACGGT TTCACAATGG TGGCAGTTGG CGACGTTATC GTGTCGCGAA 386640
CCCTGGCGAA CGGGCACCAT CCGGGCTTTA GTGAGATCGT CGAGCTCCTG CGAGCCGCAG 386700
ACGTAACTTT CGGCAACATG GAGACGCTGA TCTTCGACAT CAGATCATTC AATGGAACAC 386760
CGCAGGCTGA GTACGGGGGG GCGTATCACG TCAGCCTTCC CGAGATTGGA CCTGATCTGA 386820
AGGCGATGGG GTTCAATATT ATGGGCCGCG CAAACAACCA TTCCTTGGAC TGGGGCGTCG 386880
AAGGCATGCG TGAGACGAGC CGGATCCTCG ATGAGAGCGG CATCATCCAC GCCGGTGTGG 386940
GTGAAAGTCG CGCACAAGCC AGCGCTGCAC GTCTTTTAGA AACGGCCCGC GGTCGCGTCG 387000
CTTTGCTCTC ATGCGCCACC TCATTCACGC CGATGTCGCG TGCATGCGAT CCAGCGGGCG 387060
AGGCACCAGC CAGGCCTGGA GTCAATGCGC TCCGTCTAGA GAGAAGCGTC GTCGTCGAGC 387120
CCGACATGCT TGAGAGCCTA AGAAAGATAC GTGATGCCTT GCCCAATCCT GGGCCTAAAC 387180
ACGATGATCG CGAAATGTTG GTGCTCGCAG GAACGACCTA TAGAACCGGC AAAGACGTCG 387240
GCTACACCTA TGCGGCTAAT ACGCGAGATC TGGCCGATAT TTTAAGAAAC GTTCGGCGCG 387300
GCAAGCAATA TTCCGATTTC TGCATTTTTA CAAACCACGC ACACGAGCCG GGAAATTGGA 387360
GCGAAGAGCC GGCCGATTTT GAGCAGGCGC TTGCTCGCAA GCTAATCGAT GCGGGAGCGG 387420
ACGCGTACGT TGGACACGGA CCGCACCGAC TGCGTGGCAT CGAAATCTAC AAACGCCGGC 387480
CCATCTTCTA TAGTCTGGGG AACTTCTTTT ATGATGACCT CCGGACACCC GTTGGGGCGG 387540
ACATGTACGA TGTGTACGAC AAGGACCCAC AGGTGGACAC CGATGCCGAA GTAACCGCAG 387600
CGGAAGAAAC AATGGGTTAC CCTACGGCTG CAGGGTTCAT AGGCGCGCTC GCCGAACCGG 387660
TGTATTACGA GAGCGTCGTC GCAGTTAGCC GCTTTGAGGA GAACCAACTG GCCGAACTGC 387720
GGCTTTATCC AATCGAGCTC GGTTATTCCA AAAGGCTTGC GAACAGGGGC GTCCCTAGTC 387780
TTGCTCCACG CCCTCAGGCA ATCTCGATTC TTGAACGCCT GCAGAGGTTG TCAGAGCCGT 387840
TTGGAACGAG AATCACCATC GAGGACCGTG TCGGTCTTAT TAGACTGTAA CCACGCATCG 387900
TCACGAACCT CCCAAGCTGC GATTTCTCGC CCGTATGGGG TCGCTCTTAT CGGCTGAGCA 387960
GGGAACTGAT AACAGATGGC TCAAGGAAAG CGATGTTCCA AGCCGGTTTC AGTTGACTTG 388020
TAGAACTGAT CCCTGCTTAT CCTCGTTCGT GTAGAAGGAA TGAGGGAGCG GTTTGGTGTC 388080
GCTGTCTGAG AGCGAGCTAC ATGCCAAGCG TGCAGTTGCT GATGCGGTAC CGTAATCTCA 388140
TCCGTAAACG CCATGATCTG CCGGCTCAGA ATATCCAGCG AAGTGCGTGC ATAAGCCCAG 388200
GCCGTTGGCA TCGGACAGCG GTTGCTGCGA CTTTGCCAGA TTAGATAGCG CCGGGATTGG 388260
CCACAAGACC AGATTCTGCC AATGCGCCCG CAGCGCATGT GCGACCATGG TGCGCTGGCG 388320
AACGAGCAAC GCTCGCGCGC GGTGGATCAC GAGAATGCCT TGCTGCTCCA CCGTCGTCAC 388380
AGGCACGAAC CGCATCGTCG GGAGCCGCAC AGCTTCGCAG ATTGCTTCGG CGTCCAGCTC 388440
GCTCTTGCCA CGCTTGACAC AGGCATCACA TAGGAGAGCG GGACGCTCGT GCCCATCGCC 388500
GCGAGCGTGC GAGGCCAATG ATGCGCTGTT CCGCGCGCTT CCATCCCGAT CAGGCATGAT 388560
GGTAGCTTCG AGAAGAAGGG CAGCATTTGC GTGTCCCTCC GGCGGGGACC GACTTGCGAA 388620
GTTCCGCGTC TCCCGTGAGT CTGCGCTCTT AAGAGTGGAC TTAAGAGCGC ACTGAGCAAT 388680
TCATGGGACA TGCAGTTTGC TGACGGGGCC AGAGCGGCGT CGGCGATGGT CGTTGGAAGA 388740
TCGCGCGCAG ATACTGGCGG AGGCCTTCGC GCCGGGCGCG GTGGTTTCCG AAGTTGCGCG 388800
CCGCTTCGAG GTTTCGACTG GCCTGATCTA CACATGGCGG CGGCAAGCTT TGGTCCAGCA 388860
GGCCGAGCCC GCCCTTTGTG CCAGCCAAGC TTGTTGACTC TGGCAGCAGT GATGCGGTCG 388920
AACTGGCCAT GGCAGTGGAG TTCCCGAACG GCGTGAAGGT GAGAATTGGC TCAGCGGCTC 388980
CATGCGACCT GGCAGCCGCG ATCATGCGAG CGCTCAAATG ATCAGGCCGC CTCGCGCGGT 389040
GGCGCCGACT TCATCCTTGC GCTAAATGCA GGTCGTTTGC GCAGCATGGG TGCCCCGTCG 389100
ATCTTCTCCC TGTTGGCTCT CCGCAACAGC AACGATTTCG TCCTCGATTT CGCCCGATCG 389160
GAGATCCTAC CCTTCGCCAA GGTCCCGATC TTCTTCGGCG CCAGTGCCTT CGACCCCCGC 389220
TCCTCGGTTG AAGCTGAACT GGGGAGGATC GCGGAAGCCG GCTTCGGTGC TGTCGTCAAC 389280
TTCCCGACTG CGATCTTCCT CAACGGTCGC TTTAGGGCCG ATATAGAGTG CGCCGGACTG 389340
GGTTTTCAGC GTGAGCTCGA AATGCTCCGC GTTGCCCAAG AGCGCGGCAT GGCGACACTC 389400
GCGTATGTCT GCAGCATGAC TGAAGCAGAG CAGGCGGCAG AGGCTGGCGT GGACATCATC 389460
AATCTTAATC TCGGCTGGAA CGTCGGCGGA TCCGTCGGAA GCCGCAGCGG GCTTGGTCTG 389520
GCACAGGCGG CCGAGTACGC CAAGATCGTG TTCCGGCAGA TAAGGGGAAT TTCCCAGGAG 389580
ATACTGTGCC TGCTCGGGGG CGGCCCGATC GTTAGCCCCG ATCAGATGTA CGAGGCCTCG 389640
ATCGTCGCCA AGGCAGACGG TTATATCGGC GGCTCGACCA TCGACCGGGT GCCGCTCGAA 389700
GCCTCGATGG AACGGATCAC ATCAGCCTTT AAATCTGTTG GCACCTTGCA AAAGCGCATC 389760
GATGAGCTGG AGCGAAAGCT CGAGCACGTG CAGCGGGAGT ATTTGACTGT CGGGCGCTCG 389820

CCGTTGATCC ACCAGATCAA GCAGCGGATT GAAAAGCTCG CAGCCTCGCC CCTGTCGGTC 389880
ATGATTACCG GAACCAGGAA CCGGAAAGAA GCTGCTCACA CGCGCCATTC ATGAAGCTGC 389940
CAGACGTCCT GGCAGCAAAT TAATCAACGC GCCTACGGCG GATGTTATTG GCGAGTCGCT 390000
CTTCGGTTCG GCCCCGGCGC AGGGTGGACG CGAGGTGCTC GGGCTGCTCC AATTCCATCC 390060
CAAGGCGACG CTGGTGATTG AGGATTTCGA AACTTTGTGC GAAGACGCTC AGAAGCGGCT 390120
GACCGAGGTC ATCGAGACTG GCGTTTACCG GCGGCTTGGT GATAACGACA GAGGTTTTCG 390180
AGGGACGTCT GATCCTGACA TCGATGCAGC CGCTGTCACA ACTTAGCTCA AGCGGGAAGG 390240
TAATCTCCTC GCTCGCTTCG CGCCTGGTTC CCGGGCATGT CTTCCTGCCC AAGCTACGCG 390300
ACCGCCTGGA AGACCTGCCT CTGCTTGCGG AGCACTTCCT GCAGGCGCTC AGAAAGGATC 390360
GGCGCAGCCT CAAGCTGTCG GTCGATTACT CGGCCTACCG CCTTCTGATG GCCTATTCCT 390420
GGCCAGAAAA CGTCCGCGAA CTGCGCTCCG TGCTCGAGAC AGCCGCCATC CGCTGCGAGG 390480
GCGATTGGAT CAAGGCGGAG AATTTGCCGC CCTTGGACCA CGCGGATATG CGTGAATCGC 390540
ACCCGGCAAA CGAGCGCGAA TGGATCCTGG ACGCGCTCCA GCGTCATCGC TTCCGCCGCG 390600
GTGAAGCCGC TCGCCATCTC GGGATTTCGC GCAAGACGCT GTACAACAAA ATGCGCGCCT 390660
ATGGTCTGCC ATTGCAGCCA CGGGACAGCC CTTAAGCTAC GGCCAAGCGA CCACGCCGAC 390720
TGTCTATTGC GGATAAGGCG GCAGCAAGCC ATGACACCGC ATCTCAGATG ATGTGGTCTG 390780
CCAACAGCAG ATGCCAACAA TGACGCGGCG TGCTCCGAAT CCCCCGGAAA AGGACGTTCC 390840
CCCGCTTCCG AAAACGGCGA CTGGTTTAGC AAAAAATGGC GTAAGGAAGC ATAATTTCTG 390900
CGAGCGCTCC CTCGATAACG CACGATCACG CCTTTAATTC GAATCTAGTC TCACACACCG 390960
ATTGCTTCGG ATGGGGGGAC GAATGCCGCT GTTGAATGTA TCGCCATTGA AGTGGCCGAT 391020
AAGTCTAAAG ATTCCGGCTA TGACCGTCTG CGTAGCGGTA CTGTCCTGCA CGACGGTCGC 391080
GACATTCGCG GGAATTACTT CAGTCGCAAC AACCAGGAGC TTGATCGAGA AGCACCTTAA 391140
TTATGTCGCT ATCTCGATGC GAGACGCGCT CCTCACAAAA CTCCAGGCGA CCAAGTTCGA 391200
AGTCGAAGGG CTCTCCGCCA ATCCTGGGCT GCTTCAACTC TTTAATAATA CGAGTGTTGG 391260
TTTCGCGGCG GTGTCGCCAA CAGACCTCAC GGCAGCGTCG GTAGATAAAG TCGAGAGTGG 391320
GAAGTTTAGT CTCGCGGCAA CACCAACCAC GAAATTCTAT GTCGACAATT ATCAAAAGCT 391380
GGATGCTTGG CTCAAGCCGT TGGCCGCCAA GCAGTCATAT TCGGGGATCC TTTTAGCAAA 391440
TGCTGAGGGC GCAATCATCT ATAGCACCGG AACTAATCCG GTCGGCCCCG TAGATGCCAA 391500
CGGATCGATC AACTTGGCCA TCGCAAGCTC AGCTAACAGT CAGGAGGCGG TGATGACTGA 391560
TTTCACGCCT CCAACGTTGT CCGCACCGGG GCAGGCTCTA TATGCCGTTG CGATCGTTCA 391620
CCCGTTCATA CCTGACAAAC GAAATGGTAC TCTACTGATT TCCATGAGTA CCGAAATGCT 391680
AAATGCAGTC ATGGGTCAAG CAAAGGGTTT CGGCCCACAC AGCGAAGCGC TCATTGCCGG 391740
GAGCGACGGC AAACCTCGCT CCTTGTCGTC CATATCCAGA GACGAAGACG CTGAAGCGGT 391800
CGTCAACAGT GACCTGCTCT CCCAAGGTAT AAAGACGTCC AATTTCGGCG CTCAAGAGGT 391860
TCTTTCTGCA TCACAACAAC TGAAATGGCA GGATCACCAG TGGTCGATCA TTGCCCTAGA 391920
GCCGGAGGCA GATGTTGTCT CGGCGTCAAC TGCAATGTTG ATCAAGATTA TCGGCATTAC 391980
GGCTGCCACT GCGATTCTGG CGCTGGCAAT GGCCATCCTC GCCTCTCGCT CAATATCAGG 392040
GCCACTGGCG GGGCTGGTGT CTATTATGAA AAGACTCGCA AACGGTGACA TAAACGTGCG 392100
TGTCGCTGGT GTCGATCGCC GCGACGAGAT GGGCGAAATG TCTAGAGCAG TTCTCGTCTT 392160
TCGAGATAAC GCGATAGCTA GGGTTGCTGC GGAGGACGAT GCAAGGAGCG CTGAAGAGGC 392220
GATGGAGCAC GACCGCAGGA TGATGGAGAT GGAGCGGTCA GAGCGGCTCG ACGAGCAAGC 392280
CCGAGTTATG GCTCAAATTG GCGGAGGCTT AGCAGCACTC TCCGATGGAG TGTTTAGTAG 392340
ACCCATCACC GTGGATTTCC CAGAAGAGTA TCGTCCGCTC AAGAGCGATT TCAACCGCGC 392400
ACTAGGGCAA CTCAGAGAGA CAATACGAAC TGTCGCGGCT CAAGCCGCGT CGATGTCGTC 392460
TATCGTCTCC GAAATAAGCT GCGCAACTGA TGTCCTGGCG AAACGGACGG AACATCAGGC 392520
TATCGTGCTC GATGGTGCCG TGAATACGAT GGATGCAATA TCAAACGATG TAAGCGTCAC 392580
AGCGAACGCG GCAAATAATG CTGATGCGCT TGTCCGCGAC GCCCACTCCG CTGCAGCCGC 392640
GTCGGACGAG ATTGTTTCGA GCGCAATAGC GGGCATGTTG GAGATCGAAG AGTCTTCTGC 392700
AAAGATTGCA ACGATCGTCT CGGTCATCGA GGAGATTGCA CATCAAACGA ACCTTTTAGC 392760
ACTCAACGCT GGTGTTGAAG CATCCAGAGC GGGGGAAGCC GGGAAAGGAT TCGCGGTCGT 392820
TGCTTCGGAG GTCCGGGCGC TCGCACAGCG ATCCTCTGAC GCCGCCAAGG AAATTAAGGA 392880
CTTGATCAAT GTTTCTACCC AACGTGTGGA GCGCGGCAAA GAGCTTGTCG ATAGCGCGAG 392940
TGAATTGCTC AAGCATATAG CCGCGCGTGT CGATCTGATC AGGACCACGG TTTCCAACAT 393000
TGCTGCAACG GCAACGAGCC AAGCAAAGCA TCTGTCTGAA TTTCAGACTA CGATCGCTGA 393060
GATTGATCAA TCTACTCAGC AGACAGCGGC GATGGCCGAG GAATCGGACG CCGCTTGTCG 393120
ATCCTTGAAC GCCGAGGCGC AGCATCTGTT GGAACTAATT CAACAATTCG AACTGGGAGG 393180
GGGTTCTTCC ACCCGACAGC CTCAAAGCCC GCCCACACAG AGATACTTCA TGTCGAGGTA 393240
GTCTTCCAGC CCATGCCTGG ACCCCTCCCG GCCAATACCC GATTGCTTGA CGCCCCCGAA 393300
GGGCGCGGCC TCGGATGACA TCCGGCCGGT GTTGATCCCG ACCATGCCAT ATTCCAGCGC 393360
TTCTGCCACC CGCCAAACCC GCTTCAGGTT GGAGGCGTAG AAGTAAGCTG CCAGACCATA 393420
GATCGTGTCA TTCGCCTCGC GCACAACGTG ATCTGCATCA TCGAACCGTA ACAGAGGTGC 393480
AAGCGGTCCG AAGGTCTCTT CCTCAGCCAG ACGCATCGTT TTAGAAACAT CCGTGACGAC 393540
CGTCGGTTCG AAGAACGTGC CGGATGATCC AGTTCGACGG CCGCCACTGC GAACCCTTGC 393600
GCCCTTCTGG ACGGCATCGC TGATGTGGAG TTCAATCTTT TTGAGAGCCT CTTGATTGAT 393660
CAATGGACCG ATCGCCACAT TCGGATCAAA GCCGTCACCG ACCTTCAGAG TGCGCACCCG 393720
CTCGGTGAAC TTTTCGGCGA ACTCGGCATA GACGCCGGAT TGAACATAAA TCCGGTTGGC 393780
AGACACGCAG GTCTGTCCCG CATTGCGGAA TTTTGCCTGA ATCGCTCCGT CGACCGCGGC 393840
GTCGATGTCG GCATCGTCGA AGACGATAAA GGGGGCGTTG CCACCGAGTT CGAAGCTGAT 393900
CCTCTTGATC TGGTCGGAGC ACTGACGCAT CAGCAACCGG CCGACCTCGG TCGAGCCTGT 393960

GAAGCTGATC TTCCGGACCT TCGGATTGGT GCAGAGTTCG CGGCCGATCG CGTCCCCCTC 394020
CGACGCATAG ACCAGGTTCA GGACGCCGTC CGGAAAACCG GCCAGTTTTG CCAATGCAAA 394080
CATCGCGCCT GCGACCAGAG GCGTCTGCTC GGCGGGCTTG AGCACGACGG TACAGCCTGC 394140
CGCAAGCGCC GGCGAAATCT TGCGCGCCAC CATCGAGGCG GGAAAGTTCC AGGGTGTGAT 394200
GGCGCCGACG ACGCCGATCG GCTGTTTGAT CACCAGCATG CGCCGGTCGG TCGATGGCGC 394260
CGAGATGGTT TCGCCATAGA TGCGGTTCGC CTCTTCCGCA TACCATTGAA GATAGGCTGC 394320
CGCATGCTGC ACTTCGGATT TCGCCTCGCC AAGCGGCTTG CCCATTTCGG CGGTCAGGAT 394380
GGCTGCGAGG TCATCGCTGT GTTCAAGAAT GAACCGGTGC CATTTCCAGA GGATATCCGA 394440
TCGCGCCCGC GCGGTCAGTC CCGACCACGG TTCCTGTGCG GCATCAGCCC TTTCGATGGC 394500
CGCGTGGGCA TCGGCAGCAC CCATATCCGG CACTTCGGCC AGCAATTCGC CGGTGGATGG 394560
GTTAAACACG GAGAACCGTT GCCGCTGGCC AGCGACCGAA GGGTGCGGTT CATTGGCAAG 394620
GTTGCGAAAA AGCTCGGGAC ATTTCAGGTG CCGCGTCAAT GCCGATGTCA GTGTCATTCT 394680
TCGTTCCTCC CACGCGCGTT CGATTGCGCC TCTTCCCGCC TGTCGCGAAA GGCAAAGCCG 394740
GAGCAATGAT CGCCATTGCG CCGGCTCGTC CCTTGGACTG CTCTCTCCGT TAAGTCAGAG 394800
AGATGACGGC TTCAATCTCG ACCTTGGCAC CTTTTGGCAG CGCCTTGACT TCATAGCAGG 394860
CGCGCGCCGG GTAGGGGGTG GAGAAGAAGC CCGTGTAAAC GCGATTGATG TCCGCGAAGT 394920
CGCCAAGGTC GGTGAGCAGC ACTGTCGTCT TGACCGTCTT CGACAGATCC GTGCCGGCGG 394980
CCCTGGCGAT CGCCTGAAGG TTCTTGAGGC ATTGTTCGGC CTGTTCGACA GCATTGGCCG 395040
AATTGAACTC CCCGGTGGCC GGATCGATTG GCAACTGCCC GGAGACGAAA AGCAGATCGC 395100
CAACCTTGAT GGCCTGGGAG TAGGGGCCGA CCGCGCCGGG GGCATCGTTT GTAGAAATGG 395160
GTTCGATCAA AGTTTTAGTC CTCCATTGGT ATCCAGTGAG TGCTCAAGCG TAGCGGCTGA 395220
CGGCGAGGTC GGTGGCGTCG ATCTCGGGCT TCCGGCCAGA TACCAGGTCC GCAATGACGC 395280
GGGCAGAGCC GGAGCTCATC GTCCAGCCAA GCGTGCCGTG CCCGGTGTTA AGGAAGAGAC 395340
CGGCGATCTT CGTTGGGCCG ATGACGGGCG TGCCGTCCGG CGTCATTGGG CGCAGACCCG 395400
ACCAATAGGA TGCTTCTTTT GCATCGCCGC CGGGAAAGAG GTCCATGACG GAATGCTCGA 395460
GTGTGCGTCG CCGGGCGGGG CCGAGATCAT TGGTGTATCC GGAAATTTCT GCCATGCCGC 395520
CGACGCGGAT GCGGTCGCCA AGGCGCGTGA TGGCGATCTT GTACGTCTCG TCCATGATGG 395580
TCGATTCCGG CGCCCGCGAG GCATCGGTGA TCGGGATCGT CAGCGAATAG CCTTTGACCG 395640
GGTAGACCGG CAGCTTGATG CCGTGACGCT TCAGCAGTAG CGGCGAATAG CTGCCGAGTG 395700
CGACGACAAC AGCATCGGCC GCGAGCCTTT CCCAGTTGGT TACGACACCC CTGACCTTGT 395760
CGCCCTCGAC ATCCAGTTTC CTGACTTCCG TGCCCCAGGA GAAGCGGACG CCCAATTGCT 395820
CCGCCTTCTT CGCAAGCGCG TTGGTGAACT TGAAGCAGTC GCCGGTCTCG TCCTTCGGCG 395880
TCAGCAGCCC ACCGACGATC TTGTCGCGCG CGGGTCAGCA ACGCGAAGCT CGCCGTGGAA 395940
GTTGGGCTTT CGCCGTCAGC CTGCCTGAGG CGTATCAAGC TTATGGAGCA GGCAGGTGTC 396000
ATCAGGGGCT ATACGGCGCT TGTCGATCCG ACGCAGTCGG AATCGACCAT AGCCGTCATC 396060
ATCAACATTA CGCTGGAGCG GCAGACGGAG GAGTACCTCG ACAAGTTTGA AGCGGCCGTG 396120
CGCAAGCACC CCGAAATTAG GGAGTGCTAT CTAATGACCG GCGGATCAGA CTACATGCTG 396180
AGGGTGGACG TCGAGAATGC CGGGGCATTC GAGCGCATAC ACAAAGAGGT CCTGTCGACG 396240
TTGCCTGGGG TGCTGCGTAT CCATTCGAGC TTCTCGATTA GAAATGTGTT AGCGGGCCGT 396300
CTGAAAGCAA AAAGATGAAA CTTGCCCATT TGAGAGATTG CGCGGCAGTG AGGTAGGCTG 396360
TGTACCTCAT ATGACCGCTG CCCCTCAAGA TCCGCAGGGG CTACAGGCCA CAGAAGATGT 396420
GAGCTCAGCA ATCGAAGGCA CTAGGTCGTG GTGACGATTT AACGGATTGA GATTCCCAAG 396480
AAGGGGCAAA TCAGATTCAA CACTGACTTT TGGAGGTCAG TGATGGATTG CGATGGGCTT 396540
CGGGACGATC AATGGGAACG TATCAGAGGT TTTGTGCCCG GGGGCACGAA GGGCAAGCGT 396600
GGCCCGCGCA CGAACAACCG GCTGTTTCTG GATGCGCTGC TGTGGATGGC CCGTTCGGGC 396660
GGCCGCTGGC GAGACCTGCC AGAACGACTG GGTGACTACC GCGCCGTAAA ACGACGCTAT 396720
TACCGCTGGA TCGAGATGGG CGTGCTCGAC GAGATGCTTG CCGTGCTTGC CCGCGAAGCT 396780
GATTTGGAAT GGTTGATGAT CGATTCGACT ATCGTGCGCG CCCATCAGCA TGCGGCCGGG 396840
GCGCGCAGGG CTAAAGGGGG GCGGATGCCC AGGGCTTGGG TCGGTCTCGA GGCGGGCTGA 396900
GCACCAAAAT CCATGCTGCC GGCGATGCGC TCGGCCTGCC GCTTCGCCTC ATCGGCACGG 396960
CGGGACAGCG CAACGACATC ACAGCCGCCC ATGATCTCGT CGACGGTCTC CACGCCGATG 397020
CCCTGCTGGC CGACAAGGGC TACGATGCCG GACATCTCAT TGAGAAACTG GAGAAAACAG 397080
GAACTGAAAT CGTCATCCCG CCCAAACGGG ATCGCAAAAT GCAAAGGGCC TACGACGCCG 397140
CACTCTATAA AGAACGCAAC CGCATCGAGC GCTTCTTCAA CAAGCTCAAA CAGTTCCGCC 397200
GCGTCGCGAC ACGCTATGAC AAGTTGCTCG CCAACTTCAT GGGCTTTGTC AAACTCGCCG 397260
CTATCGCCAT ATGGCTCAAA TAGTTAAATC GTCACTACGA CCTAGCGGGC CTATGCGCTA 397320
TCTTCCCGTC GCGTCACGAC GGGAAGAATG GCTTGAAACG CGATTCTTAA TTGACGGCTG 397380
CATGGCTGCT TGCCAATTCG CCAGCCGGGT CGACAACAGC GGACGGCCCC CTCCGCGGCG 397440
GTTGACCCGG AAGGTTGTCG CGAGGCGGCA TCGAGACGAT CGCCGCGCCC ACATCAATGG 397500
CGTCGATCTC CCGACGCCTT CTTTATTTCT TTGCCGCCGA GCAATAGCTT GAGCCTCGCT 397560
TTCGCGTCGG TGTGTGCCAA GCGTTGGAAG CTTTGGCCCG AGGGGTTTCA ACGTCCGCTT 397620
TGTCCCAGCC GGTGGAGAAT CCATGCCATG CGAAACGCGG CTTCTTCTGC TACTCCTGGT 397680
CCATGTGACA CACCCATATG GCCGCCGATC ATCCGAGTGC GGAAGATTAG GTCCGGATCA 397740
CGATCAACGG CCTTTGATCG GCGCTGCGCA ACATAGCGAG CGGGCTGATA GTAGAGCACC 397800
TGACTGTCGT GGAGGGCCGC GTCGATGTAT GTCGGCGGAT ACCGTCGATC AGGCGTCAGA 397860
TTATAGTAAG GGTCATAGCT TCGTAGATAT TGATAGTCGT TAGCAAGATG CGGATCTCCA 397920
TACTCTGCAG TTTCTCTTAG GGCGTATGGG AGCGTGAAGT CCAACTCCGT GTCGATGATA 397980
TCGGCAAGTG GAACCTCGGC GAGCACAGCT CGAAAAAGGT CAGGGCGCAG GACAGCGGCG 398040
GCCAGAACCG TACCGCCGCC AGCGCTCCTG CCCTCGATGA CAATGCCGTC ACGGCTGGCG 398100

AACCGATGCT CGACGAGGCA CTCGGCCGCA GCGATCAAGT CCGTGTGCGT CAGTCGCTTT 398160
TGATCGCGGG TCGCCGCTTC GTGCCACGCC CGACCGAGTT CGCCGCCTCC TCGCACGTGC 398220
ACGATGCCAA AGGCTACCCC GCGATCGAGT AGGCTCAAGC GAGCGGTCAT TGATGAAGGC 398280
CAGCCAAAGA AAGCCGGTAA GCTTTGTGCT CCGTAGCAGC CATACACGTT CAGCAAAACC 398340
GGACCGTCTT CACCTCTATC TCGCCGCGCT ACTATCGAGA TTGGAACCTC CACTCCGTCC 398400
TCTGCCTTCG CCATTACGAC CCTAGCCTCG TACAGTTCTG GTTCGAAACC TGATACCAGG 398460
GTTCGGTAGA GGACTTTGGA CTTATCCGTT AGGAGGTCGT GTTGGATGAA TATGTCAGGC 398520
GTCACAAAAG AACAGATTTT GTAGGTCAGC GCAGAGACCC GGTAAGGATG TCGTGCGCAG 398580
GAGTAGCTGC CGCCTGCCGA TAGACCAACG GTCACGGTGC AGCTATGTTC GACGGGGACG 398640
ATACTGGGCC CGACTCGTCC ATTTCGGTGG TGCGCGACTA AGCGAGGTTG AATCCCTTCG 398700
CGCTCTAACA CAATCACATG CTCTTCAAGG ACATGAATCT CCTCGAGCGT TATGCCGGCC 398760
CGATGTGGAA CCACTTCCTG CCAGCGCGAC GGTGAGGTGT CATCAATCGC CGTGCGCACG 398820
AGGCGCAAAT TCGGGCCAGT GTCGTTAACT CGAAAAAGAA ACTCGTTGCC CCAGTGTTCC 398880
GCGTAGATCT CGTGCCCCAG TTCACGTGCA AGGATCCGGC GCCACATGTC TGTCGGTCGT 398940
TCGGCAGGAA GACACCATAC CTCCGCGGCG GCGCGCTGGA TGCGAGAAGA CATGTCCGAG 399000
GTGATGATCA CGTCTATGAA CAGGTAAGCG CCGCTGCCCG ACCGCCGAAC CACCAGGGCC 399060
AGTCGCTCAT TCACTTCTTC GAACACCACC TCGGAACGTC CGGTCTCGAC GTCCAATCGA 399120
ACAACACGGT CGTGTTGGCG CCGATCGGCG CGCTCGCGCG TAAAGAAGAG CGTGCGATTG 399180
TCCGCTGCCC AAACTAGTCG CCCAGCGCGG GAGGGGTCGC GCCAGATCTC GCGGCCGTTG 399240
GTCATGTCCC TCACTCTCAA TTCGTAGCGC TCGTTACCGA TGAGGTCGAA GCTGAATGCT 399300
ATGTAGCGAC CGTCATCGCT CGGTTCGAGC GCGCCGAGAG AATAGAACAC TTCTGCCCCA 399360
GGGAGCGTGT TCGGATCGAA GACAAGCTCT TCTGGACCTC CAGTTACCGG CCGGCGCCAC 399420
CATGCTGAAT GTAGCAAGCC GCTTTGACTT TTCTGGAAAT AGAAAAAAAA GCCGACTTGG 399480
AACGGTGGTG GTGCGCCATC GCAAGAATCT CGCTTCTCGA TTTCGGCGAT TAGATCGGCT 399540
TTGAGTTCTG CTACATAGGA CGTCACTTCA TCCGCGTAAT GGTTCTCGGC TTCTAAATAT 399600
GCGAGGACAT CAGGATCTTC CCTGTCGCGC AGCCAGCCGT AGGAGTCGAT CGTGACGTCG 399660
TTATGAAGCA CTCGAATCCG GCGTTCGCTT CGGGGAAGCG GGGGCTGCAG GGACTTATTT 399720
TCCATCAGAA TGCTCCTGGA ATTGGATTGC AGACATAGCG TTTTGCAGGG CTGAGCCAAA 399780
GCCGACGGTT GCCGAGCAAC AACGGGAAGA ACTCTGAGGC GTCGGAAGGC GATGGGTCCA 399840
GGAAGTTGAG CCAACGCTCC GTGTTCGCGA ACCGGTCAAT TCCCCTGTGT GGCAAGCCGA 399900
CCCCGCGGAG GCGCACCTCA AGCTCCCCAC CAATGGTTGC AGAACAGCTT GCGACAGGCA 399960
CGGCTCACTT CTCCGGGTTT ATACGTCAGT CCCAGCTAGA AAGGCTTCTG CGGCCAAGAC 400020
GAAGTAAGAA GCTTAAGCCC AGGGCCACAC AATAATTCGG AAACGAAGGC GCTTTTGGGC 400080
GCAATGTAGG TGAGCACGAC GGAATCCTGC AAAGTTTCGC CCGCTCGCGC TTTAATGCGG 400140
CGAGGCGCGG CTGTCTCAAC GCCAGATGCG GAATATCATC CTCCGATTGG ACTGCGTGAG 400200
CAAAATTTCC GCGTCGTGGG ACTGGAAAAG TCCATGGACG TGCAGGATTG CTGCGAGATG 400260
AGCCTTTAAA CACCCGAAGC CTGCAATTCT TGCGAGTCTT TGCAGGATCA CTGCAGAGCC 400320
GCGCCGATAC AATCGGGCGA TGGACCACGA TTCATCTCAA AACAGGGGAG CACCAAATGG 400380
TCGACGTGTA CAGCGAAGTC ATCAAATTCT TTGGCGTACG TCCGAAAGGC GCTGCACTAG 400440
CGAGTGCCGC CGTGTGCCTA CTCGTCTGGG CTAGTGCGGC AGAAGCGGTG ACCCTCGAGG 400500
AAGTTAAGGC ACGAGGGTAC ATTCGAATTG CGATCGCCAA CGAGATTCCA GCCAGCTACA 400560
CAGATGCTAA CGGCGACGTT AAGGGGCGAG AAGCGGAAGT GGTCCAACAT GTCCTTGAAC 400620
GCATGTGCGC GCTTCGCAAA AACTGGACAT TGATTCCGCT AAATCCCGGA CAGCAATTTC 400680
ATTAAAGTCC GGACAGTTTG ATGCGGTTGG TCCTCGGCAG CCTGGTTAGC ATCAGGGCTG 400740
ATTGATTTCG CCGTTTTCTG CCCCAGTCAA GAGGGGTGCG CTTTTTTGCT TTCGCATGCT 400800
CTCGCCCCGG AGGGTGATGC GGTGAGCATT GTGGACGATA CGGTCGAGTA TGGCGTCGGC 400860
GACCGTGCTG TCGGCAATAA GGTCGTGCCA GCTTGCCACG GGAACCTGAG CTGTGATCAG 400920
GGTGGACTTT CGCTGATAAC GCTCCTCGAC GATTTCGAAG AGGTGAAAGC GCTGCTGATC 400980
GGAGAGCGTA TGGGTTCCAA AGTCATCGAG GATGAGGAGC TGGACGCGGG TGAGCCTGTC 401040
GATGAGGCGG GGGAAGGAGC CGTCGAGGCG GGCAAGCGCG AGCTCCTCGA ACATTCGGGG 401100
CACGCGCACA TAGAGCACGG AGTGACCGAG CCTGGCCGCT TGCCTGCCGA AGGCACAGGC 401160
CAGCCATGAC TTGCCGGTGC CGGTGTGGCC GGTGATGATC AGGCCCTCGT GGGCGGTGAG 401220
CCATTGCCCC TGGGCGAGCG CCATGGTGTT GCGCCGGTCG AGACCGCGGG CGGCGGCGAA 401280
GTCGATATCT TCGATACAGG CCTGGGCAAA GCGTAGCTTG GCGGAGGCGA GCCGGTTACG 401340
GATGCGCTTG TCGGCGCGCA GGGTGACTTC GCGGTCGAGC ATCAGTCCGA GCCACTCATC 401400
GCGGCTGAGC TCATTGGTGC CGGACTGCTC GGTCAATTCG CGCCAGGCGG CGGCCATGCC 401460
CGTCAGCCCG AGGGCCTGCA TCTGGTCGAG GGTGGGGTTC GTCAGCATTC TTGCTTCCTT 401520
CACTGGTAGT AGGATCGGCC ACGGATATTG GTATGCGCAG GCGTGGGGGC CGCGTGTTCA 401580
GCCTGCGGTC TTTCCCGGTC GAGGCCGGAT TTGAGGATGG AGGCGACGGA GGAATAGGTG 401640
ATGGCATTAA TGGTGAGCGC CCGCTCACAG GCCGCCTCGA GGCGCTGGGA TCCATAGCGC 401700
GGCGCCAGCG ACAGAATGCC CATGGCCGAG CGGTATCCCT GTTCCGGATG CGGCCTGTCG 401760
CGCATCATGC GTTCGACCAG GATGGCGGCA TTGGGGCCGA TCTGGGTCGC ACGGCCGATC 401820
AGATTGGCCG GCGTGGTGTT GGCATAGCGC TGATGCGCCT TGGGCATATG GTCGTTGACG 401880
GTGACGTGGC CGGAACGCTG GGAGCGGCGA ACATGGCTGG CGACACGCTG GTGGTCGTGG 401940
AAGATCTCGA CCACCCGGTG GGTGAGCCGC ACCTGGAGGG TGCATCCGAT CAGCCGATGC 402000
GGCACCGAGT AGAAGGTCTT GTCGACCTCG ACATGATAGT CCGGATGGAC CTTCGCCGAC 402060
TTCCATTCCG CATATTCGAA CGGTATCGCC GGCAAGGGCT TCAAGGCTGG CCGCTCGATC 402120
TCCTCGAACA GCTCGCGGCG GCTTTTGCCG ACATGGCGCA TCGTCCGGTT GTTCAGGTCC 402180
TCGAGCAATT CGGCAATCGC CGTGTTGAGG GCGGCAAGCG AGAAGAAGGT ACGGTTCCTG 402240

AGCCGGGCCA GAATCCAGCG TTCCACGATC AATACCGCGC CTTCGACCCG GCCTTTGTCG 402300
CGCGGTTTCC TGCTGCGGGT CGGCAGGATC GTGGTGTCGT AATGCTCCGC CATGGCGGCG 402360
AACGTCGCAG TCAATGTCGG CTCGAACCAA AGGGCCTTGG CCACCCCCGA TTTGAGGTTG 402420
TCGCACACGA TTGCCTTGGT GACCCCACCA TAGAAGGTCA GAGCACGCAC CTGACCGTCG 402480
ATCCAATCCG GCAACTGCTG GCTAAAGCTG GCATGGGCGA AGGTCAGGTT GGAGGCGCCC 402540
AGCACCGCGA CAAAGATTTG GGCCGGATGG ATGACACCGG TTGCCGGATC GATCACCGGC 402600
ACCGTCGGCC CGGCATAGTC GGTCTGCATC ACGGCGCCCG CCGCGTGCCG ATTGCGGAAC 402660
GCTACACTGG TGCGCTGCCG AAAGGCGGCA ACCTGCTCGC AGAACCAGGT GAAGCCGTAA 402720
CCATCAGGAT GGCTGGCGCG GTATTCCTGC CATAGAAGCG TCAGCGTCAC GCCTTTGCGC 402780
TTCAGCTCCC GAACCACCAG CGCCCAGTCC GGCTCGCTGA GATCGCGCGG CGGTCGACCG 402840
GCTCGGCCGA ACAGCCGCCG CTCCAGCTTT GCATCCTCGT CCGCGCCGAT TGGCAACGGC 402900
CACGAAAGCC CGGCTTCCCG AGATCGCAGC AAATAGGTCG ATACCGAGCT CTTGCCGATC 402960
TTCAGCCGCT CGGCAATCTC GCGCACCGAA AGACCCTCTT CATGGGTCAG GCGCAGAATA 403020
GTCCGGATAT CCCTCACTGT CGTTCGTCTT GCTTGCTTCC GTCTCGGCAT CGGCCCCTCT 403080
CAACGTTGTC GTGAGAGGCC TAACCAACAA GACGGCGCAG CCGCGAACCA ACCCGTCAAA 403140
CCGCCGCCGG AAACTGTCCG GGATTTAGCG GAATCGCTGT CCGGGAATTA CTGAAAACTG 403200
TGTCCGGAGA TTACCGGAAA TCCTGTCCGG ACTTTGCCGA AACCCGCACG CATGGACATC 403260
AAGCGTGAAA ACATCCAATG GATCGTTACG TCCTTTAGCT CTTTGATACC AGGCTTACAG 403320
GCAAATCGCT TCGACATGAC GGCAGCTGGA ATGGCTATCC GCCCAGAAAG ATGTCAAAAG 403380
GTAATCTATA GCGAGCCAAC CAGTTCCTAT GGCGAGGGTC TACTGGTCCC AAAAGGTAAT 403440
CCCAAAAATC TTCACTCTTA CGAGGACATT ACCACGCAAG GTAAAGTTGC GATCATGGCC 403500
GGTGCTGACC AGCTCAGAAT GATGCAGAAG CTGGGTGTTG AAGAAGGAAA TATTATCACC 403560
ATCGCATCGA ATGCAGATGC TATTGCAGCT GTCTCTCAGG GACGCGCTGA CGCCTACGCA 403620
GCATCCGCCA CTACCGCCGC TGATTTGGCA GGTAAGAGCG ATAAAGTCGA ACTCGCAGCA 403680
CCCTTTAAGG ATCCGATTAT TGACGGGAAG CCCCAAAGGA GCTGGGGCGG CTATACGTTC 403740
AATAAAGAAT CCGGCGACCT TCGCGACGCG ATCAACAAGT CGCTTCTTGA GTTTCAAAAG 403800
ACTGAGAAAT TCAAAGAGAT TCAATTGGCG TATGGCATGA CAAAGGAAAG CATTGCCGCG 403860
ATCCCAACGA AAACCACCGA TGTCCTTTGT TCACCGTAAA GCGGAAGATT GATGGGTCGC 403920
TCATCCAGCG ACCCATTCCT AAGTCCTTCT TTCGAATACG ACTCGCGACA GCCTTGTGAC 403980
AACCATTGTG ACCGGGTGCA CCAGCGACAC TGAATGGGAC AAAAGTCTAC AAATGCATTC 404040
AGAGGAGCGT TGCGGCCTCA GCAGACCAAC ACGCTGTTCC TCGCGCCAAG TTCGGGTTTC 404100
GAGGTCTTTC AATTAATGGA ACGCAGTTTC GAGCGTACGG GCGTCGTTCC TCGGTGATTG 404160
ACAGCCGGAG AAAGCGTCAT TATGCAGGAT GTCTGCAATT CTCAAGAAAT CCTGCAGGAT 404220
TACGCTATGG AACTTGATCG GGCAGATGTG GCGCTCCTCA ATGCCGTACA GAAAAATAAC 404280
CGGCTCACTT CCGAAGAACT CGCCGACAAG GTTGGCCTTT CACCCACAGC ATGTCAGAGG 404340
AGACTTAAAC GGCTCCGTAG CCTAGGTGTG ATCGAAGCGG ATGTGTCCAT AGTGTCTCCC 404400
AAGGCTGTCG GCCGCCCCGT AACGATGATT GTTATGGTGT CATTGGAACG AGAACGTGCC 404460
GATATTGTCG ACAGGTTCAA GTCCTCTATT CGAAATACGC GGGAGGTAAT GATTGGTTAC 404520
TACGTCACAG GTGATGCGGA CTTCATCCTA ATAGTGACAG CAAAGGACAT GGAGGAATAC 404580
GAGGAGTTTA CTCGGAGGTT TTTCTATGAG AATCACGACA TCAAGGGCTT TAAGACCATG 404640
GTGGTTATGG ATCGAGTGAA AGCCACATTT GCAGTTCCAA TCGAAATCTA GTCCGAAACT 404700
ATCGTGTTCG CCCGACGCCA TAGCCGGCGA ACTCGCACAA ACGTAGGCTG GCGCGACCTG 404760
CTAGCGCAAT GACCTGGAGG CCGGATGAAG TGCCGTCGGT CAATCATCTG GGGTAGCGTG 404820
ACCGGCCCTG GAAATAGTAG ACCCCTTCTG TGTCTCTAAC ATAAAGGCGC CTGTCCACTA 404880
GGACGCGATC CTCGTACGCC GCACGTGTCA GTTAGAAACC AGCAGTACAG GAGGACGTCC 404940
GCCAATCCCT CGCACTGTCA GTCTTCGGAC TAGAAGCAGA GCGCATTACA ACCCCATGCG 405000
GGGAAGTCCG GGTGGTTCCC GTCCAAACCC TGCGAAACCC GCCTGAATTT TACTGATTTC 405060
CTGCGGCCGA GGCAGAAAAA TGCCGAATCC TGGCGGCCGA GTGCATTGAT CATCCCCAGC 405120
GGCGGCTTCG TAAGCCCGCT CAAAAAGGGG AACCAAATGA CACATCTAAA GATCTCCAAA 405180
ACCGCACCTG CTGTTGCTCG CTTCCTTCCT GCCGGCCGCA TTGCGAGTGT GGCTGCGGCC 405240
CTAAGTTTGT TCTTGTCAGC GCAGCTTGCA GGTGCCGTGA CGCTCGAGGA AGTGAAAGCG 405300
CGCGGGTACA TCACTATCGC AATAGCGAAT GAGATGCCCG GCAGCTATAC CGACCCGAAC 405360
GGCGAGGTGA AGGGCTCTGA GGCCGATGTT GCGCGGCGCG TCCTCGAGAA ATTGGGGATC 405420
AAACCAGAGA ACATTCAATG GGTCGTCACG ACTTTTGGGT CGTTAATTCC CGGGCTGCAA 405480
GCCAACCGCT TTGACATGAC CGCCGCCGGG ATGGCCATTC GACCAGAGCG TTGCGAGAAG 405540
GTTATCTACA GTGAGCCGAA TTCCAGTTAT GGCGAGGGAA TGCTGGTGAT CAAAGGCAAC 405600
CCGCGAAACT TTCACTCCTA TGAAGACGTG GCTCAACAGG GGAAGATTGC GATCATGGCG 405660
GGCGCCGATC AGTTGCGAAT GATGCAGGCA TTGGGCGTCC CAAACGAGAA CATAATAACG 405720
ATCGCAGCAA ATGCCGACGC TATTTCAGCG GTCGCCACCG GGCGCGCCGA CGCCTATGCA 405780
GCGGCGGCCT CAACTGCAGC CGACCTTGCC AAAAAGAGCG ACAAAGTCGA ACTCGCCACG 405840
CCTTTCCAAG ATCCGGTCAT CGATGGCAAA ATTCAGCGCA GTTGGGGTGC CTTCAGCTTC 405900
AACAAGGAAT CCGCCGACCT GCGTGACAAG TTCAACGAGG CGCTCCTGGA GTTCCGCAAG 405960
ACGGATGAGT TCAAGCAGCT CTTGCTCGGG TACGGTTATT TGCCAGAGTC GATCGCGGCG 406020
ATACCCGACA AGACGACCAA AGAGTTATGC TCGAATTGAG GCAGCAAGAA CGATTGAACG 406080
TCGACCGAAA CATCCGTCGA CGTCTCTCCG GTGCGAACGC GCCGCGTCTA CCTTCCACTT 406140
AAAGGCGAGC TATCAATGGA TTGGCACGAC TACCTCGGAC CGTTGGGTCG TGGCGCCTGG 406200
GTCACGATCC AATTCACTCT TTACTCTATG TTCTTTGGGG CTGTCTGCTC TTTCGCGTTC 406260
GGGATTGGAA AGCTTTCCAA GAACCCGTTC GTCAAAGGCT TTTCAATCCT CTACATTGAG 406320
ATCTTCCGCG GAACATCTCT ATTGGTCCAG TTGTTCTGGC TCTTTTTCGC CCTGCCGATC 406380

GCCGGGGACA TGATGGGCAT CGATCTGCGA CTTTCTCCCG TAGTGGCTGG CGTTCTCGCT 406440
CTTAGCCTTA ACCTGGGGGC TTATGGTGCG GAAATCGTTC GAGGGGCAAT TCAGGCGGTC 406500
AGTCCTAGTC AATATGAGGC AGCAACGGCG TTGAACTTCA GCTCGTCACA AGCTCTCTGG 406560
CGCGTGGCGT TACCGCAAGC GATCCCGGAG ATGATGCCGA GTTTCTCCAA TCTCGCGATC 406620
GCCGCTTTGA AAGATACCTC ACTGGTATCG CTCATTACAT TGCATGACTT GACTTTCGCC 406680
GCGGAGCAAC TGCGCAATTT CTACCAAGAT AGCACGACCG TATATACAAT GGTCCTTCTT 406740
ATGTATTTCG GGATAGCACT CGTACTGTCT TTCTTCATGC GCCTCATAGA ATCGTCGGTA 406800
ACCCGCTGGC GCGGACATCG GAGGTAGAAA TGCTCTACGG TTTTACATGG GATACCGGCA 406860
ACGGAGAGTT AGCATTCGCT ATTTCCATTT TGCCAATGCT TTTGATGGGA TTGATAACCA 406920
CTCTCCAGGC GGCCTTTCTG GGCTTCTTCG TCGCATGCGT CCTAGGAATG GTGTTCGCGG 406980
TTTTACGGGG GATGCGTACG AGATGGGTAG CCTGGCCGGC CGCTGTCCTA ATCGAGTTCA 407040
TAAGGGACAC GCCATTAATC GCCCAATTGT TCTTCTTGTA CTATGTACTT CCTGAGTATG 407100
GAATAATATT CCCTGCCTTT TTGACCGGAG CCTTGGCCCT TGGCATCCAG TATAGCGCGT 407160
ATATTTCCGA AGTTTATCGG GGCGGAATAC AGGCTGTTGA CCACGGGCAG CGGGAAGCGG 407220
CCAAGTCGCT TGATCTTCCT CCTGCGCGCA CGTTTACGCA CGTGATCCTT CCGCAGGCCA 407280
TTCCTCGTGT CATACCCGCT CTCGGAAATT ATCTCGTCTC TATTATGAAG GACGTTCCCG 407340
TTCTTTCGGT CGTTACAATT GTTGAGATGC TAAACGCGGC TAAGATCATC GGAGACCAGA 407400
CTTTCAATTA TCTCGTGCCG CTTTCTATGG TTGGTGGCAT CTACCTTATC CTAACAATCG 407460
TTGCCTCGGC GCTCGTTCGG ATCGTCGACG TCAACCTTCC CAAAAGAGGG GTTCCCCTGA 407520
GATGAGTGAC TCCATCATCG TTTTCGACAA GGTAAAAAAG GCCTACGGCA ATTTCACCGT 407580
GATCAACGAA CTCGATTTCG AGGTTCGACG TGGGGAAAAA GTATCTATCA TTGGCCCTTC 407640
CGGCTCCGGA AAGTCGACTG TCCTTCGCAT ACTGATGACG CTCGAAGGCA TCAACGATGG 407700
AGCAGTCTAC GTGGGCGGGG AGCCTCTTTG GCATGAGCTA AAGAACGGTT CTATGATGCC 407760
TGCGACAGAA AAACATCTCA GGAAGATGCG CACCCAACTG GGCATGGTCT TTCAGCAGTT 407820
CAATCTCTTT CCACACATGA CGGTGCTCAG AAATCTCACG GAGGCTCCGC GGGTTGTACT 407880
CGGCCTATCG AAAGAGGAAG CGCGCCGACG TGCCGAGGAA CTTCTTGAGT TGGTCGGCCT 407940
AGTTGATCAC GCACATAAAT TTCCAGCTCA GCTTTCGGGG GGCCAACAGC AGCGTGTGGG 408000
GATTGCCAGA GCTTTGGCGA TGCGACCGAA GATTATGCTG TTCGATGAGC CGACTTCCGC 408060
TCTAGATCCT GAACTGGTCG GCGAAGTCCT TAACGTCATC CGGAGGCTCG CGGCCGAGCA 408120
CGACCTGACG ATGCTGATGG TTACACACGA GATGAGATTC GCGCGCGAAA TATCCGATCG 408180
TGTCTGCTTC TTCGACAAAG GACGAATCCG TGAAGAGGGC ACGCCAGAGC AATTGTTTAG 408240
TCAGCCTAAG GAAGAGCGTA CGCGCGAATT TCTCAAGGCG GTTCTTGACG GGTGATGATT 408300
GGCGGCCGGC CAGTGGGCCC ATGGCCAGCA GCGTGACTGC TTCAATTTGA GATCCGAACT 408360
CATCCCGAGC CGGTGCATTC CTATCTCGGG TGCCTTCTTG CCGTCGCGAT GATCATCGCG 408420
TTTGCGTGGA CAAGCGGCTC CGTTTTGGCA GTACTCGATC GGGCTGGCTG GTCTCGGCGC 408480
CACAGGATCG ACGGCGACGT AGGCGCCTCA GACTCGTTGT ACGGGTGGGA CGGTCGAGCC 408540
GTTTGTGAGG CAACGAAATG AGCTTAATCG CCAATCCACC GTACCTAAAA GATTCGCATT 408600
CCGCTTGCGT CCTGCGCAAA CGAATGGAAG GGCCGGAATC TGCGTGAAAC GCTCACCGAA 408660
CGCCGGAATC CTGCAGCATC AGCACCTAAA TCGGCAAACC TGCTCGTCTG TTGATGATAA 408720
TCTAGTTTCA AACAGCTTTA TTGGATGAGG GCAGCAATGA ACTTCCGTAT CAATAACAGT 408780
CTTCTAATCG AAGCCGAGCG CGACGCGGTT CTTGAATTGC GTCACGCGAT GCATCGGGAG 408840
CCGGAACTTT CCAACAACGA GTGGAAAACG CAGCAGCGAA TAAGAGGGAT GCTTGAGCGG 408900
TTCGGCCTGA AAGGGGCGAC TGTTTTCCAT AATACAGGGC TCTACATCGA CATCGAAGGG 408960
TCTGCTTCCG GACCAAAGAG AGCGGTTGCT GTCCGCGGCG ACATCGATGC CCTTCCCATA 409020
CAAGAAACTC GCGATGATTT GCCGTACCAG TCTCACGTGG AGGGAGTGAT GCATGCTTGC 409080
GGCCACGACC TCCACGCTTC CATCGCCATG GGGGTGGCGT TGGCCTTTCA CCGAATGCGA 409140
AACAATTTCG CTGGCAAACT GCGCGTCTTT TTTCAACCGG CAGAAGAGGC TGAACCGCTT 409200
GGCGGTCGCA CGGTGCTCGA AGAGCGACTC CTTGAGGGCT TCGACAATGC TGTGGGCTTC 409260
CATGTTACCC CGTCGATACA GGTGGGCAAA TTCGGCGCCC GCGAAGGAGC AGTGAGCAAA 409320
TCGTCGGACC AATTCAAGGT TACCGTGTCT GGCAGTGCAG CCCATGGCTC AACACCCCAT 409380
AATGGCATCG ACGCAATTAC GATTGCGGCA GCTTTTGTCA ACGAAGTGCA GAAGGTCATT 409440
TCAAGGGAAG TGCCGGTCGA TGATCGATCG GTCATCACGA TAGGCACAAT CCACGGCGGA 409500
GAGGCGACTA ATATTATTTG TCCGAAGGTC GTGATGGAGG GAACAATAAG AACGACGAAC 409560
CCAGAACTGC GACCGCTATT GTCGCAACGG GTGAGAGAAA TTGCCGAAGG CGTCGCAGCA 409620
CTTCATCGCG GAAAAGCCGA GGTCGTCGTT ACATCAGGTG AACCGGCGGT CATCAATGAC 409680
CCCGAAATGG TGCGCCTCTT CCGCGACGCC GTGTCCGATA TGGCGGGTTC CGACGCGTTG 409740
ACGCAGGGCA AGGCCATTAG CGGGAGCGAT GACTTCGGTT TCTACTCTCA ATGCATTCCA 409800
TCGATCTATT TCTGGTTCGG CAGTGGCGAA CCCGGGAACG AGTCCGGTGT GCATACCCCC 409860
ACGTTCGCGG TTTCCGACGA CGTTCTCATC CCGACGACCG AACTTGCTGT GAAGTATTGT 409920
TTCGATTTGC TGCATGGGCA GTCTGCACGT TGATCGACTT GTATGCCAGG ATGTTTGCCA 409980
CTGTATTAGG TGAATGAATT GTCGAACTTA AGTCTAGAAT CGATCGAGCG AGCGCGTGAG 410040
CGGATCGAAG AGCACGTCTT CCGTACCCCT CTGACGACAT CAAGGTCGCT AACCGAACTT 410100
ACCGGAACTC AGGTCAGCCT CAAGCTCGAG CACTATCAGC GCACTGGTAG CTTTAAGCTG 410160
CGGGGTGCGA CAAACGCAAT TCTTCAACTC AGCCCGTCGG ATCGGGCACG TGGGGTTATT 410220
GCGGCATCTA CGGGCAATCA CGGACGGGCT CTTTCCTACG CCGCAAAAGC GGTCGGCTCT 410280
CGCGCCACCA TCTGCATGTC GGATCTTGTT CCAGAAAACA AGGTTTCCGA GATCCGGAAG 410340
CTTGGCGCGA CAGTTCGGAT AGTGGGATCG TCACAAGACG ATGCGCAAGT CGAAGTCGAG 410400
CGGCTCGTCG CGGAGGAAGG CCTCAGCATG ATCCCGCCTT TCGATCACCC GCATATCATC 410460
GCCGGCCAGC GAACCGTCGG TCTTGAGATC GTTGAGGCGA TGCCGGACGT CGCGATGGTA 410520

CTGCTTCCAC TGTCGGGTGG CGGCTTGGCT GCAGGCGTTG CAGCAGCGGT GAAGGCACTG 410580
CGGCCTCATG CGAGGATCAT CGGTGTCACT ATGGATCGAG GCGCCGCGAT GAAGGCCAGC 410640
ATCGAAGCTG GGCATCCGGT ACAAGTGAAG GAGTATCGGA GCTTGGCTGA TTCTTTAGGT 410700
GGAGGAATCG GCATGGCCAA CGCTTGGACC TTTCAAATGT GCAGAGCCCT TCTGGACGAT 410760
GTTGTCCTCG TTAATGAAGG CGAAATAGCC GCGGGAATTA GACATGCTTA TGAGCATGAG 410820
CGTCAGATAC TCGAGGGCGC CGGCGCTGTC GGTATCGCAG CGCTTCTCTC CGGCAAGGTG 410880
GCTGCTCGCG GAGGTTCAGT AGGAGTCGTT TTGTCAGGTC AAAACATCGA CATGGGCCTG 410940
CATCGGGAGG TCATTAACGG AGTTGTCAGA GCGACCGAGG AGGATTGATG CCTACAATGA 411000
AAATACTCAC TGAGGGGGAG TTGAGGCAAA TCGTGCCGCT CGACCTCGAC GCGGTTAAGT 411060
GCACCGAAGA CGCTTTCCGC GCACTCGCCG TACCAAACGC CGTAAAAATG CCGCCCATTC 411120
TGCGCTTGGA CGTGCCTGAC AGCAGGGGTG AAGTCGATGT GAAAACTGCG TATATTCCCG 411180
GTTTGGGCGG TTTTGCGATT AAGATCAGCC CCGGCTTCTT CGACAACCCA AAGATCGGCC 411240
TTCCGAGCAC AAATGGGATG ATGGTATTGC TCTCGAGCCA AACGGTTTTG GTTCAAGCGC 411300
TTCTTTTAGA CAATGGTTAC CTCACCGACG TTCGTACGGC GGCCGCTGGC GCCGTTGCTG 411360
CAAAACATTT GTCGCAACAA GATGCTTCGG TCGCAACCAT CTTTGGAGCG GGCGTTCAGG 411420
CGAGACTGCA ACTGCGTGCG CTGACTCTGG TGAGGCCTAT CCGCCAAGCC CGTATATGGG 411480
CGCGCGATCA GCTTAAGGCG GAGGCACTCG TCGAGCAGTT GAAGCTTGAG CATAGCTTCG 411540
CCATCAGCGC GTCCGATGAT CCACGGCAAG CCGTTTCCGG TGCCCATATC GTTGTTACGA 411600
CGACGCCCGC AGACCGGCCA ATCCTCGTAG CGTCCTGGCT TGAAGCTGGG CAGCATGTAA 411660
CTGCAATGGG CTCCGACGCT GAGCATAAAA ACGAACTTGA TCCAGCGGCA ATCGCGCGCG 411720
CGGATGTTTA TGTGGCGGAT AGTCTTACAC AGACACGGAG ACTGGGTGAA CTTCACCATG 411780
CCATAGAGGC AGGCCTCATC GCGCGGGACG CTGCTTTTCC GGAGTTGGGG GAGGTGATTG 411840
CGGGCGTGAA AACCGGACGT ACCCGCGAGT CAGATATCAC TATAGCAGAT CTTACGGGGA 411900
CCGGTGTCCA GGACACGGCA ATTGCCACGC TTGCCTTTCA GCGCGCCGAA GAACGCAGTG 411960
CCGGAAGCCT ATTCGAGAGC TGAACAACTC GCGCTCCTGA CGCCTCAGCC ATGTTGAGGC 412020
GCTGACTTCT CATCTGTTCA CACGTTACCT TAGTATCCGC CGTCCTGCGG ACAATCATGC 412080
AACTAAATGC TCAGACCATA TCCTACAGGA GGCAGCCATG CCGGTTATCA AGGGACCCCA 412140
AGTATTTCCT CGCGCCGAAT TCCTTAGGCG ACTCAATGCT GTGAAGCTGG AGATGGGCCG 412200
GCTTGAAATC GATGCGCTCA TTGTCGGCAG TTCTGCTGAT ATCACGTATC TGACCGGCTA 412260
TACGGCTAAG TCCGGATATG TGCCACAGGC TCTCGTAATC TCATCGAATG ACGAAGAGCC 412320
CACATTCATC CTGCGGCGCC AAGACGCGCC TGCGGCGATC CACCAGACAT TCATGGATCG 412380
GGACAAAGTT ATCGGGTACT CTGAAGCCCT CATCGGGAAC CCCGATAAGG ACGGCTATGA 412440
CGCCGTAGTT GACTTCTTGA ACGAACGCGA TGGTGCCAAC AAGCGCGGTG TTGGAGTGCA 412500
GCTTGGCTAT CTGTCCGTAA GATCAGCTGA AAAGTTTAAG ACGCGCCTGC CAAGCGCGCG 412560
AATTGTTGAC TGCACCCATT CGGTCGCGTG GATCCGCATG ATCAAATCCG ATCTCGAAAT 412620
TTCGATGATG CGTGATGCGG CCGCAATCGC AGACGCAGGA GTCCAAAAGG CATTCGAAGT 412680
CATTCGACCG GGAGTGCGCG AAGCCGACGT TATGGCTGAC GTCGCAGCTC AACTTGCGCG 412740
GGGGACAAAC GGTAAAGCGG GAACAGATCT CGCGTCGATG TATTTCTGTT CTTCACCCCG 412800
AACCGGCACA TGCCACATTC GATGGAGTGA AGATGTCATC CGCGATGGTT CTCAGATCAA 412860
TCTTGAAATC GCGGGCGTTC GTCACGGCTA TGTCTCAGCA ATAATGCGGA CTTTCTCCGT 412920
AGGTGCCCCA TCCGATCGAC TTCGACGCAT TCATGACGCG GAGGTACTGG GTTTGGAGGC 412980
CGCGCTCAGT ACGGTAAAGC CTGGCGCTAC TTGTAGCGAC GTCGCAAACG CATTCTACCG 413040
CACGATCGAA AAGTCCGGCT TTCAAAAGGA CTCCCGCTGC GGATATGCGA TCGGGATCGA 413100
CTGGAGCGAA CCAACTGCAA GCCTGAAGGA TGGTGATATG ACCAAACTCA AGCCAAATAT 413160
GACCTTCCAC CTGATGCTCG GCAATTGGAT TGAAGAAGAT TTTGGCTACG TACTCAGTGA 413220
GACCTTCCGT GTCACCGAAG CCGGCTGCGA AGTGCTGACG AAGTCGCCGC GGCAACTGTG 413280
TGTGATCTAA AGAAGGCAGC CAACGAAAGT TGGTTTCTAA CAACGTGACC CACTTCGATA 413340
ATCAGACGCA AATTGGCTGG TTCGGCCGCG GAGAAGCGTC AATTCTCAAA CCGTCAGCAG 413400
GGAGCCTTAG CGTCGTTGGA CGCGAGCGCT CTCGAAGCGT CAAGGAACCA AAATGCGTAT 413460
TCTTCATTTC TCGGACGCTG AGTATCAGCA GCGGATGCTC CGAGTAAAAC AGCGGATGCA 413520
AGCTCAAGAC ATCGATGTCT TGATTGTCAC TGAGCCGGCC AACATCAACT ATCTCACTGG 413580
ATACGATGCT TGGTCCTTCT ATACGGTTCA GGCACTGTTG GTGTTCCAAG ACGTTGATTT 413640
ACCGATGTGG ATTGGCAGAT CAATAGATAA GCAATCCGCA ATTGTTACAA CAAATCTTCC 413700
TGATGAACGG ATTATACCAT ATCCGGACAT ATATGTTCAT TCCCCAGATC GGCACGCCGC 413760
GCAATTTATT GCGCAAATGC TGTTAAGGGA GGCTCCGCGT GCCAAGACTG TCGGCGTGGA 413820
AATGGGCGCT TATTATTATA CAGCGCGTGA TCATGCTGAA CTGCAGAAGG CATTGCCAAA 413880
TGTCGCGTTC AAGGACGCAG AGTTGCTCGT AAATTGGGTG CGCTTCATCA AGAGCGAGCG 413940
GGAAATCGCT TACATGCGAG AAGCCGGCGT GATTACTGAA CGCATGATGA AACGGGCGGT 414000
AGAAGTCGCA GCGCCAGGTG TACGACAGTG TGACGTGGCC GCTGCAATCT ACCATGCACA 414060
AATGTCGGGC ACAGAGAACT ATGGCGGGCT TCCTGCCACC AGCCCACCAC ACATGGGGTT 414120
CGGTGAGCGC GCTAGGGAGC CGCACCCGAT ATTTACCGAT CGACGTATCG ACACAAATTC 414180
TGTTGCCAAT ATCGAATTGT CGGGATGCCG ACTTCGATAT CACGCACCCA TGAGCAGGAC 414240
AGTCTATTTC GGCAGGCCGC CCCAGAGTTA CCGGGACTTA GCGTCCTATG TGATTGAGGC 414300
CGTCGAAACG TCACTCGATG CTGTCCGACC TGGTGTGACG TGCGAAGGCA TCGAATTAGC 414360
GTGGCGCAAG AGCATGAGCG CTCATGGCAT CGAGAAGGAA AACCGGCTGG GTTACTCCAT 414420
TGGCATTGCC TACACACCGA CATGGGGCGA GCGAACGGCG AGCATAAGGC GGACGGATCT 414480
TACTGTTCTT GAGCCTGGGG CGGCATTTCA CCTCATGGGT GGATTGTGGT TGGCAAACAC 414540
CGGAATAACG ATCACGCAGT CTTTCGTTGT AACAGCAACG GGTCACGAAC CCCTTACGGC 414600
TACGCCTCGG GAACTGGTCG TTAAAGACTG ACGCCAGATG GGCCCGCCGA GGCTATGAGC 414660

ACTTCAACGC GACGTGTAGT CCGATTTCGA CTTTCGTTTG CGTCGCAGCC GTCGCCTGTA 414720
TGCGTTTTTT TTGGACTCAA TTCTCAACTG CTTTGGCAAG CGGCGCCGTG TCACGTGTCC 414780
TTCCATCTGA TCTGCGCGTC CAACAACGAG CTCAGAACTA AATCGCGGCC GCGACTTCTG 414840
CAGGACGAAA AAAGGGCGGT CTGGCGGCGC TTGCGACAGT TTTGCCCAGT CGTTTTTCCT 414900
CGGATTGCAC AATCGATGAA CGCGCTCGCC CTCCGGGGCG CAACTAGAGA AGGAAGCGCC 414960
TTGGCCTTTT TCGGTCAACT CGCCAAATAT CTGAACAGGA TTGGAAAGCG TGCGGAAAAA 415020
TCGAAGCCCT CCGATTGGGA AAAGTATGAC GTAAAATATC CGAATAAGCC GCATTCGGTC 415080
AGAAGCGTCA CCTTAAATGC AGGATTTCGT CGCAGCGGAA ATCATTCTGC CGGAAATGTG 415140
CACCCTGCGT CGCCTATGAT CAAAGTGCAA GGTGGCTAGC GGCTCGAGCG TGAGGGACGC 415200
GCCAGACCAG CGTTTCAACG CAGTAACAAC TAAAAAGTGG GAACCAAAGC GATGACAATT 415260
TCTCGGCGTG ATCTCTTCAA GGCAGGCTTG GCTGCAGGAG CTGCTCTCTC AGTTCCTTCG 415320
CTTCTTCGGG CGCAGACCGC AGTTGCGGCG GATAAAACTG TTCGGATGGC ACTGGAAAAT 415380
CTCAGCGTCT TTGATCCGGT GGCCACGACA GCCGGCATCA GCCAAACCCA TGCTCTGGCG 415440
ATCTACGACA CACTGTTCTC GGCTGACTCG CAGTCTCAAC CTCATCCGCA AATGGTGGGG 415500
AACTGGGGCG TTTCCGATGA TAAGAAGACG TACACATTCG AATTGCGCGA CGGTTTAGGA 415560
TGGCATGACG GTACCCCTGT CACCGCAGCT GACTGCGTGG CGTCAATTCG CCGCTGGGCG 415620
CAGGTTGGAT CCGGCGGACA GATCCTTATG TCGCGAGCTA GCGACATCTC AAAAAAAGAT 415680
GACAGGACAT TTGTCATCTC GCTCAAGGAG CCCCTGGGGG CTTTGCCAAG CATCCTGGCC 415740
TTCGAGGGTC CGTTTATCAT GCGGGAGAAA GACGCCGGAC TACCTCCGAC GGAGCAGGTC 415800
ACTGCGAACA TCGGATCAGG ACCGTTTAAG TTCAATCACG ACCTAGCCAA ACCTGGCGCG 415860
AGTTTCACGT ACGACCGCAA CGAAAAATAT GTGCCGCGCA AAGAGCCGCC TGACGGAATG 415920
GCTGGCGGCA AGACGGTCTA TGTCGATCGG GTCGTGTGGG ATATCGGCGT GCTCGCCGAC 415980
CCGCAGACTT CCGTGGCCGC TCTTCAGACG GGCGAAATCG ACTTTCTCTA CAGGCCGCCG 416040
ATTGATCTGT TGCCTCTAAT CGAGAGCGAT CCCAACCTGA AACTCGAGGC GCTGAATAGG 416100
GCTGGTGTGG ACATGACTCT GCGCATGAAT TGCCTTCAGG CGCCGTTTAA CAGCGTAAAG 416160
GCTCGACAGG CATTGCTCCA CCTCGTGGAC CAAGAGGCAG TATTGCGCGC TGCGTACGGC 416220
AATCCCCAGT ATTTCAAGCC CGTCACGTCA ATGTTCGGCA ACACGGCAGC GGTTACCAAC 416280
GACGAAAATA CGGGATGGTT CAAACCAGGC GGCGATCCCG AAAAAGCAAA GCAACTTTTC 416340
AAAGAGGCTG GCTATGCGGG CGAAAAAATT GTCATTCTGC AAGCGACCGA TTGGGCCGAG 416400
CAAAGCAATG CTTCACAGGT CGTGGCGGCG AAGCTTCGTG AAATCGGCGT CAACGCCGAG 416460
CTCGCGCCGA GCGACTGGGG CGGCCTTGTC TCCCGCCGCT CGAAGAAAAA CCCCGCCAAC 416520
AACGCCGGCT GGAGCATCTT TATCACTGAC CAGTCTGAGG CAACACGTGC CAACGTTTTT 416580
ACCGACATTA GCCTTGCCAT GAACGGCGAA AAGGCATGGT ATGGTTGGCC AAAGAATGAT 416640
GAATATGAGG CGCTCCGGGC CAAATGGTTG ACCCTCGAAA CGCTCGATGA GCGCAAGGCG 416700
CTTGCGCGCG AAATGCAGAA GCTGTGGTGG GACTACGTCC CTGAGGTTCC GCTCGGTCAA 416760
AACATTGTAC CTAGCGCTTA CAGCAAGACG CTTACCGGCC TCATCCCTGC AACACTGCCG 416820
CTTATGTGGA ACATGCAGAA GGCCTGATAC CGTGGGTGTT TACATTCTTC GCAGACTGGT 416880
TTCGACCATC GCCGTAATGG CGATGGTCGG GATTTTCATC TTCCTGCTTC TCAGGCTGGC 416940
GCCAGGTGAT CCGGCGGCGG TCATCGCAGG ACCAACCGCC ACGGAGCAGA TGGTTGCTAA 417000
CATCCGCGAG GAGTTAGGTC TTAACGAACC GTTGCCTGTT CAGTTCGTCC ATTGGGCTTC 417060
TGATGTTTTG AGGGGGAACT TCGGAGCCTC GGTTTTCACG GGGGTGCCCG TTCTCCAACT 417120
GCTTTCCCAG AGGTTGGAAC CGACGATCTC CCTGTCTGTG TTGACAATGA TCCTTTCGGT 417180
CACCGTTGGA GTCTCCTTCG GTGTACTGGC GGCATGGCGA TCTGGCGGTT TCGTCGACCG 417240
CGCCCTCGCG ACTTTTTCAG CTATCGGCTA TTCCGTCCCG GTCTTCGTCA TTGGCTATAT 417300
CCTCATCTAC TTCTTCGCCA TTCAAACTCG TTGGCTTCCG GTTCAAGGAT ATACGTCCAT 417360
CAATCAAGGT GTGGCGCCCT GGTTCTTACA TCTGATACTC CCAACGGTCA CTTTGAGCGT 417420
CCCCTACATC GCCTTCATTG CGCGGATTAC GCGGGGCAGC ATGCTCGAAG TGCTGTCGGA 417480
AGACTATATG CGAACAGCTG CCGCCAAGGG TGCCTCGCCA TTTGCCATGC TATTCCATCA 417540
TGCACTGAAG AACGCCGGCG TACCCATTCT TACTGTGATC GGGATAAGCT TCGCTTATAT 417600
GATCGGAGGC GTCGTTCTGA CAGAGACCGT GTTTAATGTG CCTGGCATCG GCCGCCTCGT 417660
CGTCGATGCA ATCAAAAACC GTGATTATCC CATCATCCAG ACCGTGCTGG TCCTAATTTC 417720
CGGCCTGTAC GTTCTGATCA ACCTACTCGT CGATCTTGCC TATACCCTGA TCGATCCGCG 417780
TATCCGGTAC TAAAGATGGC CCTTACCACT TCGCGCGCTT CGGGATCGCC TCGTATTCAA 417840
ACCCACACCG TTGTCCGCAT CGCAAAGCGG CATCCGCTGG TCCTCCTCGG GGGAGGGATC 417900
CTGCTGTTGC TTATCTTGCT CGCGCTTGCT GCGCCCCTTT ACAGCGGTGA TCCCTTGGTG 417960
ATGGACCCCT TCAAGCGTCT CCAGCAGCCC TCCGCCTCAA TGTGGTTCGG CACCGACAAC 418020
CTGGGGAGGG ATGTCTTCGC CCGAACAATC TATGGAGCGC GCATATCACT CATCGTGGGA 418080
TTGCTTTCAG CAGTGTGCGC AGCGGTTTGC GGTCTTTTGA TCGGTGTAAT CGCCGGCTAC 418140
AGCCGCACCT TTGATAACAT TATCATGCGG GTTATGGACG GACTGATGTC CATCCCGACG 418200
TTCTTGCTCG CTATAGCGCT TCTCTCTCTC ACGGGCCCCG GAATTGGGAT TCTCATCGTT 418260
GCAATTGCGA TTCCAGAAAC ACCCGCTGTT ACGAGATTAG TGCGTTCCGT GGTTCTGAGC 418320
GTTCGTTCGC GACCCTATGT AGAAGCAGCG CTGTGTGGTG GGGCACGCCT GCCTAGGGTG 418380
CTTTGGCGGC ATATTTTGCC GAGCACCATT CCGCCCTTGA TGGTTCAAAG CGCGACTGTG 418440
TGTGCCAGTG CGATCATGAC CGAGGCTGGT TTGAGCTTTA TCGGCGTGGG CGTGCCCTCC 418500
GAGATCCCAA GTTGGGGTAA TATGATCGCC AACTCACGCC TCTTTCTCGC GATCGCTCCC 418560
TTGACGATCT TTGCTCCTGG ACTGTGTCTC GCCGTCACTG TTCTGGCGGT CAACCTGCTC 418620
GGGGATGGCC TGCGCGACAT GTTCGACCCC CGTTCGAAGC GGAGGCGTTA AAATGAGAAT 418680
GCAATCAAAT CCGAAAGCCG ATCTGCTTCT GGACGTTCGC GACCTGGAGA CGCACTTTTA 418740
CGGCGAAGAG AGCGTTACTC GTGCTCTGGG CGGTGTCAAC TTCCATGTGA AAAGTGGAGA 418800

GATGCTGGGT ATTGTAGGTG AATCGGGATG CGGGAAGAGT GTAACTGCTC TTTCGATTCT 418860
TCGCTTACTC CCAAAGCAAA CAGCTAAAAC AGTCGGGGGC GAGGTCATCT TCAAGGGGCG 418920
CAACCTTTTA GAACTGTCTG AGCGCCAGAT GCGTGAGGTC CGGGGTAACC GGATCGCCAT 418980
GATTTTCCAG GACCCGATGA CAAGCTTGAG TCCTGTTCAC ACTGTTGGGC GCCAGATCGC 419040
TGAAGCAGTC CAAATTCACG GCCGCGTGTC TCGCGCCGAG GCCCTCGAAA AGGCATTGGA 419100
GATGCTTCGA CTTGTTCGCA TTGCCGATCC CGAGCGACGC CTCAACAACT ATCCGCACGA 419160
AATGTCCGGC GGAATGCGCC AGCGCGCCAT GATCGCCATG GCGCTCGCAT GCTCGCCTGA 419220
ACTTTTGATT GCTGATGAGC CGACGACGGC GCTCGATGTA ACGATTCAGG CGCAAATTCT 419280
GCGGCTGATC GTCGATCTGA AGGACCGTAT GGGAACGGCC GTGATGTTCA TCACGCACGA 419340
TCTTGGTGTT GTCGCTGAGA CCTGCCAACG TGTCATCGTG ATGTATGCCG GCCGCATTGT 419400
GGAGCAGGCG AGCGTCACTG ACCTGTTCGC TCGCCCCACA CATCCTTACA CGCGAGCCCT 419460
CATGAATTCT GTGCCCGATC GGCGGCGCGG TCGGCAATCT CGCCTTCCCG AAATTCCGGG 419520
GGTTGTACCG AGCCTCCGCG AGCCTCTTGT CGGCTGCAGC TTTGCTGAGC GCTGCCCCTT 419580
TGCAATTGGG GTATGTCGCG AAAAGATGCC TGTCCTAAGC GAGATACAAC CTGGACACGC 419640
TGCCGCTTGC TGGCGTTCGA ATGAAGTGGT GAACCTATGA GCGACGCGCT GCTGAAAGTT 419700
GAGAGCCTGA CCAAGCACTA TCCTATCGAA GCGGGATGGT TCAAGACGAA CGTTCCTGTA 419760
GTTCGGGCAG TTGAGGACGT GTCGTTCACT GTCCAGGCAG GAGAAACGCT CTGCGTTGTC 419820
GGTGAATCAG GCTGCGGGAA ATCCACGCTT GCGCGGCTCC TGATGCGGCT CATAGATCCG 419880
ACGAGTGGGC GAGTGCTGGT GGAGGGGACC GACATCGCAG GACTAAGAAA GAACGAGCTC 419940
AGAGCTTGGC GCCGGCGGAT GCAGATGGTG TTTCAGGACC CGTACTCCTC TCTGAACCCG 420000
CGTCTCACCG CGGGCCAGAT TATCACCGAA CCGGTCGAGA ACTTCGAATG TCTAAGTAGA 420060
AGACAGCGCC AGGAACTTGC TACGGATCTA CTCCGCAAGG TAGGAATGTC GCCGGAGATG 420120
GCGTCTCGAT ATCCGTCGGA AATGTCGGGC GGTCAGCGCC AGCGCTTGGG GATTGCTCGA 420180
GCGCTGGCAC TTAAACCTTC ACTCATCATC GCTGACGAAG CCGTTTCAGC GCTTGACGTC 420240
TCTGTGCAGG CTCAAATCCT AAATCTGCTC ATGGATCTCC AGCAAGAAAC AGGGATAGCC 420300
TTGATTTTTA TCTCCCATGA CCTTGCGGTC GTGGAGCATA TCGGCCACCG CGTTGCGGTC 420360
ATGTACCTGG GGCGCATCGT CGAACTCTCG CCCCGCGACG CTCTTTTTGC CAAGCCAGTT 420420
CACCCCTACA CAGAGGCATT GATTGCGGCC GCGCCAGTTC CAGATCCTGC TCGCGCCAGG 420480
CTAGAGGTCC CCTTGGAGGG AGAAGTGCCG AGCCCCGTCA ATCCACCGAG CGGATGCGCG 420540
TTTCACCCAC GGTGTCCGCT CGCGGTTGAT CGGTGTCGCG CTGAAGTGCC GCCACTCGTC 420600
CCTATGCCCG ATGGTCGTGC GGTGGCTTGC CACGTACGGG CCCCGGCTGC CGCGGCTCAT 420660
ATTTCGCCGA GCGCTCTCGC GTCTCTTGCA CTGCCAAAAA CCGTTCAGCC CGGCGGAACA 420720
ATTCTCGCCC GTCGTTGAAT CAAAAAGCTA GCCCAGAACA CCAAGGAAGA AATATGGAGA 420780
AGTGCTCACA CGAGAGCGGT CGTCATTCGG CGGAGAATGA CGGCAAATAC GATATCACTG 420840
GTTCGACGGC GACGAACGTC GTGGACGGTT TCACAATGGT GGCAGTTGGC GACGTTATCG 420900
TGTCGCGAGC CCTGGCGAAC GGGCACCATC CGGGCTTTAG TGAGATCGTC GAGCTCCTGC 420960
GAGCCGCAGA CGTAACTTTC GGCAACATGG AGACGCTGAT CTTCGACATC AGATCATTCA 421020
ATGGAACACC GCAGGCTGAG TACGGGGGGG CGTATCACGT CAGCCTTCCC GAGATTGGAC 421080
CTGATCTGAA GGCGATGGGG TTCAATATTA TGGGCCGCGC AAACAACCAT TCCTTGGACT 421140
GGGGCGTCGA AGGCATGCGT GAGACGAGCC GGATCCTCGA TGAGAGCGGC ATCATCCACG 421200
CCGGTGTGGG TGAAAGTCGC GCACAAGCCA GCGCTGCACG TCTTTTAGAA ACGGCCCGCG 421260
GTCGCGTCGC TTTGCTCTCA TGCGCCACCT CATTCACGCC GATGTCGCGT GCATGCGATC 421320
CAGCGGGCGA GGCACCAGCC AGGCCTGGAG TCAATGCGCT CCGTCTAGAG AGAAGCGTCG 421380
TCGTCGAGCC CGACATGCTT GAGAGCCTAA GAAAGATACG TGATGCCTTG CCCAATCCTG 421440
GGCCTAAACA CGATGATCGC GAAATGTTGG TGCTCGCAGG AACGACCTAT AGAACCGGCA 421500
AAGACGTCGG CTACACCTAT GCGGCTAATA CGCGAGATCT GGCCGATATT TTAAGAAACG 421560
TTCGGCGCGG CAAGCAATAT TCCGATTTCT GCATTTTTAC AAACCACGCA CACGAGCCGG 421620
GAAATTGGAG CGAAGAGCCG GCCGATTTTG AGCAGGCGCT TGCTCGCAAG CTAATCGATG 421680
CGGGAGCGGA CGCGTACGTT GGACACGGAC CGCACCGACT GCGTGGCATC GAAATCTACA 421740
AACGCCGGCC CATCTTCTAT AGTCTGGGGA ACTTCTTTTA TGATGACCTC CGGACACCCG 421800
TTGGGGCGGA CATGTACGAT GTGTACGACA AGGACCCACA GGTGGACACC GATGCCGAAG 421860
TAACCGCAGC GGAAGAAACA ATGGGTTACC CTACGGCTGC AGGGTTCATA GGCGCGCTCG 421920
CCGAACCGGT GTATTACGAG AGCGTCGTCG CAGTTAGCCG CTTTGAGGAG AACCAACTGG 421980
CCGAACTGCG GCTTTATCCA ATCGAGCTCG GTTATTCCAA AAGGCTTGCG AACAGGGGCG 422040
TCCCTAGTCT TGCTCCACGC CCTCAGGCAA TCTCGATTCT TGAACGCCTG CAGAGGTTGT 422100
CAGAGCCGTT TGGAACGAGA ATCACCATCG AGGACCGTGT CGGTCTTATT CGCCTCTAAT 422160
CGCATAGACG GTCGACGCGC CATAAGGTTC CATTAACATG GCTCAGTAGC TTCATTGAGC 422220
CTGCAAACGG CGGCTTGGCG GCCGTTTGCG AGCGCGCCAG CTGAATACGC GCGCCCCGTG 422280
TTCAGATGAT CGAAGGTCAA GGATTGCAGC ATTGCAAGAT GTTCTCGGCT GCGATGGGGC 422340
AAACTTGGTT TCCAGGTCCG TGACTTCGCA TAATGGGAAC CTCAATAGAG GTAAGTTCCT 422400
CATCTGTCGT ACGCCCTCGA CTCGCCACAT CCGTCGCGGC CTCCGTCGCG CGGATCCTGA 422460
TCCGGCGTAG AGGGCGCTCA GTTTGCAGCA TTCCTGCGCG CAAGTGATCG AATACGCAGT 422520
CAATTCCATT TCATGGTCAC GTACATTTTA GGTCGAAACA AGCGCGTTCG AGCGCCTGAC 422580
AACAGGAACT CATCGCCAAA TCCTTCGCAG CCCGAGAGGA AGCATTTATG ACCATCGATA 422640
TTAAGGACAT CAGCGAGAAG GACCGCAATA CGGTCCTGCA TCCGTTCACA CAGCTGAAGG 422700
ACTTTGCGAC CGGAAAGCTT CGTGAGCCGA CGATCGTCGA GACGGGCAAG GGGATCCGCA 422760
TCCAGGACGC GCGTGGTAAC CAGCTGATCG ACGGCTTTGC CGGCCTCTAT TGCGTCAATG 422820
TCGGCTACGG CCGCACCGAA GTCGCGGAGG CGATCTCACG CCAGGCCTAT CGCCTTGCCT 422880
ATTATCATTC ATATGCGGCA CACACGACGG ATGAACTCGC AATTCTGTCG GATCGCCTCG 422940

TGAAGATGGC CCCGGGCAAG ATGAGCAAGG TGTTTTACGG CATGTCCGGT TCGGACGCCA 423000
ATGAGACCCA GGCCAAGCTC GTCTGGTACT ACAACAATCT GCGTGGCAAG CCGACGAAGA 423060
AGAAGATTAT CTCGCGCGAA CGCGGCTATC ATGGCTGCAG CGTCGTCTCC GGCTCGATGA 423120
CCGGCATGAG CTTCTATCAC GATCACATGG ACCTGCCGCT GCCGCAGATC TGCCACACCG 423180
GCGTGCCGCA CCATTATTGG GGTGCAAACC CGGGCGAGAC GGAACGCGAG TTCTCCGCCC 423240
GTCGCGCCGC CGAGCTCGAC GAGATGATCG AAACACTGGG TCCCGATAAT GTCGGCGCCT 423300
TTATCGCCGA GCCTGTGCTT GGTACCGGCG GTATCACGCC GCCGCCGGAA GGCTATTGGG 423360
AAGCGATCCA GGCCGTGTTG AAGAAGCACG ACGTGCTGCT GATCGCCGAC GAAGTCATCA 423420
CCGGCTTCGG CCGCACCGGC TCCATGTTTG GCTCGCAGCA TTACGGCATC GAGCCTGACC 423480
TGATCACCGT CGCCAAGGGT CTGACCTCGG CCTACTTCCC GCTCTCAGCC TCAATCGTCG 423540
GCGAGAAGGT CTACAAGGTG CTGGAAGACG GCGCCGATCG CGTCGGCGCC TTCTCCCATG 423600
GCTATACCTA TTCCGGTCAT CCCATTGGGG CGGCCGCAGC AAATGCGGTG CTCGACATCG 423660
TCGAAAAGGA AGATTTGCCG GGCAATGCCC GTGAGGTCGG CGGCTATTTC CAGGCGCAGC 423720
TCAAGGAGAA GTTCGCCCAG CTGCCGATCG TCGGCGAAGT GCGCGGTGTC GGCCTGATGG 423780
GCGCCATCGA GTTCGTTGGC GATCGTGAGA ACAAGAAGCG TTTCGACCCA TTGCTGAAGG 423840
TCGGAGCTCG TGTCTCCAAA GCTGCCCGCG ATCGCGGCCT TATTGCCCGC GCCATGCCGC 423900
ATGGCGACAT TCTCGGCTTT GCGCCGCCGC TCGTCACCAC CAAAGAGGAA GTCGATGAGA 423960
TCGTCGCCAT GGCCGAGAAG GCCGTTCGCT CTGTCATGGA TGAGTTGGTC CGCGACGGTC 424020
AGAAGCTCTG AGCGATAGTT AAGCCTTCGG CGGCGGTGGA TGCCGGCCGC CGAAGGCTTG 424080
TGATCAACGA CTGCATGAAG CCGGAAAGGG GCAGGAGATC ACCTTTGCCT CGCAGACCAA 424140
CGCGACCTCC GGACGAGAGG TCATCAGGAA TAGGGAACCT GGCTATGACT CCCGCCAAAC 424200
TCCACATGAC GACCAGCCTG GCGCCGATCG CTGTTCACAG CCCCTATGAT GGGTCGCTGC 424260
TTGGGTCAGT TGAGGCGACC GATCCTGCCG ACATCGATCG CCTCCTGGCA ACCGCCCGCC 424320
GGGGCGCCGA GATCTCGCGC AATCTGCCGC GGCACAAACG AGCGAGCATC CTCGAAGGCG 424380
CAGCCCAGAT GGTCGAAAGC CGTCACGACG CTTTTGCCGA GATCATCGTT CGCGAAGCCG 424440
GCAAGACCAT CGTTCAGGCG CGGAAAGAGG TGCTGCGCTG CGTCAACACG CTGAAGCTGT 424500
CGGCGGAAGA AGCAAAGCGC AATGCCGGCG AGATCGTTCC CTTCGATGCC TATACCGGCT 424560
CGGAGCAGCG CCAGGGCTGG TTCACCCGCG ACCCGCTCGG TATCATCACT GCCATCACGC 424620
CTTACAACGA CCCGCTGAAC CTTGTCGCGC ACAAGCTCGG CCCGGCGATT GCCGGCGGCA 424680
ATGCAGTGAT GCTGAAGCCA TCCAACCTGA CGCCATTCTC GGCGATCAAG CTGGTGGGCG 424740
CGCTGCGGGA GGCGGGCCTG CCTGAGGAGG TCATCACCAT CTCCCATGGC GACCGGGAAC 424800
TGGTGACAGC GATGATCGCT GCTCGTGAGG TGCGCATGGT GTCCTTTACC GGCGGCTTTG 424860
CCACGGGCGA GGCGATCAGC CGCGCGGCTG GCCTGAAGAA GCTTGCCATG GAGCTCGGCG 424920
GCAATGCGCC TGTGATCGTG ATGAATGACT GCGACTTCGA CAAGGCAGTC GAAGGTTGTG 424980
TCTCCGGCGC CTTCTGGGCG GCCGGGCAGA ACTGCATCGG CGCGCAGCGC ATCTTGATCC 425040
AGTCTGAGCT TTATGGCCGC TTCCGCGATG CTTTTGTCGC GGCGACAAAG AAGCTCAAGG 425100
CGGGAGATCC CCTTCAGGAG GACACCGACG TCGGTCCGAT GATCTCCAAG CAGGTGGCGG 425160
AACGCACCGA AGCCGCCGTC AACGAAGCGA TCAAGGCAGG CGCGACGCTG CTTTGCGGCA 425220
ACTATCGCGA AGGCTCCCTT TATCATCCGA CTGTTCTCGA AGGCACGCCG CTGACCTGCC 425280
GGCTGTGGCA CGAGGAAGTG TTTGCGCCGG TGGTCATGCT TGCACCCTTC GACACGCTCG 425340
ATAAAGGGAT CGAGATGGCC AACGATCCGG ACTACAGCCT GCATGCCGGT ATCTTCACCA 425400
ACGATCTCAA CGTCGCACTG GAGGCAGCAA ACAGGATCGA GGTGGGCGGG GTGATGATCA 425460
ACGACTCCTC CGACTATCGT TTCGATGCCA TGCCATTCGG CGGCTTCAAA TACGGCAGCA 425520
TGGGCCGTGA AGGTGTGCGC TTCGCCTATG AAGATATGAC TCAGCCAAAG GTCGTCTGCA 425580
TCAATCGAGG GTGAGCCGTA AGTTCCAAGC GGTTGACTGA ATGCGCAAGC GTACAGGCGC 425640
GGGGCAAATG CTCCTCACCA CCGGCGTAAC TTGAGGGGAG ATTATGGATA AGACCCACGG 425700
CAAGATCTGC GTCGCCGAGG TCGAGGAGAT CGCGGCGGTC GGCAGCCTCG ATCCTGACCA 425760
GATCCACCTG CCTGGTATTT AAGTGCATGG CCGATCAGGG GCCAGCACGA AAAACGCGTC 425820
GAACAACGCA CGACGCGCGC GGCGGCTTGA GGGTAGGGGG AAGAAATGGC CTGGGGGTGC 425880
GCGTGCTCAA CACGCTGCTG TTCGCGACGA GGCGCCGATC GGCCAGGAAG CGTCTTGCAA 425940
CGTTCTGCAT CGACGGCGGC AGGGCATTTG CCATGTGCGT GGAATCGTGT GAAGGAAGGT 426000
GTAAGTGTCA TGACCGGCGC CGAGTTTCGT CTCTGCGATG TCGCGCTCGA CTGCTCGTTC 426060
AAAACCCGCG ACCAGCGAGT TGAGCGCGAA CAGGCACTCG CGGTCCTCGA CCTCCTCGAG 426120
GCGAGTACCT TCGTGCCCGT CGGCCATCAC GGCGGCCCGT ATCGCCTCAA GATCTCGATG 426180
GCCGCCGGCC GGCTGGTATT TCATATCGCC GACGATAAGG GAGCGCATAT CGTCAGCCAC 426240
CATCTTTCGC TCGCCCCTTT CCGTCGGCTG CTTAATGACT ACAGCCGCAC TTGCGAAGGC 426300
TACTACGAGG CCATGTGCCG CTCCAACCTC GAGCGGCTGC AGGCGATCGA TATGGGCCGA 426360
CGGGGCATTC ACGATGAGGC TGCCGAGCTA CTGAGGGAGC GTCTCTCTGC TAAGGTCGAC 426420
ATCGACAAAG ACACAGCGCG TCGGCTGTTC ACGCTCATTT ACGCGCTGCT CGCTCGCAAC 426480
TCCGGCCATC AGATCCTTTT GAATTGAATA GCCTGCGATC AACGCCCACA AGCGGGGGGG 426540
ACTCCCCACC GTTTAACAAG CGGATTTTTG AGCGGCCAGA GAGGGGTCGA AGGTGGTGCC 426600
AGTTTTGAGC ATGGCGCAGG CGACGGCGAG GAGGCGGTCG GCGACTGATC GCAGGGCGCG 426660
ACCGTGGCTG TGACCTCGGC TTCGAAGGGC GGCGTATTTC AAACGGTTGT GAGGTCGTGC 426720
TGAACGGCGA CGCGCGCCCA ATGGTACATG GCGTTGGCCA GTCGGTCATG GCAGGCCTGT 426780
CTTCTGACGA CGATGCAGCT CTTGCCCGAG CGCTTGGTGA CCGGCGCGAC TCCTGTCAAG 426840
CTGCGCAAGG CGGCGTCGTC ACGTCGCTGC AGGGCATCGA ACGCTTCTGC GAGCAGCGTG 426900
GCGAGGACAA CCCTTCCCAC TCCCGGCAAG GATGCGAGGA TCTCCACGTC ATGCTGCTTC 426960
TTCTGCCCCG GCTCCACCTG CGCGGTTGCG ATGAGGCTGG CGGTCAGGCC ATCCAGCCGA 427020
TGATGCGCCT GTTTGAGCTG CCGGTTGACG AGGCGGATGC GGGCAATGAG CGTAGTGACG 427080

TGGGCGCTGG CGGATTCGGT CGTTCCGGCG GCGACCTTGA GCGGCGGCTG GCGCAATATA 427140
TCGAGCACAT GGGGGGGCGT CGAAGCGGCG GATGCGATGG CGCTTGAGAA GCTTGGCGAT 427200
CGTCGCCTCG CGGATGCGCG CGGCTTTGTC CGGCGTCGGC ACGGTGTCCC AGAGATCGAG 427260
CAACCACTCT GCCCCGAGGT CGTTTTCGAG CTCAAGCAGT GCGGGGAAGT AGCGCCAGAG 427320
CTGCTCGCGC ATACGGTTCG TCAAACGGTT GCGCTCGGCG CTGAGGTCTT CGGCGATGCG 427380
CGACCACTCG CGCAATTCGA TGACGACGGG GTCGGAGACC GCGAGCAGCC GAAAGCAACG 427440
CGGATCGGTG CGCAGGGCGG AAGCCATCAC CTCGGCATCG CGGCTGTCGT CCTTGGCACC 427500
GGCCATGGTG AAGCGGTCGC GGAAACGATC CATTTGCTTT GGATTAATGG CATTCACCTT 427560
GAACCCGCGC TCAATCAGCG TCTCGACCAC GGGGCCGTGC GGCACCTCGA TTGCGACCTG 427620
GATTTCGTCC CCCTCAACCG CGCCGCTCGT CGCCATCAGC CAGGCCGCCA TTTCGGCGAG 427680
CCCTTCGCCG CCGTGCCTGA AGACCCTTTC GCCGATTTTT CGGCCATCAC CGTCCGTGAG 427740
GAACACATGA TGGCTCTCCG ACGCCCAGTC GACGCCAGCA TAACAATGTC TTTGATCAGT 427800
CATGCCTTCT CCCCGTTCGA GCATACGAGC CACCGCGATC TTCGCCGATC CCTGTACTGG 427860
CGCTCGGAAA AGCACAGGTC CGGCGCGAAC TCCCCACGGG GCATCGATCA CGGCCAATCC 427920
GACGGGGCAC GTGTCCCCCC CAGGTGCTCG AGGCACAGGG GGCGATGGGT CGCTCCCGAC 427980
GAATCGGCTC GGTTCAGGAA GGCTACGCCA ATTCGGGCCG TCGCCCATGG TGCACGCCAC 428040
GTTCCGCCAC GCTTCACGTC GCGCGACCCA CGTCGAGCAT CCAAGAACCG CATGAGAAGG 428100
GTACAGGGGG TTGGTCAGCG TCCATCAGAA ATCATAGGAG GAAGCACGAC GTGCATGCAA 428160
TATCACAGCA TCCGAGTCAG CCCGGCAAGA CACAATCTCG ACGCAACGCT CCTCAAGCGG 428220
TTACAGGTGC GTGATGCAAA CGAATTCTGC TGGCTTTCTT GAAAGCGTAG ACCCAAAACT 428280
TCCGCCATGC CATGTCGTTC CTTGATCTAC CGGAGGGGCT GCCAGAACGG ATCATCCAGT 428340
GCAACTCTCC CTACACGGTC CGCTTCGGTG TACGGCTGCG CGGCCGCATG TACAGCTTCA 428400
TCGGCTGGCG ATCGGTGCGC GAGCACTGCG AACCAGTGAA GGGCGACATT CGATATGCGT 428460
CAAATGCTGA CGCAGAGGAG GTCGAGGCGC TTGCCGCACT AATGACGCTC AAATGCTCGC 428520
TTGTTGACGT GCCGTCGGT GGCTCCAAAG GGGCACTCAA GATCGACCCG CGAGGATGGA 428580
CCCCGCAGGA GCTCGAGCAC ATCACTCGAC GCTTTACGCA GGAGATGAAC AAGCGCCCCG 428640
ATCGGGCCAG GCGTCAATGT GTCGGCTCCG ATATCGGCAC CGGTGAGCGG GAAATGGCCT 428700
GGATGATGGA TGAGTTCCGC CGCGCCAATC CTACCGACGT GGTCACTTCG GGTGCTTGTG 428760
GCCGC[?]GGTAA AAAGGCGGGA TCGCCGGGCG AGCGGAGTCT ACGGGCAGGG 428820
GCGTTCAATT CGCGATCCAG AGCTCCCTTC GGGACACCCG CACGCCGGGT TTGGATGGCC 428880
GGCGCAATCT CAAAGGCGCC TCCACTGTTA TACAGGGGTT TGGCAATGTC GGCTATCACG 428940
CCGCCAGGTT TTTGTCCGAA GAGGATGATG CACGCGTGAC CGTTCTTGCT GAACGTGACG 429000
GGTATGTCGC CAATCCGGAA GGCCTCTCCA TTGAGGCCCT CAAGCAACAT CAGATCCGAA 429060
CAGGCAGCAT TCTCGGCTTT GATGGAGCCA AGTCCATTGC CGGCGATATG TGCGGCGTCG 429120
AACAGCCCTG CGACGTTCTG ATTCCAGCGG CGATGGAAAA TGCTATTCAT GCTGAAAACG 429180
CGGAGCGCAT GAAGGCACAT CTCGTCGTCG AAGCCGCCAA CGGACCTGTC ACCTTCGAAG 429240
CGGACGAGAT CCTTCGCTCC CGCGGCGTGA CCATTCTTCC CGATCTTTAT GTTAATGCCG 429300
GAGGTGTCGT CGTCAGTTAC TTCGAGCGGG TGAAGAACCT CACGCACATC CCCTTCGGGC 429360
TCATGGAGCG GCGGCGGCGG GAACGGGGCA ACCACACAAT CGCTACGGCG CTTGAACGGA 429420
TGACAGGCAA GGAGTCCCCC GCAGACATGC GCGACGAATT CCTCGAAGGC GGCGCCGAAA 429480
TCGACCTCGT TCGTTCCGGC CTGGAAGACG TCATGCGCAG CACCTGGACG CGCATTGCCG 429540
ACCTGATGGA ACAGCAACCG GAACTCGGTG ACTACAGAAC TGCCGCCTAT GTCGCGTCTA 429600
TCCGGCAGGT CGCTGATTTA TGAGGCGATT GGGATATGAC CGCGTATGGC GAAAACAGCG 429660
TGCGGCGCTA CCGACTACGC TTACTCTCAC GCATGGCTCC ACTCCCGGCG ATGCATGCCC 429720
AAGTGGCGAG TATCATCGGG GGATATCGCA CCTCTTCTTC GATGGGAGCG AACTCTACGG 429780
CCATCACAGT CGAGCGCAGA GAACATGTGT CTGGAGGCAA AGTGGCTTCA GCGCTACGGC 429840
GGGGGGTCAA TTCTTCGATT CGCTTGACAA AACCCACGAG AACTGGATGG AGGCCAACCG 429900
ATACATCGAC ATGGACGATC TCAGAGAGCA CAAGAAGCTG GCTCTCCGCA ACGCCGCATG 429960
ACCAGCCCCA TCGCCGCCCC AATTTGCTGA ACTTGACGCA CAGAACCTCA TTTTGACAAG 430020
GCGAGCTTCG GGGGTGCTGG TGCTTCTAGG GCTCGGCGCA TTGGTTTCGT TCCCGAGTTG 430080
CCGGCCAGTT GTCCGATAGC TTGGGTGCCG CGCGGGTCGG CCGAACGATC GCTATCCTTC 430140
CTGGTTTCGA TCGCCTTTTT TGGGCTTGCG CCGACAATAC CGCTGCTGGC GATCGCGCTT 430200
TGGCATGTGT CATGGTTCGA TGGACATGGC GATGAACAGA TGGGTGCGGA TTCCGACGAT 430260
CGCGCGCATG CATTCCAATC TGATGCCGCG CACCGTTCCG ATTTGATCGC GCGCATGTGA 430320
GGCGACCAAT CGTTGGGGGT TAAATGGGAC TACGAACTTA TCCTGGTCAA GCTGGCGCCG 430380
ATGAGATCGC TGTGGACGGC TGAAGACTGC CTCCAACGGA GCGAAGCAGT CCGGCGCCGC 430440
GCTTCCCAAC GCGCCGTAGG GGCCGGTCTG CTTCGCGGGC GGCGTGTCCG ACATTCTTTT 430500
GGCCAGGATA TTGTCCACGG GTTTGGGGTC CGGCCTTTCA GGTCTGGATT TCGGCCGGTT 430560
GACGCAGCCG ACGCATCGAT TCGCCGCTCA TTTCAAGCCT GTGAGCGTTG TGAACCAGGC 430620
GATCCAATAT TGCGTCGGCA TAGGTGGGGT CGCTGATTTT TTCGTGCCAT CGGGCTACGG 430680
GGAATTGGCT GGTGACGAGC GTCGAGCGTC GGCCGTATCG TCCCTCAAGG ATCTCGAGCA 430740
GATAATGGGG AGCATTACCG TCGATGGGCT GAAGCCCGTA ATCATCGAGG ATGAGAAGTT 430800
CGACGGCGCC GAGACTTTTC AGGCGCGTTG CGAGGCTGCC GATACCGCGG GCCTGTTCCA 430860
GGCTCTGCAG CAATGCCGGC ATCCGGACGT AAAGGACCGA ATGATTGTCG CGGCAGGCCT 430920
TGTGTCCCAA GGCACAGGCC AACCAGCTTT TCCCGATGCC GGTCGGGCCG ACAATGGCAC 430980
AGTTCTCGTG TTTTCTGATC CAGTCGCCGG CAATCAGCAG GTCGAGGATG GTGCGATCGT 431040
ATTCGCGCGG TGCACGGCGA ATGATATCTT CGGGAACAGC GGGATGGTGC AGTTTCGCGT 431100
TACGAAGTCG GAGCGCAAGA CGCTTGTTGT TGCGCCAGGT GGCTTCCTGA TCCAAGAGCA 431160
GCGCCAGCCA TTCGCCATGA CTGAGGTTGT CGCTCTCGTC ATTATCGGCC AGTCTTGTGA 431220

AGGCGTCTGC CATACCGGCA AGCCCCAATT GCTGCAGCAG GTTGAGGGTA GGATGTTTGA 431280
GCATTTTTAT TCCTTTCAAT GGTAGTATTC GGAGCCGCGG AGGTTGGAAT GCTGGATCGG 431340
TTCCTGCTCG AGGCGCTCGG ACCTTGGGAG GCGGTCGAGT TCCTTTTCGA GAATGGATTT 431400
GATGCCGGGA TAGTTGAGCA CCTGGAATTC CAGCGCGCGC TGCGCAGCCG CTTCAAGGCG 431460
ATCGACGCCA AACCGTCTTT CCAGGCCGAT GATGCCGACA CAGGCACGGT ATCCCTGTTC 431520
GGGATGCGCG CGCTTCTCAA TGATCCGATC GACGAGAACG CGCAGCATCG GCCCGATCCG 431580
CTTGGCCTCG CTGCGGATCT TCGCCGGCGT CCATTCGCGA TAACGCTGAT GGTTTGGCGG 431640
CATATGCACC GCAACCGTCG TGTGGCGGCC GTTGCTGCTG CCACGCACAT GCGCGGCAAT 431700
TCGGTCGCCG CCGAGGAATA TTTCCACCGT GCGAATGGTG TATCGAACCT CGACTTCGCT 431760
ACGCGCAAAG CGGTAAGGGA CGGAATAAAA ATGCCGACTG AGTTCAATAT GATAATCGAT 431820
GCCAACACGA CACCGACGCC ATTCCGCATG CACCCAGGGC TCGGCAGGTA GCGGCTTCAG 431880
ATTCGGGGCA TCAAGTTCGT CAAACAACGC GCGCCGCGTC TTGCCGTACT GGCGTATCAC 431940
CCGTTCGTCA TTGAGCCGGG TGATCAGATC GGCAATGGCC TTGTTGAGAT CAGCGAGGCT 432000
ATAAAAGGTC CGGTTCCGCA GCCGACCCAG AAGCCAACGC TCTACGATTC CGACGCAATT 432060
TTCGACTTTT GCCTTGTCCC TCGGTTTTCT CGGGCGTGCT GGAAAAACTG CCGTGCTGTA 432120
ATGGCGCGCC ATATCCGTAT AGGAACGATT GACCATCGGA TCGAAGTGAC AAGCCTTGAT 432180
CACCGCGCAT TTGGTGTTGT CGCTGACCAG GAGTTGCGTC ACCCCACCAA AAAACCGGTA 432240
GGCTGCGTTG TGCGCCTCGA TCCAGTCGGG CAACTGTTCG CTCCACGTCG CCAGCGCGAA 432300
CGACAGGCTG GACGCCCCCA TCACCGCCAC GAAGATATGT GCGTCCCGCA CCACGCCGGT 432360
TTTGCGGTCG ACGACTGCCA CCTTGTCACC GGCATAATCG ACAAACAGCT TCTCGCCGCC 432420
GGCATGGCTT TGCCGCATCA CCAGAGGCAG CCGGCCATGC CAGTTTCGGA ACAACTCACA 432480
GTATCTCGAA TATCGGAACC CGTCTGGATC CCGAGCAATG TACTCCTCCC AGAGCACCTG 432540
CAACGTCACA TGCTTGCGCT TCAATTCGCG CGCGACGGCA GACCAGTCGG GCTCGGACTG 432600
CTTCCGACGC CCAGCTTTCT TACCGGGCGT CCCGTAAAGG CGCTGCTCCA GCTCGCGGTC 432660
ACTGATCGTA TCCGGTAAAG GCCATGTCAG TCCTTCCCGG TGCAATCGCT TTAACGTGTC 432720
GCGGACCGAA GTCTCCCCGA CGCCCAGTTG CTTGGCAATG ATGCGGTTCG AAAGACCGAC 432780
ATCCAGCCGC AGTTTCAAAA TCTCGCGCAC CTGGCGCATC AGGAGTCTCC TCGTCGGCAA 432840
TTCACTTCTC CATGGCGTTG CTTCCACCTG GAGAAATAAC CCCAACGATC GGGCCTCACG 432900
ATATCGACTT ACTGCGCGCG ATTATCTCGG AATGCTGCGC GGAGAATTGT CGGAATGGTG 432960
CGCGTCATCA TCTCGGAACG GTGCGCGCGA TCAAATCGGA ATGGTGCGCG CGATGAGCCC 433020
GGATTCCGCA CAGATGGGCG AGCGAGGTGG AGAAACACAT GAGGCGGTCA GTGATGTCGT 433080
CGTTTCAGGC CATGTGGAGT GTGGGGGCTG TTCTGGGCGC AGCAAACAGG CCCCAGTATA 433140
CATTCATTTC CTTGTTACTG CCGTGATGAC CGGCGGGATC CTCGGACCCT TCCTTCTGCT 433200
CGATTGGCGA TCAACTATCC AGACATTCAT AGAAATACCG CTGGACCGTT GAGCAGGGCG 433260
GAAAGTCCTT TGGCAACATC CGCCATTGGC AGCCCGTTGA GGCGATATAG CAGCGCGTTC 433320
ACAACCTCGC GCAAATCGGT CTTGCGCGGG CGGCCGAGAC GGCGCGGCGC AGGCACGAAC 433380
GGTGCAATAT ATTCCCATTC GCGATCCGTG ACATCGCTTG CATATCAGCT CGTCCGACGG 433440
ATATAATTCC GCCGGGTGGT TTCAGTCCAA GCCATCGTAG GCTCCATCGA ATCTTCGCAA 433500
ATCCGACGGA ATCACAACTG ACTGAAATCA CTCAATTCTT TTTGGGCAGC CTCTTAGACC 433560
TCAATTCCCC ACGTCACAGG ATACTTGCCT TTGCAGCAGG CATGGTGGGT TCCTTCGTTA 433620
GCGGGTTGGG CAGGCTCCCG CGGGATCGAC TGCGCGGACG GGATCTCAGG TGGTTTTTCG 433680
ACCGGCAGGC GCACTGCGGC AGGTCGGTAT GCTTGCGAAT CACGCATCGG CGGCAAAGGG 433740
GAACGGATGC TCCAAGTCAA GATGGCAACA AGGCATCAAG CAGATCGGAG GCAACAGTGC 433800
TGTTGGAGGC ATTCGAGGGC ACAGGTTCAT CCACGCCGGG TCAACCCACG TTTGGCTGGC 433860
TCGGAAGCCA GTGTTGTATT CATGAGGTTT CTTTTCGCGC GGCATCATTC ACGATCTTCC 433920
TTGCCTCCAC GGTTACGGGT AGGCTGGTGT CTTCCGGAAG GTCGGGCAGT AAGAGCCGCC 433980
AGCCATTTCT GAGCACCACA AAGCCGCCCC ACAGGCTTTC ATTCTCAACC TTGACAATCG 434040
GCTCTTCGAT ATCTTTCTTG GGAACATACG CTGACAAGCC GCTGCCGCTT CTGCGGATCG 434100
TTACCCTCAT CTGGCTTTTC CTTCGTTGAC ACCCCTGGCG CATTCAGTGG TTTTTGGCGT 434160
TGAGGTCCCC CCGAGGCTCC TCAGTTCACG CGTACGCATT CCAACGACGA TGCCGCGATC 434220
AATCCATTCG ACGGCATAAA TGTAGAACTG CTGGAGAAAT GTCCCTACAT CGCGCACAAA 434280
ACCCGCGTCG CCTTTCCTGA CGAGGCGTTC TCCGATTTCC TTCCCGGGAT AGGTGCCGTC 434340
ATTTTTTATG TGGCGTGTGG CACAAACTCT CTCACCCGGC ATAAATCGTG GAGGATTGTG 434400
GATTTCGACT TCCTCTTCGC GTCCAATACT CATTACCATT CCTCTTGTGG TTGCGATTAC 434460
CAGGGGGAGA CTCAAGGTCC GGCCGCGCAC GGAAGCCCGG CTATGGCATT CGGGGCTTAA 434520
GCCGGCACGC AGGTCTTCGG CATGGGACAT ACAGAGGCGC ATTGCTGTAT CTCGAAAACG 434580
CCCTTGCATT CGGTGCATTT CTTCGGATCT ATCACGTACT TTTCGCCCTT TAACTCGATA 434640
GCGTTGGAAG GGCATTCGAA CTCGCAGGCG CCGCACTGGG TGCACTGGGA TGCGATGATT 434700
TTGAAGGCCA TATTATTCAG CTCCTGGTTA GGTCTCGATC GATGGGCCAA GCTCAAGCCG 434760
TTGCCTGCGA TGGGGCGGTA CCAAACTCTG TCGCGTGAAG AGCGCTGATC GCGGCCTCGA 434820
TGTAGTCATA GCCATAGGTG TCCGTTGCCC GGATCCCAGC TTCCATGAGC CTCTCCTTCG 434880
GACAATCTCC TATCCTTGCG CAGAGCACGA TGTCTATGCC GTCGAGTGCA GCGATGATGC 434940
CAGCGAGGGC GGCGTCCTCG CCCCAGCCGC CATGGCAATA CGGCTCGACC TTGCGGTGCC 435000
CCACGAATTT AATCCCTGTT TGCGAGGCTT CGTAAACCTG GAACTCCTTC GCATGGCCGA 435060
AGTGTTCATT GATGCGTCCG CCGCCTTTCG TTGCGACAGC CACCTGAAGC GATCCACGGA 435120
TGTTCGCAGA TGCGACCGTT GCGATCGCTT TCTCCTTAGC CTCTAAATGG TCATCGCGTA 435180
TGCGTGCGAC CACCTCCCGG TAGGCCTGAC GCTTGGCACC GTCGTAATGG GGTTCGAGCG 435240
GAACGTGGTC GAGCGTGAAC TCCTGGCCAC GGTCAGTGCC GAGAAGGCCG ACTGCATCGG 435300
CACGGCACTG TCGACAGTGA CGCATCAGCT TGGCACCGCC TTCAAGACGG TCGTGAAGCG 435360

CCTTCAGTTC GAGCGCATTT GGGCCGCGCT GTCCGGTCAG GCCAAAGGAG GTTCCGTGTG 435420
CCGGGTCTGA AATCAGCGGC ATGACATTGT GCAGAAACGC GCCGCGCTCC TTGACCCATT 435480
TGTTGACCTC GATCAGGTGC AGGTCATTGA CGCCGGGGAT CATAACCGAA TTGATCTTGG 435540
TGAGGATGCC GCGTGCGGTC AGCATCTCAA GTCCCAACAT CTGCTGCTCA TGCAGGATTT 435600
TGGCAGCATC CACGCCGGTG TAGCGGCGAT GCCCATAAAA GATCCACGGG TAGATCTTCT 435660
CGCCGACGGC GGGGTCGACC ATGTTGATGG TGATTGTCAC GTGGCTGACG TTCATTTCCG 435720
CCAACTTGTC GACATGCTCC GGTAGAGCAA GTCCATTGGT GGAAATGCAC AGCTTGATGT 435780
CCGGAATCTC GCTGGCGACG CGTTCGAAAG TCGCCCTTGT CTTGTTCCAG TCGTAGCAAG 435840
CATCGCCCGG GCCCGCGATG CCGAGAACTG AAAGCTGCGG CACTTCGTTG GCGACCGCAA 435900
CCACCTTGCG CAGCGCCTGG TCGGGTGTCA GCTTTTCGGA TACGACGCCA GGCCGACTTT 435960
CGTTGGCGCA GTCATATTTG CGATTGCAGT AATTGCATTG GATGTTGCAG GCTGGTGCGA 436020
CTGCGACGTG CATACGGGCG AAATAGTGGT GCGCTTCCTC TGAAAAGCAA GGGTGATCCT 436080
TGATCTTCTC CCAGGTGGCA GAATCCATGT CGGTTGGCCT TGAGGACGAG CCGCAGGACG 436140
AGGACGCGCA ACCACCGGAT TTTGCGGTTG TCAGCAACTG CCCAGAGGAT GTCGTGTTCG 436200
TCAGGCTTTC AAGCGAAATT ATCGGTGCAC TCATCAAACG GCTCCACTGG AGTAAAAAGT 436260
CGTCGTCCAA AAAAGCAAGC GCTGTGCCAA CAGGATAATA TCGAAATATC AATAGTTTGG 436320
CGCTTCGTGT TCGATTTGAA CCAATAGCCC CCGACGCAGT TTTGTCGAAT TCGCGACAGA 436380
AGCGGCCAAA TTGGACGATA CACCCGCGCG CCTGAGGAGG AGGGGAATGG CCGAGCCCAG 436440
ATAAGAGGGC TCACCGTCTC TAGATTTTTT TCACCTGTAT ATGATGCCGC CGTAAAGCAT 436500
AGCCGACTTG CCGCGGTGTG AGGCCGAGGA TCCGAGCTGC CTTCGCCTGA ACCCAGCCGG 436560
CCTTCTCCAT CGCGCTGATT AGCAGCTCGC GGTCCGATAG ATGTGGTGCC ACCGTTAAGC 436620
CGCCAGGGCC GGCGGGGTAA CCGAGACTGG CCGGCGATTC GACTGGCAAA CGTTTGCTCA 436680
TGGCCAATCC ATTGAGCGAA TGGCTGCCAA GCGGTCTGTC AGCAGTTTTC CCGAGCATTG 436740
AAGAAAAGCA CTGGTCATTC TGGCAGGAGA AATCTGATGG AACAATCGTC GCCGAACGTG 436800
CGAGAGTGGC GGTCCTGCGG ACGCAGTTTT CAAGCTCTCG GACGTTGCCG GGAAATTTGC 436860
ATCTTGACAG GATCTCGAGT GCCGCAGGCG TGAATTCGAG ATTGCGATTG TTTTCCCTGT 436920
TGAACCGGTC GAGGAAAACC TTCGCAAGGC GTGGAATATC TCCATTTCGC TCCCTGAGAG 436980
GCGGTAAAAA GAGAGGCACT ACATTGATGC GATAATAAAG GTCAGCCCGG AACTCCCCCT 437040
CCGCCACCGC CGTCTCCAGG TCCTTGTTGG TGGCGCATAT GAGTCGGACG TCCACCTTGA 437100
GCGTTTTTGT GCCGCCAACT CGCTCAAGCT CACCTTCCTG CAAGACGCGC AACAGTTTCG 437160
CTTGAAAGGC CGGCGAGATC TCGCCGATTT CGTCGAGCAA CAGCGTCCCG CCATCCGCCA 437220
GTTCGAAACG GCCTGCGCGC TGAGAGATGG CCCCCGTAAA GGCGCCCTTC TCATGCCCAA 437280
ACAGTTCCGA TTCCAGGACG CCTGCAGACA GTGCCGCGCA ATTCAACTTC ACAAACGGCT 437340
TTTTTCTGCG AGGTGAAAGC TCGTGGATTG CCTTTGCAAA GAATTCCTTG CCTGTGCCGC 437400
TTTCGCCCCT GAGAAGCACC GCAGAGTTGG TTTGTGCAAC GACTTTGACG CTTTCAACCA 437460
CTTGCCTGAG TGCGGGGCTT TCCCCGACAA TCCAGTCGAT TCTCGTAGGC CGTTGTCGGG 437520
TTGGTTCAGG CAGTTCCTTG TCGCCTCCAC TAATCTTGCG ATCGCCCTCC TCGCCGATCG 437580
GTCGACTATC GCGGCTCTCG TGGCCATTCA GCCGAACCGC TCGTGCCGCA AGGTTGGCGA 437640
CCATCGACAG GAAACGCACC TCTTCCTCAA ATTGTGTTCT GGTGGCTGCG CCATCCTTGG 437700
CGCGGTCGAT CCATAATGTT CCAAGCGTTT CTTGATCGGC CTTCATCGGG ACACCAATGA 437760
AGGTGACTTG GCCAGTGCCG CTCACGATAC TCTGAAGCTC ATCCTGGAAC AGTTCCGACT 437820
TGCAAGTGTC CGGTACGACC AGCGGCGCAC CTTTTGCGAC GATTCTATCT ATTGCAGCCT 437880
TCGCAGCAAT GTGGCGCGCG CCTGATGATG GAGGCTCAAC CCCGAACGTC GCAGTTGTCT 437940
CGGGCTCTCC TTCAGCGCCA ACAACGACGA TTGCGCCGCG CCGCATTGGC AAAAGTGCGG 438000
GGAGGGTGTT AATCACATTG GCAAGCTTGA TCTCGAGGCT GGCTGAAGTG ATCAAAACCT 438060
TCGATATCCG ATAGAGACCG CTGCACCCCA AGTCCCGTTT GTGCATGGTT TTAGGGGATA 438120
ATTCAGTCAT ACACTCATCG AGCCAATTCT TACGAGGAAA CACCTTGAGC GTCGTACGAA 438180
CGGCCCTTGG CTCCCTACGA GAAAATGCCG CCTTGCTGAC ATTATCAAGT GCTTGCGCTT 438240
GCACCTGTGA GCGCCCGTCA ACGTACCCAT CTTGTTAGAC AAAAGTGAGA TGCAAGTCAT 438300
CCGAATTTAA AGAGCACACC GAATCCACCT CGCGGATAGC TCCAATCAAT GTCACCGCTC 438360
TCTTCGCACA GTACTCTACA TGTCCCGCAT TCCATGCATC CATCCGCAAT AACCTCTACT 438420
TGGCCGTCTT CATTCACCTC GTAGCATTTC GCTGGGCAAA CAACGGTCAG CGCAAGCAGG 438480
TTTGGGCTCG GAGACTGATG CTGCCGCACC TTGATGTGCG GCCGCCCCGC GTCCACCAGG 438540
TAACGGTTTT GGTAAAGCTT GTCTTCAATG CGAACGACTG AAGCTGTCAT CTCAATGCTC 438600
CCCTTTAGCG CCATGCCAGT GCCAAGCGGA CTGCGTCGCT GACCAGTCCC CATCGCGAAC 438660
GCGCTTTAAT AAGAGCGGCC GTGGTCGCTC TTTCCTTCTC GATCTTGGGT GTGCCGTCGA 438720
CGCGCACGAA GTTTTGCGCC GCCTGCGACA TCAACTGCGG ATAAGTCGTG AAGAAATTGT 438780
GTGAATTCGT GTGGAGCAGA GCGGGCATGT CCTTGTACTT TCTGAGGTCT TTCAGAACGA 438840
AAGACTTGTC CAGCATGGCC TTGTAAAGTG AGAGATTGTC ACTCGTCATC GGGTGTTTTC 438900
GGCTCTTGAT TGCGGCGATC GCCTCGCCGG CGATGCGGCC CGACGTCATG GCGAGGTTCG 438960
AGCCCTCGCG GTGCACGGCG TTGTTGAGCT GCGCCGCGTC GCCAACCACG ACCCAGCCAT 439020
CGCCAAAGAG CTGCGGAATT GCCTTGAATC CGCCCTCCGG AATGAGGTGC GCCGCATATT 439080
CCTTAATCTC CGAGCCCGCA AGTAGCGGCC GGATTGATGG GTGGTTCTTG AATGCGTCAA 439140
GTAGCGCGTA AGGACTTTCC AGGGTCGCTG CGAAATGGGA GACAAGGCAG CCGATGCCAA 439200
GAGAGATCGA TTCCTTGTTG GTGTAGAGGA ATCCCAAGCC GGCCATTCCG CGCGAGATTG 439260
TCCCTGCCGC CTCAATAACA CAGCCTTGAT CGCCGGTAAG GCCGAAGCGT TCATTGATGA 439320
CCTCTTCCGG TAGGAAATGC ATTTCCTTGA CAGCGAGTGC CACGGCTTCG GGTTTCGGCA 439380
CCTTGCGAAA TCGTGCTCTC GTGCCGAGGA GGCCGTTTAC GCCTTCGGCG AGCACGACCA 439440
CGTCCGCATG GATGACCCCG CCCGCTCGAT CGGTATGGAC GCCTATCACC TTGCCGGCAG 439500

CATCAAGAGC CAGTTCCGTC ACCGTCGTCT CGCACAAAAC TATCGCGCCC GCCGCTCGGA 439560
CTTTCCGCGA AAACCACTTG TCGAACTGGG CGCGAATGAT CGTGTAACGG TTTGGCGCCG 439620
GCTCATTGAA GTCGTCCGAC CGGTAGTGCA TTCCGGTGTG TGACGTGTCG TCCGTTACCC 439680
AGAATCGCTG CTCGACCAGA TGCCGCTCAA GAGGTGCATC ATCCCGGAAA TCGGGAATGA 439740
TTGTCTCCAG CATGTTGGCG TACAAGATGG CGCCTTGGAC GTTCTTGGAG CCCGGATATT 439800
CTCCGCGCTC CAACTGCAAC ACTTTTAGAC CGCGACTTGC CATGGTGTAT GCAGCCGCGT 439860
TTCCAGACAT ACCGGCACCG ACGACGATGG CGTCGAATCT TTCTTCGGTC ATAGGTTTTA 439920
CCTCAGTTCA CAACTCTCAG ACGATTGGCC GGCGCCAACC GTTTGGTGAA AGCTTCCGTC 439980
AACGCCGGCA GAAAGCTGAC AGCATCGGTG ACAACACCGA CATGGGCGAA ATCGAAGATG 440040
GGCGCGTGTG GATCGGTGTT GATAGCGACA ATCAGGTCAG CTCCCTCGAC ACCGACGCGG 440100
TGCTGGACCG CGCCTGATAT TCCAGCCGCT ATATAGAGCT TCGGGCGGAT CGTTTTGCCA 440160
GTTTGGCCGA TCTGCCGATC AGCTGGCATC CAGCCCTTTT GGACGAGCGG CCTGGAACAG 440220
CCGTAGTCAC CGCCGATCGT CAGCGCGAGG TCTTTGATAA GCAGCAGGTT CTCTGCATTG 440280
CTGAGGCCCA GCCCGCCGGC AACCACCACA TCCGCATAGG CGAGATTGGC GTTGCCCGAT 440340
CGGTCGTCGG AAAGGAAACC GACGACCTTC GTGACGATAT CTTCCTCCAA CATCCGCCAA 440400
TCGTGCCGGA TTACCCGCCC GATCGGCTTG TTTGACCGCT GTGGCATGCG CATGACCCTT 440460
GGCCGCACAG TTGCCATTTG GGGCCGGCAA TTGAGCGTAT AAATCGTGCA AAGCAACGAG 440520
CCACCGAACG TGGGCCGAGT AGCTGCAAGC GAACCGTCCT CGTCGACATC AAGTCCGGTG 440580
CAGTCGGCTG TGAGCCCCGT CAGCAGTGTG GTCGCCACGG AACCGGCGAG GTCGCGACCC 440640
AGCGTCGTCG CACCGAGAAG CAGAATCTCA GGCTTATGGG TAATGACCAG ATCTGTCAGT 440700
GCTTTGGTGA AAGGCTCGTT TCTATAATCC GTGAGCAGCG GCTCCTCGAC GAGATACACC 440760
AGATCCGCAC CATAGGCGAA GGCCTCAGCA GCGGCGTGCA AGGTAGAATC CCCCGGAGGC 440820
CCGAGAATAA TACCTGCGAG CTGAACCCCC AGTTGGTCGG CAAGTCTGCG GCCTTCGCCG 440880
AGAAGTTCGA ACGAGACCGG GTGGACATGG CCGCGTTCCA GCTCCATGAA AACCCAGACG 440940
TGCCGGTAGT CTCTGAATTG CTCAGGCAGG TCCTTTTTCA TGCTGGCACG GGCTTGGGCC 441000
GGTGGTGATG CTTTACTTTT TCGAGTTCCC ATAATCTGCT CCTATCTGCT CACGCTACGA 441060
GTGGAGGAGA GCTCGTGCTC CAGTGCCGGC TGATGGGCGA AGATTGAAGT GACCAGTTCG 441120
TTAGCTACGT CCTGCATTGC CTTCTCGACG GTGTCGATCT GCACCGCTTT TTGCTCGCGC 441180
GGGGTAGGGG CAAAGACCCG CTTGACGACC GTCGGCGAAC CGCGCAGCCC GCATTTTGTG 441240
ATGTCTTCAA TGCCAGCCTC CACCGCATTC CACTTGACGA TTTCGCTGCG CGCGGCCCGC 441300
AAGGCATCAT CAAGTGAGCC GCGGCGAATG GCATTCGATC CTTCCAGCAT GGTGATCAGG 441360
CAAGGCAATT CGCTCTTCAG GACGTGCGTC CCGCCTTCCG CGCGACGCTC GACCGTAATC 441420
GCGCGCGTGC GGAGATCAAT GGAAGTGACC TTTGCGACAT AGGTCAACTG CACGAGGTCG 441480
AGGCGCTTGG CTATTCCGGG GCCGACCTGG GCGGTATCGC CGTCGATCGT CTGCTTGCCT 441540
GTGAAGACGA TATCAGGCGA TCCAAAGCTA TTGCCAATCT TCGCGATTGC CTGAGAAAGA 441600
GCAAATGAAG TCGCCAGCGT GTCGGAGCCG GCAAAGTGCC GGTCGGTCAG GAGTATCGCG 441660
CGGTCTGCGC CATAAGTGAG CGCCTTGCGT AAAGATTCTT CGGCCATGGG GGGGCCCATG 441720
GTGAGCACCG TGACCTCGCC GCCATGGTGG TCACGCACTT GGAGTGCCTG TTCGAGGGCA 441780
AACAGGTCGT AAGGGTTGAT GATAGTCGGC ACGCCTTGGC GCATGATCGT GTTCGTCACC 441840
GGATGGACGC GTATCTGCGC CGAATCCGGG ACTTGCTTGA TACAGACTAC GATGTGCATG 441900
AGGTCTCTTT AGCTCCGATC GCGTGTAAAA TGACGTCTTT CGCCTCCCTT CCAAGCACCA 441960
ACCATGCCAA CCAACTGTGG TCCAAGAAGC CCATGAATTC TAAGGAGCTT TTTCGGCTCA 442020
CAGGATGCGA CCTGCGTGAT AGCGTTGTCG GCTTCATGAC ATCGAATCGT CGCAACCGCT 442080
CCGTACTTGT CGTTTTCCGA TGATCTTAGG CCGAGTCCCA ATGACAAATG TGCGGTTAGG 442140
CGCACCGCTG ATCCGACGTG AAAACGGCGA AGTGGTTGCA AACTCCGCGC TTTATGTAAA 442200
GTGCCCCGAA GCTCTTCGGC CCATGGCACT TGTGCGCGGA AAGCGACAGC ATGAAGTGTC 442260
TTGCTGATCC TTCTCGTGGA TGACCTAGCA TTCTCGTTCC CCTCTGTTTC TGCGATCAGC 442320
CGAAAGACTT CCCGCAGGCG CAGTTCTCTC GTGCGTTGGG GTTATCAAAG ATGAATCCGG 442380
CGGAATCGAC ACCCGTGGTG AAGTCCACGG TCATGCCGCT GACATGAGGT TGAGAGGCTG 442440
AGTCTACGTA CACCTTGACC CCACCCGCCT CAATGACAGC GTCGCCGTCG CGCGACTCGC 442500
TGTCCAAGCC CAGGTGGTAT TTAAAACCCG AGCAGCCGCC CGCTTCCACT TTGATGCGTA 442560
GCCCATCCGC CGGCTCGCAA GTCTGTGAAA GCGCGAACTT TATTGCGGCA ATCGCACTGT 442620
CGGTGAGCGT TATCATGGGT TCGTTTCTCC TGGTTTCGAG TCGCTTGCGA ACGATGCACC 442680
TAGTGTGCCA ACCAGAAACC ATCACGAAAA CAACGCGTTG TTCAAACACG GCCATGTCGT 442740
TTGTACGACA CAGTCGTAGT TACGACATTT TGGGTGATTA AACCGAGTTC GTGTGAGGCT 442800
CTCCGCGACG CGCCGAGCAT CGACGTTTAT CCTCGGCAGG GGCTGCTGAG ACTCCGGCGC 442860
GGAGATTGGA TACCCGGTCG ATTGAAGGAA ACTAAACCCT CAGCGGGAAT GTGCCGTACC 442920
GTTCCGTGCC GCAGCATGAA CTGCTCCGGC GCAATACCTT CATGTCGGTG TACATAGGCC 442980
AAATGCTCCT CTTGCGAACG CGAGTTCGAT CACGACATCA ATTGTGATGA CAGACTTGCT 443040
CATGGGAACT CGCCTTTAAC TTCGGTTCCG TGCCCGAAGA GCGGCATAGC AGGAAGCAGG 443100
ATCTCGGTGA AATCGATTAT CTCGATGGCA CGCATTAGCT CAATGGATCG AGGGATCGTC 443160
CGCGGAGCGG ACCGGCAAGT CACCTTGAGC GGCGATTTTC CTGCTGCGCG GGGTTCATGA 443220
AAGTGCGCTG GAGGCTGCTC AGTGGGTAGC TGCGGCGCTC ACTGTCAGCA CCGGCTCCGC 443280
AACATCAGAC TGGATAGCTC ACATGGCAAA AATCAAAATT TAATCGGGAC ACGACCGAGA 443340
CGTGCGGTGT CGAAAGTCCT CAGCGTTTCA AGAATGTTCT GCGGAGAAGA TGCCCAATAA 443400
GGATCCGACT CGCAGTTGTC CGAGAAACCT TCTTCTTCGA ACCATTGCTC CACCACGCTA 443460
TCATTGACCA CGGCAGCGTA GCGCCACGAG CGCATTCCGA AGCCGAGATT GTCCTTTGCA 443520
ACCAGCATGC CCATCTTGCG AGTGAATTCG CCCGAACCAT CCGGGATAAG CCTGACCTTC 443580
TCTAAGCCCA ACGCCTTGCC CCATGCGTTC ATGACGAAGG CGTCATTAAC TGACAGGCAG 443640

TAGACCGCTT CGATTCCTAC CTTCCCGAAT TCGTCGTAAA GTCTCTCAAA GTCAGGCAAT 443700
TGCTGAGTTG AGCAGGTCGG GGTGAACGCA CCGGGAAGCG AAAACAGAAC GACGCGCTTG 443760
CCACTGAAGT AATCCTCGGT TGTCCTGACT TCCCATCGGT ATGGGTTTGG CCCTCCTATT 443820
GTCTCGTCGC GAACACGCGT GCGAAAGGCA ACGAAAGGGA CTCTTTTTTT CACAGGCATT 443880
GAACATCGCT CCTTCAAAAA AGAACTGAGC ATTCGCCGCC TCTGGACCCT CTGGCGAAGC 443940
ACCGTTACTT AAATAATCAG TTCGTCGGCC AACGAACAGA CAGCAAGCGG CGTGCCACTT 444000
TAGATCCAAG CTTCAACAGC ACTGCTTTTG GTACGGGCAT GGCAATCTTG TGCGCGGACT 444060
GGCGCACCTT CTCGTGCGTT TCCGGTTAAG AAACTCGACC GCTGCTCTTG TCAGAGAGCC 444120
GACATCATGT CGCCTTTGTC GTCCTTCCGA CACGCCGCAT TTCCCCCTGC TTCCTACAGA 444180
CCTCCGTTTA GCCTTTGAAT GGAACGCACG AACTTAACCC TTCGGCTCAG CAGACTGGAT 444240
TGGCACGAAT TTTGAAACGC CCCCGGGGGG CACAATTGCC GTGCAGGCAT CTCTGGGGCG 444300
CCCTCGTGTG GAACGAAAGC AGGGAAGGAT GGGAACATGT CAAATCTAGC TCAGGTGCGC 444360
GCTTTGTCGA AAGGGAGAGT AACACTCGGA ATCAGAATGA CCGATCGGCC CGGTTGGCGA 444420
CAGTTATCCG AATTGCTGGA CCTCGGACCC TGGCCGCCTA CGGACCTCGA AATGGATTTC 444480
GATCAGTACG TGTTTGCATG TGTCCTTTCG CGTGCACTCG AGGAAATCGA TGCCGGCGAG 444540
GCAACTGCAA CAGAGGCAAC TGGTCTTTCG CAAGTAGAGC TGCGGGACAT CCTCAACCGC 444600
AGTTTTCCCG CCCCAACTAT TCATGTTTTT CGCTTGGAAG AGGTGAGAGA TTCGGAGCCC 444660
GGCCCCGAGG AGGCACTTCT GCGCGGTCTG CTGCTCGCGC ATGCTCGAGC GGGCGATTCG 444720
GCGAGCGTGC TGTTCGCCAA AATCATAGCC CGGCGTGCCT TGCGCCACGA CCATCTCTGG 444780
AGGGAGCTTG GTCTTTTTGA TCGAGTCGAA CTCAGCCGAT TGCTCGCCAG CCATTTCCCA 444840
ACGCTTGCAG CCGGCAATAC CCAAAACATG CGGTGGAAGA AGTACTTTTA TCGCAAGCTG 444900
TGTGAGGCCG AGGGCTATTC GCTGTGCACG GCGCCTTCTT GCCCGCAATG CAAGGAATTG 444960
GAGAGCTGCT TTGGCTCTGG GGAAGGCGAA AGTGCTTCAC CAGTCGGGAA AGTTGGCGGA 445020
GAGGCTTGAG CTCAAGCGCC GCCTAGGTCG GCTATAGACG GCTGGGAGGC CTTTTCGTCA 445080
TCTCTGCATG CGCGGATTAC GAGTGTGCAT GCACCAAGTG GAGGAAATCA TCTTGATCGT 445140
AGAAAACTTG GCGGAGGTCC GCGGCAAGAC ACCCCATTAT AGACATCTAT ACGTCCAGGT 445200
CCTCGCGGCG ATCGCCGTGG GCATCCTGCT CGGGTATTTC TATCCGGATG TCGGCTCCAA 445260
GATGAAGCCG CTCGGCGACG CCTTCATCAT GCTCGTCAAG ATGATCATCG CGCCGGTGAT 445320
CTTCCTGACG GTCGCGACCG GCATTGCCGG CATGACCGAT CTCGCCAAGG TAGGCCGTGT 445380
CGCCGGCAAG GCGATGATCT ACTTCCTGAC CTTTTCCACC CTCGCGCTCC TCGTCGGCCT 445440
CGTGGTCGCC AATGTCGTGC AGCCGGGTGC CGGCATGCAC ATCGACCCGG CTTCGCTCGA 445500
TGCAAAGGCG ATCGCCACCT ATGCGGAAAA GGCGCATGAG CAGTCGGTCA CCGGCTTCCT 445560
CATGAACATC ATCCCGACGA CGCTTGTCGG CGCCTTTGCC GAGGGCGACA TCCTCCAGGT 445620
GCTGTTCATC TCGGTGCTGT TCGGCATCTC GCTCGCGATC GTCGGCAAGA AGGCCGAGGC 445680
CGTCGTCGAT TTCCTGCACG CGCTGACGTT GCCGATCTTC CGGCTGGTGG CAATCCTGAT 445740
GAAGGCCGCC CCGATCGGCG CCTTCGGTGC TATGGCGTTC ACCATCGGCA AGTACGGCGT 445800
GGCATCCATT GCCAATCTCG CGATGCTGAT CGGCACCTTC TATCTCACCT CGTTCCTGTT 445860
CGTCTTCATG GTGCTCGGCG CGGTCGCACG CTACAACGGC TTCTCGATCG TCGCGCTCAT 445920
CCGCTACATC AAGGAAGAAC TGCTGCTCGT GCTCGGGACG TCCTCCTCGG AAGCGGCGCT 445980
CCCGGGGCTG ATGAACAAGA TGGAGAAGGC AGGCTGCAAG CGCTCGGTCG TCGGCCTCGT 446040
CATTCCGACC GGCTACTCCT TCAACCTGGA CGGCACCAAC ATCTACATGA CGCTCGCGGC 446100
GCTGTTCATC GCCCAGGCGA CCGATACGCC AATCTCCTAC GGCGATCAGA TCCTGCTGCT 446160
CCTCATCGCC ATGCTGAGTT CGAAGGGGGC AGCTGGCATC ACCGGCGCTG GCTTCATCAC 446220
GCTTGCCGCA ACCCTCTCCG CGGTTCCCTC CGTGCCGGTC GCCGGCATGG CGCTGATCCT 446280
CGGCATCGAC CGCTTCATGT CCGAGTGCCG GGCAATTACC AACATAATCG GCAATGCGGT 446340
CGCAACGATT GTGGTGGCGA AGTGGGAAGG CGAGCTTGCC CCGGCGCAGC TTGCAACCAC 446400
CCTTGCAGGC AAGGCGCCGG TGGAGACCAT GTCGGGGTTG TCAAGCCAGC GGAGTGACAC 446460
TGTTGAACTC GGACAAAAAG TGCTGTTTGG TGCAACCAAT TCCGCAGATC GTACTCTTGC 446520
CGGTCGCCCA GGGGGGCGCG ATTCCCGTCG AATTGCTCCC GATCATTCCG CTCAGGTCTT 446580
CGGCGGTCCG CTAAGCTTAT GAGTGACTTA AGGAGAAAGC GAGTGAAGAC CAACCCCATC 446640
CCGGATCATG TTCCGCCCGC ACTCGTGCGG CACTTCAGTC TCTTCACGTC GCCTGGCATG 446700
GCGCCGACGC CTAACGGGGA TCCGCACGCC GCCGTGGCTT GCGTTCATGA TGATGGCCCG 446760
CCGATCTTTT ACTCTCCTTC CAACACGCGC GATGGACGAG GTACCTGGGT GATAACACGG 446820
GCGAGAGACC AGCGCCGGGT CCTTGAGGAT ACAGAGACCT TTTCCAGCCA TCGCAGCATC 446880
TTCGCCTCGG CACTTGGCGA ACACTGGCCG GTCATCCCCC TCGAATTGGA CCCCCCGGCC 446940
CATGGAGTGT TTCGTGCACT GCTAAATCCT CTGTTCTCAT CCAGGCGGGT GTTGGCGCTG 447000
GAGCCGACTA TCCATGCCAG AGCAGGCGCG CTGATCGACT GCATTGCGAA AGAGAAGACC 447060
AGCTGTGACG TGATGAAGGA TTTCGCCTTG CCCTTTACGT TCAGCGTTTT CCTCAGCTTT 447120
CTGGGACTTT CCCAGAGGCG ATCCGAAGTA CTTGTCGGCT GGGTAAGCGA TTTGCTCCAC 447180
GGCAACGCAG AAAAGCGAAG GGCAGCAGCC CGCTCGGTCG TGGCCTTTAT TGACGAAATG 447240
GCAGCGATGC GCCGCAAGTC GCCAGCTGTC GATTTCATGA CCTTCGTCGT TCAGGCGAAA 447300
ATCGAGGGCC GCTCCTTAAC AGAAGAGGAA GTCCGCGGCA TCGGTGTGCT TTTCTTGGTC 447360
GCGGGGCTCG ACACGGTTGC CGCCGCCATT GGCTTTGACA TGGCCTATCT TGCACGCAAT 447420
CCAAAGCACC AGGAGTTGTT GCGAAACGAA CCGGCTCGGC TCGGGCTCGC CGCTGAGGAA 447480
CTGCTGCGCG CCTATTCAAC CGTTCAGATA ATCCGCGTGG CCACGAAGGA CATCGAGTTC 447540
GAAGGCGTGC CTATCCGTGA GGGTGATTAT GTTTCCTGTC CCGCGATGAT TGCTAATCGA 447600
GATCCGTCTG AATTTAAGTG CCCCAACACT ATCGATCTGG CACGACAGGA TAACCAGCAC 447660
ACCGCCTTTG GCTATGGACC ACACCTTTGC CACGGAGCGC ATCTCGCACG GCGAGAAATC 447720
GTCATTGGCC TACGTGAATG GTTAGCGCGC ATCCCAGCTT TCCGCATCAA GGAAGGTACG 447780

GCGCCGATAA CTCATGGCGG CCATGTATTC GGGATCAGTA ATATCATCCT AACTTGGGCC 447840
TGATTGGACG GGCGAGGCGG TAGCAATGAA GCGCCGATCG CTCCCAGGTC TTCATCGCTT 447900
ACCGCATCTC ACCGCACCAT GATGAATGAA GAAAGCATAA CCAATCTATT CGACCGAGAA 447960
GCAATCCGCG ACTGTCTCCA TCGCTACTGT CGAGGGATCG ACCGGGCAGA CGAGGCTGCA 448020
CTGCGCAGCT GTTATTGGCC CGATGCGCGT GACAATCACG GAAGCTACTC CGGCTCTGCC 448080
GCAGGGTTTA TTCAGCATGC ACTCAACGTC TTCAAAACTA AGCCGCGCAA CATCCACCAG 448140
CTTACGAACA TAATCATTGA GTTCCTGGGC GCGACAGAGG CCGCCGTCGA GAGTTATTTC 448200
ACCGCGCTGC AGCGCGGTCA CGACGCAACA GGCGAGATAA AGCAGGTGCT CTTGTGCGGC 448260
CGCTACTGTG ATCTGTTTCA GAAAAGGGAA GGGGAGTGGC GCATCGTCGA ACGGACGGTG 448320
GTCTATGATT GGGTTGAGAA ACAGAGACCA CCGAAGTCCT CGGAAGCAGA GCGGTTCGGT 448380
CCTCGCCAAC CGATTGGGGC ACCGCATCCG AATGATCCCG TTTACGAACT TTCGAAGCGG 448440
CGAGTTGCTG CGAAAAATCA TCCAAAGGAC CTGATCGGCA GTGGAGAAGA AGAACCATGA 448500
TACGACTGCG CTACCCTCCG ATTCCTCGAG GCCGAGCCCG CGGAGCAAAG AGACGCATGG 448560
ACGATCGCCA GGTTCAGCCA GGGCGGGTCA TCGTCGTCAC AGGTGCCGCA GGGGGTATCG 448620
GCCGCGCGCT CGTCGATATT TTTGCCGCGA ATGGAGATGT CGTTGTCGCG GTGGACCTCC 448680
CGGACAGCGG CGTAATCGAA CTTGGCCAAA ATCTCGGAGA GCCCCATCTT GGCCTCGAGG 448740
TTGACGTGTC GCGAGAGGAC GATGTCGTCG CATTGCGTGC CCTTCTGGAA AAGCGATTCT 448800
CGCGGATTGA AGTGCTCGTC AATAATGCGG GGATTGGGCC GACCATGGCT GCGACCGCCG 448860
ATACTGCTCT CGAAGATTTT CAACGCGCTC TAGCGATAAA CCTTGTCGGG GCTTATTCGG 448920
TGGCGTGCGA AACCGCGAAG CTGATGAAAC CTGGCGCCGC CATCGTCAAT GTCGCTTCGC 448980
TGGCTGGGCT GCTCGGCAAT CCGAAGCGCA GTGCCTACGC AGCTTCGAAA GCAGGTCTGA 449040
TCTCAATCAC GAAATCGCTG GCGTGCCGAT GGGCATCGCG CGGCATCCGT GTGACAGCGG 449100
TAGCGCCCGG TCACGTGCGC ACGCCTATGG TTGCCGAATT GGAACGCGCT GGCAAGCTCG 449160
ACGTCAGCGC GATAAGGCGC CGCGTACCGT TGGGCCGAAT AGCGCGCCCC GACGAAATCG 449220
CGCGGGCCGT GCGCTTTTTG GCGAGCGCAC AGGCGAGCTA CATAACCGGG TCGACGCTTG 449280
TGGTCGATGG GGGCTGGATG TCAGTCAACC AACCGGGTGG TGCCCATCAG GCTCAGGATA 449340
GGACGCCCGG AGCAGAATTC ATGCGGCCGG TCGAGGACAC CGATGCGCGA ACAGTGATCG 449400
TCATGGGCGG CGCTACAGGG GTAGGCGCGG CCATCGCTCG GCGCTTTGCC GAAAACGGCG 449460
ACACGGTTGT GATCGCCGAC GGAGACGGTG AAGAAGCGGT AAAGCTCGCC GGCTTGCTCG 449520
GCGACAAGCA CCTGTCAAGG CGCGTGGACA GGACCGTCGA AACCGAGGTA GTCTCGCTCT 449580
TCGAAGAATT GCGGGAGCGC TTCGGCCACC TCGACGTCTT CGTCAACGGC ATGAATGAGA 449640
TACTCGTCCC CAATACCGAG GAGTCGCCGG AAGTCCTCAA ACGCATACTG GATGTCAATC 449700
TTACTGGCGC CTTCACCTGC GTTCGCGAGG CCGCCATATC GATGCGCTCT GGCAGCGTAA 449760
TCCTGAATCT CGGGGCAAGT TTAAGCCTTT CACCGCTCGC GCCAAGTCAC GCCTATGGCG 449820
CATACAATGC TGGCATAGAC ATGCTGACCC GCTGCACGGC GGCCGAACTC GGGCCATTGG 449880
GCATCCGCAC AGCCACAGTC GCTCCAGGTT ACATCCGCAC ATGTGCCGCC AACCGGCTGG 449940
CAGCGGTCGC CGGCATGGAT TCGGCTTCGC TCCGACAGCG AATTCCTTTG GGCAGGGTCG 450000
GGGACGCAGA AGAAGTCGCC GAGGCTGCCT ACTTTCTGGC CTCGTTTGAT GCCTCCTACA 450060
TCAACGGCTC GATCCTGCAC GTGGACGGAG GCTTGATCTC GTCCAGAGAA GCGGGGTGGG 450120
GCAGCGAAGT CGATGGAGCA ATTTCGGCGG AGATGAGGCC GCAGCGCCGA CCCGCGGCGC 450180
GATGGCGGTT GCTCTCTCCC TGAATTGCAT TTGCACGCTT TTAGCGCTTG GCGCTCTGAA 450240
TGCGTTAGGA GGCCATGAGC AGGGCCATAG GGAAACCTTC TCGTCAATCG AGGCGCGACC 450300
CGCGACTGCG CGATTCCAGA TCCGAAAACA AGGAGACACC ATGGAGTTCG CAACGTTCAT 450360
TCTCGCTGCC CAGCGCGGCT ATCATCAGTC TTCCGCTAGC GTCATCCGCA ACTCGATAGA 450420
GCAGGCAATT CTTTCGGAGC AGGCTGGCTT CAGCACAGCC TGGTTCGCCG AGCACCACTT 450480
TAACAACTAC AGTCTCGTCC CGTCGCCACT ACTGATGGTA GCCCACTGTG CCGGATTGAC 450540
CAGCACCATT CGCCTCGGAA CCGCAGTCTG CGTGCTGCCG CTCTACCAAC CACAGCGCCT 450600
GCTGGCGGAA ATCGGCTTTG TCGACGTCGT TGCCAACGGT CGACTCGAAC TCGGCGTTGG 450660
TTCGGGATAC CAGCAGTTCG AGTTCGACCG CTTCGGCGTC AATATAGATG AGGCGCCGGC 450720
CATCTTTTCG GAATGCCTGG ACATTTTGCT GAAGGGACTA AAGCAAAAAA TCTTCACCCA 450780
CAGCGGTCGC TACATGCAGA TACCCCCGAC GGCGATTTCG GTGCGCACCC TCCAAAAGCC 450840
GACGCCACCG ATCTGGATTG CTACCGCATC CTCCAAAACT ATGGCCCGAG CCTATCGTGA 450900
GGGTCACAAT CTTTTCGTCA CGGCTCTCCA TGACGGCTTG GAAACTCTGG GCTTGCTGCG 450960
TGGCATCATT AAGACCGCCG CTGGATCCGA GGGCAAGGAG GTTCGCGACT CCAAGGTATC 451020
GCTACTGCGG TGCTGCTATG CCAGTGATGA TGGAGCGGAA ATCAACAGCT ATATCGATAA 451080
CGCCCGCTTC CAGCGCCGGC TGTCCGAGGC ACTGCAACAG CGTCGGCAAC AAAGTAAGGA 451140
CGGCTATATG TTGGAGGAGA TGCCGACGCA TCAGGATCTA TCGTTCGATA CTATGCGCAA 451200
GAACCTGCCC ATTGGCAGCA TCAATCGCGT GATTGATCGC CTTCTGGAGG AGATCGATGT 451260
CTTGAAGCCG GACCAGATTG CAATTCAGAC CCAGCTGGGG GATTTTGACC AAAAGACGAT 451320
GTTGCGCCAG ATCGAGCTTT GGGGAGACAA GATAATCCCG GCAGTCCAGA AATCTCTCGG 451380
GCGGTCGGAG GCTTGAGTTT ATATCGTCCT GACCTTCGGG TCAGCTCACT TCCTCAGGCG 451440
CCGTCAGAAT GCCCGGCAAG TTTTAAGAGC CATCGTAAAT GTCGGCACAG ATTACAGCTG 451500
AACTCCTCGT GAAAGTTGGG CGTCATGGGC CGCATACCCA TTCGCACGAC AGACAAGCAG 451560
CCTACCGGCG GCCGTACGCA TTTGAGCTCC GATAGTTCAG TCCGCGATTT TAATCGGACG 451620
AGGCCCTGAA ATACCCATCT TAAGTTGATC GGACGGCGGA CACTTAGTGC TGGCAAAGCC 451680
TCCGAGAAGA TGCTTCCCAA ACCCGCGGCT TGTACCTGAT CGGCGGCAGT GGGCTCCGAG 451740
CGCCTGATAG GCAACGCTAT GCGACAATTG CTGGGTACAA TTGTCGGCTC CGCGACACAT 451800
GCTTGCGTTT TAATGCGGGC GCTCTCTTTT TGGTCTAAGC GGCTGAGAAA TCTGGAAAAT 451860
CGGGCAGCCA ACCGCTGCGC TCCACCGACT TGGCACGGCT TTTGAAGCCT TCCTTGTGCA 451920

GGCGGCTTGA AGCTGCCGCT GTATTCGTGT TGCGGGCAAC CGCGATGGTT TCGAACAACG 451980
AAGGAAAGCA ACATGGCAGG TCTGCGTCAA ATCGCGTTTT ACGGCAAGGG CGGTATCGGC 452040
AAGTCGACCA CCTCGCAGAA CACGCTCGCC GCCCTTGTCG ACCTCGGGCA GAAGATCCTC 452100
ATCGTCGGCT GCGATCCCAA GGCCGACTCC ACCCGGCTCA TCCTCAACGC GAAGGCGCAG 452160
GACACGGTCC TGCATCTGGC GGCAAAGGAG GGATCGGTGG AAGACCTCGA GGTCGAGGAC 452220
GTGCTCAAGG TCGGCTACAA GGGCATCAAA TGCGTCGAGT CCGGCGGCCC CGAACCGGGT 452280
GTCGGCTGCG CCGGCCGCGG CGTCATCACC TCGATCAACT TCCTGGAGGA AAATGGCGCC 452340
TATGACGATG TCGACTACGT CTCCTACGAC GTGCTGGGCG ACGTGGTGTG CGGCGGCTTC 452400
GCGATGCCGA TCCGCGAGAA CAAGGCGCAG GAAATCTACA TCGTCATGTC CGGCGAGATG 452460
ATGGCGCTCT ATGCCGCCAA CAACATCGCC AAGGGGATCC TCAAATACGC CCATTCGGGC 452520
GGCGTGCGGC TCGGCGGGCT GATTTGCAAC GAGCGCCAGA CGGACCGCGA GCTCGATCTC 452580
GCCGAGGCGC TGGCGGCCAA GCTCAATTCC AGGCTCATCC ACTTCGTGCC GCGCGACAAC 452640
ATCGTCCAGC ACGCCGAGCT CAGGAAGATG ACGGTGATCC AGTATGCCCC GGAGTCGCAA 452700
CAGGCGGCGG AGTATCGCGC GCTGGCCGAC AAGATCCATG CCAATTCCGG CCAGGGCACC 452760
GTCCCGACCC CGATCACCAT GGAGGAGCTG GAGGACATGC TGCTCGATTT CGGCGTCATG 452820
AAGACCGACG AGCAGATGCT TGCCGAACTT CAGGCCAAGG AAGCGGCGGC AGCGGCCCAG 452880
TGACCGCCGC CGCAGACGCT GCCCGGGACG GTGATCCGGT GCGACATTCC ACCAATGGTG 452940
CCTCTTCTTG GAGGACACGC AAAAAAGGGG GCAGGCCCAA TGAGCCTCGA TTACGAGAAT 453000
GACAGTGCGC TCCATCAGGA GCTGATCACG CAAGTGCTGT CGCAGTACCC ACACAAGGCG 453060
GCCAAGCGTC GCCAAAAGCA CCTCAGTGTC GCATCGGACC GCGAGGCGGT CGGGGAGGAG 453120
GGCGAGACCC TCTCCGAATG CGACGTGAAG TCGAACATCA AGTCGATCCC CGGGGTGATG 453180
ACGATCCGCG GCTGCGCCTA TGCGGGCTCG AAGGGCGTGG TCTGGGGCCC GGTCAAGGAT 453240
ATGGTCCACA TCTCGCACGG CCCGGTTGGC TGCGGTCAAT ATTCCTGGTC GCAGCGCCGC 453300
AACTATTATG TCGGCACCAC CGGCGTCGAC ACCTTCGTGA CGATGCAGTT CACCTCCGAC 453360
TTTCAGGAGA AGGACATCGT CTTTGGTGGC GACAAGAAGC TGGAACAGGT CATCGACGAG 453420
ATCGAGGAGC TGTTTCCCCT CAACAACGGC ATCACCATCC AGTCCGAATG TCCGATCGGC 453480
CTGATTGGCG ACGACATCGA AGCGGTGTCG CGCAAGAAGG CCGCCGAACA CGAAACGACG 453540
ATCGTGCCGG TGCGCTGCGA AGGCTTCCGC GGCGTCTCGC AGTCGCTCGG CCATCACATC 453600
GCCAACGACG CCATCCGCGA CTGGGTGTTC GACAAGGCGG ACGGCAAGAC GGACGTCGAG 453660
TTCGAAACCG GTCCCTACGA TGTCAACGTC ATCGGCGATT ACAACATCGG CGGCGACGCC 453720
TGGGCGTCGC GCATCCTGCT CGAGGAGATC GGGCTGCGCG TCGTCGGCAA CTGGTCGGGC 453780
GACGCCACGC TCGCGGAAGT GGAGCGGGCC CCCAGGGCCA AGCTCAACCT CATCCACTGC 453840
TACCGGTCGA TGAACTACAT CTGCCGGCAC ATGGAGGAAA GATACGCCAT CCCCTGGATG 453900
GAATACAACT TCTTCGGCCC CTCCCAGATC GAAGCCTCTC TGCGCAAGAT AGCCAGGCAT 453960
TTCGGCCCGA CGATCGAAGA ACGGGCCGAG AGGGTCATCG CCAAGTACCG GCCGCTGGTC 454020
GACGCCGTGA TCGACAAGTA CTGGCCGCGC CTCCAGGGCA AGCGAGTGAT GCTCTATGTC 454080
GGTGGTTTGC GCCCGCGCCA CGTCATCACC GCCTATGAGG ACCTCGGCAT GCAGATCGTC 454140
GGCACCGGCT ACGAATTCGC CCACAACGAC GACTACCAGC GCACCGGCCA CTACGTGAAG 454200
ACGGGCACGC TGATCTATGA CGACGCGACC AGTTACGAAC TGGACACGTT CATCGAGCGG 454260
ATCCGCCCCG ATCTGGTCGG CTCCGGCATC AAGGAGAAGT ATCCGGTGCA GAAGATGGGC 454320
ATCCCGTTTC GCCAGATGCA CTCCTGGGAT TATTCCGGCC CCTATCACGG CTATGACGGC 454380
TTCGCCATCT TCGCCCGCGA CATGGATCTC GCCATCAACA ATCCGGTCTG GGATCTCTAC 454440
GACGCGCCCT GGAAGAAAAT GACCGTGCCG ACGGCCGCAG TTGCAGCCGA ATGATCGGCC 454500
GGTCTTGCGC GGCACGAGGA CCGCGGCAAG ACCGGAAACC TCTCGAACAT CCCTGGGCCT 454560
AAGAGGCCAG ATCAAGAAGG TCAAACACCA TGCCGCAATC GGCTGAGAAG ATACTCGACC 454620
ATGCGCCGCT GTTTCGCGAG CCGGAATACC GGCAGATGCT CGCGGAGAAG AAGCTGAACT 454680
TCGAATGTCC GCACCCCGAA CGGCTCGTCA CGGACCAGCG CGAATACAGC AAGGGCTGGG 454740
AATATCGCGA GAAAAACCTC GCCCGCGAGG CGCTCGTCGT CAACCCCGCC AAGGCCTGTC 454800
AACCGTTGGG GGCGGTGTTC GCAGCCGCCG GCTTCGAGCG GACGATGTCG TTCGTCCATG 454860
GCAGTCAGGG CTGCGTGGCC TATTATCGCT CGCACTTGTC GCGCCACTTC AAGGAGCCGG 454920
CTTCGGCCGT TTCGTCCTCG ATGACCGAGG ATGCGGCGGT GTTCGGCGGC CTGAAGAACA 454980
TGGTCGACGG GCTCGCCAAT ACCTACGCGC TCTACGATCC GAAGATGATT GCCGTCTCCA 455040
CCACCTGTAT GGCCGAGGTC ATCGGCGACG ACCTGCATGG CTTCATTGAG AACGCCAAGA 455100
GCGAAGGCGC AGTCCCGCCC GAATTCGACG TGCCGTTCGC TCACACGCCC GCCTTCGTCG 455160
GCAGCCATGT CGACGGCTAT GACAGCATGG TCAAGGGCAT CCTGGAGCAC TTCTGGAAGG 455220
GCCAGGCGCG CACCCAAGCG GCCGGCACGA TCAACATCAT CCCGGGCTTC GACGGCTTTT 455280
GCGTCGGCAA CAACCGCGAG CTTCAGCGCC TGCTCACCCT GATGGGCGTG TCCTACACCT 455340
TCATCCAGGA TGCCTCCGAC CAGTTCGATA CGCCGTCCGA CGGCGAATAC CGCATGTATG 455400
ACGGGGGCAC GACGATCAAG GCGCTGCGGG CGGCACTCAA TGCCGAGGCG ACGCTGTCGC 455460
TGCAGCACTA CAACAGCCGC AAGACGCTCG AATATTGCCG GGAGGTCGGT CAGGCCACCG 455520
CCGCCTTCCA TTACCCGCTC GGGATCAACG CCACCGACGC GTTCCTGATG AAGGTGTCGG 455580
CGATTTCCGG CCGGGAAATC CCCGAGACGA TACGCCTGGA ACGCGGCCGG CTGGTCGACG 455640
CCATGGCCGA CAGCCAATCC TGGCTGCATG GCAAGACATA CGCGATCTAC GGCGATCCGG 455700
ACTTCGTCTA CGCCATGGCC CGCTTCGTCA TGGAGACCGG CGGCGAGCCG CGGCATTGCC 455760
TCGCCACCAA CGGCACGGCG GCCTGGCAGG CCGAGATGAC CGAGCTGCTC GCCTCTTCTC 455820
CCTTCGGCAA GCAGGCAAAG GTCTGGCCGG GAAAGGATCT CTGGGCCCTG CGCTCGCTGC 455880
TCTTCACCGA GCCGGTCGAC CTGCTGATCG GCAATTCCTA CGGCAAGTAT CTCGAGCGCG 455940
ATACCGGCAC GCCGCTGATC CGGCTGATGT TCCCGATCTT CGACCGCCAC CACCACCACC 456000
GCTTTCCGCT CATGGGCTAC CAGGGCGGCC TGCGCCTGCT GACGACGATC CTCGACACGA 456060

TCTTCGACCG CCTCGATCGC GAAACGATGC AGACGGCGGT GACCGATTAT TCCTATGACC 456120
TGACCCGCTA AGAACGGCGG TCGGCAAAAC GCCGGCCGTC TCCTGATGCA ATCTGGAGAT 456180
TAGCGAATGT CTTCGCTCAA TGTCAAAATC CAAGATGTCT TCGATGAGCC TGCCTGTGAG 456240
AGAAATCGCA GCAAGGATTC CAACGCGCGC AGTAAAGGCT GTTCGAAACC GCTGACCCCC 456300
GGCGCGGCGG CCGGAGGCTG CGCCTTCGAT GGCGCCAAGA TCGTGCTGCA GCCGATCACC 456360
GACGTTGCGC ATCTGGTTCA TGCACCACTC GCCTGCGAAG GCAATTCCTG GGACAACCGA 456420
GGTACGGCGT CGTCCGGTCC GACGCTTTGG CGCACCAGCT TCACGACCGA TCTCACCGAG 456480
CAAGACATAG TGATGGGGAA CAGCGAGCGG AAGCTCTTCA AAGCGATCCG CCAGATTAGA 456540
GAAGCGTATC AGCCGCCGGC AATCTTTGTC TATGCGACGT GTGTAACGGC ACTTATCGGC 456600
GACGACATCG AAGCGATCTG CAAGCGCGCT GAGGAAACGT GCGGCCTACC GGTGGTGCCG 456660
ATCAATTCGC CGGGCTTCGT CGGTTCGAAG AACCTCGGCA ACAAGCTGGC TGGCGAGGCG 456720
TTGCTCGACC ATGTCATCGG CACGGTGGAG CCAGATGATG CCGGCCCTTG CGACATCAAT 456780
ATCCTTGGCG AGTTCAACCT GTCTGGTGAG TTCTGGTTGG TAAAGCCACT CTTGGAGAGG 456840
CTTGGCATTC GTGTTCGCGC CTGCATTCCC GGTGACGCCC GCTATCTCGA CATTGCCTCC 456900
GCTCATCGCG CTCGAGCAGC CATGTTGGTG TGCTCTACGG CGTTGATCAA CCTTGCCCGC 456960
AAAATGGAGG AGCGCTGGGA TATCCCGTTC TTCGAGGGAT CCTTCTACGG CATCACCGCC 457020
ACCTCGGAAG CACTTCGACA GATTGCCGAT CTGCTCGTAA AGAAGGGTAC CGATCTAGAG 457080
ATCCTCGACC GCACCGATGC ACTCATTGCG GAGGAGGAGG CGATTGCGTG GAAAAAGCTC 457140
GAGGAATACC GGCCGCGACT TAAAGGCAAG CGGGTGCTCA TCAACACCGG CGGCGTGAAA 457200
TCCTGGTCGC TTGTTCATGC GCTGATGGAG ATCGGCATGG AGATCGTTGG CACCTCGGTC 457260
AAGAAATCGA CGGTCGAGGA CAAGGAGCGC ATCAGACAGG TGCTTAAGGA CGATCTCCAG 457320
ATGTTCGAGT CGATGTCGCC CCGTGAGCTC TACGCCATGC TCTCAGAACA TAAGGCCGAC 457380
ATCATGCTGT CGGGCGGGCG CACGCAATTC ATTGCGCTGA AGGCCAAGAT GCCCTGGGTC 457440
GATATCAACC AAGAGCGTCA CCGCCCCTAT GCGGGCTATG ACGGCACGGT GGAACTCGTG 457500
CGCCAGATCG ACCTTGCGAT TCACAACCCG GTCTGGTCGC AGGTGAGGGA GCCGGCACCG 457560
TGGGAAAGCC GTCCTGCCAC ACAGGAACCA CTGCCGAACA CGCGCCCCGA CACCGATGCT 457620
ACGAGTGTCG GGCGCTCAAT TCGCCGGCAC GACGCCGGTG ACTTCGTTGA GTGTTGAGGA 457680
AAGCAAATGG TCCACATCCA TCGCCAATCT AAATCAGCGA CGGTCAATCC GCTCAAGTCG 457740
TCCCAACCGC TGGGTGCCGC GTTAGCCTTT CTCGGGGTCG ATGGCGCGAT ACCGCTGTTC 457800
CACGGCAGCC AAGGCTGCAC TAGCTTCGCG CTAGTGCTGT GCGTGCGCCA TTTCAAGGAA 457860
ACGATCCCGC TGCAGACGAC GGCAATGGAC GAATTGGCAA CAGTCTTGGG CGGGGCAGCC 457920
CATCTGGAAG AGGCGATTCT GAACTTGAAA AAGCGGGCGA ATCCGCGGCT GATCGGAATC 457980
TGCACGACGG CACTTGTGGA AACCCGTTCT GAAGATTTCG CAAGGCAAAT CGCCAACATC 458040
AAAATGACGC ATGCGGAGGA ACTTGCAGGC ACAGAGGTCG TGCTGGCAAA CACGCCGGAT 458100
TTCGATGGCG CCTTGGAGGA GGGCTGGGCG AGGGCCGTCG CCGCGATGAT CCAACAGATT 458160
ACACTGCGGC GTCAGCAGGC GCCTCGCTCG AGAAAGGCAA CACTGATCGA GAGGATTACA 458220
AAACCTAGCG AGCAGCCGTG GAAGCAACAG AAGGTTGCGA TCCTGCCGGG ATGGCACCTC 458280
ACGGTAGGCG ATATCGAGCA GTTGCGCGAG ATGGTGGAAG GTTTCGGGCT GAGGCCGGTG 458340
ATCGTGCCGG ACGTCTCAGG CTCCCTCGAC GGTACGGTGC CCGACCGCTG GATGCCGACA 458400
GCGTATGGAG GTACAAGCAT CGAGGATATA CAGGAGTTGG GTAGGGCGGT GCGGTGCATC 458460
GCCATCGGGG AACATATGCG GCGCCCGGCT GAGCTACTGC AGACGTTGAC GGGTGTGCCT 458520
TACGTGTTGG TCCAGTCGCT AACGGGATTG AAGAACGTCG ACCAGTTTGT CTCGCTTCTT 458580
TCCGAGATTT CCTGCGTGCC GGCGCCGGCG AAGATCCACC GTCACCGCTC ACAGCTGCAA 458640
GACGCGCTTC TCGACGGACA CTTCCATTTC GCCGGCAAGA AGATCGCGAT CGCCACCGAG 458700
CCGGACCAGT TGTACCAATT TGCCACCTTT TTTACGGGAC TTGGCGCCGA GATCATCTCG 458760
GCCGTTACGA CTACGGGTGA GTCAGAAATT ATCGAGAAAG TCCCGGCCGA AAAGGTCCAG 458820
ATCGGCGATC TCGGTGATCT CGAAGATCTA GCCGGCGGCG CTGATCTTCT CGTTACCCAT 458880
TCGCACGGTC GCCAGGCAGC GGAGCGCCTC GGCATCCCTC TGCTGCGGAT CGGCTTCCCG 458940
ATATTCGACC GCCTCGGCAG CCAGCACAAA CTCACAGTTC TCTATCGTGG CACGCGCGAC 459000
CTGATCTTCG AGGCGGCCAA CATCATCCAG GCCAACCAGC CCGCGCCGTC GCTTGAGCAA 459060
ATCGATGCAA TGCGAAAGCG GAGAAATGCC GGATGAATTC CGTTCGTCGC CTCTCGCTCG 459120
TCACTGACGA AATCCAGCCG CGGCGTTCCG GCGCATTGCG CATAGCGATC GCGACGCAGG 459180
ACATGAAAGT CCTAAACGCC CATTTCGGGT CGGCCAAGCA CTTTGCAGTC TACGACGTGT 459240
CGCGCGACGG CTGGGATTTC GTGGAAGCTG TGAGCTTCGA TGACGTCTCC GACGAATCCG 459300
GGAAGCATCC GATCGAGGGC GAGGACCGCA TCACCCCAAA GGTGACCGCG TTGACAGGCT 459360
GTCATCTCCT CTTTTGTCGG GCTATCGGCG GCCCTTCCGC AGCCAGGGTC GTCTCTGCGA 459420
AAATTCATCC GATTAGAGTG GGAGAGCCGG AAGCCATTCA AGACGTACTT TCACGAACCC 459480
AGAAAATGCT CAAGACGGCC CCTCCGCCGT GGTTGCGCAA GGTGCTGGCG CAAGCAGGCG 459540
TCGCAGAGAA AAAACCATTC GAGGACGAGG ACTGAATCAT GAAAAAAACG TCCATCAATA 459600
CGGGCACGCC TGATGTCAAG GTGGACGAGG CGGCCCTTGT CACGCCATTT GTCAAATGCC 459660
TCGCGCGGCT GGTCCGCGCG CAGGATACCT ACGGATCGCA GGACCGCGCA TCGGACGCGG 459720
AGTTGTTGGC CCACTTCATC ATCACTGAAG AGCAGCGTCG AGAAATCCCG ATCATCGGCG 459780
ATCCCGGTCC GGACGTCATG TTGCGGTTCA ATATCTTTTA TACCGCCGTC GCGCTTTCAA 459840
TCGAAGCACG TACCGGTTTG GTAGCGTCGC CGATCACGAT GATCAGCCAT GAGGGCTTCG 459900
GACGAGTGCT TCTAACGACC GGGCGGCTGG TCGTATTTTC GAAAACAGTG CGCGACATCC 459960
ACCGCTTCGG CTTCGCGACA CTTCGCAAGC TTGCGGAAGC CGGCGCAAAG CTGGTGGATG 460020
ATGCGACGGC CGCCATTGAA ACCTATCCCG AAGTGGCTCG GGCCTGACGA TCGATCCGGC 460080
AAAAGGCATG CTGCCGGCGC CAAGGCGACC GGCCAAGGAT TAGGGCTACG CCCGTGGGCG 460140
GCTTGAGGGC GAGTAAATAG TGGATTTTTC CGAAACGGAG GGTATGAGCC CGTGCATCAA 460200

CCTCAGCGAC GCGCGCCGCC GTCTGCCCCG GGCAGTTTAT CTGTACCGCT TGAGTACTCG 460260
TTCGGTAGCC GCCGATGAGA ACCATGTGTA GTAATTCAAA CAGCCGCTTC GACACTCTTG 460320
TCGGTAAAGC GACACATTCT TGTCGACTGT TCCCTTCTCT GCCTCCCGGC TAAATGCCTG 460380
AAATGCTAAC ATAACAAGCT TCTTTTCAAG TCGAGCACCC GAGTGGCACG AGTCTTGCCG 460440
ACATGCCATC TAAGAGGCTG GAAAGCCGGG GATCGGCAGG GTACGAATGA GGCAAGCAAC 460500
GTGGCAGGTC TAGGTCAGAT CGCATTCGGC TGCGAGGGAG GCATCGACAC GTGCACCGCC 460560
GCCCAGAATA CGCTCGCTGT TTTTTTTGAT CTCGGACCGA AAGTCGGTAG TGTCGGCCGC 460620
AACCTGAAGA GTAACTCCGG CCGCTTCATC CTGGAACCAA TGCCGCGGGA CAGCGCGCCA 460680
GCGTGGTACG CGATCATCCG CGATTTCGAT GCGGACGTGC CGTGGAGGCT CAAAGCTTTC 460740
CACACCGCCG CCGGGCTTGC GATCGAACCG TCCGTTCGCT CCGTGTACGA GGGCTTCGCG 460800
GTGCTTCTCA CGACCGGGCA GAGTCGTATC GACCGAACAC CATCCGTCAG TCTTCCAATA 460860
GGAAGCTCGC TGGAGACCGA AGCGATAACC GCCATCCACG CACCCAAGTG GCGCGGTTGA 460920
CGGTCGATCT AGGCGATGAC GAACCGCCGG CTCTCATTTC CCCTGGAGAG CCGACGTCAG 460980
GCAGCGGGAC CACTCCTCCC CACGCGCACC GGTCCCGCTG CCGCGATCTT TCCACAGGGC 461040
GAGGCTCGAA ATGCCTAGCC AAGTCTTTGC AATGGCCGCT GACTAGCAGA CCGCCCCCGA 461100
AAAGTCGGCC GACAAGACCG GAATTTCGGC GGATGTGACG GATGAGGTCA CGTCCGATCT 461160
CGGAAGAGAT TTTACTTTTT TGCCTGTGGC GGATGCGGAG AGACAGATCC TGATTGGAGA 461220
ACCAACGATG ACAAGTCATT TCGTTACCCG CGACGGCTCC ACATGGATGC CGCAGTATCT 461280
GACGGCCATT GACGCCATGA CGTGCATTGG TTGCGGCCGC TGCTTCAAGG TCTGCTCGCG 461340
CGAGGTCATG CATCTGCACG GCATCGACGA ATCCGGTGAG ATCCTCGGGG CCTGCGATGG 461400
CGAGGACGAC GACTTCGCTG GCGAGCTCAG TCGCACGATC ATGGTTGTCG ATCACGCCGG 461460
CCGCTGCATC GGCTGCGGAG CCTGCGCCCG GGTATGCCCG AAGAACTGCC AGACCCATGT 461520
AGCGGCCGAC GAAATTGTTG CTTGATCTGA CGAACGGACT CCGGAAAACG ACTTTGGCCG 461580
TTATAAATGC GATCATATTC GAGACGACAG TCCCCAAAGA GCCCTTGCCG TCGCGCAAGC 461640
TGACGAAAGA CGTAGACATC GCCGACCGTC GGCCCCGTCC CGCAGCTAGC AGCCGACGTA 461700
GAACAACGAA CTGCCCAGCC ACCTGAAAGC CAGAGTCCGC CGAACTTGGA ATGAGACAGA 461760
ATTGGCACAA TATTGATTTC CTCGCGTGAT TCAGGGTGAA TCCCTGCCTT GGAGTCAAGA 461820
GGCAATTGGC GCCCGCGGGC ACTCAGAGAG GACCTTCGAA TGCCCCGTTG GCATCGGTCA 461880
GCAACGGAAT GCGTCAGCAG CCTGAGCGAT CAGCAGTTAG GTTACAAAAG CACTGCTGCT 461940
CGAAGCGCAC ATGCCGACGC AAATTGGTGA AGAAACCACG GAGCATATAG CCCGCTGCCT 462000
CCGGATTGAC AGTAGCATCC GCCGTCGCCA GCCGACCCAA CACTTCCGCC AATTCCTCGG 462060
CGAAGCATTC GTCTTCGCAA TGCTCATACT TGAGCAGACC CAAAGTAGTT AAAACTGATG 462120
GATCGTCCCC TAGCTTCTCC TCTGTCCACG CGAAAACAGC AGCTTCTTCT CTCTCGTGAA 462180
GATTCCGTAA GTGCGGGCCA AGCATCTTTC CGGCGTGAGC GCATATACTA CGGTCGACTC 462240
CGTGCGGAAG GCTGTTTGCA ATGGCCTCGA GACGGTCACA GAGAGACAGT TGGTCCTCGT 462300
GCGAACCTGC CAATAAGGCC AGCCGCTCGG CGGTGGCTGG AGCTAATGAC CGGTTTGTGG 462360
GGGGCGCAAA CGTCTTGATC CTAGCGATCA TACACTCCAG GCCCTTTCCG TGCTTTCTCT 462420
AAGGTAATCT TCCGTGCTCA AGGATGAGGT AATGCGTGGT GCGGGGCCTT GACTTGAATC 462480
AAGGTCTATC GGCAATAGGA CTAAATAATC ATCTCATTGT CTGATGGATC GTACGCGGCG 462540
TTTCCCCCCA AGCGCTCTGC TGTTGTGAGA TCGCCATGAG CACGTCGGGA GTCCCAGCTA 462600
ATTGTGAGAC GCTGATCCGT TGTGCATGGA CCCACGGCAT GAGCGCGTCG ATGTCTTTGG 462660
CGAGTGGCGG TTGGCGATGC CGATGAAGAA ATTGGTAGAC TGGGTTCAAC ACCATTCATC 462720
TTGCAAATCG GACCCAGCTA CCATTGCCTT GATCATGGCC GGCACAGAGA GCGCATTGCG 462780
GCGCTTGGGT TTCTTATCGG CTTTTTCGAC GAAGCCCTGC ATGTCGCAGG GAGAGCCTTG 462840
GCGGACACGA ATATGCGGCG CCGCATAGAC CCAGGAACGC AGCAATAACA CGATCGGCAG 462900
GCGTGAACAG GAAGTATGGC ACCCCTATAG TGCCAATGAC CGCCCGGAAC CATGTCGGGC 462960
GGGACCGAGC GAGCTCGCCT ACTCGGCAGA AGGCCAGCCT CTACGGATCG CCGCCTGTCG 463020
GTTTTGGACG CGGCGGTCCA CTTTTAGCCG CCAGGCTGTC GTTGTTCGAC ATACGACAAT 463080
GTTACAATGC CGACATAGCT AGTTCGATCC AGGTACTTGA ATTTAAACGG GTTTTCCGTG 463140
GCACGTATCT TGCTCTGCTG ACGTGTCAGC GTCGACGAGA ATTGTGCGGA GAATGTCGAA 463200
ATGTCAGAAC CGTTCTTTAG GCCGGGGTCG GGGCCCGCGC CCGGCCACCC TACTGCAGAA 463260
CCTTGGAGAA GAAGGAAGAG GTGGTACTCG CCAAGCGATG GTCGAGCTGG CACTGCACGG 463320
TCGCATTTGC CACAGCGGTC GACGAGCCAT GATGGAGTGT GTTCGCGTAT CTGTGCATTT 463380
GTCCTATGTA TGGTCGCACT GCAATTTCTG ATGACCTCAG CAATGGCGGC CGACGAGAGC 463440
CCGCTTCGCG AAGCCGAGGT TGCAAATTTC ATGCTGGGCA ACGGCATGGA GGTAGTCGTC 463500
ATACCCGATC ACCGGGCGCC GATCGTCACG CAGATGATCT GGTATAAGGT CGGCAATGCC 463560
GACGAGCCAC CCGGCAAATC CGGCATTGCT CATTTCCTGG AGCATCTGAT GTTCAAGGGA 463620
ACGAAGAAGC ATCCGTCCGG AGAGTTTAGC GCAAAGATCG CGGAGATCGG CGGTGAGGAG 463680
AACGCCTTCA CGGGTTCCGA CTACACCGCC TACCATCAGA CGGTCACGCC AGAGTCGCTC 463740
AGAACAATGA TGGAATTCGA AGCTGACCGG ATGCGCCATC TCGTTCTCAC CGATGCGGTG 463800
ATCGTACCCG AACGCGATGT CATTCTTGAA GAGCGACGCT GGCGCGTCGA GAACGATCCG 463860
GAGCAGCTAC TTGAGGAGGA GATGCAGGCG ACCTTGTATC AGAACCACCC TTACCGCATC 463920
CCGACGATTG GCTGGATGCA TGAAATGGAA CAGCTTAATC GCGAAGACGC ACTAAAGTTC 463980
TATGACCGCT ACTATGCCCC CAACAATGCC ATTCTGGTCG TGGCAGGCGA CGTGGACGCC 464040
GGCAGGGTGC GGCAGCTAGC GGACGAGACT TTCGGGACCC TGCCTCGCGG TCCGGATCTG 464100
CCGGCGCGGG TGAGGCCGCA AGAGCCGGAG CAGAACACTA AACGGATCGT CGCGCTCACC 464160
GACCCGCGCG TCACGGTGCC GAGCTTCCAA AAATCCTGGG TAACGACGTC CTACGGAACA 464220
GCCGAGCAGG GCGAAGCGGA AGCCCTCGAT ATCCTGTCGG AGATTCTCGG CGGGGGAACG 464280
CGCAGCCGGA TCTATCAGGA GCTGGTGGTC AAACAGGCAA TAGCCTCCTC TGGCGGGGCC 464340

TATTTCAACG GAAGGTCGCT CGATCCGTCG AGCTTCACGG TTTTTGGCTC GCCGCGCGGC 464400
GAGGCGAAGA TTGAGGAGGT GGAGGATGCG ATAGACGCTG AAATTCGGAA GATCATCGAG 464460
TTCGGCATTA CCGACGTCGA GCTCGAGAAG GCCAAAAACC GCTTCGTGCG CTCGATAATC 464520
TTCGCGCGCG ACAGCCAGTC GGGAATGGCC GGCATTTATG GGGCGGCGCT TGCGACAGGC 464580
GATACTGCAC ACGACGTAGA GGCGTGGCCA CTTAGGATCC GCGCCGTAAA GGCGGCAGAG 464640
GTGCAAGCGG CTGCCAGGAA ATACCTCAGT CCCGACCGGT CCGTAGCGGG GTATCTGCTG 464700
CCCCGCGAAA GCGCTACCTC AGGAGATAAA AGCCGATGAT ATCGTTCAGC CGACTGCGCA 464760
TCGGGGCCAA GCCCTCACTC GTCGTGGCAT GCATATTGCT GGGCCTTGCC GCGTGTCCAG 464820
CGCTTGCGGG CATGGCCATA GAGGAGGTCG AGACATCGAG CGGGATAAAG GCGTGGCTGG 464880
TGCAGGACTA TTCGCTACCG ATCGTCACCA CCCGCTTCGC ATTTCGGGGC GGCAGGACGC 464940
AGGATCCATC TGGGAAGGAA GGCATTGTGA ATCTGATCAC AGAGCTGTTG GACGAAGGGG 465000
CCGGTAATCT TGACAGCGAT GCCTTTCAGG AGCGGCTGGA CGATGCTGGC GCCGAGATGC 465060
TCTTTGAGGC GGGGCCCGAT GCAGTCTACG GCAATATGCG GGTGCTTGCG GAGCGAAAGG 465120
ATGAGGCTTT CCAACTTCTC CGGCTGGCAA TCGAGCAGCC GCGCTTCGAC CAACAGCCGG 465180
TGGACCGCCT TCGTGCCCAA ATCGTCTCCA GCATCCTGGC AAGAGCCAAG GATCCGGAGA 465240
CGGCCGCACA GTTCGCATGG ATGAGGGCGA TCTATGGTGA TCATCCGTAT TCGCGTCGGG 465300
AGGAGGGCAC CGTGCAAACA CTTGCCGCTG TCACGACCTC CGATCTGAAG GCGGTTCATG 465360
AGCGGATTTT CGCGCGCGGC AACCTTACCA TTGCCGTGGT GGGAGCGATC GATCCCGGCA 465420
CACTCAAGCG TGATCTGGAT CGAATATTCG GCGGATTGCC TGCTGGCCCC TCCCTAACGC 465480
CGGTCGTTGA CGCAGTGCCT AAGCTGGGGC GCGCCATTCG CGTCGCGTAT GACTTACCTC 465540
AGGCCCAACT GAGCCTAGCT TACCCGGGAA TCCCCCGAAA GGACCCGCAG TTCTTTGCAG 465600
CCAATCTCAT GAACCAAATC CTTGGCGGAG GCGCGTTCAC ATCCAGGCTC TGGAATGAGG 465660
TGCGCGAGAA GCGGGGCCTC GCTTACGGCA TTTACTCAAC CTTGGAGAAC ATCGACCATG 465720
CTTCGGCGCT CGTCATCGGC ACCGGAACCC GCCCGGATCG GGCCGCAGAG ACGCTCTCCC 465780
TCATCCAAGC CGAGGTCAGG CGAATGTCAG AGGAGGGCGT CAGTGAGGAC GAGCTCACTG 465840
CGGCAAAGAA AAAGCTCATC GGCGGTTATG CGATCGAAAA TCTGAACTCA TCTAGTGCGG 465900
TCGCCCAAAC ATTGGTCCAA ATCCAACTCG AAGATCGCGG CATCGAGTAC GTCGAGCGCC 465960
GCAAACAACT GATCCAGGCC GTCACCGTGG AGGACGTACG GGCAGTCGCG AAACGGCTGC 466020
TCTCCGCCGA CCCCACCGTA ATGACTGTTG GACCGTCCCT CAAGGGAACG AACCGATGAC 466080
CGTCCAAAGG AAAGCTAGGC TGGCAGCGTC CGGCCGACCT TCTCCAACGC CCCGGCGCGG 466140
CACTACCTTG GGAGGGGGGT AGTCAAGATC ACCTCTCGCG GTCACCAGAT GTTCAGTTCC 466200
AGGCTCTATG CGACTCCTGA AGCAGCAAGG GAGTGCATCG CAGCCTCCCT CCGCCCGACG 466260
GCAGGAGAGA ATGTGGTGTC TCGGCGAAAA CAGGCGAATG CTCTCTAGCA CGACTGCTCC 466320
TGCGATGCTG GTGACCTGCT GGCAACGAGA CTGCTTGACC CTGCAGCAGC GGTTCTCGCG 466380
TCACTCACGA AAGCGCATCA GCACCACCGC TAAGGCTTCA CACTTGAAGC ATAATCCGAA 466440
TGTGCTTAGG GTGGCCGCCG AAGACTCCAG ATGTCTCAAT TGAGTTTCTG ACTTTCAGTT 466500
GAGGTGTGTT AAAGGAGTGT AGGTGCGGGA GGTGGAGCTC ATATCATATC TCCCGAAGAA 466560
CTAAGATAGC CGAAATTGAG GAGACGAATG TGCTGAGTAC TAACTCTGAA CAAACAAGAC 466620
GTAAGCCAAA CGCTGTGGAT GCCCATGTCG GTCAACGAAT CCGCCAGAGG CGTGAGTGGC 466680
AGAACATGTC CCAAACGACT CTCGGCGAAG CTATCGGGGT GACGTTTCAA CAGGTTCAAA 466740
AATACGAAAA GGGCGTGAAC CGCGTCGGTG CGGGTCGGCT TCAGCAGATT TCGAAGGCTC 466800
TGAAAGTGGA GCCTTCCTAC TTCTTTGAGG ATACGCTTAA TAAAATCCGG TCGGAGGAGC 466860
GGTCCGCTTC AAATCAGATT AACATCCCAC CCGAAGTCGT TGAATTCGTT GTAAGCAAGG 466920
AAGGGATCGA GCTCATCAGG GCCTTTTCTC GCGTGGGCGA CTATCGTGTG CGCCGCCGAA 466980
TCGTGATGCT GGTCAAGTCT TTGGGCGCAC ACGAGCGGTG ATGTATGGAT TGGGCCGCTT 467040
CTGTGTGGAG ACAGAGTGTC TCTATAAAGT CGGACAAGCA CCACCGCACT GGGAAATGAG 467100
TCACCAGTCT GTCCGATACT CTCGTACGGC CCTTTACCCT TTAAACGTCC TGAAATACCA 467160
AGGCGATACT CGCCGATCAA TTCTCCGAAT CTTGGCGGGC TGAGCGGCGC GCCGGTCCTT 467220
CGGGATACTG CGGCGACCAT CGACTGCGAG CTTGAGGATC TCATAGAACG ACACAGCCAT 467280
GCGATTATGC TCGGCCGTTT TGTCGACATT TGCAAGGGCA ACGGTCGTTC ATTGGTCTAT 467340
CGTGGCGGCC GATATTTGCA ATTGCCGGGT AGCGTTATAG GCAAAATCGC TCCGGGACTC 467400
ACCGCACTGA AAAGCTATTG AACGTGGTTT TTGCGCAAGC CTGTCTATGA TCTCAATCAA 467460
TTCATCTGTA GTTCATTCGT GCTAGCTTGA GCGCAGCCTT AGTTGCCGAC CACGATCCGA 467520
CTTGAAGCGA GCGATGCTTT CTACACCGGT TTCACCGGTC AGAGCGGAAA AGAAGAGTCC 467580
AGCCGGCCCC GATTTCCTAT TCTGAGACCT CAGAACGGAG ATCGAGATCT TTTCATTTGT 467640
AGCCTGCCGC GGAATGGGCG AGCGCTGACC TGAGTTCTCA GGGTCAGCTA GGCAAGCGCC 467700
GGAATACGAC TGGACGTGAT CAATCGACGT CAGAAGCTAT GGGATGTTGG AGAAGAAATG 467760
CGTAGGTTGC TTACTCCCTG GCGCGGTGTC GCTGCAGGCT TTCTGTTGAA CGGGATCCTC 467820
CTCGGGACCT GGGCTTCTCG CGTTCCCGCC GTGATGGGTC ACTTTCACGT TGCCAAAGCG 467880
AGCTTTGGGG TTTTGCTGTT GCTTTTAGGG TTGGGCGCAT TAATTTCGTT CCCCATTACC 467940
GGAAGGCTGT CCGATAGCTT GGGTGCAGTG CGGGTCGCCC GAATGATCGC GATCCCGTTC 468000
CTGGTTTCGA TTGCCGCTCT AGGGCTTGCG CCCACAATAC CGCTGCTGGC GATCGCCCTA 468060
TTTCTCTTTG GCATGTGTCA TGGCTCGATG GACGTAGCGG TGAATAGTTG GGCGAGCGAA 468120
GTGGAGAAAC ACATGGGACG GCCAGTGATG TCGTCGTTTC ACGCCATGTG GAGTGTGGGG 468180
GCCGTTCTCG GCGCAGCAGG CGGTTACATT GCTACAGTCT TTCAGACCCC AGTGTACGTC 468240
CATTTTCTTG TTACGGCCGT GACGATCGGC GGGTTGCTCG GACCCTTCCT TCTGCTCGAT 468300
TGGCAATCAA CCATCCGAAC GCATGAAAGT GGTGCAGTGG GATTGGTGTT ACCTAACAGT 468360
GGCCTTTTTC TTGTGGGGCT CATTGCTCTC GCCTCTGGAC TAGGAGAGGG TACAGCTCTC 468420
GACTGGAGTG CTGTATATTT GCATGATGTA GTCGGGACCG AAGAATCGGA CGCCGCCTTG 468480

GGGTACATGG GCTTTTCTGC GGCCATGGTG ATGATGAGGC TTAAGGCGGA TTCTCTGGTT 468540
ACGCGATGGG GATCAGCCAC TGTTGCACGC ATAAGCGGTT TCTCGGCTGT CTTTGGCATT 468600
TTGTTGATCG TCCTCGGTGA AACCTTGCCG TTAGTCGTGG CGGGCTTCGT GCTGATGGGG 468660
GTCGGCTATG CGGCCGTCCT ACCATTGGCT TTCAGTCGGG CAGCGGCCGA TCTTGTAGTG 468720
CCGGCGGGGA AAGCCATCGC GTCCGTTGCA ATCTTCGCAT ATGGCGCAAT GACGTTGGGA 468780
CCCTTTGCAA TTGGACTCCT CGCCGAGGCA GCCACTATGC GTTTATGCTT CTTTATCGTC 468840
GGACTATTTG CAGCCTTGGT GGCAGTATTG GCTCCAGTGC TCAAGCAATA GAACAAATCA 468900
CCCCCTGCTT TATCGCACCT TGAGAGATTT TAGCGTTTTG TCCATGAGAG CGACGAATAC 468960
TCCGCCTTTT GCTGACCGAG GCCGCGAGTA TTCTTCATCG GCAGGCGCGG AACGGAACGG 469020
TTGGAGATCA CGCCGACGCC ATTTTTGAAG GCATGGGTGA ACCAGGCATG AAATGGATCG 469080
AGGTGACCGG CCGAGGCCAG CGGCGGTATC GGACTGACGA AGATGAGTAA TCAAAAGTCG 469140
ATAAAGCGTT AATTCATATC ATCAATGGCT GCGATGAAAA CAAATGATTT TACTGAATAG 469200
ATGCGGGCGA AATAGAAGAA CGCTCATCAA GTTGGAGTCA GTGAATGTGC GGCACTGTTA 469260
GCCCAACTCG CGTTGACTGT CGGACAAAGC GACTATCCTC TAGGAATTGA ATGGAGAGAA 469320
CTATGTCTGA TGCAAAGCTG CAACGCGTAC TTTCGTCTCT TACGGAAGTG TACAGACAGC 469380
TAAATTCTCT GCCGTCCAGT CAGCCATCTG ACGCCGGCTA TGTCAAACTC GATACAAACG 469440
AGAATCCATT TGCGTTGCCA AAAGCGGTAA TGCAGAGCGC AGTTGCTGCT CTCGAGCGGC 469500
AGTATTTATA TCCAGAGGAT GACAACATCA GTTTGCGGGA AGCCGCCGCT GCGTCCTATG 469560
ATCTTTCCGC GGATCAGGTG ATCGCCGGTA ACGGTTCGTC CGAACTTCTT TCGCTCATAT 469620
ATAAAGCATT CCTTGGTCCG GGTGACAGTG TCGCGATGCT GTCGCCCGGC TTTGCATACA 469680
ACCGCAAGCT CGCCCAGTTG CAAGGTGCTC GATTACTTGA AATCAAATGG GGCGAGTCGT 469740
CCTTGTTGCC GATACACGAA TTGCTCTTCG GTCCTGCAAA GCAGGCAAAG TTCATCCTGC 469800
TGGCTAATCC GAACAACCCG ACAGGAACAT TTGTCCCGAT TGCTGATATT GAAAGCCTCG 469860
TCGCACTATC TGACCAGTTG GTGGTACTCG ATGAGGCCTA CGTGGACTTC GCTCCCGACA 469920
GCGCTCTGCG TCTCGTTAAC CGCTATTCGA ATCTTCTGCT CTTAAGAACG TTTTCGAAGA 469980
GCTATGCGGC GGCCGGCATT CGCGTCGGCT TCGGCTTCGG TCATCCTGAG GTGATAGGGA 470040
GGCTGCGCAA TATCCAGAAC ATGTTCAATA TGAATGTGAT CGGCCATGCG GTTGGCGTCA 470100
GCATCCTGGC CCACCGCGCC ACCTATAATG AGAACCACCG CCATATCAGG CATGAGAGAG 470160
AGCGGGTGAG GGTGGCGCTG TCGCGACTTG GGTTCTCTGT AACGCCCTCC CATGCAAACT 470220
TTTTGCTGGC GCGGGTGCCT GCAGGACGAG ACGGCGTTTG GTGGCACGCC TGCTTGAAAC 470280
GGAAAAAGAT ACTCGTGGCC GTTCTTCCCG ATGAAGGTCT GGAAGATTGC ATTCGCGTCA 470340
GCATCGGGAC GAAACCACAA ATGGATGCGT TTCTTGCGGC TGTCGAGGAC ATTTCGGGAG 470400
CTCAGCAATC TCGATGATGG GACCGTATAG CAGCTTCAGG CTCGGCTCTT TTGCACGTGG 470460
GAAAAGACAG TGGCGACAGG AAAGAGAGCG ACAACCGGAA GACTGGTCTC CCACAGCGCT 470520
AACTACGCCA CGCCCGTGAG GAAGCTGAGT TCATAGCCTG GCGCCTGCAT GTGTCTAAAG 470580
GCGTGCTCCA CAACACAAGG TCGCTGGGAC GGAAACATCT GCCGGTAGGT GCACTGAAAA 470640
ACTGTTGAGC AATAGCCGAG CACGCAAGGA TGTGGCCTGT CGGCGGCTTA ACGATTTGGC 470700
TTCCGCCACC GTTAGGCAAG GGCAGGCTCA CCGTGAATAG CGATGTTCGG CCAAATAAAG 470760
CCCCCAAGGT CTTATTTCGC TCGAATTGAA GTCCGGGGCT ACCAGGGTTA AGTGAGACGT 470820
TAAATGAGAA AAATCCTTCC ATCAAAGCTA GCTTTCGGCA TATTCGACCA TTTAGACGAG 470880
GACGGTAACG CCATGGCACG GCAATACGCA GACCGCCTCA CGCTAGCGGA AGCATGTGAT 470940
CGTCTCGGCT TTTACGCTTA CCACCTAGCG GAGCACCATT TTTCGCCGCA TGGGAGAAGC 471000
CCATCGCCGA ATCTGTTCCT ATCGAGCGTC GCGCAGCGCA CCCGTCAACT TCGTCTTGGC 471060
CCGCTTGTCA TGCTGCTTAG CCTTTCTCAT CCGCTGCGTG CGTTTGAGGA GATCTGCATG 471120
CTTGACCATT TGAGCGGCGG TAGGGCCGAG TTGGGCATAG GGCGCGGCTC TCTTCCGATA 471180
GAGCTAGGTT ACTTCGGGAT CGATCCGGAC GCGGTGGCTG GACGCTATTT CGAAGCCAGC 471240
GAAATCCTGA TGGAGGCGAT GAGGGGCGGC AATCTTTCCT ACCGCGGCGA CCACTTTGAA 471300
CTAAACAATG TCCCCTTGAT CCTGAGGCCT CATCAACGCC CGCACCCACC GACTTGGATC 471360
GCCACCAACC GACCTGAGTC TGCACGCTGG GCCGCTACAA ACGGCGCAAA TGTCGCATGT 471420
GTGGGACCCG CCTCGCTTGT TCGCAGGATT ACTGATGCCT TCCGTTCCGA GGAAGGACTC 471480
AGCTCTGACG CGAACTGCAA CGCGTCATTT CTCGGACTGC TCCGCATGAT TGTCGTCGGG 471540
CACTCTGCAG AACATGCATA CTCGCTTGCG GCTCCTGCCT TCCAACGATG GCTGAATAAT 471600
TTCAAATTCC TGTACGACCT CCATGCAATT CCAGTTCCGC CAAATCTGCC CCTGACCTTC 471660
GATGCAGCAA TCGAAAGTGA ATTGTGCGTA GTGGGAACGG CGGCTGTTGT GCGACGGACT 471720
CTGCTTGATC AGTTGGAAGA GGCGGGCGCC AACTATCTCC TTTGTCAGGT CGCGTTTGGA 471780
GATTTGCCTT TGGATGCTTC GCTGTATACC GCAATGACCA TTCAATCGGA ACTAATGGAT 471840
CGAGTTGGCT GAGTGCCATG GCGCCTGACG AAGTCAGGCG TGTGTAAAAG TGACGGCTTC 471900
CTGTCGTATC AGGCGCAACT CCAATTCGGC CCAAAAATCA ACAATGGGCT GGTCGGCGCT 471960
AAGTACATCT GACTCGAAGG TGTTTTATCG GGTAGCGTGG CTTCTGGGAA GAGGTAAGAT 472020
GGCCGAGATC GACGCAATAT TGATAACAGA CGTAAACGGA ATAGGAACGA CCGCGGATGA 472080
TCAACTAGCA TTTGTCAGTT TGATTGCTGA TCAAGGGGAA ACTACCACAG TAGCATTCGG 472140
TCCAGAAATT GGCAGCAGAA TAGCTGCTTC GTTCATGGCC GCATGCGGAC AGCTCCAACA 472200
TCAAATCGCT ACGAGAACGG GGAAGGAGGA AAGAAAGTTC AAGCCCTTTG CTGCAGCCGG 472260
ATTCAGCGTC AGAGCTGGTC TGGCCGCCGA TGGCTCCAAT TCGGGAATGT TATCAATTTC 472320
AACCGTAGCG GGCGCTGAGG TTCACTTTAT AGCAACGGAA CGTTCTCTTC GAGAACTCGA 472380
AAACCAATTA ACGTTATTGC TCGAACAATT GCGGTTACGT TCGCGACCGA ATTAATGCGA 472440
TTGTAGCGGT AACAAAGCTC TTATGACTAT CATGGCTAGC AAGGCCTGGC AGTAATGGAT 472500
ATCACCCATT TGGGTAGAAT TCACGCGGGC AATTCGAACG ACAATCATTT TGGGACGAGC 472560
GGCGCTGGCT GGTCGTCCAT GTTGAGTGCA TATTTTCCCA AACGGCGAAT ATGCCCGGTG 472620

TTGGGACTGA GAGTGGCGGC AAGCTTCGGC GTGAAGCCCG CCTGACCACC TGCCGAAGGC 472680
ATGTCGCTTG CTACCGTAAT ACCAACTTCG CTCGGGATCT GATCGAGCGG GAAGTCTCCG 472740
GCGCCGCATC CCAATCGTGC GCTATGCGGA TCTGTCAATC AACAGCGCGT CGCCCTGCCG 472800
CCGCCACGGC CTTTGCCACC AGCCGCATTG AGGCTGGTGG CGTGAGCCGA CTGCCATAAT 472860
TGTCGTGCCG GGCGCAACTC CACCGCTACG TTGCCTGGAT GCGCGACGAA CGCGTTCGCT 472920
GTTTCGTAGT GGCGGATCGG TCTCCTCCTT GTCCATCGCT CGCCAGCGGG TAACTGCTCA 472980
TCGTCTCTCT TTTCGGCACC CCCGCACCCG CAGGCGATAG TGACAGCGCC GATAGGCTTG 473040
CACCTGCGTG CGACCACTCT TCCATCTCCT CAATAGCCTC CATCCAAATA AGCGATTTTA 473100
CATATCTGCT GAGATTTCCC ATTAGAATAG CGTGGCGTTT TGGGTTGAAT TCAATTGGGA 473160
CAAGGATAGC AGTAGACGCG GAGCCTTCTC CACACACGGC TGCCCCATCG AGTGGTATGG 473220
GCTGTCGGCA AACTGCTCCG GTCCGAAGCC GCACTCTCCC GCGCCCTGCT TGAAACCGCC 473280
GAGCCCCCGG TTCGCCCGCA GGGGCCGTGT CAACCGTATA GTCGCTCTAA AGAGGACCAC 473340
CTGTGAAACA TATCGTTGCA ACCTTATTCG GGATACTGTT TGCTACAGCT GCCAATGCCG 473400
CCGGCGATAG TCATGTACTG GCGAGCTTTC CCGAGCTTGA ACGCGCGCTG ACTACCGGCA 473460
AACCGGTGAC TGTGACGGTC GATTTAGGCA TGTGCACTCC GGCGACTTCC GATACACCGT 473520
CGACCAAGAC GCGTGGAGGC GTTAGCATAG ACGGCTATAG GATCACATCT GACGGCACCC 473580
TCGCTTTTGC AGATCAGCAT TTTACCATAG ATCGTGACGG TAAGCCGGTT ATCCAGTTTC 473640
TTCGTTACCG GATCCGGCCG GACGGTGGCG CCGAATTCAC CATGGTCGTC TTCAATGTGC 473700
CCAGTTACGA GCGAAAAGGA ACGAGCCGGG TCTATAAGTG CTCAATTGGT CACGGATTGA 473760
GCTTCTTCTC TTCACAGTGA TCTGGCATGT TGCAGCACGA TCGCGCGGGT TCGGGTGGCG 473820
GATCGTCCCG GATCGGCGGC TGAAGGTTTC CCTGGGGCAC CCGCCCGTGC CATTTGCAGT 473880
GGTCCCGATA GGTAAAGGAA CCCGCCTGCC GCTATCCTTG AGCTTCTTCA GCGGTCTGTT 473940
ATGCCGTCGC TGGTCTGCCA AGCATAGCGG TCATCGAGGT CCTCTTCGAT CACGCCGGCC 474000
GCGTATAACT GATCGAGCAG GTCGGCGACA GCAAGTACGC TTATTCCATC CGTCGAGGCC 474060
CGCATAACCA ACTCCGAGGC TGTAAAGTTG CCGCACGCGA TTAGATGGCC AACCGAGATC 474120
GGAGCGTAGT CGAGCACCCG GTGTTCCATG CTTCGCGAGC GGAGATGGAA GCAGCGGTCT 474180
CCCGCTGTGT ACTCCAGGTC CCTGCGAAAG CGGATCGGCA GGTCGGGGGC TGGGCTCTCG 474240
AGCATATGTT GATCAATCAT GCTCTTGAGC CCGTCCCGGA ACTCGTCAGG CGTCCTGAAT 474300
GACCGTTGAC CCACTATGCG ATACCCGAGG GGCCAGCGTC CGCTACCCGG TACCGGCGTC 474360
ACTAGTTGCA AACGTCTCTG CGGCATATTT ACGAGGAAGC CGGAAACACA GGCGATCGTC 474420
GTGTGATTTC GCTCCGGAAA GGCGATTGCC TCTTGCCTCG CACTGAGTTT CTCCTTCCGC 474480
TTGCGCGCCC GCCCCGTGAA AGCCTTTGCG GTCGGAGCGT CCTTGCCCTG CATGATGAGT 474540
TCGACCCCTG TCAGTTCTTC AGGCGAAAAC GCCCTGTGGA TCTGATTGAG ATGTGCCGTG 474600
CTGAGCACAG AGAAGCGATT CATCGTAGTG CCATAAAGAT TGAAGAGGCC GAGCACGCGC 474660
CTGGTGAGTG CCTCATCCTT GAGCGGGGCG GCCGTCGTTG TTTGCGGCAA TGCGCCCGTG 474720
ATCTGATAGT AGTCGAAGAG AAAGCGGTCG TACTGCGGGT TGTCCATTGG CTCTGTGGCC 474780
CAGTAGCAGA ATCCAGTACG AGCTGCCGGG CCAAACACCT CGCTCGCGAC ACCAACCACG 474840
CCGCGCCAAA GCTCTGCATG CTGTTTGCTA TACTCGTAAT AGCCTTTGAA CGGAGCGGCC 474900
GAGAGGCCGC AGAACCAGCA ACCGACGGTG CACCCTTCGC TCAGCTCGAA AGCGATGATG 474960
GGGTGCGTGA TCGACGGCGC TGATCCGCCC AATTCGTCAT TACAACGCCG AATTTGGCGC 475020
TCGCGCCAGG CATGAAAGCG AGGGTTGGTC ATTGACATAT CGCCATGGTC GCGCAGGAGG 475080
TCGCGATGGC GCAACATGTC GCGCATGTAC TCGTCCCACA TTATCGACAG CGGCCATGGG 475140
GCAGACTCCG GCTTGTAGCG GTAAGTAAGA TGATCATCGC GCCAGAGCGG CAGTATCTCC 475200
AGTGGGTCAA CTTCAATGCC ATAGCGCTCT GTTACCGCGC GAGGGGCGTC GACGCTCTCC 475260
GAAACCGCGG TGCGGAACTT TATGTCACCA GCCAGCCGTT CCATGAAACG CTTGATGTGC 475320
GACAAGGTGC GCCGTTGCTC CGGCGTGCGC CGGTTAAAGA TCTGTCGCCA ATATTCGCCT 475380
TTATAAAGGT GAGCCCTTGA AGCCGTTTCT TGTAGGCCAG ATCGCATGTC TCGTTCCCGA 475440
TGATATTTGC TGTCATGCTC TCTTGGCACC GATCGTCCAA GCCAATGCGC TCCGGTAAAA 475500
AATCAGCTTG AAACATGAAC AACAATTACA ACAAGTTGAA CGACCGCGGC GACGGCGGCG 475560
CCTTTTTCAG CAATATGCAA CGGCCGCGTG AGCCCATCCG TAACGGGACT ATCCAAAGCA 475620
CTAGATTGTA CTAAGCGCGG CAAGGCCGGA ACGCGCGAGC CACGCTTGCT GTTCGCCATC 475680
GCTTCGAGCT TCTTGCCAGT AGACTTCCGT CGGCTGTCCA ATCGGATGTA GTTTTGAACT 475740
TCGTCAAGCG TGATGTTGTA GCCGAGACTC TTTCCGGCCG CTACGATCGG TGCAAGACCC 475800
GTGGCACTTG GCTTGAGGTT TGCAGGCAGC CTGCCTTTGT CTAAATCGTT GACGAATCGC 475860
TCTACGTCAG CTCGCGGCAT AGCTAATTCC TTTGCTCATA GTTGCCAATT GCAGGCGTGG 475920
ATGATGCCGT GAACGAGCAA GCGCCGTGCC AACCTAAAAA TGTCGTCATT TTGTGCATTT 475980
TCAGAGTGCA TGCATTGTCA ACTTACCGAC CTTTAGTCCC GAACCGGCCA ATTGGCCGCT 476040
CCGAAATGTA GACATCCGCA AAATTCTTCA CTAAGGCAGG CTCTCTTTTT TGCCAGCTCA 476100
GTTGCGGATT CCGCCAGTCG GTTCATTCAG TGAAACATCG GGATACGCTC AAAAGCGTGA 476160
CGACGGCGTA GCGGCGTCAG AGAGAAGCGT CGACGAATCG TGCGGAGTGT GGGGGTCACA 476220
AACGGCCGGC ATAGCAGGTC GACGACCCCC ACGGCGGTGG GGAGATCAGA GCTGTATGCG 476280
TATCATCCGG ACATGACCCA GGCTCATGCA ACCCTGACCG CGACCAGCGC TGAAGCGGGC 476340
CGACGGCATC GGGTTGGCAC TTTAGGCACC GACGCATTAG CAATGGACGC GCATCTGCGC 476400
CGACTGGGTA TCGGCGATGT GCATTATGAT CTGTCTAGGT TTCGATCGCC TTGGTCTTCC 476460
GACCCTTCCA TGCACCACGT GCCAACGAGT TTTATTACAG CCTGCTAAAC TAACCGCTTC 476520
ATTCCAACGC GTCCGGAGCC GCGACTACTT TGTCGGACTG ATGACATCGC TGTGTCGCAA 476580
CCCCGCCGCA GTCACTGATT TCGGATGGCT GCAAGAGTTC TTCCAATGGT ACGAAGACTT 476640
GGATTGGCGC AGCAGCTTTA GTCGGATCGC GATCCTCAAG CACCTTGAAA ACACGATGTT 476700
TGAGTGGCGA CGAAGTTGCA AAGTCCCTGT AAGCGCGCTC GAGTGCGGCG CGCGCACGGG 476760

TGGCGGCCGT GCCTGTGGGG AGATCGGAAA AGTCCTCCGC GACCAGATAC TGACCCATGC 476820
GCTTCATTAT GTGGAGCCGT GATGTGTTTA GCACCTTTGG GTCATAGGTG ACGCCGAGGG 476880
CATCGAAGAA CTCCTCGGCG GCCGACAAAT CCTTCAGCCA GGTGAGAATG TCAGTGCCAT 476940
CAGGGGCAAA GGTCGCATCA TCATAAGACA TTGCATTCTC CTACTGGCCG CGGAATAGCT 477000
GCTTTCGTTC CAACACATGG TCCCGGCCCT GCACCCTATG ATGACCCTGG GAAGAGCTGT 477060
GATCGAGACG CCATCAGTGA CGGCTATTCG GACATGCCCA AGCGCTCAAA CAATCGTATG 477120
TGCCGGCAGA GCGTCTACCC GGCAAATACT GGACGAGATT CACGCAGTTT TTTCACAACT 477180
GCGGGTATCA CCCGCAACAC CCGATCTATA TCCTCTTCAC AGTTATAGCC TGACAACGAG 477240
AAGCGGACTG CTCCGACCGC CTGCCTGTGG GGAGTACCCA TGGCTTTTAG CACATGGCTC 477300
GTCGATAAAG AACGAGAGGT ACAGGCGGAT CCGGAGGAGC AGGCGATGCC ATGGCGATTG 477360
AGCAGAAACT GCATGGCTTC ACCACCGACG CCTTCAAAGG CAATGCTCGT TGTGTTGGGC 477420
AACCGCGTCA CCGGATCACC CATTACGAAG ACTTGGGGGA CACGCTCGAG AAGTCCTTTC 477480
TCCAATCGAT CGCGGAATGA TCTTATTATC GCATTGTCCT TGTCCATACA ATCCAACGCG 477540
AGTTCTGCCG CCTTGCCCAG ACCTACTATC CCGGGCGTGT TCTCCGTGCC CGCGCGCCGG 477600
TCGCGCTCCT GGTGGCCACC CTTGATGAGT GCGTGAAAGG GTACCCCGCG CTTTATGTAG 477660
AGTGCCCCGA CGCCCTTCGG GCCATGGAAC TTGTGCGCGG AAAGCGACAG CATGTCGATC 477720
GCCGTTGATT TCAAGTCAAT CGGTAGCTTG CCGATTGCTT GCACCGCATC GGTGTGGAAA 477780
ATTGCGCCGA CTTTTTTGGC CATCTCAGCT AGCTTTACCA CAGGAAAGAT CGTTCCCGTC 477840
TCGTTGTTGG CCCACATGAT CGAAACGATT GCTACACGGT GTGTAAGGGC GTCCTGATAA 477900
GCGTCAAGGT CAAGTCGGCC CTGGTGATCC ACTGGGACCT TGTGCACCTT GGTGCCGCGT 477960
GTCTTCTCCA GATGGGTGCA GAGCGTCAGC ACCGCAGGAT GCTCGACCGC CGAAGTTACG 478020
ATCTCAGTGC GCTCAGGCAT AGCCTCGAGC CCCGAAAGGA TCGCCGCATT GTTGCTTTCG 478080
GTCCCGCCCG AGGTAAAGGT GATCTCTGGG TCGAACTTTG CACCGATCAG TGCTTGCAAC 478140
TGCCTGCGCG CTACGTTAAT CGCTGCCCCG GCAGTAGCAC CAGCGTCATG CATCGAGGAA 478200
GCGTTGCCAA ACACATCTGC AAAGAATGGC AGCATCGCCT GGAGAACTTC CGCATCGACG 478260
CGCGTCGTTG CGTTGTTGTC GAGATAGATA GGCCTCAAGA TAATTCTCCA GCTACCGAAA 478320
CGACGAAAGG TTTACAAATG GCGATCGTGG CATTGTAGAA GTGCTTTTTG ATCCAGCATT 478380
ACTGTTCTCC CGTGCGTGGC GATCAGCCGA ACGATTTGCC CCAGGCTCAC CTCTCCCGCG 478440
CGTTGAAACG TTGAAGACTA ACCCGGGCGA ATCCACCCTT GGGATGACGT CCCGGCCAGG 478500
CCGGCCACAT GGGGTTGGGG GTCCGAGTCA ACAAATATGT TGATCCCATC CTTCTCGATA 478560
ACAGTGTCGC CCTCGCGCGA CTCAGTCTCT AATGCAAAAT GGTATATGCA CAGCCGTCCG 478620
CTTGAACCGT CATATGCATA AGCCATCGGT CGGTTCAGAA TCGCGGGAAG GAGGGAGCTT 478680
CACTGCACTA ACGGCTCTTC CGTTAAGACC TATCATGGAT CGTTTCTCCT GGTTCGAAAA 478740
GGCCTTTGCA AATCGGTCAA GCACAAACTA TGCCAACGAA AAAAACGGTT TTGAAACAAC 478800
ACCGAAACTC AAACATCGGT GTGACATTTG TCCGACAATG TCGGAAATCG GACATTGTCG 478860
AATTAGCCCA CACCAAAGAG CGCCTCTGTG CCGCAAACAA GATGCAATGT GATGACTTCG 478920
GCGGCGCAGC TCTTCGAAAG CTTGATGGCA GACGGAGGAT GCCAGCCATA GCGGCGATTG 478980
CTGGCACTTG CCTCACTCTA CAATGGCGGC CCCCGTTCGG AGGCAGCGCC ATTCGGCAGC 479040
GAGATGCTGC AGTGTTGGGT CCCCAACGAA CGGTGCACCG CGGCGACCGA GCGACACTGA 479100
ATGACGCTCA GCGCCCGGGG CTGGCAAAGG ACGTCGAAAG CAGGCTATCC TTTAAATTCA 479160
CTGGCGGCGC AGATTTGGTC CAGAAGTTGC GCATTTCGGA TGAGGCGACG GTCGGGCGTG 479220
AACTGGTGAC GCCCGGCTTC GCCAGAGCTC CCGCAAATGC AACTACAGCG AACGCCCGCG 479280
GGCAAATGAC AGGCAGCGCT CTTCGACCAC CACACCTCGG GGAAGCCCAG TCCGTATTTC 479340
GGCAGCTCTT TCGGCCGGGC CAGCGCGTCC CAATAGGCGA TTTGGGCGGC CAGCTTGTAA 479400
TAGAGCGGAA TGACGTAATT ATGGGCGAGC AGGACGCGAT CGAGTGCCTT CGTTGCGGCA 479460
ACCAGCGTTT CGCGATCCTT TGCGAAGATC ACGCGCTCAA TCAAGGCGTC GACGCCAGGG 479520
TCGGAGATGC CGGCATAATT CCTGGAGCCC TGGCGTGTGG CGGCTGCCGA TCCCCAGTAG 479580
TCCGCCTGTT CGTTTCCGGG GCTCAAGGAC TGACCCCAAA CTTCCCAGGT CACATCGTAG 479640
TCGAAGGCAC GCTTGCGATT GGTATATTGC GATGGGTCCA CCGTGCGCAC GCGCGCTTCG 479700
ATACCGATCC GCTTAAGGTT CTGCGCATAG GGCAAGGCGA CGCGCTCGAA TGACGGGCTG 479760
CTCAACATGA TCTCGAAGGA GAACGGCTTG CCCGTCTCAG TATTCACCAT CCGGTTACCG 479820
TTGAGCTCGA ACCCCGCCTT GTTCAGGAGC TCGATCGCCT TGCGCAGGTT TTCGCGCGCC 479880
TTTTGCGGCG TGCCGCCGAC CGGGTTGCTA TAAGGGGTGG TGAACACTTC GGGCGGCACG 479940
AGGTCCTTGA CCTCGTTGAG GTTCTTCAGT TCCTTGCCTT CCGGCAGACC GGAGGAGGCG 480000
AGTTCTGTGG CGAAGAAGAA GCTGTTCACG CGCTGATACT GATTGTAGAA GATGGTCCGG 480060
TTCAGTTCTT CGAAGTCGAG CGCATAGTTC AATGCCTGGC GCACCCTCTC GTCGTCGAAG 480120
GGCTTGCGGC GCATGTTCGG CACCATCGCC TGCATCACGG CCGTTGCCCG GAAGGGGTTG 480180
GGAATTTCCT CGCGTTTAAC GCGGCCATCC TTCACCGCCG GAAAATCGAA GGCCGTAGCC 480240
CAGCGCATCG CCTGGTTCTC CCGCCAATAA TCGGTGTTGC CGGAGCGGAA GGCCTCGAAC 480300
TCGACGTCGC GATCGCCAAA AAAGGAATAG GTGATCGAAT CGAAATTGTT CTGCCCGACA 480360
TTGACGTTGA GCGCGACGCC CCAGTAATCG GGCCGGCGCT CATAACGAAT GGTCCCGCCG 480420
GGGGCGAACG AAGCGATCCG GTAGGGACCT GACCCCATCA CCGGTTCAAG CGTCGTTCGC 480480
GAAATGTCGC GCGGCTTGCC GTCCGGTCCA GTGCCCTCCC ACCAGTGCTT CGGCACAATC 480540
CGGATCTGCC CGAGGATATG AGGAAGTTCG TGATTGTTTT TGTCGTCGAA GTGAAAGGTG 480600
ACGTCCCGAT CCCCTGTCTT TTCCGCCTTC ACGACATGCC GATAGTAGCT CTGATAGAGC 480660
GGGTTCAGTT CCTTCGCCTT GTCGAAGCTG AAGACGACGT CTTCCGGCGT CACCGGCTTG 480720
CCGTCGGCCC ATTTCGCTTC CTGCCTCAGC CGGAACGTGG CCGAGGAGAT GTCGTCGGGG 480780
AAGGAAACGC CTTCGGCCAG AAGCCCGTAG GCCGTCGAAA TCTCGTCCTC TGACGGTTTC 480840
ATGAGCGTGT CGAAGACGAA ATCCAGGCCG ACGGCCGTTT CGCCCTTCAC CAGCAGGGGA 480900

TTGAACGTGT CGAAGGTGCC TGTCTGTGAA AGCCTGAGAT CTCCGCCCTT AGGTGCCTCG 480960
GGATTCACAT AGTCGAACTT TTTGAAGCCG GGCGGATATT TGAGATCATC GACGAGCGAC 481020
AGTCCGTAGT GCCAGACCGG CTGCTCTTGA GCCTCCGCAG CCGGCACCAG TAGTAAGGAC 481080
ATGGCCATGA ACGCGGTCGC AAACTTCAGT CTTCCAATGA CGCCGATGTC CAGCATCTCG 481140
TCCTTTCTCC TTGTTTTTAT CCATTCGCCG CCACGGTGTT TGTGGGGCGG CTCCAAGTCG 481200
TTGCAGGATC GGCATGTCAG TGGCCGATCG TCCCTAGATA TGGGCAATTC ACACTCCTCA 481260
AAAATTGATT TTCTAAATTC TGTTCATAGC GTCTATGGAT ATGCGGAGCT TTGTGCCTGG 481320
TAGGGTCGCG GCCTGTGTCG CTGTACCCGT TGTCACTTTT TCTGGCGGCG GCGATGACGT 481380
GGATGTCGAC CAGTGTCGCA AAGTCTTCCT TCGTCGCTGA CAAGCGTCGA CGGGATAACC 481440
AGTATCAGGA AACCGGTCAT CAACTGTTCT TGCGCCTTTT CCGCACAGCC GGCGACCGCC 481500
TCGGCAATGA GCGAGGCCGG ATCGATAAGC ACTGCCCGCA CCCGACTGCA TCACATTGGT 481560
GACCGCGAGG TGGACGGCAA GTACCAAAGT CGAGAAGGTC AGGTATCAAA CTCGCCTTGC 481620
CGGCGATGGG ACCAACCTTG GCGAGACCGG TTATGCCTGC GATCTCCGTC GCGACCTTCA 481680
GGAAGATCAC CGGTGCAATG ATCATTCCAA GCTTTCCGAT GTCCGGATAG AAATATCCAA 481740
ACAGGATGCC CGCGATCGCT GCCAGAACCT GGGCATAGAG ATGGCGATAT AAGGGTGGCT 481800
TGGCGCGGGT CTCCGCGGAA TGCTCAATGG TCATCAGGAG ATCCTCCATA TGGGGCGCGC 481860
CCGGTCGCCT GCCAAGCCGA TTCTCCGAAA ACCGACAGCG GCGACTGTCA TGTGAAGCTG 481920
ATGAGCAATG ACCGTGCCAG TTGCCGTTAA ACCGCAGATC CTGACGCCCG AGGTGCTTAC 481980
TGTTCTCGAC CCGACCAGCC CCGCCATCCC GGTAACCGGC CACGGCGACA TACTGATGGT 482040
AGTGCAGGCG ATTAGGACGA CGCCTCTGAC ATTATCGCCA AGCTCTTCGC CGCCGACGTG 482100
ATCCACGAAT TTGAGTGCGC GAACACCATC GGTCTGGCAC GACGGGACTG CCACCTCACC 482160
GCCTTCTGTC CATAATTCTC ACCTTTGTCT CAAGGCAAAC CTTGGCCGGC AAGAAATGTT 482220
ATTTGCCTCC ATCAAATGAC CGCCGAGACA TCTTAATCGG CCTCCAGCCA AAGTCCGCAG 482280
CCGCAGCATC GATTATCGAT AAAGACCCGG GTGGAGGATA GCGAGATGAC AGATTTTTGG 482340
CGCCACCGAC AGAACTGGCA CAAATCTTGA ACCTGCTCAT TGCGAGGCGG CGGAGCTGCT 482400
CATTGCGGCA ACAATCCTAT GCCTCGACTG CGCCACGTAT GAAGATTTCA CAATACTATT 482460
CAATTGAGTT CTTGCGACAA ATGGGCGTTG ACGATGTGCA AGATGGTCGC CAGAGCCGCC 482520
GTAACGAGAG GAAAGCGAAA TGTCCAGGAC CGCAGAAAAG CCCTTCGAAA CTGTCGAAAA 482580
CTCGATCGTC GGTCAATCCA CGGCAATCAA TAGGGGCGAG CAATTGACGC ATGCTATTGA 482640
GTTACAGGAG CTGGCCGGAT CCGTCTTCAA GTCAACAATG ATGGTGGGAT TTGAACTGTC 482700
GCTGAACACT TTCGCTGCAG TCCAAGCCAA CACCTTCGCT AATTTGCACC ATCTTAGCGA 482760
GTTTCTCGGG GCAAAATCGC CGTCCCAAGT TTTCGACCTA CAGTGTTCCT TCCTGCGCAA 482820
GCGTATAGAC ATGGGTGTCG AGCAGGTCAA AGAATCCCAG GCGCTCAGCA CCAAGGCTTT 482880
GATTGATATG TCGAAGCCAA TCAAGGAATT CTTTGAAAAG GCTATAGCAG ACCTCAAGAC 482940
GGCCTGATCG TCAGCGATCC CCGCCGTGGC AGGCCGTGAC CCGCCACGGC TTTGCGCTCA 483000
GTAGCCTGGA GTACCATTCG ATGATTTCCG CCGATTTGTC GGATTGATGA CATCGGCTTG 483060
TGTTATAGGT GGGGGGAAAT CCTGGACGCT ATCTACGGCT TCTTTCGCCC TCCTGCAGGA 483120
AGGCGGCACC TCCCCATTGC GCATTCAGGG ATCACATCGG CCCCAAGGTT TCTGCTCCGG 483180
TCTCTTGTGC GCACCGCAGC GCAGGTCCTT GCGCACAAGT AGTCACGTTG ATCTTCATTC 483240
AGCTCCCTCG ACGTCTCGAA GACCCAACCG CGCAACATAC ACCGGATCAC GAATGGACGT 483300
CGCGTAGGCC GCGGCTCACG AAAGGCAGGG ATGTTGAACT GGGACGACTT CTGGCCTCGC 483360
CTGACGCGTC CTAATAGGGA CCACACCGTC AGCGCCGGCC GAATGACACG TGGCACCGCG 483420
ACCTGCACGG CGCCTGATCT GCAAACGAAG CGACTATCAA GTTCGCAACA TCTATCCTGG 483480
CCGCCCAGCG CGGCTGTCAT GAATCGTGGG ACACTCTGCA AGCCGGCTTC AGCACAGCCT 483540
GGTTCGCCGA GCACCACTTC GACAACTACA GCGGTCCGTC GCCTTTGTCG ATCGTGACCC 483600
ACTGCGCCGG TTTGACTACG ACCATTGGCG CGTCGGACGC TTTCGACAAT TGTTTGTGAA 483660
ATTGTCGGCT CCGCGACACA GGCTTGCTTC TGGGTCGGCT ACTTCTCCTA ATCTAAGTAG 483720
CTGTAAAAGA AGGTAAACCA GCTTCTGCTG GGCTCACCCT CCTCGACTTG GCACGGCTTT 483780
TGAAGCCTTC CTTGTGCAGG CGGCTTGAAG CTGCCGCTGT ATTCGTGTTG CGGGCAACCG 483840
CGATGGTTTC GAACAACGAA GGAAAGCAAC ATGGCAGGTC TGCGTCAAAT CGCGTTTTAC 483900
GGCAAGGGCG GTATCGGCAA GTCGACCACC TCGCAGAACA CGCTCGCCGC CCTTGTCGAC 483960
CTCGGGCAGA AGATCCTCAT CGTCGGCTGC GATCCCAAGG CCGACTCCAC CCGGCTCATC 484020
CTCAACGCGA AGGCGCAGGA CACGGTCCTG CATCTGGCGG CAAAGGAGGG ATCGGTGGAA 484080
GACCTCGAGG TCGAGGACGT GCTCAAGGTC GGCTACAAGG GCATCAAATG CGTCGAGTCC 484140
GGCGGCCCCG AACCGGGTGT CGGCTGCGCC GGCCGCGGCG TCATCACCTC GATCAACTTC 484200
CTGGAGGAAA ATGGCGCCTA TGACGATGTC GACTACGTCT CCTACGACGT GCTGGGCGAC 484260
GTGGTGTGCG GCGGCTTCGC GATGCCGATC CGCGAGAACA AGGCGCAGGA AATCTACATC 484320
GTCATGTCCG GCGAGATGAT GGCGCTCTAT GCCGCCAACA ACATCGCCAA GGGGATCCTC 484380
AAATACGCCC ATTCGGGCGG CGTGCGGCTC GGCGGGCTGA TTTGCAACGA GCGCCAGACG 484440
GACCGCGAGC TCGATCTCGC CGAGGCGCTG GCGGCCAAGC TCAATTCCAG GCTCATCCAC 484500
TTCGTGCCGC GCGACAACAT CGTCCAGCAC GCCGAGCTCA GGAAGATGAC GGTGATCCAG 484560
TATGCCCCGG AGTCGCAACA GGCGGCGGAG TATCGCGCGC TGGCCGACAA GATCCATGCC 484620
AATTCCGGCC AGGGCACCGT CCCGACCCCG ATCACCATGG AGGAGCTGGA GGACATGCTG 484680
CTCGATTTCG GCGTCATGAA GACCGACGAG CAGATGCTTG CCGAACTTCA GGCCAAGGAA 484740
GCGGCGGCAG CGGCCCAGTG ACCGCCGCCG CAGACGCTGC CCGGGACGGT GATCCGGTGC 484800
GACATTCCAC CAATGGTGCC TCTTCTTGGA GGACACGCAA AAAAGGGGGC AGGCCCAATG 484860
AGCCTCGATT ACGAGAATGA CAGTGCGCTC CATCAGGAGC TGATCACGCA AGTGCTGTCG 484920
CAGTACCCAC ACAAGGCGGC CAAGCGTCGC CAAAAGCACC TCAGTGTCGC ATCGGACCGC 484980
GAGGCGGTCG GGGAGGAGGG CGAGACCCTC TCCGAATGCG ACGTGAAGTC GAACATCAAG 485040

TCGATCCCCG GGGTGATGAC GATCCGCGGC TGCGCCTATG CGGGCTCGAA GGGCGTGGTC 485100
TGGGGCCCGG TCAAGGATAT GGTCCACATC TCGCACGGCC CGGTTGGCTG CGGTCAATAT 485160
TCCTGGTCGC AGCGCCGCAA CTATTATGTC GGCACCACCG GCGTCGACAC CTTCGTGACG 485220
ATGCAGTTCA CCTCCGACTT TCAGGAGAAG GACATCGTCT TTGGTGGCGA CAAGAAGCTG 485280
GAACAGGTCA TCGACGAGAT CGAGGAGCTG TTTCCCCTCA ACAACGGCAT CACCATCCAG 485340
TCCGAATGTC CGATCGGCCT GATTGGCGAC GACATCGAAG CGGTGTCGCG CAAGAAGGCC 485400
GCCGAACACG AAACGACGAT CGTGCCGGTG CGCTGCGAAG GCTTCCGCGG CGTCTCGCAG 485460
TCGCTCGGCC ATCACATCGC CAACGACGCC ATCCGCGACT GGGTGTTCGA CAAGGCGGAC 485520
GGCAAGACGG ACGTCGAGTT CGAAACCGGT CCCTACGATG TCAACGTCAT CGGCGATTAC 485580
AACATCGGCG GCGACGCCTG GGCGTCGCGC ATCCTGCTCG AGGAGATCGG GCTGCGCGTC 485640
GTCGGCAACT GGTCGGGCGA CGCCACGCTC GCGGAAGTGG AGCGGGCCCC CAGGGCCAAG 485700
CTCAACCTCA TCCACTGCTA CCGGTCGATG AACTACATCT GCCGGCACAT GGAGGAAAGA 485760
TACGCCATCC CCTGGATGGA ATACAACTTC TTCGGCCCCT CCCAGATCGA AGCCTCTCTG 485820
CGCAAGATAG CCAGGCATTT CGGCCCGACG ATCGAAGAAC GGGCCGAGAG GGTCATCGCC 485880
AAGTACCGGC CGCTGGTCGA CGCCGTGATC GACAAGTACT GGCCGCGCCT CCAGGGCAAG 485940
CGAGTGATGC TCTATGTCGG TGGTTTGCGC CCGCGCCACG TCATCACCGC CTATGAGGAC 486000
CTCGGCATGC AGATCGTCGG CACCGGCTAC GAATTCGCCC ACAACGACGA CTACCAGCGC 486060
ACCGGCCACT ACGTGAAGAC GGGCACGCTG ATCTATGACG ACGCGACCAG TTACGAACTG 486120
GACACGTTCA TCGAGCGGAT CCGCCCCGAT CTGGTCGGCT CCGGCATCAA GGAGAAGTAT 486180
CCGGTGCAGA AGATGGGCAT CCCGTTTCGC CAGATGCACT CCTGGGATTA TTCCGGCCCC 486240
TATCACGGCT ATGACGGCTT CGCCATCTTC GCCCGCGACA TGGATCTCGC CATCAACAAT 486300
CCGGTCTGGG ATCTCTACGA CGCGCCCTGG AAGAAAATGA CCGTGCCGAC GGCCGCAGTT 486360
GCAGCCGAAT GATCGGCCGG TCTTGCGCGG CACGAGGACC GCGGCAAGAC CGGAAACCTC 486420
TCGAACATCC CTGGGCCTAA GAGGCCAGAT CAAGAAGGTC AAACACCATG CCGCAATCGG 486480
CTGAGAAGAT ACTCGACCAT GCGCCGCTGT TTCGCGAGCC GGAATACCGG CAGATGCTCG 486540
CGGAGAAGAA GCTGAACTTC GAATGTCCGC ACCCCGAACG GCTCGTCACG GACCAGCGCG 486600
AATACAGCAA GGGCTGGGAA TATCGCGAGA AAAACCTCGC CCGCGAGGCG CTCGTCGTCA 486660
ACCCCGCCAA GGCCTGTCAA CCGTTGGGGG CGGTGTTCGC AGCCGCCGGC TTCGAGCGGA 486720
CGATGTCGTT CGTCCATGGC AGTCAGGGCT GCGTGGCCTA TTATCGCTCG CACTTGTCGC 486780
GCCACTTCAA GGAGCCGGCT TCGGCCGTTT CGTCCTCGAT GACCGAGGAT GCGGCGGTGT 486840
TCGGCGGCCT GAAGAACATG GTCGACGGGC TCGCCAATAC CTACGCGCTC TACGATCCGA 486900
AGATGATTGC CGTCTCCACC ACCTGTATGG CCGAGGTCAT CGGCGACGAC CTGCATGGCT 486960
TCATTGAGAA CGCCAAGAGC GAAGGCGCAG TCCCGCCCGA ATTCGACGTG CCGTTCGCTC 487020
ACACGCCCGC CTTCGTCGGC AGCCATGTCG ACGGCTATGA CAGCATGGTC AAGGGCATCC 487080
TGGAGCACTT CTGGAAGGGC CAGGCGCGCA CCCAAGCGGC CGGCACGATC AACATCATCC 487140
CGGGCTTCGA CGGCTTTTGC GTCGGCAACA ACCGCGAGCT TCAGCGCCTG CTCACCCTGA 487200
TGGGCGTGTC CTACACCTTC ATCCAGGATG CCTCCGACCA GTTCGATACG CCGTCCGACG 487260
GCGAATACCG CATGTATGAC GGGGGCACGA CGATCAAGGC GCTGCGGGCG GCACTCAATG 487320
CCGAGGCGAC GCTGTCGCTG CAGCACTACA ACAGCCGCAA GACGCTCGAA TATTGCCGGG 487380
AGGTCGGTCA GGCCACCGCC GCCTTCCATT ACCCGCTCGG GATCAACGCC ACCGACGCGT 487440
TCCTGATGAA GGTGTCGGCG ATTTCCGGCC GGGAAATCCC CGAGACGATA CGCCTGGAAC 487500
GCGGCCGGCT GGTCGACGCC ATGGCCGACA GCCAATCCTG GCTGCATGGC AAGACATACG 487560
CGATCTACGG CGATCCGGAC TTCGTCTACG CCATGGCCCG CTTCGTCATG GAGACCGGCG 487620
GCGAGCCGCG GCATTGCCTC GCCACCAACG GCACGGCGGC CTGGCAGGCC GAGATGACCG 487680
AGCTGCTCGC CTCTTCTCCC TTCGGCAAGC AGGCAAAGGT CTGGCCGGGA AAGGATCTCT 487740
GGGCCCTGCG CTCGCTGCTC TTCACCGAGC CGGTCGACCT GCTGATCGGC AATTCCTACG 487800
GCAAGTATCT CGAGCGCGAT ACCGGCACGC CGCTGATCCG GCTGATGTTC CCGATCTTCG 487860
ACCGCCACCA CCACCACCGC TTTCCGCTCA TGGGCTACCA GGGCGGCCTG CGCCTGCTGA 487920
CGACGATCCT CGACACGATC TTCGACCGCC TCGATCGCGA AACGATGCAG ACGGCGGTGA 487980
CCGATTATTC CTATGACCTG ACCCGCTAAA AGCGGCGGTC GGCAAAACGC CGGCCCGGGA 488040
ACCGCACCAT GGGATCGCGA GCGTTGAAAT GGGCTGATCG TTTAGCTCAT GCGCTATCGG 488100
AACTGCGCTC GGAAGTGCAT TCGGACAGCC ACCTTATCAT TCTAAAGGGG CATAGCGGCA 488160
TATGAATTTT GCATGGAGAG AGACAGACGG TTGGTAGTAT TGCCGGCCAC TCACGGGATC 488220
GCCTCGATTA AAGAGGGCCC GTGCTTCAGG ACGAGGACTA AATGATGACA GCGTTATCTG 488280
ATCCCCGGTT TACTCGAGCT GTCAGTGACG ATGGATCGTT GGCCACCCCT TTCATGAAAT 488340
GCCTCGCGCG GTTGGTCCGC GCTCATGATT CCTACGGATT ATGGAAGGAT AAATGTGACG 488400
CCGAGTTGCT GGCCAACTTC ACAGTTACGG AGGAGCAGCG GCGAGCTATC CCCGTCATCG 488460
GCGACCCCGA GCCAGACGTG CTGTTGAGGC TCGACCTTTT TTACGCCGCC GTCGGCGTCG 488520
TTATCGAGGA GCGCTCGGGG CTTTTGATCT CGCGAACGCT GGAGATCAGC GATGAGGGCA 488580
TCGGCCGAGT GCTCTTCACA ACCAGGCGGC TGGTGGTGCT GTCGAAGACC CTACGTGACG 488640
TTCACCGGTT CGGCTTCAAT ACACTTGGCA AATGCGCCAA GACTGGCACG AAGTTGGTCA 488700
AGGATGCGAT CAAAAGCATC GAAACTTATC CGGACGTGGC GCGGGCATAA TGTGCCTGCA 488760
AGGAATAAGA CCATGACAGA TCTTGAAGAA CTCAAGCAGA GGGTTCAAAA ACTGCAGTCA 488820
CGCGCTGCGA CTGCGAAGAC GCAATTGCAT GACCTTGCCG AGTGCCTTCC GAACTATTGG 488880
ACCGAAATAG TGGCCGTCGC CGAAAAAACA TTCGACGCTT TTGCTCAGTT GGACGCTGCC 488940
AAAAGAGAAC TCGCCGCGTC GGAGAATTCA CGATGAAAAG CTCGTTCGTG ACCCGCCGCT 489000
TCAGCTGGTG CCGGAATATC TATTATGCAT CGGCTGCGGC TGCTGACTCG GGGCGCGAGG 489060
TATGCACCTT CACGGGATCA ATGACGCAGG TGAAATTCTC GGTGTTTTGC GACGCCGAGG 489120
ACGATCATTC CAGCAACCAG ATCATGTGAT CATGTCCGGC GACAGCGCCG GCCGCTGTAT 489180

CGGCTGCGGA GCCGGAGTGC GTGTTTACCC AAACTGCCAG AATCATGTTG CGGCCGACGA 489240
CATTGCCGCA TGATCCCACA AACAAACCAG GAAAACGGCG TTGTCTTTTG GTATCTTTCA 489300
TCGAATTCTA TGGCTGTTCC TGTGCGCCAA CACTCTTATA GTCTACCTCG TAACGGGGTC 489360
CATCAGTGAC GCAGTCGTCA CGACTATGGT TGGTTCGCTG CTTCTGCAGC TAACTTATTT 489420
CGCAAACGTA CTCTTTCTGC TCTGGCGGGC TCACTGCGCC CGGAGAGCTC GTCAAACGAC 489480
CGGGCAATTC CATGGAGAGG AACAGCCGGG GGACCCTCGT ATAGCGGGCA CTCACGGGCG 489540
CACGGATGGG GATCCGTGCT TCGAGGACGA GGACTCTCGA TAGGCCCCTT GCTAACCGCT 489600
TCATGAAATC TCTCTCGTGC GGCTAAATCC CCGCTCAGCT TTGCTTTCGG TGTGCAAGGA 489660
CAAGTGCCCG CGCTGGATTT CTGGCCGACT TCACTGTCAC CGAGGACCAG CGCCGTGCGA 489720
CCGCGTCAGC CCTTGAAGCG CGGAACAAGA GAGTGCGTAA GCTGCAGCCG TTCTGGAAAG 489780
CGAACATGGA ATTTGCGTAA TGTCGCCAAG GACCTTCCAA TCAATGGAGC TGATCATGGT 489840
AGTCGCCGAG AATACGTTGG CGCCTTTGCT CAGGTTAGCG CTGCCGGATT GCTGCCCGAC 489900
GGCACAATCT GGGGAATGCT ACCGCTTCAG ATGGTCCTAC ATTGAATCTT ACTGTGGGTT 489960
CTCCCTACAG ATGCGCCACC TTTTAACATA ATCTTGACGA TAGTGAAAAG TGATGCTGGA 490020
TCTGACGAAT GCGAAGCGCA TAGGAATCAT CGGCGGCGGC ATTGTCGGTT GGCTTGCAGC 490080
GATAGCGCTT CGCCGTGTTT TCGACGTTGA TGTCGATGTA ACGGTCATAG AGGCTCCGAC 490140
GGTGTTTCCG CTTGGCCCAG GGGAGGGGGG CTCGCTCAAC CTGATTGACA CGTTGTGTCG 490200
CAATGAGCTC GACCTGGACG TTTTCATTGG TGAAGCCGGC GCCACCCACA AGCTTGGTGT 490260
GCTCTATGAA AATTGGAGGG GTGGAGGAAT CCCGGATCGC TACTACCGCA TGTTCGGCGG 490320
ATCGGGCATA CCGGAAATCG AATGTCGCGT CGGCGGTTTC TTCCCTCTGC TGTCGGCGAG 490380
AATTGCCGCA GGTGAGAACC TTCACACGTG CATCCCTGGC TTTGAGTTAA TCACAAAGAA 490440
GGCATCGCAA GTGGAAATCG ACGAGTTGCT GGCAACCGGT GAGTCTGGAC TCTACCCTTC 490500
GTTTCACTTC AATCACGCCG GCTTCGAGCG ATACCTAAGG CGCGTCGGGC TGGCACGTGG 490560
GATCACTTCT CGTAGGGCTG TGGTACACGG GATGAGACTC GACGACCGAG GGCATGTAAA 490620
CGCTTTCCAG CTCGGAGGCG AAGAACTTGA AGTCGACTTC GCCGTCGATG CGTCCGGATT 490680
TGCCAGATTG GGTCTCGGCA AAGTCTTTAA TACGCGCTGG TGTTCGTTCG CAAATGTGCT 490740
GCCTACTGAT CGTGCGATCA TCTTTGAGCT TGAGCCGCGG GGATCATCCC CCGTTACCCG 490800
TGCCACCGCC ATGAAAGCCG GATGGATGTG GGAAGCCCCA TTGAATCGCT CGATCAGTGC 490860
CGGTTATGCA TTCAGCAGCA GATATGCGGA CGCTGCGATG GCAATCGCCG AGGTTGAGAA 490920
TCACTATGGA TTCCGTGTGG AGGCGAAACA CGAGCTTTCG CTGGATCAAG GTTACTTTTC 490980
GACCGCATGG GTTAACAATT TCGTGGCTTT GGGAACAGCG TCGGGTTTTG TTGAGCCCCT 491040
GGAGGCTGCA CTTGCCGCCC ACACCTTCGA AGCGTTAAGG AATTTGGAGC GCATTCTCGC 491100
CAACGGAAGC GGCATTGTGC CCGCTCGGGC CATCGAAGGC TATAACAGCG CAAACGCACG 491160
GTGCTGGACG GGTGTTCGGG ACTTCCTGAG GCTGCACTAC GATTCCAAGC GAATTGACAC 491220
ACCGTTTTGG CGAGATCTTG CCGCAGCCGA GTTGCCGGAA GGATATGCCA ACCTGAGGGC 491280
TTGCTTCCAA AAACGGACAC CACGCTTTAT CGATATCCAA CCGTATGTCG GAAGCGGATG 491340
GCAGAGCCTC TTCCACGAAA TTGATTGGAT TTCAGTGGCG GTCCCTCTAG GAGTAGTGCC 491400
GCAGGCGGCC GCATGCGCCG AACTACGCAG GCTCTCTACG GAAAGTCGAA GTGAGGTCCA 491460
GGCCTATGTT GATCGGCTGA AGGGAACAAT AGCCAAGATC AGCAGCACGA GGGGATACAT 491520
GCACTAATCG TCACACAAAA GCCACTCCGC GGCCGAAGCC GTGTACCAGG GTCTTGCTTC 491580
ATGATCATGG CGGAGCGGCA CATTGATCGT GAGCAGGGTG TCTCAAAGCC GGTCAAATCC 491640
GCGGCTGCGA ACGACTAGGG TCGATATTCC ACTGAATCAC GCGGAGCTGT CATGTGCAAC 491700
GCTTCCTGTA GTAGTACCTG CCGCATCCAG ATGCTCGCCT GATCCCTGTT GTGTAGGCTA 491760
GGCCACTGGA CAGCTTGGGC GAATGGTGGA AGTGCCAGTG GAAGTTCGGC GACCCGCAGC 491820
GGCGTTGTTT TTGCGAAATG TTCCACCAGC CGCAACGGCA TGGTCGCTAT GCGATCGGTT 491880
CCCGACAGCA AAGGCGGGAT TAGACTAAAT CCCGGCACGA CGACTTCGAT ACGCCTCTTG 491940
AGACCGTGCT CAAGCAACAA CCAATTCTCG ATGGAGGGCT TTAGCGTACG TCCGAACTTA 492000
GCCGCAATAT GACCCATCGA TCCGTAGTTT TCGAAAGAAA GCTGCCGTTT TAACTGCTTG 492060
TTAGTGGGGC AACCTACGCA AACCAGCGTG TCGTCGAGCA GTTTCGCCTT TGGATGATCG 492120
CTCGACGCGA ACAATTCCGG AAGGATCACG AAATCGACGT CACCGTACCG GAGAAGTTCA 492180
TCGGGATCCT CAGTGAGAGG CAGCAACTCG AAGCCGATGC CAGGCGCCTC TCGCGCCAGC 492240
CGCTTCACGA CTCTTTCAAA AAACACCAGC ATCATGACGT CGGAAAGCCT GATCCTGAAG 492300
CGTCGCTCCG ACTGAACTGG GTTAAACATA TCCCAAGAAA TGATGGAGAA CTGGATGTGC 492360
AGCAGAGCAT CGCGGACTGC GGGGGCGAGC GCTATCGCAC GCGGAGTTGG GATAAGTTCG 492420
CGACCTCGCA TCGTGAACAA GTCGTCGCCG AAATAGGTGC GTAGGCGGGC GATGGCGGCG 492480
CTCATGGCCG GCTGACTGAG GTTGATACTG CGAGCAGCTG CGGTAACGCT TCGCTTTGTC 492540
ATCAGAGCAT CGAGCGCTAC GAGCAGATTA AGATCAAGTC CCTTAAAACG CATTATATAA 492600
TTCAACCAAA GCGTTTCTGC CCTCATAGAA CAGGTAGTTT TCAGTGATGT TACAGAGTGA 492660
GCGCCGTTGC CTTGTTCCCG CTGCTCAGAA GAACATGAGG GAGTGGAGCG CACTCCGTCC 492720
CGAGTACCGT AAAGAGCGGG ACTATCCCAC CACATTCGAC ACACCAAATG AGTGGTGCGT 492780
TGCGACAAAA CTTCGGCTTG CAGAGGCCTT TCCAGCGTGC TGATGAGAAT GGAGTGCAAC 492840
CCGGACCCCG GCACTTTCGG GAGCTGACGT CGGTGACCAG CTGTTCCCCG ATAACAGAGT 492900
GACCCTCGGT TAGCCTGTTC TTCAATGCGA TATTGGTTTT GCGGGCGGTC TCAATTTCGG 492960
ACGTGGTTTC CAATCTGCTG GGGGCATCAG TGGCTCCGGG CAGTCCGCAG TTTTCGAGCC 493020
GATCTGGCAC GCGGCTGTGG AACTCTTCGC CCATCGCCTC AATGAACCAT TCCTGCGTGC 493080
CAGCAAAGGC CTTGAGTTCG ACCCTCTTTA GCGACGCCGA CCTCCTCAAC CCGATCGACG 493140
CGTCGGGTTA ATGTCTCGAC AGCAGGATCA GGAGAGGACA CCGGTTCGAT CCACTGCGAC 493200
CCGCCCAGGA TCACATTGTC TTGTGCGGGC GTGCCGCCTG GCTGGAACTT TCCTGAGTGC 493260
ATCAATGGCA TCCTTCTGAT AATCAGACGA AGCGATGGCG GATACTAGTG CCGCATCGAC 493320

CCGGTCGCGC TATGCAAAGT TCATATCCCG CCATGCGCTT GAGATATGGT TCTAAACTCT 493380
TTGCAGGATC AGTGCTGCCA GCCGAACAGT CCTGACCCAC AGATGGAGTA AAACGACCGA 493440
CCTCTCACTT TCGCAGAGGA CGAATAGCTC CTGATTGAAC CTTCAGGAGC TTGACATCGC 493500
GGACTGCAGA CGGCAGGCGA CACACCCGCG AAGCCTTTTT GTATGCCAGC TTGCTCCTAG 493560
TTTCTTAGTT CGCCGCGAAT CGCCTCACTT CAACCGCGTC GGAACCAAAC GTTGATCCGA 493620
TGTGATGAGC CATTAATGCC AGACGTGCCC CACTCCACAT TGGTTTCCGA AGAGCACCAT 493680
TAAATCTGCG CCCAATATCT ACTGCTGCTG TTAATCAGCC TGGCTGACAG AAAGCTGACG 493740
CGGAGGGGTT AGGGCGTTGT TCATGTACAT CCACAAAACT TCCTCCAGCG AATTTAGGAT 493800
TGGGTAATAG TCATTCGCGC TAGTGTGTTC TGGATGTCCA TCAGTGTTCG TCTCGCGATC 493860
CTTGATCGAA CCGGAGCTCG CCGTTCAGCC TGAGTGGTTC ATGTAACGGC AAAGCAGTGT 493920
GCGACGATAG CTCGACCCCC GAAGTAAATG TAGCGAAATG CATCCATGAT GCAGATGCCT 493980
TCGATCCGAA GAATCGATTT GCTCAATCAG TTTCTCTTAA GGCAAAGGCT GATCTAATGA 494040
AGGTGTAAGG GATATCAATG CGCAGAGAAA TCTCAGGTGT ATTTTTGGCA TCATGGGCTC 494100
GCCGACCGGT GTATTTCTGC TCGTCAACAG ACCTTCGCCC CCGCTATCAC AGGTGTTTGG 494160
GTTACATGCT CCTTCGATTA GCCCTCGCAC GGAGCGGGTA CGAAAACTGC GATCAGTCGC 494220
GCGGTACTGC TGTCACCCCG GTCATAGTCC GCGGTGACGT GCTCAATTGG CTGTCCTGCC 494280
TTGATAGGAG AATGAAATGC GAACGTTGCT CGTGGATACG GATCTTACCC GCGCGGTCCG 494340
AGGCGCGCTC GGTGACGGCG GCTTTGCCGT TGATGTAGTT GGCACGCTGG AACAGGCGTC 494400
GAGCGCATTT TTTTCAGCGA GCTATGAAAT TCTCCTGCTG GAGTTGGTAC TGCCAGATGG 494460
CGATGGACTG GATTGGCTGA GGCAGCTAAG GAGCGACGGG TATTCAGTTC CTGCCGTCAT 494520
TATGAGCAGG CTCGACGATC TCGAGAAACG AATTTCGGTA TTCAATAGTG GCGCGGACGA 494580
TTTTCTCCGT AAACCCGTCT CTACGGATGA GCTCATCGCC AGAATGCGGG CCCTTCTGCG 494640
CCGATCGACA CAGATCACTT GCCCCATCAT TGAATTTGGC AACCTCCACT TCGATCCGAT 494700
CGGCCGACAG GTGTCGGTTG ACGGTCATCC GCTAATGATC GCACGTCGCG AACTATGCAT 494760
TCTAGAGCAT CTGCTTAACC GCGCAGGTCG CATCGTGCCG CGTGCGCGGT TGGAAGATCA 494820
ACTCTATTCG TTCAACGACG AAGTTTCAGG CAACGCGCTT GAAGCCGGAA TCTACCGCTT 494880
ACGCGGGTAT CTCAGTAGGT CAGGTGCCAC GTTGCGGATC AGGACCGTGC GCGGCATTGG 494940
CTACACTCTT GAATTGACTG ACGCGTCATC AGCATAGTCG AATTTGAAGG AAGGACTGCC 495000
TTGAAAGCCA CTCCAACTGG TCATGCTAGC GCCGATCGAT GGCCGACCCC CATCGTCGGT 495060
TCAAGGCCAA CTGTTCCATA CTGGAGCAAT CTTCTCTACG CGCTCGTTCT GTTGTCTCCT 495120
ATGTCCGCCG CTGCCGAAAA CAACGGGGAT GAAGCGGTGC CTCGTGCCGC TCCCAACAGC 495180
ATCAACGCCA CGCTGAACCT TTCCTCTTCG CTGGGCAAGA CAGTTCATTT GCCCGCGCCA 495240
GCCGCGACCA TCTTTGTGGC CGACCCAACA ATTGCTGATT ATCAGGCACC CTCCAATAGA 495300
ACGATCTTCG TCTTTGGCAA GAAATTTGGT CGGACCAGCC TATTCGCTCT GGACGAAAAC 495360
GGCGAGGCTC TCGCCGAGTT GCATGTTGTA GTAACGCAGC CGATCGCAGA TTTGCGTGCC 495420
ATGTTGCGAG ACCAGGTCGG CGACTACCCG ATCCACGTCA GCTATACGCC ACGCGGCGCA 495480
ATCCTCAGCG GTACAGCGCC CAATGCCGAA GTCGTTGACA TTGCAAAAAG AGTTACTGAG 495540
CAATTTCTCG GTGACGGTGC GCCGATCGTC AACAATATCA AGGTCGCCGG CTCTTTGCAG 495600
GTTAATCTCA GTGTGCGCGT AGCGGAAGTC TCCCGCAGCG GTCTGAAAGC ACTCGGCATC 495660
AACTTGTCCG CGTTCGGTCA ATTCGGCAAT TTCAAGGTGG GTGTGTTAAA CAGAGGTGCT 495720
GGACTTGGTT CGGCAACTGG TAGCGGCGGT ACAGCAGAAA TCGGATTCGA CAATGATGCT 495780
GTCAGCGTCG GCGCGGTTCT CGACGCGCTT GCTAAGGAGC ACATAGCTTC CGTCTTGGCC 495840
GAGCCGAACC TCACTGCTAT GTCGGGTGAA ACTGCCAGCT TCCTCGCCGG CGGCGAATTT 495900
CCCATTCCTG TCCTGCAGGA AAATGGCAAA ACCTCGGTGG AATTTCGCCG CTTCGGCGTC 495960
AGTCTTGAAT TCGTACCAAC TGTTCTCGAC AACAACCTAA TCAACATTCA CGTAAAGCCG 496020
GAAGTCAGTG AACTATCGTT ACAAGGTGCC GTTCAAGTCA ACGGGATCGC CGTGCCGGCA 496080
GTTTCCACGC GGCGTGCCGA TACAGTCGTC GAGCTCGCGA GCGGGCAGAG CTTCGTGATT 496140
GGCGGTCTCA TCAGGCGTAA CGTCAACAAT GATATCAGTG CATTTCCCTG GCTTGGCCGA 496200
ATACCGATCC TTGGCGCTCT GTTTCGTTCG TCCTCGTTTC AAAAGGAGGA GTCGGAATTA 496260
GTTATTTTGG TTACGCCTTA CATCGTCAGG CCAGGCTCCA ACCCCAACCA AATGAGCGCA 496320
CCCACGGACC GGATGGCTCC GGCTTTGGGC ACTCCGCCGC GAGCCCGCGC TGCTATCAGC 496380
ACCGACGCGC CAAGCGTCAA GGGCGATCTC GGCTTCATCA TCGAATAGTG GTGCCGAATG 496440
ACGTTGCGGA TCATAGCTCA CCTGCTCGCT TTGACAGCAA GCTTAGCAGG CTGCACAAGC 496500
ACTGCACCGA TCTATGTCGA GCAACCGACA CCGATTTTTG TCCGACAGGA GTCCACCGTC 496560
TTGAAGTTGG AGAGCTTTCA CGCTTCCGAA CAGCAGCGTC TTCTTGCCTT CCTATGGAAG 496620
GCCAGTCGCG GCCGGAGGGA TGCCCTTCAC CTCGTTATCA GCGGGTCGTC CCGGCTCAGC 496680
GCAGAGGCGG TCCATCAAGC CAGACAGATG GGCATCGGCG CTTCCAACAT CCATTTGCTT 496740
GACCAAAATG ACCGCGGGCA CCTGCGGATA GAGGCCGTTG TCTATCATGC CCTCCCTCCA 496800
ATCTGCCGGT CGCTTTCCAG CCAGTTACTT AATGACGAAT TCTTCGACCA GCCGATCGGC 496860
TGTTCGACGA GCCATAATCT GGCAGTAATG ATCAACGATC CGCGCGATTT GCTCGGCAAT 496920
AGATTTGTCA AGCCCAGCGA TGGTGATCGG GCGGCCATAC CAGTTACCAC CTACAGGACC 496980
TCGACGGGTA AAGGTGGCTT ATGAGGCATG AACGCGACTG TTCTCGATGA TCGCTTCATC 497040
CGGGTTGGGT GTCCCCTCAA CTCACTCAAC CCGCATGAGC GGCGCGGCGC TCCCGACTTT 497100
GTTTACCGCG GTTGCAGTCA CTGAGAATGT GGCGAGATCG GATGGGATCA CTTGCCGGTG 497160
TGTCAGTCGG CACGGAAATT GTTGAGCCCC TATCGGCGCT TGTGCACAAG CAAGATCGAT 497220
CGACGCCCAG ACAGCCAGTT AGAGGGTCGG TACGTCAGTC TCGCCGATAC GAAGCTGAGT 497280
CGACGTGTAT ATAGGTCTGA CTGCGTCAAT ATCTTGGACG CTTGTAACGT TTGGCGCCGG 497340
TCAGACGGGG GTGCTAAGAA TTCCTGCCCC TTTCAGGGCA TACGCTGTGC GCTTCGATCG 497400
TGAACGCCTA CTCTGCAGGA AAGCCCGCAC CCGAGTCCGC AGATCAAATG TCAAAATCCA 497460

CCGATGGCCT GATGCGAATG ACTCCCTCGC CTCGGAATTC GGCAACGTGC CGCATGCCCA 497520
TCATCAAAAA CGTTGTTGTC GGGACCTCTG CGGTTGGCAT TAGCCCCGTG GCCCTCAGCA 497580
TGCTCATCTG GTCTTCTGAG GCCATGTGAG GAGCTAAGAA CCAGTGGTCC CACGCACGAA 497640
CGCCGCTATC CATAACGAAA TCAGAAAGTT CAGCCGGCTC GGCCCCTGCG GACGAGGACG 497700
GCCAAGCGTG CGATGGCCGG GCTTGCGACG GTCCAGAGTG TGATGGTCCC ACCTGCCACG 497760
GTCCAGCTTG CGGCCCCGCG GCATCCACGC GCTGCCATAT CAAATCCTGA TCGTATTCGG 497820
GCACCTGCAG CGCGCGAGCA TGCGGTTGCT GCTCGCTAGG CTGGGGAGAC CTTCCCCTCT 497880
CAGCGGTCAG TGGCGCGGGA GAAGCTGTAG CCCGCGTATG CAGGTCCTGC TGCCAATCAA 497940
GCAATGACGG ATTCGCCCAA TCCAAATACT GCGAGTAGGG ATGCTTGGTT TCTAAGTCGG 498000
TGGCATGCGG CGGTGGGCTT GGGTTCAGTG GGGTGGCGGA CGGGGATAGC CCGGCTTGCG 498060
ACGGTCCAGA TCGTGATGGC CCCACTTGCC ATGATCCTTC TCGCGGTCCC GCGGCTTCCA 498120
GGTGCTGATC CTGGTCGTGT TCGGGCGCGC CCTGTAGCAC CCGAGCATGC GGTCGCTGCT 498180
CGCTAGGATG GGGAGACCTT TCCCTAGTCC TGGTATACAG GTCATGCTGC CATTCACAAG 498240
GTGATGGATA AGGATATGCC GAATCCAAAT ACTGCGAGTA GGGATGCCTG GTGTCCAAAT 498300
ATGGGGAATC GGCTTCAACC TGCGGCAATA GTGAGTCAGC GGCATGTGGA GGGCTATATT 498360
GAAATCCGCT CAGCTGATGC GCAAATGCAC TCGCGTTGGC GGGTGGTGGA GAGTCCGGCT 498420
GTGGGCTTGC GTTTAGTGGG CTGGTTGAAT TGATATCCAT CCTGTTTCTC CTTTGAAATA 498480
TGGAGGCCCC AAGCCTTTCC GATGCGGAGG CGGCATCACC ATCTAATCCG CCCTGCAATT 498540
GTTAAGAGAC ATACCTTTCG AGAAGCTGAC AATCAGATGG ACCGAATGAA GACCGGACTT 498600
ATGTCCGCTC GAACGTCGCG TCGCTGGCGA AGGCCGTTGA CAGATCCAGG ATGATAGAGA 498660
CAACAGCTGT CAACATGGCA CGCGCCAAGA GACAATGTTT CAACACGATA TTTCTTCTTT 498720
AGCGACGTGA TTGGCGCGCC TTCAGCCACA GGCCGAGCGC CACGAGAAAA GACAGGCCGT 498780
AGAGGCCCGT GACGAGCGGG AAAGCTGCCT TGAGCCCAAA CCGTTGCGTG GCGGCGACTC 498840
CTGCAATGAT CCCGACGACC GAAGCGCCCT GCTGGCAGGT CTGCGCAAGC CCCAGGACCG 498900
ATCCCTGCCT TTCGCTTCGC GTCGCGGCCG AGACGAGCGA CAGGAGAACC GGTGTCGTTC 498960
CTCCAAGCAG CGCGCCCCAG GTAAAATAGA GCGCTGCGAA CAGCCCTATC GCACGGGTCG 499020
AGCCAGCGAG CCCGGTGGCG ACGAAGCATC CGCCAGCGAT CAGCACGTTA CATCCGAGCA 499080
CAAAAGACGG CTCTCGCCCT TCGAAGGTCC GGGCCCACAC CGGCGCTGCG ACCACAAAGC 499140
CGAGCGCAAG AAGGCCATAG CTCAATCCCG TGATCCAATG CTGAGAGCCG TAGACCTCCG 499200
ACATATAGAG GGAAAAGGGC ACCTGCAGCA CCATCCTGCT CGCAAGCAGC AATCCCATCA 499260
GAGCAAGTAG TCCGAACACG GACACCCCCG TGGAAGGCGC GCAAGAGACT GTTGTTGCTT 499320
TCGCGGTTTT CGTGAGTCCC GCTCCTCCTA GTGGAACCGG CAGGCTTGTC CAGGCGACTG 499380
CGGCACAGAA TGCGCAGATC ACCCCGGCGG TGAGGTTGAC GGCCGCAAAA GGCAAGGCGT 499440
CGAGGATGAG CCCGCCGAGG AAAGCGCCGC CCAGGGAGCC GACATTCGTC GCCACCTGCA 499500
GCCAGGCGAA GAGGCGGGCG CGATCGCGCC CGCCGGTGAC CTCAACGCCG TAGGCCTGGG 499560
CCGGAGCGAT ATAGCCAGCG CAGGCCCCCT GCAGAAAGCG CAACGCCAGA ATGGTCCAGA 499620
CATCCTGGGC GAAGGCGACG AGCAGTTGCG TGATCGCCAG GCCCAGCAAG GCCCGGACCA 499680
TCATCAGGCG ATTGCCGTAC CGGTCGCCCA TACGTCCCCA AAAGGCACTC GTCAGCGACA 499740
CGCCGAGCAT CGGGCAGACG TAGACGCCGA TACCCGCGAG GCCAAAGACG CTGTCCGAGG 499800
GGCTCAATGC CTTGATCTGA ATCGGCCAGA AGGGACCGTT CATCTCCATG GCGCCCATGG 499860
AGATGAATTG AAGGCCGAAC AGCAGCGCGA AGACCGGTCC GAAACCGCGC AGGACCGCGA 499920
CTGTCCTGGT CATTCCGCAC TCGCCAACGG ATTGGGCAAT TGGTGCTCGA TGCGGTAGTC 499980
GCGATAGCGC TCGAGATGCA TCCGCAGGAC CGAGCGTGCC GGCCAGGGCC GTTCGAGGAA 500040
GGCCTGGCGT TCCTCGAGCC AGAACGCATC CGAGAGCATG CGCGGCCGCA ACGTCTCGAA 500100
CGCCTCTTCC GTTTCTTCCC TGAGGACGCG CCAAAGGCTG TCACCCGCAA CCGAATATTG 500160
CTCGGTCAGG CAAAGGGCGA TCTCGTGAAG ATGGCAGACA AAGCACGCAT TGATGAGGAA 500220
CGAGCGCACG AGGCTGATAT CGTCGTCGAA GGTGGTTGGC AGGATGCCCC CTCGACTGAA 500280
CGGCTGCAGC TCATAACCGC GTTCGGTGAA GAGCGGTGCA AAACTGCGGC CGTCACCGAA 500340
ATCGCGGATC AAGAGCTTCC GTGGCAGGCC GCGATCATCG AAAAGAATCG TGCTGTTTTG 500400
TTGATGCGCC TCGAATGCAA TGCCGTAGAG GAGATACATG GCCAGAGTCG GTCGGACAAC 500460
GGTGCGAGCA TAGAGCCGGA AGAACGCAGC GATCGCCGCT TCGCTCTCGT CGCCGCTTTT 500520
TCCGATGAGT TCGCAGATCA GTGGCCGGCC GTCGACAGGG CCAGCGGTCA GAAGCGCGGC 500580
GACGGTTACC GGCAGCAAGC CATCTCGACG GGCAAGAGCA TCGGCCTTGC GATAGACCAC 500640
CGAAAGAAAG CGGCCGAGAT GCTCGTCGCC GGTTTCGGGA TGACGCAGAA TCGCCCCAAG 500700
CTCTTCAGTA AGGATCTCCA GCCTGCACCT GAGGTCATCC TCCTCCGACA CAATGTCGGA 500760
AATCAGCGTG CTGAGGCGCG GTCCCATATG GATCGACTTC GCCTGCAGCG TCCGCTGCTC 500820
GCTCGTCATC CAGATCGCAA CCGGCAGTTT GATGAAGGGT CTTGGCGCCT CGGTCGTCGG 500880
CAGCATCGTG CGAAACGACA TGGACGGCAA CGTCACGATC TCCGGGCCGT CAGGATCGAA 500940
AATGCCGGCT TCGATCTCTG CCGCGAATTC GCGGCGCACG AAGTGCTCCA GATGCCAGGC 501000
GTGGACGGGC AGAGGCAGCC AGTCCTCGGG TGACTGGCCC CGCGCTTTCA GGCCCTCCGC 501060
CCAATCGCGC GAGAGGTCCG GGAAGTTCTC AGCGAACCAT TCCGAATAGC TTCCGACGTG 501120
CGGCATCTTC TCGACGTAGG CCCAGCTGCT GCGGAGCGCC GCAATGCGCA GGGGCACGCG 501180
CGCGCCGAAC TCGGGGGACA GTGCGGCGAC CTCCTCCGCC GCCAGGCCGG GCTTGGCCTT 501240
CCAGGTCGGA TAGAACGGGT GGCCTTCGAG CGCGCCCCAT TGGTCGAGGG TCATCGCGGC 501300
CAAATGCGTC GGCAAGCTTC TTTCGAGATA GGCAAGGAAG CCGGGCGCAC CGGCTTCGGC 501360
GATTTCCTGG CGCAGCCTGG CGCTCCAGCG CCCGCGCTCG CGGCGCGCCA GCATGTCGTT 501420
GCGCATGCTG TTGTCCACGT CGCGCAGCAG ATGCTCCAGT CCGTCAGAAG CGGGCGTGAT 501480
CGCGAGAGAG GAGGCAACCT CCCGCATCAG GGCGGCCGGA TCATCGATCC GCTGCCGTGC 501540
CCCGGTGCCG TCCAGCACTT CGATCTGTCC GCGGTTCCGC AAGGTTCCGG CGGGCGCGAG 501600

GCTGAGATCG GTGAAGTGCA GCATGCGCTG CGAGGGCCAG AGGGGGAACC AGGCCTGGCG 501660
GCCTTCGCGT GCCCACAGCA AGGCGTTCGG CGCGAGCAGG CGCTCGGCGA ACAGGCAGCG 501720
CACCAGCCGG TTGATCGCGT TGCGCGATGC CTGCGTCAAA AGCGCAAGAT CCTCGTTCTC 501780
GACGGGATTC GTCACGGAGG GCGTCTTCTC GTCCATCAGG CGGCCTCCGG CCAGTCGGCT 501840
GGGACATAGT CTGGTGTACC CGCAGCCAAT TTGCCGTGGT AGTCGCACGC CCAGCGATAG 501900
ATCTTGGCGA TCGCCGGTAC CGTCGCCTCG AGCCGTTCTG CCATCGTGAC GAGCAGCGCC 501960
TGTCCGAAAG CGATATCCTC GTGGAAGGCG CGGCTTCCGC GATCGATGAC GAGCCCCGGG 502020
CCATGTGGAT TGGGAACGAG GGGGGCAGGA ATGCCCGCAT AGGCGCGATT TGTGCGTAGA 502080
AGCGTGTACA TGGTTCGGTC ATCCCCAATC TGGTCGCCAT AGGCCTCGAT CAGTTCCTGC 502140
TGAAGCGGCT TCACTGAGCT GAGATCAATG CCGAGACGTG CCTCGCTCGC CCGGCGGATC 502200
GCTTGGTTTT CCGCATCGCA AGCTTCCAGG AGCTCGGCGC CTGCTTGTGG GCAGTCGCTC 502260
CACCAGCACA GGGCTTCGTC GAACGGTCTT TTTTCCCACG GTGCGCCGGG GCCGATGAGC 502320
GCGTAGAGCA CGGCCGGGTG CATCAGCGCG TTGCCCGGCG TCAGCGTGAT TTCGAGATAG 502380
TCCTTGAGCA GGTTGACGGG CGCCTCGTAG ATGTGATTCA ACATCGCCAT CAGTGCGGCG 502440
GCGCTCGCAG CGCTTTCCCG GCGATGCAGG GCAACGAAGA GCTCTGCCTT GGCGCCGCCC 502500
ATCCGCACGC GCTGGCCTGC AATCAGATCA TAGGCGATAT GGGGCACGTC CTTCATGCCC 502560
CAGATCACCA CATTGTCACG CCCCGAAAGC ACCTTTGCCG CGAGCCAGTC GAAGCCGCAA 502620
AAGCCGGGAA TCGCGCCGAT ATAGACGCTC TTATCCGTCG GCAAATACGG CGCTATACGA 502680
TGCAAGAGCG CGGATCTTGC CTGGGCGGGT TGCGTTATGA GGACCATGTC AGCGTTGTCG 502740
AGCGCCGCGC TCGGGTCGGT TCCGATGTAG TCGGGCCTAG CACTCAGGGT GCGACCGTCT 502800
CGCGTCACCG CTTGCCAGAG ACCGTCCCCG CTCGCCCATC GCGCGGCCAC GGTGGCGGAC 502860
GTGGTCAGGA CCGAAACATC GATGCCCGGG TTCTGCTTGA AAAGGACCGC ATTCAAATGT 502920
CCGGTGCGGC CAGCGCCGCA AATGGTCACC TTCATGCTGC CCGCGCCTTT CCGAATAGCC 502980
AGCCATCCAA CTGGGCAGCG ATTGCCGGGT CGAGCAGGCG GCGCTTCGCC ATCCATTCCT 503040
CATCGAAAAT CGTGCCAAGA TATTTCTCGC CGCCATCGGC AACCGTCGTG ACGACGGTGC 503100
CGGTGAGCTT GCCGGCGCCA ATGAACTCCA GCGCCTTGTA GATGGCGCCG CCGGTCGAGC 503160
CGCCAACGAG CAGTCCCTTG CGGCGGGCGA TGTAGCGCGC CGTCTCGAAG GCTTGCGTAT 503220
CGGTCACCTG CACGCCTTCG TCGATGCAGC CATAGTCCAG CACCTTGCCG ACCTCATCGC 503280
CGGCGGGCGT GCCCGTTCCT GACTGGTAAT AGGGGTGCCC CGGCTTGCCG AAAACGATCG 503340
AGCCGGCCGG CTCCACGGCA ATAGTGCGTA CGGCCGGATT GTTACGCTTC AGACGCTGGG 503400
AGATTCCGGT CATCGAGCCG CCGGTCCCGA CGCAGCCGAC AAAGGCGTCG ATGCCGTCGG 503460
GAAGCTGGGC AACCAGTTCG TCCACGAGGC CGGTATAGCC TTCCGGATTT GCCGGGTTGT 503520
CGGACTGGTT CATGAACAGC GCGCCCGGAA GCTGCGCGCC GAGTTGCGCT GCGAGGCGCT 503580
GGCGCTCGAC GACTGCGACC TCGTCTTCGC GAAAGTCGCC TTCGACATAG CGGATCTCAG 503640
CCCCTAGCGC CCGCATCATG CGGATCTTGT CGGGCGCTGC ATGGTGGTCC ACCACGGCAA 503700
TGAAGCGCAG CCCGAATTCC AGCGCTGCGA GCGCCAGCCC CGTGCCAGTA TTTCCCGACG 503760
ATGATTCAAC GATCGTGCCG CCCGGAGGGA GGCGCCCATC CTGAAGGGCC GCAATCACCA 503820
TGCTGCGAGC CATACGATCC TTCATCGAGC CGCCGGGATT GTTCTTCTCG ATCTTCAGAA 503880
CCAAGGTGGC GTTGCGGCCC GGCACGTCGA TCGACATGAC CGGCGTCTGG CCGATCAATT 503940
GTGTTACGGT CGTGTGCAGC ATCTAAACCT CTCCAATTGT GTCGGGAACG AGATTGGGCC 504000
GCCCTTCGGC GTCTTCGATG ACGCAGAAGC GAGCGGGCAT GGGATGGCGA TGGAACTCGT 504060
TTTCGAGGAG GTCCATCTGG TAGCCGCCGG TGTTGGCGTA TACGAGCAGG TCGCCAGCGC 504120
GCGGCGCGGT CGGGAACGTC AGCCACCGGT TGCTGATGAC GTCCTCGTCG AGGCAGCTAT 504180
GGCCGGCCAG ATAGGCGCGG ACCGGCGGCA ATTGAACGGT CGCCTTGGTG GCAGGCACGA 504240
GAATAGGGTC GATCAGGAAT TCGGAGGCGA ACCAAGTTTC GCAGGCGCTG AAGCTGCTAC 504300
CTTCGACAAA GATGACGTGC GAATCCGGAC CGAGCGCCTT CACCCGCGAG ATTCGAAAGA 504360
CAGTGATCGC CGCCTGGTCG GCCAGCGCGC GGCCGGGCTC CATCGCCAGC GTGAGACCCT 504420
CACGCGCCAG GTAGCCGGCT ACGCTTCGGC CCTGGTTCAT CTCCGCCTCC AAGAGCCGGT 504480
GCAGCCAGTC GGCTGCTGAA AGAGTGCTGC CATAGGGATA GAAAGAGTCC GGTATTTTCC 504540
CGGTGCGGTA GTCCTCGGGC GCCTGCGCCG CGAGATGCGC TTTGTACCTG GCTCGGTCGA 504600
CATATTGGAT AGGCAGGCCA CCGCCAATAT CGATCATGCC CGGAAAAAAT CCCATCCGCC 504660
TGGCTTCGGC AATGAGGTCG GCGGCCTCTC TGAGCGCTGC CACACGGGTC TCCCGGCGAT 504720
AGCCGCTGAG GTGGAAATGC AGCCCGTCGA AACGCACCCT GCCTTCACCG GCAAGCCGAG 504780
CGAGGCAATG AACGACCGCG TCGGGCGGCA TGCCGAAGCG GCTCTTGCTT TGGTCCCTCG 504840
GCCGCAAACG CAAGAGGATG GACTGTTGGC CAGCATCTGC CGGCAGACCG TGGATGAGAT 504900
CCTCCAGTTC TTCCGGCGAG TCCACCGATA TCAGCGCGTT GCAATTGATC AGCTCTTGAT 504960
GAAATGCGCT CGTCTTCGCC GGTCCCGTCG CCACGAGCCT GGCACCATCG GCCCCAAGCC 505020
GCCTTGCGTC GCGAAGCTCG TAGAGGCTGG AGACGTCGAG CCCGGCGCCT GCGCTGAGCG 505080
CTGCCTGCAT GAGCCCGGGC GATTTGTTGG CCTTCGCCCC GTAATAGATC GCATGCTCGA 505140
GGCGGCGCTC CGTCAGTACG CCCTTCAAGG CGGCAAGATT CTCCCGCAAC GCATCCGGCC 505200
AGACCAGATT GAGCGGGGAG CCGTGGCGGG CGGCCCAATC GAAAAGAAGA GGACCGTATT 505260
TGGTGAGAAG GTCCGCAGTC GCCGATCGCA GAATCGGTGG CAGCCCATGG CCGATCTTCT 505320
GGCAATGCAG CGTCATCACA GCGCCCCCGC GGAAAAGACC GGCGCGATCT CCATCGGAGT 505380
GGGACTGCTC AAAAGCTCAA GCGGCACATC GCATGTCGGC AAGCCGTGGG CAAAAACGTG 505440
CCGGGTCGTC TTGTAGGGAA ACTTGCGGCC GCTGCGCGCC ATCGCGAAGA TTTCAGCCGG 505500
AGTAATGTTC CCACCTGGGC GCCAACTCTC TACCCGCACC GAATCATTGT CGAGGAGAAC 505560
CACGGGCATC CTGGTGAGCC GGAGGCGATG CGCAACCGTC AACCGGTGAT AGTCGTCAAT 505620
CACGAACAAG GCATCCTTCT CGGCTGTTAT TGGCGCGGTC CATGAGCCTG CTTGCAGGAT 505680
TTGGGCCTTT AGTGCATCGA CCCTGTCAGG GTTCACCTCT TCTGTTGGGA TCAATTGTGC 505740

TGCGGAGAGA AGGCAATGTT CCATGCGGTC CGGCCTCACG CTCGCTCTTT CGCATCGCAC 505800
ATATCTTGAT TATCCGCGTT CACGTCTAGT CTCCTGCGCG TTCGCTGCCT CGTCGCTCGC 505860
AAATGTGCAA GCGACAGCGG CCGGCGAGGC GCGGTGAGGA TACCCACATG GCAAAATGGG 505920
GTCATGAACT AGCCGGGAAG GCCGACGGCC TAGGCCGGAT GGTCGGCCGC AAGGCGTGCC 505980
TTCAGGTCGT TCAGATAGCC TTCTGCATAG TGCTGCGCCA TTGAAAGCGG CAGATTAATC 506040
CCGGCCGTCG CGGCCTCTTC AATCGCCTTC CTTGCGAGCC CGTTGCGAAT AGCCAGCTTG 506100
ACCCCGGGTC CCGAGACAAG GCCGACTGCT TGTGCTGCGA CCCCTATGCC AGCTTCTATC 506160
AGCGCCTGCT TCATATTGCC TCCGGTAATC GCTGTACGGA GGAGATTTGC GGCTTGCGCC 506220
TCGCATGCAA GCGTCATGGA TACGAGATCG GCCACCTGTC CGATTCCCGG AATGAAGCTG 506280
AGCACTCCCA CGGCCGTTGC AGCCCAGTCA AGCACTTTAG ATCCTACCCG CAGCACGGAG 506340
TCGACGACAT GCATGAGGGC TCCGCCGTTC TTTTTCGGTG ACTTGATGTC AGGCTGGTCC 506400
GCCAGGCCCA TCTTCATGTC TGCGACAGCG TTCTGCGCAG CCTCAGTTTC CGGCACATGG 506460
AACTTTGGCT GCTGAACGGT GCGGTTTGCC GTAGTCATAT TTGTGTAGAA ACGGCCAAAG 506520
TCTTCTTTGC TGACGTTGCC AGAATCGACC GTATGTCCGC CACCGAAGTC GAAAAATCCA 506580
TGATGAGTCT TATAGCCCGT AATCGCATTG TTTAGCAGAC CGAACAGAAG AGGATCTGAA 506640
AGGAGCTGCG AGGCAGCTTC ACGTACTTTC GGATCCAAGC TCTTGTCGTC TGCCATACTC 506700
GCCAGCTTGT CGCGTGTGAG ATCACCGCTC CCGAAAAATA ACTCCTGATG GCTGTTAATT 506760
GTCTTGAGCG TGTCCAGCTC GGCTTGTGTG AGGCTCATCG ACGAGGAGGC CACTTGCAGG 506820
AAGTCCTCTT TGTGGACCTT ATCTATATTA CCACCGTACA ACTGCTTCCA TTCTTCCGGA 506880
TGACTGACGA AGTATTGAGC CGCCGCAATG ACCTGGGGTG GACATTTGCC TGTTTTCGCT 506940
TCGCCGTCGA CGATCTGCTT GAAGTCGGCC AGACTCAGGT TCTTGGGAAG ATATTCCGAG 507000
TACCGGTAAA GCTCCCGTAG AGCATCGTTC TCGGTCATGA CCGATGGCTG GGCATTCTCG 507060
GCGCTGTCGG ATGGGATGTA GTTCTGCGCG TAGCTTTGCG CTTGGCTTTC CTGAAATGCG 507120
GCAACTTGTG GGTGATGTTT GGAAAATTCA GACAAGTCCT TTGCACTGAT CTTGCCTCCA 507180
CAGCGACCAT CGCCTTGCGA GCCGATTGCA TAAAAAAGTT CGGGATCCTG TAGCAATGCC 507240
TCGATTGCCG CTTTCAGATC CGGCGGAGTA GAGGGATCAT TGGCTTTAGC CTCCAGCGAC 507300
TCCCAACTGA GTGGGCACAG ATCCTTGTGA CGGTTCAGCA CTGCGACAAT CTGCAACTCA 507360
GGCTTAGTCA GCGATCCACC GTTCCAGGTA ATTCTCGAGC TTTGTATGGG AGCTGGCGCG 507420
ACATAATGGT CATCGATCAC AACAGCCGAC TGCGGGGTCT GATCCATTGA TTCGATCGCC 507480
GCCCTCATGT GGGGGGGCAG TAGGGCCAAA GGGTCCTTCT GCAAATCCTC CAAGGTCGAC 507540
TCGGCATCCG GCGGCGGCAG GTCCTCCCGA CTGGCTAAAG GACCAATCCT GTCTAGCATG 507600
TTAGTGGACA GCATCGCCTC GAAGGACAAT ACGCTTTGCT GCCCCTGGGC TACCCGAGGC 507660
GCGAAAGCTT TGGCGAGCGA CGCCTGGGCG AACTGTGCAG GATTAGAGCT GATCATTGGT 507720
AAAAGGTTGC TGGCCGACAT TGTGCATCCT CAGACGTGAT GTTGTCCCTC CACAAGGACC 507780
TTCTATAAGG CGCTTGCGCT CACGAAGTGA CAATCTTAAA GCCTCAGCTG GAGTAACCGT 507840
GCTTTTGATT GCTTCGCTCC ATGCTAGCTA CAAAGGTCAA TTCCGGCACA ATGGTCGGCG 507900
ACATCGTTGG AGCTACCGAT TCACATCATG AGCGGTTTAG CTTTCGAGAA ACTGACGAGG 507960
CTCAGACGAA TAACAATCAA GAGGTGACCG CAGTGAGGTT CTAATCCTAA AGTCTGAATT 508020
TCATTTCTTG CTCAGCTCAG CCTTCCCGCC ATCGTCGTTC TCTGGCAGCA TCTCAGAAGT 508080
ATAAAAGACT TCCGGTCTCC CGCCGCGGAC GACCGTGGTG GAGGAGCCCC GAAACAGTAC 508140
CATCACAGAT TCCCTGGCGG GCGTATCGGT TGCGCGAGGC TGCGCCTGCG CCACTGCCAG 508200
TAGACCGTTC AACGTCTGCT CGATGAGCGC CATGAAGCGC GGCGGGCCGG ACACTAAGAC 508260
CAGGCCATTT CCCGGCGCGG GTCTCACCGG ATAGCGCTCA TCAGAGATGT CAAGCTTATC 508320
GAGGGCGAGC TTGAAAGCAC TAAAGTGAAC TGAACTCAAC ACAAGCATCC GAGTTTGTGC 508380
TTCTTTTGCA GCAGACACAT AGAGCACAAC CCCGTCATAA TACCATTGGA GATCGTAGAG 508440
ATCGGTCAAT CGGTCGAGGA ACTCGCGCGG TGACAACTCC GGTATACGCC CGCGAATCCG 508500
CCCCTTCACC TCTGCGCTGA TGTTAACACT GATTTTCAGG TTGTTGCCGA ACTCCTGCAA 508560
CGCGGCAGAG AGATCCTGAT CTAGAACCGT ATACTTATAG GAGGTCGAGG GGAGTGGCAG 508620
GGTGGCCCCG AGAGTTGTGT GGATTCCGGC AAATAGAAAA AGCCCGACAC AGAGCAGTCT 508680
CCTAAACATA TGAAGCGGTG TGAATGGGAT CGGCGTTGTC GGCATCTCGT CTTGATAAAC 508740
CAAATCTGAA AATCTCCCAT GCGGTTCAAC TCGACTAACA TCACTCTTCA ATGGGCAAGC 508800
GACGCTGCCG GTAGTCAGCG TGTCGTCAGC TCGCCTCGCT AGAGTTCCAC GTCAAAGAGT 508860
TAAGTAATCG AGTCCTTCTC ATGATGCTGC CTGTCACGTC AATCTCTAAC AGTCTCCCCA 508920
GGGTCGCGTC ATCGTCATTC GGCGAGCAGG CTCAGTTTGA GCGTGCTCTG GCACAGGCGG 508980
CTGATTCGAT GAAAAACGAT ACTGCTTCAA CGCCAGTGAG GACGGCGCCT ATTTCTCCGC 509040
CGATGGACGT TCACCGCGCG GCGCCAACGA GCCCGCTGGA GGATCGCGTG CTGCAGACGA 509100
TTTCTTCGAT TTGCCCGGAT AGTATAGTTG CTGCTGCCGC GCCCAACCAT AAGGCGGCCC 509160
TTATAAGCGG CGCTCCGCCC GGGCCGTCGC AGAAGCTCCC TGTCGAAGGT GGGGCGGGCA 509220
CCGAAAGGTT GGGAATCCCG CAAGGGGGGC ATGATTTTGA CGTGATGGTT GCCGGTCTGC 509280
GGGATCTTTA TAATGGCGTG ACCCAAGTTG CTCTTGTTTC GAAGGGTATC AGCGGCATCA 509340
CCTCATCCGT AAACAAACTA TTAAAGGAAG GGTGAGTTGC GCGCATGTTC GGCTCAGCCC 509400
ATGGCGACAC AACGAGCAGC GACACGTCTG GCCGGCGACC GCTTCGCCTC GTCGTGCTGC 509460
CACTTCTCCT GGCTCTGAGC AGTTGCAAGG TCGATCTCTA TACCCAGCTG CAAGAGCGCG 509520
AGGCGAATGA GATGCTCGCG CTTCTGATGG ACAGCGGGGT TGATGCGGTC CGGGTGGCCG 509580
GTAAGGATGG CACCAGCACA ATCCAGGTCG ACGAAAAGTT GCTCGCCTTC TCAATCAAGC 509640
TCCTGAATGC CAAGGGGCTG CCGCGCCAAT CTTTCAAGAA CCTTGGCGAA ATATTCCAAG 509700
GTTCGGGCCT GATTGCATCA CCGACCGAGG AGCGTGCACG TTACGTCTAT GCCCTTAGCG 509760
AAGAGTTGTC GCATACGATC AGCGATATCG ATGGCGTGTT CTCGGCGCGT GTTCATGTGG 509820
TACTGCCGCA TAACGACCTG CTGCGAGCAG GCGACACACC GTCATCAGCC TCGGTCTTTA 509880

TCCGCCACGA CGCCAAAACG AATCTCCCAG CTCTGCTGCC TAAGATAAAG ATGCTCGTCG 509940
CCGAAAGCAT CGAAGGCTTG GCCTACGACA AGGTAGAAGT GGTTTTGGTG CCGGTGGAAA 510000
GGTCCGCACA AGAGCAACGG TCTTTACTGG CGACTGACTT GGCTCAAGCA TCTAGGCCTA 510060
TCCCAGAGCC GCTCCTGGCT GTCGCGGTGG GTGTTTCCGC TGCAGTGTTC GCTGTGACGT 510120
GTTACCTCTT GTTCATCGTT CTTGGGCATC GGCGCAGGCA ATTAACTGGC GAACTATCTA 510180
GAGTCCAGGA GCGCCCCGGT GTTTCCGCTC TCGCGGCTAT TCGTAAAAAG ATACCGGGGC 510240
TAGGGCGTAG GTGAAATTAC CAATGCCGAT AGCGATACAG ACTACCCAAC CTGATGTTTC 510300
GTTCCAATCG CACTCCGTTT CCAGGAGCGA GCTTGCTGCG TCGACCCATC CGACGCGTCT 510360
TGCCGCCCGT CTCGATCCGG AGTTGTCGGC TGCGACAGTG GTGCAGTTGC AGAAGTGTGC 510420
GAGGCTTCAG CCACGACTGG CAGAATTGCT GCTTGGCAAT GACATGGATT GGAACCGAAT 510480
CGGATGGGGG CCCGATCTCC TGCGCGGCCA TGATCCTCGT CGCGCTGCTC TTCTTGCCGG 510540
AAGTATTTGG CATGCCCGTT CGCTTCTGAA AGTCGTTTCT CAGCGTGACC TCGCCCGGCT 510600
CGTTGAACGT ATTGGCGCTG ATGCGCACGC ATTTGGCATC CGACATCTGG CGCATGCTAT 510660
CGCAGACAAA TTGATCTCTG ATCCGGAAAA ACTTGCGTTG CAAATCGAAC ACGATGGACA 510720
TGCTTGTCTT GGTGCTTGGC TCAACATAAG GCCAGCGCTT GAGCGGAATC GCGTGCTTCT 510780
ACGCTTGCCG CTCGGTACAG CCGCGGAGAA CCCGGCGCCT GAGCACGATG GTGCCTCAAG 510840
CGGGTTGTTT TCGCTCGTGA TAGCCCATTT CGAGATGGAG AGCCCGTAAT ATGACAGCCG 510900
ACATTTCAGC GGCACCCGTC GCGCCGCAGA TGCGCCCATT GGGGCCACTG ATACCCGCGA 510960
GTGAGCTTAA TATCTGGCAC AGCGCTGGAG ATGCGCTTGC CGCAGCAAAA CGGCATCAGC 511020
AGCGGGTCCG TACCTGGGCA CGCGCCGCTT ATCAGAGGGA GCGGGCGCGC GGCTACGCCG 511080
AGGGGTTGAA TACAGGCGCC GAGGAAATGT CAGGGCTGAT TGCACGAGCA GTCACCGAAG 511140
TAGCGCAACG AAAGGCAGTT CTGGAAAAGG AATTGCCACA GCTTGTCATA GAGATACTGA 511200
GCGATCTTTT AGGTGCTTTC GATCCGGGTG AGCTGTTGGT GAGGGCTGTT CGTCACGCGA 511260
TTGAGCGCAG ATACAACGGC GCGGAAGAAG TTTGCCTTCA TGTGTGTCCC ACGCAAGTCG 511320
ATATGCTCGC GCGCGAGTTC GCCGGTTGCG ACGGACGGGA GAAGCGGCCG AAGGTTCGAA 511380
TTGAACCGGA TCCTACGTTG TCGCCGCAAG AGTGTGTGCT GTGGAGCGAG TATGGCAACG 511440
TCGCTCTTGG ACTTGATGCG CAGATGCGCG CGCTGCGCCT TGGCTTCGAG TACCTTTCTG 511500
AAGAAGGAGA ACTGTGATCA CTCCGGTACC ACAGCATAAT AGCCTTGAGG GGACTCCTCT 511560
CGAGCCAGCG ATCTCGTCTT TGTGGTCCAC GGCAAAGAGA ATCGATACGC GCGTCGTGCG 511620
AGGACGGATC ACGAGGGCTG TTGGTACCCT GATCCATGCC GTCTTGCCAG AGGCTCGGAT 511680
TGGGGAACTT TGTCTGCTGC AGGATACGCG AACCGGACTG TCGCTCGAAG CAGAGGTAAT 511740
CGGCTTGTTG GACAATGGCG TGCTGCTCAC GCCGATCGGA GGCTTGGCGG GTCTGTCCAG 511800
CCGCGCGGAA GTCGTCTCCA CTGGACGAAT GCGTGAAGTG CCGATCGGCC CCGATTTGCT 511860
TGGCCGTGTG ATCGACAGCC GCTGCCGTCC ACTCGACGGC AAAGGCGAAG TTAAAACCAC 511920
CGAAGTTCGT CCGCTGCACG GCAGGGCGCC CAATCCAATG ACGAGGCGCA TGGTTGAGCG 511980
GCCATTCCCG CTTGGGGTGC GCGCGCTCGA TGGTCTCCTC ACATGCGGTG AGGGGCAGCG 512040
GATCGGGATT TATGGCGAGC CTGGCGGGGG CAAGTCGACG CTAATATCAC AAATCGTAAA 512100
AGGTGCCGCC GCCGATGTCG TCATAGTCGC GCTGATAGGC GAGCGTGGCC GCGAAGTACG 512160
TGAATTCGTA GAGCGGCATC TTGGGGAGGA AGGCCTCCGC CGTGCGATAG TTGTGGTCGA 512220
GACCTCCGAT CGCTCGGCAA CTGAGCGGGC GCAATGTGCT CCCATGGCGA CCGCCCTGGC 512280
GGAGTACTTT CGCGAACAAG GGCTGCGTGT CGCTCTCCTG TTGGACTCGC TGACGCGCTT 512340
CTGCCGTGCG ATGCGCGAGA TAGGTCTTGC TGCGGGAGAG CCGCCGACCC GGCGGGGCTT 512400
CCCTCCTTCT GTTTTTGCCG CCCTGCCAGG CCTGCTGGAG CGCGCCGGCC TGGGTGAGCG 512460
CGGTTCAATC ACAGCCTTCT ATACTGTGCT TGTCGAAGGT GATGGCACAG GCGATCCGAT 512520
CGCCGAGGAA TCGCGCGGCA TTCTCGACGG ACATATCGTC CTCTCACGCG CTCTTGCGGC 512580
GCGATCCCAT TTCCCAGCTA TCGACGTGCT GCAGAGTCGC AGCCGAGTTA TGGATGCGGT 512640
CGTTTCTGAG ACGCATCGCA AGGCAGCATC CTTCTTTCGT GATCTCCTCG CGCGCTATGC 512700
AGAGTGCGAG TTTCTGATCA ACGTCGGCGA ATACAAGCAA GGCGGCGACC CCCTAACTGA 512760
CCGCGCCGTC GCGTCAATTG GTGAGTTGAA AGAGTTTTTG CGCCAGAGCG AAGACGAGGT 512820
ATCAGATTTT GAGGAGACGG TCGGATGGAT GTCGCGTTTG ACCTCGTGAA TTATGCTTAC 512880
GCTTCAAGGC TACGGCTTTT AAAGGAAATG CAAGAGCGCG ACGCCCGTAG GAAGCTGTCG 512940
AAGAAGGAAG CTCAGCACCA CATGGCCATC GTAGCCGCAC AGAATGCCTC TCGGGAGCTT 513000
GAAATCGCAC AGCAACAGCG TGCTGGAAAG GAAGCTCAAC TCTACCAAGA ACTGACATCG 513060
CTGAACACCT TGTCTAGCGC CGCACTCGAC CATCACCACC TTCATATAGA GCGGCTTGCG 513120
GCTGAGATCA CTATCCGAGG CCAGGTACTT GACGACGCGC GCATCGCTCA AGAGCAGGCC 513180
GAGACAGCGG CGTCTGAGAC AAAGGCCCTG TGGGTGAAGC GTTCGGAAGC ACGGCACAAA 513240
TGGCAACAGA TCCAGGACGA CCTCCGGCGC GCCGTCGATA TTCTTTCTGA AGCCGCAGGT 513300
GAGATAGAGG CCGACGACGA GATATTGCTG CGGTATGGGA GGGGTTCGCT CGACCAGAGG 513360
TCACGCAACG AGTTCCGGTA ACTGCAAGTT TCTGATTGAC CGCACGTGCA GCTAAGATCC 513420
GCTTTGATGA CTGGCGTGGC CAGACGCACC ATATTCGAAC CAGCGCTGAC ACTGTCCCCC 513480
GAGGTCGTCG CGTGGATCAA CGACATAGCG GCTTCACGCG GGCCGTTTCA GAGCCGTGTC 513540
GGCGACAAAC CACTTTCGGT GTCGATGGAA GGACTTGTTT GGCAACACGA GTCTTCTGCC 513600
ATTCCGATGT TTGACTGCGT TTGGGATTTG GGAGGTGAAA CGGTCGTCTT GTCGCTCTCT 513660
CGGCCGCTTG TAGAGGCGCT CGTCTCGACA GTGCAGAGCG GGCTTGCTTT TCCTACCGAG 513720
CCAACTGCAT CGCTCATTCT CGAACTTGCG CTCGAGCCGC TCATCGCTCG ACTGGAGGAT 513780
AAGACGAACC GGACCTTGCA TCTCCTTCGC GTGGGTAAAG CCATAACATT GGCTCCTTAT 513840
GTGGAACTGG AGATCGTCAT CGGCCCGGTC AGTGGCAAGG GTCGCCTGTT CCTCTTCTCG 513900
CCTCTTGATG GCTTGGTGCC GTTTGCGTTC CGCGCGCTGG CCGAACTGCT TGCCCAGTTG 513960
CCGCGGCAAC CGCGCGAGCT TTCCCCAGAG CTTCCGGTGA TAATTGCAGG TGAGATCGGC 514020

ACGCTTCGAG CTTCGGCGGC GCTCCTTCGA AAAGCATCCG TCGGTGATGC TCTGTTGCCA 514080
GACATATCGC CGTTCGGCCG TGGCCAAATC GCTCTGTCTG TGGGTCAGTT ATGGACCAGG 514140
GCAGACCTTG AGGGCGACCA CCTAGTCCTG CGGGGACCCT TCCGCCCGCA ATCACGCCCC 514200
TTGGAGTGTG CGCATATGAC AGAAATCGAA TCGCAACTAA GGCCGTCGGA TGCCGATCTC 514260
GATGACATCG AGATCGTGCT TGTCTTTGAA TGCGGCCGCT GGCCCATCTC GTTGGGCGAA 514320
TTGAGGAGTG CCGGCGATGG GCATGTTTTT GAACTTGGGC GGCCAATCGA TGGCCTTGTA 514380
GACATAGTTG CCAATGGTCG GTGTATCGGA CGTGGCGACA TCGTGCGCAT CGGAGATGAT 514440
CTGGGCATCA GACTCCGTGG AAGGTTGGCA TGCAATGACT GAAATGCAGC CGGCAATTCT 514500
GGCCCTTCTT GCGATAACCG CGGCACTCGG CTTGCTGGTG TTAGCGGTCG TCACAACCAC 514560
GGCATTTGTA AAAGTATCTG TTGTTCTATT CCTCGTCCGC AACGCACTCG GGACCCAGAC 514620
GATACCACCT AACATAGTGC TGTACGCAGC CGCACTTATC CTCACCATGT TCGTTAGCGC 514680
ACCCGTTGCT GAGCAGACCT ATGATCGGAT AACAGATCCG AGACTCCGTT ATCAATCGCT 514740
CGACGACTGG GCTGAAGCCG CCAAAGCAGG AAGTCAGCCC TTGCTTGAAC ATCTCAAGAA 514800
GTTCACAAAC GAAGAGCAGC GGCGATTTTT CCTCTCCTCG ACGGAAAAGG TCTGGCCTGA 514860
GGAAATGCGC GCTGGAGTTA CAGCAGATGA TTTCGCCATT CTTGTACCAT CTTTTCTGAT 514920
ATCAGAACTA AAGCGCGCGT TCGAAATCGG CTTCCTCCTT TATCTACCCT TTATCGTCAT 514980
TGATCTTATC GTCACGACGA TATTGATGGC CATGGGGATG TCGATGGTAT CCCCGACGAT 515040
TATAGCTGTC CCGTTCAAAC TATTCTTGTT CGTCGCTATT GATGGCTGGT CAAGATTGAT 515100
GCACGGACTT GTGCTCAGTT ATACGATGCC GGGAGCTTTA TGATGACCGG ATCCAGTATT 515160
GTTAGTCTAA TGAGCCAATC ATTGGTGGTT TTCATGATCT GGATTCTGCC CCCGCTCATT 515220
GCATCGGTGA TTGTTGGCCT AACCATTGGC ATCATCCAGG CAGCAACGCA GATCCAGGAT 515280
GAAAGCCTGC CACTCACTGT GAAGCTCCTG GTCGTTGTCG CGGTGATTGG CCTGTTTGCG 515340
CCTGTGCTGA GCGCTCCGCT CATCGAGCTC GCGGACCAGA TCTTTACCGA GTTTCCAGCA 515400
ATGACACTCG GCTATTAGTC TGCGCTATGT ATTTGTCACC TGCCGAGATT CAGATCTTGC 515460
TCCATGCGGC TATCGAACTC GTTGCGGCAG CCGGTCTTGG AGCGGCCCGC GCGCTGGGCA 515520
TAATGCTGAT CCTTCCTGTA TTTACACGAT CTCAAATTGG CGGGCTGATC CGCGGCTGCC 515580
TGGCTATCGC CTTCGGACTA CCCTGCTTGG CACACGTCAG CGACGGATTG CAGGCGCCGG 515640
ATCCGGAAAC AAGTCTGATC CAGATACCCC TTCTCGGACT AAAGGAAGTG TTTGTCGGCG 515700
TGCTACTTGG CACTTTTCTC GGAATTCCTT TATGGGGCCT TCAGGCGGCG GGTGAGTTTA 515760
TCGACAACCA GCGCGGCATC ACCAGCCCGT CTACTCAGGC CGACCCAGCA ACGAACAGTC 515820
AGGCTTCCGC TATGGGAGTA TTTCTCGGAA TCACTGCAAT TACGATCTTC GTCGCAGCGG 515880
GAGGCGTGGA AGCTGTGCTG AGCGCCCTCT ATGGTAGCTA CTCAATCTGG CCCGTATATC 515940
GGTTTCAGCC AACATTGAGT ACCCAAGGCG CAGTGGAGTT ATTCGGGCTC CTCGACCACA 516000
TCATGCGCAC GACATTATTA GTCTCGGGGC CTGTAGTGTT CTTTTTGGGA CTGATTGATA 516060
TATCAATGAT GATGCTACGT CGCTTTGCGC CACAATTCAA ATCTGGCCAG CTCTCCCCGC 516120
CGATCAAGAA TATCGTCTTT CCGATCATCA TGGTTACCTA CGCTACCTAT TTGCTCGAGG 516180
GCATTAAACT TGAGATCACG CAAGCGGACG GCACGCTTGG GTGGCTCGAC AAGTTGCTGA 516240
AATGAGCGAT ACGAGCGAAG AGAAGTCACA CGGCGCTACC CCTAAGAAGC TGAGCGACGC 516300
GCGCAAAAGA GGTCAAATAC CGCGTAGCTC GGATTTCGTC CGCGCGGCCG CCACTTGCGC 516360
TGGGCTTGGC TACTTATGGC TAAGAGGTAG CGTTATCGAA GATAAATGCA GGGAAGCGCT 516420
ACTATTAACC GACAAGCTGC AAAACCTGCC TTTCAATCTC GCGGTCCGGC AGGCGCTGGT 516480
TCTGCTAGTC GAACTCACCC TGGCAACCGT TGGCCCGCTG CTCTCAGCTC TTTTTGGCGC 516540
GGTAATCTTG GCTGCATTAC TGGCCAATAG AGGATTTGTA TTCTCTCTCG AGCCAATGAA 516600
GCCCAACTTC GATAAAATCA ATCCTTTTCA ATGGCTGAAG CGCTTGGGGT CGGCGCGTTC 516660
GGCAGTCGAG GTTGGCAAAA CGCTGTTCAA GGTGTTGGTT CTCGGCGGGA CCTTTTCCCT 516720
CTTCTTTCTC GGTTTATGGA AGACGATGGT CTATCTGCCG GTCTGCGGAA TGGGGTGTTT 516780
CGGCGTCGTC TTTACCGGAG CGAAACAGCT GATCGGAATT GGCGCCGGCG CACTTCTGAT 516840
CGGCGGACTG ATCGATCTGC TGTTGCAGCG TGCGCTATTT CTGCGCGAAA TGCGCATGAC 516900
GAAGACGGAA ATAAAGCGCG AGTTGAAGGA ACAGCAAGGA ACGCCAGAAT TGAAGGGTGA 516960
ACGGCGTCGC ATTCGCAACG AGATGGCTAG CGAGCCTCCG CTTGGTGTGC ATCGCGCCAC 517020
GTTAGTCTAC AGAGGAACTG CGGTGCTGAT CGGTCTGCGT TACGTTCGTG GTGAGACCGG 517080
AGTTCCGATC TTAGTTTGCC GCGCCGAGGG CGAGGCCGCT TCAGATATGT TTCGCGAGGC 517140
GCAGAATCTG CGTCTCAAGA TTGTCGATGA TCATGTCCTA GCGCACCAAC TCATGAGCAC 517200
AACGAAACTG GGCACCGCCA TCCCCATGCA ATATTTCGAG CCCATCGCGA GAGCACTCTT 517260
GGCCGCAGGT TTGGCCTGAG CATCCCTGAA TTCGGAAAAG AACTTTGAAG ACATTGACTT 517320
CCGGGCGTGA TTCCTTTGGC TTTAAGTCGG GACGCAATGG ATGGCCCAGG AAGAAACAGT 517380
GCTGCCGCAA GCACATCGCA CGGCGGGATC ATCTGGCAAT CGGTTTCTGC GAATGGCGGC 517440
AAACGTGGCT CGCGCTTGTC GTTCAGGTTT GGCATGTCCG CCGGTGGCGA CACGTTGTTT 517500
CAAATGGTAG TTGTGGCTGA GAATCGATAG AGGTGCCGCA CGTGTTGGGG TCGGACGTAA 517560
CGGCGGCCGC AGGGACGCTG GTCCGGCGCC CCCGCCATAT CCGTGCGTGC AGAGTTCCCG 517620
CGTCATGACC CGTGCCCGGG CTAAAGCAAC AGGGTCGTAG ATTTCGAATG ACAACACGTC 517680
TGTGGCGGAC ATTGCTCGTC GCCACGAGAT CGATCGGTCA TTTTTTCTAC AGATGGCGTC 517740
AAATGCTGCG AACCTCTGCG ATAACGTCGA CGCTTCAGCA AATTGGAAAC GCGGCGGCGC 517800
AAACAGTTGG CTTAAGCCCA AACTTCCACG ACACGTCCGT TCCAGCCAGA TCTGCCACCC 517860
AAGCCGATAA TGGTTCAATG GTTCCGAGCT CCGGCCGTCC GCGCAAGCAG CAACAGAGCC 517920
AGTGAACCCG AGTATCGGCA AATAGCGGCA GAAAGCTTTT TGTCCGCGCT GAAAGTCCCA 517980
CTAGTCAGCT GAACGTCAGT TGTGCGCGTC TATCATCTTG ACCCGTTTTC CCTACCAGTC 518040
GCTCTCAGTG CAAAAAAATA GGAGTACAAG ATCACCATGT ACGGTCGAAT TGATAGCTCG 518100
TCCGATTTCC ATTACACGCA GAGTGCCAGC AAGCAAATGG ATGCAGAAAC CCAAGAGTTC 518160

GCGGACACGT TTGCCCGAAT GCACTTAGAC AGGTCCAACG GCGGTTCATC GTCGGCGGCC 518220
AGATATACCC TCGATCACGA ACCTCCGGTG GTGCCGATTG ATCTCGAGAC TTTCAGGAGG 518280
GAGATCAGGA AATTTCATGG CAAAGAAATC ACTGACATCG CCAACAATCC ACAGGAATAT 518340
TCAGACTTCG TGTCCGCAAA AGCCAGACGC ACTGCGGACG TTGCTCAGCA ATACGGCATT 518400
CGTCGGGATT CTGAGAACGC TCGATATTTC AGTTACCAGT TGGGAAACCA GTGTGTTGGA 518460
CTGATGAGAA CGGAAGGTGG GTTCAGCATG GAAGAAGAGT TCGAATCCAA AAGTTGGAGA 518520
GACCAATTTC CTGGTCACCA AGAGATTACC TCCACCGTGG ATCTTCAAGT CGCCCATCCT 518580
CTCGTTGAGA ATGCAGGCGA TATTCTGCTC GAGCACCAAC TTCGGAGGGA CGGCGAACGA 518640
CCGTTGCTGA ACTGGCGCGC GGAAAACCCA GAGGCGAAAG CCCGTGCAGC GATGATGGGG 518700
TTTGTTGAAG TGGATGATTG CGACATGGTC CTTGACCCCA AACAGCATCC CGACAAATGG 518760
ACGCAGACCA GTGCCGCTGA ATGGCGGCGT AAAGACAAAC CGCCGCTCTA TCTCCGCAAA 518820
TTTGAGGATG CCGAAACCGC ACAGTGTTCA ACCAGCTGCT CTTACGAGAC TTACGAAGAT 518880
GACTTCATGT GACCGGATTC TCGCCAGATT TGGTCGCGAC GGCCGGCCCC ATCTGCCAAG 518940
ACCGGCCCTC ATGTCACGGA CAGGGAACGA AAGAGCGTTA CAAACTGCCA CGCGCTAACG 519000
AGCGATACCC ATTCGGTGTT TAGGCGCAAA GGGGCTTGAA CGCAGGGTTT CTTTGAGACC 519060
ATCGTAGTAG TTCGTTCGGG GTTTTGCACC CGAGTCCCGT AAAACACCTC GAGGCCGTTC 519120
TTTACATTGA GGCCGGAGCG TCTTTGATGC CCGGCCTCTT TTGCCATCGG GTGGCTCCGA 519180
CACCGCAATG GCGATACGCA AAGCAGTTGG TCTCGCCGGC CCGAAGTTTC GTGTAAAGCA 519240
AGCCTTTGCG CATTCGAGGC TTCATCGAAC CTGAGAGAGC TTCACGCTCG CGCAAAATGC 519300
CGAATTTCTC GCCCTGCTGG TCCAACTGCA CACGTTAGCG TTTTTTTGTC CTGGAGCCGA 519360
CCGGCTTTTC GAACAGGTTG TCGCTTGCTG TCGGCTGCCC GTTGTATGCT ATGTCACTCA 519420
AGTGCATCGG ACTGAGCGCC GCCTCGGCCT GCCGGAGTTC CAGGGAGTCC AGATCGTGCA 519480
GCACGTTTAT ATGCAATAGC GCTTCTTTTT TTGACAATTG TCAGCTTTTC GAAAGCTGGA 519540
GCTCATAAGT AGATGTTATC TACAATTCGC CGCTAAGGCA GGAGTAGCTG ATGGTCGGAG 519600
TGATTGGAAG TGGAGTTGGC TCCATCGGCG TTTCCCTGGC CCGCAAAGGG GGGCATGGAC 519660
ATTCGACTGG ACAGCCGCCG CGCGATTCAG GCGGGCCCTC TGGTCACAAC AGGCCGGATC 519720
GCGGGAGCGG CGTTACGGAT GGCCCGACCA TTTCTGGGGA TCGCTCGCAG GCTGCAATTC 519780
AAAGCGAAGC CTTCGAACTA GCTCTTCGAT CGGTTGCGCT ACAACTTATG AACGATGCCA 519840
TGGCTGATGC CGACGAAGCT ATGGCAGAAA CTGAAGAGGA TGCCTGACGC TGGGTGGCCC 519900
CCGTCAGGAG GTGCGTGCCG TTAGTGACGG CACAATTGTA ACAGAAAGGG AAATGACATG 519960
TCTAAAATAG GTACTCTCAC GAGTGCAGTT GGAGCTGGCG CCGCGGCTGG CCAGAACGTA 520020
GCGGCCAAAG GTGCTGGGGC TGCGGCCTTC CAGGCCCAAA TCGCAGAACT GGCAGCGGTG 520080
AGCGCAGAGG CTACGGCTAG GAGTATGCTA CTGCGCACTG TTACGACCGA GCTCCAGACA 520140
ACCAAGAAGG CGGCGGATGA ACGCGTACAG TGATGCGGAT GGGGCTCTAG GCACTGCGTA 520200
GCACAATTGG CACGAAGTGG AGCCGAGATG AGCCGCATGC CACTCGCCGT GCGGCTTCTC 520260
TTTTGTCTGG ATATCGTCAG TGAACGACGC CATTTCGCTT CAATTCAAAG TTATCTCAGG 520320
GCTCTATTGT GAACTGACTG GTACGACGGC TCTAGAAACG AGCTTGATCG GTAGCGGCCT 520380
CGACGCCGAC ATAGTGTTCG TCGAGCAGGG CCTCGCGCCG CATCATTTTC GCGTCACTCT 520440
TCTGGGCAAA ACACTTGAGG TCGAGGCGCT TGCTGCCGGT CTCAGCATAG AGGGAAACGG 520500
GAATATTGCC GCAGGCGAGC GGGTTGTCGC GCCCCTTCCT GTCGTCATTC ATGCAGGCGC 520560
GATGTCCATT CTCTGGTCAG TGCAGGATGC AGCGTCGTCT GGTTCGATCG GTAAACCACG 520620
CCTCTCGATA TCTGTGCTCG CCTTGGTGTT GCTCGGTTCT CTCGGGATCG GCGTGCTCTC 520680
GGCCATTTTC TCTTACTACG ACAACGCTGT TGTATCGAAC GCCGATTTAT CTGGTGAGGC 520740
CCGGGAACCC AAGCTGCCTG ATAATCGCAC TGATGATGAG ACTGCATTCA CAGCCGCTAA 520800
AGCCCTGCAA CAGGAAGTCG ACAGAGCCGG TCTGTCCAAC ATTAAGATCA GCGCCGCTGA 520860
GGGTGTTGTG ACAGTCGAAG GCACGGTCAC GTCTGCTTCG GCCATCAGTT GGCATAAAGT 520920
GCAGCAGTGG TTCGATCATC GGACGAGGGG CGCTCTAGCG CTGTTGAATG GAGTGATTAT 520980
CGACGATGAG AAGGCGCCAT CCGCAATCGC AGTCGAAGCT GTCTGGCGTG GGTCGCTTCC 521040
GTATCTTGTC ATCAAAGGAG AGAAATATTT CGTCGGCGCT CTTTTGGATG ATGGTTGGAT 521100
GGTCGAACGG ATCGAGGACG GTCGTGTAAT GCTAAGCCGA AATGGCCGCC TTGCTGCTGT 521160
CCCGTATTAA AGTATTCCAG CGCAGGGCTG ACGATCTTGG ACAGACTTGA TGACCGGTCA 521220
ACTTCGCACT TGCCTTTGAA TCTTATGCGC AGCCATCCGC CGCAGGGGTC GGCCTCACGG 521280
ACGACGCGCC TGCGGTCTTA GAGCTACGTT GCACCTGTGT AGCAGCAGAG TGGTTCAGAA 521340
ACGATCGTGA AGGGACCTCA TGGCCAACGC CCTGCGTAGA TTCACCGAGT ATGCGCCGGC 521400
CAATCCGGAC TTGATGGTCG CGTTGATGCT GCTTCTGGCC GTCAGCATGA TGGTCATGCC 521460
AATACCGGTC ATGGCGGTCG ATGCGCTGAT AGGCTTCAAC ATGGGTTTGG CTGTACTGCT 521520
GCTGATGGCG GCCCTGTATG TCAGCACGCC ACTTGATTTT TCCTCTTTGC CCGGCGTCAT 521580
TTTGCTTTCC ACGGTATTCC GTCTGGCGCT CACCGTCGCG ACGACGCGAC TAATTCTGGC 521640
CGAGGGCGAG GCAGGCAGCA TCATCCACAC GTTCGGCAGT TTTGTGATCT CAGGCAATAT 521700
TGTCGTGGGT TTCGTTATAT TTCTGGTAGT GACCATGGTG CAGTTCATGG TTCTCGCGAA 521760
AGGCGCCGAA CGCGTGGCAG AAGTGGCGGC GCGCTTCACC CTCGATGCTT TGCCAGGCAA 521820
GCAAATGGCG ATCGACGCAG AGTTGCGGAA CGGTCACATC GATGCCGACG AATCCCGCAG 521880
GCGGCGCGCC GCATTAGAAA AAGAAAGCAA ACTTTATGGG GCGATGGACG GCGCGATGAA 521940
GTTTGTGAAG GGTGATTCCA TCGCCGGGCT AGTGGTTATC TGCATCAACA TGCTGGGTGG 522000
AATTTCCATC GGCCTGCTCT CGAAGGGCAT GTCGTTCGCC CAGGTGCTGC ATCACTACAC 522060
TCTGCTGACG ATAGGTGATG CGTTAATCTC GCAGATTCCC GCCCTGCTGC TCTCAATTAC 522120
AGCGGCAACC ATGGTTACTC GTGTAACTGG GGCTTCGAAA CTCAACCTCG GTGAGGACAT 522180
AGCCAATCAA CTCACCGCCA GTACACGAGC ATTGCGGTTG GCGGCCTGCG TCCTGGTGGC 522240
CATGGGCTTC GTTCCTGGTT TCCCTCTGCC TGTCTTTTTT ATGTTGGCCG CAGTCTTCGC 522300

GGCGGCAAGC TTCGTCAAAG GTGACGTCCT AGATGCCGAC AAAGTCGATG CTACAACTGT 522360
AACTCCGGCG GAGTCTCAAA CGCCAAACGT GGCTGCGCAG CCAAATCCCA TTGGCGTCTT 522420
CCTCGCGCCG AGCCTTACGA ATGCGATCGA CCAGGTCGAA TTGCGGCAGC ACATTGCGCG 522480
TATTTCCCAA CTAGTCTCGG CCGATCTCGG CATTATCGTT CCTCCGATCC CAGTCGATGT 522540
CGACCAGCAG CTGCCCGAGT CGCAATTCAG GATAGATGTC GAAGGCGTGC CAGTCGAACA 522600
GGATTTGATT AATCCGGCGC AGCTGTCCCT CGCAGACGAT CTGAAGAAGA TTGAGTCAAG 522660
CGGCATCCCT TTTCGGCATG ATCCTGAAAC CCACAGAATT TGGGTTGAAC AAAGCCACGA 522720
GCCGGCGCTC AAAGCCGCCG GTATCCGGCA TCATAGTCCC AGCGAACTCC TTGCGATGCG 522780
TGTCCATGCG ACGTTGACTT GCCATGCGCC GCGCTTGGTG GGTATCCAAG AGACCCGCCA 522840
ACTACTGGGC CGGATGGAGC AGGAATACTC TGATCTGGTG AAGGAGGTGC TGCGTACCAC 522900
GCCGATCCCC CGGATTGCAG ATGTGCTGCG CCGCCTCTTG GGCGAAGGTA TACCAATCCG 522960
AAATACCCGG CTCGTCTTGG AGGCATTGGC CGAATGGAGC GAACGTGAGC AAAACGTCGC 523020
CCTGCTCACG GAACACGTTC GTTCTGGAAT GAAGCGGCAG ATCTGTCACC GCTATGGCAG 523080
ACACGGTGTC CTACCTGCCT TCGTCATGGA ACGTGAGACT GAGGATGTGG TGCGCTGCGC 523140
GGTTCGGGAA ACGGCTGCAG GCCCCTACCT CGCACTAGAG GATCGGCAAA GCGAGGCGCT 523200
GCTGTCACAG ATGCGGCAGG TCTTTTCGAG CACGGCACCG GGCCAGACGC GCCCGATCGT 523260
CTTAACTTCA ATGGATGTCC GACGCTTCGT CCGCGGTTTT CTTACCCGAA ACGGTATCGA 523320
GCTTGCCGTA CTGTCTTATC AGGACCTCGC CTCCGATTTT AAAATTCAAC CCGTCGGATC 523380
CATCAGGCTC CCGCCCAGTA ATGGAACGTC AGGGGAACCT CGCAGTATCC GCCCTTCTGC 523440
CACTACTGGA TGATAGGTGA GAACCAAACA TGAATTCACA TGAGAATAGA GTCGCCGCGC 523500
CGTTGCTTTC ATTTCGTTTG AGCTTAGTTC TCTTCGCAGT ACTCTCCGTT CTGCCTCTCG 523560
GCGGTTGTGC TAGGTGGGAT AACCCCGTTC TTTCAGTGAA GGAGACGTCA GCCGCACAAT 523620
TGCTCGGCGC CAACCAAATC AATGCGGCTA CGCGCCAACG CATTTTGCGC GCCGTCGGTG 523680
AGGACGCTCA AGAGCGGGCT TTGCGGGATG ATCTGAAGCA GCATCCAGGC AATGTCGATG 523740
CGGCAATCCG TCTTACAAAA GCATTGGTGG CGCAGAAACG TCCGCATGAG GCGCTTCAGG 523800
TTTTAGACAA CGTCTTGGTC GTGACCCCAG ACAATTTGCG TGCATTGAAT GCGAAGGCAG 523860
TCATCTTGGA CATTGAGGGG CGGCATGACG CGGCACAAGA GCTGTACCGG CAAGCTCTCG 523920
AAACCAACCC AGAGAATCAG ATGTTGCACC ATAACCTCCA TCTGTCTCTT GCGTTTGAAG 523980
GGAAGTCCGA ACAGAGGACT TTGCCACAAT CGCGATAACG TTGCTGTGAT GAATTCACCT 524040
TTGAGAAGCA GACGCAGCAC GTCGTGCGTG CCTGGGGACG AGGCAGGCCC TATAGCAATT 524100
GAGCGCTGAA CACGACATTA AAGGGACGGA ACTGAGCGGC TAATCCTATT TTCTGACAGC 524160
GGCTCTGGTT TGAATTGGCG CTGTGTCCGT GGCGCTCGAC ACCGAGGGCG CGGCCGGCGC 524220
GCGGCGTACA GCGTTTTGTC GGTCCGGTTA TTGACCTTGC CCGCATCCGC CTATCTCAGG 524280
GTCGCGTTAC CGCTGAACTC ACTGCCGTTG TCCTGACGAT CATCATCGGT TCGCTGCGCT 524340
CCAGGATAAA TCCCAGCCGA GCTCACGGGT GCAACACGCA GGCCAGGGCC ATTATAAGAG 524400
TAGTGCGTAA AGGGGGCTCA TCAAATGGAG CCAATTTGGT TCCCTAGGCG ATTAAACAGC 524460
ATGCGGCATC AAAAGTGTCA CCCTACAGAA TATTTATAGC ATTAATATTG CCCCATAGTG 524520
TTGCTTCTGC GTAACCGTAT TTAACATAGG GTCAAGTGCG TGATGCCTAT ACGCGGACAA 524580
AACGAAACTA TTCCCTCAAA TAAGCAGAAG GATGTCGACA AGACCTTGAA CAAACTTGGT 524640
ACGAAGTTTT CTGGCACGCG TGATAAGCAC CTAGGATACC CGTACAACCT CGACTTTGAC 524700
CACAGACCAA TCTCTCAATC CCACAAATTT CTAAATAACA TGGGTGCCAT ATGTCGGCTC 524760
ACACTATGCG ACCGAGGTCT GTGACTAGAG TGCGAGGTGG TCGACTGGAC CATGCGGGTC 524820
GGGGAGTGCG CTGATCCAGA AGAATACTGG GGAACTGTGG GCGCAAGTGA AACTGAAGGT 524880
AATCGATGGC CATATACCTT GCGCGCGAGA CCCTTGATAA TCCTATTCTT ATACACTCGA 524940
CAGAGGGTAG TGTGCTGAAG GTTCTGCAGA ATTCTCTGAG AGGGGCGGTT ATGGCAGCCT 525000
GGTGATGACG AACGTGGATC ATGACGTGCT GATCGCAACG CTCGACCGGC TGAAGCTGAC 525060
GGCGATCCCC GACCAGCTCG ACACGTTGCT GGACGAGGCA GCGCGTTCGC GAGCGTATCG 525120
AAAGAACGAT CAGGCTTGGG TCGAGCAGAA GAACGGCGCG ATCGTGCGAC GGCTGGTCGG 525180
TTACGGAAGG CTCGTCGGCG CGGAGGCGAC GGTTGTGCTT GGGCGGCTCT ACGACGTGGT 525240
GCGTCTGTAC GGCAATCTGT TCCAGCCTTC GTTCAAGCTG CGAGAGAAGA CACGGATCGG 525300
CGCCCGCGTC GTCAAACGCT ACCATCCGCC CGTGCCGCCT ATCGCGCGGG TGGTTGCCCA 525360
TTCCGGTGTG GCCGAGGCCG ATAAGGAGCG GCTGCAGGCG ATGTTGGAAA GGGAAGATCC 525420
GGTGATGCTG TTTGCCGGAA TTCGTGCCGC ACAGGAGGAG CTCGGAAAGC GGGTCGATCG 525480
CCGTGGCTTG AATGCCGGGA CCGAAGAACC GCTCGCCATC GATCTGCAGC GATTCACCGT 525540
GAGCTTGAAG ACAGCCTGGC AGGCTGGAGA GAAACGGCCG ACACATCGGC GGCCCTATCG 525600
GCGAACCAAG CCTTACCCGA AAAGGCCGAG CATGCTGGAG CCATTCGAGC CCCAGATTCG 525660
AGCGTGGCTT GAGGCAGATC CGGCGCTCTC GGCAGCCCGC GGTGCTCCAG CGCCTTGTGA 525720
GCGCCGATCC GTCACGCTTC ACCAAGAAAG CGCTGCGGAC GGTGCAAATG GCCGTCAAGG 525780
CTTGGCGCAT GGAAATAGCC GGGCAGATCA TACTCGATGG CAACTGGATG AAGCGCGCGC 525840
CCGTATCTCC TCCCGCCGCG GCGCAGGGCT GTCCGGGGAA AGTTGCGGCT GAATCGAAAT 525900
GTCGCAGGCG CATGCCCCGT CAGACGGGGA TTTTCCGTCT ACCTTCTGGT TGTCGAGACT 525960
CAGAAAGAGG ACGGGGCATG CGGTTCACAC GTAGCATTCT TGGCAAGCTG ATTGAACCGG 526020
TCAATCGGCG CCGCTTCCAG ACGATAGTGG ATGATCACGA CGGGGACGCC TACGACAAGG 526080
CGTTCCGCAG CTGGGACTGC TGGTGCTGAT CTATGCCCAG TTGAGCGGTG CGGAGAGCCT 526140
GCGCGGCCTG GAGGCCGGCT GGAACGCCAA CAGCCATCAC CACTATCACC TGGGCAGCGA 526200
CCGCTTGCCG ATGCCAACGC CCGCCGCCCG GTTGCCGTCT TTGCCGAGAC CTTCGGCCTG 526260
CTTGCCGGCC AACTCGACCG ACAGACGCGC CGCGAGGGCC GGGCCATGCT GCGGCTGATC 526320
GATTCCACCC CGATCCCGCT CGGCAAGCTG TGCGGCTGGG CCAAGTCGAA CGGCCGCATC 526380
CGCGGCATGA AGATGCATGT CGTCTATGAC CCCGACAGCG ACTGCCCGCG CCTCCTCGAC 526440

ATCACCGATG CCAACGTCAA CGACGCCCAG ATCGGTCGCA CCATCGCCAT CGAGAGCGGC 526500
GCCACCTATA TCTTCGACAA GGGCTATTGC CACTACGGCT GGTGGACGGC GATCGCCGAG 526560
GCGAAGGCGT TCTTCGTGAC CCGGCCGAAG TCCAACATGG GCCTCAAGGT GGTGCGCCAA 526620
CGGCGCATCA AGGTCGCCGA GGGCGACGGC TTCACCGTCA TCGACGACGC CACCGTGCGC 526680
CTGGCCAGCA AGGGTGATTC CAAGCTGCCG ATCCCGTTGC GCCGCCTCAC CGTCAAGCGC 526740
GCCGACGGCG ACACCATCAC GCTGCTGACC AATGATCGCA AGCGGCCCGC CGTGGCCATC 526800
GCGGCGCTCT ACAAAGGCCG CTGGCAGATC GAACTCCTGT TCCGCTGGAT CAAGCAGCAC 526860
CTCAAGATCC GCAGTTTCCT TGGCAACAAC GACAACGCCG TGCGCCTGCA GCTCTTCGCC 526920
GCGATGATCG CCTATGCGCT ACTGCGCATC GCGGCGCGCC TTAACCGCAT CACCATGCCG 526980
ATCCTGCGCT TCACCGACTT GGTTATCCGC TGCCTCTTCG AGCGCCGCGA CATCGCCGCC 527040
ATCGAGCGGC CGCCACCGGT CAATCCCAGC CACAGGAGAC CCCGGTGCTC CCCCCATCAA 527100
ATGAGCTTCG CCTATGTCTG AATTTCCCCG GACAGCCCTG CGCCGCGGCG GGAGGATATA 527160
CGGGCCATCT CAGTGCCCCC AACTTCAGTA ACATTCCTGG ATGAGGCAAT AGGGGGGGTC 527220
AATTCTCCGA TTCGCTTGAC ATGCCGGCTC TATCTCCCCC GATGCTCTCT CAGAGGTTAT 527280
TCACGCAAAT CGCCATCGCA CCGTCATTCT CGCATTGACC TGTGGACCGA CCATGAAAGG 527340
GGGCGCACGA CGATATCGCG TCCTGCATCA AGGTTCTGGA CCAAGTCGGG ATCGATCCGA 527400
ACCGACGCTT TGTCCACGTC GAGGGCGCGC TGAATGCAAT GGTACTTCCC TTTGTCGATG 527460
GCTTACAGAG GGGCATCCGC CCTTCTTTCC GCCGTCAGCT ACCTGCGCAG ATGATGCGAC 527520
CCCGATGGGA TCGCGAAACG GCCACGCCGT TCTCGCGATT TGGAACGGGC GCATGCGCCT 527580
TGACATCGCT GGTTTCCGCA ACAATCTTCG TGCCTGCATC GAACGCGCGG AGGGCTTCGC 527640
AGCAGCACTT CGCTCAGTGG GCGTCTTGGT ATTGCTCAAT CCGTGGTCAT TGACGGTTGT 527700
GTTTCCAAAA CAATCGGGGA GGCTCGTCAA CGAATATCAA CACGCATGCA ATGGAGGTTC 527760
TGCCCACGCG ATCGTCAGCC AAGCGTCAAA GCAGATCTCA TCTATGATCA TGGCCTTCCT 527820
TGGAGGCAGC TTCCTGATTG CTGGTGACGG CCTGACGACC GGAAGCATTG TCGCGTCAGC 527880
CTCGCTCTTG TCGCCGGCCT AGGTTACGCC AGCTTTATCT TCGCATGGAA GCTTTTCCGC 527940
GCTCTTCGCA GCCGTGTTCT TCGGGGAATG GCTAACAGGT CCAGAGTGTC TGCGGCACTG 528000
GCTATCGTCG TTGGTGCGTT GATCACTTCC ATGCCTGAGC GCCGTGTCGC CTTGGATGTT 528060
TCTATGCGGG CGTTTATATG GCGCTGCTTT TTGTGGTGGT TGACCTGCGA GAGCATCGGC 528120
CCGAGCCGCC GCACTACAAT AGAAAACCTG TCGCCGGGGG ATAGTGAATG TGCGCGGCGG 528180
ACTACGGTAG GTCAGCCGCC GTTGCACCGT GCCTGGTGCG TGCGGGCGGT CGGCCCTCAG 528240
CAGGCGCTCG GCCCGTAATC GGGCCTCGCC ACCGCCGGCA TGCCGTGGCT CGGGTCCTCC 528300
CTGCGCTTAC ACGGACCCCT GGGCACGAAT ATCAGGAGCA GGGCTTCCGG CCCGGGGCAG 528360
GGGAGTAGGG AATCGCATTT GACTATTTTG GTTGCATTTG GAGGGCCGGG GGATTCTTGA 528420
GATAGGCAGT TCGTGGAGCA GAAACCGCCA TTCCTTATCT TGAAAGCTGT TTCGGGGCGT 528480
CTCCATGCAA ATAGGCCCAA TTGGCGAAGG GGAGTAGGGC ACCGGTCGAT CCTTCTCCCG 528540
ACGAACTGCT CAAGCACGTC TCGCTGCTCA GTTCGGAGCA CATCAGTCGC GATCGTCGCA 528600
AGCAGCGGAT GGTGCCGCCC CGGCGCGAGT GGGCTTGAAT GCCTGAAGAA AGACTCCAGC 528660
GAGCTTACGG ACGTATGCAT GTTGCTCGGT CGCCATCACC GATAAGTTCC CTTTCTCCAC 528720
AATCGCCTCG AGACTGGGCT TGATCTGCAG CTGCCGAATC CAGCCGACTT TCGATCGCAT 528780
CTTCATCGGC ATCATTGGCA GCCTGGAGGG CCAAAATCGT GTGGCGGAAC AGTCGCAGCG 528840
CTCCCCGATC CCGCTCAGGG TTTCAACATC ATTCTCGGTC AGGAGCGCGG CACCAGGAGA 528900
TCGCCAAGAA TTGAGCTTCT ACAGCGGGTC GGCTATCGCC AAGCGCGGGC CATTTTTGAG 528960
GCAGCCTACA CCGTTGCCGG CATAGCTGAA GCTTAAAGAA ACCGATGCAC CTCGCGAGGT 529020
GAGCAACGGA CCGTAAGTTC GCCGTCAGCT TAACGAAGCA TCGTGCGAAG CGCGGTCTAA 529080
GCGGCCCCAT ATTGGCAATA ACCGTGCGAA GGGAAACGAA CGCCATGCAC AGTCCCATCA 529140
GTGGTTCATT CACAAGCTCT ACGCAAGTTC ACGATCCGAT TCATCCAGCG AACAGCGATG 529200
GTTTCAGGGA GACGCTTGCG AATGTTGAAC TCCGGACGAA ATCACCAAGT GCGGAGTGCC 529260
CCGATAAGAT GGGGTGCTGC GCCAGCAAGC CACAAGCATC TGATCCGAAC AATCCCAGTA 529320
CCTCCTCTCC TGCGAGACCG AGCACCTCCC TGTTCCGATA CAGGACGGCC GAATTGGCCC 529380
AGGCGAACGC AGATGGAATC TGCGTCGGCT TGACTGCGGA GTGGCTGCGT AACCTCAACA 529440
GTCATCCGTC AATCCGAATG GAGGCCCTAG TACCCGGATC GCAAAGGCAC GCCTCAGCCA 529500
CTGTTCGGCA GAAGGAGTAC GAGAATCTGA AGGTTCACCT CCGAAGACAA GGAGCAGGAC 529560
CTTCTGAGGC CGACTTCGCG GCGCAAAACA CTATGTTGCA GAAAGCAGGC CTTGCTCCAT 529620
CCGGAAAGGA GAAAGTATAC AAAGTCGGCG AGCCTAACTT CCCGCGCATG CTAACCAAGA 529680
TTACAGCCGA CGGATCCAAC CATTTGCTCA GCTTGTATTT CGCTGAGGGC GGCGCACACA 529740
CCGTTGCGAC CTCGGCAATG GATGGAAACA CCACGCTCTT CGATCCTAAT TTCGGCGAAT 529800
TTACTGTGCA ATCAGATCAG ATAGATGATT TGTTCCGAAG CCTAGCCAAT CGCTACAGCA 529860
ATCCAAACCG GCAGCATTTA ACAACGGTGA CCACCCAAAA GATGACATGA GTCGGGCGTC 529920
CTGCGCGGCG TAGTGTTCAC TGAAGTCCAC GTAAATCCTC GTCGATGCTT GTGCTGAAGA 529980
GGTGGGATTT TGGTACGCTG AGCAGCAATA GCTGCTGCGT GCGGGGCAGG AATTGGACGA 530040
CGCGCCGGCG GACCAGGTGG GTTTGAGCAG CAGCTGGCCA TCTGCAGCTC AGCTTATCGG 530100
AGAACTCCGC CGAGCCGATT TCGCCCGACA CTGGTTCGGT AGAGGTGCAT GGAGGTATTC 530160
AGCGCATTAG CCTCCGCGGT TCGATGCTGA TGCCGCCGCA ATCCAACTGG CAGGACAATC 530220
GGTTCAGCAG TGTCGCGGCA CAGGTCGACG TTCCTCGCGC CCACATTCGC GGTCATGCAA 530280
GGTTCTCCAT CTGACTTGCG CAACAGGACT TTTCGACATT CCAGCCGATG AAGACAGCTT 530340
TGCAGCGCGA CAACCAAGAT GAGACGCCAG CGGCAAGCAC GACCAGGTCG TCGATTATCG 530400
GCATGTCATC CATTCGCTGC GCAAAAAGCC GATGGCGCTC CTTCAGCTTG TCTATCGCGA 530460
CAAGCTGTTT CCGCGGCAGG AATACCGAAG GGCCTTCGAG ACCCTGCTCG ACCGGCTTTC 530520
CGACAAGCAG GCCTGCAAGA TCACCGTCGA GCTCTTGGCC TTGGCCCACG ATCGGGGCTG 530580

CGAGCGGGAA CTGGCAGAGC GGCTCGCTAA AACACTCGAT GCGGACGAAC TGCCGGACAT 530640
CATCGCGCTG AGGACTTTCT TCGCGCCCGA TCCGGCACAG TTGCCCACCG TCAACGTTCG 530700
TCTCGCCTCC CTCCAGGGCT ATGAGGCCCT GATCGACGCG CGTCATCTGG AGGACGCCGC 530760
ATGAAAAACG CCCCTGCCAT CGACGCCGCC ACGCTCAGCA CGCTGCGCAA TGAACTCCGC 530820
CTTCCGGCCA TCAAGGTCCT TTGGCCGGAC TTTGCCGAAC GGGCCGACAA GGAGGGGTGG 530880
CCAGCGGCCC GGTTCCTGTC GGTGATCGCC GAGCACGAAC TGGCCGAACG CGATCGCCGC 530940
CGCATCGAAC GCCATCTCGC CGAGGCGCGA TTGCCACCGG GAAAGACGCT CGACAGCTTT 531000
GACTTCGACG CCGTGCCGAT GGTCTCCAAG GCACAGGTCA TGGCGATCGC CGCCGGGCGA 531060
TAGCTGGCTC GCCAAGGGAG CGAACATCCT GTTGTTCGGC CCGCCCGGTG GCGGCAAGAG 531120
CCATCTCGCC GCCGCAATCG GCCTCGCGCT CATCGAGAAC GGTTGGCGGG TGCTGTTCAT 531180
GCGTACGACC GAGCTCGTTC AGAAGCTGCA GGTGGCACGT CGTGAACTCC AGCTCGAATC 531240
CGCCATCGCC ACGAAGCCCA ACCGACATGG ACAGTCGCGC TGCGCTGCGG GCAGCGATCG 531300
AGACAGCTTC AACGACAGAC CAGGGTCAAC GATCGCAAAA ACAATCAGTG CTACGTACCG 531360
GGTACGCCGG GCCCTGCTGC CAATGGCAAC CGTGATCGAA CGCATGGGTT GTCGGCCCGC 531420
GCCATTGCCC GGCGTCGTGC CTAGGCAGCT CTGATCGGAG CTGACGCTTA CGCGCTCGAG 531480
ATCGATCCTC GCTTGCAGGC CCCGCATCCG GTCTGGGGGG TGGGACATCC GGGAGGGTGA 531540
CGCCACTTAG CCTGGCTCGC TGGTGACTTC AACGCCAGCC GTCGGTACGC GACAGTCATA 531600
ACGCAGGCAT CAAACTCCGC CAGAAGCTCA CTGATGACGC GGCAGACGAT GTTCATCAAG 531660
CCAATCGGCT GCCTGCTCTC GCAGGTCAAC AACCGCAAAA TGCAGCGCCA TATGAACGCT 531720
CGCATAGAAA CATCTAAGGC GCTGTGACTG TTCCTCGCCG CGATTTTTGG CCCCTGCAGG 531780
CTGCCAATGA CGCCGATGAA GATGCGATCG GGACTATAGA CCGGAAAGTC GCTGACACCG 531840
GCTGCTGCAG ATCAAGCCCG GTCTTGAGGG GATGGTGGAG AAAGGGGAGG TATCGCCTCT 531900
GGTGATAGCG ACCAGCAACG AGCACACGTT CGTAAGCTCG CGGGAGGCTT CCTTCCAGGC 531960
GTTCACGTTC GACTCGCCCC GGGGGCTATA GACGAACACC TTATGCCCAA GCCCGACTGA 532020
GTGAGCATGT TACATGTTCA TCCACGGTAT GGATGATAGC TATGCAAAAA CTCGATTTTT 532080
CCATTCTCGC GAAATTCGAT TAAGAAACTC GTACAGGTTG GAGGCAGAGT GGACAGTCAG 532140
ACACCGGCCA CGCAGATATG ATTGGCAATC CGTCCGCGGA TGCATTGAGG CAGGCTTCAT 532200
GAGCGAATTC AAGTGGGGTC ACATTCGCGG CGATCTGAAT TCTCGGCTCC CCAGCTATAG 532260
CCGCAGGCAG CGCAATCTCG GGTGCTCACC CGTACGTGCG TGCCCTCCAG TACATCACAA 532320
GCCCTCGCTT GGTTTAGCTC GGTGCGACGG AAATCGACAG GCGCCGCCGC CGAGGGTGCT 532380
GAATTGGCGC AACTGGCAGA TTCATGAGGC ACTTGTTTGA AAGCAAAGCT CGGTCGATCT 532440
ATTGGCCGCT TCGGCGACGA GCGGGGCAAC ATAGCTGTTC TTTGCTTTAC AACGGCAGCG 532500
CAACGCCGAA CCCGCCAAGC GTTTTTTGAG GAGACCTGCT CCGGCTTGAG TTCGGAAGAC 532560
ACTTCCCGAG ACACTGCATC GCCAGGTAAC GCATCTCATC GGCTCCGTTG AAGCTCAGTT 532620
TATCGCGGGT GCCATCTTGT GGCCCACAAA CACCACTTAC GAGGAGAAGA CATCAATGGA 532680
TCAGCCCGCT TGGAAGAATC CGCGTACGGC CCTGATCGTC GATGGCAGTG ACGCTGCACG 532740
TCAACGGCGT ATACCGAATC TGGCGTCGGT CGACCTAAAC CTGCTAGTGG AGCTCGAGGC 532800
CTTATTGCAG TATCGAAACA TCACGCATGC GGCTCAACAT GTCGGTAGGA GCCAACCGGC 532860
AATGAGTAGA GCCCTATCAA GGCTGCGTGA CATGTTCAAT GACGATCTCT TGGTACGCGG 532920
CTCGAGTGGC CTAGTCCCAA CACCGCAGGC CGAACACTTG GCGCAAATGC TGCCGTCGGT 532980
ATTGAATGCT ATCCGCGAAC TGGTGAGCTG CAGTTCCGGC TTAAGGGATT TGCGGTCGAA 533040
GGTAACTATG GCAATGCCCG ATCACCAATC GCTAGTTCTG CTGCCATATC TATTGCCGCG 533100
TTTAGGTGAG CGTGTGCCTC ATGTTGACAT CGTCACCGAG CCACTTTTGG ACGGCGCTCT 533160
ACGGCGTCTC GAGCAAGGTG AGATTGACTT TGCGATCGGG CAGATTGGTG CCGCTCCTCC 533220
AGGCTACTTG CGGCGCGGTC TCTATGCGGA CCGCTTCACT TGCCTGCTTC GCCACGACCA 533280
CCCTGCATTA GAGCAGGAGT GGAGCGTCGG AACCTTCGCG GCGCTGCGTC ACGCCTCTAT 533340
TGCCTCGGAT TCCAACGAGG GCCTGGGTCA AGTCTATGAT GGGCTGGTCA GGTTCGGACT 533400
ACCCGGTCCG ATAGTGGTCT CCAACGTGCT GACTGCGGCA GTTGTAGTGG CGATGACTGA 533460
CCTAGTGCTG ATGATACCGA ACCGAGTTGC AACTCGGGTG GCGACTATGC TGCCGCTCGC 533520
TATTGTAGAT CCACCCGTGG AGCTGAAGCC ATACGAAGTT GCGTTGATCT GGCACCAGCG 533580
CTGCCATCAT GACTTGGAAC ATCGCGTGCT GCGTCGCGAA ATCGCCGCCG CAGCGCGGAT 533640
GGGCCGGCTA GACGTGTCAC AAGACCAAAG GGCGAGGCGG CAGAACGATA GTTGAACTCA 533700
CCGCTGAACG GAAGGGTGCA GCGAAGGACA GCCCGACACC GCGTGCGATT GATCCCATCA 533760
AGCGCCGGGT GTTTGACGCT TTAGCTTCCT CAAGGCATCA GTTCATCTCG GGGTTCTCGC 533820
TGCCATGTCA TCGCACTCGC CGTGGACGCT GGCGCCACTC TGCTAAGTGC CGCTTTGCAT 533880
CGTCAACATT TGACCTATAT TGCACTGCAA CAATGGTAGG CTTGCCTGAT CCACCATTAT 533940
GGTCACAGGT TCCCTTCAAG CGGGAACGCG CGCTCGCTGC GCACGATAGA AGGAGTTCCA 534000
TCTCATGAAA AATTTAATCC ACAACGTGGG CCCAAACGAC GCCCGTAGGG ACCACGTCGA 534060
TATAGGTGAT GCGGTTCGGC GATCTCGCGA AGCGGTCGGA TATAGCGTCG ACGACCTCGC 534120
ATTAACATGC GGTCTGACAT GCGCCGAAAT TTCAAGGATC GAATTAGGTG CTGATGTCGA 534180
CCCTGAACGG CTCAGGCGTG TAGTCGCTGC CCTTCGAGTG CCAGCCACCA CCTTCGCATT 534240
GAAACATTCA TGAACAGCAT TCAGTGGAAA CAAGCTCCGC AAGCTCGAGT ATAGTCCCCG 534300
ACGCGATTGC CACCTATGCC GACACCCTCA TTTCAATAGG TGGCGTACAG TCGAACCACA 534360
CGCGGAGTGT CGCTGCGGTT GCAGCGAAGA CCGGCATGAA ATGCCTCCTG GTTCAGAACA 534420
GTTGTGCCAT CGGCCGTGAT ACACGACCCG GCGCTTCTGC ATTCCTATGC ACATCAATCC 534480
AGCCGAGAAC ATCTCGGTAT CTGTGCGAGA ACGACTTCAC CATCTGCGTC GGCGGCGAAA 534540
TCATCGATAA ATCAGTCAGG CCTGGAACCT CTTCGTCAAC AACAAAGTGC TTCGTCACCT 534600
CCATAAAGTC AGGCAAATGG GCGCAGGTGA AAACTTAAAT GCAATCGAGT CGACATTCAC 534660
CCTCACGAGT TCTCCTAACG ACCAAACTCT TCTCATCAAA CGTTCGTGCT CCTCAAGTTC 534720

## Description

### BACKGROUND OF THE INVENTION

This invention relates to a symbiotic plasmid of the broad host-range *Rhizobium* sp. NGR234 and its use. In particular, this invention relates to the isolation and analysis of the complete sequence of the NGR234 symbiotic plasmid pNGR234*a*, and the open reading frames (ORFs) identifiable therein as well as the proteins expressible from said ORFs.

Together with carbon, hydrogen and oxygen, nitrogen is one of the essential components in organic chemistry. Although it is present in vast quantities in the atmosphere, nitrogen in its diatomic form N₂ remains unassimilable by living organisms. The nitrogen cycle begins by the fixation of nitrogen into ammonia which is chemically more reactive and can be assimilated into the food chain. A large fraction of the total nitrogen fixed every year is produced by microorganisms. Among these, the soil bacteria of the genera *Azorhizobium*, *Bradyrhizobium*, *Sinorhizobium* and *Rhizobium*, generally referred to as rhizobia, fix nitrogen in symbiotic associations with many plants from the *Leguminosae* family. This highly specific interaction leads to the formation or specialized root-, and in the case of *Azorhizobium*, stem- structures called nodules. It is within these nodules that rhizobia differentiate into bacteroids capable of fixing atmospheric nitrogen into ammonia. In turn, ammonia diffuses into the vegetal cells and sustains plant growth even under limiting nitrogen conditions.

The *Rhizobium*-legume interaction presents many interesting features. Obviously, the possibility of using this symbiosis as an "environmentally friendly" way to provide some of the most important world crops (such as soybean, bean and many other legumes) with fixed nitrogen without using nitrate-rich fertilizers, has important economic consequence. It is also an ideal model to study a non-pathogenic interaction between bacteria and a highly developed, multicellular organism such as the host plant. Furthermore, the various steps involved in the establishment of a functional nitrogen symbiosis, which include some dramatic morphological changes as well as processes of cellular differentiation, require a complex exchange of molecular signals. Despite many decades of studies, it is only recently that the *Rhizobium*-legume interaction has been partially understood at the molecular level. The establishment of a functional symbiosis can be divided into two major steps as follows.

### (A) Rhizosphere ecology and nodulation:

Rhizobia are soil bacteria that proliferate in the rhizosphere of compatible plants, taking advantage of the many compounds released by plant roots. In return it has been shown that the presence of rhizobia in the rhizosphere reduces susceptibility of plants to many root diseases. In the case of low nitrogen levels in the soil, compatible rhizobia can interact with host plants and start the nodulation process (Long, 1989; Fellay *et al*., 1995; van Rhijn and Vanderleyden, 1995). Molecular signalling between the two partners begins with the release by the plant of phenolic compounds (mostly flavonoids) that induce the expression of nodulation genes (referred to as *nod*, *nol* and *noe* genes). The NodD1 gene product appears to be the central mediator between the plant signal and nodulation gene induction (Bender *et al*., 1988). It is modified by the binding of flavonoids and acts as a positive regulator on the expression of the remaining nodulation genes. Among them, the *nodABC* loci encode products responsible for the synthesis of the core structure of lipooligosaccharides called Nod factors (Reli 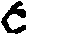 *et al*., 1994). More nodulation genes are involved in strain-specific modifications of the Nod factors as well as in its secretion. It seems established now that variability in the structure of Nod factors may play a significant role in the determination of the host-range of a given *Rhizobium* strain, that is in its ability to efficiently nodulate different legumes. For example, the strain *Rhizobium meliloti* can only nodulate *Medicago*, *Melilotus* and *Trigonella* ssp., whereas *Rhizobium* sp. NGR234 can symbiotically interact with more than 105 different genera of plants, including the non-legume *Parasponia andersonii*.

The structure of many Nod factors, their isolation from *Rhizobium* strains and their commercial application in agriculture have been described (NodNGR-Faktoren: Reli 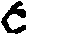 *et al*., 1994; WO 94/00466; NodRm-Faktoren: WO 91/15496). Secreted Nod factors act in turn as signal molecules that allow rhizobia to enter young root hairs of a host plant, and induce root-cortical cell division that will produce the future nodule. Invaginated rhizobia progress towards the forming nodule within infection threads that are synthesized by the plant cells. Bacteria are then released into the cytoplasm of dividing nodule cells where they differentiate into bacteroids capable of fixing atmospheric nitrogen.

With respect to regulation of the nodulation genes, other regulatory genes with similarities to *nodD1* (genes that belong to the *lysR* family) have been identified in various strains (Davis and Johnston, 1990). The function of those genes, called *nodD2*, *nodD3* or *syrM*, is only partially understood. Some *nodD* genes have been described (WO 94/00466; CA 1314249; WO 87/07910; US 5023180). Also, recombinant DNA molecules including the consensus sequence of the promoters of *nodD1*-regulated genes, called *nod*-boxes (Fisher and Long, 1993), have been disclosed (US 54847186; US 5085588). Finally, recombinant plasmids with the *nodABC* genes or, in one case (*Bradyrhizobium japonicum*), a sequence influencing host specificity have been disclosed (US 5045461; US 4966847).

### (B) Symbiotic nitrogen fixation:

Inside the nodules, rhizobia differentiate into bacteroids that express the enzymatic complex (nitrogenase) required for the reduction of atmospheric nitrogen into ammonia. The nitrogenase is encoded by three genes *nifH*, *nifD* and *nifK* which are well conserved in nitrogen fixing organisms (Badenoch-Jones *at al*., 1989). Many additional loci are necessary for functional nitrogenase activity. Those originally identified in *Klebsiella pneumoniae* are known as *nif* genes, whereas those found only in *Rhizobium* strains are described as *fix* genes (Fischer, 1994). Some of these gene products are required for the biosynthesis of cofactors, the assembly of the enzymatic complex or play regulatory and different accessory roles (oxygen-limited respiration, etc.). Many of these genes are less conserved among the various rhizobial strains and in some cases their function is still not fully understood. The high sensitivity of the nitrogenase complex to free oxygen requires a very strict control of most *nif* and *fix* gene expression. In this respect, the FixL, FixJ, FixK, NifA and RpoN proteins have been identified in representative *Rhizobium* species as the major regulatory elements that, in microanaerobic conditions, activate the synthesis of the nitrogenase complex (Fischer, 1994). Recombinant DNA molecules containing *nif* genes/promoters have been disclosed: *nifH* promoters of *B. japonicum* (US 50008194), *nifH* and *nifD* promoter of *R. japonicum* (EP 164245), *nifA* of *B. japonicum* and *R. meliloti* (EP 339830), *nifHDK* and hydrogen-uptake (*hup*) genes of *R. japonicum* (EP 205071).

Many more genetic determinants ploy a significant role in the *Rhizobium*-legume symbiosis. Genes (exo, *lps* and *ndv* genes) involved in the production of extracellular polysaccharides (EPS), lipopolysaccharides (LPS) and cyclic glucanes of rhizobia play an essential role in the symbiotic interaction (Long *et al.*, 1988; Stanfield *et al.,* 1988). Mutation in these genes negatively influences the development of functional nodules. In this respect, some exopolysaccharides of the NGR234 derivative strain ANU280, have been disclosed (WO 87/06796). Although Nod factors seem to play a key role in the nodulation process, experimental data indicate that other signal molecules produced by the bacterial symbionts are required for functional symbiosis and may play a role in coordinating various steps such as the controlled invasion process, the release or rhizobia from the infection thread into the plant cell cytoplasm, the bacteroid differentiation process, etc. Moreover, the need for rhizobia to survive in the rhizosphere and to compete adequately with other microorganisms requires many more unidentified genes that, although they may not be characterised as proper symbiotic loci, do affect the efficiency of the various strains to induce functional nitrogen fixing symbiosis in field conditions. Finally, in our view genetic engineering of improved rhizobial strains cannot he pursued without a more extended knowledge of the structure and complexity of the *Rhizobium* symbiotic genome.

In this respect we decided to determine the complete DNA sequence of a symbiotic plasmid of *Rhizobium* sp. NGR234. In contrast to *Bradyrhizobium* and *Azorhizobium* that carry symbiotic genes on large chromosomes (ca. 8 Mbp) and to *R*. *meliloti* that harbours two very large symbiotic plasmids of 1.4 and 1.6 Mbp, NGR234 carries a single plasmid of ca. 500 kbp, pNGR234*a*. Moreover, it has been shown by transfer of pNGR234*a* into heterologous rhizobia, and even into non-nodulating *Agrobacterium tumefaciens*, that most nodulation functions are encoded by this plasmid (Broughton *et al.,* 1984). The fact that NGR234 is able to interact symbiotically with more plants than any other known strain, and that a complete ordered cosmid library of pNGR234*a* was available, reinforced NGR234 as the best choice for a large-scale sequencing effort on a symbiotic plasmid (Perret *et* *al.*, 1991; Freiberg *et al*., in press).

Automated fluorescent methods have been used to sequence cosmids from eukaryotic organisms, including *Saccharomyces cerevisiae* (Levy, 1994), *Caenorhabditis elegans* (Sulston *et al*., 1992), *Drosophila melanogaster* (Hartl and Palazzolo, 1993), and *Homo sapiens* (Bodmer, 1994), as well as chromosomes from the prokaryotes *Haemophilus influenzae* (Fleischmann *et al.,* 1995) and *Mycoplasma genitalium* (Fraser *et al.*, 1995). In most large-scale sequencing centres this technology is based mainly on the shotgun approach. After random fragmentation of DNA (e.g. cosmids, bacterial artificial chromosomes (BACs), entire chromosomes) using sonication or mechanical forces, size-selected fragments are subcloned into M13 phages, phagemids or plasmids and sequenced by cycle sequencing using dye primers (Craxton, 1993). A disadvantage of this method is that DNA regions with elevated GC contents produce large numbers of compressions (unresolvable foci in sequence gels) in the dye primer sequences leading to several hundred compressions per assembled cosmid sequence. It is known that the use of dye terminators - fluorescently labelled dideoxynucleoside triphosphates - instead of dye primers reduces the number of compressions (Rosenthal and Charnock-Jones, 1993). Therefore, dye terminators are frequently being used for gap closure and proofreading after assembly of the shotgun data.

To sequence GC-rich cosmids with the highest accuracy, the effectiveness of shotgun sequencing with dye terminators in comparison to dye primer sequencing was investigated. To improve the incorporation of dye terminators into DNA, a modified *Taq* DNA polymerase carrying a single mutation was used (Tabor and Richardson, 1995). This enzyme has properties similar to a thermostable "sequenase" and is commercially available as Thermo Sequenase (Amersham, Buckinghamshire, UK) or AmpliTaq FS (Perkin-Elmer, Foster City, CA, USA). Concentrations of dye terminators needed in the cycle sequencing reactions can be reduced by 20 - 250 times. It was found that dye terminator shotgun sequencing leads to compression-free raw data that can be assembled much faster than shotgun data mainly obtained by dye primer sequencing. This strategy thus allows a several-fold increase in speed to sequence individual cosmids. This was demonstrated by comparing assembly of the sequence data of two cosmids from pNGR234*a* generated by different chemistries: Cosmid pXB296 was sequenced with dye terminators, whereas data for pXB110 were obtained using the common dye primer method. Also disclosed is the analysis of the entire pXB296 sequence.

Moreover, the dye terminator shotgun sequencing strategy used to generate the sequence data for pXB296 was also used to sequence all the other remaining overlapping cosmids of the plasmid pNGR234*a*. In summary, 20 cosmids have been sequenced together with two PCR products and a subcloned DNA fragment derived from a cosmid identified as pXB564 in order to generate the plasmid's complete nucleotide sequence.

After its assembly, the analysis of the entire nucleotide sequence of pNGR234*a*, especially the determination of putative coding regions and the prediction of their expressible proteins and putative functions, was performed. Initially, analysis of the region covered by cosmid pXB296 was extended to cosmids pXB368 and pXB110. Thus, in approximately 100 kb of the plasmid (position 417,796 - 517,279) most ORFs and their deduced proteins with different putative functions were predicted. Subsequently, the rest of pNGR234*a* was analyzed.

### SUMMARY OF THE INVENTION

The present invention provides the complete nucleotide sequence of symbiotic plasmid pNGR234*a* or degenerate variants thereof of *Rhizobium* sp. NGR234.

The present invention also contemplates sequence variants of the plasmid pNGR234*a* altered by mutation, deletion or insertion.

Also encompassed by the present invention are each of the ORFs derivable from the nucleotide sequence of pNGR234*a* or variants thereof.

In a preferred embodiment, the ORFs derived from the nucleotide sequence of pNGR234*a* encode the functions of nitrogen fixation, nodulation, transportation, encapsulation, secretion (of proteins), transcriptional regulation, DNA-binding, peptidolysis, proteolysis, transposition, integration, rhizopine biosynthesis, or encode proteins exhibiting similarities to proteins of amino acid metabolism or related ORFs, or proteins putatively involved in the degradation of xenobiotic compounds.

In another preferred embodiment, the ORFs are under the control of their natural regulatory elements or under the control of analogues to such natural regulatory elements.

The present invention also provides the sequences of the intergenic regions of pNGR234*a* which, in a preferred embodiment, are regulatory DNA sequences or repeated elements.

The present invention also contemplates the use of the disclosed nucleotide sequences or ORFs in the analysis of genome structure, organisation or dynamics.

Also provided by the present invention is the use of the nucleotide sequences or ORFs in the subcloning of new nucleotide sequences. In a preferred embodiment, the new nucleotide sequences are coding sequences or non-coding sequences.

In yet a further preferred embodiment, the nucleotide sequences or ORFs are used in genome analysis and subcloning methods as oligonucleotide primers or hybridization probes.

The present invention further provides proteins expressible from the disclosed nucleotide sequences or ORFs.

Also contemplated by the present invention is the use of the disclosed nucleotide sequences, individual ORFs or groups of ORFs or the proteins expressible therefrom in the identification and classification of organisms and their genetic information, the identification and characterisation of nucleotide sequences, the identification and characterisation of amino acid sequences or proteins, the transportation of compounds to and from an organism which is host to said nucleotide sequences, ORFs or proteins, the degradation and/or metabolism of organic, inorganic, natural or xenobiotic substances in a host organism, or the modification of the host-range, nitrogen fixation abilities, fitness or competitiveness of organisms.

The present invention also provides plasmid pNGR234*a* of *Rhizobium* sp. NGR234 comprising the disclosed nucleotide sequence or any degenerate variant thereof.

The present invention also provides a plasmid harbouring at least one of the disclosed ORFs or any degenerate variant thereof.

The plasmids of the invention may be produced recombinantly and/or by mutation, deletion, insertion or inactivation of an ORF, ORFs or groups of ORFs.

The present invention also provides the use of the disclosed plasmids or variants thereof in obtaining a synthetic minimal set or ORFs required for functional *Rhizobium*-legume symbiosis, the modification of the host-range of rhizobia, the augmentation of the fitness or competitiveness of *Rhizobium* sp. NGR234 in the soil and its nodulation efficiency on host plants, the introduction of desired phenotypes into host plants using the disclosed plasmids as stable shuttle systems for foreign DNA encoding said desired phenotypes, or the direct transfer of the disclosed plasmids into rhizobia or other microorganisms without using other vectors for mobilization.

The nucleotide sequences of the present invention were advantageously obtained using known cycle sequencing methods. The preferred dye terminator/thermostable sequenase shotgun sequencing method used to generate the nucleotide sequences of the present invention, when applied to cosmids and when compared to other sequencing methods, was shown to yield sequence reads of the highest fidelity. Consequently, the speed of assembly of particular cosmids was increased, and the resultant high-quality sequences required little editing or proofreading. Thus, the preferred sequencing method described herein was successfully used to generate the complete nucleotide sequence of all the overlapping cosmids of plasmid pNGR234*a*, thereby resulting in the assembly of the complete sequence of the plasmid.

The complete sequence of pNGR234*a* is disclosed for the first time in this application, as are the majority of the ORFs predicted within the sequence. Putative functions have been ascribed to the novel and inventive ORFs disclosed herein and the proteins for which they code.

### DETAILED DESCRIPTION OF THE INVENTION

### Comparison of Different Shotgun Sequencing Strategies

The following is a more detailed description of certain key aspects of the present invention.

GC-rich cosmids were examined to investigate whether they could be sequenced much more efficiently using dye terminators throughout the shotgun phase instead of dye primers. As a test case, cosmid pXB296 with a GC content of 58 mol% from pNGR234*a*, the symbiotic plasmid or *Rhizobium* sp. NGR234, was exclusively sequenced using dye terminators in combination with a thermostable sequenase [Thermo Sequenase (Amersham)]. Another rhizobial cosmid with identical GC content, pXB110, was sequenced using traditional dye primer chemistry and *Taq* DNA polymerase.

Using the dye terminator/thermostable sequenase shotgun strategy, it was shown that most, if not all, compressions could he resolved and reads were produced with the highest fidelity among all sequencing chemistries tested. As a result, a much faster assembly of cosmid pXB296 in comparison to pXB110 was obtained. The shotgun data could be assembled into a high-quality sequence without extensive editing and proofreading. By measuring the error rate in overlapping regions between individual cosmids from pNGR234*a*, as well as the cosmid vector sequence itself (data not shown), it was estimated that the accuracy of the pXB296 sequence is higher than 99.98%. Using other thermostable sequenases such as AmpliTaq FS (Perkin-Elmer), similar results were expected because thermostable sequenases have similar properties.

Dye primer chemistry in combination with Thermo Sequenase was also examined. Although the peak uniformity of signals was much improved over dye primer/*Taq* DNA polymerase data, the number of compressions in GC-rich shotgun reads was not reduced significantly. Compressions in shotgun raw data enormously increase the overall effort of editing, proofreading, and finishing a cosmid as shown for pXB110 (Table 1).

Because of their longer reading potential, dye primer reads are helpful for gap closure. However, using ABI 373A sequencers (Applied Biosystems, Inc. (ABI), Perkin-Elmer, Foster City, CA, USA), dye primer reads are, on average, only ∼50 bases longer than dye terminator reads.

Using the experimental conditions of the present invention, shotgun sequencing with dye terminators and a thermostable sequenase is superior because for GC-rich cosmid templates it removes most of the compressions and this leads to a several-fold improvement in assembling and finishing of cosmid-sized projects. Although dye terminators are slightly more expensive than dye primers, the overall saving in time for finishing projects has, in our experience, a much greater effect on general costs.

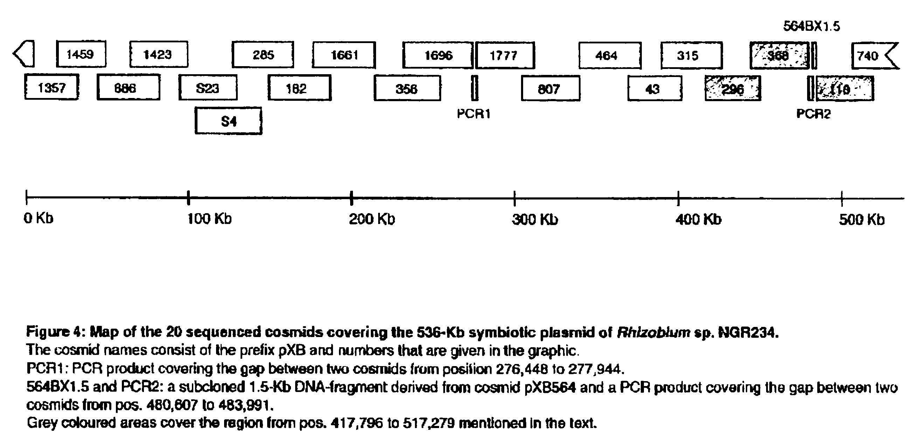

It has been shown that the strategy of the present invention is effective for high-throughput shotgun sequencing of GC-rich templates. This strategy was therefore used to sequence the remaining 19 overlapping cosmids of the symbiotic plasmid pNGR234*a* of *Rhizobium* sp. NGR234. In total, 20 cosmids, two PCR products (1.5 and 2.0 kb in length) and a 1.5 kb restriction fragment were sequenced in order to generate the complete pNGR234*a* sequence (Figure 4).

### Genetic Organization of pXB296

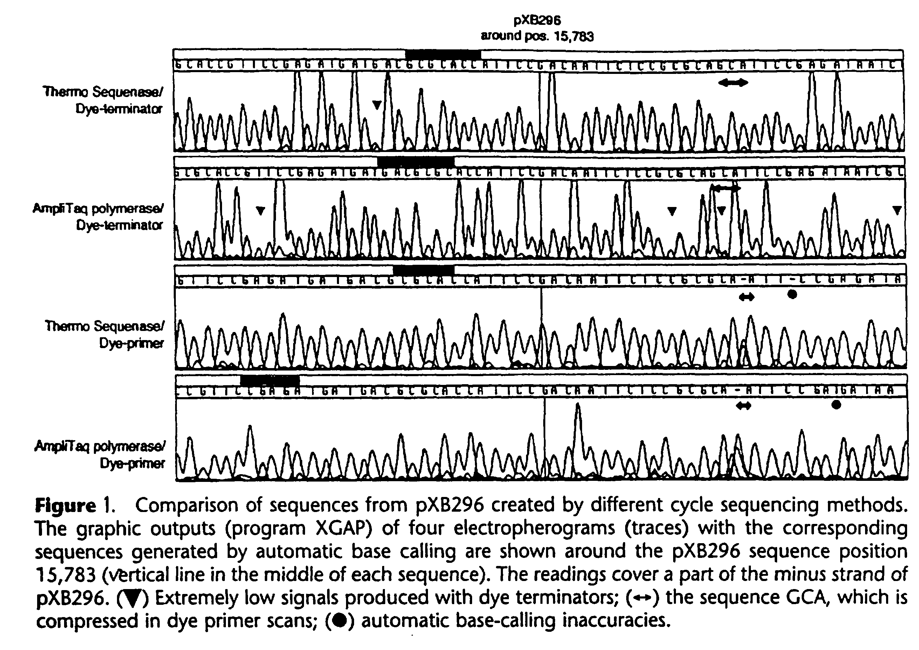

All 28 predicted open reading frames (ORFs) in pXB296 (Figure 1) show significant homologies to database entries. The first putative gene cluster (cluster I) containing ORF1 to ORF5 corresponds to various oligopeptide permease operons (Hiles *et al*., 1987; Perego *et al.*, 1990). Only ORF5 shows

**Table 1**

| **Comparison of the assembly of the sequence data from cosmids pXB296 (dye terminator shotgun reads) and pXB110 (dye primer shotgun reads)** | | |
|---|---|---|
| Data assembly | pXB296 | pXB110 |
| Average length of the shotgun reads (bases) | 332 | 378 |
| No. of shotgun reads used for assembly | 786 | 899 |
| No. of shotgun reads assembled with 4% mismatch^{a} | 736 | 308 |
| No. of shotgun reads assembled with 25% mismatch^{a} | 775 | 879 |
| No. of contigs^{b} longer than 1 kbp | 3 | 25 |
| No. of contigs left after editing^{c} | 2 | 4 |
| No. of additional reads (gap closure and proofreading)^{d} | 32 | 191 |
| Total length of cosmid insert (bp) | 34,010 | 34,573 |
| Sequencing redundancy (per bp) | 8.0 | 10.5 |

| | | |
|---|---|---|
| ^{a}Assembling program: XGAP; principal autoassembling conditions: normal shotgun assembly, joins permitted, minimum initial match = 15, maximum no. of pads per reading during the alignment procedure = 8, maximum no. of pads per reading in contig to align any new reading = 8, alignment mismatches 4% and 25%, respectively. | | |
| ^{b}Contiguous parts of sequence created by overlapping reads. | | |
| ^{c}Lengths of contigs: 6-10 kbp (pXB296); 2-12 kbp (pXB110). | | |
| ^{d}Reads necessary for closing gaps and making single-stranded regions double-stranded by primer walking on selected templates and, in case of pXB110, for solving ambiguities (compressions) by the resequencing of clones with universal primer and dye terminators. | | |

homology to a gene from a different bacterium, *Bacillus anthracis* (Makino *et al*., 1989). Each homologue encodes membrane-bound or membrane-associated proteins suggesting that all five ORFs are involved in oligopeptide permeation.

Organization of the predicted gene cluster IV, including the *nifA* homologue ORF16 (*fixABCX*, *nifA*, *nifB*, *fdxN*, *ORF*, *fixU* homologues, position 16,746 - 24,731), the predicted locations of the σ⁵⁴-dependent promoters and the *nifA* upstream activator sequences (Figure 1), correspond to the organization found in *Rhizobium meliloti* and *Rhizobium leguminosarum* bv. *trifolii*. (Iismaa *et al*., 1989; Fischer, 1994). NifA is a positive transcriptional activator (Buikema *et al.*, 1985), whereas *nif* and *fix* genes are essential for symbiotic nitrogen fixation. Identification of σ⁵⁴-dependent promoter sequences, together with the upstream activator motifs upstream of ORF21, ORF22, and ORF23, suggests that these ORFs may play an important, but still undefined, role in symbiosis.

Inevitably, large-scale sequencing uncovers differences with already published sequences. van Slooten *et al*. (1992) cloned a 5.8 kb *Eco*RI fragment from *Rhizobium* sp. NGR234 and sequenced 2067 bp by manual radioactive methods (EMBL accession no. S38912). This sequence exhibits 2.4% mismatches with the corresponding sequence in pXB296. It contains the gene *dctA* (encoding a C₄-dicarboxylate permease), which is 144 bases shorter than in pXB296. In this respect, a single nucleotide deletion in position 29,248 of the cosmid sequence close to the 3' end of the gene causes a frameshift leading to a DctA product extended by 48 residues. van Slooten *et al*. (1992) also failed to identify the *nifQ* homologue, ORF23 (position 27,169 - 27,861), presumably because they overlooked a small *Xho*I fragment located between positions 27,349 and 27,536 on pXB296. Expression studies allowed these investigators to define a putative σ⁵⁴-dependent promoter in a 1.7 kb *Sma*I fragment (position 27,094 - 28,818 in pXB296). This fragment stretches from the upstream region of ORF23 to the 5' part of *dctA*. The 58 bp intergenic region between ORF23 and *dctA* contains a stem-loop structure but no obvious promoter sequence. Possibly the promoter that controls *dctA* is located upstream of ORF23 (e.g. the minimal consensus sequence included in GGGGGCACAATTGC at position 27,098 - 27,111). Although clones containing *dctA* complemented mutants of *R. meliloti* and *R. leguminosarum* for growth on dicarboxylates, the growth of the NGR234 *dctA* deletion mutant was not affected (van Slooten *et al*., 1992). Nevertheless, this mutant was unable to fix nitrogen in nodules. Because *dctA* is now possibly part of a larger transcription unit, the symbiotic phenotype may also result from the inactivation or downstream genes.

Interestingly, the GC content of the predicted pXB296 ORFs ranges from 53.3 mol% to 64.6 mol%, with an overall cosmid GC content of 58.5 mol%. Genomes of *Azorhizobium, Bradyrhizobium*, and *Rhizobium* species have GC contents of 59 mol% to 65 mol% (Padmanabhan *et al.,* 1990), with 62 mol% reported for *Rhizobium* sp. NGR234 (Broughton *et al*., 1972). Although pXB296 covers <7% of the complete symbiotic plasmid sequence, its lower overall GC value suggests that symbiotic genes might have evolved by lateral transfer from other organisms. In this case, methods of the type applied in the present invention will become even more relevant in sequencing the whole genome.

### Genetic Organization of the 100 kb region covered by cosmids pXB296, pXB368 and pXB110

Extending the analysis of pXB296 to a 100 kb region stretching from position 417,796 to 517,279 on the symbiotic plasmid pNGR234*a* has led to the assignation of the 76 ORFs listed in Table 4 (excluding the first incomplete ORF noted in the analysis of pXB296 ("ORF1" of Table 3)). The ORFs K1 to L4 are identical to the ORFs 2 to 28 of the analysis of pXB296 (Table 3). The cosmid pXB110, which was sequenced with the dye primer shotgun sequencing strategy in order to compare it with the dye terminator shotgun sequencing strategy used to sequence cosmids pXB296 and pXB368, covers nearly this entire region. A PCR product and a restriction fragment of cosmid pXB564 also had to be sequenced in order to fill in the gap from position 480,607 to 483,991 between cosmids pXB368 and pXB110 (Figure 4). Among the predicted ORFs, 7 ORFs and their deduced proteins show no homologies to database entries. The other predicted ORFs and their deduced proteins do exhibit such homologies and therefore play putative roles in nitrogen fixation (ORFs K11 to K19, K22, L8 to L12, L23 and L24), nodulation (ORFs M15 and M20), transportation (ORFs K1 to K4, K23 and L25), secretion of proteins or other biomolecules (ORFs M21 to M27), transcriptional regulation/DNA binding (ORFs L16 and M6), in amino acid metabolism or metabolism of amino acid derivatives (ORFs K5, K6, K8, L17, L18 and M11 to M14), degradation of xenobiotic compounds (ORFs L1 to L3), in peptidolysis/proteolysis (ORFs L14 and L15) or transposition (ORFs K7, K9 and K10). The role of some ORFs like the luciferase-like ORFs (L4 and L19) in rhizobia is still not clear. In the 100 kb region, the duplication of a 5 kb sequence (position 451,886 to 456,157 and 483,764 to 488,035) including the genes *nifHDK* is remarkable. These genes encode the basic subunits of the nitrogenase. Furthermore, the transcriptional regulator *nodD2* is very interesting because its role seems not to be identical to a previously identified *nodD2* in a closely related strain (Appelbaum *et al*., 1988; data not shown). Also the *pmrA*-homologous ORF M6 putatively plays an important role in regulating symbiotic processes because a *nod* box (binding region for the basic regulator *nodD1*; Fisher and Long, 1993) is located upstream of this ORF (position 493,962 to 494,000). Finally, the presence of ORFs (M21 and M23 to M26) homologous to type III secretion proteins, which have only been known previously in plant or animal/human pathogenic bacteria, shows that there only seems to be a subtle difference between symbiotic and pathogenic abilities of microorganisms.

In a second stage, the remaining 436 kb of pNGR234*a* were analyzed. Several ORFs and their deduced proteins were identified that belong to functional groups not previously identified in the analysis of cosmids pXB296, pXB368 and pXB110 (replication of the plasmid, conjugal transfer of the plasmid, functions in oligosaccharide biosynthesis and cleavage, functions in sugar or sugar-derivative metabolism, functions in lipid or lipid-derivative metabolism, functions in chemoperception/chemotaxis, functions in biosynthesis of cofactors, prosthetic groups and carriers, etc.).

Although functional analyses of selected ORFs in pNGR234*a* still have to be performed, large-scale sequencing gives a global picture of their genomic organization and possible roles. Determination of putative functions of predicted genes by homology searches and identification of sequence motifs (promoters, *nod* boxes, *nifA* activator sequences, and other regulatory elements) will aid in finding new symbiotic genes. High-fidelity sequence data covering long stretches of the genome are a prerequisite for these studies. The use of the dye terminator/thermostable sequenase shotgun approach has allowed the completion of the entire ∼500 kb sequence of pNGR234*a* and has opened up new avenues for the genetic analysis of symbiotic function.

### Example 1

### GENERAL METHODS

### Bacteria and Plasmids

*Escherichia coli* was grown on SOC, in TB or in twofold YT medium (Sambrook *et al.*, 1989). The cosmid clones pXB296 and pXB110 (Perret *et al.*, 1991) were raised in *E. coli* strain 1046 (Cami and Kourilsky, 1978). Subclones in M13mp18 vectors (Yanisch-Perron *et al.*, 1985) were grown in *E. coli* strain DH5αF'IQ (Hanahan, 1983).

### Construction of Cosmid Libraries

Cosmid DNA was prepared by standard alkaline lysis procedures followed by purification in CsCl gradients (Radloff *et al.*, 1967). DNA fragments sheared by sonication of 10 µg of cosmid DNA were treated for 10 min at 30°C with 30 units of mung bean nuclease (New England Biolabs, Beverly, MA, USA), extracted with phenol/chloroform (1:1), and precipitated with ethanol. DNA fragments, ranging in size from 1 to 1.4 kbp, were purified from agarose gels using Geneclean II (Bio101, Vista, CA, USA) and ligated into *Sma*I-digested M13pm18. Electroporation of aliquots of the ligation reaction into competent *E. coli* DH5αF'IQ was performed according to standard protocols (Dower *et al.,* 1988; Sambrook *et al.*, 1989).

### M13 Template Preparation

Fresh 1 ml *E. coli* cultures in twofold YT held in 96-deep-well microtiter plates (Beckman Instruments, Fullerton, CA, USA) were infected with recombinant phages from white plaques grown on plates containing X-gal (5-bromo-4-chloroindoyl-β-D-galactoside) and IPTG (isopropyl-β-thiogalactopyranoside). Rapid preparation of ∼0.5 µg of single-stranded M13 template DNA was carried out as follows: 190 µl portions of the phage cultures grown for 6 hr at 37°C were transferred into 96-well microtiter plates. Lysis of the phages was obtained by adding 10 µl of 15% (w/v) SDS followed by 5 min incubation at 80°C. Template DNA was trapped using 10 µl (1 mg) of paramagnetic beads (Streptavidin MagneSphere Paramagnetic Particles Plus M13 Oligo, Promega, Madison, WI, USA) and 50 µl of hybridization solution [2.5 M NaCl, 20% (w/v) polyethylene glycol (PEG-8000)] during an annealing step of 20 min at 45°C. Beads were pelleted by placing microtiter plates on appropriate magnets and washing three times with 100 µl of 0.1-fold SSC. The DNA was recovered in 20 µl of water by a denaturation step of 3 min at 80°C. When required, larger amounts of single-stranded recombinant DNA (>10µg) were purified using QIAprep 8 M13 Purification Kits (Qiagen, Hilden, Germany) from 3 ml of supernatant of phage cultures grown for 6 hr at 37°C.

### Sequencing

Two sequencing methods were used: dye terminator and dye primer cycle sequencing, each in combination with AmpliTaq DNA polymerase (Perkin-Elmer) and Thermo Sequenase (Amersham). All reactions, including ethanol precipitation, were performed in microtiter plates. Reagents were pipetted using 12-channel pipettes. Where necessary, sequencing reaction mixtures, including enzymes, were pipetted into the plates in advance and held at -20°C until needed.

### Dye Terminator Cycle Sequencing

For dye terminator/AmpliTaq DNA polymerase sequencing, 0.5 µg of template DNA, and the PRISM Ready Reaction DyeDeoxy Terminator Cycle Sequencing Kit (Perkin-Elmer) were used. Cycle sequencing was performed in microtiter plates using 25 PCR cycles (30 sec at 95°C, 30 sec at 50°C, and 4 min at 60°C). Prior to loading the amplified products on electrophoresis gels, unreacted dye terminators were removed using Sephadex columns scaled down to microtiter plates (Rosenthal and Charnock-Jones, 1993).

Dye terminator/Thermo Sequenase sequencing was performed using the same experimental conditions except that the reaction mix contained 16.25 mM Tris-HCl (pH 9.5), 4.0 mM MgCl₂, 0.02% (v/v) NP-40, 0.02% (v/v) Tween 20, 42 µM 2-mercaptoethanol, 100 µM dATP/dCTP/dTTP, 300 µM dITP, 0.017 µM A/0.137 µM C/0.009 µM G/0.183 µM T from Taq Dye Terminators (Perkin-Elmer; no. A5F034), 0.67 µM primer, 0.2-0.5 µg of template DNA, and 10 units of Thermo Sequenase (Amersham) in a 30 µl reaction volume. Unincorporated dye terminators were removed from reaction mixtures by precipitation with ethanol.

### Dye Primer Cycle Sequencing

Dye primer/AmpliTaq DNA polymerase sequencing reactions were performed according to the instructions accompanying the Taq Dye Primer, 21M13 Kit (Perkin-Elmer). Cycle sequencing was carried out on 0.5 µg of template DNA with 19 PCR cycles (30 sec at 95°C, 30 sec at 50°C, and 90 sec at 72°C) followed by six cycles, each consisting of 95°C for 30 sec and 72°C for 2.5 min. Prior to electrophoresis, the four base-specific reactions were pooled and precipitated with ethanol.

Identical PCR conditions and the Thermo Sequenase Fluorescent Labelled Primer Cycle Sequencing Kit (Amersham) were used for dye primer/Thermo Sequenase sequencing reactions.

### Sequence Acquisition and Analysis

Gel electrophoresis and automatic data collection were performed with ABI 373A DNA sequencers (Perkin-Elmer). After removing cosmid vector and M13mp18 sequences from the shotgun sequence data, the data were assembled using the program XGAP (Dear and Staden, 1991) and edited against the fluorescent traces. To close remaining gaps, to make single-stranded regions double-stranded, and to clarify ambiguities, additional cycle sequencing reactions with selected shotgun templates were carried out using either custom-made primers (primer-walks) or universal primer.

The complete double-stranded DNA sequence of cosmid pXB296 was analyzed using programs from the Wisconsin Sequence Analysis Package (v. 8, Genetics Computer Group, Madison, WI, USA). Homology searches were performed with BLAST (v. 1.4; Altschul *et al.,* 1990) and FASTA (v. 2.0; Pearson and Lipman, 1988). Several nucleotide and protein databases were screened (GenBank/Genpept, SwissProt, EMBL, and PIR). Identities and similarities between homologous amino acid sequences were calculated with the alignment program BESTFIT (Smith and Waterman, 1981).

### Example 2

### Comparison of Fluorescent Traces Created by Different Cycle Sequencing Methods

When using a thermostable sequenase [Thermo Sequenase (Amersham)], the concentrations of dye terminators (Perkin-Elmer) can be reduced by 20- to 250-fold in comparison to the concentrations needed for *Taq* DNA polymerase without compromising the quality of the sequencing results (Table 2).

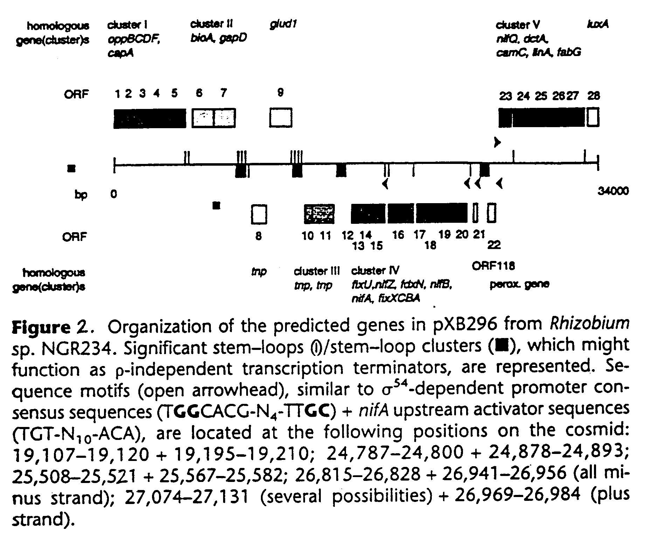

To compare the dye terminator and dye primer cycle sequencing procedures, representative templates derived from the pXB296 library were sequenced by both methods, each performed with Thermo Sequenase and *Taq* DNA polymerase (Figure 2). In general, dye terminator traces do not contain the many compressions (on average, one compression every 50 bases in single reads) that are common with dye

**Table 2**

| **Concentrations (in µM) of dye terminators in each cycle sequencing reaction with two different thermostable DNA polymerases** | | | |
|---|---|---|---|
| Dye terminator | AmpliTaq DNA polymerase | Thermo Sequenase DNA polymerase | Dilution factor for dye terminators^{a} |
| A *Taq* | 0.751 | 0.017 | 40 |
| C *Taq* | 22.500 | 0.137 | 160 |
| G *Taq* | 0.200 | 0.009 | 20 |
| T *Taq* | 45.000 | 0.183 | 250 |

| | | | |
|---|---|---|---|
| ^{a}Thermo Sequenase vs. AmpliTaq. | | | |

primers if mixes do not contain nucleotide analogues like deoxyinosine or 7-deaza-deoxyguanosine triphosphates or if sequencers are used without active heating systems. In addition, dye terminator traces obtained with Thermo Sequenase show more uniform signal intensities over those obtained with *Taq* DNA polymerase, thus resulting in a reduced number of weak and missing peaks (e.g. a weak G-signal following an A-signal in Thermo Sequenase traces or a weak C-signal following a G-signal in *Taq* DNA polymerase traces). Using ABI 373A sequencers, errors in automatic base-calling of Thermo Sequenase/dye terminator scans only arise after 300-350 bases. The average number of resolved bases in dye primer gels (378 bases) is 46 bases longer than in those produced with dye terminators (332 bases). Furthermore, in Thermo Sequenase/dye primer sequences the peaks are very regular and the number of stops and missing bases decreases in comparison to *Taq* DNA polymerase/dye primer electropherograms. The number of compressions, however, is not significantly reduced.

### Example 3

### Shotgun Sequencing of Entire Cosmids Using Dye Terminators or Dye Primers

To compare the efficiency of both methods, cosmid pXB296 of pNGR234*a* was shotgun sequenced using a combination of dye terminators and thermostable sequenase (Thermo Sequenase), whereas another cosmid, pXB110, was sequenced using a combination of dye primers and *Taq* DNA polymerase (Table 1). Over 93% (736 clones) of 786 dye terminator reads of pXB296 were accepted by XGAP with a maximal alignment mismatch of 4%. By increasing this level to 25%, so that most of the remaining data could be included in the assembly, 775 reads led to three 6 to 10 kbp stretches of contiguous sequence (contigs), two of which were joined after editing. To close the last gap and to complete single-stranded regions with data derived from the opposite strand, only 32 additional dye terminator reads using custom-made primers were required. It took <1 week to assemble and finalize the 34,010 bp DNA sequence of pXB296 (EMBL accession no. Z68203; eightfold redundancy; GC content, 58.5 mol%).

In contrast, only 308 (34%) of 899 shotgun reads obtained by *Taq* DNA polymerase/dye primer cycle sequencing of pXB110 were included in the first assembly (4% alignment mismatch). At the 25% alignment mismatch level, 879 reads were assembled, leading to 25 short contigs (1-2 kbp). These contigs had to be edited extensively in order to join most of them. "Primer walks", covering gaps and complementing single-stranded regions, were not sufficient to clarify all the remaining ambiguities in the assembled sequence. Every 100-150 bp, a compression in one strand could not be resolved by sequence data from the complementary strand. Therefore, it was necessary to resequence clones using dye terminators and universal primer. In total, 191 additional dye terminator reads had to be created. As a result, assembling and finalizing the 34,573 bp sequence of pXB110 (10.5-fold redundancy; GC content, 58.3 mol%) took much more time than pXB296 did.

### Example 4

### Analysis of Cosmid pXB296

Putative ORFs were located on the 34,010 bp sequence of pXB296 using the programs TESTCODE (Fickett, 1982) and CODONPREFERENCE (Gribskov *et al.*, 1984), the latter in combination with a codon frequency table based on previously sequenced genes of *Rhizobium* sp. NGR234 (as well as the closely related *R. fredii*). All 28 ORFs and their deduced amino acid sequences exhibited significant homologies to known genes and/or proteins. The positions of the ORFs along pXB296, as well as the best homologues, are displayed in Table 3 and Figure 1. Ribosomal binding site-like sequences (Shine and Dalgarno, 1974) precede each putative ORF except for ORF9 (position 11,214-12,455). If one disregards the homology to known glutamate dehydrogenases in the first 32 amino acids deduced from this ORF, a downstream alternative start codon (position 11,220) preceded by a Shine-Dalgarno sequence can be identified. Most of the ORFs are organised in five clusters (ORFs with only short intergenic spaces or overlaps between them). Cluster I, containing ORF1 to ORF5, encodes proteins homologous to *trans*-membrane and membrane-associated oligopeptide permease proteins and to a *Bacillus anthracis* encapsulation protein. Cluster II, includes ORF6 and ORF7, which are homologous to aminotransferase and (semi)aldehyde dehydrogenase genes. Homologies to transposase genes [ORF8; cluster III (ORF10 and ORF11)] and to various *nif* and *fix* genes [cluster IV (ORF12 to ORF20); ORF23, part of cluster V] are also reported.

Presumed promoter and stem-loop sequences that might represent ρ-independent terminator-like structures (Platt, 1986) are shown in Figure 2. Significant σ⁵⁴-dependent promoter consensus sequences (5'-TGGCACG-N₄-TTGC-3'; Morett and Buck, 1989), as well as *nifA* upstream activator sequences (5'-TGT-N₁₀-ACA-3'; Morett and Buck, 1988), are found upstream of the *nifB* homologue ORF15, the *fixA* homologue ORF20, ORF21, ORF22, and ORF23. ORF23 is part of cluster V in pXB296, which includes the *dctA* gene of *Rhizobium* sp. NGR234 (van Slooten *et al.*, 1992). Surprisingly, the published *dctA* sequence shows important discrepancies. Therefore, a fragment encompassing this locus was amplified by PCR using NGR234 genomic DNA as template. By sequencing this fragment, the cosmid sequence of the present invention was confirmed.

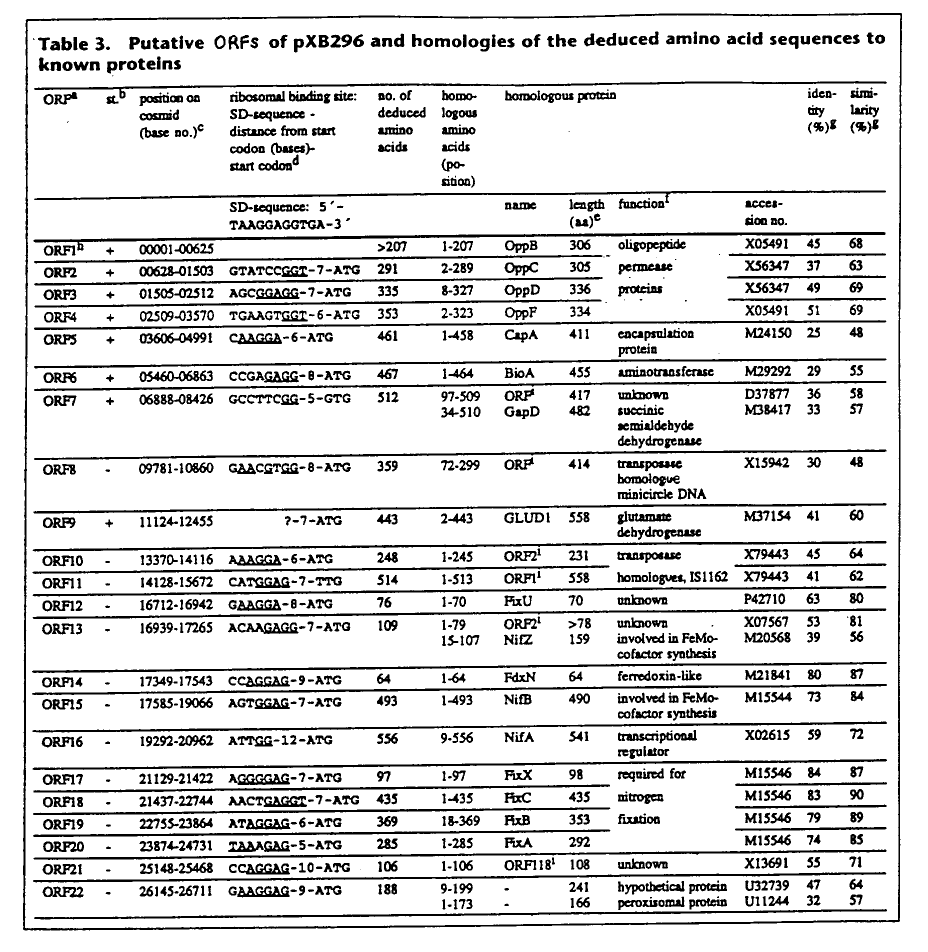

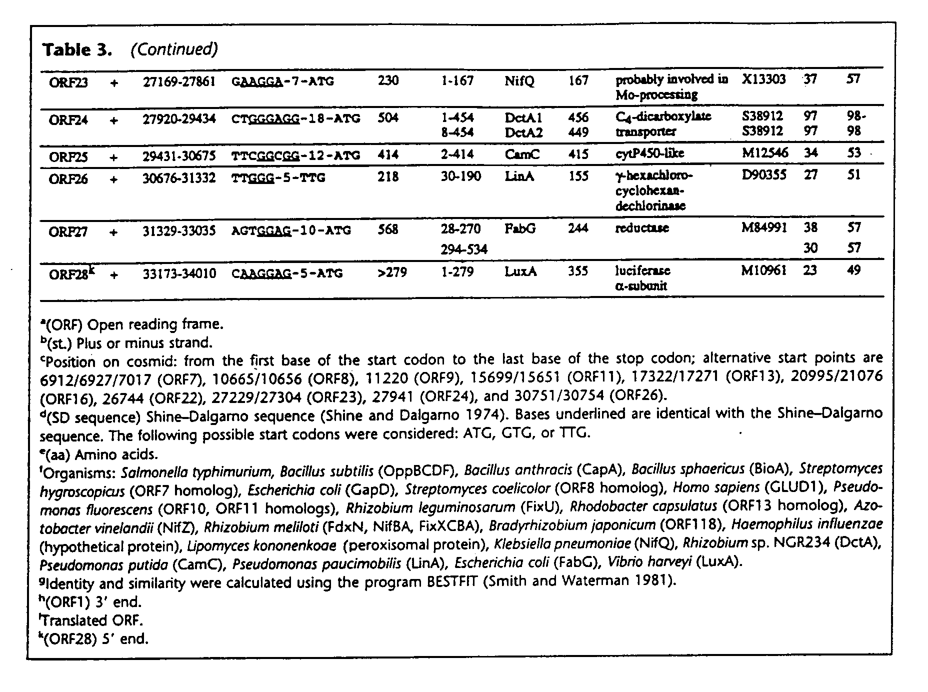

### Example 5

### Analysis of the Complete Plasmid pNGR234a

Using the thermostable sequenase/dye terminator cycle sequencing method herein described, 20 overlapping cosmids (including pXB296) of the symbiotic plasmid pNGR234*a* of *Rhizobium* sp. NGR234 were sequenced, together with two PCR products and a subcloned DNA fragment derived from cosmid pXB564 that cover two remaining gaps (position 276,448-277,944 and position 480,607-483,991). The map of the sequenced cosmids is shown in Figure 4. The entire assembled 536 kb sequence of pNGR234*a* is given in Figure 3.

The analysis of the complete nucleotide sequence revealed few regions of 98-100% identity to already published sequences in public databases. These sequences are listed in Table 5. These sequences had been derived either from *Rhizobium* sp. NGR234, derivatives of it or closely related strains of it. Therefore, the ORFs and their deduced proteins, 98-100% homologous to *nifH*, *nodA*, *nodB*, *nodC*, *nodD1*, *nodS*, *nodU*, *nolX*, *nolW*, *nolB*, *nolU* and "ORF1", represent already known genes/proteins (Table 5 and References). Some other ORFs and their deduced proteins, nearly identical to public database entries, were either only partially known before the disclosure of the present invention or exhibited significant differences, for instance, *dctA*, *host-inducible* gene *A*, *nifD*, *nifK*, *nodD2*, *nolT*, *nolX*, *nolV*, "ORF140", "ORF91", "RSRS9 25 kDa-protein gene" (Table 5 and References).

As a first step, approximately 100 kb of pNGR234*a* was analyzed between position 417,796 to 517,279 using the programs TESTCODE (Fickett, 1982) and CODONPREFERENCE (Gribskov *et al*., 1984). In this ∼100 kb of sequence, 76 ORFs were found and ascribed putative functions (see Table 4). It should be noted that since the sequence of cosmid pXB296 forms part of this 100 kb region, all of the ORFs

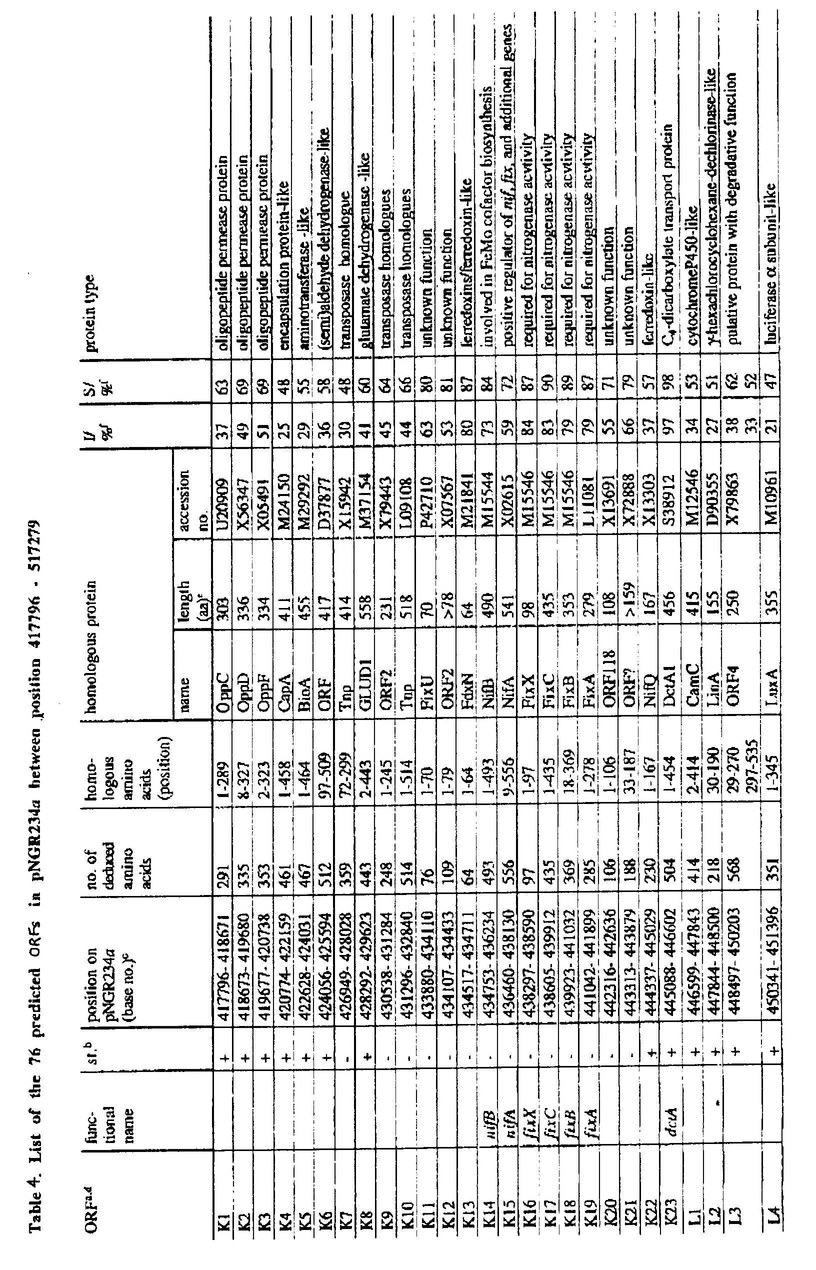

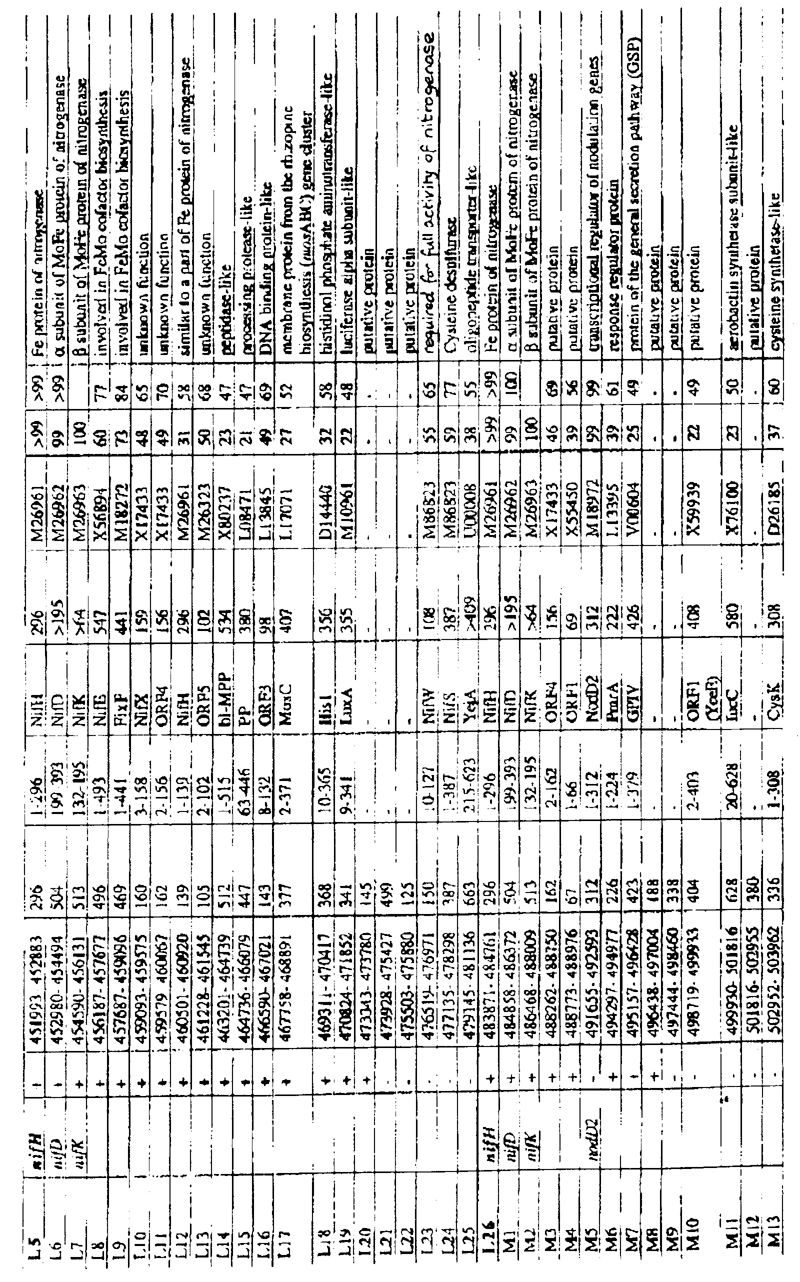

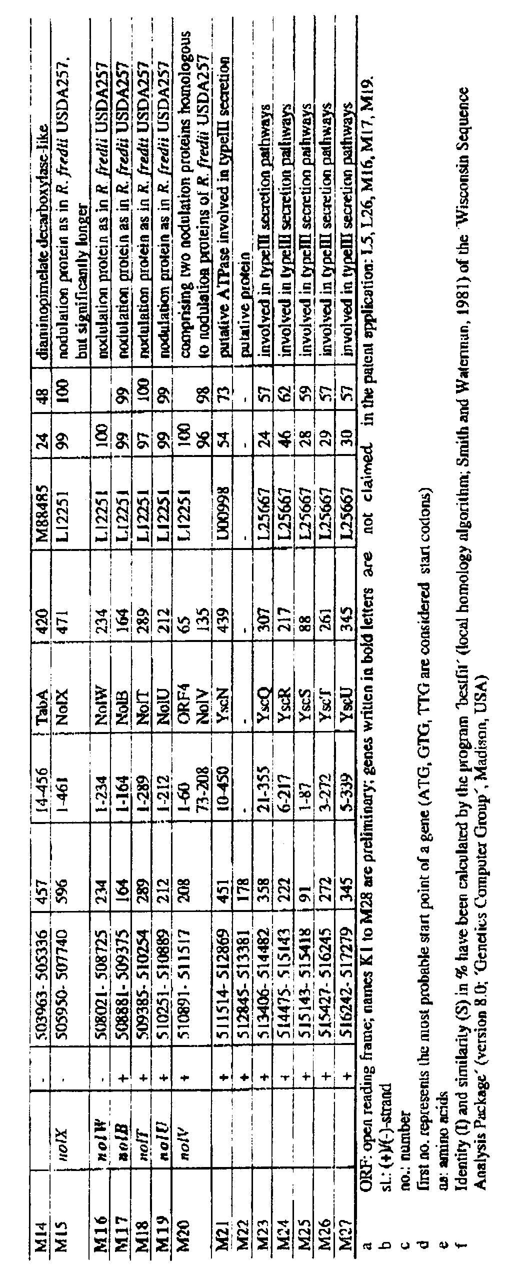

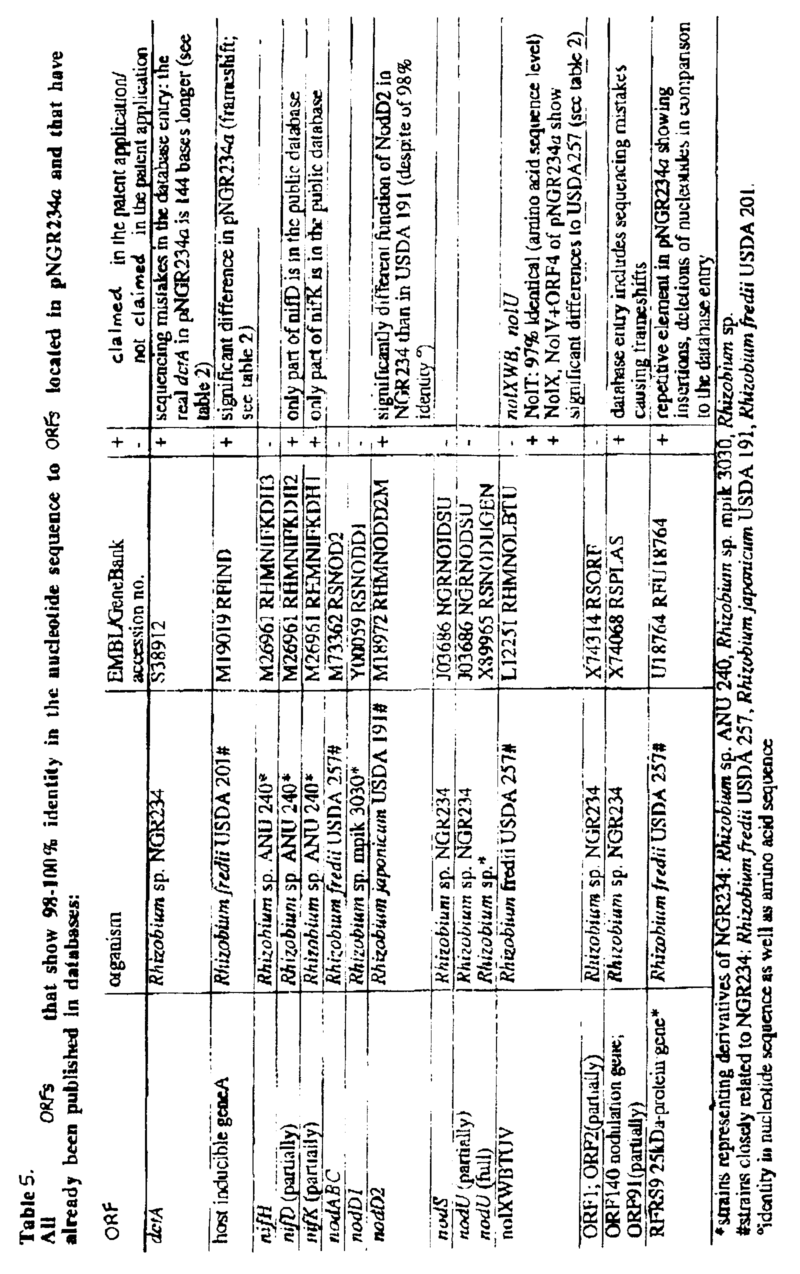

identified in Table 3 (except "ORF1") are reproduced in Table 4. Most of the 76 ORFs and their deduced proteins showed homologies to public database entries that could help identify their putative functions. Only ORFs L5 and L26 (duplicated *nifH*) as well as M16, M17 and M19 (*nolW*, *nolB* and *nolU*) were identical to database entries (98-100% homology). In the case of 7 ORFs and their deduced proteins, no homologous sequences in public databases have been found.

As a second step, the remaining 436 kb of pNGR234a were analyzed using the methods noted above.

### REFERENCES

Altschul, S.F., G. Warren, W. Miller, E.M. Myers, and D.J. Lipman. 1990. Basic local alignment search tool. *J. Mol. Biol.* **215**: 403-410.
Appelbaum, E. R., D.V. Thompson, K. Idler and N. Chartrain. 1988. *Rhizobium japonicum* USDA1 191 has two *nodD* genes that differ in primary structure and function. *J. Bacteriol*. **170**: 12-20.
Badenoch-Jones, J., T.A. Holton, C.M. Morrison, K.F. Scott and J. Shine. 1989. Structural and functional analysis of nitrogenase genes from the broad host-range *Rhizobium* strain ANU240. *Gene* **77**: 141-153.
Bender, G.L., M. Nayudu, K.K.L. Strange and B.G. Rolfe. 1988. The *nodD1* gene from *Rhizobium* strain NGR234 is a key determinant in the extension of host-range to the non-legume *Parasponia. Mol. Plant-Microbe Interact*. **1**: 259.
Bodmer, W.F. 1994. The Human Genome Project. *Rev. Invest. Clin*. (Suppl.) 3-5.
Broughton, W.J., M.J. Dilworth, and I.K. Passmore. 1972. Base ratio determination using unpurified DNA. *Anal. Biochem.* **46**: 164-172.
Broughton, W.J., N. Heycke, H. Meyer z.A., and C.E. Pankhurst. 1984. Plasmid-linked *nif* and *"nod"* genes in fast growing rhizobia that nodulate *Glycine max*, *Psophocarpus tetragonolobus*, and *Vigna unguiculata. Proc. Natl. Acad. Sci. USA*. **81:** 3093-3097.
Broughton, W.J. C-H. Wong, A. Lewin, U. Samrey, H. Myint, H. Meyer z.A., D.N. Dowling, and R. Simon. 1986. Identification of *Rhizobium* plasmid sequences involved in recognition of *Psophocarpus*, *Vigna*, and other legumes. *J*. *Cell Biol*. **102:** 1173-1182.
Buikema, W.J., W.W. Szeto, P.V Lemley, W.H. Orme-Johnson, and F.M. Ausubel. 1985. Nitrogen fixation specific regulatory genes of *Klebsiella pneumoniae* and *Rhizobium meliloti* share homology with the general nitrogen regulatory gene *ntrC* of *K. pneumoniae. Nucleic Acids Res*. **13:** 4539-4555.
Cami, B. and P. Kourilsky. 1978. Screening of cloned recombinant DNA in bacteria by in situ colony hybridization. *Nucleic Acids Res.* **5:** 2381-2390.
Craxton, M. 1993. Cosmid sequencing. *Methods Mol. Biol.* **23:** 149-167.
Dear, S. and R. Staden. 1991. A sequence assembly and editing for efficient management of large projects. *Nucleic Acids Res.* **19:** 3907-3911.
Davis, E.O. and A.W.B. Johnston. 1990. Regulatory functions of the 3 *nodD* genes of *Rhizobium leguminosarum* bv*. phaseoli. Mol*. *Microbiol*. **4:** 933-941.
Dower, W.J., J.F. Miller, and C.W. Ragsdale. 1988. High efficiency transformation of *E. coli* by high voltage electroporation. *Nucleic Acids Res.* **16:** 6127-6145.
Fellay, R., P. Rochepeau, B. Reli 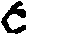 , and W.J. Broughton. 1995. Signals to and emanating from *Rhizobium* largely control symbiotic specificity. In *Pathogenesis and host specificity in plant diseases*. *Histopathological, biochemical, genetic, and molecular bases* (ed. U.S. Singh, R.P. Singh, and K. Kohmoto), Vol. I, pp. 199-220. Pergamon/Elsevier Science Ltd., Oxford, U.K.
Fickett, J.W. 1982. Recognition of protein coding regions in DNA sequences. *Nucleic Acids Res*. **10:** 5303-5318.
Fischer, H.-M. 1994. Genetic regulation of nitrogen fixation in Rhizobia. *Microbiol*. *Rev.* **58:** 352-386.
Fisher, R.F. and S.R. Long. 1993. Interactions of NodD at the *nod* box: NodD binds to two distinct sites on the same face of the helix and induces a bend in the DNA. *J. Mol. Biol*. **233:** 336-348.
Fleischmann, R.D., M.D. Adams, O. White, R.A. Clayton, E.F. Kirkness, A.R. Kerlavage, C.J. Bult, J.F. Tomb, B.A. Dougherty, J.M. Merrick, *et al.* 1995. Whole-genome random sequencing and assembly of *Haemophilus influenzae* Rd. *Science* **269:** 496-512.
Fraser, C.M., J.D. Gocayne, O. White, M.D. Adams, R.A. Clayton, R.D. Fleischmann, C.J. Bult, A.R. Kerlavage, G. Sutton, J.M. Kelley, *et al*. 1995. The minimal gene complement of *Mycoplasma genitalium. Science* **270:** 397-403.
Freiberg, C., X. Perret, W.J. Broughton and A. Rosenthal. 1996. Sequencing the 500-kb GC-rich symbiotic replicon of *Rhizobium* sp. NGR234 using dye terminators and a thermostable sequenase: A beginning. *Genome Research*, in press.
Gribskov, M., J. Devereux, and R.R. Burgess. 1984. The codonpreference plot: Graphic analysis of protein coding sequences and prediction of gene expression. *Nucleic Acids Res.* **12:** 539-549.
Hanahan, D. 1983. Studies on transformation of *Escherichia coli* with plasmids. *J. Mol. Biol.* **166:** 557-580.
Hartl, D.L. and M.J. Palazzolo. 1993. Drosophila as a model organism in genome analysis. In *Genome research in molecular medicine and virology* (ed. K.W. Adolf), pp. 115-129. Academic Press, Orlando, FL, U.S.A.
Hiles, I.D., M.P. Gallagher, D.J. Jamieson, and C.F. Higgins, 1987. Molecular characterization of the oligopeptide permease of *Salmonella typhimurium. J. Mol. Biol.* **195:** 125-142.
Iismaa, S.E., P.M. Ealing, K.F. Scott, and J.M. Watson. 1989. Molecular linkage of the *nif/fix* and *nod* gene regions in *Rhizobium leguminosarum* biovar *trifolii*. *Mol. Microbiol.* **3:** 1753-1764.
Levy, J. 1994. Sequencing the yeast genome: An international achievement. *Yeast* **10:** 1689-1706.
Lewin, A., E. Cervantes, C.-H. Wong and W.J. Broughton. 1990. *nodSU*, two new *nod* genes of the broad host range *Rhizobium* strain NGR234 encode host-specific nodulation of the tropical tree *Leucaena leucocephala. Mol. Plant Microbe Interact*. **3:** 317-326.
Long, S.R. 1989. *Rhizobium*-legume nodulation: life together in the underground. *Cell* **56:** 203-214.
Long, S., J.W. Reed, J. Himawan and G.C. Walker. 1988. Genetic analysis of a cluster of genes required for synthesis of the calcofluor-binding exopolysaccharide of *Rhizobium meliloti. J. Bacteriol.* **170:** 4239-4248.
Makino, S.-I., I. Uchida, N. Terakado, C. Sasakawa, and M. Yoshikawa. 1989. Molecular characterization and protein analysis of the cap region, which is essential for encapsulation in *Bacillus anthracis. J. Bacteriol* **171:** 722-730.
Martinez, E., D. Romero, and R. Palacios. 1990. The Rhizobium genome. *Crit. Rev. Plant Sci.* **9:** 59-93.
Morett, E. and M. Buck. 1988. NifA-dependent in *vivo* protection demonstrates that the upstream activator sequence of *nif* promoters is a protein binding site. *Proc. Natl. Acad. Sci. USA*. **85:** 9401-9405.
Morett, E. and M. Buck. 1989. *In vivo* studies on the interaction of RNA polymerase-σ⁵⁴ with the *Klebsiella pneumoniae and Rhizobium meliloti nifH* promoters: The role of *nifA* in the formation of an open promoter complex. *J*. *Mol. Biol.* **210:** 65-77.
Padmanabhan, S., R.-D. Hirtz, and W.J. Broughton. 1990. Rhizobia in tropical legumes: Cultural characteristics of *Bradyrhizobium* and *Rhizobium* sp*. Soil Biol. Biochem.* **22:** 23-28.
Pearson, W.R. and D.J. Lipman. 1988. Improved tools for biological sequence comparison. *Proc. Natl. Acad. Sci.* **85:** 2444-2448.
Perego, M., C.F. Higgins, S.R. Pearce, M.P. Gallagher, and J.A. Hoch. 1991. The oligopeptide transport system of *Bacillus subtilis* plays a role in the initiation of sporulation. *Mol. Microbiol.* **5:** 173-185.
Perret, X., W.J. Broughton, and S. Brenner. 1991. Canonical ordered cosmid library of the symbiotic plasmid of *Rhizobium* species NGR234. *Proc. Natl. Acad. Sci. USA.* **88:** 1923-1927.
Perret, X., R. Fellay, A.J. Bjourson, J.E. Cooper, S. Brenner, and W.J. Broughton. 1994. Subtraction hybridization and shotgun sequencing: A new approach to identify symbiotic loci. *Nuclei Acids Res.* **22:** 1335-1341.
Platt, T. 1986. Transcription termination and regulation of gene expression. *Annu. Rev. Biochem.* **55:** 339-372.
Radloff, R., W. Bauer, and J. Vinograd. 1967. A dye-buoyant-density method for the detection and isolation of closed circular duplex DNA: The closed circular DNA in HELA cells. *Proc. Natl. Acad. Sci. USA.* **57:** 1514-1521.
Reli 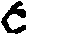 , B., X. Perret, M.T. Estrada-García, J. Kopcinska, W. Golinowski, H.B. Krishnan, S.G. Pueppke and W.J. Broughton. 1994. Nod factors of *Rhizobium* are a key to the legume door. *Mol. Microbiol.* **13:** 171-178.
Rosenthal, A. and D.S. Charnock-Jones. 1993. Linear amplification sequencing with dye terminators. *Methods Mol. Biol.* **23:** 281-296.
Sambrook, J., E.F. Fritsch, and T. Maniatis. 1989. *Molecular cloning: A laboratory manual*, 2nd ed., Cold Spring Harbor Laboratory Press, Cold Spring Harbor, NY, U.S.A.
Shine, J. and L. Dalgarno. 1974. The 3'-terminal sequence of *Escherichia coli* 16S ribosomal RNA: Complementary to nonsense triplets and ribosome binding sites. *Proc Natl. Acad. Sci.* **71:** 1342-1346.
Smith, T.F. and M.S. Waterman. 1981. Identification of common molecular subsequences. *J. Mol. Biol.* **147:** 195-197.
Stanfield, S., L. Ielpi, D. O'Brochta, D.R. Hesinki and G.S. Ditta. 1988. The *ndvA* gene product of *Rhizobium meliloti* is required for Beta(1-2)glucan production and has homology to the ATP binding export protein HlyB. *J. Bacteriol.* **170:** 3523-3530.
Sulston, J, Z. Du, K. Thomas, R. Wilson, L. Hillier, R. Staden, N. Halloran, P. Green, J. Thierry-Mieg, L. Qiu, *et al.* 1992. The *C. elegans* genome sequencing project: A beginning. *Nature* **356:** 37-41.
Tabor, S. and C.C. Richardson. 1995. A single residue in DNA polymerases of the *Escherichia coli* DNA polymerase I family is critical for distinguishing between deoxy- and dideoxyribonucleotides. *Proc. Natl. Acad. Sci.* **92:** 6339-6343.
van Rhijn, P. and J. Vanderleyden. 1995. The *Rhizobium*-plant symbiosis. *Microbiol*. *Rev.* **59:** 124-142.
van Slooten, J.C., T.V. Bhuvanasvari, S. Bardin, and J. Stanley. 1992. Two C₄-dicarboxylate transport systems in *Rhizobium* sp. NGR234: Rhizobial dicarboxylate transport is essential for nitrogen fixation in tropical legume symbioses. *Mol. Plant Microbe Interact.* **5:** 179-186.
Yanisch-Perron, C., J. Ira, and J. Messing. 1985. Improved M13 phage cloning vectors and host strains: Nucleotide sequences of M13mp18 and pUC19 vectors. *Gene* **33:** 103-119.

## Claims

1. The nucleotide sequence as shown in Figure 3 or degenerate variants thereof.

2. The nucleotide sequence of claim 1 which has been altered by mutation, deletion or insertion.

3. ORFs derivable from the nucleotide sequence of claim 1 or claim 2; excluding the ORFs identified as L5, L26, M16, M17 and M19 in Table 4.

4. ORFs K1 to K23, L1 to L25 and M1 to M27 as identified in Table 4; excluding the ORFs identified as L5, L26, M16, M17 and M19 in Table 4.

5. The ORFs of claim 3 or claim 4 which encode the functions of:
(a) nitrogen fixation,
(b) nodulation,
(c) transportation or encapsulation,
(d) secretion (of proteins or other biomolecules),
(e) transcriptional regulation or DNA-binding,
(f) peptidolysis or proteolysis,
(g) transposition or integration,
(h) rhizopine biosynthesis,
or which encode:
(i) proteins exhibiting similarities to proteins of amino acid metabolism or related ORFs, or
(j) proteins putatively involved in the degradation of xenobiotic compounds.

6. The ORFs of any one of claims 3 to 5 which are under the control of their natural regulatory elements or under the control of analogues to such natural regulatory elements.

7. Intergenic sequences derivable from the nucleotide sequence of claim 1 or claim 2.

8. The intergenic sequences of claim 7 which are regulatory DNA sequences or repeated elements.

9. Proteins expressible from the nucleotide sequences or ORFs of any one of claims 1 to 6.

10. Use of the nucleotide sequences or ORFs of any one of claims 1 to 8 or the proteins of claim 9 in the analysis of the structure, organisation or dynamics of other genomes.

11. Use of the nucleotide sequences or ORFs of any one of claims 1 to 8 or the proteins of claim 9 in:
(a) screening nucleotide sequences,
(b) subcloning nucleotide sequences,
(c) amplifying nucleotide sequences by PCR, or
(d) gene trapping.

12. Use according to claim 11, wherein said nucleotide sequences are coding sequences or non-coding sequences.

13. Use according to claim 12, wherein said coding sequences are regulatory sequences or repeated elements.

14. Use according to any one of claims 10 to 13, wherein said nucleotide sequences or ORFs are oligonucleotide primers or hybridization probes.

15. Use of the nucleotide sequences, individual ORFs or groups of ORFs of any one of claims 1 to 8 or the proteins of claim 9 in:
(a) the identification and classification of organisms and their genetic information,
(b) the identification and characterisation of nucleotide sequences, amino acid sequences or proteins,
(c) the transportation of compounds to and from an organism which is host to at least one of said nucleotide sequences, ORFs or proteins,
(d) the degradation and/or metabolism of organic, inorganic, natural or xenobiotic substances in a host organism, or
(e) the modification of the host-range, nitrogen fixation abilities, fitness or competitiveness of organisms.

16. The plasmid comprising the nucleotide sequence of claim 1 or claim 2.

17. A plasmid which harbours at least one ORF of any one of claims 1 to 8 or any degenerate variant thereof or which harbours at least one ORF or any degenerate variant thereof which encodes one or more of the proteins of claim 9.

18. The plasmid of claim 16 or claim 17 produced recombinantly.

19. The plasmid of any one of claims 16 to 18 or any variant thereof produced by mutation, deletion, insertion or inactivation of an ORF, ORFs or groups of ORFs.

20. Use of the plasmid of any one of claims 16 to 19 in:
(a) obtaining a synthetic minimal set of ORFs required for functional *Rhizobium*-legume symbiosis,
(b) the modification of the host-range of rhizobia,
(c) the augmentation of the fitness or competitiveness of *Rhizobium* sp. NGR234 in the soil and its nodulation efficiency on host plants,
(d) the introduction of desired phenotype(s) into host plants using said plasmid as a stable shuttle system for foreign DNA encoding said desired phenotype(s), or
(e) the direct transfer of said plasmid into rhizobia or other microorganisms without using other vectors for mobilization.
